(19) Europäisches Patentamt
European Patent Office
Office européen des brevets

(11) EP 3 033 418 B1

(12) **EUROPEAN PATENT SPECIFICATION**

(45) Date of publication and mention of the grant of the patent:
**11.12.2019 Bulletin 2019/50**

(21) Application number: **14755411.7**

(22) Date of filing: **14.08.2014**

(51) Int Cl.:
***C12N 5/0797*** *(2010.01)* ***A61K 35/30*** *(2015.01)*

(86) International application number:
**PCT/GB2014/052509**

(87) International publication number:
**WO 2015/022545 (19.02.2015 Gazette 2015/07)**

(54) **STEM CELL MICROPARTICLES AND MIRNA**

STAMMZELLENMIKROPARTIKEL UND MIRNA

MICROPARTICULES DE CELLULES SOUCHES ET MIARN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.08.2013 GB 201314573**
**09.10.2013 GB 201317887**

(43) Date of publication of application:
**22.06.2016 Bulletin 2016/25**

(73) Proprietor: **Reneuron Limited**
**Guildford**
**Surrey GU2 7AF (GB)**

(72) Inventors:
- **HICKS, Caroline**
  **Guildford**
  **Surrey GU2 7AF (GB)**
- **SINDEN, John**
  **Guildford**
  **Surrey GU2 7AF (GB)**
- **STEVANATO, Lara**
  **Guildford**
  **Surrey GU2 7AF (GB)**
- **CORTELING, Randolph**
  **Guildford**
  **Surrey GU2 7AF (GB)**

(74) Representative: **Elkington and Fife LLP**
**Prospect House**
**8 Pembroke Road**
**Sevenoaks, Kent TN13 1XR (GB)**

(56) References cited:
**WO-A1-2013/150303 WO-A1-2014/013258**
**WO-A2-03/046141 KR-A- 20100 081 003**

- **DUKJIN KANG ET AL: "Proteomic Analysis of Exosomes from Human Neural Stem Cells by Flow Field-Flow Fractionation and Nanoflow Liquid Chromatography-Tandem Mass Spectrometry", JOURNAL OF PROTEOME RESEARCH, vol. 7, no. 8, 1 August 2008 (2008-08-01), pages 3475-3480, XP055066064, ISSN: 1535-3893, DOI: 10.1021/pr800225z**
- **CHENJIE YANG ET AL: "Immunosuppressive Exosomes: A New Approach for Treating Arthritis", INTERNATIONAL JOURNAL OF RHEUMATOLOGY, vol. 176, no. 12, 1 January 2012 (2012-01-01), pages 7385-8, XP055155835, ISSN: 1687-9260, DOI: 10.1038/labinvest.2010.152**
- **E. VAN DER POL ET AL: "Classification, Functions, and Clinical Relevance of Extracellular Vesicles", PHARMACOLOGICAL REVIEWS, vol. 64, no. 3, 1 July 2012 (2012-07-01), pages 676-705, XP055155968, ISSN: 0031-6997, DOI: 10.1124/pr.112.005983**
- **INDIRA VISHNUBHATLA ET AL: "The Development of Stem Cell-derived Exosomes as a Cell-free Regenerative Medicine", JOURNAL OF CIRCULATING BIOMARKERS, vol. 3, no. 2, 30 April 2014 (2014-04-30), pages 1-14, XP055155696, DOI: 10.5772/58597**
- **Valentina Fonsato ET AL: "Human Liver Stem Cell-Derived Microvesicles Inhibit Hepatoma Growth in SCID Mice by Delivering Antitumor MicroRNAs", Stem Cells, vol. 30, no. 9, 20 September 2012 (2012-09-20), pages 1985-1998, XP055161662, ISSN: 1066-5099, DOI: 10.1002/stem.1161**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **Stefania Bruno ET AL: "Effects of Mesenchymal Stromal Cell-Derived Extracellular Vesicles on Tumor Growth", Frontiers in Immunology, vol. 5, 11 August 2014 (2014-08-11), XP055161795, DOI: 10.3389/fimmu.2014.00382**

## Description

### Field of the Invention

This invention relates to neural stem cell microparticles, their use and production thereof, in particular neural stem cell microparticles and their use in therapy of glioma, in particular glioblastoma.

### Background of the Invention

Stem cells have the ability to self-renew and to differentiate into functionally different cell types. They have the potential to be a powerful therapeutic tool, for example in the growing field of Regenerative Medicine, in particular regenerative therapy requiring tissue replacement, regeneration or repair (Banerjee et al. 2011). Endogenous stem cells have also been implicated as targets (endogenous "cancer stem cells") of anti-cancer therapy, where it is proposed to treat the cancer by eliminating the cancer stem cells that are thought to drive cancer growth and metastasis. More recently, engineered mesenchymal stem cells have been proposed as delivery vehicles in anti-cancer therapy (Dai et al., 2011; Shah et al. 2012). However, there are drawbacks to the use of stem cells in therapy: there is a need for a consistent and substantial supply of stem cells with functional and phenotypic stability and the associated high costs and time delay caused by cell generation, storage, transport and handling; there is a requirement for immunological compatibility to avoid rejection of the stem cells by the recipient; and there are complex regulatory issues related to potential safety risks of tumour or ectopic tissue formation. Further, despite the therapeutic efficacy of stem cell transplantation, there is no convincing evidence for a direct long-term effect of the transplanted stem cells, for example through engraftment and differentiation into reparative or replacement cells.

Neural stem cells (NSCs) are self-renewing, multipotent stem cells that generate neurons, astrocytes and oligodendrocytes (Kornblum, 2007). The medical potential of neural stem cells is well-documented. Damaged central nervous system (CNS) tissue has very limited regenerative capacity so that loss of neurological function is often chronic and progressive. Neural stem cells (NSCs) have shown promising results in stem cell-based therapy of neurological injury or disease (Einstein et al. 2008). Implanting neural stem cells (NSCs) into the brains of post-stroke animals has been shown to be followed by significant recovery in motor and cognitive tests (Stroemer et al. 2009). It is not completely understood how NSCs are able to restore function in damaged tissues but it is now becoming increasingly recognised that NSCs have multimodal repairing properties, including site-appropriate cell differentiation, pro-angiogenic and neurotrophic activity and immunomodulation promoting tissue repair by the native immune system and other host cells (Miljan & Sinden, 2009, Horie et al., 2011). It is likely that many of these effects are dependent on transient signalling from implanted neural stem cells to the host milieu, for example NSCs transiently express proinflammatory markers when implanted in ischaemic muscle tissue damage which directs and amplifies the natural pro-angiogenic and regulatory immune response to promote healing and repair (Katare et al., Clinical-grade human neural stem cells promote reparative neovascularization in mouse models of hindlimb ischemia. Arteriosclerosis, Thrombosis and Vascular Biology, in press). In chronic stroke brain, NSCs also have a substantial neurotrophic effect. For example, they promote the repopulation of the stoke-damaged striatal brain tissue with host brain derived doublecortin positive neuroblasts (Hassani, O'Reilly, Pearse, Stroemer et al., PLoS One. 2012;7(11)).

Furthermore, on the basis of a large body of NSC restorative effects in animal models with chronic stroke, a clinical trial using neural stem cells is being carried out by ReNeuron Limited (Surrey, UK), to trial the treatment of disabled stroke patients using its "CTX0E03" conditionally-immortalised cortex-derived neural stem cells (Clinicaltrials.gov Identifier: NCT01151124).

Mesenchymal stem cells (MSCs) are lineage-restricted stem cells which have the potential to differentiate into mesenchymal cell types only, namely of the adipocytic, chondrocytic and osteocytic lineages (Pittenger et al. 1999; Ding et al. 2011). MSCs (also referred to as Mesenchymal Stromal Cells and Mesenchymal Progenitor Cells) are derived from a variety of sources including bone marrow, blood, adipose and other somatic tissues. The therapeutic potential of MSCs, however, is more directed towards the application of their pro-angiogenic and immune modulating properties as undifferentiated cells. Production of human MSCs is limited by the inability of these cells to expand in numbers stably beyond approximately 15-20 population doublings.

Mesenchymal stem cell-conditioned medium (MSC-CM) has a therapeutic efficacy similar to that of MSCs themselves, suggesting a paracrine mechanism of MSC-based therapy (Timmers et al. 2007). WO-A-2009/105044 discloses that particles known as exosomes, secreted by MSCs, comprise at least one biological property of the MSCs and suggests the use of these MSC particles in therapy, while Théry et al. 2011 provides a general review of exosomes and other similar secreted vesicles. Whereas some of the drawbacks of using stem cells directly as therapeutic agents are overcome by using the mesenchymal stem cell-derived exosomes (e.g. storage, transport and handling), the problem remains of providing a consistent and substantial supply of functionally and phenotypically stable stem cells to produce the exosomes. For therapeutic use, the exosomes preferably need to be produced on a large scale. In the absence of a stem cell line, replenishment of the cells through repeated derivation from a source of stem cells is required, which incurs recurring costs for testing and validation of each new batch. Furthermore, the diseases and disorders that can be treated by MSCs may be limited.

WO-A-2013/150303 and WO-A-2014/013258 disclose microparticles produced by neural stem cells, methods for making those microparticles and uses of those microparticles, in particular for use in regenerative therapy. Fonsato et al (Stem Cells. 2012 Sep;30(9):1985-98) report that microvesicles derived from human adult liver stem cells (HLSC) may reprogram *in vitro* HepG2 hepatoma and primary hepatocellular carcinoma cells by inhibiting their growth and survival.

There remains a need for improved stem cell-based therapies.

## Summary of the Invention

The invention provides a neural stem cell exosome for use in a method of treating glioma, in particular glioblastoma.

The disclosure is based on the surprising finding that neural stem cells contain microparticles that are therapeutically useful. In particular, the inventors have surprisingly identified neural stem cell microparticles that are able to: inhibit cell migration of fibroblasts; inhibit migration of cancer cells; induce differentiation of cancer cells; and/or induce or enhance an immune response against cancer cells. These properties make the neural stem cell microparticles suitable for use in therapy, in particular for treating cancer.

Cell migration is well-known to play an important role in the progression of diseases such as cancer (for example during angiogenesis, tumour formation, metastasis and tissue invasion), fibrosis (for example during the accumulation of fibroblasts in the fibrotic tissue), atherosclerosis and rheumatoid arthritis. Microparticles that inhibit cell migration are therefore useful in the treatment or prevention of diseases that involve unwanted cell migration, such as cancer, in particular metastatic cancer, fibrosis, atherosclerosis and rheumatoid arthritis.

Microparticles of the disclosure are shown, in the Examples, to inhibit fibroblast migration. Fibroblasts and the migration of fibroblasts are known to play a role in angiogenesis and so the microparticles of the disclosure, which inhibit fibroblast migration, are also useful for use in the therapy of unwanted or undesirable angiogenesis.

Additionally, the Examples show that glioblastoma cells, pre-treated in vitro for 24 hours with neural stem cell exosomes did not engraft into the striatum of Balb-C mice *in vivo*. Histopathology demonstrated the presence of necrotic cell bodies at the site of implantation and evidence of a host cellular response. These data indicate that these microparticles are suitable for use in the treatment of cancer, particularly a cancer of the CNS such as a glioblastoma, by promoting the destruction of cancer cells by the immune system.

Neural stem cell microparticles that are able to inhibit fibroblast cell migration and induce or enhance an immune response against cancer cells are also disclosed herein and have been isolated from neural stem cells cultured in a multi-compartment bioreactor for 11 weeks. Accordingly, one way to obtain these neural stem cell microparticles is to isolate them from neural stem cells that have been cultured in a multi-compartment bioreactor for at least 10 weeks, for example 71 days or more. The microparticles of the disclosure may also be obtained from other culture conditions and periods, in particular culture conditions that allow stem cell differentiation.

The Examples further show that tumour (glioblastoma U373) cells show significantly reduced migration when treated with neural stem cell exosomes. The exosomes of the invention may therefore be used to treat a glioblastoma, by inhibiting tumour cell migration.

Additionally, neural stem cell exosomes are shown in the Examples to promote differentiation of tumour (glioblastoma U373) cells *in vitro*. The Examples also show this differentiation *in vivo*, where tumour (glioblastoma U373) cells, treated with neural stem cell exosomes and implanted into mouse brains, demonstrate a reduction in the stem cell marker nestin. Cancer stem cells drive tumourigenesis, are linked with metastasis, high grade and poor prognosis. A more differentiated tumour typically correlates with improved prognosis, so exosomes that are able to effect differentiation are expected to be useful in the treatment of cancer. Therefore, the ability of microparticles isolated from neural stem cells to reduce the stemness of cancer cells indicates that these microparticles are useful in the treatment of cancer, in particular a cancer that is positive for nestin expression such as melanoma, breast cancer or glioblastoma. Typically, the cancer is a cancer of the CNS such as a glioblastoma. Nestin is reported to correlate with aggressive growth, metastasis, and poor prognosis in cancers, so agents that reduce nestin expression are greatly needed. Neural stem cell microparticles that are able to inhibit tumour cell migration and promote differentiation of tumour cells have been isolated from a neural stem cell line cultured under standard conditions. Accordingly, one way to obtain neural stem cell microparticles that are able to inhibit tumour cell migration and promote differentiation of tumour cells is to isolate them from neural stem cells that have been cultured under standard conditions. These cells may be from the CTX0E03 cell line (deposited with the ECACC as Accession No. 04091601). The standard culture conditions typically maintain the characteristics of the cell line, in particular the stemness of the cell line, typically do not permit differentiation, and typically provides proliferating cells. Typically, the cells proliferate with a doubling time of 2 to 4 days and are passaged when sub-confluent.

The Examples include a pilot *in vivo* study of the administration of exosomes of the invention to human glioblastoma xenografts, observing tumour sensitivity to the microparticles, a trend towards a reduction in tumour volume, and increased survival. Histopathology of the tumour cells shows, in one animal, a particularly dramatic and effective ablation of the tumour mass. The Examples also provide Next Generation Sequence (NGS) analysis of the miRNA content of neural stem cell exosomes. One of the Examples revealed the presence of a set of miRNAs: hsa-mir-1246, hsa-mir-4488, hsa-mir-4492, and hsa-mir-4532, each of which is shown to reduce glioma cell proliferation. These data provide further evidence that microparticles containing the miRNAs may be used to treat cancer.

A first aspect of the invention provides a neural stem cell exosome for use in a method of treating glioma. The glioma may be glioblastoma. The neural stem cell exosome may inhibit glioblastoma cell migration; and/or induce differentiation of a glioblastoma cell, typically a glioblastoma cell that is positive for nestin expression. In another aspect described herein, the exosome promotes destruction of glioblastoma tumour cells by inducing or enhancing an immune response against the tumour cells.

A microparticle may be an exosome, microvesicle, membrane particle, membrane vesicle, exosome-like vesicle, ectosome-like vesicle, ectosome or exovesicle. According to the invention, the microparticle is an exosome. The microparticle disclosed herein may be derived from a neural stem cell that has been cultured in an environment that allows stem cell differentiation. The microparticle disclosed herein may or may not be isolated from partially-differentiated neural stem cells; as discussed below, the presence of GFAP (an astrocyte marker) or DCX (an early neuronal marker) on the cells indicates that the neural stem cells have begun to differentiate. As disclosed herein, an environment that allows stem cell differentiation is a multi-compartment bioreactor. The microparticle disclosed herein may be isolated from neural stem cells that have been cultured in a multi-compartment bioreactor for at least 10 weeks. The microparticle may be isolated from cultured neural stem cells that have been confluent on the membrane of a multi-compartment bioreactor for at least one week, at least 2 weeks, typically at least 3 weeks, at least 4 weeks, at least 5 weeks or more. Conversely, microparticles of the invention can be produced from neural stem cells that have not begun to differentiate, for example by isolation from sub-confluent cultured neural stem cells, or by isolation from cells that have been confluent for less than one week on the membrane of a multi-compartment bioreactor or in a standard cell culture flask such as a T-175 flask. As used herein, the term "confluent" is given its usual meaning in the art, wherein the cells in the culture are all in contact and have no further room to grow; confluent cells cover substantially all of the membrane in the multi-compartment bioreactor.

The microparticle may be derived from a neural stem cell line. In some embodiments, the neural stem cell line may be the "CTX0E03" cell line, the "STR0C05" cell line, the "HPC0A07" cell line or the neural stem cell line disclosed in Miljan et al Stem Cells Dev. 2009. In some embodiments, the microparticle is derived from a stem cell line that does not require serum to be maintained in culture. The microparticle may have a size of between 30 nm and 1000 nm, or between 30 and 200 nm, or between 30 and 100 nm, as determined by electron microscopy; and/or a density in sucrose of 1.1-1.2 g/ml. The microparticle may comprise RNA. The RNA may be mRNA, miRNA, and/or any other small RNA. The microparticle may comprise one, two, three or four of hsa-miR-1246, hsa-miR-4492, hsa-miR-4488 and hsa-miR-4532; alternatively, it may comprise 1, 2, 3, 4 or 5 of hsa-miR-181a-5p, hsa-miR-1246, hsa-miR-127-3p, hsa-miR-21-5p and hsa-miR-100-5p; or it may comprise 1, 2, 3, 4 or 5 of hsa-miR-181a-5p, hsa-let-7a-5p, hsa-let-7f-5p, hsa-miR-92b-3p, and hsa-miR-9-5p. The microparticle may comprise one or more lipids, typically selected from ceramide, cholesterol, sphingomyelin, phosphatidylserine, phosphatidylinositol, phosphatidylcholine. The microparticle may comprise one or more tetraspanins, typically CD63, CD81, CD9, CD53, CD82 and/or CD37. The microparticle may comprise one or more of TSG101, Alix, CD109, thy-1 and CD133. The microparticle may comprise at least 10 of the proteins present in Table 20 or Table 22. The microparticle may comprise at least one biological activity of a neural stem cell or a neural stem cell-conditioned medium. At least one biological activity may be an anti-cell migration activity, a pro-differentiation activity or an anti-angiogenic activity. The microparticle of the invention is typically isolated or purified.

The disease to be treated is a cancer of the CNS, namely a glioma. An exemplary glioma is a glioblastoma, which may be a giant cell glioblastoma or a gliosarcoma.

The neural stem cell exosome is used to treat the cancer.

In a further embodiment, the exosomes of the invention treat the cancer by inhibiting migration of the cancer cells.

In yet a further embodiment, the exosomes of the invention treat the cancer by inducing differentiation of the cancer cells. Typically, differentiation is induced in cancer cells that express nestin.

As described, the exosomes disclosed herein treat the cancer by inducing or enhancing an immune response against the cancer cells. The immune response typically comprises the activation and/or proliferation of glial cells such as microglia.

In one embodiment, the therapeutic exosome is isolated from proliferating neural stem cells that have been cultured under conditions that typically maintain the characteristics of the cell line, in particular the stemness of the cell line. These are typically the standard culture conditions for a given cell or cell line, which do not permit differentiation of the stem cells. Typically, proliferating cells have a doubling time of 2 to 4 days. These neural stem cells are typically passaged when sub-confluent.

As disclosed herein, it has also been found that it is possible to alter the production of microparticles by stem cells, by culturing the stem cells (optionally for at least 10 weeks) and adding components to the culture medium, by culturing the stem cells (optionally for at least 10 weeks) under hypoxic conditions, or by co-culture with other cell types (optionally for at least ten weeks).

Accordingly, the disclosure provides a method of producing a stem cell microparticle that inhibits cell migration, typically a neural stem cell microparticle that inhibits cell migration. The stem cells may be cultured under conditions that allow the efficient removal of metabolic waste. The method may comprise culturing the stem cells for at least 10 weeks in an environment that allows stem cell differentiation and collecting the microparticles that are produced by the cells; microparticles produced by this method are typically able to inhibit fibroblast migration. The microparticles may be isolated from partially-differentiated neural stem cells. In one embodiment, an environment that allows stem cell differentiation is culture in a multi-compartment bioreactor, typically for a prolonged period of time, for example more than seven days and usually more than ten weeks.

The method may alternatively comprise culturing the cells under conditions that do not allow differentiation to occur, and collecting the microparticles that are produced by the cells; microparticles produced by this method are typically able to inhibit glioblastoma migration.

The method may comprise isolating a microparticle from a stem cell-conditioned medium. The stem cell-conditioned medium may comprise one or more additive components or agents which stimulate the release of microparticles by the stem cells into the medium. The one or more components may be selected from transforming growth factor-beta (TGF-β), interferon-gamma (IFN-y) and/or tumour necrosis factor-alpha (TNF-α). The microparticles may be isolated from stem cell-conditioned medium wherein the stem cells were cultured under hypoxic conditions. The microparticles may be isolated from stem cell-conditioned medium produced by stem cells co-cultured with a different cell type, typically endothelial cells, in order to create the NSC niche environment.

The disclosure also provides a composition comprising a neural stem cell microparticle according to the first aspect and a pharmaceutically acceptable excipient, carrier or diluent. In one embodiment, the microparticle of the invention inhibits glioblastoma cell migration, typically as determined in a transmembrane or wound healing (scratch) assay. In another embodiment, the microparticle of the invention induces differentiation of a glioblastoma cell, typically as determined by cell morphology and/or marker expression. A decrease in the stem cell marker nestin typically indicates differentiation.

**Brief Description of the Drawings**

Figure 1 shows the effect of neural stem cell exosome treatment on human dermal fibroblast migration in a transmembrane assay. The top panel depicts the assay apparatus and the bottom panel compares the number of cells that migrated through the membrane in the presence of medium alone (a, "basal"), in the presence of 20μg/ml exosomes isolated from "0"-week CTX0E03 neural stem cells (b, "Exosome (0)") and in the presence of 20μg/ml exosomes isolated from CTX0E03 neural stem cells cultured for 11 weeks in the Integra CELLine AD1000 culture system (c, "Exosome (11)"), determined after 6 hours and after 24 hours assay incubation.

Figure 2 depicts the human dermal fibroblast cells that migrated through the membrane in the presence of each of the basal, 0-week and 11-week exosomes.

Figure 3A shows the results of a wound closure/scratch assay representing the migration activity of normal human dermal fibroblasts (NHDF) in response to conditioned medium from CTX0E03 cells cultured for 2 weeks and exosomes purified from the conditioned medium of CTX0E03 cultured for 2 weeks in the Integra CELLine AD1000 culture system. Figure 3B shows the results of a scratch assay after 72 hours, comparing the effect of 10μg 2-week CTX0E03 exosomes to basal conditions (without exosomes). Figure 3C shows the % of healed areas for basal conditions, 2μg/ml exosomes, 6 μg/ml exosomes, 20 μg/ml exosomes and an LSGS (low serum growth supplement) positive control. The top panel of Figure 3C shows exosomes isolated from CTX0E03 cells cultured for 2 weeks in the Integra Celline system and the bottom panel of Figure 3C shows exosomes isolated from CTX0E03 cells cultured for 6 weeks in the Integra Celline system. Figure 3D compares 2-week CTX0E03 cells to a negative control (saline) in an *in vivo* injection wound healing assay.

Figure 4 depicts electron micrographs of CTX0E03 conditionally-immortalised neural stem cells producing microparticles. Panels A-E show intracellular multivesicular bodies (MVBs) containing exosomes between 30nm and 50nm in diameter and Panel F shows microvesicles >100nm in diameter released from neural stem cells through a process of budding at the cell membrane.

Figure 5 is an outline protocol for the identification, characterisation and production of microparticles from stem cells.

Figure 6 shows the FACS detection (at 2ug/ml, 1:250) of (i) CD63 in 2-week Integra cultured CTX0E03 exosomes (top left panel) and microvesicles (top right panel) and (ii) CD81 in 2-week Integra cultured CTX0E03 exosomes (bottom left panel) and microvesicles (bottom right panel).

Figure 7 shows the results of NanoSight analysis undertaken to determine the particle size and concentration of CTX0E03 exosomes (Figure 7A) and microvesicles (Figure 7B) cultured in the Integra Celline system for 1, 2, 3, 4, 5 and 6 weeks.

Figure 8A shows the amount of protein (measured by BCA assay) extracted from 15ml of media containing microparticles purified from the Integra system compared to normal culture conditions (3 days T175). Figure 8B shows the amount of isolated total RNA measured at 260/280nm extracted from 15ml of CTX0E03 conditioned media containing microparticles purified by filtration from the Integra system compared to normal culture conditions (3 days T175).

Figure 9 shows the quantity of purified exosomes obtained per ml culture medium from standard CTX0E03 (T175) cultures vs. the Integra CELLine system at the 3 week time point.

Figure 10A shows the concentration of exosomes harvested from two different flasks after 1 week, 2 weeks and 3 weeks of CTX0E03 Integra CELLine culture system. Figure 10B shows the concentration of exosomes harvested from a single Integra CELLine flask during a 6 week continuous culture of CTX0E03 cells.

Figure 11 shows the fold change of expression levels of various mRNA markers measured in CTX0E03 cells cultured for 3 weeks in the Integra CELLine system compared to standard ("control") CTX0E03 (T175) cultures.

Figure 12 shows the fold up and down regulation of various miRNAs in exosomes obtained from CTX0E03 cells cultured for 3 weeks in Integra bioreactor culture and microparticles obtained from standard CTX0E03 (T175) cultures, assessed against a baseline expression level in CTX0E03 cells in standard (T175) culture.

Figure 13 depicts miRNA deep sequencing results. The miRNA profiles obtained from deep sequencing of miRNA from CTX0E03 cells ("CTX"), microvesicles ("MV") and exosomes ("EXO") cultured under standard (T175) conditions are shown in Figure 13A and 13B (results from two standard cultures, "EH" and "EL"). Figure 13C shows the percentage of miRNAs that are up-shuttled, the same, or down-shuttled in the exosomes compared to producer cells, for (i) the standard culture, (ii) 6 week Integra bioreactor culture and (iii) 11 week bioreactor culture (3 samples). Up-shuttled > 2, same <2>, and down-regulated < 2 fold change (log2) accordingly. Figures 13D to 13H show the miRNAs that are shuttled into exosomes compared with the cells producing them. Up-shuttled miRNAs are expressed as fold change calculated using the log2 of the normalized ratio of exosomes/cell producer. The normalization is obtained by dividing reads of each miRNA by total miRNA reads. (D) summarises the most abundant miRNAs in exosomes obtained from the standard CTX0E03 cultures ("EH" and "EL"); (E) shows exosomes obtained from CTX0E03 cells cultured for 6 weeks in an Integra bioreactor, and lists up-shuttled miRNAs with more than 250 reads per exosome sample; (F) shows the miRNAs up-shuttled in exosomes when compared with the producer cells cultured for 11 weeks in an Integra bioreactor. 9 miRNA species are up-shuttled, all of which have more than 250 reads; (G) shows a second sample of the miRNAs up-shuttled in exosomes when compared with the producer cells cultured for 11 weeks in an Integra bioreactor. The diagram lists up-shuttled miRNAs with more than 250 reads per exosome sample; and (H) shows a third sample of the miRNAs up-shuttled in exosomes when compared with cell producer cultured for 11 weeks in an Integra bioreactor, showing up-shuttled miRNAs with more than 250 reads per exosome sample.

Figure 14 is an electropherogram showing the total RNA content profile in 2-week CTX0E03 cells, exosomes and microvesicles as determined by Agilent RNA bioanalyser.

Figure 15 is a schematic presentation of the percentage of coding genes fully overlapping exon, and non-coding transcripts located with intron or intergenic sequences (produced by running NGS BAM files against GENCODE sequence data set).

Figure 16 depicts the top ranking preferentially shuttled novel miRNAs in exosomes and MV compared to CTX0E03 producer cells.

Figure 17 shows Venn diagrams comparing the proteomic data from CTX0E03 exosomes and microvesicles (17A and 17B), and comparing neural stem cell exosomes with mesenchymal stem cell exosomes (17C and 17D). Figure 17A illustrates the number of unique proteins within CTX0E03 exosomes and microvesicles, isolated from week 2 Integra culture system. Figure 17B compares the biological processes associated with the identified proteins within the CTX0E03 exosomes and microvesicles. Figure 17C compares the CTX0E03 neural stem cell exosome proteome to a Mesenchymal Stem Cell exosome, and Figure 17D compares biological processes associated with the identified proteins in the MSC derived exosomes with the neural stem cell derived exosomes.

Figure 18 shows the 30 biological processes found to be associated with NSC derived exosomes and not mesenchymal stem cell exosomes.

Figure 19 shows the presence of necrotic cell bodies and evidence of a host cellular response in the striatum of Balb-C mice 24 hours after implantation of glioblastoma U373 cells that were pre-treated for 24 hours with exosomes isolated from CTX0E03 cells cultured for 11 weeks in a multi-compartment bioreactor.

Figure 20 shows a reduction in nestin expression in glioblastoma U373 cells that have pre-treated in vitro for 24 hours with exosomes isolated from a proliferating culture of CTX0E03 cells and implanted into the striatum of Balb-C mice.

Figure 21 shows that glioblastoma U373 cells that have been treated *in vitro* with exosomes isolated from a proliferating culture of CTX0E03 cells, appear morphologically differentiated and express Glial fibrillary acidic protein (GFAP).

Figure 22 shows that seeding glioblastoma cells together with 20$\mu$g/ml CTX0E03 exosomes (Figures 22A and 22C) or have been pre-treating glioblastoma cells with 10$\mu$g/ml CTX0E03 exosomes for 24hours (Figure 22B) reduces glioblastoma migration towards 10% FBS.

Figure 23 shows the inhibitory effects of individual miRNAs on the proliferation of glioma cells: (A) plot of percentage of U373MG cell proliferation, compared to 24hrs control, measured by CyQUANT assay following transfection with hsa-mir-1246, hsa-mir-4488, hsa-mir-4492, or hsa-mir-4532; (B) plot of percentage of U373MG cell proliferation, compared to 0hr control, measured by CyQUANT assay following transfection with hsa-mir-1246, hsa-mir-4488, hsa-mir-4492, or hsa-mir-4532; and (C) plot of percentage of U87 cell proliferation, compared to 0hr control, measured by CyQUANT assay following transfection with hsa-mir-1246, hsa-mir-4488, hsa-mir-4492, or hsa-mir-4532.

Figure 24 shows glioblastoma xenograft individual tumour volumes of mice on the day of assignment to each treatment group.

Figure 25 shows the mean body weights of the mice during the xenograft study. The dotted vertical line indicates the commencement of the dosing phase (on day 12).

Figure 26 summarises the mean tumour volume for the treatment groups measured during the study.

Figure 27 displays the tumour volume data (% pre-dose) of Figure 26 in a truncated format up to study day 25.

Figure 28 shows the final tumour weights, expressed as group mean + standard error of the mean (tumour weight).

Figure 29 shows survival analysis utilising mean tumour diameter (15mm) as the humane survival endpoint.

Figure 30 shows the absolute individual body weights of each mouse in the study.

Figure 31 shows the relative individual body weights of each mouse in the study.

Figure 32 shows the raw data for individual tumour volume measurements.

Figure 33 shows the individual tumour volume plots.

Figure 34 details the tumour weights.

**Detailed Description of the Invention**

The invention provides a neural stem cell exosome for use in a method of treating a glioma, for example a glioblastoma.

The disclosure provides that the present inventors have surprisingly identified that neural stem cells produce microparticles that inhibit cell migration of fibroblasts and cancer cells, induce differentiation of cancer cells, and/or induce or enhance an immune response against the cancer cells. These microparticles are shown to inhibit cell migration and are therefore useful in therapy of diseases comprising unwanted, undesired or deleterious cell migration. The microparticles inhibit fibroblast migration and are therefore also useful in therapy of diseases comprising unwanted, undesired or deleterious angiogenesis, in which fibroblasts play a key role. The microparticles also inhibit tumour cell migration, induce differentiation of tumour cells and enhance an immune response against cancer cells, and are therefore useful in the treatment of cancer. The microparticles of the disclosure can be characterised and identified by these properties, using the assays described herein or other assays known to the skilled person.

The microparticles are advantageous over the corresponding stem cells because they are smaller and less complex, thereby being easier to produce, maintain, store and transport, and have the potential to avoid some of the regulatory issues that surround stem cells. The microparticles can be produced continuously, by isolation from conditioned media, for example in a bioreactor such as a multi-compartment bioreactor, which allows for large scale production and the provision of an "off-the-shelf" therapy. The multi-compartment bioreactor is typically a two-compartment bioreactor. An exemplary multi-compartment bioreactor is the CeLLine AD1000 bioreactor that is commercially available from Integra Biosciences AG, Zizers, Switzerland (Item No. 90025).

The inventors have found that the properties of neural stem cell microparticles differ depending on the culture conditions of the stem cells that produce the microparticles, in particular the length of time that the neural stem cells are cultured before the microparticles are harvested. In particular, the inventors have surprisingly identified neural stem cell microparticles that inhibit cell migration and/or induce differentiation of a stem or cancer cell.

In one disclosed embodiment, microparticles that inhibit fibroblast migration can be isolated from neural stem cells that have been cultured in a multi-compartment bioreactor for at least 10 weeks, e.g. more than 10 weeks. This is particularly surprising because microparticles isolated from the same neural stem cells that have been cultured for less than 10 weeks, for example about 2-6 weeks, have been shown to enhance fibroblast cell migration as seen in wound healing assays.

In another embodiment, microparticles that are able to induce or enhance a beneficial immune response against cancer cells can also be isolated from neural stem cells that have been cultured in a multi-compartment bioreactor for at least 10 weeks, e.g. more than 10 weeks.

In a further embodiment, microparticles that are able to reduce tumour cell migration and/or induce cancer or stem cell differentiation are isolated from a proliferating neural stem cell culture. This culture may be in a standard cell culture flask (such as a T-175 flask) or may be in a multi-compartment bioreactor. When the cells producing microparticles of this embodiment are cultured in a multi-compartment bioreactor, they are typically cultured for 4 weeks or less, for example 3 weeks or less, 2 weeks or less, or 1 week or less. This is because, as described elsewhere herein, prolonged culture in a multi-compartment bioreactor allows the stem cells to begin to differentiate, i.e. to express markers for defined neural cell types. Typically, the microparticles that are able to reduce tumour cell migration and/or induce differentiation are isolated from neural stem cells that are negative for markers of differentiated neural cells (e.g. $GFAP^-$ and/or $DCX^-$) but are positive for one or more markers of neural stem cells (e.g. $Nestin^+$).

Figure 1 (lower panel) and Figure 2 show that exosomes isolated from a non-proliferating CTX0E03 culture significantly abrogate migration of human dermal fibroblasts. This is in contrast to exosomes isolated from a proliferating CTX0E03 culture, which significantly promote migration of human dermal fibroblasts. Accordingly, in one embodiment, microparticles that inhibit cell (e.g. fibroblast) migration may be isolated from non-proliferating neural stem cells. Optionally, these non-proliferating stem cells may be partly differentiated, i.e. express one or more early markers of differentiation. In one embodiment, the neural stem cells from which these microparticles are isolated are positive for DCX (doublecortin), which is an early neuronal marker. In another embodiment, the neural stem cells from which the microparticles are isolated are positive for GFAP (Glial fibrillary acidic protein), which is an astrocyte marker.

Figure 22 shows that exosomes isolated from a proliferating CTX0E03 culture inhibit the migration of glioblastoma cells towards a positive chemoattractant. Accordingly, in one embodiment, microparticles that inhibit cell (e.g. glioblastoma) migration may be isolated from proliferating neural stem cells that are typically negative for markers of differentiation. In one embodiment, the neural stem cells from which these microparticles are isolated are negative for DCX (doublecortin), which is an early neuronal marker. In another embodiment, the neural stem cells from which the microparticles are isolated are negative for GFAP (Glial fibrillary acidic protein), which is an astrocyte marker.

Cell migration is well-known to play an important role in the progression of diseases such as cancer (for example during angiogenesis, tumour formation, metastasis and tissue invasion), fibrosis (for example during the accumulation of fibroblasts in the fibrotic tissue), atherosclerosis and rheumatoid arthritis. The identification of microparticles that are able to inhibit these processes therefore provides a new therapy for these diseases.

Transmembrane and wound healing assays are physiologically relevant cell-based assays that are predictive of *in vivo* mechanisms of cell migration and allow the identification of compounds that are effective in promoting or inhibiting cell migration, and the recognition of potential undesirable effects. Furthermore there is a good correlation between the results obtained in scratch assays and transmembrane assays (Hulkower et al. 2011), so that these assays can be compared.

The data presented below demonstrate that microparticles that inhibit cell migration can be isolated from neural stem cells. The microparticles of the disclosure can be produced by any method, not limited to those disclosed or exemplified herein. Whether or not a microparticle is able to inhibit cell migration can be readily determined using the assays described herein.

It has further been found that, surprisingly, culturing stem cells (of any type, not limited to neural stem cells) in an environment that allows the stem cells to begin to differentiate, increases dramatically the yield of microparticles produced. Typically, the stem cells are NSCs, for example CTX0E03, cultured for at least 10 weeks, for example for 11 weeks, but optionally no more than 20 weeks, 30 weeks or 40 weeks.

It has also been surprisingly observed that culturing stem cells (of any type, not limited to neural stem cells) in a multi-compartment bioreactor results in partial differentiation of the stem cells, into stem cells in a more differentiated form. This differentiation in culture does not require the addition of an agent to induce differentiation. This differentiation typically requires a culture period of at least one week, at least two weeks, at least three weeks, at least six weeks, at least eight weeks, or at least ten weeks, for example about 11 weeks, but optionally no more than 20 weeks. The changes to the stem cells that occur in culture in a multi-compartment bioreactor are reflected by the microparticles produced by the cultured stem cells. Therefore, by culturing stem cells in a multi-compartment bioreactor, it is possible to induce differentiation of the cells. Accordingly, microparticles from partially differentiated stem cells can be produced by harvesting microparticles from stem cells, for example NSCs such as CTX0E03, cultured in a multi-compartment bioreactor, typically for at least one week, at least two weeks, at least three weeks, at least four weeks, at least five weeks or at least six weeks, at least eight weeks, or at least ten weeks, for example about 11 weeks, but optionally no more than 20 weeks. Typically, the NSCs have been cultured for more than ten weeks. In one embodiment, the disclosure provides a method of producing microparticles by isolating the microparticles from partially-differentiated neural stem cells as described above.

It has also been found that it is possible to induce the secretion of microparticles from stem cells. Typically, the stem cells are NSCs, for example CTX0E03, typically cultured for at least 10 weeks, for example for 11 weeks or more, but optionally no more than 20 weeks. This finding, which also is not limited to neural stem cells and can be used for the production of microparticles from any stem cell, allows for an improved yield of microparticles to be obtained from a stem cell culture. Several agents have been identified that enhance the secretion of microparticles to different degrees, which has the further advantage of being able to control the amount of microparticles that are secreted. Culturing stem cells under hypoxic conditions also improves microparticle production. Further, it has been found that co-culturing a stem cell with a different cell type, in particular an endothelial cell type can beneficially alter the microparticles that are produced by the stem cell.

In a further embodiment, the disclosure provides microparticles, typically exosomes, produced by serum-free stem cells. Typically, the stem cells are NSCs, for example CTX0E03, cultured for at least 10 weeks, for example for 11 weeks, but optionally no more than 20 weeks. Serum is required for the successful culture of many cell lines, but contains many contaminants including its own exosomes. As described below, the inventors have produced microparticles from stem cells that do not require serum for successful culture.

*Neural Stem Cell Microparticles*

The disclosure provides, in one aspect, microparticles that inhibit cell migration and/or induce differentiation of a stem or cancer cell, obtainable from a neural stem cell. The microparticle is, in one embodiment, obtainable from a neural stem cell that has been cultured in a multi-compartment bioreactor, typically for at least 10 weeks, for example 11 weeks or more, or 12 weeks or more. In another embodiment, the microparticle is obtainable from a proliferating neural stem cell that has been cultured in: a standard cell culture flask such as a T-175 flask; or in a multi-compartment bioreactor for 4 weeks or less.

A neural stem cell microparticle is a microparticle that is produced by a neural stem cell. Typically, the microparticle is secreted by the neural stem cell. The microparticle of the invention is an exosome. Microparticles from other cells, such as mesenchymal stem cells, are known in the art.

A "microparticle" is an extracellular vesicle of 30 to 1000 nm diameter that is released from a cell. It is limited by a lipid bilayer that encloses biological molecules. The term "microparticle" is known in the art and encompasses a number of different species of microparticle, including a membrane particle, membrane vesicle, microvesicle, exosome-like vesicle, exosome, ectosome-like vesicle, ectosome or exovesicle. The invention provides a neural stem cell-derived exosome. The different types of microparticle are distinguished based on diameter, subcellular origin, their density in sucrose, shape, sedimentation rate, lipid composition, protein markers and mode of secretion (i.e. following a signal (inducible) or spontaneously (constitutive)). Four of the common microparticles and their typical distinguishing features are described in Table 1, below.

*Table 1: Various Microparticles*

| Microparticle | Size | Shape | Markers | Lipids | Origin |
|---|---|---|---|---|---|
| **Microvesicles** | 100-1000nm | Irregular | Integrins, selectins, CD40 ligand | Phosphatidylserine | Plasma membrane |
| **Exosome-like vesicles** | 20-50nm | Irregular | TNFRI | No lipid rafts | MVB from other organelles |
| **Exosomes** | 30-100nm; (<200nm) | Cup shaped | Tetraspanins (e.g. CD63, CD9), **Alix**, TSG101, ESCRT | Cholesterol, sphingomyelin, ceramide, lipid rafts, phosphatidylserine | Multivesicular endosomes |
| **Membrane particles** | 50-80nm | Round | CD133, no CD63 | Unknown | Plasma membrane |

Microparticles are thought to play a role in intercellular communication by acting as vehicles between a donor and recipient cell through direct and indirect mechanisms. Direct mechanisms include the uptake of the microparticle and its donor cell-derived components (such as proteins, lipids or nucleic acids) by the recipient cell, the components having a biological activity in the recipient cell. Indirect mechanisms include microvesicle-recipient cell surface interaction, and causing modulation of intracellular signalling of the recipient cell. Hence, microparticles may mediate the acquisition of one or more donor cell-derived properties by the recipient cell. It has been observed that, despite the efficacy of stem cell therapies in animal models, the stem cells do not appear to engraft into the host. Accordingly, the mechanism by which stem cell therapies are effective is not clear. Without wishing to be bound by theory, the inventors believe that the microparticles secreted by neural stem cells play a role in the therapeutic utility of these cells and are therefore therapeutically useful themselves.

The microparticles and stem cells of the disclosure are isolated. The term "isolated" indicates that the microparticle, microparticle population, cell or cell population to which it refers is not within its natural environment. The microparticle, microparticle population, cell or cell population has been substantially separated from surrounding tissue. In some embodiments, the microparticle, microparticle population, cell or cell population is substantially separated from surrounding tissue if the sample contains at least about 75%, in some embodiments at least about 85%, in some embodiments at least about 90%, and in some embodiments at least about 95% microparticles and/or stem cells. In other words, the sample is substantially separated from the surrounding tissue if the sample contains less than about 25%, in some embodiments less than about 15%, and in some embodiments less than about 5% of materials other than the microparticles and/or stem cells. Such percentage values refer to percentage by weight. The term encompasses cells or microparticles which have been removed from the organism from which they originated, and exist in culture. The term also encompasses cells or microparticles which have been removed from the organism from which they originated, and subsequently re-inserted into an organism. The organism which contains the re-inserted cells may be the same organism from which the cells were removed, or it may be a different organism.

Neural stem cells naturally produce microparticles by a variety of mechanisms, including budding of the plasma membrane (to form membrane vesicles and microvesicles) and as a result of the fusion of intracellular multivesicular bodies (which contain microparticles) with the cell membrane and the release of the microparticles into the extracellular compartment (to secrete exosomes and exosome-like vesicles).

The neural stem cell that produces the microparticles of the disclosure can be a fetal, an embryonic, or an adult neural stem cell, such as has been described in US5851832, US6777233, US6468794, US5753506 and WO-A-2005121318. The fetal tissue may be human fetal cortex tissue. The cells can be selected as neural stem cells from the differentiation of induced pluripotent stem (iPS) cells, as has been described by Yuan et al. (2011) or a directly induced neural stem cell produced from somatic cells such as fibroblasts (for example by constitutively inducing Sox2, Klf4, and c-Myc while strictly limiting Oct4 activity to the initial phase of reprogramming as recently by Their *et al,* 2012). Human embryonic stem cells may be obtained by methods that preserve the viability of the donor embryo, as is known in the art (e.g. Chung *et al.* 2008). Such non-destructive methods of obtaining human embryonic stem cell may be used to provide embryonic stem cells from which microparticles of the disclosure can be obtained. Alternatively, microparticles of the disclosure can be obtained from adult stem cells, iPS cells or directly-induced neural stem cells. Accordingly, microparticles of the disclosure can be produced by multiple methods that do not require the destruction of a human embryo or the use of a human embryo as a base material.

Typically, the neural stem cell population from which the microparticles are produced, is substantially pure. The term "substantially pure" as used herein, refers to a population of stem cells that is at least about 75%, in some embodiments at least about 85%, in some embodiments at least about 90%, and in some embodiments at least about 95% pure, with respect to other cells that make up a total cell population. For example, with respect to neural stem cell populations, this term means that there are at least about 75%, in some embodiments at least about 85%, in some embodiments at least about 90%, and in some embodiments at least about 95% pure, neural stem cells compared to other cells that make up a total cell population. In other words, the term "substantially pure" refers to a population of stem cells of the present disclosure that contain fewer than about 25%, in some embodiments fewer than about 15%, and in some embodiments fewer than about 5%, of lineage committed cells in the original unamplified and isolated population prior to subsequent culturing and amplification.

A neural stem cell microparticle comprises at least one lipid bilayer which typically encloses a milieu comprising lipids, proteins and nucleic acids. The nucleic acids may be deoxyribonucleic acid (DNA) and/or ribonucleic acid (RNA). RNA may be messenger RNA (mRNA), micro RNA (miRNA) or any miRNA precursors, such as pri-miRNA, pre-miRNA, and/or small nuclear RNA (snRNA).

A neural stem cell microparticle retains at least one biological function of the stem cell from which it is derived. Biological functions that may be retained include the ability to inhibit cell migration, for example of fibroblasts or fibroblast-like cells, or of a tumour cell such as a glioblastoma cell. In one embodiment, the at least one biological function is that of a neural stem cell that has been cultured in a multi-compartment bioreactor, for at least 10 weeks and optionally no more than 20 weeks. Alternatively the at least one biological function may be that of a neural stem cell-conditioned medium from a neural stem cell that has been cultured in a multi-compartment bioreactor, for at least 10 weeks and optionally no more than 20 weeks. In another embodiment, the at least one biological function is that of a neural stem cell that has been cultured in a T-175 flask under standard conditions.

Figures 1 and 2 (Example 1) demonstrate that exosomes isolated from the conditioned medium of CTX0E03 cells that have been cultured for 11 weeks have the ability to inhibit fibroblast migration in a transmembrane assay model of cell migration. Accordingly, one biological function that microparticles of the disclosure may retain is the ability to inhibit migration of fibroblast or fibroblast-like cells, for example of normal human dermal fibroblasts (NHDF).

In contrast, exosomes isolated from the conditioned medium of CTX0E03 cells that have been cultured for 0-6 weeks *promote* cell migration as determined using a scratch/wound closure assay. Examples 1 and 2, Table 2 and Figures 1-3 demonstrate that exosomes isolated from the conditioned medium of CTX0E03 cells that have been cultured for 0-6 weeks retain the ability to close a wound in a "scratch" model of wound healing. The results in Figure 3A show that the migration activity of normal human dermal fibroblasts (NHDF) cultured in CTX0E03 conditioned media is almost the same as the migration activity observed on the addition of purified exosomes.

The Examples also demonstrate that exosomes isolated from the conditioned medium of CTX0E03 cells that have been cultured for 11 weeks have the ability to promote the destruction of cancer cells by the immune system. Accordingly, one biological function that exosomes disclosed herein may retain is the ability to promote the destruction of cancer cells by the immune system.

The Examples further demonstrate that exosomes isolated from the conditioned medium of proliferating CTX0E03 cells have the ability to inhibit tumour cell migration. Accordingly, one biological function that exosomes of the invention may retain is the ability to inhibit tumour cell migration of glioblastoma cells.

Yet further, the Examples demonstrate that exosomes isolated from the conditioned medium of proliferating CTX0E03 cells have the ability to induce differentiation of tumour cells. Accordingly, one biological function that exosomes of the invention may retain is the ability to induce differentiation of glioblastoma cells. Differentiation may readily be determined by known assays, including cell morphology and the presence of stem cell markers (e.g. nestin) and/or differentiated cell markers (e.g. DCX, GFAP). In one embodiment, differentiation is determined by assaying for the stem cell marker nestin (as demonstrated in the Examples). A reduction in nestin expression indicates differentiation of the cell.

*Inhibition of cell migration*

Microparticles of the disclosure are able to inhibit cell migration. Typically, the migration of fibroblasts or glioblastoma cells is inhibited. Cell migration assays are known in the art. Two exemplary assays are described below, and are used in the Examples.

Transmembrane assays (sometimes referred to as "transwell" assays) are known in the art and an exemplary assay is described in Example 1. The assay uses a chamber separated into two compartments by a porous filter membrane. The cells are seeded on one side of the membrane, while medium containing the purified microparticles is placed on the opposing (lower) side. Example 1 uses fibroblasts, but other cells may be used. After an incubation period (e.g. 6-24 hours), the membrane is fixed and stained to reveal migrated cells (e.g. cell nuclei). The number of cells which have migrated through the pores of the membrane is counted microscopically.

An alternative transmembrane assay was used in Example 6, as shown in Figure 22. Here, glioblastoma cells are seeded on one side of the porous filter membrane. These cells are either seeded together with 20μg/ml microparticles (Figures 22A and C) or have been pre-treated with 10μg/ml microparticles for 24 hours (Figure 22B). Medium containing a chemoattractant is placed on the opposing (lower) side. In Example 6, the chemoattractant is FBS. After an incubation period (e.g. 6-24 hours), the membrane is fixed and stained to reveal migrated cells (e.g. cell nuclei). The number of cells which have migrated through the pores of the membrane is again counted microscopically.

Cell migration is calculated as the number of cells that have migrated through the pores of the membrane in relation to basal conditions (without the microparticles). Inhibition of cell migration in this assay may typically be defined as a decrease in the number of cells that have migrated through the membrane, typically the number of migrated cells is less than 90%, more typically less than 80%, more typically less than 75%, less than 60% or less than 50% of the number of cells that have migrated through the membrane under basal conditions (without the microparticles) after the same incubation period (e.g. 6 hours or 24 hours). As a guideline, inhibition of cell migration is achieved if after 24 hours incubation in the transmembrane assay, the number of human dermal fibroblasts or glioblastoma cells that have migrated through the membrane in the presence of 20μg/ml of the microparticles is less than 80% of the number of fibroblasts that migrated under basal conditions (i.e. in the absence of the microparticles).

In one embodiment, "inhibition of cell migration" is a statistically significant reduction in cell migration of human dermal fibroblasts or glioblastoma cells in a transmembrane assay with a $p$ value of $p<0.05$, typically $p<0.001$, in the presence of the microparticles, compared to the migration in the absence of the microparticles. Typically, this is determined after a 24 hour assay incubation period.

Cell migration may also be determined using an *in vitro* scratch (wound closure) assay, for example the assay of Example 2. Scratch assays were first used as models of wound healing for epithelial or mesenchymal cells. In this assay, cells are seeded into an assay plate and allowed to attach, spread, and form a confluent monolayer. Example 2 uses fibroblasts, but other cells may be used. A pin or needle is used to scratch and remove cells from a discrete area of the confluent monolayer to form a cell-free zone into which cells at the edges of the wound can migrate. Alternatively, a removable insert having a defined shape is placed on contact with the well bottom before the cells are seeded and allowed to form a confluent monolayer excluding the area covered by the insert. The insert is then removed, allowing the cells to migrate onto the newly revealed surface. Using either setup, molecules of interest as potential therapeutics (e.g. the purified microparticles of the disclosure) are added to the well and images of cell movement are captured at regular intervals, for example within a 24-72 hour period, for data analysis.

Cell migration/wound closure is calculated as the area covered by cells in relation to the initial wound area as determined at 0 hours. Inhibition of cell migration in this assay is typically defined as a decrease in wound closure, typically a wound closure less than 90%, more typically less than 80%, more typically less than 75%, less than 60% or less than 50% of the wound closure observed under basal conditions (without the microparticles) after 24 hours. After 48 hours, the wound closure is typically less than 90% or less than 80% of the wound closure observed using in the absence of the microparticles.

Inhibition of cell migration may also be defined as delaying a wound closure of 100%, as determined by the scratch assay, by at least 24 hours compared to the wound closure observed under basal conditions. Typically, this delay is achieved by using 2μg/ml of the isolated microparticles, as used in Example 2.

The proteomic analysis in Example 18 indicates that neural stem cell exosomes comprise biological functions associated with the production, packaging, function and degradation of genetic material. Accordingly, in one embodiment, exosomes of the invention retain these functions, typically one or more of RNA polymerase function, RNA degradation function, ribosome function and spliceosome function.

The microparticle obtained from the neural stem cell has a diameter of 1000nm or less. Typically, the microparticle of the disclosure will have a diameter of 200nm or less, for example 100nm or less. As noted in Table 1 above, microvesicles have a diameter of 100nm to 1000nm. Exosomes are typically defined as having a diameter of 30-100nm, but more recent studies confirm that exosomes can also have a diameter between 100nm and 200nm, (e.g. Katsuda et al, Proteomics 2013 and Katsuda et al, Scientific Reports 2013). Accordingly, exosomes typically have a diameter between 30nm and 150nm. Membrane particles have a diameter of 50nm to 80nm and exosome-like particles have a diameter of 20nm-50nm. The diameter can be determined by any suitable technique, for example electron microscopy or dynamic light scattering. The term microparticle includes, but is not limited to: membrane particle, membrane vesicle, microvesicle, exosome-like vesicle, exosome, ectosome-like vesicle, ectosome or exovesicle.

Figure 4 panels A-E show the presence in neural stem cells of multivesicular bodies (MVBs) containing exosomes between 30-50nm in diameter, while panel F shows microvesicles >100nm in diameter. Table 21 and Figure 7 (below) show that typical neural stem cell exosomes were measured to have a diameter ranging from approximately 70nm to approximately 150nm, which is consistent with the size of exosomes (from mesenchymal stem cells) described in the art. Accordingly, exosomes of the invention typically have a diameter between 30nm and 200nm, more typically between 50nm and 150nm. As noted above, exosomes are typically positive for the Alix marker (UNIPROT Accession No. Q8WUM4).

Figure 4F and Table 21 shows the observed size of typical neural stem cell microvesicles, with a mode diameter of approximately 150nm - 200nm, or a median diameter of approximately 180nm - 350nm. Accordingly, microvesicles of the disclosure typically have a diameter between 100 and 1000nm, more typically between 150nm and 350nm.

Some microparticles of the disclosure express the CD133 surface marker. Other microparticles of the disclosure do not express the CD133 surface marker.

"Marker" refers to a biological molecule whose presence, concentration, activity, or phosphorylation state may be detected and used to identify the phenotype of a cell.

Exosomes are endosome-derived lipid microparticles of typically 30-100nm diameter and sometimes between 100nm and 200nm diameter, that are released from the cell by exocytosis. Exosome release occurs constitutively or upon induction, in a regulated and functionally relevant manner. During their biogenesis, exosomes incorporate a wide range of cytosolic proteins (including chaperone proteins, integrins, cytoskeletal proteins and the tetraspanins) and genetic material. Consequently, exosomes are considered to be inter-cellular communication devices for the transfer of proteins, lipids and genetic material between cells, in the parent cell microenvironment and over considerable distance. Although the invention is not bound by this theory, it is possible that the exosomes are responsible for the efficacy of the neural stem cells. Therefore, exosomes from neural stem cells are themselves expected to be therapeutically efficacious.

*Microparticles designed to have desired functions*

Microparticles retain at least some of the functions of the stem cells that produce them. Therefore, it is possible to design microparticles by manipulating the stem cell (which can be any stem cell type and is not limited to neural stem cells, although the neural stem cell microparticles of the disclosure are expressly included as an embodiment) to possess one or more desired functions, typically protein or miRNA. The manipulation will typically be genetic engineering, to introduce one or more exogenous coding, non-coding or regulatory nucleic acid sequences into the stem cell. For example, if an exosome containing VEGF and/or bFGF is desired, then the exosome-producing stem cell can be transformed or transfected to express (high levels of) VEGF and/or bFGF, which would then be incorporated into the microparticles produced by that stem cell. Similarly, iPS cells can be used to produce microparticles, and these cells can be designed to produce the proteins and nucleic acids (e.g. miRNA) that are required in the microparticles produced by the iPS cells. The disclosure therefore provides *ad hoc* microparticles, from any stem cell type, that contain a function that is not naturally present in the stem cell from which is produced, i.e. the microparticles (e.g. exosomes) contain one or more exogenous protein or nucleic acid sequences, are not naturally-occurring and are engineered.

As disclosed herein, isolated or purified microparticles from the conditioned medium of neural stem cells that have been cultured for more than 10 weeks, for example for 11 weeks, and optionally no longer than 20 weeks, are loaded with one or more exogenous nucleic acids, lipids, proteins, drugs or prodrugs which are intended to perform a desired function in a target cell. This does not require manipulation of the stem cell and the exogenous material can optionally be directly added to the microparticles. For example, exogenous nucleic acids can be introduced into the microparticles by electroporation. The microparticles can then be used as vehicles or carriers for the exogenous material. As disclosed herein, microparticles that have been isolated from the cells that produced them may be loaded with exogenous siRNA, typically by electroporation, to produce microparticles that can be deployed to silence one or more pathological genes. In this way, microparticles can be used as vehicles to deliver one or more agents, typically therapeutic or diagnostic agents, to a target cell, for example to enhance or complement their endogenous inhibition of cell migration. An example of this is a neural stem cell exosome comprising exogenous siRNA capable of silencing one or more pathological genes.

*Microparticle Marker*

The disclosure provides a population of isolated neural stem cell microparticles, wherein the population essentially comprises only microparticles of the disclosure, i.e. the microparticle population is pure. In many aspects, the microparticle population comprises at least about 80% (in other aspects at least 85%, 90%, 91%, 92%, 93%, 94%, 95%, 96%, 97%, 98%, 99%, 99.5%, 99.9% or 100%) of the microparticles of the disclosure.

The isolated neural stem cell microparticle of the disclosure is characterised in that it has a distinctive expression profile for certain markers and is distinguished from microparticles from other cell types. When a marker is described herein, its presence or absence may be used to distinguish the microparticle. For example, the term "may comprise" or "may express" also discloses the contrary embodiment wherein that marker is not present, e.g. the phrase "the microparticle may comprise one or more tetraspanins, typically CD63, CD81, CD9, CD53, CD82 and/or CD37" also describes the contrary embodiment wherein the microparticle may not comprise one or more tetraspanins, typically CD63, CD81, CD9, CD53, CD82 and/or CD37.

The neural stem cell microparticle of the disclosure is typically considered to carry a marker if at least about 70% of the microparticles of the population, e.g. 70% of the membrane particles, membrane vesicles, microvesicles, exosome-like vesicles, exosomes, ectosome-like vesicles, ectosomes or exovesicles show a detectable level of the marker. In other aspects, at least about 80%, at least about 90% or at least about 95% or at least about 97% or at least about 98% or more of the population show a detectable level of the marker. In certain aspects, at least about 99% or 100% of the population show detectable level of the markers. Quantification of the marker may be detected through the use of a quantitative RT-PCR (qRT-PCR) or through fluorescence activated cell sorting (FACS). It should be appreciated that this list is provided by way of example only, and is not intended to be limiting. Typically, a neural stem cell microparticle of the disclosure is considered to carry a marker if at least about 90% of the microparticles of the population show a detectable level of the marker as detected by FACS.

The markers described herein are considered to be expressed by a cell of the population of the disclosure, if its expression level, measured by qRT-PCR has a crossing point (Cp) value below or equal to 35 (standard cut off on a qRT-PCR array). The Cp represents the point where the amplification curve crosses the detection threshold, and can also be reported as crossing threshold (ct).

In one embodiment, the disclosure relates to microparticles produced by a neural stem cell population characterised in that the cells of the population express one or more of the markers Nestin, Sox2, GFAP, βIII tubulin, DCX, GALC, TUBB3, GDNF and IDO. In another aspect of the dicslosure, the microparticle is an exosome and the population of exosomes expresses one or more of DCX (doublecortin - an early neuronal marker), GFAP (Glial fibrillary acidic protein - an astrocyte marker), GALC, TUBB3, GDNF and IDO.

The neural stem cell microparticles of the disclosure may express one or more protein markers at a level which is lower or higher than the level of expression of that marker in a mesenchymal stem cell microparticle of the same species. Protein markers that are expressed by the CTX0E03 cell microparticles are identified herein and below. In some embodiments, the microparticles may express a protein marker at a level relative to α tubulin or other such control protein(s). In some embodiments, the microparticles of the disclosure may express that protein at a level of at least +/- 1.2 fold change relative to the control protein, typically at least +/-1.5 fold change relative to the control protein, at least +/-2 fold change relative to the control protein or at least +/-3 fold change relative to the control protein. In some embodiments, the microparticles may express a protein marker at a level of between $10^{-2}$ and $10^{-6}$ copies per cell relative to α tubulin or other control protein. In some embodiments, the microparticles of the disclosure may express that protein at a level of between $10^{-2}$ and $10^{-3}$ copies per cell relative to α tubulin or other control protein.

The neural stem cell microparticles of the disclosure may express one or more miRNAs (including miRNA precursors) at a level which is lower or higher than the level of expression of that miRNA (including miRNA precursors) in a mesenchymal stem cell microparticle of the same species. miRNA markers that are expressed by the CTX0E03 cell microparticles are identified below. In some embodiments, the microparticles of the disclosure may express the marker miRNA at a level of least +/- 1.5 fold change, typically at least +/- 2 fold change or at least +/- 3 fold change (calculated according to the ΔΔct method, which is well-known) relative to U6B or 15a, or any other miRNA reference gene, also referred to as an internal control gene.

The neural stem cell microparticles of the disclosure may express one or more mRNAs at a level which is lower or higher than the level of expression of that mRNA in a mesenchymal stem cell microparticle of the same species. In some embodiments, the microparticles of the disclosure may express the marker mRNA at a level of least +/- 1.5 fold change, typically at least +/- 2 fold change or at least +/- 3 fold change (calculated according to the ΔΔct method) relative to ATP5B or YWHAZ, or any other reference gene, also referred to as an internal control gene.

Exosomes of the invention typically express specific integrins, tetraspanins, MHC Class I and/or Class II antigens, CD antigens and cell-adhesion molecules on their surfaces, which may facilitate their uptake by specific cell types. Exosomes contain a variety of cytoskeletal proteins, GTPases, clathrin, chaperones, and metabolic enzymes (but mitochondrial, lysosomal and ER proteins are excluded, so the overall profile does not resemble the cytoplasm). They also contain mRNA splicing and translation factors. Finally, exosomes generally contain several proteins such as HSP70, HSP90, and annexins that are known to play signalling roles yet are not secreted by classical (ER-Golgi) mechanisms.

The lipid bilayer of an exosome is typically enriched with cholesterol, sphingomyelin and ceramide. Exosomes also express one or more tetraspanin marker proteins. Tetraspanins include CD81, CD63, CD9, CD53, CD82 and CD37. Exosomes can also include growth factors, cytokines and RNA, in particular miRNA. Exosomes typically express one or more of the markers TSG101, Alix, CD109, thy-1 and CD133. Alix (Uniprot accession No. Q8WUM4), TSG101 (Uniprot accession No. Q99816) and the tetraspanin proteins CD81 (Uniprot accession No. P60033) and CD9 (Uniprot accession No. P21926) are characteristic exosome markers.

Alix is an endosomal pathway marker. Exosomes are endosomal-derived and, accordingly, a microparticle positive for this marker is characterised as an exosome. Exosomes of the invention are typically positive for Alix. Microvesicles of the disclosure are typically negative for Alix.

*Microparticle proteome*

Tables 19 and 21 list all proteins detected by mass spectrometry in exosomes and microvesicles, respectively, isolated from CTX0E03 cells cultured for two weeks in an Integra Celline multi-compartment bioreactor. Exosomes of the invention may contain at least a proportion of the proteins identified in Tables 19. Thus, in one embodiment, exosomes of the invention comprise at least 70%, at least 80%, at least 90%, at least 95%, at least 99% or at least 99.5% of the proteins listed in Table 19. Similarly, microvesicles of the disclosure typically comprise at least 70% at least 80%, at least 90%, at least 95%, at least 99% or at least 99.5% of the proteins listed in Table 21. In a further embodiment, the proteome of a microvesicle of the disclosure or exosome of the invention is least 70%, at least 80%, at least 90%, at least 95%, at least 99% or at least 99.5% identical to the proteome provided in Table 19 (exosome) or Table 21 (microvesicle). When determining the protein content of a microparticle or exosome, mass spectrometry is typically used, for example the LC/MS/MS method described in Example 18.

Tables 20 and 22 show the 100 most abundant proteins detected by mass spectrometry in exosomes and microvesicles, respectively, isolated from CTX0E03 cells cultured for two weeks in an Integra Celline multi-compartment bioreactor. Exosomes of the invention may contain at least a proportion of the proteins identified in Tables 20. Typically, an exosome of the invention comprises the first ten proteins listed in Table 20, more typically the first 20, the first 30, the first 40 or the first 50 proteins listed in Table 20. Similarly, a microparticle of the disclosure typically comprises the first ten proteins listed in Table 22, more typically the first 20, the first 30, the first 40 or the first 50 proteins listed in Table 22. In one embodiment, an exosome of the invention comprises all 100 proteins listed in Table 20. In one embodiment, a microvesicle of the disclosure comprises all 100 proteins listed in Table 22. Typically, the 100 most abundant proteins in an exosome of the invention or microvesicle of the disclosure contain at least 70 of the proteins identified in Table 20 (exosome) or Table 22 (microparticle). More typically, the 100 most abundant proteins in an exosome of the invention or microvesicle of the disclosure contain at least 80, at least 90, at least 95, 96, 97, 98 or 99, or all 100 of the proteins identified in Table 20 (exosome) or Table 22 (microparticle).

*Microparticle miRNA content*

Example 17A-C (and the related Figure 13A&B) shows the results of deep sequencing of miRNA present in CTX0E03 cells (standard culture) and in microvesicles and exosomes produced by these cells. This Example shows that, surprisingly, the number of different miRNA species present in the microparticles is greatly reduced compared to the number of different miRNA species present in the cells; the microparticles contain fewer than 120 different miRNAs whereas the cells contain between 450 and 700 miRNA species. The microparticles contain a majority of hsa-miR-1246.

The data in Example 17 (Tables 5-10) also show that the microparticles are characterised by four main miRNA species, namely hsa-miR-1246, hsa-miR-4492, hsa-miR-4488 and hsa-miR-4532. These four miRNAs are the only miRNAs present at a read count of greater than 1000 in the microparticles; these four miRNAs are present in massive excess compared to the other miRNAs in the microparticles. This is in contrast to the profile in the cells, which contain a much greater number of miRNAs present at high (read count greater than 1000) or very high (read count greater than 10,000) levels. Although not bound by theory, the inventors propose that hsa-miR-1246, hsa-miR-4492, hsa-miR-4488 and hsa-miR-4532 are selectively trafficked (or otherwise incorporated) into the microparticles and are thought to play a role in the function of the microparticles. A composition may comprise two, three or all four of hsa-miR-1246, hsa-miR-4492, hsa-miR-4488 and hsa-miR-4532. This composition is optionally a pharmaceutical composition, comprising a pharmaceutically-acceptable carrier, diluent, vehicle and/or excipient. The pharmaceutical composition is suitable for use in therapy, typically in the same therapies as the microparticles of the disclosure, as noted above.

Exosomes of the invention may contain at least a proportion of the miRNA species identified in Tables 7-10. In one embodiment, exosomes of the invention comprise at least 70%, at least 80%, at least 90%, at least 95%, at least 99% or at least 99.5% of the miRNAs listed in Tables 8 and 10. Similarly, microvesicles of the disclosure typically comprise at least 70% at least 80%, at least 90%, at least 95%, at least 99% or at least 99.5% of the miRNAs listed in Tables 7 and 9. In a further embodiment, the total miRNA profile of a microvesicle of the disclosure or exosome of the invention is least 70%, at least 80%, at least 90%, at least 95%, at least 99% or at least 99.5% identical to the total miRNA profile provided in Tables 8 and 10 (exosome) or Tables 7 and 9 (microvesicle). When determining the total miRNA profile of a microparticle or exosome, deep sequencing is typically used, for example the method described in Example 17.

Typically, in one embodiment exosomes, of the invention contain one, two, three or all four of hsa-miR-1246, hsa-miR-4492, hsa-miR-4488 and hsa-miR-4532. Each of these miRNA markers is typically present at a read count (optionally determined using the deep sequence technique described in Example 17) of at least 1000 per microparticle. hsa-miR-1246 may optionally have a read count of at least 2000, 5000, 10,000, 20,000, or 25,000 per microparticle.

Hsa-miR-4492 may optionally have a read count of at least 2000, 3000, 4000 or 5000 per microparticle. Hsa-miR-4532 may optionally have a read count of at least 2000 or 3000 per microparticle.

In one embodiment, each of hsa-miR-1246, hsa-miR-4492, hsa-miR-4488 and/or hsa-miR-4532 is present in the microparticle, e.g. exosome, at a higher read count than is present in the cell that produced the microparticle. In particular, miR-1246 typically has a read count in the microparticle at least twice the read count in the cell, more typically at least 4, 5, 6, 7, or 8 times the read count in the cell, and optionally 10, 15 or 20 times the read count in the cell.

In one embodiment, exosomes of the invention contain hsa-let-7a-5p, has-miR-92b-3p, hsa-miR-21-5p, hsa-miR-92a-3p, hsa-miR-10a-5p, hsa-100-5p and/or hsa-99b-5p at a lower read count than is present in the cell that produced the microparticle. Typically, each of these miRNAs has a read count of less than 1000 in the microparticles of the invention, more typically less than 100, for example less than 50. Optionally, exosomes of the invention contain hsa-let-7a-5p at a read count of less than 50 or less than 25.

In one embodiment, exosomes of the invention contain fewer than 150 types of miRNA (i.e. different miRNA species) when analysed by deep sequencing, typically fewer than 120 types of miRNA.

In one embodiment, hsa-miR-1246 is the most abundant miRNA in the exosomes of the invention (optionally determined using the deep sequence technique described in Example 17). Typically, at least 40% of the total count of miRNA in exosomes of the invention is hsa-miR-1246. Typically, at least 50% of the total count of miRNA in exosomes of the invention is hsa-miR-1246.

hsa-miR-4492 is typically the second-most abundant miRNA in the exosomes of the invention. Typically, at least 3% of the total count of miRNA in exosomes of the invention is hsa-miR-4492. More typically, at least 4% of the total count of miRNA in exosomes of the invention is hsa-miR-4492.

Typically, at least 2% of the total count of miRNA in exosomes of the invention is hsa-miR-4532.

Typically, at least 1% of the total count of miRNA in exosomes of the invention is hsa-miR-4488.

In one embodiment exosomes of the invention contain one or both of hsa-miR-4508, hsa-miR-4516 at a level at least 0.1% of the total miRNA content of the particle.

One or more of hsa-miR-3676-5p, hsa-miR-4485, hsa-miR-4497, hsa-miR-21-5p, hsa-miR-3195, hsa-miR-3648, hsa-miR-663b, hsa-miR-3656, hsa-miR-3687, hsa-miR-4466, hsa-miR-4792, hsa-miR-99b-5p and hsa-miR-1973 may be present in the exosomes of the invention.

Typically, each of hsa-let-7a-5p and hsa-100-5p is present at less than 1%, more typically less than 0.1% or less than 0.05% of the total miRNA count in exosomes of the invention.

In a typical exosome of the invention, at least 50% of the total count of miRNA is hsa-miR-1246, and less than 0.1% of the total miRNA count is hsa-let-7a-5p.

In one embodiment, at least 90% of the total count of miRNA in exosomes of the invention comprises hsa-miR-1246, hsa-miR-4492, hsa-miR-4488 and hsa-miR-4532. Typically, at least 95% or 96% of the total count of miRNA in exosomes of the invention comprises hsa-miR-1246, hsa-miR-4492, hsa-miR-4488 and hsa-miR-4532. Less than 10% of the total miRNA content of these exosomes is an miRNA that is not hsa-miR-1246, hsa-miR-4492, hsa-miR-4488 and hsa-miR-4532.

Combinations of the miRNA embodiments discussed above are provided. For example, an exosome of the invention typically contains each of hsa-miR-1246, hsa-miR-4492, hsa-miR-4488 and hsa-miR-4532 at a read count of at least 1000 and contains each of hsa-let-7a-5p, hsa-miR-92b-3p, hsa-miR-21-5p, hsa-miR-92a-3p, hsa-miR-10a-5p, hsa-100-5p and hsa-99b-5p at a read count of less than 100. Typically, at least 90% or at least 95% of the total miRNA in these exosomes is hsa-miR-1246, hsa-miR-4492, hsa-miR-4488 and hsa-miR-4532.

An exosome of the invention typically has hsa-miR-1246 as the most abundant miRNA and hsa-miR-4492 is the second-most abundant miRNA. In this embodiment, at least 40% of the total count of miRNA in exosomes of the invention is hsa-miR-1246 and at least 3% of the total count of miRNA in the microparticle is hsa-miR-4492. At least 2% of the total count of miRNA in these exosomes is hsa-miR-4532 and at least 1% of the total count of miRNA in these exosomes is hsa-miR-4488. Each of hsa-let-7a-5p and hsa-100-5p is present at less than 0.1% of the total miRNA count in these exosomes.

Plotting the deep sequencing results in the exosomes and microvesicles as relative fold change compared to the cells confirms that hsa-miR-1246, hsa-miR-4492, hsa-miR-4488 and hsa-miR-4532 are significantly upregulated in the exosomes and microvesicles compared to the cells.

This comparison also shows that miRNA hsa-miR-3195 is the miRNA that is most upregulated, in both exosomes and microvesicles. Although the absolute reads of hsa-miR-3195 are in the range of -40 for exosomes and microvesicles, there is no hsa-miR-3195 detected in the cells. Accordingly, hsa-miR-3195 is uniquely found in the exosomes and microvesicles and, optionally, in one embodiment, an exosome of the invention comprises hsa-miR-3195.

In one embodiment, microparticles of the disclosure comprise one or more of the following miRNA precursors:

AC079949.1 (SEQ ID NO:738)
GGCCGCGCCCCGTTTCCCAGGACAAAGGGCACTCCGCACCGGACCCTGGTCCCAGCG;

AP000318.1 (SEQ ID NO: 739)
CCCACTCCCTGGCGCCGCTTGTGGAGGGCCCAAGTCCTTCTGATTGAGGCCCAACCCGTGGAAG;

AL161626.1 (SEQ ID NO:740)
CGCCGGGACCGGGGTCCGGGGCGGAGTGCCCTTCCTCCTGGGAAACGGGGTGCGGC;

AC004943.1 (SEQ ID NO:741)
GCTTCACGTCCCCACCGGCGGCGGCGGCGGTGGCAGTGGCGGCGGCGGCGGCGGTGGCGGCGGCGGCGGCGGCGGCGCTC;

and

AL121897.1 (SEQ ID NO:742)
GCCGCCCCCGCCGCCGCCGCCGCCGCCGCCGCCGCCGCCGCCGCCCGCTTTCGGCTCGGGCCTCAGGTGAGTCGGAGGGGCCGGGCGCC

In one embodiment, microparticles of the disclosure comprise one, two or three of the following mature miRNAs derived from the precursors listed above (as detailed in part D of Example 17):

ggcggagugcccuucuuccugg (derived from AL161626.1-201) (SEQ ID NO:743)
ggagggcccaaguccuucugau (derived from AP000318.1-201) (SEQ ID NO:744)
gaccaggguccggugcggagug (derived from AC079949.1-201) (SEQ ID NO:745)

Accordingly, in one aspect, the disclosure provides a composition comprising one or more of the miRNA precursors AC079949.1, AP000318.1, AL161626.1, AC004943.1 and AL121897.1 in combination with a neural stem cell microparticle of the disclosure. In another embodiment, the disclosure provides a composition comprising one or more of the mature miRNAs ggcggagugcccuucuuccugg (derived from AL161626.1-201), ggagggcccaaguccuucugau (derived from AP000318.1-201) and gaccaggguccggugcggagug (derived from AC079949.1-201) in combination with a neural stem cell microparticle of the disclosure. Optionally, the composition is a pharmaceutical composition comprising one or more of the miRNA precursors and/or one or more of the mature miRNAs and a pharmaceutically-acceptable carrier or diluent in combination with a neural stem cell microparticle of the disclosure.

Example 17 shows that neural stem cell microparticles isolated from CTX0E03 cells comprise a variety of non-coding RNA species. It is expected that microparticles isolated from CTX0E03 cells cultured for at least 10 weeks, e.g. for about 11 weeks, in an Integra Celline multicompartment bioreactor will contain at least a proportion of those non-coding RNA species. Thus, in one embodiment, microparticles of the disclosure comprise one or more of ribosomal RNA, small nucleolar RNA, small nuclear RNA, microRNA, large intergenic non-coding RNA and miscellaneous other RNA (e.g. RMRP, vault RNA, metazoan SRP and/or RNY).

Example 12 shows miRNAs present in microparticles produced by the CTX0E03 cells and having a Cp below 35 as determined by a qRT-PCR array. Microparticles isolated from CTX0E03 cells cultured for at least 10 weeks, e.g. for about 11 weeks, in an Integra Celline multi-compartment bioreactor may, in one embodiment contain 1, 2, 3, 4, 5, 6, 7, 8, 9, 10, 15, 20, 25, 30, 35, 40, 45, 50, 60 or more, or all, of the following miRNAs (identified according by name according to Ambros *et al* and accessible at www.mirbase.org):

| |
|---|
| hsa-let-7a |
| hsa-let-7b |
| hsa-let-7c |
| hsa-let-7d |
| hsa-let-7e |
| hsa-let -7f |
| hsa-let-7g |
| hsa-let-7i |
| hsa-miR-100 |

(continued)

| |
|---|
| hsa-miR-101 |
| hsa-miR-103a |
| hsa-miR-106b |
| hsa-miR-10a |
| hsa-miR-10b |
| hsa-miR-124 |
| hsa-miR-125a-5p |
| hsa-miR-125b |
| hsa-miR-126 |
| hsa-miR-127-5p |
| hsa-miR-128 |
| hsa-miR-129-5p |
| hsa-miR-130a |
| hsa-miR-132 |
| hsa-miR-134 |
| hsa-miR-137 |
| hsa-miR-141 |
| hsa-miR-146b-5p |
| hsa-miR-150 |
| hsa-miR-155 |
| hsa-miR-15a |
| hsa-miR-15b |
| hsa-miR-16 |
| hsa-miR-17 |
| hsa-miR-181a |
| hsa-miR-182 |
| hsa-miR-183 |
| hsa-miR-185 |
| hsa-miR-18a |
| hsa-miR-18b |
| hsa-miR-192 |
| hsa-miR-194 |
| hsa-miR-195 |
| hsa-miR-196a |
| hsa-miR-205 |
| hsa-miR-20a |
| hsa-miR-20b |
| hsa-miR-21 |
| hsa-miR-210 |

(continued)

| |
|---|
| hsa-miR-214 |
| hsa-miR-218 |
| hsa-miR-219-5p |
| hsa-miR-22 |
| hsa-miR-222 |
| hsa-miR-23b |
| hsa-miR-24 |
| hsa-miR-26a |
| hsa-miR-301a |
| hsa-miR-302a |
| hsa-miR-302c |
| hsa-miR-33a |
| hsa-miR-345 |
| hsa-miR-375 |
| hsa-miR-378 |
| hsa-miR-424 |
| hsa-miR-7 |
| hsa-miR-9 |
| hsa-miR-92a |
| hsa-miR-93 |
| hsa-miR-96 |
| hsa-miR-99a |

In one embodiment, the CTX0E03 microparticles contain 1, 2, 3, 4, 5, 6, 7, 8, 9, 10, 15, 20, 25, 30 or more of the following miRNAs (which are selected from the list above):

| |
|---|
| hsa-let-7g |
| hsa-miR-101 |
| hsa-miR-10a |
| hsa-miR-10b |
| hsa-miR-126 |
| hsa-miR-128 |
| hsa-miR-129-5p |
| hsa-miR-130a |
| hsa-miR-134 |
| hsa-miR-137 |
| hsa-miR-155 |
| hsa-miR-15a |
| hsa-miR-15b |
| hsa-miR-16 |

(continued)

| |
|---|
| hsa-miR-17 |
| hsa-miR-182 |
| hsa-miR-183 |
| hsa-miR-185 |
| hsa-miR-18b |
| hsa-miR-192 |
| hsa-miR-194 |
| hsa-miR-195 |
| hsa-miR-20a |
| hsa-miR-20b |
| hsa-miR-210 |
| hsa-miR-218 |
| hsa-miR-301a |
| hsa-miR-302a |
| hsa-miR-302c |
| hsa-miR-345 |
| hsa-miR-375 |
| hsa-miR-378 |
| hsa-miR-7 |
| hsa-miR-9 |
| hsa-miR-93 |
| hsa-miR-96 |
| hsa-miR-99a |

*miRNAs present in exosomes from cells cultured in a bioreactor for longer periods*

Examples 17D and 17E (in particular Figures 13D to 13H and Tables E2 to E4) demonstrate that hsa-miR-1246, hsa-miR-4492, hsa-miR-4532, and hsa-miR-4488 are still present in exosomes isolated from CTX0E03 cells that have been cultured in a bioreactor for six weeks. hsa-miR-4492, hsa-miR-4532, and hsa-miR-4488 are shown to be almost absent in exosomes isolated from CTX0E03 cells that have been cultured in a bioreactor for eleven weeks.

Exosomes of the invention may contain at least a proportion of the miRNA species identified in Table E3, or at least a proportion of the miRNA species identified in Table E4.

Hsa-miR-181a-5p, hsa-miR-1246, hsa-miR-127-3p, hsa-miR-21-5p, and hsa-miR-100-5p are shown to be the top 5 miRNAs present in the EXO 6W sample. Accordingly, in one embodiment, exosomes of the invention comprise 1, 2, 3, 4 or 5 of these miRNAs. In another embodiment, exosomes of the invention comprise at least 50%, at least 60%, at least 70%, at least 80%, at least 90%, or all of the miRNAs listed in Table E3. When determining the total miRNA profile of a microparticle or exosome, deep sequencing is typically used, for example the method described in Example 17.

Hsa-miR-181a-5p, hsa-let-7a-5p, hsa-let-7f-5p, hsa-miR-92b-3p, and hsa-miR-9-5p are shown to be the top 5 miRNAs present in EXO 11W samples. Accordingly, in one embodiment, exosomes of the invention comprise 1, 2, 3, 4 or 5 of these miRNAs. In another embodiment, exosomes of the invention comprise at least 50%, at least 60%, at least 70%, at least 80%, at least 90%, or all of the miRNAs listed in Table E4. When determining the total miRNA profile of a microparticle or exosome, deep sequencing is typically used, for example the method described in Example 17.

Hsa-miR-486-5p is observed to be shuttled into all three of the samples of exosomes obtained from CTX0E03 cells that have been cultured in a bioreactor for six weeks. Accordingly, in one embodiment, exosomes of the invention comprise hsa-miR-486-5p.

*Individual miRNAs are able to reduce cell proliferation and have therapeutic utility*

The data in the Example 20 and Figure 23 show that each of the four main miRNA species identified in neural stem cell microparticles, namely hsa-miR-1246, hsa-miR-4492, hsa-miR-4488 and hsa-miR-4532, significantly reduced cell proliferation in glioma proliferation assays. In addition to these data supporting the therapeutic efficacy of the microparticles that contain these miRNAs, these data also show that each of these individual miRNAs is therapeutically useful on *its own.* In one embodiment, the individual miRNA is useful in the treatment of cancer (optionally glioblastoma), as described below.

*Proteins detected by a dot-blot*

Example 13 shows proteins present in microparticles produced by the CTX0E03 cells, as detected by a dot-blot. Microparticles of the invention may typically contain 1, 2, 3, 4, 5, 6, 7, 8, 9, 10 or all of the following proteins:

| |
|---|
| EDA-A2 |
| Galectin-3 |
| IGFBP-2 |
| IGFBP-rp1/IGFBP-7 |
| IL-1a |
| LECT2 |
| MCP-1 |
| SPARC |
| TIMP-1 |
| Thrombospondin-1 |
| VEGF |

Galectin-3 and Thrombospondin-1 are also identified as present in exosomes and microvesicles in Example 18. TIMP-1 is identified in Example 18 as being present in exosomes. Microparticles of the invention may contain one or more of Galectin-3, Thrombospondin and TIMP-1.

Example 13 also shows that the microparticles produced by the CTX0E03 cells may also express 1, 2, 3, 4 or 5 of the following proteins:

| |
|---|
| EGF-R/ErbB1 |
| MDC |
| Endostatin |
| Follistatin |
| Csk |

EGF-R and Csk are also identified as present in exosomes and microvesicles in Example 18.

*Neural Stem cells in multi-compartment bioreactor culture*

As shown in Example 15 and Figure 11 below, after multi-compartment bioreactor culture for three weeks, neural stem cells express a number of markers at significantly higher levels than neural stem cells cultured according to standard procedure in a standard single-compartment T175 flask. Neural stem cells cultured for even longer periods, e.g. at least 10 weeks, may also express a number of these markers at significantly higher levels than neural stem cells cultured according to standard procedure in a standard single-compartment T175 flask or neural stem cells cultured in a multi-compartment bioreactor culture for three weeks. In one embodiment, microparticles of the disclosure are isolated from NSCs that have been cultured, typically in a multi-compartment bioreactor, for at least 10 weeks, typically at least 11 weeks, at least 12 weeks, at least 13 weeks, at least 14 weeks or at least 15 weeks. Optionally, the NSCs have been cultured for no more than 20 weeks, e.g. between 10 and 20 weeks, between 11 and 20 weeks, between 12 and 20 weeks, between 13 and 20 weeks, between 14 and 20 weeks or between 15 and 20 weeks.

CTX0E03 neural stem cells cultured for three weeks in a multi-compartment bioreactor express DCX, GALC, GFAP, TUBB3, GDNF and IDO at a higher level than neural stem cells cultured in a standard single-compartment T175 cell culture. Neural stem cells cultured for even longer periods, e.g. at least 10 weeks, may also express a number of these markers at significantly higher levels than neural stem cells cultured according to standard procedure in a standard single-compartment T175 flask or, optionally, than neural stem cells cultured in a multi-compartment bioreactor culture for three weeks. Accordingly neural stem cells that produce microparticles of the disclosure may express one or more of DCX, GALC, GFAP, TUBB3, GDNF and IDO. Cells cultured in a two-compartment bioreactor typically show increased expression of one or more of DCX, GALC, GFAP, TUBB3, GDNF and IDO compared to the stem cells cultured under standard conditions for three weeks. The expression level of these markers in the multi-compartment bioreactor-cultured cells is typically significantly higher than in the cells cultured in a standard single-compartment T175 culture flask. Typically, a stem cell cultured in a multi-compartment bioreactor, that produces microparticles of the disclosure, expresses one or more of DCX1, GALC, GFAP, TUBB3, GDNF or IDO at a level least 2 fold higher than in CTX0E03 cells cultured in a T-175 flask according to standard culture procedure.

In one embodiment, microparticles, typically exosomes, are obtained from neural stem cells that show increased expression of one or more of DCX, GALC, GFAP, TUBB3, GDNF and IDO compared to the stem cells cultured under standard conditions or, optionally than in a multi-compartment bioreactor culture for three weeks. For example, microparticles can be obtained from freshly filtered conditioned medium collected from Integra CeLLine bioreactor cultured neural stem cells.

The upregulated markers include DCX (doublecortin - an early neuronal marker), GFAP (Glial fibrillary acidic protein - an astrocyte marker), GALC, TUBB3, GDNF and IDO. CTX0E03 cells are able to differentiate into 3 different cell types: neurons, astrocytes and oligodendrocytes. The high levels of DCX and GFAP after only three weeks in a multi-compartment bioreactor indicates that the cultured stem cells have partially differentiated and have entered the neuronal (DCX+ cells) and/or astrocytic (GFAP+ cells) lineage. Accordingly, in one embodiment the disclosure provides a microparticle that inhibits cell migration, produced by a neural stem cell population that expresses (i) one or more markers associated with a neuronal lineage, typically DCX and/or (ii) one or more markers associated with an astrocytic lineage, typically GFAP. These cells may optionally have been cultured for at least 10 weeks in a multi-compartment bioreactor. In another embodiment, the disclosure provides neural stem cell microparticles, typically exosomes, that express (i) one or more markers associated with a neuronal lineage, typically DCX and/or (ii) one or more markers associated with an astrocytic lineage, typically GFAP. These cells, or the microparticles (typically exosomes) derived from these cells, express DCX and/or GFAP at a higher level than the corresponding stem cells in standard (T-175) culture or, optionally, than the cells cultured in a multi-compartment bioreactor for three weeks. Typically, these cells or microparticles express DCX and/or GFAP at a level at least 2 fold more than the stem cells in standard culture, more typically at least 2.5 fold more than the corresponding stem cells in standard culture (or cultured in a multi-compartment bioreactor culture for three weeks), at least 5 fold more than the corresponding stem cells in standard culture (or cultured in a multi-compartment bioreactor culture for three weeks), at least 7.5 fold more than the corresponding stem cells in standard culture (or cultured in a multi-compartment bioreactor culture for three weeks) or at least 10 fold more than the corresponding stem cells in standard culture (or cultured in a multi-compartment bioreactor culture for three weeks). For expression of DCX, the fold change in the cells or microparticles compared to the corresponding stem cells in standard (T-175) culture (or cultured in a multi-compartment bioreactor culture for three weeks) can optionally be at least 20 fold, at least 50 fold, at least 100 fold, at least 500 fold or at least 1000 fold more than the standard stem cells (or cells cultured in a multi-compartment bioreactor culture for three weeks).

The term "bioreactor" is to be given its usual meaning in the art, i.e. an apparatus used to carry out a bioprocess. The bioreactors described herein are suitable for use in stem cell culture. Simple bioreactors for cell culture are single compartment flasks, such as the commonly-used T-175 flask (e.g. the BD Falcon™ 175 $cm^2$ Cell Culture Flask, 750 ml, tissue-culture treated polystyrene, straight neck, blue plug-seal screw cap, BD product code 353028).

Bioreactors can have multiple compartments, as is known in the art. These multi-compartment bioreactors typically contain at least two compartments separated by one or more membranes or barriers that separate the compartment containing the cells from one or more compartments containing gas and/or culture medium. Multi-compartment bioreactors are well-known in the art. An example of a multi-compartment bioreactor is the Integra CeLLine bioreactor, which contains a medium compartment and a cell compartment separated by means of a 10 kDa semipermeable membrane; this membrane allows a continuous diffusion of nutrients into the cell compartment with a concurrent removal of any inhibitory waste product. The individual accessibility of the compartments allows to supply cells with fresh medium without mechanically interfering with the culture. A silicone membrane forms the cell compartment base and provides an optimal oxygen supply and control of carbon dioxide levels by providing a short diffusion pathway to the cell compartment. Any multi-compartment bioreactor may be used according to the disclosure. As shown in the Examples below, CTX0E03 cells that have been cultured in the Integra CeLLine AD1000 bioreactor for 11 weeks produce microparticles that are able to inhibit cell migration.

Example 16, Table 4 and Figure 12 show that the miRNA content of exosomes produced by neural stem cells that have been cultured in a multi-compartment bioreactor, for three weeks, is different from the miRNA content of stem cells cultured in standard T-175 flasks and from microparticles produced by the neural stem cells cultured in a single-compartment T175 culture flask for three weeks. The miRNA content of exosomes of the invention may also differ from the miRNA content of stem cells cultured in standard T-175 or microparticles derived therefrom. In one embodiment, the invention provides a microparticle, typically an exosome, wherein at least two, three, four, five, six or seven miRNAs are up or down regulated compared to in the corresponding stem cells cultured in standard T-175 flasks, as calculated by Fold Regulation (see Example 16), and wherein the microparticle inhibits cell migration. The Fold Regulation of each miRNA is optionally at least two-fold up or down.

In one embodiment, neural stem cell exosomes of the invention express one, two, three, four, five, six or seven of the following miRNAs at a higher level than is expressed in the corresponding stem cells cultured in standard T-175 flasks, as calculated by Fold Regulation (where an asterisk indicates an miRNA where at least a two-fold regulation increase is preferred):

| |
|---|
| hsa-miR-146b-5p* |
| hsa-let-7c* |
| hsa-miR-99a* |
| hsa-miR-132* |
| hsa-miR-378* |
| hsa-miR-181a* |
| hsa-let-7b* |

In one embodiment, neural stem cell exosomes of the invention express one, two, three, four, five, six, seven, eight, nine, ten or more of the following miRNAs at a lower level than is expressed in the corresponding stem cells cultured in standard T-175 flasks, as calculated by Fold Regulation (where an asterisk indicates an miRNA where at least a two-fold regulation decrease is preferred):

| |
|---|
| hsa-miR-7* |
| hsa-miR-106b* |
| hsa-miR-101* |
| hsa-miR-302a* |
| hsa-miR-301a* |
| hsa-miR-183* |
| hsa-miR-219-5p* |
| hsa-miR-18a* |
| hsa-miR-15a* |
| hsa-miR-182* |
| hsa-miR-33a* |
| hsa-miR-96* |
| hsa-miR-18b* |

In a further embodiment, NSC exosomes of the invention comprise (i) an increased level of at least one, two, three, four, five, six or seven of the miRNAs indicated above as being increased in exosomes compared to the corresponding cells in standard culture and (ii) a decreased level of at least one, two, three, four, five, six, seven, eight, nine, ten or more or more of the miRNAs indicated above as being decreased in exosomes compared to the corresponding cells in standard culture. For example, a neural stem cell exosome may contain a fold-regulation increase in three or more or more of the miRNAs indicated above as being increased in exosomes compared to the corresponding cells in standard culture and a fold-regulation decrease in three or more of the miRNAs indicated above as being decreased in exosomes compared to the corresponding cells in standard culture. In another exemplary embodiment, a neural stem cell exosome may contain a fold-regulation increase in five or more of the miRNAs indicated above as being increased in exosomes compared to the corresponding cells in standard culture and a fold-regulation decrease in five or more of the miRNAs indicated above as being decreased in exosomes compared to the corresponding cells in standard culture.

The term "expressed" is used to describe the presence of a marker within a cell or microparticle. In order to be considered as being expressed, a marker must be present at a detectable level. By "detectable level" is meant that the marker can be detected using one of the standard laboratory methodologies such as qRT-PCR, or qPCR, blotting, Mass Spectrometry or FACS analysis. A gene is considered to be expressed by a cell or microparticle of the population of the invention if expression can be reasonably detected at a crossing point (cp) values below or equal 35. The terms "express" and "expression" have corresponding meanings. At an expression level below this cp value, a marker is considered not to be expressed. The comparison between the expression level of a marker in a stem cell or microparticle of the disclosure, and the expression level of the same marker in another cell or microparticle, such as for example an mesenchymal stem cell, may preferably be conducted by comparing the two cell/microparticle types that have been isolated from the same species. Preferably this species is a mammal, and more preferably this species is human. Such comparison may conveniently be conducted using a reverse transcriptase polymerase chain reaction (RT-PCR) experiment.

As used herein, the term "significant expression" or its equivalent terms "positive" and "+" when used in regard to a marker shall be taken to mean that, in a cell or microparticle population, more than 20%, preferably more than, 30%, 40%, 50%, 60%, 70%, 80%, 90% 95%, 98%, 99% or even all of the cells of the cells/microparticles express said marker.

As used herein, "negative" or "-" as used with respect to markers shall be taken to mean that, in a cell or microparticle population, less than 20%, 10%, preferably less than 9%, 8%, 7%, 6%, 5%, 4%, 3%, 2%, 1 % or none of the cells/microparticles express said marker.

Expression of microparticle surface markers may be determined, for example, by means of flow cytometry and/or FACS for a specific cell surface marker using conventional methods and apparatus (for example a Beckman Coulter Epics XL FACS system used with commercially available antibodies and standard protocols known in the art) to determine whether the signal for a specific microparticle surface marker is greater than a background signal. The background signal is defined as the signal intensity generated by a non-specific antibody of the same isotype as the specific antibody used to detect each surface marker. For a marker to be considered positive the specific signal observed is typically more than 20%, preferably stronger than 30%, 40%, 50%, 60%, 70%, 80%, 90%, 100%, 500%, 1000%, 5000%, 10000% or above, greater relative to the background signal intensity. Alternative methods for analysing expression of microparticle surface markers of interest include visual analysis by electron microscopy using antibodies against cell-surface markers of interest.

"Fluorescence activated cell sorting (FACS)" is a method of cell purification based on the use of fluorescent labelled antibodies. The antibodies are directed to a marker on the cell surface, and therefore bind to the cells of interest. The cells are then separated based upon the fluorescent emission peak of the cells.

Microparticle markers (including surface and intracellular proteins) can also be analysed by various methods known to one skilled in the art to assay protein expression, including but not limited to gel electrophoresis followed by western blotting with suitable antibodies, immunoprecipitation followed by electrophoretic analysis, and/or electron microscopy as described above, with microparticle permeabilisation for intraparticle markers. For example, expression of one or more tetraspanins may be assayed using one or more of the above methods or any other method known to one skilled in the art. RNA levels may also be analysed to assess marker expression, for example qRT-PCR.

## *Microparticle Function*

As noted above, a neural stem cell microparticle typically retains at least one biological function of the stem cell from which it is derived. Biological functions that may be retained include the ability to: inhibit cell migration, for example of fibroblast or fibroblast-like cells, or of tumour cells such as glioblastoma cells; inhibit wound healing, for example in a scratch assay; or treat a disease or condition that involves or is characterised by undesirable or excessive cell migration, such as cancer, fibrosis, atherosclerosis or rheumatoid arthritis.

In one embodiment, the at least one biological activity is that of a neural stem cell that has been cultured, typically in a multi-compartment bioreactor, for at least 10 weeks and optionally no more than 20 weeks. Alternatively the at least one biological activity may be that of a neural stem cell-conditioned medium from a neural stem cell that has been cultured, typically in a multi-compartment bioreactor, for at least 10 weeks and optionally no more than 20 weeks. Figures 1 and 2 (Example 1) demonstrate that exosomes isolated from the conditioned medium of CTX0E03 cells that have been cultured in a CeLLine bioreactor for 11 weeks have the ability to inhibit fibroblast migration in a transmembrane assay model of cell migration. Accordingly, one biological function that microparticles of the disclosure may retain is the ability to inhibit migration of fibroblast or fibroblast-like cells, for example of normal human dermal fibroblasts (NHDF).

Example 2, Table 2 and Figure 3 demonstrate that CTX0E03 stem cell exosomes, obtained from cells cultured for 2 weeks and 6 weeks, retain the ability to close a wound in a "scratch" model of wound healing. The results show that the migration activity of normal human dermal fibroblasts (NHDF) cultured in CTX0E03 conditioned media is almost the same as the migration activity observed on the addition of purified exosomes. In contrast, microparticles of the disclosure are able to inhibit cell migration. Accordingly, one biological function that microparticles of the disclosure may retain is the ability to inhibit migration activity of normal human dermal fibroblasts (NHDF). NHDF migration assays are known in the art. Stimulation of NHDF migration may be determined using an *in vitro* scratch (wound closure) assay, for example the assay of Example 2. Wound closure is calculated as the area covered by NHDF cells in relation to the initial wound area as determined at 0 hours. Inhibition of NHDF migration in this assay is typically defined as a decrease in wound closure, as defined above.

CTX0E03 cells are known to inhibit T cell activation in a PBMC assay and, in one embodiment, the microparticles of the disclosure retain this ability to inhibit T cell activation in a PBMC assay. PBMC assays are well-known to the skilled person and kits for performing the assay are commercially available.

The proteomic analysis in Example 18 indicates that neural stem cell exosomes comprise biological functions associated with the production, packaging, function and degradation of genetic material. Accordingly, in one embodiment, exosomes of the invention retain these functions, typically one or more of RNA polymerase function, RNA degradation function, ribosome function and spliceosome function.

*Immunogenicity*

The (allogeneic) neural stem cell microparticles of the disclosure typically either do not trigger an immune response *in vitro* or *in vivo* or trigger an immune response which is substantially weaker than that which would be expected to be triggered upon injection of an allogeneic stem cell population into a patient. In certain aspects of the disclosure, the neural stem cell microparticles are considered not to trigger an immune response if at least about 70% of the microparticles do not trigger an immune response. In some embodiments, at least about 80%, at least about 90% or at least about 95%, 99% or more of the microparticles do not trigger an immune response. Preferably the microparticles of the disclosure do not trigger an antibody mediated immune response or do not trigger a humoral immune response. More preferably the microparticles of the disclosure do not trigger either an antibody mediated response or a humoral immune response *in vitro*. More preferably still, the microparticles of the disclosure do not trigger a mixed lymphocyte immune response. It will be understood by one skilled in the art that the ability of the cells of the disclosure to trigger an immune response can be tested in a variety of ways.

CTX0E03 cells transplanted in a rodent model of limb ischemia have been previously demonstrated a faster and transient up-regulation of host genes involved in angiogenesis, such as CCL11, CCL2, CXCL1, CXCL5, IGF1, IL1$\beta$, IL6, HGF, HIF1$\alpha$, bFGF, VEGFA, and VEGFC, compared to vehicle treated controls. hNSC treatment transiently elevates host innate immune and angiogenic responses and accelerates tissue regeneration.

The CTX0E03 cell line has been previously demonstrated, using a human PBMC assay, not to be immunogenic. Accordingly, microparticles produced by CTX0E03 cells are also expected to be non-immunogenic. The lack of immunogenicity allows the microparticles to avoid clearance by the host/patient immune system and thereby exert their therapeutic effect without a deleterious immune and inflammatory response.

*Neural Stem Cells*

The neural stem cell that produces the microparticle may be a stem cell line, i.e. a culture of stably dividing stem cells. A stem cell line can to be grown in large quantities using a single, defined source. Immortalisation may arise from a spontaneous event or may be achieved by introducing exogenous genetic information into the stem cell which encodes immortalisation factors, resulting in unlimited cell growth of the stem cell under suitable culture conditions. Such exogenous genetic factors may include the gene "myc", which encodes the transcription factor Myc. The exogenous genetic information may be introduced into the stem cell through a variety of suitable means, such as transfection or transduction. For transduction, a genetically engineered viral vehicle may be used, such as one derived from retroviruses, for example lentivirus.

Additional advantages can be gained by using a conditionally immortalised stem cell line, in which the expression of the immortalisation factor can be regulated without adversely affecting the production of therapeutically effective microparticles. This may be achieved by introducing an immortalisation factor which is inactive unless the cell is supplied with an activating agent. Such an immortalisation factor may be a gene such as c-mycER. The c-MycER gene product is a fusion protein comprising a c-Myc variant fused to the ligand-binding domain of a mutant estrogen receptor. C-MycER only drives cell proliferation in the presence of the synthetic steroid 4-hydroxytamoxifen (4-OHT) (Littlewood et al.1995). This approach allows for controlled expansion of neural stem cells *in vitro,* while avoiding undesired *in vivo* effects on host cell proliferation (e.g. tumour formation) due to the presence of c-Myc or the gene encoding it in microparticles derived from the neural stem cell line. A suitable c-mycER conditionally immortalized neural stem cell is described in United States Patent 7416888. The use of a conditionally immortalised neural stem cell line therefore provides an improvement over existing stem cell microparticle isolation and production.

Preferred conditionally-immortalised cell lines include the CTX0E03, STR0C05 and HPC0A07 neural stem cell lines, which have been deposited at the European Collection of Animal Cultures (ECACC), Vaccine Research and Production laboratories, Public Health Laboratory Services, Porton Down, Salisbury, Wiltshire, SP4 0JG, with Accession No. 04091601 (CTX0E03); Accession No.04110301 (STR0C05); and Accession No.04092302 (HPC0A07).

The derivation and provenance of these cells is described in EP1645626 B1. The advantages of these cells are retained by microparticles produced by these cells.

The cells of the CTX0E03 cell line may be cultured in the following culture conditions:

- Human Serum Albumin 0.03%
- Transferrin, Human 5μg/ml
- Putrescine Dihydrochloride 16.2 μg/ml
- Insulin Human recombinant 5 μ/ml
- Progesterone 60 ng/ml
- L-Glutamine 2 mM
- Sodium Selenite (selenium) 40 ng/ml

Plus basic Fibroblast Growth Factor (10 ng/ml), epidermal growth factor (20 ng/ml) and 4-hydroxytamoxifen 100nM for cell expansion. The cells can be differentiated by removal of the 4-hydroxytamoxifen. Typically, the cells can either be cultured at 5% $CO_2$/37°C or under hypoxic conditions of 5%, 4%, 3%, 2% or 1% $O_2$. These cell lines do not require serum to be cultured successfully. Serum is required for the successful culture of many cell lines, but contains many contaminants including its own exosomes. A further advantage of the CTX0E03, STR0C05 or HPC0A07 neural stem cell lines, or any other cell line that does not require serum, is that the contamination by serum is avoided.

The cells of the CTX0E03 cell line (and microparticles derived from these cells) are multipotent cells originally derived from 12 week human fetal cortex. The isolation, manufacture and protocols for the CTX0E03 cell line is described in detail by Sinden, et al. (U.S. Pat. 7,416,888 and EP1645626 B1). The CTX0E03 cells are not "embryonic stem cells", i.e. they are not pluripotent cells derived from the inner cell mass of a blastocyst; isolation of the original cells did not result in the destruction of an embryo. In growth medium CTX0E03 cells are nestin-positive with a low percentage of GFAP positive cells (i.e. the population is negative for GFAP).

CTX0E03 is a clonal cell line that contains a single copy of the c-mycER transgene that was delivered by retroviral infection and is conditionally regulated by 4-OHT (4-hydroxytamoxifen). The C-mycER transgene expresses a fusion protein that stimulates cell proliferation in the presence of 4-OHT and therefore allows controlled expansion when cultured in the presence of 4-OHT. This cell line is clonal, expands rapidly in culture (doubling time 50-60 hours) and has a normal human karyotype (46 XY). It is genetically stable and can be grown in large numbers. The cells are safe and non-tumorigenic. In the absence of growth factors and 4-OHT, the cells undergo growth arrest and differentiate into neurons and astrocytes. Once implanted into an ischemia-damaged brain, these cells migrate only to areas of tissue damage.

The development of the CTX0E03 cell line has allowed the scale-up of a consistent product for clinical use. Production of cells from banked materials allows for the generation of cells in quantities for commercial application (Hodges et al, 2007).

Pollock *et al* 2006 describes that transplantation of CTX0E03 in a rat model of stroke (MCAo) caused statistically significant improvements in both sensorimotor function and gross motor asymmetry at 6-12 weeks post-grafting. These data indicate that CTX0E03has the appropriate biological and manufacturing characteristics necessary for development as a therapeutic cell line.

Stevanato et al 2009 confirms that CTX0E03 cells downregulated c-mycERTAM transgene expression both *in vitro* following EGF, bFGF and 4-OHT withdrawal and *in vivo* following implantation in MCAo rat brain. The silencing of the c-mycERTAM transgene in vivo provides an additional safety feature of CTX0E03 cells for potential clinical application.

Smith *et al* 2012 describe preclinical efficacy testing of CTX0E03 in a rat model of stroke (transient middle cerebral artery occlusion). The results indicate that CTX0E03 implants robustly recover behavioural dysfunction over a 3 month time frame and that this effect is specific to their site of implantation. Lesion topology is potentially an important factor in the recovery, with a stroke confined to the striatum showing a better outcome compared to a larger area of damage.

Neural retinal stem cell lines (for example as described in US 7514259) may also be used according to the invention.

The term "culture medium" or "medium" is recognized in the art, and refers generally to any substance or preparation used for the cultivation of living cells. The term "medium", as used in reference to a cell culture, includes the components of the environment surrounding the cells. Media may be solid, liquid, gaseous or a mixture of phases and materials. Media include liquid growth media as well as liquid media that do not sustain cell growth. Media also include gelatinous media such as agar, agarose, gelatin and collagen matrices. Exemplary gaseous media include the gaseous phase to which cells growing on a petri dish or other solid or semisolid support are exposed. The term "medium" also refers to material that is intended for use in a cell culture, even if it has not yet been contacted with cells. In other words, a nutrient rich liquid prepared for culture is a medium. Similarly, a powder mixture that when mixed with water or other liquid becomes suitable for cell culture may be termed a "powdered medium". "Defined medium" refers to media that are made of chemically defined (usually purified) components. "Defined media" do not contain poorly characterized biological extracts such as yeast extract and beef broth. "Rich medium" includes media that are designed to support growth of most or all viable forms of a particular species. Rich media often include complex biological extracts. A "medium suitable for growth of a high density culture" is any medium that allows a cell culture to reach an OD600 of 3 or greater when other conditions (such as temperature and oxygen transfer rate) permit such growth. The term "basal medium" refers to a medium which promotes the growth of many types of microorganisms which do not require any special nutrient supplements. Most basal media generally comprise of four basic chemical groups: amino acids, carbohydrates, inorganic salts, and vitamins. A basal medium generally serves as the basis for a more complex medium, to which supplements such as serum, buffers, growth factors, lipids, and the like are added. In one aspect, the growth medium may be a complex medium with the necessary growth factors to support the growth and expansion of the cells of the disclosure while maintaining their self-renewal capability. Examples of basal media include, but are not limited to, Eagles Basal Medium, Minimum Essential Medium, Dulbecco's Modified Eagle's Medium, Medium 199, Nutrient Mixtures Ham's F-10 and Ham's F-12, McCoy's 5A, Dulbecco's MEM/F-I 2, RPMI 1640, and Iscove's Modified Dulbecco's Medium (IMDM).

*Culture Period*

In the context of this disclosure, "culturing" cells for specified periods of time (e.g. at least 10 weeks) refers to a time period wherein day zero or "day 0" is the time point at which the cells are transferred to the culture vessel. The culture vessel may be a flask, for example the standard T-175 cell culture flask. Typically, the culture vessel is a multi-compartment bioreactor such as the Integra CELLine bioreactor, and day zero is the day on which the stem cells are transferred into the bioreactor. Accordingly, cells "that have been cultured for at least 10 weeks" refers to cells that have been cultured for at least 10 weeks following transfer into the culture vessel. In this 10 week period, the cells are not passaged or subcultured, i.e. they are not transferred to a new culture vessel. Optionally, cells can be removed from the culture vessel during the culture period, typically for sampling, but this does not change the cells that remain in the culture vessel, which have been in that culture vessel since day 0.

In one embodiment, as described in Example 10, on day zero approximately $15x10^6$ CTX0E03 cells in a total of 15ml of complete growth medium are introduced into the cell compartment of the CeLLine bioreactor, followed by the addition of a further 460ml of complete growth medium to the cell compartment.

*Pharmaceutical Compositions*

The neural stem cell exosome of the invention is useful in therapy and can therefore be formulated as a pharmaceutical composition. A pharmaceutically acceptable composition typically includes at least one pharmaceutically acceptable carrier, diluent, vehicle and/or excipient in addition to the exosomes of the invention. An example of a suitable carrier is Ringer's Lactate solution. A thorough discussion of such components is provided in Gennaro (2000) Remington: The Science and Practice of Pharmacy. 20th edition, ISBN: 0683306472.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The composition, if desired, can also contain minor amounts of pH buffering agents. The carrier may comprise storage media such as Hypothermosol®, commercially available from BioLife Solutions Inc., USA. Examples of suitable pharmaceutical carriers are described in "Remington's Pharmaceutical Sciences" by E W Martin. Such compositions will contain a prophylactically or therapeutically effective amount of a prophylactic or therapeutic microparticle preferably in purified form, together with a suitable amount of carrier so as to provide the form for proper administration to the subject. The formulation should suit the mode of administration. In a preferred embodiment, the pharmaceutical compositions are sterile and in suitable form for administration to a subject, preferably an animal subject, more preferably a mammalian subject, and most preferably a human subject.

The pharmaceutical composition may be in a variety of forms. These include, for example, semisolid, and liquid dosage forms, such as lyophilized preparations, liquid solutions or suspensions, injectable and infusible solutions. The pharmaceutical composition is preferably injectable. A particular advantage of the exosomes of the invention is their improved robustness compared to the stem cells from which they are obtained; the exosomes can therefore be subjected to formulation, such as lyophilisation, that would not be suitable for stem cells.

Pharmaceutical compositions will generally be in aqueous form. Compositions may include a preservative and/or an antioxidant.

To control tonicity, the pharmaceutical composition can comprise a physiological salt, such as a sodium salt. Sodium chloride (NaCl) is preferred, which may be present at between 1 and 20 mg/ml. Other salts that may be present include potassium chloride, potassium dihydrogen phosphate, disodium phosphate dehydrate, magnesium chloride and calcium chloride.

Compositions may include one or more buffers. Typical buffers include: a phosphate buffer; a Tris buffer; a borate buffer; a succinate buffer; a histidine buffer; or a citrate buffer. Buffers will typically be included at a concentration in the 5-20mM range. The pH of a composition will generally be between 5 and 8, and more typically between 6 and 8 e.g. between 6.5 and 7.5, or between 7.0 and 7.8.

The composition is preferably sterile. The composition is preferably gluten free. The composition is preferably non-pyrogenic.

In a typical embodiment, the microparticles are suspended in a composition comprising 6-hydroxy-2,5,7,8-tetramethylchroman-2-carboxylic acid (Trolox®), $Na^+$, $K^+$, $Ca^{2+}$, $Mg^{2+}$, $Cl^-$, $H_2PO_4^-$, HEPES, lactobionate, sucrose, mannitol, glucose, dextron-40, adenosine and glutathione. Typically, the composition will not include a dipolar aprotic solvent, e.g. DMSO. Suitable compositions are available commercially, e.g. HypoThermasol®-FRS. Such compositions are advantageous as they allow the microparticles to be stored at 4°C to 25°C for extended periods (hours to days) or preserved at cryothermic temperatures, i.e. temperatures below -20°C. The microparticles may then be administered in this composition after thawing.

The pharmaceutical composition can be administered by any appropriate route, which will be apparent to the skilled person depending on the disease or condition to be treated. Typical routes of administration include intravenous, intra-arterial, intramuscular, subcutaneous, intracranial, intranasal or intraperitoneal. For treatment of a disorder of the brain, one option is to administer the microparticles or miRNA intra-cerebrally, typically to the site of damage or disease.

The microparticles or miRNA will be administered at a therapeutically or prophylactically-effective dose, which will be apparent to the skilled person. Due to the low or non-existent immunogenicity of the microparticles, it is possible to administer repeat doses without inducing a deleterious immune response.

## *Therapeutic uses*

The microparticles and miRNA of the disclosure are useful in the treatment or prophylaxis of disease. Accordingly, the disclosure includes a method of treating or preventing a disease or disorder in a patient using a microparticle or miRNA of the disclosure. The term "patient" includes human and other mammalian subjects that receive either prophylactic or therapeutic treatment.

The Examples below demonstrate that neural stem cell exosomes have been identified that *inhibit* cell migration. Inhibition of migration has been observed in human fibroblasts. This inhibition is particularly surprising because, as also shown in the examples and as described in PCT/GB2013/050879, neural stem cell microparticles have previously been shown to *stimulate* fibroblast migration. Inhibition of migration has also been observed in glioblastoma cells.

The exosomes of the invention are used in the therapy of glioma, for example glioblastoma. In a further embodiment, the exosomes of the invention treat the cancer by inhibiting migration of the cancer cells. In yet a further embodiment, the microparticles of the invention treat the cancer by inducing differentiation of cancer cells, typically differentiation of a nestin-positive cancer cell. In another aspect described herein, the microparticles may treat the cancer by inducing or enhancing an immune response against the cancer cells. Glioma is a CNS cancer, so the immune response typically comprises the activation and/or proliferation of glial cells such as microglia.

The cancer may be a solid tumour cancer, for example a sarcoma or carcinoma. The solid tumour cancer may also be a solid lymphoma. Exemplary solid tumour cancers include breast cancer, lung cancer, prostate cancer, bowel cancer, renal cancer, hepatic cancer, pancreatic cancer, cervical cancer, testicular cancer, gastric (stomach) cancer, uterine cancer, ovarian cancer, cancers of the head and neck, mouth cancer, thyroid cancer, oesophagus cancer, brain cancer including glioma (e.g. glioblastoma) and meningioma, Kaposi's sarcoma, Castleman's disease, cutaneous T-cell lymphoma (CTCL), cutaneous B-cell lymphoma, and skin cancer such as basal cell carcinoma, squamous cell carcinoma and melanoma.

In one embodiment, the solid tumour cancer is breast cancer, typically ductal carcinoma in situ, lobular carcinoma in situ, invasive ductal carcinoma, invasive lobular carcinoma, inflammatory breast cancer or Paget's disease.

In another embodiment, the solid tumour cancer is lung cancer, typically squamous cell carcinoma, adenocarcinoma or large cell carcinoma, or a small cell lung cancer.

In a further embodiment, the solid tumour cancer is prostate cancer, typically prostate adenocarcinoma.

In a further embodiment, the solid tumour cancer is skin cancer, typically a basal cell carcinoma, squamous cell carcinoma or melanoma.

The cancer may be a liquid tumour, which is typically a tumour of the blood, bone marrow, or lymph nodes. Such cancers include leukemia, lymphoma and myeloma. Exemplary liquid tumours include acute lymphoblastic leukemia, acute myelogenous leukemia (AML), multiple myeloma, Hodgkin's lymphoma and non-Hodgkins lymphoma.

The cancer is a a glioma. An exemplary glioma is a glioblastoma, which may be a giant cell glioblastoma or a gliosarcoma. The *in vivo* xenograft pilot data in the Examples demonstrate trends in the treatment of glioblastoma.

The Examples below demonstrate that exosomes of the invention (in the case of Example 4, exosomes isolated from proliferating CTX0E03 cell culture) reduce the expression of nestin on tumour cells. Accordingly, in one embodiment, the cancer to be treated is nestin-positive. Nestin-positive cancers include melanoma, breast cancer, CNS cancers such as glioma and typically glioblastoma, pancreatic cancer, gastrointestinal stromal tumours (GISTs), dermatofibrsarcoma protuberances, thyroid tumours and prostate cancer (see, for example, Ishiwata et al World J Gastroenterol. 2011 January 28; 17(4):409-418). The nestin-positive breast cancer is typically "triple negative, nestin positive" breast cancer ($ER\alpha^-/PR^-/Her2^-$ /$Nestin^+$). Triple negative breast cancer is an aggressive disease, recurring and metastasizing more often than other kinds of breast cancer, and treatments for this are urgently needed. The effectiveness of microparticles of the invention in treating this cancer can readily be tested *in vivo* using a triple negative breast cancer mouse model, for example as described by Kaur et al, BMC cancer 2012m 12:120. In vivo models for other cancers exist and can be used to test the effectiveness of microparticles of the invention; for example, xenograft models of melanoma (e.g. Rofstad Br. J. Cancer (1994), 70, 804-812) and glioblastoma (e.g. Jacobs et al, ASN Neuro. 2011; 3(3); 2011).

Nestin is also reported to be expressed in endothelial cells involved in angiogenesis (Mokry et al, Stem Cells Dev. 2004; 13:658-664) and so the ability of microparticles of the invention to reduce nestin expression provides a further mechanism to inhibit angiogenesis.

Microparticles of the disclosure may also be used to treat or prevent metastatic cancers, for example metastasis of each of the cancers listed above.

Fibroblasts are known to play a role in angiogenesis during tumour formation. Without being bound by theory, it is thought that this is mediated in part by a paracrine mechanism wherein factors secreted by the fibroblasts, including Fibroblast Growth Factor (FGF), act on endothelial cells in the nascent or growing blood vessel. Therefore, inhibiting the migration of fibroblasts is expected to inhibit angiogenesis. Accordingly, the microparticles of the disclosure may be used as an anti-angiogenic therapy, i.e. in the therapy of unwanted, deleterious or undesirable angiogenesis. In one embodiment, the unwanted or undesirable angiogenesis is a component or a precursor of a solid tumour, typically a cancerous solid tumour. In this embodiment, the microparticles are used in the therapy of the tumour by preventing, inhibiting or reducing angiogenesis in the tumour. Typically, the solid tumour that is treated by targeting angiogenesis is one of the tumours described above, for example a sarcoma or carcinoma. The solid tumour cancer in this embodiment may also be a solid lymphoma. Exemplary solid tumour cancers that can be treated by targeting the angiogenic component of the tumour include breast cancer, lung cancer, prostate cancer, bowel cancer, renal cancer, hepatic cancer, pancreatic cancer, cervical cancer, testicular cancer, gastric (stomach) cancer, uterine cancer, ovarian cancer, cancers of the head and neck, mouth cancer, thyroid cancer, oesophagus cancer, brain cancer including glioma (e.g. glioblastoma) and meningioma, Kaposi's sarcoma, Castleman's disease, cutaneous T-cell lymphoma (CTCL), cutaneous B-cell lymphoma, and skin cancer such as basal cell carcinoma, squamous cell carcinoma and melanoma.

In one embodiment, the microparticles and compositions containing them are not used for immune modulation. In one embodiment, the therapy is not related to immunomodulation.

The disclosure also provides a method for treating or preventing a disease or condition comprising administering an effective amount of the microparticle of the disclosure, thereby treating or preventing the disease. Typically, the disease or condition is as identified above.

In one embodiment, the microparticles for use in therapy are isolated from NSCs (typically CTX0E03 cells) that have been cultured (typically in a multi-compartment bioreactor) for at least 10 weeks, typically at least 11 weeks, at least 12 weeks, at least 13 weeks, at least 14 weeks or at least 15 weeks. Optionally, the NSCs have been cultured for no more than 20 weeks, e.g. between 10 and 20 weeks, between 11 and 20 weeks, between 12 and 20 weeks, between 13 and 20 weeks, between 14 and 20 weeks or between 15 and 20 weeks. Typically, the microparticles are exosomes. In the examples, microparticles produced according to this embodiment are shown to inhibit fibroblast migration and induce or enhance tumour destruction by the immune system.

The observed increased efficacy of exosomes isolated from NSCs (CTX0E03 cells) that have been cultured (in a multi-compartment bioreactor) for 6 weeks correlates with the observed reduction in size of the exosomes to around 70nm diameter, which also occurred after culturing the cells for 6 weeks. Accordingly, in one embodiment exosomes isolated from NSCs (typically CTX0E03 cells) having a diameter less than 100nm, typically less than 80nm, for example around 70nm diameter, are used in therapy as described above.

In another embodiment, the microparticles for use in therapy are isolated from proliferating NSCs (typically CTX0E03 cells) that have been cultured in a standard culture vessel such as a T-175 flask, or have been cultured in a multi-compartment bioreactor for 4 weeks or less, 3 weeks or less, 2 weeks or less, or 1 week or less e.g. exosomes isolated on day 0 of the multi-compartment culture. These cells are typically passaged when sub-confluent, are positive for a stem cell marker (e.g. nestin) and negative for markers of differentiated cells (e.g. GFAP or DCX). These exosomes may have a diameter greater than 100nm. In the examples, microparticles produced according to this embodiment are shown to inhibit cancer cell migration and induce tumour cell differentiation.

In prophylactic applications, pharmaceutical compositions or medicaments are administered to a patient susceptible to, or otherwise at risk of, a particular disease in an amount sufficient to eliminate or reduce the risk or delay the outset of the disease. In therapeutic applications, compositions or medicaments are administered to a patient suspected of, or already suffering from such a disease in an amount sufficient to cure, or at least partially arrest, the symptoms of the disease and its complications. An amount adequate to accomplish this is defined as a therapeutically-or pharmaceutically-effective dose. In both prophylactic and therapeutic regimes, agents are typically administered in several dosages until a sufficient response has been achieved. Typically, the response is monitored and repeated dosages are given if the response starts to fade.

The microparticles of the disclosure may optionally be combined with a stem cell to provide a combination therapy. The stem cell is optionally the stem cell from which the microparticle is derived, e.g. if the microparticle is an exosome from a CTX0E03 cell, then the stem cell for use in combination therapy may be a CTX0E03 cell, typically but not necessarily cultured for the same period of time as the cells from which the microparticles were derived. A stem cell and microparticle can optionally be (i) administered together in a single pharmaceutical composition, (ii) administered contemporaneously or simultaneously but separately, or (iii) administered separately and sequentially, e.g. stem cell followed by microparticle, or microparticle followed by stem cell. When the stem cell and microparticle are administered separately and sequentially, the duration between the administration of the cell and microparticle may be one hour, one day, one week, two weeks or more.

In one embodiment, a prophylactic therapy induces tolerance, typically immunotolerance, in a host that is to receive the stem cells from which the microparticle is derived. In one embodiment, the administration of one or more doses of microparticles of the disclosure to a patient, prior to administration of a stem cell therapy, can be used to reduce the risk of an adverse immune response, i.e. "rejection", of the stem cell therapy. In another embodiment, tolerance to the stem cells can be increased by administering stem cells together with microparticles of the disclosure, as discussed above.

Effective doses of the compositions of the present invention, for the treatment of the above described conditions vary depending upon many different factors, including means of administration, target site, physiological state of the patient, whether the patient is human or an animal, other medications administered, and whether treatment is prophylactic or therapeutic. Usually, the patient is a human.

The CTX0E03 cell line has been shown to be effective in treating stroke, peripheral arterial disease, brain damage such as motor, sensory and/or cognitive deficit, and psychiatric disorders. The cells are currently being tested in a clinical trial for treatment of disabled stroke patients (Clinicaltrials.gov Identifier: NCT01151124). WO-A-2012/004611 describes the use of the CTX0E03 cells in treating psychiatric disorders including unipolar and bipolar depression, schizophrenia, obsessive compulsive disorder, autism and autistic syndrome disorders.

As used herein, the terms "treat", "treatment", "treating" and "therapy" when used directly in reference to a patient or subject shall be taken to mean the amelioration of one or more symptoms associated with a disorder, or the prevention or prophylaxis of a disorder or one or more symptoms associated with a disorder. The disorders to be treated include, but are not limited to, cancer, fibrosis, rheumatoid arthritis, atherosclerosis, and other diseases involving deleterious cell migration. Amelioration or prevention of symptoms results from the administration of the microparticles of the disclosure, or of a pharmaceutical composition comprising these microparticles, to a subject in need of said treatment.

### *Tracing administered cells and microparticles in vivo*

The present disclosure provides a distinct marker profile for microparticles produced by neural stem cells. It is therefore possible to detect the presence of these microparticles *in vivo,* by testing a sample obtained from a patient and determining whether the marker profile in the sample matches that of the microparticles. If the sample profile matches the profile of the microparticles described herein, then this confirms the presence of the microparticles. This can be used to detect not only the presence and/or biodistribution of the microparticles themselves, but also the presence of stem cells producing the microparticles. This is particularly useful when detecting whether a stem cell administered *in vivo* has engrafted into the host tissue, and/or has migrated, for example in ADME(T) studies.

Detection of the microparticles *in vivo* can be used to monitor the course of a treatment wherein microparticles or stem cells are administered to a patient. Determining the presence, absence or amount of microparticles or cells producing microparticles of the disclosure in a patient allows the dosage regime to be altered accordingly, e.g. to increase or decrease the dose as required to provide an effective amount of microparticles or stem cells *in vivo.*

*Methods of producing microparticles*

Microparticles are isolated from stem cell conditioned media. The "conditioned medium" (CM) may be a growth medium for stem cells, which has been used to culture a mass culture of stem cells for at least about 12 hours, at least about 24 hours, at least about 48 hours or least about 72 hours, typically up to 168 hours (7 days), removed and sterilized by any suitable means, preferably by filtration, prior to use, if required.

Microparticles that are able to inhibit fibroblast cell migration have been isolated from stem cells that have been cultured for at least 10 weeks. Accordingly, one way to produce microparticles that are able to inhibit cell migration is to culture the cells in a multi-compartment bioreactor for at least about 10 weeks before the microparticles are harvested, typically at least 11 weeks, at least 12 weeks, at least 13 weeks, at least 14 weeks, at least 15 weeks, and optionally no longer than 20 weeks. Example 10 describes a typical culture protocol using a CeLLine bioreactor.

Microparticles that are able to inhibit glioblastoma cell migration have been isolated from proliferating stem cells that have been cultured for 4 weeks or less. Accordingly, one way to produce microparticles that are able to inhibit cell migration is to culture the cells so that they are able to proliferate, for example by culturing in a T-175 flask, or in a multi-compartment bioreactor for 4 weeks or less, 3 weeks or less, 2 weeks or less, or 1 week or less e.g. exosomes isolated on day 0 of the multi-compartment culture.

Typically, microparticles may be harvested from a multi-compartment, e.g. two-compartment, bioreactor which allows the cell culture, and hence the conditioned media, to be maintained for longer periods of time, for example more than 10 weeks, at least 11 weeks, at least 12 weeks, at least 13 weeks, at least 14 weeks, at least 15 weeks, and optionally no longer than 20 weeks.. The system maintains the cells and secreted microparticles within a small cell compartment (approximately 15ml) which is separated from a larger reservoir of medium by a 10kDa semi-permeable membrane. This allows the efficient removal of metabolic waste products while effectively maintaining an extremely high cell density to maximize microparticle production. Example 14, and Figures 9 and 10, demonstrate that use of a two-compartment bioreactor results in a much higher yield of microparticles than is obtained when a standard cell culture flask (T175 flask) is used.

The microparticles may be separated from other media components based on molecular weight, size, shape, hydrodynamic radius, composition, charge, substrate-ligand interaction, absorbance or scattering of electromagnetic waves, or biological activity. In one embodiment, the conditioned media is filtered using a filter of appropriate size to separate the desired microparticle, for example a 100K MWCO filter. Optionally, the stem cell-conditioned medium is concentrated prior to the isolation of the microparticles by subjecting the concentrated NSC-conditioned medium to size exclusion chromatography. The UV absorbant fractions can then be selected for isolation of the microparticles of interest.

Different microparticles can be isolated from the media by using different isolation techniques and parameters. For example, exosomes have a vesicle density of 1.13-1.19 g/mL and can be isolated by differential centrifugation and sucrose gradient ultracentrifugation at 100,000-200,000g. Microvesicles can be isolated by filtration (100K MWCO) and differential centrifugation at 18,000-20,000g. Membrane particles have a density of 1.04-01.07 g/ml and Exosome-like vesicles have a density of 1.1 g/ml.

A typical production method comprises: culturing stem cells to produce conditioned media; removing cell debris by centrifugation at 1500 rpm; isolating microvesicles (<1000kDa) by ultrafiltration through a 100K MWCO filter or isolating exosomes (30-100nm) by ultracentrifugation at 120,000g; followed by quantification using a BCA protein assay.

*Conditionally immortalised stem cells as producer cells for microparticles*

In one aspect of the disclosure, conditionally immortalised stem cells are used to produce microparticles such as microvesicles and/or exosomes. These conditionally immortalised stem cells are typically neural stem cells, but may be a stem cell of any type, for example a haematopoietic stem cell or a mesenchymal stem cell. A method of producing stem cell microparticles is therefore provided, comprising the steps of culturing conditionally-immortalised stem cells and harvesting the microparticles that are produced by the cells, as described above. Conditional immortalisation of stem cells is known in the art, as described above. For the avoidance of doubt, this method is not limited to the use of neural stem cells.

When the stem cell used to produce microparticles is a neural stem cell, it may be any of the neural stem cells described herein, for example the CTX0E03 conditionally-immortalised cell line which is clonal, standardised, shows clear safety *in vitro* and *in vivo* and can be manufactured to scale thereby providing a unique resource for stable exosome production. Alternatively, the neural stem cells may be neural retinal stem cell lines, optionally as described in US 7514259.

When the stem cell used to produce microparticles is a mesenchymal stem cell, it may optionally be a conditionally-immortalised adipose-derived stem cell ("ADSC") or a conditionally-immortalised version of the mesenchymal stem cells described in WO-A-2009/105044; these cells are CD29+, CD44+, CD49a+/e+, CD105+, CD166+, CD34-, CD45-.

*Methods of inducing microparticle secretion*

The inventors have found that it is possible to increase the production of microparticles by stem cells. This finding, which is not limited to neural stem cells and can be used for the production of microparticles from any stem cell, allows for an improved yield of microparticles to be obtained from a stem cell culture.

A first technique to increase the production of microparticles by the stem cells is to treat the stem cells with one or more of TGF-β, IFN-γ or TNF-α, typically at between 1 and 25ng/ml e.g. 10ng/ml, for between 12 to 96 hours prior to the removal of conditioned media.

As explained in Example 8 below, the frequency of the occurrence of multivesicular bodies (MVBs) was observed to be altered by the presence of TGF-β, IFN-γ or TNF-α (10ng/ml). The frequency was highest in the presence of TGF-β, followed by IFN-γ, followed by TNF-α. Therefore, adding one or more of TGF-β, IFN-γ or TNF-α to the stem cell culture medium will stimulate the production of microparticles by the cells. The microparticles can then be harvested, by separating the microparticles from other components as described above.

A second technique to increase the production of microparticles by the stem cells is to culture the cells under hypoxic conditions. Culturing cells under hypoxic conditions is well-known to the skilled person, and involves culturing the cells in an atmosphere that has less than atmospheric level of $O_2$, i.e. less than 21% $O_2$. This is typically achieved by placing the cells in an incubator that allows oxygen levels to be changed. Hypoxic culture typically involves culturing in an atmosphere containing less than 10% $O_2$, more typically 5% or less $O_2$, for example 4% or less, 3% or less, 2% or less, or 1% or less $O_2$.

The inventors have also realised that co-culturing a stem cell with a different cell type can alter the production of microparticles by the stem cell. The different cell type may be a non-stem cell, i.e. a terminally differentiated cell type. Typically, the different cell type is one with which the stem cell would interact *in vivo.* In one embodiment, neural stem cells are co-cultured with epithelial cells such as endothelial cells, typically Human Umbilical Vein Endothelial Cells (HUVEC). It has been observed that *in vivo,* NSCs and the vasculature interact, with proliferating NSCs being localized in close proximity or adjacent to blood vessels. Receptor tyrosine kinase activation and signal protein secretion has also been observed to be upregulated when NSCs are co-cultured with endothelial cells, again indicating that the vasculature modulates the proliferation capacity of NSCs.

Therefore, culturing a stem cell with a different cell type may improve the amount of microparticles produced and/or may refine the content of the microparticles, typically so that the microparticles produced by the stem cells are further biased towards astate of inhibition of cell migration. Accordingly, microparticles produced by stem cells that have been co-cultured with other cells, e.g. NSCs co-cultured with endothelial cells, are advantageous. These microparticles may be obtained by isolation from the co-cultured stem-cell conditioned media, as described herein.

Surprisingly, the present inventors have realised that the amount of microparticles produced by stem cells can be increased greatly simply by culturing stem cells in a multi-compartment bioreactor. This finding is not limited to neural stem cells and applies generally to the culture of all stem cells. Accordingly, one aspect of the disclosure provides a method of producing microparticles from stem cells that have been cultured in a multi-compartment bioreactor. The cells from which the microparticles are harvested have typically been cultured for at least one week, typically at least 8, 9, 10, 11, 12, 13 or 14 days, for example 15 days, 16 days, 17 days, 18 days, 19 days, 20 days, 21 days or more, for example at least three weeks, four weeks, five weeks, six weeks or more. To produce microparticles that inhibit cell migration, the cells from which the microparticles are harvested have typically been cultured for more than ten weeks. It can be seen from Figure 10 that the increase in microparticle production, week on week, is not merely additive but is exponential. The prolonged culture typically has been observed in the Integra Celline system two-compartment bioreactor (commercially available from Integra Biosciences AG, Zizers, Switzerland) but the findings are not limited to this specific multi-compartment bioreactor; any multi-compartment bioreactor can be used. This culture method can be used to produce microparticles from any stem cell type, including but not limited to neural stem cells and mesenchymal stem cells.

*Method for screening total RNA composition of conditioned medium*

Following centrifugation (5 min at 1500 rpm), microparticles are collected from conditioned medium through filtration (0.02-0.2μm, or 100K MWCO). Total RNA is obtained using trizol based extraction followed by purification using Qiagen RNaesy mini kit. The extract in water has a 260:280 nm absorbance suggesting that it may be RNA. Total RNA is retro-transcribed with either a protocol suitable for mRNA (Superscript II RT, Invitrogen) or miRNA (mScript RT kit, Qiagen). Validation of mRNA and miRNA presence is proven by qRT-PCR using primers for ATP5B and YWHAZ for mRNA, and U6B and 15a for miRNA housekeeping genes respectively. The RNA may further be assessed by a generic gene expression analysis assay such as an array (micro array or PCR based array), and sequencing.

The invention is further described with reference to the following non-limiting examples.

## Examples

***Example 1:*** *NSC exosomes that inhibit cell migration.*

A transwell assay was used to study the migratory response of human dermal fibroblasts to different populations of exosomes. Experiments were performed in triplicate. 200,000 human dermal fibroblast cells ("FBs") were placed on the upper layer of a cell permeable membrane (8 μm pore size; 24-well plate) and a solution (basal medium) containing or lacking 20 μg/ml exosomes was placed in contact with the underside of the cell permeable membrane (Figure 1, top panel). The exosomes were collected from CTX0E03 cells cultured for 0 weeks ("0") or 11 weeks ("11") in an Integra CeLLine AD1000 multi-chamber bioreactor. Following an incubation period (6 or 24 hours; control: 0 hours), the human dermal fibroblast cells that migrated through the membrane were stained (using a fluorescent-dye conjugated anti-actin antibody and Hoechst Fluorescent Stain for nuclei) and counted (six random microscope fields per sample) as an indicator of the cells' migratory response to exosomes.

Figure 1 (lower panel) and Figure 2 show that exosomes isolated from a proliferating CTX0E03 culture ("0") significantly promote migration of human dermal fibroblasts compared to medium lacking exosomes ("basal"), both after a 6 hour and after a 24 hour incubation period. In contrast, exosomes isolated from a more differentiated CTX0E03 culture ("11") significantly abrogate migration of human dermal fibroblasts compared to medium lacking exosomes ("basal"), both after a 6 hour and after a 24 hour incubation period.

It can be seen that cell migration is increased in the presence of exosomes from 0-week NSCs but decreased in the presence of exosomes from 11-week NCSs, compared to control ("basal").

In summary, NSC microparticles have been identified that significantly abrogate cell migration.

These data show that neural stem cell microparticles can stimulate or inhibit cell migration. This is surprising and useful in applications where either stimulating (e.g. wound healing) or inhibiting (e.g. cancer, fibrosis, rheumatoid arthritis, atherosclerosis) cell migration is desired. The involvement of fibroblasts in angiogenesis also makes the microparticles that inhibit fibroblast migration useful in applications where inhibition of angiogenesis is desired. Angiogenesis is involved in tumour formation, survival and metastasis. These data therefore demonstrate potential for the exosomes of the invention to treat many types of cancer.

***Example 2:*** *Exosomes isolated from the medium of NSCs cultured for 2 or 6 weeks promote fibroblast migration.*

Method - Wound closure/ scratch assay

- Seed 0.25x$10^6$ NHDF (normal human dermal fibroblasts) per well of a 12 well plate and allow to become confluent (24 hours)
- Remove growth factors for 24hrs
- Remove cells (scratch) and incubate with exosomes/conditioned media
- Image effected area over 48hrs
- Estimate area using Image J

*Results*

**Table 2** - Wound closure/scratch assay representing the migration activity of normal human dermal fibroblasts (NHDF) cultured in CTX0E03 conditioned media or upon the addition of purified exosomes.

| | Wound closure (%) | | |
|---|---|---|---|
| | 0h | 24h | 48h |
| CTX0E03 conditioned media | 0% | 100% | |
| 2ug/ml exosomes | 0% | 95.4% | 100% |
| Control | 0% | 48.1% | 49.7% |

Wound closure was calculated as the area covered by cells in relation to the initial wound area, as determined at 0h. Wound closure is expressed as the percentage of the initial wound area at time 0h. These data are also shown, photographically, in Figure 3A. The figure shows that, in contrast to exosomes from 11-week NSCs as described in Figures 1 and 2, exosomes from 2-week NSCs stimulate cell migration.

Figure 3B shows that 10μg CTX0E03 exosomes significantly increase wound closure (as determined in the HDNF scratch/migration assay) after 72 hours, compared to basal conditions (without exosomes).

Further experiments confirmed that exosomes purified (by ultracentrifugation; quantified by BCA protein assay; characterised as >99% positive for CD63 and CD81 and having a greater expression level of Alix compared to the corresponding microparticle fraction) from all time points (weeks 2-6) during continuous culture (using Integra CELLine bioreactors in the presence of growth factors and 4OHT) significantly enhanced fibroblast migration and wound healing, with a peak response between 5-10μg/ml compared to basal conditions. Figure 3C shows the % healed areas for basal conditions, 2μg/ml exosomes, 6 μg/ml exosomes, 20 μg/ml exosomes and an LSGS (low serum growth supplement) positive control. The top panel of Figure 3C shows exosomes isolated from CTX0E03 cells cultured for 2 weeks in the Integra Celline system and the bottom panel of Figure 3C shows exosomes isolated from CTX0E03 cells cultured for 6 weeks in the Integra Celline system. These data show that all doses of all tested NSC exosomes provide increased healing compared to basal conditions, with % healing approaching the positive control (LSGS) after 72 hours.

The data in Figure 3C also show that the exosomes isolated from NSCs cultured for 6 weeks cause faster healing (than 2 week exosomes), with the % healed approaching 100% after only 48 hours, for all doses.

Figure 3D shows the results of an *in vivo* injection wound assay in a mouse, confirming that CTX0E03 cells stimulated wound healing to a statistically-significant degree *in vivo.* This is a simple *in vivo* bioassay which can be used to confirm the efficacy of microparticles *in vivo.*

**Conclusion** Exosomes released from the human neural stem cell line CTX0E03 enhance fibroblast migration in an *in vitro* model of wound healing, suggesting that exosomes may contribute to the mechanisms by which hNSCs promote repair. Exosomes isolated from cells cultured for 6 weeks show improved wound healing efficacy *in vitro,* compared to exosomes isolated from cells cultured for 2 weeks.

## Example 3: Glioblastoma engraftment assay - Destruction of tumour cells

U373 glioblastoma cells were pre-treated *in vitro* for 24 hours with exosomes isolated from CTX0E03 cells cultured for 11 weeks in an Integra CeLLine bioreactor before implantation into the striatum of Balb-C mice brains.

As shown in Figure 19, the exosome-treated glioblastoma cells did not engraft into the striatum. Histopathology demonstrated the presence of necrotic U373 cell bodies at the site of implantation and evidence of gliosis - a host cellular immune response.

These data suggest utility of these exosomes in the treatment of cancer, by promoting the destruction of a tumour by the immune system, particularly a tumour of the CNS such as a glioblastoma.

## Example 4: Glioblastoma engraftment assay - Differentiation of tumour cells

U373 glioblastoma cells were pre-treated *in vitro* for 24 hours with exosomes isolated from standard CTX0E03 cell culture ("exosome 0" - proliferating cells, cultured in an Integra CeLLine bioreactor for less than 24 hours) before implantation into the striatum of Balb-C mice brains. Marker expression was then observed after 24 hours.

As shown in Figure 20, the exosome-treated glioblastoma cells demonstrated a reduction in nestin expression 24 hours post implantation into the striatum of Balb-C mice. Nestin is a stem cell marker; cancer stem cells drive tumourigenesis, are linked with metastasis, high grade and poor prognosis. The treatment of cancer by inducing cellular differentiation is particularly attractive because the therapy can be target-cell specific (i.e. will only target the undifferentiated, malignant, cells) and likely less toxic than standard chemotherapies.

These data suggest utility of these exosomes in the treatment of cancer, by inducing differentiation of the cancer cells, typically for treating a nestin-positive cancer and particularly a tumour of the CNS such as a glioblastoma.

## Example 5: *In vitro* glioblastoma differentiation assay - Differentiation of tumour cells

U373 glioblastoma cells were cultured for 24 hours in the presence of: (i) basal medium; (ii) +20μg exosomes isolated from standard CTX0E03 cell culture ("exosome 0" - proliferating cells, cultured in an Integra CeLLine bioreactor for less than 24 hours); or (iii) +20μg exosomes isolated from CTX0E03 cells cultured for 11 weeks in an Integra CeLLine bioreactor ("exosome 11"). The U373 cells were then stained for the presence of Nestin (a stem cell marker) and GFAP (an astrocyte marker of a differentiated cell).

As shown in Figure 21, exosome 0 promoted differentiation of the glioblastoma cells *in vitro.* The exosome 0 treated cells appeared morphologically differentiated, with the presence of long processes. Additionally, these cells expressed Glial fibrillary acidic protein (GFAP), a marker of differentiated astroglial cells. These in vitro data agree with the *in vivo* data immediately above. As noted above, more differentiated (less malignant) glioblastoma tumours are linked with more favourable prognosis. These data further suggest utility of these exosomes in the treatment of cancer, by inducing differentiation of the cancer cells, particularly a tumour of the CNS such as a glioblastoma.

In contrast, the exosomes isolated from CTX0E03 cells cultured for 11 weeks ("exosome 11") promoted "sternness" in the glioblastoma cells *in vitro,* demonstrated by nestin expression and proliferation. However, in the *in vivo* assay above, these exosomes were observed to promote destruction of the tumour cells.

## Example 6: Glioblastoma migration assays

Three separate *in vitro* transmembrane migration assays have demonstrated that treatment of glioblastoma (U373) cells with exosomes isolated from standard CTX0E03 cell culture ("exosome 0" - proliferating cells, cultured in an Integra CeLLine bioreactor for less than 24 hours) significantly reduces their migration towards a positive chemoattractant (Foetal Bovine Serum). These assay results are shown in Figure 22.

Glioblastoma cells were seeded on one side of a porous filter membrane. These cells were either seeded together with 20μg/ml CTX0E03 "exosome 0" (Figures 22A and 22C) or have been pre-treated with 10μg/ml CTX0E03 "exosome 0" for 24hours (Figure 22B). Medium containing a 10%FBS was placed on the opposing (lower) side. After a 24 hour incubation period, the membrane was fixed and stained to reveal migrated cells (e.g. cell nuclei), which were counted microscopically.

These data show that exosomes of the invention (isolated from standard "week 0" CTX0E03 cells) are able to reduce migration of glioblastoma cells. Glioblastomas are the most common and malignant brain tumors of the central nervous system and exhibit high invasive capacity, which hinders effective therapy. Therefore, therapeutics that can inhibit glioma cell migration and invasion are highly desirable. These data demonstrate the utility of neural stem cell exosomes in the treatment of cancer, typically a glioblastoma, by reducing tumour migration/invasion.

### *Summary: Treatment of cancer using neural stem cell exosomes*

The data provided above indicate therapeutic utility in the treatment of cancer using exosomes produced by neural stem cells, by one or more of: reducing tumour migration/invasion (exosome 0, glioblastoma assay); inducing tumour differentiation (exosome 0, glioblastoma assay); promoting tumour destruction (exosome 11, glioblastoma transplant); or inhibiting angiogenesis (exosome 11, fibroblast assay).

## Example 7: Preparation of neural stem cells and neural stem cell microparticles for visualisation by electron microscopy.

Method

### Embedding CTX0E03 cells for electron microscopy

- 5 x 70% CTX0E03 cultures
- Treat with +/-4OHT, IFNγ, TNFα and TGFβ (all at 10ng for 24hrs)
- Detach cells and fix overnight in 2.5% Gluteraldehyde in 0.1M Cacodylate pH7.4
- Cells spun down 300g
- Buffered osmium 2%, 1.5hrs
- Spin, wash water, overnight
- Uranium acetate 2%, 2hrs
- Spin, wash water, 30mins
- Ethanol gradient 20, 35, 50, 70, 80, 90, 100%, over weekend.
- 100% propylene oxide (PO), 1hr
- Spin, 50% Agar LV resin in PO, 1hr
- 75% LV resin/PO 5hrs
- 100% resin overnight at 60°C
- Cool to RT before cutting (60-80nm), Imaged TEM at 200Kv.

Results

Figure 4A-E shows the electron micrographs of the multivesicular bodies (MVBs) containing exosomes of approximately 30nm - 50nm in diameter. Figure 4F shows microvesicles >100nm in diameter.

**Example 8: Production of neural stem cell microparticles from a neural stem cell line.**

Method

5 Sub-confluent flasks containing the same culture of CTX0E03 cells were individually treated with either 10ng/ml TGF-β, 10ng/ml IFNγ, or 10ng/ml TNFα alongside full growth media controls with or without the addition of 4OHT. 72 hours after treatment, the cells were collected using trypzean/EDTA, washed and fixed overnight in 2.5% Gluteraldehyde in 0.1M Cacodylate pH7.4 ready for electron microscopy evaluation.

*Results*

The frequency of the occurrence of multivesicular bodies (MVBs) was observed to be altered by the presence of TGF-β, IFN-γ or TNF-α. The frequency was highest in the presence of TGF-β, followed by IFN-γ, followed by TNF-α.

Conclusion

The production of microparticles from neural stem cells can be stimulated by the addition of the factors TGF-β, IFN-γ or TNF-α. This has the potential for more efficient production of microparticles.

**Example 9: Purification, quantification and characterisation of neural stem cell microparticles.**

Method

An outline protocol for producing large quantities of microparticles is provided in Figure 5. The main steps are purification, quantification, characterisation, efficacy testing and manufacture.

(1) Purification
Microparticles can be purified from stem cell-conditioned medium by ultracentrifugation, e.g. at 100000 x g for 1-2 hours. Alternative or additional methods for purification of may be used, such as antibody-based methods, e.g. immunoprecipitation, magnetic bead purification, resin-based purification, using specific antibodies.
(2) Quantification
Purified microparticles can be quantified by quantification of total nucleic acid or protein levels, e.g. various PCR or colorimetric protein quantification methods such as such as the BCA assay. Other quantification techniques may alternatively be used, including an electron microscopy grid or an immune-assay using antibodies or antibody fragments that specifically bind to microparticle-specific markers (e.g. ELISA, immunoblotting).
(3) Characterisation
The microparticles can be functionally or structurally characterised. RNA/mRNA/miRNA and protein profiling can be used using methods well known in the art (SDS-PAGE, mass spectrometry, PCR). Constitutively secreted microparticles can be tested and compared to microparticles that have been induced by addition of an inducing agent such as transforming growth factor-beta (TGF-β), interferon-gamma (INF-y) and/or tumour necrosis factor-alpha (TNF-α).
(4) Therapeutic Efficacy
The efficacy of the microparticles can be tested by *in vitro* and *in vivo* assays. For *in vitro* evaluation, neural stem cell microparticles can be added to cultures of monocytes, PBMCs, endothelial cells and/or fibroblasts and the effect of the microparticles on these cells evaluated. Administration of neural stem cell microparticles to suitable animal models can be used to evaluate the *in vivo* efficacy. Clinical trials can be performed to evaluate safety and outcome of neural stem cell microparticles in human subjects.
(5) Manufacture/Scale-Up
Bioreactors, such as the Integra disposable T1000, can be used for the large-scale manufacture of neural stem cell microparticles. The purified microparticles are then formulated as a therapeutic product.

**Example 10: Integra CELLINE - Disposable Bioreactor for the production of Micro particles from CTX0E03 cells.**

Efficient micro particle production and harvest from a cell line relies upon maintaining optimal culture conditions for the greatest density of cells. Any restriction in the oxygen or nutrients supplied to the cells or an accumulation of waste metabolic products will limit the life span of the culture, and hence the micro particle production.

The two-compartment CELLine AD 1000 is designed to accommodate adherent cells attached to a matrix inlay within a small cell compartment, separated from a larger media reservoir by means of a 10kDa semi-permeable membrane. This membrane allows a continuous diffusion of nutrients and removal of waste products, while concentrating any micro particles produced by the cell within the smaller cell compartment. Due to the large volume capacity (1 litre) of the media compartment, the system has the potential to maintain high density cultures for longer periods of time without the need for a media change. The production of exosomes from mesothelioma tumour cell cultures is described in Mitchell *et al,* 2008.

Method

In order to obtain optimal performance of the CELLine AD1000, place 25ml of complete growth medium (RMM with growth factors and 4OHT) into the medium compartment of the flask to pre-wet the semi-permeable membrane. Allow the flask to sit for 5 minutes at room temperature before coating the matrix inlay with mouse Laminin by adding 15ml of laminin solution (20μg/ml in DMEM/F12) to the cell compartment for a minimum of 1 hour at 37°C. Remove the laminin solution and add 15ml of warm DMEM/F12 to the cell compartment to remove any excess laminin. Avoiding the matrix inlay drying, slowly introduce approximately 15x$10^6$ CTX0E03 cells in a total of 15ml of complete growth medium. Take care to remove any air bubbles from the cell compartment. Carefully add a further 460ml of complete growth medium to the cell compartment before incubating the flask overnight in 5% $CO_2$ at 37°C. The next day remove the medium from the cell compartment and replace with 15ml of pre warmed growth medium.

Every 7 days harvest the microparticles/medium from the cell compartment. Centrifuge the medium at 1500rpm for 5 minutes to remove any cell debris and store at -80°C. Carefully add another 15ml of pre-warmed complete growth medium in to the cell compartment and 485ml of complete growth medium to the medium compartment and incubate for another 7 days. Microparticles were isolated by 100K MWCO filtration. Repeat as necessary.

Marker characterisations indicated that both purified populations (microvesicles and exosomes) express CD63 and CD81 (determined by FACS - Figure 6). Only the exosomes express the endosomal marker Alix (determined by Western blot, data not shown).

Figure 8A shows the amount of protein extracted from 15ml of media containing microparticles purified using the Integra system compared to normal culture conditions (3 days T175). Milligrams of protein measured by BCA assay. Figure 8B shows the corresponding quantity of isolated total RNA measured at 260/280nm.

**Example 11:** Size distribution of Microparticles

NanoSight analysis was undertaken to determine the particle size and concentration of microvesicles ("mv1" to "mv6") and exosomes ("exo1" to "exo6") isolated from CTX0E03 cells cultured in the Integra Celline system for 1, 2, 3, 4, 5 and 6 weeks. All results are based on 5 replicate measurements.

Particle size distribution was measured using Nanoparticle Tracking Analysis (NTA). NTA detects the movement of particles in solution and relates it to particle size. Mode and median particle size was calculated for all samples. Exosome samples were analysed using the most sensitive camera settings in order to capture the smallest vesicles. Microvesicle samples were analysed using less sensitive camera settings to prevent over exposure of the larger vesicles. As a result, some smaller vesicles were not detected in the samples. Although smaller vesicles were present in the MV samples, these represent a small percentage of the sample in terms of mass.

A proportion of Exo1 was labelled with a fluorescent membrane-specific dye (CellMask™) and a combination of NTA analysis with the CellMask™ labelling confirmed that the events detected by NTA correspond to membrane vesicles (data not shown).

The results are shown in Table 3 below, and in Figure 7.

The exosomes show a drop in size at week six, from a mode of approximately 110nm to approximately 70nm, or from a median of approximately 130nm to approximately 75nm. The overall size range, from 70nm to 150nm, is consistent with the size of exosomes from other cell types, described in the art. The observed reduction in size of the exosomes to around 70nm diameter after culturing the cells for 6 weeks correlates with the increased efficacy of exosomes isolated from CTX0E03 cells that have been cultured in a multi-compartment bioreactor for 6 weeks correlates, as reported in Example 2 and Figure 3.

The microvesicles are, as expected, larger, with a mode diameter of approximately 150nm - 200nm, or a median diameter of approximately 180nm - 350nm.

**Table 3:** Size distribution of CTX0E03 microvesicles and exosomes.

| **Sample** | Count | Dilution | **Concentration x$10^{12}$/ml** | **Mode (nm)** | **Median (nm)** |
|---|---|---|---|---|---|
| **Exo1 (1)** | 5.204 | 10000 | **32.26** | **107** | **151** |
| **Exo1 (2)** | 1.734 | 10000 | **10.75** | **135** | **164** |

(continued)

| Sample | Count | Dilution | Concentration x10$^{12}$/ml | Mode (nm) | Median (nm) |
|---|---|---|---|---|---|
| **Exo1 (3)** | 6.55 | 10000 | **40.61** | **108** | **128** |
| | | | | | |
| **Exo2** | 14.33 | 10000 | **88.85** | **118** | **153** |
| | | | | | |
| **Exo3 (1)*** | 2.52 | 10000 | **15.62** | **89** | **115** |
| **Exo3 (2)** | 10.06 | 10000 | **62.37** | **115** | **146** |
| **Exo3 (3)** | 8.98 | 10000 | **55.68** | **128** | **147** |
| | | | | | |
| **Exo4 (1)** | 3.04 | 10000 | **18.85** | **111** | **136** |
| **Exo4 (2)** | 2.89 | 10000 | **17.92** | **110** | **120** |
| **Exo4 (3)** | 2.77 | 10000 | **17.17** | **116** | **134** |
| | | | | | |
| **Exo5 (1)** | 2.34 | 100 | **0.15** | **99** | **117** |
| **Exo5 (2)** | 2.02 | 100 | **0.13** | **102** | **124** |
| **Exo 5 (3)** | 2.08 | 100 | **0.13** | **116** | **127** |
| | | | | | |
| **Exo6 (1)** | 1.45 | 100 | **0.09** | **68** | **74** |
| **Exo6 (2)** | 1.19 | 100 | **0.07** | **69** | **75** |
| | | | | | |
| **MV1 (1)** | 9.314 | 200 | **1.15** | **183** | **212** |
| **MV1 (2)** | 10.76 | 200 | **1.33** | **161** | **214** |
| **MV1 (3)** | 10.738 | 200 | **1.33** | **173** | **198** |
| | | | | | |
| **MV2** | 5.89 | 1000 | **3.65** | **177** | **194** |
| | | | | | |
| **MV3 (1)*** | 5.68 | 2000 | **7.04** | **150** | **186** |
| **MV3 (2)** | 11.5 | 2000 | **14.26** | **221** | **351** |
| **MV3 (3)** | 9.57 | 2000 | **11.87** | **214** | **270** |
| **MV4 (1)** | 4.894 | 400 | **1.21** | **209** | **240** |
| **MV4 (2)** | 2.934 | 1000 | **1.82** | **195** | **212** |
| **MV4 (3)** | 2.55 | 1000 | **1.58** | **184** | **221** |
| | | | | | |
| **MV5 (1)** | 1.086 | 200 | **0.13** | **164** | **237** |
| **MV5 (2)** | 1.458 | 200 | **0.18** | **205** | **205** |
| **MV 5 (3)** | 1.3 | 200 | **0.16** | **219** | **210** |
| | | | | | |
| **MV6 (1)** | 0.346 | 200 | **0.04** | **171** | **186** |
| **MV6 (2)** | 0.37 | 200 | **0.05** | **168** | **212** |

(continued)

| **Sample** | Count | Dilution | **Concentration x10$^{12}$/ml** | **Mode (nm)** | **Median (nm)** |
|---|---|---|---|---|---|
| | | | | | |
| **Media** | 0.14 | 10 | **0.00** | **100** | **149** |
| * large aggregates. | | | | | |

**Example 12: miRNA characterization in CTX0E03 microparticles**

Methods

- 3 conditions: CTX0E03 cells in standard culture; microparticles obtained from CTX0E03 cells in standard culture; and purified exosomes derived from CTX0E03 cells in Integra CELLine system (see Examples 10 to 16)
- Investigation of miRNA array using qRT-PCR panel (Qiagen) according to manufacturer's instruction. This assay provides high precision and high sensitivity, with data normalization sensitive to method/choice of reference genes. It does not provide genome wide sequencing.

Results:

A) List of miRNAs with a cp ≤ 35 found in (i) standard CTX0E03 cells, (ii) filtered conditioned medium (0.02-0.2μm filter) i.e. microparticles and (iii) exosomes derived from Integra CELLine system (preliminary miRNA qRT-PCR miscript array (Qiagen) results).

B) Arithmetic and geometric mean of the reference (housekeeping) genes

A

| Mature miRNA | CTX0E03 std culture | CM microparticles | CM exosome Integra |
|---|---|---|---|
| hsa-miR-21-5p | 19.52 | 20.9 | 20.72 |
| hsa-let-7a-5p | 22.64 | 23.11 | 22.36 |
| hsa-miR-125b-5p | 21.64 | 23.25 | 21.74 |
| hsa-miR-9-5p | 22.58 | 23.64 | 22.94 |
| hsa-miR-92a-3p | 23.2 | 23.94 | 24.01 |
| hsa-miR-24-3p | 23.73 | 24.24 | 23.83 |
| hsa-miR-20a-5p | 23.45 | 24.43 | 25.06 |
| hsa-miR-16-5p | 23.14 | 24.72 | 24.32 |
| hsa-miR-100-5p | 23.28 | 24.74 | 23.04 |
| hsa-let-7b-5p | 24.67 | 24.75 | 23.7 |
| hsa-let-7f-5p | 23.93 | 25.09 | 23.86 |
| hsa-miR-17-5p | 24.56 | 25.24 | 26.13 |
| hsa-miR-23b-3p | 24.3 | 25.3 | 24.13 |
| hsa-miR-106b-5p | 24.4 | 25.41 | 26.16 |
| hsa-miR-222-3p | 23.25 | 25.49 | 23.17 |

(continued)

| Mature miRNA | CTX0E03 std culture | CM microparticles | CM exosome Integra |
|---|---|---|---|
| hsa-let-7e-5p | 24.57 | 25.58 | 24.16 |
| hsa-miR-26a-5p | 23.4 | 25.63 | 24.2 |
| hsa-miR-181a-5p | 25.16 | 25.7 | 24.32 |
| hsa-miR-125a-5p | 23.56 | 25.75 | 24.88 |
| hsa-miR-103a-3p | 24.65 | 25.8 | 25.77 |
| hsa-let-7i-5p | 24.37 | 25.98 | 24.23 |
| hsa-miR-99a-5p | 24.44 | 26.05 | 23.44 |
| hsa-let-7c | 25.76 | 26.12 | 24.07 |
| hsa-let-7g | 25.2 | 26.15 | 25.17 |
| hsa-miR-195-5p | 24.72 | 26.34 | 25.67 |
| hsa-miR-93-5p | 25.15 | 26.48 | 26.06 |
| hsa-miR-22-3p | 25.03 | 26.49 | 25.66 |
| hsa-miR-20b-5p | 26.03 | 26.86 | 27.42 |
| hsa-miR-18a-5p | 26.71 | 26.87 | 29.06 |
| hsa-miR-15b-5p | 25.1 | 26.92 | 26.43 |
| hsa-let-7d-5p | 26.84 | 26.96 | 26.52 |
| hsa-miR-424-5p | 25.56 | 27.72 | 26.66 |
| hsa-miR-15a-5p | 26.88 | 27.89 | 29.3 |
| hsa-miR-130a-3p | 27.23 | 28.26 | 28.49 |
| hsa-miR-33a-5p | 30.34 | 28.54 | 34.18 |
| hsa-miR-128- | 26.94 | 28.64 | 27.66 |
| hsa-miR-218-5p | 27.79 | 28.68 | 28.03 |
| hsa-miR-301a-3p | 29.53 | 28.69 | 31.57 |
| hsa-miR-134 | 28.3 | 28.76 | 28.76 |
| hsa-miR-101-3p | 28.44 | 28.82 | 31.64 |
| hsa-miR-7-5p | 29.71 | 28.82 | 30.22 |
| hsa-miR-18b-5p | 28.83 | 28.85 | 35.47 |
| hsa-miR-185-5p | 28.34 | 28.99 | 28.13 |
| hsa-miR-378-3p | 29.76 | 29.25 | 28.97 |
| hsa-miR-132-3p | 28.65 | 29.32 | 27.72 |
| hsa-miR-345-5p | 28.49 | 29.52 | 29.66 |
| hsa-miR-219-5p | 30.58 | 29.52 | 32.7 |
| hsa-miR-127-5p | 30.05 | 29.95 | 31.11 |
| hsa-miR-146b-5p | 30.53 | 30.54 | 28.07 |
| hse-miR-10e-5p | 27.1 | 30.69 | 28.32 |
| hsa-miR-210 | 29.85 | 30.83 | 30.65 |
| hsa-miR-129-5p | 32.51 | 30.98 | 31.69 |
| hsa-miR-137 | 31.46 | 31.13 | 30.95 |

(continued)

| Mature miRNA | CTX0E03 std culture | CM microparticles | CM exosome Integra |
|---|---|---|---|
| hsa-miR-182-5p | 28.34 | 31.64 | 31.27 |
| hsa-miR-124-3p | 33.38 | 31.71 | 33.07 |
| hsa-miR-96-5p | 29.77 | 32.27 | 34.67 |
| hsa-miR-192-5p | 31.42 | 32.42 | 32.52 |
| hsa-miR-126-3p | 31.73 | 32.44 | 32.05 |
| hsa-miR-194-5p | 31.11 | 32.49 | 31.72 |
| hsa-miR-375 | 33.77 | 32.94 | 30.94 |
| hsa-miR-205-5p | 35 | 33.01 | 32.72 |
| hsa-miR-183-5p | 29.88 | 33.21 | 31.74 |
| hsa-miR-10b-5p | 29.6 | 33.22 | 30.79 |
| hsa-miR-302a-3p | 29.67 | 33.6 | 31.69 |
| hsa-miR-214-3p | 34.19 | 33.76 | 32.11 |
| hsa-miR-141-3p | 35 | 33.96 | 34.51 |
| hsa-miR-302c-3p | 31.6 | 34.29 | 33.93 |
| hsa-miR-196a-5p | 35 | 34.65 | 35.75 |
| hsa-miR-150-5p | 34.59 | 34.76 | 34.59 |
| hsa-miR-155-p | 32.04 | 35.75 | 32.76 |

B

| | CTX0E03 std culture | CM microparticles | CM exosome Integra |
|---|---|---|---|
| Avg. of Arithmetic Mean | 23.54 | 23.82 | 24.79 |
| Avg. of Geometric Mean | 23.48 | 23.8 | 24.62 |

**Example 13: CTX0E03 conditioned medium analysis using a protein dot blot**

Methods

- Conditioned 24hr and 72 hrs conditioned medium (RMM and ITS medium)
- The collected media has been 'concentrated' by dialysis and the proteins biotinylated (typical total protein concentration appears to be 0.5 mg/ml). The media is then incubated with the Raybiotech L507 human protein arrays (total protein concentration 0.1 mg/ml). Following washing and incubation of the array with HRP-conjugated streptavidin, the presence of proteins is detected by chemiluminescence. The array provides qualitative data (i.e. the protein is present, but no indication of its level of expression compared to other proteins).

Results

| Cytokine Name | Cytokine Full Name | Function |
|---|---|---|
| EDA-A2 | ectodysplasin-A2 | May be involved in proper formation of skin appendages |

(continued)

| Cytokine Name | Cytokine Full Name | Function |
|---|---|---|
| Galectin-3* | Galectin-3 | Galactose-specific lectin which binds IgE. May mediate with the alpha-3, beta-1 integrin the stimulation by CSPG4 of endothelial cells migration. |
| IGFBP-2 | Insulin-like growth factor binding proteins 2 | IGF-binding proteins prolong the half-life of the IGFs and have been shown to either inhibit or stimulate the growth promoting effects of the IGFs on cell culture. |
| IGFBP-rp1/IGFBP-7 | Insulin-like Growth Factor Binding Protein Related Protein-1 Insulin-like Growth Factor Binding Protein-7 | soluble proteins that bind IGFs with high affinity. |
| IL-1a† | Interleukin 1 alpha | potent mediator of inflammation and immunity |
| LECT2† | Leukocyte cell-derived chemotaxin-2 | Has a neutrophil chemotactic activity. Also a positive regulator of chondrocyte proliferation. |
| MCP-1 † | Monocyte chemoattractant protein 1 | plays a role in the recruitment of monocytes to sites of injury and infection. |
| SPARC* | Secreted Protein, Acidic Cysteine-rich-related modular calcium-binding protein 1 [Precursor] | matricellular protein that modulates cell adhesion and proliferation and is thought to function in tissue remodeling and angiogenesis |
| TIMP-1* | Tissue inhibitor of metalloproteinasess-2 | Complexes with metalloproteinases (such as collagenases) and irreversibly inactivates them. Also mediates erythropoiesis in vitro; but, unlike IL-3, it is species-specific, stimulating the growth and differentiation of only human and murine erythroid progenitors. |
| Thrombospondin-1* | Thrombospondin-1 | multimodular secreted protein that associates with the extracellular matrix and possesses a variety of biologic functions, including a potent angiogenic activity. |
| VEGF* | Vascular endothelial growth factor | Growth factor active in angiogenesis, vasculogenesis and endothelial cell growth. |
| These proteins show expression in some instances -though may also be present in media. | | |
| EGF R/ErbB1 | Epidermal growth factor receptor | Receptor for EGF, but also for other members of the EGF family, as TGF-alpha, amphiregulin, betacellulin, heparin-binding EGF-like growth factor |
| MDC* | A disintegrin and metalloproteinase domain 11 Metalloproteinase-like, disintegrin-like, and cysteine-rich protein MDC | Probable ligand for integrin in the brain. This is a non catalytic metalloprotease-like protein. |
| Endostatin* | Endostatin | Angiogenesis inhibitor; inhibits endothelial cell migration but may not effect proliferation. May work in balance with VEGF to maintain level of angiogenesis. |
| Follistatin | Follistatin | Regulates stem cell renewal versus differentiation by inhibiting pro-differentiation proteins |

(continued)

| Cytokine Name | Cytokine Full Name | Function |
|---|---|---|
| Csk† | cytoplasmic tyrosine kinase | Activity is required for interleukin 6 (IL-6) induced differentiation. May play a role in the growth and differentiation of hematopoietic cells. May be involved in signal transduction in endocardial and arterial endothelial cells. |
| * = angiogenesis<br>† = inflammation | | |

**Example 14: Production of exosomes using the Integra CELLine system.**

CTX0E03 cells were cultured using the Integra CELLine system and exosomes were purified as described in Example 10. The concentration of exosomes purified from the medium using the CELLine system at the 3 week time point, and as a control a standard T175 system as routinely used in the art, was quantified (using a BCA assay to estimate protein content). Figure 9 shows that the production of exosomes using the Integra CELLine system is increased several fold, compared to using conventional culture (T175 flasks).

Using the Integra CELLine system, CTX0E03 cells were cultured over a 3-week period and medium was harvested at week 1, 2 and 3 for purification and quantification of exosomes, as described in Example 10. Figure 10A shows that the production of microparticles increases exponentially over the 3-week culture period, enabling efficient and large-scale production of microparticles. The concentration of exosomes harvested from a single Integra CELLine flask was then monitored over 1-6 weeks of continuous CTX0E03 culture, with the results shown below and depicted in Figure 10B:

| Integra time point | Total quantity of exosomes (ug) | Exosomes ug/ml |
|---|---|---|
| | | |
| Week 1 | 12 | 0.80 |
| Week 2 | 112 | 7.47 |
| Week 3 | 88 | 5.87 |
| Week 4 | 148 | 9.87 |
| Week 5 | 240 | 16.00 |
| Week 6 | 440 | 29.33 |

These results show that exosome production is surprisingly enhanced when stem cells are cultured in a multi-compartment bioreactor for weeks, typically at least three weeks.

**Example 15:** Characterisation of phenotype of cells obtained from the Integra CELLine and the standard (T175) culture system.

CTX0E03 cells were cultured using the Integra CELLine bioreactor and standard culture, as described in Example 10. Expression of DCX and GFAP protein markers was confirmed using marker-specific antibodies and fluorescence microscopy.

Expression of DCX, GALC, GFAP, TUBB3, GDNF and IDO markers was detected by qRT-PCR in samples obtained from the cells. Marker expression was compared between microparticles obtained from standard (T175) culture and exosomes obtained from the 3 week cultured Integra CELLine system, assessed against a baseline of the expression level in CTX0E03 cells in standard (T175) culture.

The inventors observed a striking difference in marker expression of cells obtained from the Integra CELLine system as compared to control cells obtained from standard. Markers of partially-differentiated cells were increased several fold in cells cultured in the Integra CELLine system, compared to control cells obtained from standard cultures (Figure 11). Particularly striking changes are increased expression of the markers DCX1 (doublecortin - a marker for entry into the neural lineage), GFAP (glial fibrillary acidic protein - a marker for entry into the astrocytic lineage), GDNF (glial cell-derived neurotrophic factor) and IDO (indoleamine 2,3-dioxygenase). This indicates that in neural stem cells cultured in a two-compartment bioreactor partially differentiate into cells of neural (DCX+) or astrocytic (GFAP+) lineage. The expression of DCX and GFAP in the Integra-cultured cells was confirmed by fluorescence microscopy, demonstrating that CTX0E03 cells cultured using the Integra CELLine bioreactor have a more differentiated neuronal phenotype than standard CTX0E03 cells.

**Example 16:** Characterisation of miRNA expression profiles of exosomes obtained from Integra CELLine cultures and microparticles obtained from standard (T175) cultures.

CTX0E03 cells were cultured for three weeks using the Integra CELLine culture and in the standard culture in single-compartment T-175 flasks. Exosomes were purified from the Integra culture and microparticles were purified from the standard T-175 culture as described in Example 10. The relative expression levels of various miRNAs expressed in the exosomes and microparticles obtained from either the standard culture or the Integra CELLine system were determined with an miRNA array using qRT-PCR panel (Qiagen) according to manufacturer's instruction, and converted into fold up and down regulation levels as compared to a standard CTX0E03 cell line control group (see Table 4 and Figure 12). These data show a differential miRNA expression profile between exosomes obtained from the Integra CELLine culture system for 3 weeks, microparticles, and cells obtained from the standard single-flask culture.

**Table 4:** Fold-regulation of miRNAs in microparticles obtained from standard culture or exosomes from the Integra CELLine system, relative to control (CTX0E03 cells).

| | **Standard Culture (microparticles)** | **Integra (exosomes)** |
|---|---|---|
| **miRNA** | **Fold regulation relative to control (CTX0E03 cells)** | |
| hsa-miR-146b-5p | -1.0222 | 10.5805 |
| hsa-let-7c | -1.6954 | 4.7678 |
| hsa-miR-99a-5p | -3.5349 | 3.3714 |
| hsa-miR-132-3p | -1.9163 | 3.088 |
| hsa-miR-378-3p | 1.2731 | 3.0175 |
| hsa-miR-181a-5p | -1.7431 | 2.9147 |
| hsa-let-7b-5p | -1.4658 | 2.7574 |
| hsa-miR-100-5p | -3.208 | 1.977 |
| hsa-let-7e-5p | -2.7101 | 1.9274 |
| hsa-miR-23b-3p | -2.3322 | 1.8834 |
| hsa-miR-185-5p | -1.9119 | 1.8532 |
| hsa-let-7i-5p | -3.5677 | 1.8404 |
| hsa-let-7a-5p | -1.851 | 1.7736 |
| hsa-let-7d-5p | -1.5 | 1.7654 |
| hsa-let-7g-5p | -2.2527 | 1.7092 |
| hsa-miR-222-3p | -5.8092 | 1.6779 |
| hsa-let-7f-5p | -2.8712 | 1.5948 |
| hsa-miR-218-5p | -1.9611 | 1.5619 |
| hsa-miR-24-3p | -1.6721 | 1.5511 |
| hsa-miR-9-5p | -2.2475 | 1.4109 |
| hsa-miR-126-3p | -2.1263 | 1.203 |
| hsa-miR-134 | -1.6567 | 1.1783 |
| hsa-miR-128 | -3.5842 | 1.0743 |
| hsa-miR-155-5p | -8.8458 | 1.0425 |
| hsa-miR-22-3p | -3.4782 | -1.0023 |

(continued)

| | Standard Culture (microparticles) | Integra (exosomes) |
|---|---|---|
| miRNA | Fold regulation relative to control (CTX0E03 cells) | |
| hsa-miR-26a-5p | -5.3579 | -1.0187 |
| hsa-miR-210 | -2.3107 | -1.0449 |
| hsa-miR-92a-3p | -1.9885 | -1.0693 |
| hsa-miR-93-5p | -3.056 | -1.1701 |
| hsa-miR-424-5p | -4.9189 | -1.2086 |
| hsa-miR-195-5p | -3.8951 | -1.2541 |
| hsa-miR-127-5p | -1.1316 | -1.2953 |
| hsa-miR-21-5p | -2.8845 | -1.3044 |
| hsa-miR-103a-3p | -2.6482 | -1.3287 |
| hsa-miR-16-5p | -3.5267 | -1.3692 |
| hsa-miR-125a-5p | -5.1159 | -1.434 |
| hsa-miR-10a-5p | -14.4701 | -1.434 |
| hsa-miR-10b-5p | -15.1194 | -1.4373 |
| hsa-miR-345-5p | -2.5521 | -1.4406 |
| hsa-miR-130a-3p | -2.6178 | -1.5728 |
| hsa-miR-15b-5p | -4.4025 | -1.6058 |
| hsa-miR-20b | -2.1312 | -1.6096 |
| hsa-miR-20a-5p | -2.3107 | -1.8319 |
| hsa-miR-17-5p | -1.9296 | -1.8319 |
| hsa-miR-7-5p | -1.5105 | -2.042 |
| hsa-miR-106b-5p | -2.4708 | -2.1287 |
| hsa-miR-101-3p | 1.4794 | -2.4453 |
| hsa-miR-302a-3p | -18.0634 | -2.4623 |
| hsa-miR-301a-3p | 1.4931 | -2.5257 |
| hsa-miR-183-5p | -13.9772 | -2.5847 |
| hsa-miR-219-5p | 1.6994 | -2.7321 |
| hsa-miR-18a-5p | -1.4028 | -3.2792 |
| hsa-miR-15a-5p | -2.4766 | -3.3714 |
| hsa-miR-182-5p | -12.5099 | -4.9588 |
| hsa-miR-33a-5p | 2.7927 | -9.1472 |
| hsa-miR-96-5p | -7.0047 | -18.9396 |
| hsa-miR-18b-5p | -1.3519 | -49.18 |

Values were calculated from raw data using the following equations:

$$\Delta\mathrm{CT\ (sample/control)} = \mathrm{Average\ CT\ (GOI)} - \mathrm{Average\ CT\ (HKG)}$$

$$\mathrm{Fold\ expression\ (sample/control)} = 2^{-(\mathrm{Average}\ \Delta\mathrm{CT})}$$

$$\text{Fold change} = \frac{\text{Fold expression (sample)}}{\text{Fold expression (control)}}$$

$$\text{If (fold change)} > 1 \text{ then (fold regulation)} = \text{(fold change)}$$

$$\text{If (fold change)} < 1 \text{ then (fold regulation)} = -(\frac{1}{\text{fold change}})$$

Wherein:

CT = cycle threshold
GOI = gene of interest (investigated miRNA)
HKG = housekeeping genes (reference miRNAs used to normalize the data)

**Example 17:** Total miRNA analysis

Cells can shuttle RNA into microparticles determined for release into the extracellular space. This allows the conveyance of genetically encoded messages between cells. We here collectively refer to extracellular RNA as 'shuttle RNA'. We aimed to analyze comprehensively non coding RNA species released by CTX0E03 neural stem cells (NSCs) using Next Generation Sequencing.

Non coding RNAs are divided in two categories (small and long). Small non coding RNA biotypes include ribosomal RNA (rRNA), small nucleolar (snoRNA), small nuclear RNA (snRNA), microRNA (miRNA), miscellaneous other RNA (misc_RNA, e.g. RMRP, vault RNA, metazoa SRP, and RNY), and long non coding RNA biotypes includes long non-coding RNAs (lncRNAs) and large intergenic non-coding RNAs (lincRNAs).

Here, we characterized shuttle RNAs, including small and long non coding RNAs, released from NSC derived exosomes and microvesicles (MV) and compared with the RNA contents of the producer NSCs.

***A) Total RNA contents in cells, exosomes and microvesicles identified by Agilent RNA bioanalyser***

The RNA in both exosomes and microvesicles mainly consists of small RNA species as shown in Fig. 14. The majority of the nucleotides (nt) was ≤200 as shown against the molecular ladder.

***B) RNA composition***

Small RNA sequencing libraries were generated to investigate the composition of shuttle and cellular RNA by deep sequencing (Next Generation Sequencing). The results are shown in Figure 15.

***C) Deep sequencing of CTX0E03 cell, microvesicle and exosome miRNA expression from standard (T175) cultures.***

Deep sequencing is based on the preparation of a cDNA library following by sequencing and provides information regarding the total sequence read out of different miRNAs in the microvesicles and exosomes. These deep sequence data complement the qRT-PCR array data shown above and provide a comprehensive analysis of the miRNA profile of the cells and microparticles. Unlike the qRT-PCR array analysis, deep sequencing is not restricted to identification of sequences present in the probe array and so the sequences to be identified do not need to be known in advance. Deep sequencing also provides direct read-out and the ability to sequence very short sequences. However, deep sequencing is not suitable for detection of transcripts with low expression.

Method

The presence of a variety of miRNAs in parental cells and their exosomes (80-100μm) and microvesicles (100-1000 μm), purified by differential centrifugation, was identified by deep sequencing, following construction of 1 tagged miRNA library for each sample.

Additionally, specific primers for highly shuttled miRNAs (e.g. hsa-miR-1246) were designed and used in real-time reverse transcription PCR (qRT-PCR) to trace exosomes/microvesicles following *in vivo* implantation.

Deep sequencing was performed by GATC Biotech (Germany) and required the preparation of a tagged miRNA library for each samples followed by sequencing, and miRBase scanning:

- Construction of tagged miRNA libraries (22 to 30 nt)
  ◦ Sequencing libraries were generated by ligation of specific RNA adapter to both 3' and 5' ends for each sample followed by reverse transcription, amplification, and purification of smallRNA libraries (size range of contained smallRNA fraction 22 - 30 nt).

- Sequencing on an Illumina HiSeq 2000 (single read)
  ◦ Sequencing was performed using Illumina HiSeq 2000 (single read). Analysis of one pool could include up to 45,000,000 single read, and each read length is up to 50 bases. Sequencing was quality controlled by using FastQ Files (sequences and quality scores).

- Identification of known miRNAs was performed as followed:
  ◦ RNA adapters were trimmed from resulting sequences and raw data cleaned. Raw data were clustered and for each cluster a number of reads was provided. MiRNAs were identified by miRBase scanning (Ssearch).

Results

Many microvesicle and exosome miRNAs were enriched relative to the cells, indicating that cells specially sort miRNAs for extracellular release. Furthermore, miRNA contents were similar in both exosomes and microvesicles, indicating a common apparatus of selective miRNA uptake in excreted microvesicles. Without wishing to be bound by theory, this may indicate that miRNA content in secreted microvesicles and exosomes can be used as a fingerprint to identify hNSC subtypes.

The deep sequencing analysis therefore identified a unique set of miRNAs in both hNSC exosomes and microvesicles not previously reported. MiRNA content in excreted vesicles is similar, but showed a preferential miRNA uptake compared with hNSC. These findings could support biological effects mediated by shuttle miRNA not previously described for hNSC.

The results are detailed in Tables 5 to 10, below. The data are also depicted in Figure 13, which clearly shows the significantly different miRNA profiles present in the microvesicles and exosomes, compared to the cells. In summary, these data show a massive increase in the amount (read counts) of hsa-miR-1246, hsa-miR-4492, hsa-miR-4488 and hsa-miR-4532 in microvesicles and exosomes compared to the cells. Large increases are also seen in hsa-miR-4508, hsa-miR-4516, hsa-miR-3676-5p and hsa-miR-4485. Massive decreases are seen in the amounts (read counts) of certain miRNAs, including hsa-let-7a-5p, has-miR-92b-3p, has-miR-21-5p. hsa-miR-92a-3p, hsa-miR-10a-5p, hsa-100-5p and hsa-99b-5p.

The presence of each of hsa-miR-1246, hsa-miR-4488, hsa-miR-4492, hsa-miR-4508, hsa-miR-4516 and hsa-miR-4532 in the exosomes was validated by qRT-PCR (data not shown).

Plotting the deep sequencing results in the exosomes and microvesicles as relative fold change compared to the cells confirms that hsa-miR-1246, hsa-miR-4492, hsa-miR-4488 and hsa-miR-4532 are significantly upregulated in the exosomes and microvesicles compared to the cells. This comparison also shows that miRNA hsa-miR-3195 is the miRNA that is most upregulated, in both exosomes and microvesicles. Although the absolute reads of hsa-miR-3195 are in the range of -40 for exosomes and microvesicles, there is no hsa-miR-3195 present in the cells.

As noted in Example 16 above, miRNA contents in exosomes, microparticles, and parental cells were also tested and validated using *PCR array* analysis. The following miRNAs were found present by qRT-PCR: hsa-let-7g-5p, hsa-miR-101-3p, hsa-miR-10a-5p, hsa-miR-10b-5p, hsa-miR-125b-5p, hsa-miR-128, hsa-miR-130a-3p, hsa-miR-134, hsa-miR-137, hsa-miR-146b-5p, hsa-miR-15a-5p, hsa-miR-15b-5p, hsa-miR-16-5p, hsa-miR-17-5p, hsa-miR-181a-5p,hsa-miR-182-5p, hsa-miR-185-5p, hsa-miR-18b-5p, hsa-miR-192-5p, hsa-miR-194-5p, hsa-miR-195-5p, hsa-miR-20a-5p, hsa-miR-20b-5p, hsa-miR-210, hsa-miR-21-5p, hsa-miR-218-5p,hsa-miR-219-5p,hsa-miR-222-3p, hsa-miR-22-3p, hsa-miR-23b-3p, hsa-miR-24-3p,hsa-miR-26a-5p, hsa-miR-301a-3p, hsa-miR-302a-3p,hsa-miR-302c-3p,hsa-miR-345-5p, hsa-miR-378a-3p, hsa-miR-7-5p, hsa-miR-92a-3p,hsa-miR-93-5p,hsa-miR-9-5p,hsa-miR-96-5p, and hsa-miR-99a-5p.

**Table 5: Cells EH**

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-let-7a-5p | UGAGGUAGUAGGUUGUAUAGUU | 1 | 22 | 75110 |
| hsa-miR-10a-5p | UACCCUGUAGAUCCGAAUUUGUG | 2 | 23 | 52927 |
| hsa-miR-100-5p | AACCCGUAGAUCCGAACUUGUG | 3 | 22 | 52451 |
| hsa-miR-99b-5p | CACCCGUAGAACCGACCUUGCG | 4 | 22 | 39457 |
| hsa-miR-486-5p | UCCUGUACUGAGCUGCCCCGAG | 5 | 22 | 20310 |
| hsa-miR-27b-3p | UUCACAGUGGCUAAGUUCUGC | 6 | 21 | 16900 |
| hsa-miR-92a-3p | UAUUGCACUUGUCCCGGCCUGU | 7 | 22 | 14359 |
| hsa-miR-191-5p | CAACGGAAUCCCAAAAGCAGCUG | 8 | 23 | 12591 |
| hsa-miR-21-5p | UAGCUUAUCAGACUGAUGUUGA | 9 | 22 | 11943 |
| hsa-miR-98 | UGAGGUAGUAAGUUGUAUUGUU | 10 | 22 | 11760 |
| hsa-let-7f-5p | UGAGGUAGUAGAUUGUAUAGUU | 11 | 22 | 10349 |
| hsa-miR-26a-5p | UUCAAGUAAUCCAGGAUAGGCU | 12 | 22 | 9900 |
| hsa-miR-92b-3p | UAUUGCACUCGUCCCGGCCUCC | 13 | 22 | 9794 |
| hsa-miR-127-3p | UCGGAUCCGUCUGAGCUUGGCU | 14 | 22 | 7064 |
| hsa-miR-181a-5p | AACAUUCAACGCUGUCGGUGAGU | 15 | 23 | 6956 |
| hsa-miR-182-5p | UUUGGCAAUGGUAGAACUCACACU | 16 | 24 | 5531 |
| hsa-let-7c | UGAGGUAGUAGGUUGUAUGGUU | 17 | 22 | 5103 |
| hsa-miR-379-5p | UGGUAGACUAUGGAACGUAGG | 18 | 21 | 4746 |
| hsa-miR-146b-5p | UGAGAACUGAAUUCCAUAGGCU | 19 | 22 | 4552 |
| hsa-miR-21-3p | CAACACCAGUCGAUGGGCUGU | 20 | 21 | 4089 |
| hsa-miR-1246 | AAUGGAUUUUUGGAGCAGG | 21 | 19 | 3973 |
| hsa-let-7i-5p | UGAGGUAGUAGUUUGUGCUGUU | 22 | 22 | 3015 |
| hsa-miR-4532 | CCCCGGGGAGCCCGGCG | 23 | 17 | 2847 |
| hsa-miR-183-5p | UAUGGCACUGGUAGAAUUCACU | 24 | 22 | 2695 |
| hsa-miR-151a-3p | CUAGACUGAAGCUCCUUGAGG | 25 | 21 | 2681 |
| hsa-miR-501-3p | AAUGCACCCGGGCAAGGAUUCU | 26 | 22 | 2649 |
| hsa-let-7e-5p | UGAGGUAGGAGGUUGUAUAGUU | 27 | 22 | 2449 |
| hsa-let-7b-5p | UGAGGUAGUAGGUUGUGUGGUU | 28 | 22 | 2435 |
| hsa-miR-16-5p | UAGCAGCACGUAAAUAUUGGCG | 29 | 22 | 2173 |
| hsa-miR-30a-5p | UGUAAACAUCCUCGACUGGAAG | 30 | 22 | 2001 |
| hsa-miR-30d-5p | UGUAAACAUCCCCGACUGGAAG | 31 | 22 | 1977 |
| hsa-miR-409-5p | AGGUUACCCGAGCAACUUUGCAU | 32 | 23 | 1871 |
| hsa-miR-22-3p | AAGCUGCCAGUUGAAGAACUGU | 33 | 22 | 1826 |
| hsa-miR-4492 | GGGGCUGGGCGCGCGCC | 34 | 17 | 1754 |
| hsa-miR-125a-5p | UCCCUGAGACCCUUUAACCUGUGA | 35 | 24 | 1451 |

(continued)

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-miR-222-3p | AGCUACAUCUGGCUACUGGGU | 36 | 21 | 1422 |
| hsa-miR-151a-5p | UCGAGGAGCUCACAGUCUAGU | 37 | 21 | 1386 |
| hsa-miR-181b-5p | AACAUUCAUUGCUGUCGGUGGGU | 38 | 23 | 1382 |
| hsa-miR-221-5p | ACCUGGCAUACAAUGUAGAUUU | 39 | 22 | 1363 |
| hsa-miR-186-5p | CAAAGAAUUCUCCUUUUGGGCU | 40 | 22 | 1225 |
| hsa-miR-423-5p | UGAGGGGCAGAGAGCGAGACUUU | 41 | 23 | 1080 |
| hsa-miR-125b-5p | UCCCUGAGACCCUAACUUGUGA | 42 | 22 | 1002 |
| hsa-let-7g-5p | UGAGGUAGUAGUUUGUACAGUU | 43 | 22 | 959 |
| hsa-miR-500a-3p | AUGCACCUGGGCAAGGAUUCUG | 44 | 22 | 923 |
| hsa-miR-30e-5p | UGUAAACAUCCUUGACUGGAAG | 45 | 22 | 911 |
| hsa-miR-27a-3p | UUCACAGUGGCUAAGUUCCGC | 46 | 21 | 867 |
| hsa-miR-409-3p | GAAUGUUGCUCGGUGAACCCCU | 47 | 22 | 865 |
| hsa-miR-148b-3p | UCAGUGCAUCACAGAACUUUGU | 48 | 22 | 856 |
| hsa-miR-125b-1-3p | ACGGGUUAGGCUCUUGGGAGCU | 49 | 22 | 851 |
| hsa-miR-410 | AAUAUAACACAGAUGGCCUGU | 50 | 21 | 848 |
| hsa-miR-381 | UAUACAAGGGCAAGCUCUCUGU | 51 | 22 | 842 |
| hsa-miR-99a-5p | AACCCGUAGAUCCGAUCUUGUG | 52 | 22 | 773 |
| hsa-let-7d-5p | AGAGGUAGUAGGUUGCAUAGUU | 53 | 22 | 765 |
| hsa-miR-148a-3p | UCAGUGCACUACAGAACUUUGU | 54 | 22 | 702 |
| hsa-miR-23a-3p | AUCACAUUGCCAGGGAUUUCC | 55 | 21 | 654 |
| hsa-miR-28-3p | CACUAGAUUGUGAGCUCCUGGA | 56 | 22 | 593 |
| hsa-miR-423-3p | AGCUCGGUCUGAGGCCCCUCAGU | 57 | 23 | 557 |
| hsa-miR-9-5p | UCUUUGGUUAUCUAGCUGUAUGA | 58 | 23 | 518 |
| hsa-miR-23b-3p | AUCACAUUGCCAGGGAUUACC | 59 | 21 | 508 |
| hsa-miR-941 | CACCCGGCUGUGUGCACAUGUGC | 60 | 23 | 492 |
| hsa-miR-4488 | AGGGGGCGGGCUCCGGCG | 61 | 18 | 485 |
| hsa-miR-103a-3p | AGCAGCAUUGUACAGGGCUAUGA | 62 | 23 | 459 |
| hsa-miR-25-3p | CAUUGCACUUGUCUCGGUCUGA | 63 | 22 | 436 |
| hsa-miR-889 | UUAAUAUCGGACAACCAUUGU | 64 | 21 | 411 |
| hsa-miR-378a-3p | ACUGGACUUGGAGUCAGAAGG | 65 | 21 | 410 |
| hsa-miR-30c-5p | UGUAAACAUCCUACACUCUCAGC | 66 | 23 | 378 |
| hsa-miR-4485 | UAACGGCCGCGGUACCCUAA | 67 | 20 | 358 |
| hsa-miR-125b-2-3p | UCACAAGUCAGGCUCUUGGGAC | 68 | 22 | 352 |
| hsa-miR-671-3p | UCCGGUUCUCAGGGCUCCACC | 69 | 21 | 350 |
| hsa-miR-361-5p | UUAUCAGAAUCUCCAGGGGUAC | 70 | 22 | 337 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-30e-3p | CUUUCAGUCGGAUGUUUACAGC | 71 | 22 | 294 |
| hsa-miR-1271-5p | CUUGGCACCUAGCAAGCACUCA | 72 | 22 | 288 |
| hsa-miR-589-5p | UGAGAACCACGUCUGCUCUGAG | 73 | 22 | 282 |
| hsa-miR-374a-5p | UUAUAAUACAACCUGAUAAGUG | 74 | 22 | 275 |
| hsa-miR-769-5p | UGAGACCUCUGGGUUCUGAGCU | 75 | 22 | 263 |
| hsa-miR-345-5p | GCUGACUCCUAGUCCAGGGCUC | 76 | 22 | 249 |
| hsa-miR-30a-3p | CUUUCAGUCGGAUGUUUGCAGC | 77 | 22 | 236 |
| hsa-miR-15b-5p | UAGCAGCACAUCAUGGUUUACA | 78 | 22 | 229 |
| hsa-miR-221-3p | AGCUACAUUGUCUGCUGGGUUUC | 79 | 23 | 225 |
| hsa-miR-31-5p | AGGCAAGAUGCUGGCAUAGCU | 80 | 21 | 213 |
| hsa-miR-342-3p | UCUCACACAGAAAUCGCACCCGU | 81 | 23 | 205 |
| hsa-miR-136-3p | CAUCAUCGUCUCAAAUGAGUCU | 82 | 22 | 203 |
| hsa-miR-493-3p | UGAAGGUCUACUGUGUGCCAGG | 83 | 22 | 192 |
| hsa-miR-720 | UCUCGCUGGGGCCUCCA | 84 | 17 | 154 |
| hsa-miR-7-5p | UGGAAGACUAGUGAUUUUGUUGU | 85 | 23 | 154 |
| hsa-miR-130b-3p | CAGUGCAAUGAUGAAAGGGCAU | 86 | 22 | 150 |
| hsa-miR-192-5p | CUGACCUAUGAAUUGACAGCC | 87 | 21 | 138 |
| hsa-miR-493-5p | UUGUACAUGGUAGGCUUUCAUU | 88 | 22 | 115 |
| hsa-miR-204-5p | UUCCCUUUGUCAUCCUAUGCCU | 89 | 22 | 113 |
| hsa-miR-26b-5p | UUCAAGUAAUUCAGGAUAGGU | 90 | 21 | 107 |
| hsa-miR-1307-5p | UCGACCGGACCUCGACCGGCU | 91 | 21 | 105 |
| hsa-let-7d-3p | CUAUACGACCUGCUGCCUUUCU | 92 | 22 | 103 |
| hsa-miR-340-5p | UUAUAAAGCAAUGAGACUGAUU | 93 | 22 | 100 |
| hsa-miR-134 | UGUGACUGGUUGACCAGAGGGG | 94 | 22 | 99 |
| hsa-miR-432-5p | UCUUGGAGUAGGUCAUUGGGUGG | 95 | 23 | 97 |
| hsa-miR-30b-5p | UGUAAACAUCCUACACUCAGCU | 96 | 22 | 96 |
| hsa-miR-320a | AAAAGCUGGGUUGAGAGGGCGA | 97 | 22 | 95 |
| hsa-miR-100-3p | CAAGCUUGUAUCUAUAGGUAUG | 98 | 22 | 94 |
| hsa-miR-744-5p | UGCGGGGCUAGGGCUAACAGCA | 99 | 22 | 89 |
| hsa-miR-181a-3p | ACCAUCGACCGUUGAUUGUACC | 100 | 22 | 86 |
| hsa-miR-34a-5p | UGGCAGUGUCUUAGCUGGUUGU | 101 | 22 | 85 |
| hsa-miR-181a-2-3p | ACCACUGACCGUUGACUGUACC | 102 | 22 | 81 |
| hsa-miR-190a | UGAUAUGUUUGAUAUAUUAGGU | 103 | 22 | 79 |
| hsa-miR-132-3p | UAACAGUCUACAGCCAUGGUCG | 104 | 22 | 78 |
| hsa-miR-181c-5p | AACAUUCAACCUGUCGGUGAGU | 105 | 22 | 76 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-29a-3p | UAGCACCAUCUGAAAUCGGUUA | 106 | 22 | 75 |
| hsa-miR-301a-3p | CAGUGCAAUAGUAUUGUCAAAGC | 107 | 23 | 75 |
| hsa-miR-411-5p | UAGUAGACCGUAUAGCGUACG | 108 | 21 | 75 |
| hsa-miR-128 | UCACAGUGAACCGGUCUCUUU | 109 | 21 | 74 |
| hsa-miR-4516 | GGGAGAAGGGUCGGGGC | 110 | 17 | 74 |
| hsa-miR-425-5p | AAUGACACGAUCACUCCCGUUGA | 111 | 23 | 72 |
| hsa-miR-130b-5p | ACUCUUUCCCUGUUGCACUAC | 112 | 21 | 71 |
| hsa-miR-130a-3p | CAGUGCAAUGUUAAAAGGGCAU | 113 | 22 | 67 |
| hsa-miR-30d-3p | CUUUCAGUCAGAUGUUUGCUGC | 114 | 22 | 65 |
| hsa-miR-654-5p | UGGUGGGCCGCAGAACAUGUGC | 115 | 22 | 65 |
| hsa-miR-93-5p | CAAAGUGCUGUUCGUGCAGGUAG | 116 | 23 | 65 |
| hsa-miR-487b | AAUCGUACAGGGUCAUCCACUU | 117 | 22 | 63 |
| hsa-miR-484 | UCAGGCUCAGUCCCCUCCCGAU | 118 | 22 | 62 |
| hsa-miR-24-3p | UGGCUCAGUUCAGCAGGAACAG | 119 | 22 | 61 |
| hsa-miR-4677-3p | UCUGUGAGACCAAAGAACUACU | 120 | 22 | 61 |
| hsa-miR-149-5p | UCUGGCUCCGUGUCUUCACUCCC | 121 | 23 | 56 |
| hsa-miR-197-3p | UUCACCACCUUCUCCACCCAGC | 122 | 22 | 56 |
| hsa-miR-96-5p | UUUGGCACUAGCACAUUUUUGCU | 123 | 23 | 56 |
| hsa-miR-1307-3p | ACUCGGCGUGGCGUCGGUCGUG | 124 | 22 | 55 |
| hsa-miR-34c-5p | AGGCAGUGUAGUUAGCUGAUUGC | 125 | 23 | 53 |
| hsa-miR-370 | GCCUGCUGGGGUGGAACCUGGU | 126 | 22 | 52 |
| hsa-miR-148b-5p | AAGUUCUGUUAUACACUCAGGC | 127 | 22 | 51 |
| hsa-miR-335-5p | UCAAGAGCAAUAACGAAAAAUGU | 128 | 23 | 51 |
| hsa-miR-4461 | GAUUGAGACUAGUAGGGCUAGGC | 129 | 23 | 50 |
| hsa-miR-27a-5p | AGGGCUUAGCUGCUUGUGAGCA | 130 | 22 | 49 |
| hsa-miR-363-3p | AAUUGCACGGUAUCCAUCUGUA | 131 | 22 | 47 |
| hsa-miR-431-5p | UGUCUUGCAGGCCGUCAUGCA | 132 | 21 | 47 |
| hsa-miR-877-5p | GUAGAGGAGAUGGCGCAGGG | 133 | 20 | 46 |
| hsa-miR-550a-5p | AGUGCCUGAGGGAGUAAGAGCCC | 134 | 23 | 45 |
| hsa-miR-4508 | GCGGGGCUGGGCGCGCG | 135 | 17 | 44 |
| hsa-miR-541-3p | UGGUGGGCACAGAAUCUGGACU | 136 | 22 | 42 |
| hsa-miR-135b-5p | UAUGGCUUUUCAUUCCUAUGUGA | 137 | 23 | 40 |
| hsa-miR-140-3p | UACCACAGGGUAGAACCACGG | 138 | 21 | 39 |
| hsa-miR-362-5p | AAUCCUUGGAACCUAGGUGUGAGU | 139 | 24 | 37 |
| hsa-miR-455-3p | GCAGUCCAUGGGCAUAUACAC | 140 | 21 | 37 |

(continued)

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-miR-758 | UUUGUGACCUGGUCCACUAACC | 141 | 22 | 37 |
| hsa-miR-101-3p | UACAGUACUGUGAUAACUGAA | 142 | 21 | 36 |
| hsa-miR-374b-5p | AUAUAAUACAACCUGCUAAGUG | 143 | 22 | 36 |
| hsa-miR-148a-5p | AAAGUUCUGAGACACUCCGACU | 144 | 22 | 35 |
| hsa-miR-17-5p | CAAAGUGCUUACAGUGCAGGUAG | 145 | 23 | 35 |
| hsa-miR-20a-5p | UAAAGUGCUUAUAGUGCAGGUAG | 146 | 23 | 35 |
| hsa-miR-874 | CUGCCCUGGCCCGAGGGACCGA | 147 | 22 | 35 |
| hsa-miR-193b-3p | AACUGGCCCUCAAAGUCCCGCU | 148 | 22 | 34 |
| hsa-miR-548ah-3p | CAAAAACUGCAGUUACUUUUGC | 149 | 22 | 34 |
| hsa-miR-539-3p | AUCAUACAAGGACAAUUUCUUU | 150 | 22 | 33 |
| hsa-miR-421 | AUCAACAGACAUUAAUUGGGCGC | 151 | 23 | 31 |
| hsa-miR-28-5p | AAGGAGCUCACAGUCUAUUGAG | 152 | 22 | 30 |
| hsa-miR-485-3p | GUCAUACACGGCUCUCCUCUCU | 153 | 22 | 29 |
| hsa-miR-2467-5p | UGAGGCUCUGUUAGCCUUGGCUC | 154 | 23 | 26 |
| hsa-miR-4449 | CGUCCCGGGGCUGCGCGAGGCA | 155 | 22 | 26 |
| hsa-miR-24-2-5p | UGCCUACUGAGCUGAAACACAG | 156 | 22 | 25 |
| hsa-miR-181d | AACAUUCAUUGUUGUCGGUGGGU | 157 | 23 | 24 |
| hsa-miR-323a-3p | CACAUUACACGGUCGACCUCU | 158 | 21 | 24 |
| hsa-miR-106b-3p | CCGCACUGUGGGUACUUGCUGC | 159 | 22 | 23 |
| hsa-miR-125a-3p | ACAGGUGAGGUUCUUGGGAGCC | 160 | 22 | 23 |
| hsa-miR-330-5p | UCUCUGGGCCUGUGUCUUAGGC | 161 | 22 | 23 |
| hsa-miR-1275 | GUGGGGGAGAGGCUGUC | 162 | 17 | 22 |
| hsa-miR-19b-3p | UGUGCAAAUCCAUGCAAAACUGA | 163 | 23 | 22 |
| hsa-miR-301b | CAGUGCAAUGAUAUUGUCAAAGC | 164 | 23 | 21 |
| hsa-miR-485-5p | AGAGGCUGGCCGUGAUGAAUUC | 165 | 22 | 21 |
| hsa-miR-29b-3p | UAGCACCAUUUGAAAUCAGUGUU | 166 | 23 | 20 |
| hsa-miR-3158-3p | AAGGGCUUCCUCUCUGCAGGAC | 167 | 22 | 20 |
| hsa-miR-431-3p | CAGGUCGUCUUGCAGGGCUUCU | 168 | 22 | 20 |
| hsa-miR-454-3p | UAGUGCAAUAUUGCUUAUAGGGU | 169 | 23 | 20 |
| hsa-miR-106b-5p | UAAAGUGCUGACAGUGCAGAU | 170 | 21 | 19 |
| hsa-miR-1973 | ACCGUGCAAAGGUAGCAUA | 171 | 19 | 19 |
| hsa-miR-31-3p | UGCUAUGCCAACAUAUUGCCAU | 172 | 22 | 19 |
| hsa-miR-374a-3p | CUUAUCAGAUUGUAUUGUAAUU | 173 | 22 | 19 |
| hsa-miR-433 | AUCAUGAUGGGCUCCUCGGUGU | 174 | 22 | 19 |
| hsa-miR-4417 | GGUGGGCUUCCCGGAGGG | 175 | 18 | 19 |

(continued)

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-miR-143-3p | UGAGAUGAAGCACUGUAGCUC | 176 | 21 | 18 |
| hsa-miR-19a-3p | UGUGCAAAUCUAUGCAAAACUGA | 177 | 23 | 18 |
| hsa-miR-532-5p | CAUGCCUUGAGUGUAGGACCGU | 178 | 22 | 18 |
| hsa-miR-561-5p | AUCAAGGAUCUUAAACUUUGCC | 179 | 22 | 18 |
| hsa-miR-663b | GGUGGCCCGGCCGUGCCUGAGG | 180 | 22 | 18 |
| hsa-miR-1301 | UUGCAGCUGCCUGGGAGUGACUUC | 181 | 24 | 17 |
| hsa-miR-299-3p | UAUGUGGGAUGGUAAACCGCUU | 182 | 22 | 17 |
| hsa-miR-9-3p | AUAAAGCUAGAUAACCGAAAGU | 183 | 22 | 17 |
| hsa-miR-17-3p | ACUGCAGUGAAGGCACUUGUAG | 184 | 22 | 15 |
| hsa-miR-376c | AACAUAGAGGAAAUUCCACGU | 185 | 21 | 15 |
| hsa-miR-424-5p | CAGCAGCAAUUCAUGUUUUGAA | 186 | 22 | 15 |
| hsa-miR-660-5p | UACCCAUUGCAUAUCGGAGUUG | 187 | 22 | 15 |
| hsa-miR-153 | UUGCAUAGUCACAAAAGUGAUC | 188 | 22 | 14 |
| hsa-miR-3605-5p | UGAGGAUGGAUAGCAAGGAAGCC | 189 | 23 | 14 |
| hsa-miR-3687 | CCCGGACAGGCGUUCGUGCGACGU | 190 | 24 | 14 |
| hsa-miR-4284 | GGGCUCACAUCACCCCAU | 191 | 18 | 14 |
| hsa-miR-455-5p | UAUGUGCCUUUGGACUACAUCG | 192 | 22 | 14 |
| hsa-miR-543 | AAACAUUCGCGGUGCACUUCUU | 193 | 22 | 14 |
| hsa-miR-1276 | UAAAGAGCCCUGUGGAGACA | 194 | 20 | 13 |
| hsa-miR-330-3p | GCAAAGCACACGGCCUGCAGAGA | 195 | 23 | 13 |
| hsa-miR-369-3p | AAUAAUACAUGGUUGAUCUUU | 196 | 21 | 13 |
| hsa-miR-4786-5p | UGAGACCAGGACUGGAUGCACC | 197 | 22 | 13 |
| hsa-miR-548k | AAAAGUACUUGCGGAUUUUGCU | 198 | 22 | 13 |
| hsa-miR-1226-3p | UCACCAGCCCUGUGUUCCCUAG | 199 | 22 | 12 |
| hsa-miR-188-3p | CUCCCACAUGCAGGGUUUGCA | 200 | 21 | 12 |
| hsa-miR-27b-5p | AGAGCUUAGCUGAUUGGUGAAC | 201 | 22 | 12 |
| hsa-miR-377-5p | AGAGGUUGCCCUUGGUGAAUUC | 202 | 22 | 12 |
| hsa-miR-487a | AAUCAUACAGGGACAUCCAGUU | 203 | 22 | 12 |
| hsa-miR-92a-1-5p | AGGUUGGGAUCGGUUGCAAUGCU | 204 | 23 | 12 |
| hsa-miR-135b-3p | AUGUAGGGCUAAAAGCCAUGGG | 205 | 22 | 11 |
| hsa-miR-218-5p | UUGUGCUUGAUCUAACCAUGU | 206 | 21 | 11 |
| hsa-miR-3943 | UAGCCCCCAGGCUUCACUUGGCG | 207 | 23 | 11 |
| hsa-miR-92b-5p | AGGGACGGGACGCGGUGCAGUG | 208 | 22 | 11 |
| hsa-miR-1185-1-3p | AUAUACAGGGGGAGACUCUUAU | 209 | 22 | 10 |
| hsa-miR-1273g-3p | ACCACUGCACUCCAGCCUGAG | 210 | 21 | 10 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-2355-5p | AUCCCCAGAUACAAUGGACAA | 211 | 21 | 10 |
| hsa-miR-23a-5p | GGGGUUCCUGGGGAUGGGAUUU | 212 | 22 | 10 |
| hsa-miR-30c-1-3p | CUGGGAGAGGGUUGUUUACUCC | 213 | 22 | 10 |
| hsa-miR-329 | AACACACCUGGUUAACCUCUUU | 214 | 22 | 10 |
| hsa-miR-337-3p | CUCCUAUAUGAUGCCUUUCUUC | 215 | 22 | 10 |
| hsa-miR-3609 | CAAAGUGAUGAGUAAUACUGGCUG | 216 | 24 | 10 |
| hsa-miR-378a-5p | CUCCUGACUCCAGGUCCUGUGU | 217 | 22 | 10 |
| hsa-miR-3929 | GAGGCUGAUGUGAGUAGACCACU | 218 | 23 | 10 |
| hsa-miR-4745-5p | UGAGUGGGGCUCCCGGGACGGCG | 219 | 23 | 10 |
| hsa-miR-5096 | GUUUCACCAUGUUGGUCAGGC | 220 | 21 | 10 |
| hsa-miR-656 | AAUAUUAUACAGUCAACCUCU | 221 | 21 | 10 |
| hsa-let-7a-3p | CUAUACAAUCUACUGUCUUUC | 222 | 21 | 9 |
| hsa-miR-15a-5p | UAGCAGCACAUAAUGGUUUGUG | 223 | 22 | 9 |
| hsa-miR-185-5p | UGGAGAGAAAGGCAGUUCCUGA | 224 | 22 | 9 |
| hsa-miR-25-5p | AGGCGGAGACUUGGGCAAUUG | 225 | 21 | 9 |
| hsa-miR-3065-5p | UCAACAAAAUCACUGAUGCUGGA | 226 | 23 | 9 |
| hsa-miR-3176 | ACUGGCCUGGGACUACCGG | 227 | 19 | 9 |
| hsa-miR-339-3p | UGAGCGCCUCGACGACAGAGCCG | 228 | 23 | 9 |
| hsa-miR-374b-3p | CUUAGCAGGUUGUAUUAUCAUU | 229 | 22 | 9 |
| hsa-miR-4435 | AUGGCCAGAGCUCACACAGAGG | 230 | 22 | 9 |
| hsa-miR-4448 | GGCUCCUUGGUCUAGGGGUA | 231 | 20 | 9 |
| hsa-miR-4497 | CUCCGGGACGGCUGGGC | 232 | 17 | 9 |
| hsa-miR-4521 | GCUAAGGAAGUCCUGUGCUCAG | 233 | 22 | 9 |
| hsa-miR-539-5p | GGAGAAAUUAUCCUUGGUGUGU | 234 | 22 | 9 |
| hsa-miR-548ah-5p | AAAAGUGAUUGCAGUGUUUG | 235 | 20 | 9 |
| hsa-miR-1910 | CCAGUCCUGUGCCUGCCGCCU | 236 | 21 | 8 |
| hsa-miR-376a-3p | AUCAUAGAGGAAAAUCCACGU | 237 | 21 | 8 |
| hsa-miR-382-5p | GAAGUUGUUCGUGGUGGAUUCG | 238 | 22 | 8 |
| hsa-miR-3940-3p | CAGCCCGGAUCCCAGCCCACUU | 239 | 22 | 8 |
| hsa-miR-494 | UGAAACAUACACGGGAAACCUC | 240 | 22 | 8 |
| hsa-miR-495 | AAACAAACAUGGUGCACUUCUU | 241 | 22 | 8 |
| hsa-miR-545-3p | UCAGCAAACAUUUAUUGUGUGC | 242 | 22 | 8 |
| hsa-miR-99b-3p | CAAGCUCGUGUCUGUGGGUCCG | 243 | 22 | 8 |
| hsa-miR-1197 | UAGGACACAUGGUCUACUUCU | 244 | 21 | 7 |
| hsa-miR-181b-3p | CUCACUGAACAAUGAAUGCAA | 245 | 21 | 7 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-212-5p | ACCUUGGCUCUAGACUGCUUACU | 246 | 23 | 7 |
| hsa-miR-3200-3p | CACCUUGCGCUACUCAGGUCUG | 247 | 22 | 7 |
| hsa-miR-340-3p | UCCGUCUCAGUUACUUUAUAGC | 248 | 22 | 7 |
| hsa-miR-3607-5p | GCAUGUGAUGAAGCAAAUCAGU | 249 | 22 | 7 |
| hsa-miR-361-3p | UCCCCCAGGUGUGAUUCUGAUUU | 250 | 23 | 7 |
| hsa-miR-3656 | GGCGGGUGCGGGGGUGG | 251 | 17 | 7 |
| hsa-miR-532-3p | CCUCCCACACCCAAGGCUUGCA | 252 | 22 | 7 |
| hsa-miR-574-3p | CACGCUCAUGCACACACCCACA | 253 | 22 | 7 |
| hsa-miR-107 | AGCAGCAUUGUACAGGGCUAUCA | 254 | 23 | 6 |
| hsa-miR-127-5p | CUGAAGCUCAGAGGGCUCUGAU | 255 | 22 | 6 |
| hsa-miR-18a-5p | UAAGGUGCAUCUAGUGCAGAUAG | 256 | 23 | 6 |
| hsa-miR-26a-2-3p | CCUAUUCUUGAUUACUUGUUUC | 257 | 22 | 6 |
| hsa-miR-296-5p | AGGGCCCCCCCUCAAUCCUGU | 258 | 21 | 6 |
| hsa-miR-3648 | AGCCGCGGGGAUCGCCGAGGG | 259 | 21 | 6 |
| hsa-miR-382-3p | AAUCAUUCACGGACAACACUU | 260 | 21 | 6 |
| hsa-miR-3939 | UACGCGCAGACCACAGGAUGUC | 261 | 22 | 6 |
| hsa-miR-432-3p | CUGGAUGGCUCCUCCAUGUCU | 262 | 21 | 6 |
| hsa-miR-4423-5p | AGUUGCCUUUUUGUUCCCAUGC | 263 | 22 | 6 |
| hsa-miR-4466 | GGGUGCGGGCCGGCGGGG | 264 | 18 | 6 |
| hsa-miR-454-5p | ACCCUAUCAAUAUUGUCUCUGC | 265 | 22 | 6 |
| hsa-miR-4746-5p | CCGGUCCCAGGAGAACCUGCAGA | 266 | 23 | 6 |
| hsa-miR-496 | UGAGUAUUACAUGGCCAAUCUC | 267 | 22 | 6 |
| hsa-miR-548o-3p | CCAAAACUGCAGUUACUUUUGC | 268 | 22 | 6 |
| hsa-miR-1248 | ACCUUCUUGUAUAAGCACUGUGCUAAA | 269 | 27 | 5 |
| hsa-miR-1254 | AGCCUGGAAGCUGGAGCCUGCAGU | 270 | 24 | 5 |
| hsa-miR-1296 | UUAGGGCCCUGGCUCCAUCUCC | 271 | 22 | 5 |
| hsa-miR-136-5p | ACUCCAUUUGUUUUGAUGAUGGA | 272 | 23 | 5 |
| hsa-miR-199a-5p | CCCAGUGUUCAGACUACCUGUUC | 273 | 23 | 5 |
| hsa-miR-296-3p | GAGGGUUGGGUGGAGGCUCUCC | 274 | 22 | 5 |
| hsa-miR-3177-3p | UGCACGGCACUGGGGACACGU | 275 | 21 | 5 |
| hsa-miR-324-3p | ACUGCCCCAGGUGCUGCUGG | 276 | 20 | 5 |
| hsa-miR-337-5p | GAACGGCUUCAUACAGGAGUU | 277 | 21 | 5 |
| hsa-miR-342-5p | AGGGGUGCUAUCUGUGAUUGA | 278 | 21 | 5 |
| hsa-miR-365b-3p | UAAUGCCCCUAAAAAUCCUUAU | 279 | 22 | 5 |
| hsa-miR-3676-5p | AGGAGAUCCUGGGUU | 280 | 15 | 5 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-502-3p | AAUGCACCUGGGCAAGGAUUCA | 281 | 22 | 5 |
| hsa-miR-505-3p | CGUCAACACUUGCUGGUUUCCU | 282 | 22 | 5 |
| hsa-miR-550a-3p | UGUCUUACUCCCUCAGGCACAU | 283 | 22 | 5 |
| hsa-miR-5587-3p | GCCCCGGGCAGUGUGAUCAUC | 284 | 21 | 5 |
| hsa-miR-641 | AAAGACAUAGGAUAGAGUCACCUC | 285 | 24 | 5 |
| hsa-miR-655 | AUAAUACAUGGUUAACCUCUUU | 286 | 22 | 5 |
| hsa-miR-664-3p | UAUUCAUUUAUCCCCAGCCUACA | 287 | 23 | 5 |
| hsa-miR-671-5p | AGGAAGCCCUGGAGGGGCUGGAG | 288 | 23 | 5 |
| hsa-miR-760 | CGGCUCUGGGUCUGUGGGGA | 289 | 20 | 5 |
| hsa-let-7e-3p | CUAUACGGCCUCCUAGCUUUCC | 290 | 22 | 4 |
| hsa-miR-1268a | CGGGCGUGGUGGUGGGGG | 291 | 18 | 4 |
| hsa-miR-1273f | GGAGAUGGAGGUUGCAGUG | 292 | 19 | 4 |
| hsa-miR-1286 | UGCAGGACCAAGAUGAGCCCU | 293 | 21 | 4 |
| hsa-miR-1291 | UGGCCCUGACUGAAGACCAGCAGU | 294 | 24 | 4 |
| hsa-miR-141-3p | UAACACUGUCUGGUAAAGAUGG | 295 | 22 | 4 |
| hsa-miR-1468 | CUCCGUUUGCCUGUUUCGCUG | 296 | 21 | 4 |
| hsa-miR-328 | CUGGCCCUCUCUGCCCUUCCGU | 297 | 22 | 4 |
| hsa-miR-424-3p | CAAAACGUGAGGCGCUGCUAU | 298 | 21 | 4 |
| hsa-miR-4454 | GGAUCCGAGUCACGGCACCA | 299 | 20 | 4 |
| hsa-miR-4463 | GAGACUGGGGUGGGGCC | 300 | 17 | 4 |
| hsa-miR-4671-3p | UUAGUGCAUAGUCUUUGGUCU | 301 | 21 | 4 |
| hsa-miR-4775 | UUAAUUUUUUGUUUCGGUCACU | 302 | 22 | 4 |
| hsa-miR-500a-5p | UAAUCCUUGCUACCUGGGUGAGA | 303 | 23 | 4 |
| hsa-miR-548b-5p | AAAAGUAAUUGUGGUUUUGGCC | 304 | 22 | 4 |
| hsa-miR-573 | CUGAAGUGAUGUGUAACUGAUCAG | 305 | 24 | 4 |
| hsa-miR-576-5p | AUUCUAAUUUCUCCACGUCUUU | 306 | 22 | 4 |
| hsa-miR-625-3p | GACUAUAGAACUUUCCCCCUCA | 307 | 22 | 4 |
| hsa-miR-652-3p | AAUGGCGCCACUAGGGUUGUG | 308 | 21 | 4 |
| hsa-miR-665 | ACCAGGAGGCUGAGGCCCCU | 309 | 20 | 4 |
| hsa-miR-766-3p | ACUCCAGCCCCACAGCCUCAGC | 310 | 22 | 4 |
| hsa-miR-935 | CCAGUUACCGCUUCCGCUACCGC | 311 | 23 | 4 |
| hsa-miR-937 | AUCCGCGCUCUGACUCUCUGCC | 312 | 22 | 4 |
| hsa-miR-1180 | UUUCCGGCUCGCGUGGGUGUGU | 313 | 22 | 3 |
| hsa-miR-1185-2-3p | AUAUACAGGGGGAGACUCUCAU | 314 | 22 | 3 |
| hsa-miR-132-5p | ACCGUGGCUUUCGAUUGUUACU | 315 | 22 | 3 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-16-2-3p | CCAAUAUUACUGUGCUGCUUUA | 316 | 22 | 3 |
| hsa-miR-20b-5p | CAAAGUGCUCAUAGUGCAGGUAG | 317 | 23 | 3 |
| hsa-miR-2116-3p | CCUCCCAUGCCAAGAACUCCC | 318 | 21 | 3 |
| hsa-miR-299-5p | UGGUUUACCGUCCCACAUACAU | 319 | 22 | 3 |
| hsa-miR-30b-3p | CUGGGAGGUGGAUGUUUACUUC | 320 | 22 | 3 |
| hsa-miR-30c-2-3p | CUGGGAGAAGGCUGUUUACUCU | 321 | 22 | 3 |
| hsa-miR-3187-3p | UUGGCCAUGGGGCUGCGCGG | 322 | 20 | 3 |
| hsa-miR-3615 | UCUCUCGGCUCCUCGCGGCUC | 323 | 21 | 3 |
| hsa-miR-3620 | UCACCCUGCAUCCCGCACCCAG | 324 | 22 | 3 |
| hsa-miR-3654 | GACUGGACAAGCUGAGGAA | 325 | 19 | 3 |
| hsa-miR-3662 | GAAAAUGAUGAGUAGUGACUGAUG | 326 | 24 | 3 |
| hsa-miR-3681-5p | UAGUGGAUGAUGCACUCUGUGC | 327 | 22 | 3 |
| hsa-miR-4286 | ACCCCACUCCUGGUACC | 328 | 17 | 3 |
| hsa-miR-4640-3p | CACCCCCUGUUUCCUGGCCCAC | 329 | 22 | 3 |
| hsa-miR-4717-3p | ACACAUGGGUGGCUGUGGCCU | 330 | 21 | 3 |
| hsa-miR-542-3p | UGUGACAGAUUGAUAACUGAAA | 331 | 22 | 3 |
| hsa-miR-5584-5p | CAGGGAAAUGGGAAGAACUAGA | 332 | 22 | 3 |
| hsa-miR-570-3p | CGAAAACAGCAAUUACCUUUGC | 333 | 22 | 3 |
| hsa-miR-574-5p | UGAGUGUGUGUGUGUGAGUGUGU | 334 | 23 | 3 |
| hsa-miR-628-3p | UCUAGUAAGAGUGGCAGUCGA | 335 | 21 | 3 |
| hsa-miR-654-3p | UAUGUCUGCUGACCAUCACCUU | 336 | 22 | 3 |
| hsa-miR-769-3p | CUGGGAUCUCCGGGGUCUUGGUU | 337 | 23 | 3 |
| hsa-miR-943 | CUGACUGUUGCCGUCCUCCAG | 338 | 21 | 3 |
| hsa-let-7b-3p | CUAUACAACCUACUGCCUUCCC | 339 | 22 | 2 |
| hsa-miR-1244 | AAGUAGUUGGUUUGUAUGAGAUGGUU | 340 | 26 | 2 |
| hsa-miR-1255a | AGGAUGAGCAAAGAAAGUAGAUU | 341 | 23 | 2 |
| hsa-miR-1273e | UUGCUUGAACCCAGGAAGUGGA | 342 | 22 | 2 |
| hsa-miR-1289 | UGGAGUCCAGGAAUCUGCAUUUU | 343 | 23 | 2 |
| hsa-miR-152 | UCAGUGCAUGACAGAACUUGG | 344 | 21 | 2 |
| hsa-miR-194-5p | UGUAACAGCAACUCCAUGUGGA | 345 | 22 | 2 |
| hsa-miR-195-5p | UAGCAGCACAGAAAUAUUGGC | 346 | 21 | 2 |
| hsa-miR-200c-3p | UAAUACUGCCGGGUAAUGAUGGA | 347 | 23 | 2 |
| hsa-miR-212-3p | UAACAGUCUCCAGUCACGGCC | 348 | 21 | 2 |
| hsa-miR-222-5p | CUCAGUAGCCAGUGUAGAUCCU | 349 | 22 | 2 |
| hsa-miR-3065-3p | UCAGCACCAGGAUAUUGUUGGAG | 350 | 23 | 2 |

(continued)

| Cells: CTX0E03 07EH | | | | |
|---|---|---|---|---|
| MIRNA | MIRNA.SEQUENCE | SEQ ID NO: | MIRNA LENGTH | READ COUNTS |
| hsa-miR-3115 | AUAUGGGUUUACUAGUUGGU | 351 | 20 | 2 |
| hsa-miR-3126-5p | UGAGGGACAGAUGCCAGAAGCA | 352 | 22 | 2 |
| hsa-miR-3174 | UAGUGAGUUAGAGAUGCAGAGCC | 353 | 23 | 2 |
| hsa-miR-324-5p | CGCAUCCCCUAGGGCAUUGGUGU | 354 | 23 | 2 |
| hsa-miR-33a-5p | GUGCAUUGUAGUUGCAUUGCA | 355 | 21 | 2 |
| hsa-miR-3677-3p | CUCGUGGGCUCUGGCCACGGCC | 356 | 22 | 2 |
| hsa-miR-369-5p | AGAUCGACCGUGUUAUAUUCGC | 357 | 22 | 2 |
| hsa-miR-425-3p | AUCGGGAAUGUCGUGUCCGCCC | 358 | 22 | 2 |
| hsa-miR-4426 | GAAGAUGGACGUACUUU | 359 | 17 | 2 |
| hsa-miR-4467 | UGGCGGCGGUAGUUAUGGGCUU | 360 | 22 | 2 |
| hsa-miR-4482-3p | UUUCUAUUUCUCAGUGGGGCUC | 361 | 22 | 2 |
| hsa-miR-4515 | AGGACUGGACUCCCGGCAGCCC | 362 | 22 | 2 |
| hsa-miR-4792 | CGGUGAGCGCUCGCUGGC | 363 | 18 | 2 |
| hsa-miR-659-5p | AGGACCUUCCCUGAACCAAGGA | 364 | 22 | 2 |
| hsa-miR-663a | AGGCGGGGCGCCGCGGGACCGC | 365 | 22 | 2 |
| hsa-miR-940 | AAGGCAGGGCCCCCGCUCCCC | 366 | 21 | 2 |
| hsa-miR-99a-3p | CAAGCUCGCUUCUAUGGGUCUG | 367 | 22 | 2 |
| hsa-miR-1185-5p | AGAGGAUACCCUUUGUAUGUU | 368 | 21 | 1 |
| hsa-miR-1225-3p | UGAGCCCCUGUGCCGCCCCCAG | 369 | 22 | 1 |
| hsa-miR-1237 | UCCUUCUGCUCCGUCCCCCAG | 370 | 21 | 1 |
| hsa-miR-1252 | AGAAGGAAAU UGAAU UCAU U UA | 371 | 22 | 1 |
| hsa-miR-1257 | AGUGAAUGAUGGGUUCUGACC | 372 | 21 | 1 |
| hsa-miR-1260b | AUCCCACCACUGCCACCAU | 373 | 19 | 1 |
| hsa-miR-1273d | GAACCCAUGAGGUUGAGGCUGCAGU | 374 | 25 | 1 |
| hsa-miR-1290 | UGGAUUUUUGGAUCAGGGA | 375 | 19 | 1 |
| hsa-miR-1306-3p | ACGUUGGCUCUGGUGGUG | 376 | 18 | 1 |
| hsa-miR-1321 | CAGGGAGGUGAAUGUGAU | 377 | 18 | 1 |
| hsa-miR-1343 | CUCCUGGGGCCCGCACUCUCGC | 378 | 22 | 1 |
| hsa-miR-138-5p | AGCUGGUGUUGUGAAUCAGGCCG | 379 | 23 | 1 |
| hsa-miR-140-5p | CAGUGGUUUUACCCUAUGGUAG | 380 | 22 | 1 |
| hsa-miR-146b-3p | UGCCCUGUGGACUCAGUUCUGG | 381 | 22 | 1 |
| hsa-miR-186-3p | GCCCAAAGGUGAAUUUUUUGGG | 382 | 22 | 1 |
| hsa-miR-1908 | CGGCGGGGACGGCGAUUGGUC | 383 | 21 | 1 |
| hsa-miR-1915-3p | CCCCAGGGCGACGCGGCGGG | 384 | 20 | 1 |
| hsa-miR-1915-5p | ACCUUGCCUUGCUGCCCGGGCC | 385 | 22 | 1 |

(continued)

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-miR-193a-3p | AACUGGCCUACAAAGUCCCAGU | 386 | 22 | 1 |
| hsa-miR-19b-1-5p | AGUUUUGCAGGUUUGCAUCCAGC | 387 | 23 | 1 |
| hsa-miR-208b | AUAAGACGAACAAAAGGUUUGU | 388 | 22 | 1 |
| hsa-miR-2110 | UUGGGGAAACGGCCGCUGAGUG | 389 | 22 | 1 |
| hsa-miR-219-1-3p | AGAGUUGAGUCUGGACGUCCCG | 390 | 22 | 1 |
| hsa-miR-26b-3p | CCUGUUCUCCAUUACUUGGCUC | 391 | 22 | 1 |
| hsa-miR-2964a-3p | AGAAUUGCGUUUGGACAAUCAGU | 392 | 23 | 1 |
| hsa-miR-29a-5p | ACUGAUUUCUUUUGGUGUUCAG | 393 | 22 | 1 |
| hsa-miR-3126-3p | CAUCUGGCAUCCGUCACACAGA | 394 | 22 | 1 |
| hsa-miR-3130-3p | GCUGCACCGGAGACUGGGUAA | 395 | 21 | 1 |
| hsa-miR-3130-5p | UACCCAGUCUCCGGUGCAGCC | 396 | 21 | 1 |
| hsa-miR-3140-5p | ACCUGAAUUACCAAAAGCUUU | 397 | 21 | 1 |
| hsa-miR-3155a | CCAGGCUCUGCAGUGGGAACU | 398 | 21 | 1 |
| hsa-miR-3157-3p | CUGCCCUAGUCUAGCUGAAGCU | 399 | 22 | 1 |
| hsa-miR-3180-3p | UGGGGCGGAGCUUCCGGAGGCC | 400 | 22 | 1 |
| hsa-miR-323b-5p | AGGUUGUCCGUGGUGAGUUCGCA | 401 | 23 | 1 |
| hsa-miR-339-5p | UCCCUGUCCUCCAGGAGCUCACG | 402 | 23 | 1 |
| hsa-miR-34a-3p | CAAUCAGCAAGUAUACUGCCCU | 403 | 22 | 1 |
| hsa-miR-34b-3p | CAAUCACUAACUCCACUGCCAU | 404 | 22 | 1 |
| hsa-miR-34c-3p | AAUCACUAACCACACGGCCAGG | 405 | 22 | 1 |
| hsa-miR-3658 | UUUAAGAAAACACCAUGGAGAU | 406 | 22 | 1 |
| hsa-miR-365a-5p | AGGGACUUUUGGGGGCAGAUGUG | 407 | 23 | 1 |
| hsa-miR-3676-3p | CCGUGUUUCCCCCACGCUUU | 408 | 20 | 1 |
| hsa-miR-3691-5p | AGUGGAUGAUGGAGACUCGGUAC | 409 | 23 | 1 |
| hsa-miR-376a-5p | GUAGAUUCUCCUUCUAUGAGUA | 410 | 22 | 1 |
| hsa-miR-378g | ACUGGGCUUGGAGUCAGAAG | 411 | 20 | 1 |
| hsa-miR-3909 | UGUCCUCUAGGGCCUGCAGUCU | 412 | 22 | 1 |
| hsa-miR-3928 | GGAGGAACCUUGGAGCUUCGGC | 413 | 22 | 1 |
| hsa-miR-3942-3p | UUUCAGAUAACAGUAUUACAU | 414 | 21 | 1 |
| hsa-miR-3944-5p | UGUGCAGCAGGCCAACCGAGA | 415 | 21 | 1 |
| hsa-miR-3960 | GGCGGCGGCGGAGGCGGGGG | 416 | 20 | 1 |
| hsa-miR-4326 | UGUUCCUCUGUCUCCCAGAC | 417 | 20 | 1 |
| hsa-miR-4444 | CUCGAGUUGGAAGAGGCG | 418 | 18 | 1 |
| hsa-miR-4450 | UGGGGAUUUGGAGAAGUGGUGA | 419 | 22 | 1 |
| hsa-miR-4642 | AUGGCAUCGUCCCCUGGUGGCU | 420 | 22 | 1 |

(continued)

| **Cells: CTX0E03 07EH** | | | | |
|---|---|---|---|---|
| MIRNA | MIRNA.SEQUENCE | SEQ ID NO: | MIRNA LENGTH | READ COUNTS |
| hsa-miR-4668-5p | AGGGAAAAAAAAAAGGAUUUGUC | 421 | 23 | 1 |
| hsa-miR-4673 | UCCAGGCAGGAGCCGGACUGGA | 422 | 22 | 1 |
| hsa-miR-4688 | UAGGGGCAGCAGAGGACCUGGG | 423 | 22 | 1 |
| hsa-miR-4700-3p | CACAGGACUGACUCCUCACCCCAGUG | 424 | 26 | 1 |
| hsa-miR-4731-3p | CACACAAGUGGCCCCCAACACU | 425 | 22 | 1 |
| hsa-miR-4749-3p | CGCCCCUCCUGCCCCCACAG | 426 | 20 | 1 |
| hsa-miR-4769-5p | GGUGGGAUGGAGAGAAGGUAUGAG | 427 | 24 | 1 |
| hsa-miR-4800-5p | AGUGGACCGAGGAAGGAAGGA | 428 | 21 | 1 |
| hsa-miR-491-5p | AGUGGGGAACCCUUCCAUGAGG | 429 | 22 | 1 |
| hsa-miR-501-5p | AAUCCUUUGUCCCUGGGUGAGA | 430 | 22 | 1 |
| hsa-miR-5092 | AAUCCACGCUGAGCUUGGCAUC | 431 | 22 | 1 |
| hsa-miR-541-5p | AAAGGAUUCUGCUGUCGGUCCCACU | 432 | 25 | 1 |
| hsa-miR-542-5p | UCGGGGAUCAUCAUGUCACGAGA | 433 | 23 | 1 |
| hsa-miR-551b-3p | GCGACCCAUACUUGGUUUCAG | 434 | 21 | 1 |
| hsa-miR-5690 | UCAGCUACUACCUCUAUUAGG | 435 | 21 | 1 |
| hsa-miR-577 | UAGAUAAAAUAUUGGUACCUG | 436 | 21 | 1 |
| hsa-miR-584-3p | UCAGUUCCAGGCCAACCAGGCU | 437 | 22 | 1 |
| hsa-miR-589-3p | UCAGAACAAAUGCCGGUUCCCAGA | 438 | 24 | 1 |
| hsa-miR-616-5p | ACUCAAAACCCUUCAGUGACUU | 439 | 22 | 1 |
| hsa-miR-628-5p | AUGCUGACAUAUUUACUAGAGG | 440 | 22 | 1 |
| hsa-miR-629-5p | UGGGUUUACGUUGGGAGAACU | 441 | 21 | 1 |
| hsa-miR-644b-3p | UUCAUUUGCCUCCCAGCCUACA | 442 | 22 | 1 |
| hsa-miR-664-5p | ACUGGCUAGGGAAAAUGAUUGGAU | 443 | 24 | 1 |
| hsa-miR-922 | GCAGCAGAGAAUAGGACUACGUC | 444 | 23 | 1 |

**Table 6: Cells EI**

| **CELLS - CTX0E03 07EI** | | | | |
|---|---|---|---|---|
| MIRNA | MIRNA.SEQUENCE | SEQ ID NO: | MIRNA LENGTH | READ COUNTS |
| hsa-let-7a-5p | UGAGGUAGUAGGUUGUAUAGUU | 1 | 22 | 305060 |
| hsa-miR-92b-3p | UAUUGCACUCGUCCCGGCCUCC | 13 | 22 | 242715 |
| hsa-miR-21-5p | UAGCUUAUCAGACUGAUGUUGA | 9 | 22 | 154626 |
| hsa-miR-92a-3p | UAUUGCACUUGUCCCGGCCUGU | 7 | 22 | 137412 |
| hsa-miR-127-3p | UCGGAUCCGUCUGAGCUUGGCU | 14 | 22 | 110806 |
| hsa-miR-100-5p | AACCCGUAGAUCCGAACUUGUG | 3 | 22 | 109290 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-27b-3p | UUCACAGUGGCUAAGUUCUGC | 6 | 21 | 91902 |
| hsa-miR-191-5p | CAACGGAAUCCCAAAAGCAGCUG | 8 | 23 | 89150 |
| hsa-miR-26a-5p | UUCAAGUAAUCCAGGAUAGGCU | 12 | 22 | 88724 |
| hsa-miR-99b-5p | CACCCGUAGAACCGACCUUGCG | 4 | 22 | 87399 |
| hsa-let-7f-5p | UGAGGUAGUAGAUUGUAUAGUU | 11 | 22 | 78395 |
| hsa-miR-181a-5p | AACAUUCAACGCUGUCGGUGAGU | 15 | 23 | 47686 |
| hsa-miR-486-5p | UCCUGUACUGAGCUGCCCCGAG | 5 | 22 | 41639 |
| hsa-miR-30a-5p | UGUAAACAUCCUCGACUGGAAG | 30 | 22 | 35465 |
| hsa-miR-98 | UGAGGUAGUAAGUUGUAUUGUU | 10 | 22 | 30440 |
| hsa-miR-151a-3p | CUAGACUGAAGCUCCUUGAGG | 25 | 21 | 29047 |
| hsa-miR-21-3p | CAACACCAGUCGAUGGGCUGU | 20 | 21 | 27733 |
| hsa-miR-30d-5p | UGUAAACAUCCCCGACUGGAAG | 31 | 22 | 27307 |
| hsa-let-7c | UGAGGUAGUAGGUUGUAUGGUU | 17 | 22 | 27224 |
| hsa-miR-10a-5p | UACCCUGUAGAUCCGAAUUUGUG | 2 | 23 | 26908 |
| hsa-miR-22-3p | AAGCUGCCAGUUGAAGAACUGU | 33 | 22 | 26456 |
| hsa-miR-182-5p | UUUGGCAAUGGUAGAACUCACACU | 16 | 24 | 25885 |
| hsa-miR-222-3p | AGCUACAUCUGGCUACUGGGU | 36 | 21 | 22187 |
| hsa-miR-125a-5p | UCCCUGAGACCCUUUAACCUGUGA | 35 | 24 | 20960 |
| hsa-miR-16-5p | UAGCAGCACGUAAAUAUUGGCG | 29 | 22 | 19856 |
| hsa-let-7b-5p | UGAGGUAGUAGGUUGUGUGGUU | 28 | 22 | 19774 |
| hsa-miR-151a-5p | UCGAGGAGCUCACAGUCUAGU | 37 | 21 | 19773 |
| hsa-let-7e-5p | UGAGGUAGGAGGUUGUAUAGUU | 27 | 22 | 19035 |
| hsa-miR-125b-5p | UCCCUGAGACCCUAACUUGUGA | 42 | 22 | 17965 |
| hsa-let-7i-5p | UGAGGUAGUAGUUUGUGCUGUU | 22 | 22 | 17802 |
| hsa-let-7g-5p | UGAGGUAGUAGUUUGUACAGUU | 43 | 22 | 15467 |
| hsa-miR-409-3p | GAAUGUUGCUCGGUGAACCCCU | 47 | 22 | 14133 |
| hsa-miR-30e-5p | UGUAAACAUCCUUGACUGGAAG | 45 | 22 | 13889 |
| hsa-miR-181b-5p | AACAUUCAUUGCUGUCGGUGGGU | 38 | 23 | 12606 |
| hsa-miR-186-5p | CAAAGAAUUCUCCUUUUGGGCU | 40 | 22 | 12441 |
| hsa-miR-381 | UAUACAAGGGCAAGCUCUCUGU | 51 | 22 | 9851 |
| hsa-miR-423-5p | UGAGGGGCAGAGAGCGAGACUUU | 41 | 23 | 8893 |
| hsa-miR-30c-5p | UGUAAACAUCCUACACUCUCAGC | 66 | 23 | 8737 |
| hsa-miR-410 | AAUAUAACACAGAUGGCCUGU | 50 | 21 | 8509 |
| hsa-miR-146b-5p | UGAGAACUGAAUUCCAUAGGCU | 19 | 22 | 8434 |
| hsa-miR-654-3p | UAUGUCUGCUGACCAUCACCUU | 336 | 22 | 8392 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-9-5p | UCUUUGGUUAUCUAGCUGUAUGA | 58 | 23 | 7957 |
| hsa-miR-28-3p | CACUAGAUUGUGAGCUCCUGGA | 56 | 22 | 7767 |
| hsa-miR-148a-3p | UCAGUGCACUACAGAACUUUGU | 54 | 22 | 6599 |
| hsa-miR-379-5p | UGGUAGACUAUGGAACGUAGG | 18 | 21 | 6135 |
| hsa-let-7d-5p | AGAGGUAGUAGGUUGCAUAGUU | 53 | 22 | 5972 |
| hsa-miR-183-5p | UAUGGCACUGGUAGAAUUCACU | 24 | 22 | 5477 |
| hsa-miR-25-3p | CAUUGCACUUGUCUCGGUCUGA | 63 | 22 | 5303 |
| hsa-miR-423-3p | AGCUCGGUCUGAGGCCCCUCAGU | 57 | 23 | 5225 |
| hsa-miR-889 | UUAAUAUCGGACAACCAUUGU | 64 | 21 | 4597 |
| hsa-miR-221-5p | ACCUGGCAUACAAUGUAGAUUU | 39 | 22 | 4379 |
| hsa-miR-125b-1-3p | ACGGGUUAGGCUCUUGGGAGCU | 49 | 22 | 4192 |
| hsa-miR-409-5p | AGGUUACCCGAGCAACUUUGCAU | 32 | 23 | 3970 |
| hsa-miR-4492 | GGGGCUGGGCGCGCGCC | 34 | 17 | 3864 |
| hsa-miR-148b-3p | UCAGUGCAUCACAGAACUUUGU | 48 | 22 | 3593 |
| hsa-miR-103a-3p | AGCAGCAUUGUACAGGGCUAUGA | 62 | 23 | 3518 |
| hsa-miR-1271-5p | CUUGGCACCUAGCAAGCACUCA | 72 | 22 | 3477 |
| hsa-miR-136-3p | CAUCAUCGUCUCAAAUGAGUCU | 82 | 22 | 3373 |
| hsa-miR-769-5p | UGAGACCUCUGGGUUCUGAGCU | 75 | 22 | 2957 |
| hsa-miR-4532 | CCCCGGGGAGCCCGGCG | 23 | 17 | 2915 |
| hsa-miR-378a-3p | ACUGGACUUGGAGUCAGAAGG | 65 | 21 | 2895 |
| hsa-miR-99a-5p | AACCCGUAGAUCCGAUCUUGUG | 52 | 22 | 2767 |
| hsa-miR-221-3p | AGCUACAUUGUCUGCUGGGUUUC | 79 | 23 | 2764 |
| hsa-miR-30e-3p | CUUUCAGUCGGAUGUUUACAGC | 71 | 22 | 2441 |
| hsa-miR-26b-5p | UUCAAGUAAUUCAGGAUAGGU | 90 | 21 | 2432 |
| hsa-miR-4488 | AGGGGGCGGGCUCCGGCG | 61 | 18 | 2391 |
| hsa-miR-27a-3p | UUCACAGUGGCUAAGUUCCGC | 46 | 21 | 2385 |
| hsa-miR-23b-3p | AUCACAUUGCCAGGGAUUACC | 59 | 21 | 2316 |
| hsa-miR-500a-3p | AUGCACCUGGGCAAGGAUUCUG | 44 | 22 | 2144 |
| hsa-miR-941 | CACCCGGCUGUGUGCACAUGUGC | 60 | 23 | 2114 |
| hsa-miR-23a-3p | AUCACAUUGCCAGGGAUUUCC | 55 | 21 | 2086 |
| hsa-miR-30a-3p | CUUUCAGUCGGAUGUUUGCAGC | 77 | 22 | 2045 |
| hsa-miR-30b-5p | UGUAAACAUCCUACACUCAGCU | 96 | 22 | 1936 |
| hsa-miR-501-3p | AAUGCACCCGGGCAAGGAUUCU | 26 | 22 | 1895 |
| hsa-miR-130b-3p | CAGUGCAAUGAUGAAAGGGCAU | 86 | 22 | 1862 |
| hsa-miR-1246 | AAUGGAUUUUUGGAGCAGG | 21 | 19 | 1783 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-140-3p | UACCACAGGGUAGAACCACGG | 138 | 21 | 1735 |
| hsa-miR-31-5p | AGGCAAGAUGCUGGCAUAGCU | 80 | 21 | 1705 |
| hsa-miR-493-3p | UGAAGGUCUACUGUGUGCCAGG | 83 | 22 | 1698 |
| hsa-miR-181c-5p | AACAUUCAACCUGUCGGUGAGU | 105 | 22 | 1554 |
| hsa-miR-93-5p | CAAAGUGCUGUUCGUGCAGGUAG | 116 | 23 | 1492 |
| hsa-miR-181a-2-3p | ACCACUGACCGUUGACUGUACC | 102 | 22 | 1491 |
| hsa-miR-15b-5p | UAGCAGCACAUCAUGGUUUACA | 78 | 22 | 1465 |
| hsa-miR-7-5p | UGGAAGACUAGUGAUUUUGUUGU | 85 | 23 | 1460 |
| hsa-miR-192-5p | CUGACCUAUGAAUUGACAGCC | 87 | 21 | 1453 |
| hsa-miR-425-5p | AAUGACACGAUCACUCCCGUUGA | 111 | 23 | 1432 |
| hsa-miR-204-5p | UUCCCUUUGUCAUCCUAUGCCU | 89 | 22 | 1378 |
| hsa-miR-340-5p | UUAUAAAGCAAUGAGACUGAUU | 93 | 22 | 1360 |
| hsa-miR-190a | UGAUAUGUUUGAUAUAUUAGGU | 103 | 22 | 1305 |
| hsa-miR-34a-5p | UGGCAGUGUCUUAGCUGGUUGU | 101 | 22 | 1283 |
| hsa-miR-20a-5p | UAAAGUGCUUAUAGUGCAGGUAG | 146 | 23 | 1257 |
| hsa-miR-29a-3p | UAGCACCAUCUGAAAUCGGUUA | 106 | 22 | 1206 |
| hsa-miR-361-5p | UUAUCAGAAUCUCCAGGGGUAC | 70 | 22 | 1173 |
| hsa-miR-671-3p | UCCGGUUCUCAGGGCUCCACC | 69 | 21 | 1166 |
| hsa-miR-411-5p | UAGUAGACCGUAUAGCGUACG | 108 | 21 | 1130 |
| hsa-miR-589-5p | UGAGAACCACGUCUGCUCUGAG | 73 | 22 | 1067 |
| hsa-miR-130a-3p | CAGUGCAAUGUUAAAAGGGCAU | 113 | 22 | 1020 |
| hsa-miR-320a | AAAAGCUGGGUUGAGAGGGCGA | 97 | 22 | 994 |
| hsa-miR-149-5p | UCUGGCUCCGUGUCUUCACUCCC | 121 | 23 | 948 |
| hsa-miR-335-5p | UCAAGAGCAAUAACGAAAAAUGU | 128 | 23 | 945 |
| hsa-miR-134 | UGUGACUGGUUGACCAGAGGGG | 94 | 22 | 941 |
| hsa-miR-17-5p | CAAAGUGCUUACAGUGCAGGUAG | 145 | 23 | 939 |
| hsa-miR-493-5p | UUGUACAUGGUAGGCUUUCAUU | 88 | 22 | 876 |
| hsa-miR-34c-5p | AGGCAGUGUAGUUAGCUGAUUGC | 125 | 23 | 846 |
| hsa-miR-484 | UCAGGCUCAGUCCCCUCCCGAU | 118 | 22 | 835 |
| hsa-miR-181a-3p | ACCAUCGACCGUUGAUUGUACC | 100 | 22 | 803 |
| hsa-miR-24-3p | UGGCUCAGUUCAGCAGGAACAG | 119 | 22 | 740 |
| hsa-miR-128 | UCACAGUGAACCGGUCUCUUU | 109 | 21 | 707 |
| hsa-miR-342-3p | UCUCACACAGAAAUCGCACCCGU | 81 | 23 | 698 |
| hsa-miR-454-3p | UAGUGCAAUAUUGCUUAUAGGGU | 169 | 23 | 690 |
| hsa-miR-1307-5p | UCGACCGGACCUCGACCGGCU | 91 | 21 | 616 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-487b | AAUCGUACAGGGUCAUCCACUU | 117 | 22 | 590 |
| hsa-miR-130b-5p | ACUCUUUCCCUGUUGCACUAC | 112 | 21 | 568 |
| hsa-miR-197-3p | UUCACCACCUUCUCCACCCAGC | 122 | 22 | 544 |
| hsa-miR-432-5p | UCUUGGAGUAGGUCAUUGGGUGG | 95 | 23 | 542 |
| hsa-miR-374a-5p | UUAUAAUACAACCUGAUAAGUG | 74 | 22 | 537 |
| hsa-miR-345-5p | GCUGACUCCUAGUCCAGGGCUC | 76 | 22 | 527 |
| hsa-miR-744-5p | UGCGGGGCUAGGGCUAACAGCA | 99 | 22 | 515 |
| hsa-miR-376c | AACAUAGAGGAAAUUCCACGU | 185 | 21 | 506 |
| hsa-miR-181d | AACAUUCAUUGUUGUCGGUGGGU | 157 | 23 | 497 |
| hsa-miR-363-3p | AAUUGCACGGUAUCCAUCUGUA | 131 | 22 | 493 |
| hsa-miR-539-3p | AUCAUACAAGGACAAUUUCUUU | 150 | 22 | 493 |
| hsa-miR-758 | UUUGUGACCUGGUCCACUAACC | 141 | 22 | 477 |
| hsa-miR-323a-3p | CACAUUACACGGUCGACCUCU | 158 | 21 | 443 |
| hsa-miR-107 | AGCAGCAUUGUACAGGGCUAUCA | 254 | 23 | 431 |
| hsa-miR-720 | UCUCGCUGGGGCCUCCA | 84 | 17 | 427 |
| hsa-miR-654-5p | UGGUGGGCCGCAGAACAUGUGC | 115 | 22 | 409 |
| hsa-miR-370 | GCCUGCUGGGGUGGAACCUGGU | 126 | 22 | 406 |
| hsa-miR-421 | AUCAACAGACAUUAAUUGGGCGC | 151 | 23 | 399 |
| hsa-miR-30d-3p | CUUUCAGUCAGAUGUUUGCUGC | 114 | 22 | 358 |
| hsa-miR-148b-5p | AAGUUCUGUUAUACACUCAGGC | 127 | 22 | 354 |
| hsa-miR-1301 | UUGCAGCUGCCUGGGAGUGACUUC | 181 | 24 | 346 |
| hsa-miR-374b-5p | AUAUAAUACAACCUGCUAAGUG | 143 | 22 | 339 |
| hsa-miR-125b-2-3p | UCACAAGUCAGGCUCUUGGGAC | 68 | 22 | 333 |
| hsa-miR-28-5p | AAGGAGCUCACAGUCUAUUGAG | 152 | 22 | 332 |
| hsa-miR-495 | AAACAAACAUGGUGCACUUCUU | 241 | 22 | 321 |
| hsa-miR-15a-5p | UAGCAGCACAUAAUGGUUUGUG | 223 | 22 | 320 |
| hsa-miR-100-3p | CAAGCUUGUAUCUAUAGGUAUG | 98 | 22 | 314 |
| hsa-miR-193b-3p | AACUGGCCCUCAAAGUCCCGCU | 148 | 22 | 305 |
| hsa-miR-330-5p | UCUCUGGGCCUGUGUCUUAGGC | 161 | 22 | 303 |
| hsa-miR-376a-3p | AUCAUAGAGGAAAAUCCACGU | 237 | 21 | 298 |
| hsa-miR-135b-5p | UAUGGCUUUUCAUUCCUAUGUGA | 137 | 23 | 289 |
| hsa-miR-301a-3p | CAGUGCAAUAGUAUUGUCAAAGC | 107 | 23 | 280 |
| hsa-miR-218-5p | UUGUGCUUGAUCUAACCAUGU | 206 | 21 | 276 |
| hsa-miR-143-3p | UGAGAUGAAGCACUGUAGCUC | 176 | 21 | 256 |
| hsa-miR-27b-5p | AGAGCUUAGCUGAUUGGUGAAC | 201 | 22 | 255 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-369-3p | AAUAAUACAUGGUUGAUCUUU | 196 | 21 | 255 |
| hsa-miR-877-5p | GUAGAGGAGAUGGCGCAGGG | 133 | 20 | 249 |
| hsa-miR-19b-3p | UGUGCAAAUCCAUGCAAAACUGA | 163 | 23 | 246 |
| hsa-miR-424-5p | CAGCAGCAAUUCAUGUUUUGAA | 186 | 22 | 245 |
| hsa-miR-660-5p | UACCCAUUGCAUAUCGGAGUUG | 187 | 22 | 244 |
| hsa-miR-532-5p | CAUGCCUUGAGUGUAGGACCGU | 178 | 22 | 238 |
| hsa-miR-299-3p | UAUGUGGGAUGGUAAACCGCUU | 182 | 22 | 235 |
| hsa-miR-431-3p | CAGGUCGUCUUGCAGGGCUUCU | 168 | 22 | 231 |
| hsa-miR-374a-3p | CUUAUCAGAUUGUAUUGUAAUU | 173 | 22 | 220 |
| hsa-miR-148a-5p | AAAGUUCUGAGACACUCCGACU | 144 | 22 | 214 |
| hsa-miR-4516 | GGGAGAAGGGUCGGGGC | 110 | 17 | 207 |
| hsa-miR-92b-5p | AGGGACGGGACGCGGUGCAGUG | 208 | 22 | 206 |
| hsa-miR-16-2-3p | CCAAUAUUACUGUGCUGCUUUA | 316 | 22 | 202 |
| hsa-miR-101-3p | UACAGUACUGUGAUAACUGAA | 142 | 21 | 201 |
| hsa-let-7a-3p | CUAUACAAUCUACUGUCUUUC | 222 | 21 | 199 |
| hsa-miR-4485 | UAACGGCCGCGGUACCCUAA | 67 | 20 | 195 |
| hsa-miR-455-3p | GCAGUCCAUGGGCAUAUACAC | 140 | 21 | 192 |
| hsa-miR-185-5p | UGGAGAGAAAGGCAGUUCCUGA | 224 | 22 | 188 |
| hsa-miR-1185-1-3p | AUAUACAGGGGGAGACUCUUAU | 209 | 22 | 187 |
| hsa-miR-1197 | UAGGACACAUGGUCUACUUCU | 244 | 21 | 185 |
| hsa-miR-106b-3p | CCGCACUGUGGGUACUUGCUGC | 159 | 22 | 178 |
| hsa-miR-24-2-5p | UGCCUACUGAGCUGAAACACAG | 156 | 22 | 178 |
| hsa-miR-4677-3p | UCUGUGAGACCAAAGAACUACU | 120 | 22 | 177 |
| hsa-miR-380-3p | UAUGUAAUAUGGUCCACAUCUU | 445 | 22 | 174 |
| hsa-miR-548k | AAAAGUACUUGCGGAUUUUGCU | 198 | 22 | 171 |
| hsa-miR-1307-3p | ACUCGGCGUGGCGUCGGUCGUG | 124 | 22 | 169 |
| hsa-miR-485-3p | GUCAUACACGGCUCUCCUCUCU | 153 | 22 | 168 |
| hsa-miR-494 | UGAAACAUACACGGGAAACCUC | 240 | 22 | 165 |
| hsa-miR-17-3p | ACUGCAGUGAAGGCACUUGUAG | 184 | 22 | 163 |
| hsa-miR-561-5p | AUCAAGGAUCUUAAACUUUGCC | 179 | 22 | 160 |
| hsa-miR-27a-5p | AGGGCUUAGCUGCUUGUGAGCA | 130 | 22 | 158 |
| hsa-miR-874 | CUGCCCUGGCCCGAGGGACCGA | 147 | 22 | 151 |
| hsa-miR-9-3p | AUAAAGCUAGAUAACCGAAAGU | 183 | 22 | 151 |
| hsa-miR-96-5p | UUUGGCACUAGCACAUUUUUGCU | 123 | 23 | 151 |
| hsa-miR-656 | AAUAUUAUACAGUCAACCUCU | 221 | 21 | 147 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-379-3p | UAUGUAACAUGGUCCACUAACU | 446 | 22 | 145 |
| hsa-miR-382-5p | GAAGUUGUUCGUGGUGGAUUCG | 238 | 22 | 144 |
| hsa-miR-541-3p | UGGUGGGCACAGAAUCUGGACU | 136 | 22 | 141 |
| hsa-miR-337-3p | CUCCUAUAUGAUGCCUUUCUUC | 215 | 22 | 139 |
| hsa-miR-15b-3p | CGAAUCAUUAUUUGCUGCUCUA | 447 | 22 | 137 |
| hsa-miR-20b-5p | CAAAGUGCUCAUAGUGCAGGUAG | 317 | 23 | 136 |
| hsa-miR-329 | AACACACCUGGUUAACCUCUUU | 214 | 22 | 136 |
| hsa-miR-3676-5p | AGGAGAUCCUGGGUU | 280 | 15 | 134 |
| hsa-miR-543 | AAACAUUCGCGGUGCACUUCUU | 193 | 22 | 134 |
| hsa-miR-365b-3p | UAAUGCCCCUAAAAAUCCUUAU | 279 | 22 | 133 |
| hsa-miR-125a-3p | ACAGGUGAGGUUCUUGGGAGCC | 160 | 22 | 131 |
| hsa-miR-3065-5p | UCAACAAAAUCACUGAUGCUGGA | 226 | 23 | 130 |
| hsa-miR-1296 | UUAGGGCCCUGGCUCCAUCUCC | 271 | 22 | 126 |
| hsa-miR-935 | CCAGUUACCGCUUCCGCUACCGC | 311 | 23 | 118 |
| hsa-miR-132-3p | UAACAGUCUACAGCCAUGGUCG | 104 | 22 | 116 |
| hsa-miR-4284 | GGGCUCACAUCACCCCAU | 191 | 18 | 116 |
| hsa-miR-487a | AAUCAUACAGGGACAUCCAGUU | 203 | 22 | 113 |
| hsa-miR-574-5p | UGAGUGUGUGUGUGUGAGUGUGU | 334 | 23 | 113 |
| hsa-miR-301b | CAGUGCAAUGAUAUUGUCAAAGC | 164 | 23 | 111 |
| hsa-miR-548o-3p | CCAAAACUGCAGUUACUUUUGC | 268 | 22 | 105 |
| hsa-miR-18a-5p | UAAGGUGCAUCUAGUGCAGAUAG | 256 | 23 | 104 |
| hsa-miR-485-5p | AGAGGCUGGCCGUGAUGAAUUC | 165 | 22 | 104 |
| hsa-miR-548ah-5p | AAAAGUGAUUGCAGUGUUUG | 235 | 20 | 103 |
| hsa-miR-361-3p | UCCCCCAGGUGUGAUUCUGAUUU | 250 | 23 | 101 |
| hsa-miR-433 | AUCAUGAUGGGCUCCUCGGUGU | 174 | 22 | 101 |
| hsa-miR-337-5p | GAACGGCUUCAUACAGGAGUU | 277 | 21 | 100 |
| hsa-miR-1276 | UAAAGAGCCCUGUGGAGACA | 194 | 20 | 99 |
| hsa-miR-30c-1-3p | CUGGGAGAGGGUUGUUUACUCC | 213 | 22 | 99 |
| hsa-miR-31-3p | UGCUAUGCCAACAUAUUGCCAU | 172 | 22 | 96 |
| hsa-miR-424-3p | CAAAACGUGAGGCGCUGCUAU | 298 | 21 | 96 |
| hsa-miR-550a-5p | AGUGCCUGAGGGAGUAAGAGCCC | 134 | 23 | 95 |
| hsa-miR-4454 | GGAUCCGAGUCACGGCACCA | 299 | 20 | 94 |
| hsa-miR-541-5p | AAAGGAUUCUGCUGUCGGUCCCACU | 432 | 25 | 92 |
| hsa-miR-106b-5p | UAAAGUGCUGACAGUGCAGAU | 170 | 21 | 89 |
| hsa-miR-153 | UUGCAUAGUCACAAAAGUGAUC | 188 | 22 | 88 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-135b-3p | AUGUAGGGCUAAAAGCCAUGGG | 205 | 22 | 87 |
| hsa-miR-574-3p | CACGCUCAUGCACACACCCACA | 253 | 22 | 87 |
| hsa-miR-1226-3p | UCACCAGCCCUGUGUUCCCUAG | 199 | 22 | 85 |
| hsa-miR-576-5p | AUUCUAAUUUCUCCACGUCUUU | 306 | 22 | 84 |
| hsa-miR-127-5p | CUGAAGCUCAGAGGGCUCUGAU | 255 | 22 | 83 |
| hsa-miR-155-5p | UUAAUGCUAAUCGUGAUAGGGGU | 448 | 23 | 83 |
| hsa-miR-3176 | ACUGGCCUGGGACUACCGG | 227 | 19 | 83 |
| hsa-miR-382-3p | AAUCAUUCACGGACAACACUU | 260 | 21 | 83 |
| hsa-miR-1275 | GUGGGGGAGAGGCUGUC | 162 | 17 | 82 |
| hsa-miR-671-5p | AGGAAGCCCUGGAGGGGCUGGAG | 288 | 23 | 82 |
| hsa-miR-23a-5p | GGGGUUCCUGGGGAUGGGAUUU | 212 | 22 | 81 |
| hsa-miR-25-5p | AGGCGGAGACUUGGGCAAUUG | 225 | 21 | 80 |
| hsa-miR-641 | AAAGACAUAGGAUAGAGUCACCUC | 285 | 24 | 80 |
| hsa-miR-19a-3p | UGUGCAAAUCUAUGCAAAACUGA | 177 | 23 | 79 |
| hsa-miR-377-3p | AUCACACAAAGGCAACUUUUGU | 449 | 22 | 78 |
| hsa-miR-454-5p | ACCCUAUCAAUAUUGUCUCUGC | 265 | 22 | 78 |
| hsa-miR-496 | UGAGUAUUACAUGGCCAAUCUC | 267 | 22 | 78 |
| hsa-miR-29b-3p | UAGCACCAUUUGAAAUCAGUGUU | 166 | 23 | 77 |
| hsa-miR-26a-2-3p | CCUAUUCUUGAUUACUUGUUUC | 257 | 22 | 76 |
| hsa-miR-1260b | AUCCCACCACUGCCACCAU | 373 | 19 | 74 |
| hsa-miR-2467-5p | UGAGGCUCUGUUAGCCUUGGCUC | 154 | 23 | 74 |
| hsa-miR-377-5p | AGAGGUUGCCCUUGGUGAAUUC | 202 | 22 | 74 |
| hsa-miR-330-3p | GCAAAGCACACGGCCUGCAGAGA | 195 | 23 | 73 |
| hsa-miR-1180 | UUUCCGGCUCGCGUGGGUGUGU | 313 | 22 | 71 |
| hsa-miR-99b-3p | CAAGCUCGUGUCUGUGGGUCCG | 243 | 22 | 71 |
| hsa-miR-299-5p | UGGUUUACCGUCCCACAUACAU | 319 | 22 | 69 |
| hsa-miR-374b-3p | CUUAGCAGGUUGUAUUAUCAUU | 229 | 22 | 69 |
| hsa-miR-4746-5p | CCGGUCCCAGGAGAACCUGCAGA | 266 | 23 | 69 |
| hsa-miR-331-3p | GCCCCUGGGCCUAUCCUAGAA | 450 | 21 | 68 |
| hsa-miR-340-3p | UCCGUCUCAGUUACUUUAUAGC | 248 | 22 | 68 |
| hsa-miR-92a-1-5p | AGGUUGGGAUCGGUUGCAAUGCU | 204 | 23 | 68 |
| hsa-miR-542-3p | UGUGACAGAUUGAUAACUGAAA | 331 | 22 | 66 |
| hsa-miR-431-5p | UGUCUUGCAGGCCGUCAUGCA | 132 | 21 | 65 |
| hsa-miR-1254 | AGCCUGGAAGCUGGAGCCUGCAGU | 270 | 24 | 61 |
| hsa-miR-3158-3p | AAGGGCUUCCUCUCUGCAGGAC | 167 | 22 | 61 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-362-5p | AAUCCUUGGAACCUAGGUGUGAGU | 139 | 24 | 61 |
| hsa-miR-30c-2-3p | CUGGGAGAAGGCUGUUUACUCU | 321 | 22 | 59 |
| hsa-miR-4461 | GAUUGAGACUAGUAGGGCUAGGC | 129 | 23 | 59 |
| hsa-miR-3200-3p | CACCUUGCGCUACUCAGGUCUG | 247 | 22 | 57 |
| hsa-miR-215 | AUGACCUAUGAAUUGACAGAC | 451 | 21 | 56 |
| hsa-miR-1185-5p | AGAGGAUACCCUUUGUAUGUU | 368 | 21 | 55 |
| hsa-miR-328 | CUGGCCCUCUCUGCCCUUCCGU | 297 | 22 | 55 |
| hsa-miR-655 | AUAAUACAUGGUUAACCUCUUU | 286 | 22 | 55 |
| hsa-miR-181b-3p | CUCACUGAACAAUGAAUGCAA | 245 | 21 | 54 |
| hsa-miR-376b | AUCAUAGAGGAAAAUCCAUGUU | 452 | 22 | 54 |
| hsa-miR-486-3p | CGGGGCAGCUCAGUACAGGAU | 453 | 21 | 54 |
| hsa-miR-760 | CGGCUCUGGGUCUGUGGGGA | 289 | 20 | 54 |
| hsa-miR-3909 | UGUCCUCUAGGGCCUGCAGUCU | 412 | 22 | 53 |
| hsa-miR-4508 | GCGGGGCUGGGCGCGCG | 135 | 17 | 53 |
| hsa-miR-4521 | GCUAAGGAAGUCCUGUGCUCAG | 233 | 22 | 53 |
| hsa-let-7e-3p | CUAUACGGCCUCCUAGCUUUCC | 290 | 22 | 52 |
| hsa-miR-455-5p | UAUGUGCCUUUGGACUACAUCG | 192 | 22 | 52 |
| hsa-miR-93-3p | ACUGCUGAGCUAGCACUUCCCG | 454 | 22 | 51 |
| hsa-miR-151b | UCGAGGAGCUCACAGUCU | 455 | 18 | 49 |
| hsa-miR-887 | GUGAACGGGCGCCAUCCCGAGG | 456 | 22 | 49 |
| hsa-miR-152 | UCAGUGCAUGACAGAACUUGG | 344 | 21 | 48 |
| hsa-miR-324-3p | ACUGCCCCAGGUGCUGCUGG | 276 | 20 | 48 |
| hsa-miR-1266 | CCUCAGGGCUGUAGAACAGGGCU | 457 | 23 | 47 |
| hsa-miR-302b-3p | UAAGUGCUUCCAUGUUUUAGUAG | 458 | 23 | 47 |
| hsa-miR-548e | AAAAACUGAGACUACUUUUGCA | 459 | 22 | 47 |
| hsa-miR-502-3p | AAUGCACCUGGGCAAGGAUUCA | 281 | 22 | 46 |
| hsa-miR-302d-3p | UAAGUGCUUCCAUGUUUGAGUGU | 460 | 23 | 45 |
| hsa-miR-3943 | UAGCCCCCAGGCUUCACUUGGCG | 207 | 23 | 45 |
| hsa-miR-1286 | UGCAGGACCAAGAUGAGCCCU | 293 | 21 | 44 |
| hsa-miR-3605-5p | UGAGGAUGGAUAGCAAGGAAGCC | 189 | 23 | 44 |
| hsa-miR-505-3p | CGUCAACACUUGCUGGUUUCCU | 282 | 22 | 44 |
| hsa-miR-3615 | UCUCUCGGCUCCUCGCGGCUC | 323 | 21 | 43 |
| hsa-miR-4435 | AUGGCCAGAGCUCACACAGAGG | 230 | 22 | 43 |
| hsa-miR-598 | UACGUCAUCGUUGUCAUCGUCA | 461 | 22 | 43 |
| hsa-miR-126-5p | CAUUAUUACUUUUGGUACGCG | 462 | 21 | 42 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-4671-3p | UUAGUGCAUAGUCUUUGGUCU | 301 | 21 | 41 |
| hsa-miR-652-3p | AAUGGCGCCACUAGGGUUGUG | 442 | 21 | 41 |
| hsa-miR-3687 | CCCGGACAGGCGUUCGUGCGACGU | 190 | 24 | 40 |
| hsa-miR-4286 | ACCCCACUCCUGGUACC | 328 | 17 | 40 |
| hsa-miR-590-3p | UAAUUUUAUGUAUAAGCUAGU | 463 | 21 | 40 |
| hsa-miR-1285-3p | UCUGGGCAACAAAGUGAGACCU | 464 | 22 | 39 |
| hsa-miR-2355-5p | AUCCCCAGAUACAAUGGACAA | 593 | 21 | 38 |
| hsa-miR-550a-3p | UGUCUUACUCCCUCAGGCACAU | 283 | 22 | 38 |
| hsa-let-7d-3p | CUAUACGACCUGCUGCCUUUCU | 92 | 22 | 37 |
| hsa-miR-136-5p | ACUCCAUUUGUUUUGAUGAUGGA | 272 | 23 | 37 |
| hsa-miR-1468 | CUCCGUUUGCCUGUUUCGCUG | 296 | 21 | 37 |
| hsa-miR-3609 | CAAAGUGAUGAGUAAUACUGGCUG | 216 | 24 | 37 |
| hsa-miR-548b-5p | AAAAGUAAUUGUGGUUUUGGCC | 304 | 22 | 37 |
| hsa-miR-664-3p | UAUUCAUUUAUCCCCAGCCUACA | 287 | 23 | 37 |
| hsa-miR-99a-3p | CAAGCUCGCUUCUAUGGGUCUG | 367 | 22 | 37 |
| hsa-miR-532-3p | CCUCCCACACCCAAGGCUUGCA | 252 | 22 | 36 |
| hsa-miR-10b-5p | UACCCUGUAGAACCGAAUUUGUG | 465 | 23 | 33 |
| hsa-miR-369-5p | AGAUCGACCGUGUUAUAUUCGC | 357 | 22 | 33 |
| hsa-miR-3161 | CUGAUAAGAACAGAGGCCCAGAU | 466 | 23 | 32 |
| hsa-miR-3940-3p | CAGCCCGGAUCCCAGCCCACUU | 239 | 22 | 32 |
| hsa-miR-663b | GGUGGCCCGGCCGUGCCUGAGG | 180 | 22 | 32 |
| hsa-miR-219-2-3p | AGAAUUGUGGCUGGACAUCUGU | 467 | 22 | 31 |
| hsa-miR-2277-5p | AGCGCGGGCUGAGCGCUGCCAGUC | 735 | 24 | 31 |
| hsa-miR-4448 | GGCUCCUUGGUCUAGGGGUA | 231 | 20 | 31 |
| hsa-miR-339-5p | UCCCUGUCCUCCAGGAGCUCACG | 402 | 23 | 30 |
| hsa-miR-3613-5p | UGUUGUACUUUUUUUUUUGUUC | 469 | 22 | 30 |
| hsa-miR-4775 | UUAAUUUUUUGUUUCGGUCACU | 302 | 22 | 30 |
| hsa-miR-212-5p | ACCUUGGCUCUAGACUGCUUACU | 246 | 23 | 29 |
| hsa-miR-324-5p | CGCAUCCCCUAGGGCAUUGGUGU | 354 | 23 | 27 |
| hsa-miR-4326 | UGUUCCUCUGUCUCCCAGAC | 417 | 20 | 27 |
| hsa-miR-582-3p | UAACUGGUUGAACAACUGAACC | 470 | 22 | 27 |
| hsa-miR-34a-3p | CAAUCAGCAAGUAUACUGCCCU | 403 | 22 | 26 |
| hsa-miR-106a-5p | AAAAGUGCUUACAGUGCAGGUAG | 471 | 23 | 25 |
| hsa-miR-4745-5p | UGAGUGGGGCUCCCGGGACGGCG | 219 | 23 | 25 |
| hsa-miR-769-3p | CUGGGAUCUCCGGGGUCUUGGUU | 337 | 23 | 25 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-1268a | CGGGCGUGGUGGUGGGGG | 291 | 18 | 24 |
| hsa-miR-154-3p | AAUCAUACACGGUUGACCUAUU | 472 | 22 | 24 |
| hsa-miR-188-3p | CUCCCACAUGCAGGGUUUGCA | 200 | 21 | 24 |
| hsa-miR-29c-3p | UAGCACCAUUUGAAAUCGGUUA | 473 | 22 | 24 |
| hsa-miR-539-5p | GGAGAAAUUAUCCUUGGUGUGU | 234 | 22 | 24 |
| hsa-miR-766-3p | ACUCCAGCCCCACAGCCUCAGC | 310 | 22 | 24 |
| hsa-miR-30b-3p | CUGGGAGGUGGAUGUUUACUUC | 320 | 22 | 23 |
| hsa-miR-3177-3p | UGCACGGCACUGGGGACACGU | 275 | 21 | 23 |
| hsa-miR-191-3p | GCUGCGCUUGGAUUUCGUCCCC | 474 | 22 | 22 |
| hsa-miR-296-3p | GAGGGUUGGGUGGAGGCUCUCC | 274 | 22 | 22 |
| hsa-miR-296-5p | AGGGCCCCCCCUCAAUCCUGU | 258 | 21 | 22 |
| hsa-miR-339-3p | UGAGCGCCUCGACGACAGAGCCG | 228 | 23 | 22 |
| hsa-miR-501-5p | AAUCCUUUGUCCCUGGGUGAGA | 430 | 22 | 22 |
| hsa-miR-200b-3p | UAAUACUGCCUGGUAAUGAUGA | 475 | 22 | 21 |
| hsa-miR-212-3p | UAACAGUCUCCAGUCACGGCC | 348 | 21 | 21 |
| hsa-miR-26b-3p | CCUGUUCUCCAUUACUUGGCUC | 391 | 22 | 21 |
| hsa-miR-665 | ACCAGGAGGCUGAGGCCCCU | 309 | 20 | 21 |
| hsa-miR-668 | UGUCACUCGGCUCGGCCCACUAC | 476 | 23 | 21 |
| hsa-miR-146a-5p | UGAGAACUGAAUUCCAUGGGUU | 477 | 22 | 20 |
| hsa-miR-1973 | ACCGUGCAAAGGUAGCAUA | 171 | 19 | 20 |
| hsa-miR-210 | CUGUGCGUGUGACAGCGGCUGA | 478 | 22 | 20 |
| hsa-miR-3607-5p | GCAUGUGAUGAAGCAAAUCAGU | 249 | 22 | 20 |
| hsa-miR-378a-5p | CUCCUGACUCCAGGUCCUGUGU | 217 | 22 | 20 |
| hsa-miR-4449 | CGUCCCGGGGCUGCGCGAGGCA | 155 | 22 | 20 |
| hsa-miR-138-5p | AGCUGGUGUUGUGAAUCAGGCCG | 379 | 23 | 19 |
| hsa-miR-146b-3p | UGCCCUGUGGACUCAGUUCUGG | 381 | 22 | 18 |
| hsa-miR-3065-3p | UCAGCACCAGGAUAUUGUUGGAG | 350 | 23 | 18 |
| hsa-miR-4417 | GGUGGGCUUCCCGGAGGG | 175 | 18 | 18 |
| hsa-miR-497-5p | CAGCAGCACACUGUGGUUUGU | 479 | 21 | 18 |
| hsa-miR-500a-5p | UAAUCCUUGCUACCUGGGUGAGA | 303 | 23 | 18 |
| hsa-miR-625-3p | GACUAUAGAACUUUCCCCCUCA | 307 | 22 | 18 |
| hsa-miR-628-3p | UCUAGUAAGAGUGGCAGUCGA | 335 | 21 | 18 |
| hsa-miR-1343 | CUCCUGGGGCCCGCACUCUCGC | 378 | 22 | 17 |
| hsa-miR-3648 | AGCCGCGGGGAUCGCCGAGGG | 259 | 21 | 17 |
| hsa-miR-432-3p | CUGGAUGGCUCCUCCAUGUCU | 262 | 21 | 17 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-4482-3p | UUUCUAUUUCUCAGUGGGGCUC | 361 | 22 | 17 |
| hsa-miR-542-5p | UCGGGGAUCAUCAUGUCACGAGA | 433 | 23 | 17 |
| hsa-miR-551b-3p | GCGACCCAUACUUGGUUUCAG | 434 | 21 | 17 |
| hsa-miR-7-1-3p | CAACAAAUCACAGUCUGCCAUA | 480 | 22 | 17 |
| hsa-miR-219-1-3p | AGAGUUGAGUCUGGACGUCCCG | 390 | 22 | 16 |
| hsa-miR-3656 | GGCGGGUGCGGGGGUGG | 251 | 17 | 16 |
| hsa-miR-3661 | UGACCUGGGACUCGGACAGCUG | 481 | 22 | 16 |
| hsa-miR-411-3p | UAUGUAACACGGUCCACUAACC | 482 | 22 | 16 |
| hsa-miR-5096 | GUUUCACCAUGUUGGUCAGGC | 220 | 21 | 16 |
| hsa-miR-577 | UAGAUAAAAUAUUGGUACCUG | 436 | 21 | 16 |
| hsa-let-7i-3p | CUGCGCAAGCUACUGCCUUGCU | 483 | 22 | 15 |
| hsa-miR-132-5p | ACCGUGGCUUUCGAUUGUUACU | 315 | 22 | 15 |
| hsa-miR-140-5p | CAGUGGUUUUACCCUAUGGUAG | 380 | 22 | 15 |
| hsa-miR-195-5p | UAGCAGCACAGAAAUAUUGGC | 346 | 21 | 15 |
| hsa-miR-3187-3p | UUGGCCAUGGGGCUGCGCGG | 322 | 20 | 15 |
| hsa-miR-342-5p | AGGGGUGCUAUCUGUGAUUGA | 278 | 21 | 15 |
| hsa-miR-34b-3p | CAAUCACUAACUCCACUGCCAU | 404 | 22 | 15 |
| hsa-miR-4661-5p | AACUAGCUCUGUGGAUCCUGAC | 484 | 22 | 15 |
| hsa-miR-584-5p | UUAUGGUUUGCCUGGGACUGAG | 485 | 22 | 15 |
| hsa-miR-744-3p | CUGUUGCCACUAACCUCAACCU | 486 | 22 | 15 |
| hsa-miR-770-5p | UCCAGUACCACGUGUCAGGGCCA | 487 | 23 | 15 |
| hsa-miR-3677-3p | CUCGUGGGCUCUGGCCACGGCC | 356 | 22 | 14 |
| hsa-miR-425-3p | AUCGGGAAUGUCGUGUCCGCCC | 358 | 22 | 14 |
| hsa-miR-548ah-3p | CAAAAACUGCAGUUACUUUUGC | 149 | 22 | 14 |
| hsa-miR-5699 | UCCUGUCUUUCCUUGUUGGAGC | 488 | 22 | 14 |
| hsa-miR-582-5p | UUACAGUUGUUCAACCAGUUACU | 489 | 23 | 14 |
| hsa-miR-1185-2-3p | AUAUACAGGGGGAGACUCUCAU | 314 | 22 | 13 |
| hsa-miR-1249 | ACGCCCUUCCCCCCCUUCUUCA | 490 | 22 | 13 |
| hsa-miR-1255a | AGGAUGAGCAAAGAAAGUAGAUU | 341 | 23 | 13 |
| hsa-miR-1910 | CCAGUCCUGUGCCUGCCGCCU | 236 | 21 | 13 |
| hsa-miR-301a-5p | GCUCUGACUUUAUUGCACUACU | 491 | 22 | 13 |
| hsa-miR-5001-3p | UUCUGCCUCUGUCCAGGUCCUU | 492 | 22 | 13 |
| hsa-miR-5094 | AAUCAGUGAAUGCCUUGAACCU | 493 | 22 | 13 |
| hsa-miR-628-5p | AUGCUGACAUAUUUACUAGAGG | 440 | 22 | 13 |
| hsa-miR-629-5p | UGGGUUUACGUUGGGAGAACU | 441 | 21 | 13 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-937 | AUCCGCGCUCUGACUCUCUGCC | 312 | 22 | 13 |
| hsa-miR-940 | AAGGCAGGGCCCCCGCUCCCC | 366 | 21 | 13 |
| hsa-miR-1248 | ACCUUCUUGUAUAAGCACUGUGCUAAA | 269 | 27 | 12 |
| hsa-miR-194-5p | UGUAACAGCAACUCCAUGUGGA | 345 | 22 | 12 |
| hsa-miR-199b-3p | ACAGUAGUCUGCACAUUGGUUA | 494 | 22 | 12 |
| hsa-miR-22-5p | AGUUCUUCAGUGGCAAGCUUUA | 495 | 22 | 12 |
| hsa-miR-3605-3p | CCUCCGUGUUACCUGUCCUCUAG | 496 | 23 | 12 |
| hsa-miR-3654 | GACUGGACAAGCUGAGGAA | 325 | 19 | 12 |
| hsa-miR-504 | AGACCCUGGUCUGCACUCUAUC | 497 | 22 | 12 |
| hsa-miR-1291 | UGGCCCUGACUGAAGACCAGCAGU | 294 | 24 | 11 |
| hsa-miR-1299 | UUCUGGAAUUCUGUGUGAGGGA | 498 | 22 | 11 |
| hsa-miR-188-5p | CAUCCCUUGCAUGGUGGAGGG | 499 | 21 | 11 |
| hsa-miR-222-5p | CUCAGUAGCCAGUGUAGAUCCU | 349 | 22 | 11 |
| hsa-miR-331-5p | CUAGGUAUGGUCCCAGGGAUCC | 500 | 22 | 11 |
| hsa-miR-3939 | UACGCGCAGACCACAGGAUGUC | 261 | 22 | 11 |
| hsa-miR-154-5p | UAGGUUAUCCGUGUUGCCUUCG | 501 | 22 | 10 |
| hsa-miR-18a-3p | ACUGCCCUAAGUGCUCCUUCUGG | 502 | 23 | 10 |
| hsa-miR-1908 | CGGCGGGGACGGCGAUUGGUC | 383 | 21 | 10 |
| hsa-miR-200c-3p | UAAUACUGCCGGGUAAUGAUGGA | 347 | 23 | 10 |
| hsa-miR-2116-3p | CCUCCCAUGCCAAGAACUCCC | 318 | 21 | 10 |
| hsa-miR-302a-3p | UAAGUGCUUCCAUGUUUUGGUGA | 503 | 23 | 10 |
| hsa-miR-3174 | UAGUGAGUUAGAGAUGCAGAGCC | 353 | 23 | 10 |
| hsa-miR-326 | CCUCUGGGCCCUUCCUCCAG | 504 | 20 | 10 |
| hsa-let-7g-3p | CUGUACAGGCCACUGCCUUGC | 505 | 21 | 9 |
| hsa-miR-141-3p | UAACACUGUCUGGUAAAGAUGG | 295 | 22 | 9 |
| hsa-miR-24-1-5p | UGCCUACUGAGCUGAUAUCAGU | 506 | 22 | 9 |
| hsa-miR-3115 | AUAUGGGUUUACUAGUUGGU | 351 | 20 | 9 |
| hsa-miR-3180-3p | UGGGGCGGAGCUUCCGGAGGCC | 400 | 22 | 9 |
| hsa-miR-33a-5p | GUGCAUUGUAGUUGCAUUGCA | 355 | 21 | 9 |
| hsa-miR-34c-3p | AAUCACUAACCACACGGCCAGG | 405 | 22 | 9 |
| hsa-miR-3929 | GAGGCUGAUGUGAGUAGACCACU | 218 | 23 | 9 |
| hsa-miR-4517 | AAAUAUGAUGAAACUCACAGCUGAG | 507 | 25 | 9 |
| hsa-miR-576-3p | AAGAUGUGGAAAAAUUGGAAUC | 508 | 22 | 9 |
| hsa-miR-1229 | CUCUCACCACUGCCCUCCCACAG | 509 | 23 | 8 |
| hsa-miR-1289 | UGGAGUCCAGGAAUCUGCAUUUU | 343 | 23 | 8 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-1915-5p | ACCUUGCCUUGCUGCCCGGGCC | 385 | 22 | 8 |
| hsa-miR-23b-5p | UGGGUUCCUGGCAUGCUGAUUU | 510 | 22 | 8 |
| hsa-miR-302a-5p | ACUUAAACGUGGAUGUACUUGCU | 511 | 23 | 8 |
| hsa-miR-3938 | AAUUCCCUUGUAGAUAACCCGG | 512 | 22 | 8 |
| hsa-miR-4466 | GGGUGCGGGCCGGCGGGG | 264 | 18 | 8 |
| hsa-miR-4786-5p | UGAGACCAGGACUGGAUGCACC | 197 | 22 | 8 |
| hsa-miR-589-3p | UCAGAACAAAUGCCGGUUCCCAGA | 438 | 24 | 8 |
| hsa-miR-616-5p | ACUCAAAACCCUUCAGUGACUU | 439 | 22 | 8 |
| hsa-miR-943 | CUGACUGUUGCCGUCCUCCAG | 338 | 21 | 8 |
| hsa-miR-1237 | UCCUUCUGCUCCGUCCCCCAG | 370 | 21 | 7 |
| hsa-miR-1915-3p | CCCCAGGGCGACGCGGCGGG | 384 | 20 | 7 |
| hsa-miR-3620 | UCACCCUGCAUCCCGCACCCAG | 324 | 22 | 7 |
| hsa-miR-3691-5p | AGUGGAUGAUGGAGACUCGGUAC | 409 | 23 | 7 |
| hsa-miR-4426 | GAAGAUGGACGUACUUU | 359 | 17 | 7 |
| hsa-let-7a-2-3p | CUGUACAGCCUCCUAGCUUUCC | 513 | 22 | 6 |
| hsa-miR-10a-3p | CAAAUUCGUAUCUAGGGGAAUA | 514 | 22 | 6 |
| hsa-miR-1287 | UGCUGGAUCAGUGGUUCGAGUC | 515 | 22 | 6 |
| hsa-miR-145-5p | GUCCAGUUUUCCCAGGAAUCCCU | 516 | 23 | 6 |
| hsa-miR-29b-1-5p | GCUGGUUUCAUAUGGUGGUUUAGA | 517 | 24 | 6 |
| hsa-miR-3128 | UCUGGCAAGUAAAAAACUCUCAU | 518 | 23 | 6 |
| hsa-miR-33b-5p | GUGCAUUGCUGUUGCAUUGC | 519 | 20 | 6 |
| hsa-miR-3681-5p | UAGUGGAUGAUGCACUCUGUGC | 327 | 22 | 6 |
| hsa-miR-3685 | UUUCCUACCCUACCUGAAGACU | 520 | 22 | 6 |
| hsa-miR-3918 | ACAGGGCCGCAGAUGGAGACU | 521 | 21 | 6 |
| hsa-miR-551b-5p | GAAAUCAAGCGUGGGUGAGACC | 522 | 22 | 6 |
| hsa-miR-1273f | GGAGAUGGAGGUUGCAGUG | 292 | 19 | 5 |
| hsa-miR-1273g-3p | ACCACUGCACUCCAGCCUGAG | 210 | 21 | 5 |
| hsa-miR-1304-5p | UUUGAGGCUACAGUGAGAUGUG | 523 | 22 | 5 |
| hsa-miR-1538 | CGGCCCGGGCUGCUGCUGUUCCU | 524 | 23 | 5 |
| hsa-miR-181c-3p | AACCAUCGACCGUUGAGUGGAC | 525 | 22 | 5 |
| hsa-miR-193a-5p | UGGGUCUUUGCGGGCGAGAUGA | 526 | 22 | 5 |
| hsa-miR-208b | AUAAGACGAACAAAAGGUUUGU | 388 | 22 | 5 |
| hsa-miR-219-5p | UGAUUGUCCAAACGCAAUUCU | 527 | 21 | 5 |
| hsa-miR-3159 | UAGGAUUACAAGUGUCGGCCAC | 528 | 22 | 5 |
| hsa-miR-3173-5p | UGCCCUGCCUGUUUUCUCCUUU | 529 | 22 | 5 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-3175 | CGGGGAGAGAACGCAGUGACGU | 530 | 22 | 5 |
| hsa-miR-3200-5p | AAUCUGAGAAGGCGCACAAGGU | 531 | 22 | 5 |
| hsa-miR-3662 | GAAAAUGAUGAGUAGUGACUGAUG | 326 | 24 | 5 |
| hsa-miR-3928 | GGAGGAACCUUGGAGCUUCGGC | 413 | 22 | 5 |
| hsa-miR-4709-3p | UUGAAGAGGAGGUGCUCUGUAGC | 532 | 23 | 5 |
| hsa-miR-4787-3p | GAUGCGCCGCCCACUGCCCCGCGC | 533 | 24 | 5 |
| hsa-miR-499a-5p | UUAAGACUUGCAGUGAUGUUU | 534 | 21 | 5 |
| hsa-miR-545-3p | UCAGCAAACAUUUAUUGUGUGC | 242 | 22 | 5 |
| hsa-miR-548u | CAAAGACUGCAAUUACUUUUGCG | 535 | 23 | 5 |
| hsa-miR-659-5p | AGGACCUUCCCUGAACCAAGGA | 364 | 22 | 5 |
| hsa-miR-1257 | AGUGAAUGAUGGGUUCUGACC | 372 | 21 | 4 |
| hsa-miR-1292 | UGGGAACGGGUUCCGGCAGACGCUG | 536 | 25 | 4 |
| hsa-miR-1914-5p | CCCUGUGCCCGGCCCACUUCUG | 537 | 22 | 4 |
| hsa-miR-195-3p | CCAAUAUUGGCUGUGCUGCUCC | 538 | 22 | 4 |
| hsa-miR-2110 | UUGGGGAAACGGCCGCUGAGUG | 389 | 22 | 4 |
| hsa-miR-302c-5p | UUUAACAUGGGGGUACCUGCUG | 539 | 22 | 4 |
| hsa-miR-3126-3p | CAUCUGGCAUCCGUCACACAGA | 394 | 22 | 4 |
| hsa-miR-3126-5p | UGAGGGACAGAUGCCAGAAGCA | 352 | 22 | 4 |
| hsa-miR-3150a-5p | CAACCUCGACGAUCUCCUCAGC | 540 | 22 | 4 |
| hsa-miR-3157-3p | CUGCCCUAGUCUAGCUGAAGCU | 399 | 22 | 4 |
| hsa-miR-323b-3p | CCCAAUACACGGUCGACCUCUU | 541 | 22 | 4 |
| hsa-miR-335-3p | UUUUUCAUUAUUGCUCCUGACC | 542 | 22 | 4 |
| hsa-miR-3607-3p | ACUGUAAACGCUUUCUGAUG | 543 | 20 | 4 |
| hsa-miR-3653 | CUAAGAAGUUGACUGAAG | 544 | 18 | 4 |
| hsa-miR-3663-3p | UGAGCACCACACAGGCCGGGCGC | 545 | 23 | 4 |
| hsa-miR-376a-5p | GUAGAUUCUCCUUCUAUGAGUA | 410 | 22 | 4 |
| hsa-miR-4423-3p | AUAGGCACCAAAAAGCAACAA | 662 | 21 | 4 |
| hsa-miR-4423-5p | AGUUGCCUUUUUGUUCCCAUGC | 263 | 22 | 4 |
| hsa-miR-4463 | GAGACUGGGGUGGGGCC | 300 | 17 | 4 |
| hsa-miR-449a | UGGCAGUGUAUUGUUAGCUGGU | 547 | 22 | 4 |
| hsa-miR-4511 | GAAGAACUGUUGCAUUUGCCCU | 548 | 22 | 4 |
| hsa-miR-4640-3p | CACCCCCUGUUUCCUGGCCCAC | 329 | 22 | 4 |
| hsa-miR-4800-3p | CAUCCGUCCGUCUGUCCAC | 549 | 19 | 4 |
| hsa-miR-505-5p | GGGAGCCAGGAAGUAUUGAUGU | 550 | 22 | 4 |
| hsa-miR-548a-3p | CAAAACUGGCAAUUACUUUUGC | 551 | 22 | 4 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-570-3p | CGAAAACAGCAAUUACCUUUGC | 333 | 22 | 4 |
| hsa-miR-663a | AGGCGGGGCGCCGCGGGACCGC | 365 | 22 | 4 |
| hsa-miR-877-3p | UCCUCUUCUCCCUCCUCCCAG | 552 | 21 | 4 |
| hsa-miR-103a-2-5p | AGCUUCUUUACAGUGCUGCCUUG | 553 | 23 | 3 |
| hsa-miR-1268b | CGGGCGUGGUGGUGGGGGUG | 554 | 20 | 3 |
| hsa-miR-1270 | CUGGAGAUAUGGAAGAGCUGUGU | 555 | 23 | 3 |
| hsa-miR-1293 | UGGGUGGUCUGGAGAUUUGUGC | 556 | 22 | 3 |
| hsa-miR-1322 | GAUGAUGCUGCUGAUGCUG | 557 | 19 | 3 |
| hsa-miR-150-5p | UCUCCCAACCCUUGUACCAGUG | 558 | 22 | 3 |
| hsa-miR-190b | UGAUAUGUUUGAUAUUGGGUU | 559 | 21 | 3 |
| hsa-miR-193a-3p | AACUGGCCUACAAAGUCCCAGU | 386 | 22 | 3 |
| hsa-miR-193b-5p | CGGGGUUUUGAGGGCGAGAUGA | 560 | 22 | 3 |
| hsa-miR-199a-5p | CCCAGUGUUCAGACUACCUGUUC | 273 | 23 | 3 |
| hsa-miR-20a-3p | ACUGCAUUAUGAGCACUUAAAG | 561 | 22 | 3 |
| hsa-miR-216a | UAAUCUCAGCUGGCAACUGUGA | 562 | 22 | 3 |
| hsa-miR-2682-5p | CAGGCAGUGACUGUUCAGACGUC | 563 | 23 | 3 |
| hsa-miR-2964a-5p | AGAUGUCCAGCCACAAUUCUCG | 564 | 22 | 3 |
| hsa-miR-3177-5p | UGUGUACACACGUGCCAGGCGCU | 565 | 23 | 3 |
| hsa-miR-320c | AAAAGCUGGGUUGAGAGGGU | 566 | 20 | 3 |
| hsa-miR-323a-5p | AGGUGGUCCGUGGCGCGUUCGC | 567 | 22 | 3 |
| hsa-miR-3622a-5p | CAGGCACGGGAGCUCAGGUGAG | 568 | 22 | 3 |
| hsa-miR-3912 | UAACGCAUAAUAUGGACAUGU | 569 | 21 | 3 |
| hsa-miR-3934 | UCAGGUGUGGAAACUGAGGCAG | 570 | 22 | 3 |
| hsa-miR-3942-3p | UUUCAGAUAACAGUAUUACAU | 414 | 21 | 3 |
| hsa-miR-3942-5p | AAGCAAUACUGUUACCUGAAAU | 571 | 22 | 3 |
| hsa-miR-4523 | GACCGAGAGGGCCUCGGCUGU | 572 | 21 | 3 |
| hsa-miR-4640-5p | UGGGCCAGGGAGCAGCUGGUGGG | 573 | 23 | 3 |
| hsa-miR-4671-5p | ACCGAAGACUGUGCGCUAAUCU | 574 | 22 | 3 |
| hsa-miR-4709-5p | ACAACAGUGACUUGCUCUCCAA | 575 | 22 | 3 |
| hsa-miR-4731-3p | CACACAAGUGGCCCCCAACACU | 425 | 22 | 3 |
| hsa-miR-4731-5p | UGCUGGGGGCCACAUGAGUGUG | 576 | 22 | 3 |
| hsa-miR-4762-5p | CCAAAUCUUGAUCAGAAGCCU | 577 | 21 | 3 |
| hsa-miR-5010-5p | AGGGGGAUGGCAGAGCAAAAUU | 578 | 22 | 3 |
| hsa-miR-502-5p | AUCCUUGCUAUCUGGGUGCUA | 579 | 21 | 3 |
| hsa-miR-548d-5p | AAAAGUAAUUGUGGUUUUUGCC | 580 | 22 | 3 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-548i | AAAAGUAAUUGCGGAUUUUGCC | 581 | 22 | 3 |
| hsa-miR-548j | AAAAGUAAUUGCGGUCUUUGGU | 582 | 22 | 3 |
| hsa-miR-5587-3p | GCCCCGGGCAGUGUGAUCAUC | 284 | 21 | 3 |
| hsa-miR-1225-3p | UGAGCCCCUGUGCCGCCCCCAG | 369 | 22 | 2 |
| hsa-miR-1227 | CGUGCCACCCUUUUCCCCAG | 583 | 20 | 2 |
| hsa-miR-1252 | AGAAGGAAAUUGAAUUCAUUUA | 371 | 22 | 2 |
| hsa-miR-1280 | UCCCACCGCUGCCACCC | 584 | 17 | 2 |
| hsa-miR-1288 | UGGACUGCCCUGAUCUGGAGA | 585 | 21 | 2 |
| hsa-miR-1303 | UUUAGAGACGGGGUCUUGCUCU | 586 | 22 | 2 |
| hsa-miR-1306-3p | ACGUUGGCUCUGGUGGUG | 376 | 18 | 2 |
| hsa-miR-139-5p | UCUACAGUGCACGUGUCUCCAG | 587 | 22 | 2 |
| hsa-miR-149-3p | AGGGAGGGACGGGGGCUGUGC | 588 | 21 | 2 |
| hsa-miR-16-1-3p | CCAGUAUUAACUGUGCUGCUGA | 589 | 22 | 2 |
| hsa-miR-1909-5p | UGAGUGCCGGUGCCUGCCCUG | 590 | 21 | 2 |
| hsa-miR-224-5p | CAAGUCACUAGUGGUUCCGUU | 591 | 21 | 2 |
| hsa-miR-2276 | UCUGCAAGUGUCAGAGGCGAGG | 592 | 22 | 2 |
| hsa-miR-2355-3p | AUUGUCCUUGCUGUUUGGAGAU | 468 | 22 | 2 |
| hsa-miR-2964a-3p | AGAAUUGCGUUUGGACAAUCAGU | 392 | 23 | 2 |
| hsa-miR-29c-5p | UGACCGAUUUCUCCUGGUGUUC | 594 | 22 | 2 |
| hsa-miR-3074-3p | GAUAUCAGCUCAGUAGGCACCG | 595 | 22 | 2 |
| hsa-miR-3120-3p | CACAGCAAGUGUAGACAGGCA | 596 | 21 | 2 |
| hsa-miR-3130-5p | UACCCAGUCUCCGGUGCAGCC | 396 | 21 | 2 |
| hsa-miR-3140-3p | AGCUUUUGGGAAUUCAGGUAGU | 597 | 22 | 2 |
| hsa-miR-3155a | CCAGGCUCUGCAGUGGGAACU | 398 | 21 | 2 |
| hsa-miR-3163 | UAUAAAAUGAGGGCAGUAAGAC | 598 | 22 | 2 |
| hsa-miR-3167 | AGGAUUUCAGAAAUACUGGUGU | 599 | 22 | 2 |
| hsa-miR-363-5p | CGGGUGGAUCACGAUGCAAUUU | 600 | 22 | 2 |
| hsa-miR-3676-3p | CCGUGUUUCCCCCACGCUUU | 408 | 20 | 2 |
| hsa-miR-378g | ACUGGGCUUGGAGUCAGAAG | 411 | 20 | 2 |
| hsa-miR-4467 | UGGCGGCGGUAGUUAUGGGCUU | 360 | 22 | 2 |
| hsa-miR-4498 | UGGGCUGGCAGGGCAAGUGCUG | 601 | 22 | 2 |
| hsa-miR-4654 | UGUGGGAUCUGGAGGCAUCUGG | 420 | 22 | 2 |
| hsa-miR-4659a-3p | UUUCUUCUUAGACAUGGCAACG | 603 | 22 | 2 |
| hsa-miR-4662a-5p | UUAGCCAAUUGUCCAUCUUUAG | 604 | 22 | 2 |
| hsa-miR-4683 | UGGAGAUCCAGUGCUCGCCCGAU | 605 | 23 | 2 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-4738-3p | UGAAACUGGAGCGCCUGGAGGA | 606 | 22 | 2 |
| hsa-miR-4746-3p | AGCGGUGCUCCUGCGGGCCGA | 607 | 21 | 2 |
| hsa-miR-4748 | GAGGUUUGGGGAGGAUUUGCU | 608 | 21 | 2 |
| hsa-miR-4792 | CGGUGAGCGCUCGCUGGC | 363 | 18 | 2 |
| hsa-miR-491-5p | AGUGGGGAACCCUUCCAUGAGG | 429 | 22 | 2 |
| hsa-miR-5000-3p | UCAGGACACUUCUGAACUUGGA | 609 | 22 | 2 |
| hsa-miR-503 | UAGCAGCGGGAACAGUUCUGCAG | 610 | 23 | 2 |
| hsa-miR-5189 | UCUGGGCACAGGCGGAUGGACAGG | 611 | 24 | 2 |
| hsa-miR-548aq-3p | CAAAAACUGCAAUUACUUUUGC | 612 | 22 | 2 |
| hsa-miR-548av-3p | AAAACUGCAGUUACUUUUGC | 613 | 20 | 2 |
| hsa-miR-5584-5p | CAGGGAAAUGGGAAGAACUAGA | 332 | 22 | 2 |
| hsa-miR-5690 | UCAGCUACUACCUCUAUUAGG | 435 | 21 | 2 |
| hsa-miR-573 | CUGAAGUGAUGUGUAACUGAUCAG | 305 | 24 | 2 |
| hsa-miR-597 | UGUGUCACUCGAUGACCACUGU | 614 | 22 | 2 |
| hsa-miR-622 | ACAGUCUGCUGAGGUUGGAGC | 615 | 21 | 2 |
| hsa-miR-636 | UGUGCUUGCUCGUCCCGCCCGCA | 616 | 23 | 2 |
| hsa-miR-1193 | GGGAUGGUAGACCGGUGACGUGC | 617 | 23 | 1 |
| hsa-miR-1224-3p | CCCCACCUCCUCUCUCCUCAG | 618 | 21 | 1 |
| hsa-miR-122-5p | UGGAGUGUGACAAUGGUGUUUG | 720 | 22 | 1 |
| hsa-miR-1228-5p | GUGGGCGGGGGCAGGUGUGUG | 620 | 21 | 1 |
| hsa-miR-1244 | AAGUAGUUGGUUUGUAUGAGAUGGUU | 340 | 26 | 1 |
| hsa-miR-1247-5p | ACCCGUCCCGUUCGUCCCCGGA | 621 | 22 | 1 |
| hsa-miR-1255b-5p | CGGAUGAGCAAAGAAAGUGGUU | 622 | 22 | 1 |
| hsa-miR-1269b | CUGGACUGAGCCAUGCUACUGG | 623 | 22 | 1 |
| hsa-miR-1272 | GAUGAUGAUGGCAGCAAAUUCUGAAA | 624 | 26 | 1 |
| hsa-miR-1273c | GGCGACAAAACGAGACCCUGUC | 625 | 22 | 1 |
| hsa-miR-1273e | UUGCUUGAACCCAGGAAGUGGA | 342 | 22 | 1 |
| hsa-miR-1282 | UCGUUUGCCUUUUUCUGCUU | 626 | 20 | 1 |
| hsa-miR-1290 | UGGAUUUUUGGAUCAGGGA | 375 | 19 | 1 |
| hsa-miR-1294 | UGUGAGGUUGGCAUUGUUGUCU | 627 | 22 | 1 |
| hsa-miR-1306-5p | CCACCUCCCCUGCAAACGUCCA | 628 | 22 | 1 |
| hsa-miR-1321 | CAGGGAGGUGAAUGUGAU | 377 | 18 | 1 |
| hsa-miR-135a-5p | UAUGGCUUUUUAUUCCUAUGUGA | 629 | 23 | 1 |
| hsa-miR-137 | UUAUUGCUUAAGAAUACGCGUAG | 630 | 23 | 1 |
| hsa-miR-142-5p | CAUAAAGUAGAAAGCACUACU | 631 | 21 | 1 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-143-5p | GGUGCAGUGCUGCAUCUCUGGU | 632 | 22 | 1 |
| hsa-miR-15a-3p | CAGGCCAUAUUGUGCUGCCUCA | 633 | 22 | 1 |
| hsa-miR-186-3p | GCCCAAAGGUGAAUUUUUUGGG | 382 | 22 | 1 |
| hsa-miR-192-3p | CUGCCAAUUCCAUAGGUCACAG | 634 | 22 | 1 |
| hsa-miR-19b-1-5p | AGUUUUGCAGGUUUGCAUCCAGC | 387 | 23 | 1 |
| hsa-miR-200a-3p | UAACACUGUCUGGUAACGAUGU | 635 | 22 | 1 |
| hsa-miR-204-3p | GCUGGGAAGGCAAAGGGACGU | 636 | 21 | 1 |
| hsa-miR-214-3p | ACAGCAGGCACAGACAGGCAGU | 637 | 22 | 1 |
| hsa-miR-29a-5p | ACUGAUUUCUUUUGGUGUUCAG | 393 | 22 | 1 |
| hsa-miR-3064-5p | UCUGGCUGUUGUGGUGUGCAA | 638 | 21 | 1 |
| hsa-miR-3116 | UGCCUGGAACAUAGUAGGGACU | 639 | 22 | 1 |
| hsa-miR-3125 | UAGAGGAAGCUGUGGAGAGA | 640 | 20 | 1 |
| hsa-miR-3127-3p | UCCCCUUCUGCAGGCCUGCUGG | 641 | 22 | 1 |
| hsa-miR-3130-3p | GCUGCACCGGAGACUGGGUAA | 395 | 21 | 1 |
| hsa-miR-3140-5p | ACCUGAAUUACCAAAAGCUUU | 397 | 21 | 1 |
| hsa-miR-3157-5p | UUCAGCCAGGCUAGUGCAGUCU | 642 | 22 | 1 |
| hsa-miR-3179 | AGAAGGGGUGAAAUUUAAACGU | 643 | 22 | 1 |
| hsa-miR-3181 | AUCGGGCCCUCGGCGCCGG | 644 | 19 | 1 |
| hsa-miR-3187-5p | CCUGGGCAGCGUGUGGCUGAAGG | 645 | 23 | 1 |
| hsa-miR-3190-5p | UCUGGCCAGCUACGUCCCCA | 646 | 20 | 1 |
| hsa-miR-3198 | GUGGAGUCCUGGGGAAUGGAGA | 647 | 22 | 1 |
| hsa-miR-320b | AAAAGCUGGGUUGAGAGGGCAA | 648 | 22 | 1 |
| hsa-miR-323b-5p | AGGUUGUCCGUGGUGAGUUCGCA | 401 | 23 | 1 |
| hsa-miR-3591-5p | UUUAGUGUGAUAAUGGCGUUUGA | 649 | 23 | 1 |
| hsa-miR-3619-5p | UCAGCAGGCAGGCUGGUGCAGC | 650 | 22 | 1 |
| hsa-miR-3659 | UGAGUGUUGUCUACGAGGGCA | 651 | 21 | 1 |
| hsa-miR-3674 | AUUGUAGAACCUAAGAUUGGCC | 652 | 22 | 1 |
| hsa-miR-3679-3p | CUUCCCCCCAGUAAUCUUCAUC | 653 | 22 | 1 |
| hsa-miR-375 | UUUGUUCGUUCGGCUCGCGUGA | 654 | 22 | 1 |
| hsa-miR-378b | ACUGGACU UGGAGGCAGAA | 655 | 19 | 1 |
| hsa-miR-3908 | GAGCAAUGUAGGUAGACUGUUU | 656 | 22 | 1 |
| hsa-miR-3911 | UGUGUGGAUCCUGGAGGAGGCA | 657 | 22 | 1 |
| hsa-miR-3913-5p | UUUGGGACUGAUCUUGAUGUCU | 658 | 22 | 1 |
| hsa-miR-3917 | GCUCGGACUGAGCAGGUGGG | 659 | 20 | 1 |
| hsa-miR-3944-3p | UUCGGGCUGGCCUGCUGCUCCGG | 660 | 23 | 1 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-429 | UAAUACUGUCUGGUAAAACCGU | 661 | 22 | 1 |
| hsa-miR-4421 | ACCUGUCUGUGGAAAGGAGCUA | 718 | 22 | 1 |
| hsa-miR-4443 | UUGGAGGCGUGGGUUUU | 663 | 17 | 1 |
| hsa-miR-4459 | CCAGGAGGCGGAGGAGGUGGAG | 664 | 22 | 1 |
| hsa-miR-4473 | CUAGUGCUCUCCGUUACAAGUA | 665 | 22 | 1 |
| hsa-miR-4479 | CGCGCGGCCGUGCUCGGAGCAG | 666 | 22 | 1 |
| hsa-miR-4497 | CUCCGGGACGGCUGGGC | 232 | 17 | 1 |
| hsa-miR-4504 | UGUGACAAUAGAGAUGAACAUG | 667 | 22 | 1 |
| hsa-miR-4520b-3p | UUUGGACAGAAAACACGCAGGU | 668 | 22 | 1 |
| hsa-miR-452-5p | AACUGUUUGCAGAGGAAACUGA | 669 | 22 | 1 |
| hsa-miR-4636 | AACUCGUGUUCAAAGCCUUUAG | 670 | 22 | 1 |
| hsa-miR-4659b-3p | UUUCUUCUUAGACAUGGCAGCU | 671 | 22 | 1 |
| hsa-miR-4664-3p | CUUCCGGUCUGUGAGCCCCGUC | 672 | 22 | 1 |
| hsa-miR-4665-5p | CUGGGGGACGCGUGAGCGCGAGC | 673 | 23 | 1 |
| hsa-miR-4666a-5p | AUACAUGUCAGAUUGUAUGCC | 674 | 21 | 1 |
| hsa-miR-4673 | UCCAGGCAGGAGCCGGACUGGA | 422 | 22 | 1 |
| hsa-miR-4681 | AACGGGAAUGCAGGCUGUAUCU | 675 | 22 | 1 |
| hsa-miR-4682 | UCUGAGUUCCUGGAGCCUGGUCU | 676 | 23 | 1 |
| hsa-miR-4690-5p | GAGCAGGCGAGGCUGGGCUGAA | 677 | 22 | 1 |
| hsa-miR-4699-5p | AGAAGAUUGCAGAGUAAGUUCC | 678 | 22 | 1 |
| hsa-miR-4700-3p | CACAGGACUGACUCCUCACCCCAGUG | 424 | 26 | 1 |
| hsa-miR-4706 | AGCGGGGAGGAAGUGGGCGCUGCUU | 679 | 25 | 1 |
| hsa-miR-4721 | UGAGGGCUCCAGGUGACGGUGG | 680 | 22 | 1 |
| hsa-miR-4728-3p | CAUGCUGACCUCCCUCCUGCCCCAG | 681 | 25 | 1 |
| hsa-miR-4742-5p | UCAGGCAAAGGGAUAUUUACAGA | 682 | 23 | 1 |
| hsa-miR-4747-3p | AAGGCCCGGGCUUUCCUCCCAG | 683 | 22 | 1 |
| hsa-miR-4749-5p | UGCGGGGACAGGCCAGGGCAUC | 684 | 22 | 1 |
| hsa-miR-4755-3p | AGCCAGGCUCUGAAGGGAAAGU | 685 | 22 | 1 |
| hsa-miR-4763-5p | CGCCUGCCCAGCCCUCCUGCU | 686 | 21 | 1 |
| hsa-miR-4766-3p | AUAGCAAUUGCUCUUUUGGAA | 687 | 21 | 1 |
| hsa-miR-4781-3p | AAUGUUGGAAUCCUCGCUAGAG | 688 | 22 | 1 |
| hsa-miR-4793-3p | UCUGCACUGUGAGUUGGCUGGCU | 689 | 23 | 1 |
| hsa-miR-488-3p | UUGAAAGGCUAUUUCUUGGUC | 690 | 21 | 1 |
| hsa-miR-4999-5p | UGCUGUAUUGUCAGGUAGUGA | 691 | 21 | 1 |
| hsa-miR-5001-5p | AGGGCUGGACUCAGCGGCGGAGCU | 692 | 24 | 1 |

(continued)

| CELLS - CTX0E03 07EI | | | | |
|---|---|---|---|---|
| MIRNA | MIRNA.SEQUENCE | SEQ ID NO: | MIRNA LENGTH | READ COUNTS |
| hsa-miR-5002-5p | AAUUUGGUUUCUGAGGCACUUAGU | 693 | 24 | 1 |
| hsa-miR-5004-5p | UGAGGACAGGGCAAAUUCACGA | 694 | 22 | 1 |
| hsa-miR-5006-3p | UUUCCCUUUCCAUCCUGGCAG | 695 | 21 | 1 |
| hsa-miR-5088 | CAGGGCUCAGGGAUUGGAUGGAG | 696 | 23 | 1 |
| hsa-miR-544a | AUUCUGCAUUUUUAGCAAGUUC | 697 | 22 | 1 |
| hsa-miR-548al | AACGGCAAUGACUUUUGUACCA | 698 | 22 | 1 |
| hsa-miR-548aq-5p | GAAAGUAAUUGCUGUUUUUGCC | 699 | 22 | 1 |
| hsa-miR-548at-5p | AAAAGUUAUUGCGGUUUUGGCU | 700 | 22 | 1 |
| hsa-miR-548au-5p | AAAAGUAAUUGCGGUUUUUGC | 701 | 21 | 1 |
| hsa-miR-548b-3p | CAAGAACCUCAGUUGCUUUUGU | 702 | 22 | 1 |
| hsa-miR-556-3p | AUAUUACCAUUAGCUCAUCUUU | 703 | 22 | 1 |
| hsa-miR-5582-3p | UAAAACUUUAAGUGUGCCUAGG | 704 | 22 | 1 |
| hsa-miR-5586-3p | CAGAGUGACAAGCUGGUUAAAG | 705 | 22 | 1 |
| hsa-miR-5588-5p | ACUGGCAUUAGUGGGACUUUU | 706 | 21 | 1 |
| hsa-miR-5683 | UACAGAUGCAGAUUCUCUGACUUC | 707 | 24 | 1 |
| hsa-miR-5696 | CUCAUUUAAGUAGUCUGAUGCC | 708 | 22 | 1 |
| hsa-miR-5701 | UUAUUGUCACGUUCUGAUU | 709 | 19 | 1 |
| hsa-miR-5706 | UUCUGGAUAACAUGCUGAAGCU | 710 | 22 | 1 |
| hsa-miR-592 | UUGUGUCAAUAUGCGAUGAUGU | 711 | 22 | 1 |
| hsa-miR-603 | CACACACUGCAAUUACUUUUGC | 712 | 22 | 1 |
| hsa-miR-624-3p | CACAAGGUAUUGGUAUUACCU | 713 | 21 | 1 |
| hsa-miR-885-5p | UCCAUUACACUACCCUGCCUCU | 714 | 22 | 1 |
| hsa-miR-933 | UGUGCGCAGGGAGACCUCUCCC | 715 | 22 | 1 |

**Table 7: Microvesicles EI**

| MICROVESICLES CTX0E0307EI | | | | |
|---|---|---|---|---|
| MIRNA | MIRNA.SEQUENCE | SEQ ID NO: | MIRNA LENGTH | READ COUNTS |
| hsa-miR-1246 | AAUGGAUUUUUGGAGCAGG | 21 | 19 | 32723 |
| hsa-miR-4492 | GGGGCUGGGCGCGCGCC | 34 | 17 | 16225 |
| hsa-miR-4488 | AGGGGGCGGGCUCCGGCG | 61 | 18 | 12878 |
| hsa-miR-4532 | CCCCGGGGAGCCCGGCG | 23 | 17 | 6746 |
| hsa-miR-4508 | GCGGGGCUGGGCGCGCG | 135 | 17 | 531 |
| hsa-miR-4516 | GGGAGAAGGGUCGGGGC | 110 | 17 | 500 |
| hsa-miR-3676-5p | AGGAGAUCCUGGGUU | 280 | 15 | 357 |

(continued)

| MICROVESICLES CTX0E0307EI | | | | |
|---|---|---|---|---|
| MIRNA | MIRNA.SEQUENCE | SEQ ID NO: | MIRNA LENGTH | READ COUNTS |
| hsa-miR-4485 | UAACGGCCGCGGUACCCUAA | 67 | 20 | 44 |
| hsa-miR-4497 | CUCCGGGACGGCUGGGC | 232 | 17 | 43 |
| hsa-miR-21-5p | UAGCUUAUCAGACUGAUGUUGA | 9 | 22 | 33 |
| hsa-miR-3195 | CGCGCCGGGCCCGGGUU | 716 | 17 | 28 |
| hsa-miR-3648 | AGCCGCGGGGAUCGCCGAGGG | 259 | 21 | 26 |
| hsa-miR-663b | GGUGGCCCGGCCGUGCCUGAGG | 180 | 22 | 24 |
| hsa-miR-3656 | GGCGGGUGCGGGGGUGG | 251 | 17 | 19 |
| hsa-miR-3687 | CCCGGACAGGCGUUCGUGCGACGU | 190 | 24 | 19 |
| hsa-miR-4466 | GGGUGCGGGCCGGCGGGG | 264 | 18 | 19 |
| hsa-miR-4792 | CGGUGAGCGCUCGCUGGC | 363 | 18 | 19 |
| hsa-miR-99b-5p | CACCCGUAGAACCGACCUUGCG | 4 | 22 | 18 |
| hsa-let-7a-5p | UGAGGUAGUAGGUUGUAUAGUU | 1 | 22 | 15 |
| hsa-miR-1290 | UGGAUUUUUGGAUCAGGGA | 375 | 19 | 7 |
| hsa-miR-1291 | UGGCCCUGACUGAAGACCAGCAGU | 294 | 24 | 7 |
| hsa-miR-182-5p | UUUGGCAAUGGUAGAACUCACACU | 16 | 24 | 7 |
| hsa-miR-5096 | GUUUCACCAUGUUGGUCAGGC | 220 | 21 | 7 |
| hsa-miR-1273f | GGAGAUGGAGGUUGCAGUG | 292 | 19 | 5 |
| hsa-miR-26a-5p | UUCAAGUAAUCCAGGAUAGGCU | 12 | 22 | 5 |
| hsa-miR-4284 | GGGCUCACAUCACCCCAU | 191 | 18 | 5 |
| hsa-miR-92b-3p | UAUUGCACUCGUCCCGGCCUCC | 13 | 22 | 5 |
| hsa-let-7b-5p | UGAGGUAGUAGGUUGUGUGGUU | 28 | 22 | 4 |
| hsa-let-7c | UGAGGUAGUAGGUUGUAUGGUU | 17 | 22 | 4 |
| hsa-let-7f-5p | UGAGGUAGUAGAUUGUAUAGUU | 11 | 22 | 4 |
| hsa-miR-100-5p | AACCCGUAGAUCCGAACUUGUG | 3 | 22 | 4 |
| hsa-miR-1248 | ACCUUCUUGUAUAAGCACUGUGCUAAA | 269 | 27 | 4 |
| hsa-miR-1973 | ACCGUGCAAAGGUAGCAUA | 171 | 19 | 4 |
| hsa-miR-21-3p | CAACACCAGUCGAUGGGCUGU | 20 | 21 | 4 |
| hsa-miR-3654 | GACUGGACAAGCUGAGGAA | 325 | 19 | 4 |
| hsa-miR-92a-3p | UAUUGCACUUGUCCCGGCCUGU | 7 | 22 | 4 |
| hsa-miR-1273g-3p | ACCACUGCACUCCAGCCUGAG | 210 | 21 | 3 |
| hsa-miR-23b-3p | AUCACAUUGCCAGGGAUUACC | 59 | 21 | 3 |
| hsa-miR-3609 | CAAAGUGAUGAGUAAUACUGGCUG | 216 | 24 | 3 |
| hsa-miR-3615 | UCUCUCGGCUCCUCGCGGCUC | 323 | 21 | 3 |
| hsa-miR-3653 | CUAAGAAGUUGACUGAAG | 544 | 18 | 3 |
| hsa-miR-3960 | GGCGGCGGCGGAGGCGGGGG | 416 | 20 | 3 |

(continued)

| MICROVESICLES CTX0E0307EI | | | | |
|---|---|---|---|---|
| MIRNA | MIRNA.SEQUENCE | SEQ ID NO: | MIRNA LENGTH | READ COUNTS |
| hsa-miR-4448 | GGCUCCUUGGUCUAGGGGUA | 231 | 20 | 3 |
| hsa-let-7d-5p | AGAGGUAGUAGGUUGCAUAGUU | 92 | 22 | 2 |
| hsa-miR-16-5p | UAGCAGCACGUAAAUAUUGGCG | 29 | 22 | 2 |
| hsa-miR-181a-5p | AACAUUCAACGCUGUCGGUGAGU | 15 | 23 | 2 |
| hsa-miR-181b-5p | AACAUUCAUUGCUGUCGGUGGGU | 38 | 23 | 2 |
| hsa-miR-222-3p | AGCUACAUCUGGCUACUGGGU | 36 | 21 | 2 |
| hsa-miR-24-3p | UGGCUCAGUUCAGCAGGAACAG | 119 | 22 | 2 |
| hsa-miR-3196 | CGGGGCGGCAGGGGCCUC | 717 | 18 | 2 |
| hsa-miR-4419b | GAGGCUGAAGGAAGAUGG | 718 | 18 | 2 |
| hsa-miR-4461 | GAUUGAGACUAGUAGGGCUAGGC | 129 | 23 | 2 |
| hsa-miR-4486 | GCUGGGCGAGGCUGGCA | 719 | 17 | 2 |
| hsa-miR-663a | AGGCGGGGCGCCGCGGGACCGC | 365 | 22 | 2 |
| hsa-miR-9-5p | UCUUUGGUUAUCUAGCUGUAUGA | 58 | 23 | 2 |
| hsa-let-7i-3p | CUGCGCAAGCUACUGCCUUGCU | 483 | 22 | 1 |
| hsa-let-7i-5p | UGAGGUAGUAGUUUGUGCUGUU | 22 | 22 | 1 |
| hsa-miR-1225-5p | GUGGGUACGGCCCAGUGGGGGG | 720 | 22 | 1 |
| hsa-miR-1244 | AAGUAGUUGGUUUGUAUGAGAUGGUU | 340 | 26 | 1 |
| hsa-miR-125b-5p | UCCCUGAGACCCUAACUUGUGA | 42 | 22 | 1 |
| hsa-miR-1275 | GUGGGGGAGAGGCUGUC | 162 | 17 | 1 |
| hsa-miR-1280 | UCCCACCGCUGCCACCC | 584 | 17 | 1 |
| hsa-miR-134 | UGUGACUGGUUGACCAGAGGGG | 94 | 22 | 1 |
| hsa-miR-149-5p | UCUGGCUCCGUGUCUUCACUCCC | 121 | 23 | 1 |
| hsa-miR-191-5p | CAACGGAAUCCCAAAAGCAGCUG | 8 | 23 | 1 |
| hsa-miR-221-3p | AGCUACAUUGUCUGCUGGGUUUC | 79 | 23 | 1 |
| hsa-miR-22-3p | AAGCUGCCAGUUGAAGAACUGU | 33 | 22 | 1 |
| hsa-miR-26b-5p | UUCAAGUAAUUCAGGAUAGGU | 90 | 21 | 1 |
| hsa-miR-30c-5p | UGUAAACAUCCUACACUCUCAGC | 66 | 23 | 1 |
| hsa-miR-30d-5p | UGUAAACAUCCCCGACUGGAAG | 31 | 22 | 1 |
| hsa-miR-3182 | GCUUCUGUAGUGUAGUC | 721 | 17 | 1 |
| hsa-miR-320a | AAAAGCUGGGUUGAGAGGGCGA | 97 | 22 | 1 |
| hsa-miR-34a-5p | UGGCAGUGUCUUAGCUGGUUGU | 101 | 22 | 1 |
| hsa-miR-3607-3p | ACUGUAAACGCUUUCUGAUG | 543 | 20 | 1 |
| hsa-miR-361-5p | UUAUCAGAAUCUCCAGGGGUAC | 70 | 22 | 1 |
| hsa-miR-3652 | CGGCUGGAGGUGUGAGGA | 722 | 18 | 1 |
| hsa-miR-409-3p | GAAUGUUGCUCGGUGAACCCCU | 47 | 22 | 1 |

(continued)

| MICROVESICLES CTX0E0307EI | | | | |
|---|---|---|---|---|
| MIRNA | MIRNA.SEQUENCE | SEQ ID NO: | MIRNA LENGTH | READ COUNTS |
| hsa-miR-423-3p | AGCUCGGUCUGAGGCCCCUCAGU | 57 | 23 | 1 |
| hsa-miR-423-5p | UGAGGGGCAGAGAGCGAGACUUU | 41 | 23 | 1 |
| hsa-miR-432-5p | UCUUGGAGUAGGUCAUUGGGUGG | 95 | 23 | 1 |
| hsa-miR-4417 | GGUGGGCUUCCCGGAGGG | 175 | 18 | 1 |
| hsa-miR-4426 | GAAGAUGGACGUACUUU | 359 | 17 | 1 |
| hsa-miR-4449 | CGUCCCGGGGCUGCGCGAGGCA | 155 | 22 | 1 |
| hsa-miR-4800-3p | CAUCCGUCCGUCUGUCCAC | 549 | 19 | 1 |
| hsa-miR-484 | UCAGGCUCAGUCCCCUCCCGAU | 118 | 22 | 1 |
| hsa-miR-486-5p | UCCUGUACUGAGCUGCCCCGAG | 5 | 22 | 1 |
| hsa-miR-493-3p | UGAAGGUCUACUGUGUGCCAGG | 83 | 22 | 1 |
| hsa-miR-5095 | UUACAGGCGUGAACCACCGCG | 723 | 21 | 1 |
| hsa-miR-556-3p | AUAUUACCAUUAGCUCAUCUUU | 703 | 22 | 1 |
| hsa-miR-644b-5p | UGGGCUAAGGGAGAUGAUUGGGUA | 724 | 24 | 1 |
| hsa-miR-664-5p | ACUGGCUAGGGAAAAUGAUUGGAU | 443 | 24 | 1 |
| hsa-miR-760 | CGGCUCUGGGUCUGUGGGGA | 289 | 20 | 1 |
| hsa-miR-941 | CACCCGGCUGUGUGCACAUGUGC | 60 | 23 | 1 |
| hsa-miR-98 | UGAGGUAGUAAGUUGUAUUGUU | 10 | 22 | 1 |
| hsa-miR-99a-5p | AACCCGUAGAUCCGAUCUUGUG | 52 | 22 | 1 |

**Table 8: Exosomes EI**

| EXOSOMES CTX0E03 07EI | | | | |
|---|---|---|---|---|
| MIRNA | MIRNA.SEQUENCE | SEQ ID NO: | MIRNA LENGTH | READ COUNTS |
| hsa-miR-1246 | AAUGGAUUUUUGGAGCAGG | 21 | 19 | 83958 |
| hsa-miR-4492 | GGGGCUGGGCGCGCGCC | 34 | 17 | 22482 |
| hsa-miR-4488 | AGGGGGCGGGCUCCGGCG | 61 | 18 | 20618 |
| hsa-miR-4532 | CCCCGGGGAGCCCGGCG | 23 | 17 | 6419 |
| hsa-miR-4516 | GGGAGAAGGGUCGGGGC | 110 | 17 | 904 |
| hsa-miR-4508 | GCGGGGCUGGGCGCGCG | 135 | 17 | 723 |
| hsa-miR-3676-5p | AGGAGAUCCUGGGUU | 280 | 15 | 174 |
| hsa-miR-4485 | UAACGGCCGCGGUACCCUAA | 67 | 20 | 43 |
| hsa-miR-21-5p | UAGCUUAUCAGACUGAUGUUGA | 9 | 22 | 41 |
| hsa-miR-4497 | CUCCGGGACGGCUGGGC | 232 | 17 | 28 |
| hsa-miR-663b | GGUGGCCCGGCCGUGCCUGAGG | 180 | 22 | 26 |
| hsa-miR-4792 | CGGUGAGCGCUCGCUGGC | 363 | 18 | 24 |

(continued)

| EXOSOMES CTX0E03 07EI | | | | |
|---|---|---|---|---|
| MIRNA | MIRNA.SEQUENCE | SEQ ID NO: | MIRNA LENGTH | READ COUNTS |
| hsa-miR-4454 | GGAUCCGAGUCACGGCACCA | 299 | 20 | 22 |
| hsa-miR-1291 | UGGCCCUGACUGAAGACCAGCAGU | 294 | 24 | 17 |
| hsa-miR-26a-5p | UUCAAGUAAUCCAGGAUAGGCU | 12 | 22 | 17 |
| hsa-miR-3195 | CGCGCCGGGCCCGGGUU | 716 | 17 | 17 |
| hsa-let-7a-5p | UGAGGUAGUAGGUUGUAUAGUU | 1 | 22 | 15 |
| hsa-miR-182-5p | UUUGGCAAUGGUAGAACUCACACU | 16 | 24 | 15 |
| hsa-miR-99b-5p | CACCCGUAGAACCGACCUUGCG | 4 | 22 | 15 |
| hsa-miR-5096 | GUUUCACCAUGUUGGUCAGGC | 220 | 21 | 14 |
| hsa-miR-3648 | AGCCGCGGGGAUCGCCGAGGG | 259 | 21 | 13 |
| hsa-miR-3654 | GACUGGACAAGCUGAGGAA | 325 | 19 | 13 |
| hsa-miR-4466 | GGGUGCGGGCCGGCGGGG | 264 | 18 | 12 |
| hsa-miR-3687 | CCCGGACAGGCGUUCGUGCGACGU | 190 | 24 | 11 |
| hsa-miR-4284 | GGGCUCACAUCACCCCAU | 191 | 18 | 11 |
| hsa-miR-3656 | GGCGGGUGCGGGGGUGG | 251 | 17 | 10 |
| hsa-miR-3609 | CAAAGUGAUGAGUAAUACUGGCUG | 216 | 24 | 8 |
| hsa-miR-644b-5p | UGGGCUAAGGGAGAUGAUUGGGUA | 724 | 24 | 8 |
| hsa-miR-664-5p | ACUGGCUAGGGAAAAUGAUUGGAU | 443 | 24 | 8 |
| hsa-miR-92a-3p | UAUUGCACUUGUCCCGGCCUGU | 7 | 22 | 7 |
| hsa-miR-92b-3p | UAUUGCACUCGUCCCGGCCUCC | 13 | 22 | 7 |
| hsa-let-7b-5p | UGAGGUAGUAGGUUGUGUGGUU | 28 | 22 | 6 |
| hsa-let-7f-5p | UGAGGUAGUAGAUUGUAUAGUU | 11 | 22 | 6 |
| hsa-miR-127-3p | UCGGAUCCGUCUGAGCUUGGCU | 14 | 22 | 6 |
| hsa-miR-1290 | UGGAUUUUUGGAUCAGGGA | 375 | 19 | 6 |
| hsa-miR-4449 | CGUCCCGGGGCUGCGCGAGGCA | 155 | 22 | 6 |
| hsa-miR-4461 | GAUUGAGACUAGUAGGGCUAGGC | 129 | 23 | 6 |
| hsa-miR-100-5p | AACCCGUAGAUCCGAACUUGUG | 3 | 22 | 5 |
| hsa-miR-1248 | ACCUUCUUGUAUAAGCACUGUGCUAAA | 269 | 27 | 5 |
| hsa-miR-1973 | ACCGUGCAAAGGUAGCAUA | 171 | 19 | 5 |
| hsa-miR-3653 | CUAAGAAGUUGACUGAAG | 544 | 18 | 5 |
| hsa-miR-4417 | GGUGGGCUUCCCGGAGGG | 175 | 18 | 5 |
| hsa-miR-125b-5p | UCCCUGAGACCCUAACUUGUGA | 42 | 22 | 4 |
| hsa-miR-151a-3p | CUAGACUGAAGCUCCUUGAGG | 25 | 21 | 4 |
| hsa-miR-16-5p | UAGCAGCACGUAAAUAUUGGCG | 29 | 22 | 4 |
| hsa-miR-21-3p | CAACACCAGUCGAUGGGCUGU | 20 | 21 | 4 |
| hsa-miR-23a-3p | AUCACAUUGCCAGGGAUUUCC | 55 | 21 | 4 |

(continued)

| EXOSOMES CTX0E03 07EI | | | | |
|---|---|---|---|---|
| MIRNA | MIRNA.SEQUENCE | SEQ ID NO: | MIRNA LENGTH | READ COUNTS |
| hsa-miR-4419b | GAGGCUGAAGGAAGAUGG | 718 | 18 | 4 |
| hsa-miR-1273f | GGAGAUGGAGGUUGCAGUG | 292 | 19 | 3 |
| hsa-miR-1273g-3p | ACCACUGCACUCCAGCCUGAG | 210 | 21 | 3 |
| hsa-miR-181b-5p | AACAUUCAUUGCUGUCGGUGGGU | 38 | 23 | 3 |
| hsa-miR-221-3p | AGCUACAUUGUCUGCUGGGUUUC | 79 | 23 | 3 |
| hsa-miR-3615 | UCUCUCGGCUCCUCGCGGCUC | 323 | 21 | 3 |
| hsa-miR-9-5p | UCUUUGGUUAUCUAGCUGUAUGA | 58 | 23 | 3 |
| hsa-let-7c | UGAGGUAGUAGGUUGUAUGGUU | 17 | 22 | 2 |
| hsa-let-7e-5p | UGAGGUAGGAGGUUGUAUAGUU | 27 | 22 | 2 |
| hsa-let-7i-5p | UGAGGUAGUAGUUUGUGCUGUU | 22 | 22 | 2 |
| hsa-miR-103a-3p | AGCAGCAUUGUACAGGGCUAUGA | 62 | 23 | 2 |
| hsa-miR-106b-5p | UAAAGUGCUGACAGUGCAGAU | 170 | 21 | 2 |
| hsa-miR-1273e | UUGCUUGAACCCAGGAAGUGGA | 342 | 22 | 2 |
| hsa-miR-221-5p | ACCUGGCAUACAAUGUAGAUUU | 39 | 22 | 2 |
| hsa-miR-222-3p | AGCUACAUCUGGCUACUGGGU | 36 | 21 | 2 |
| hsa-miR-30d-5p | UGUAAACAUCCCCGACUGGAAG | 31 | 22 | 2 |
| hsa-miR-3960 | GGCGGCGGCGGAGGCGGGGG | 416 | 20 | 2 |
| hsa-let-7d-3p | CUAUACGACCUGCUGCCUUUCU | 92 | 22 | 1 |
| hsa-let-7d-5p | AGAGGUAGUAGGUUGCAUAGUU | 53 | 22 | 1 |
| hsa-let-7g-5p | UGAGGUAGUAGUUUGUACAGUU | 43 | 22 | 1 |
| hsa-let-7i-3p | CUGCGCAAGCUACUGCCUUGCU | 483 | 22 | 1 |
| hsa-miR-10a-5p | UACCCUGUAGAUCCGAAUUUGUG | 2 | 23 | 1 |
| hsa-miR-1181 | CCGUCGCCGCCACCCGAGCCG | 725 | 21 | 1 |
| hsa-miR-1225-3p | UGAGCCCCUGUGCCGCCCCCAG | 369 | 22 | 1 |
| hsa-miR-1244 | AAGUAGUUGGUUUGUAUGAGAUGGUU | 340 | 26 | 1 |
| hsa-miR-125a-5p | UCCCUGAGACCCUUUAACCUGUGA | 35 | 24 | 1 |
| hsa-miR-1296 | UUAGGGCCCUGGCUCCAUCUCC | 271 | 22 | 1 |
| hsa-miR-1307-5p | UCGACCGGACCUCGACCGGCU | 91 | 21 | 1 |
| hsa-miR-146b-5p | UGAGAACUGAAUUCCAUAGGCU | 19 | 22 | 1 |
| hsa-miR-149-5p | UCUGGCUCCGUGUCUUCACUCCC | 121 | 23 | 1 |
| hsa-miR-151a-5p | UCGAGGAGCUCACAGUCUAGU | 37 | 21 | 1 |
| hsa-miR-15b-5p | UAGCAGCACAUCAUGGUUUACA | 78 | 22 | 1 |
| hsa-miR-181a-2-3p | ACCACUGACCGUUGACUGUACC | 102 | 22 | 1 |
| hsa-miR-181a-5p | AACAUUCAACGCUGUCGGUGAGU | 15 | 23 | 1 |
| hsa-miR-191-5p | CAACGGAAUCCCAAAAGCAGCUG | 8 | 23 | 1 |

(continued)

| EXOSOMES CTX0E03 07EI | | | | |
|---|---|---|---|---|
| MIRNA | MIRNA.SEQUENCE | SEQ ID NO: | MIRNA LENGTH | READ COUNTS |
| hsa-miR-198 | GGUCCAGAGGGGAGAUAGGUUC | 726 | 22 | 1 |
| hsa-miR-204-5p | UUCCCUUUGUCAUCCUAUGCCU | 89 | 22 | 1 |
| hsa-miR-20a-5p | UAAAGUGCUUAUAGUGCAGGUAG | 146 | 23 | 1 |
| hsa-miR-219-5p | UGAUUGUCCAAACGCAAUUCU | 527 | 21 | 1 |
| hsa-miR-22-3p | AAGCUGCCAGUUGAAGAACUGU | 33 | 22 | 1 |
| hsa-miR-23b-3p | AUCACAUUGCCAGGGAUUACC | 59 | 21 | 1 |
| hsa-miR-26b-3p | CCUGUUCUCCAUUACUUGGCUC | 391 | 22 | 1 |
| hsa-miR-299-5p | UGGUUUACCGUCCCACAUACAU | 319 | 22 | 1 |
| hsa-miR-29a-3p | UAGCACCAUCUGAAAUCGGUUA | 106 | 22 | 1 |
| hsa-miR-30e-3p | CUUUCAGUCGGAUGUUUACAGC | 71 | 22 | 1 |
| hsa-miR-31-3p | UGCUAUGCCAACAUAUUGCCAU | 172 | 22 | 1 |
| hsa-miR-3198 | GUGGAGUCCUGGGGAAUGGAGA | 647 | 22 | 1 |
| hsa-miR-323a-3p | CACAUUACACGGUCGACCUCU | 158 | 21 | 1 |
| hsa-miR-342-3p | UCUCACACAGAAAUCGCACCCGU | 81 | 23 | 1 |
| hsa-miR-3607-3p | ACUGUAAACGCUUUCUGAUG | 543 | 20 | 1 |
| hsa-miR-3651 | CAUAGCCCGGUCGCUGGUACAUGA | 727 | 24 | 1 |
| hsa-miR-378a-3p | ACUGGACUUGGAGUCAGAAGG | 65 | 21 | 1 |
| hsa-miR-379-5p | UGGUAGACUAUGGAACGUAGG | 18 | 21 | 1 |
| hsa-miR-423-3p | AGCUCGGUCUGAGGCCCCUCAGU | 57 | 23 | 1 |
| hsa-miR-423-5p | UGAGGGGCAGAGAGCGAGACUUU | 41 | 23 | 1 |
| hsa-miR-425-5p | AAUGACACGAUCACUCCCGUUGA | 111 | 23 | 1 |
| hsa-miR-4258 | CCCCGCCACCGCCUUGG | 728 | 17 | 1 |
| hsa-miR-4426 | GAAGAUGGACGUACUUU | 359 | 17 | 1 |
| hsa-miR-4443 | UUGGAGGCGUGGGUUUU | 663 | 17 | 1 |
| hsa-miR-4448 | GGCUCCUUGGUCUAGGGGUA | 231 | 20 | 1 |
| hsa-miR-4697-3p | UGUCAGUGACUCCUGCCCCUUGGU | 729 | 24 | 1 |
| hsa-miR-4700-3p | CACAGGACUGACUCCUCACCCCAGUG | 424 | 26 | 1 |
| hsa-miR-4700-5p | UCUGGGGAUGAGGACAGUGUGU | 730 | 22 | 1 |
| hsa-miR-4797-3p | UCUCAGUAAGUGGCACUCUGU | 731 | 21 | 1 |
| hsa-miR-484 | UCAGGCUCAGUCCCCUCCCGAU | 118 | 22 | 1 |
| hsa-miR-486-5p | UCCUGUACUGAGCUGCCCCGAG | 5 | 22 | 1 |
| hsa-miR-494 | UGAAACAUACACGGGAAACCUC | 240 | 22 | 1 |
| hsa-miR-500a-5p | UAAUCCUUGCUACCUGGGUGAGA | 303 | 23 | 1 |
| hsa-miR-644b-3p | UUCAUUUGCCUCCCAGCCUACA | 442 | 22 | 1 |
| hsa-miR-663a | AGGCGGGGCGCCGCGGGACCGC | 365 | 22 | 1 |

**Table 9: Microvesicles EH**

| **MICROVESICLES CTX0E03 07EH** | | | | |
|---|---|---|---|---|
| MIRNA | MIRNA.SEQUENCE | SEQ ID NO: | MIRNA LENGTH | READ COUNTS |
| hsa-miR-1246 | AAUGGAUUUUUGGAGCAGG | 21 | 19 | 78791 |
| hsa-miR-4492 | GGGGCUGGGCGCGCGCC | 34 | 17 | 6012 |
| hsa-miR-4532 | CCCCGGGGAGCCCGGCG | 23 | 17 | 3410 |
| hsa-miR-4488 | AGGGGGCGGGCUCCGGCG | 61 | 18 | 1737 |
| hsa-miR-4485 | UAACGGCCGCGGUACCCUAA | 67 | 20 | 319 |
| hsa-miR-4508 | GCGGGGCUGGGCGCGCG | 135 | 17 | 221 |
| hsa-miR-4516 | GGGAGAAGGGUCGGGGC | 110 | 17 | 114 |
| hsa-miR-4466 | GGGUGCGGGCCGGCGGGG | 264 | 18 | 61 |
| hsa-miR-4497 | CUCCGGGACGGCUGGGC | 232 | 17 | 51 |
| hsa-miR-3195 | CGCGCCGGGCCCGGGUU | 716 | 17 | 41 |
| hsa-miR-1973 | ACCGUGCAAAGGUAGCAUA | 171 | 19 | 30 |
| hsa-miR-21-5p | UAGCUUAUCAGACUGAUGUUGA | 9 | 22 | 22 |
| hsa-miR-4284 | GGGCUCACAUCACCCCAU | 191 | 18 | 20 |
| hsa-miR-4792 | CGGUGAGCGCUCGCUGGC | 363 | 18 | 12 |
| hsa-miR-92a-3p | UAUUGCACUUGUCCCGGCCUGU | 7 | 22 | 12 |
| hsa-miR-1291 | UGGCCCUGACUGAAGACCAGCAGU | 294 | 24 | 11 |
| hsa-miR-3676-5p | AGGAGAUCCUGGGUU | 280 | 15 | 10 |
| hsa-miR-100-5p | AACCCGUAGAUCCGAACUUGUG | 3 | 22 | 8 |
| hsa-miR-3656 | GGCGGGUGCGGGGGUGG | 251 | 17 | 8 |
| hsa-miR-663b | GGUGGCCCGGCCGUGCCUGAGG | 180 | 22 | 8 |
| hsa-let-7a-5p | UGAGGUAGUAGGUUGUAUAGUU | 1 | 22 | 7 |
| hsa-miR-1290 | UGGAUUUUUGGAUCAGGGA | 375 | 19 | 7 |
| hsa-miR-3687 | CCCGGACAGGCGUUCGUGCGACGU | 190 | 24 | 7 |
| hsa-miR-4461 | GAUUGAGACUAGUAGGGCUAGGC | 52 | 23 | 6 |
| hsa-miR-664-5p | ACUGGCUAGGGAAAAUGAUUGGAU | 91 | 24 | 6 |
| hsa-miR-92b-3p | UAUUGCACUCGUCCCGGCCUCC | 13 | 22 | 6 |
| hsa-miR-125b-5p | UCCCUGAGACCCUAACUUGUGA | 42 | 22 | 5 |
| hsa-miR-3653 | CUAAGAAGUUGACUGAAG | 544 | 18 | 5 |
| hsa-let-7f-5p | UGAGGUAGUAGAUUGUAUAGUU | 11 | 22 | 4 |
| hsa-miR-16-5p | UAGCAGCACGUAAAUAUUGGCG | 29 | 22 | 4 |
| hsa-miR-181a-5p | AACAUUCAACGCUGUCGGUGAGU | 15 | 23 | 4 |
| hsa-miR-3609 | CAAAGUGAUGAGUAAUACUGGCUG | 216 | 24 | 4 |
| hsa-miR-9-5p | UCUUUGGUUAUCUAGCUGUAUGA | 58 | 23 | 4 |
| hsa-let-7c | UGAGGUAGUAGGUUGUAUGGUU | 17 | 22 | 3 |
| hsa-miR-1244 | AAGUAGUUGGUUUGUAUGAGAUGGUU | 59 | 26 | 3 |

(continued)

| **MICROVESICLES CTX0E03 07EH** | | | | |
|---|---|---|---|---|
| MIRNA | MIRNA.SEQUENCE | SEQ ID NO: | MIRNA LENGTH | READ COUNTS |
| hsa-miR-127-3p | UCGGAUCCGUCUGAGCUUGGCU | 14 | 22 | 3 |
| hsa-miR-181b-5p | AACAUUCAUUGCUGUCGGUGGGU | 38 | 23 | 3 |
| hsa-miR-21-3p | CAACACCAGUCGAUGGGCUGU | 20 | 21 | 3 |
| hsa-miR-26a-5p | UUCAAGUAAUCCAGGAUAGGCU | 12 | 22 | 3 |
| hsa-miR-30c-5p | UGUAAACAUCCUACACUCUCAGC | 66 | 23 | 3 |
| hsa-miR-3960 | GGCGGCGGCGGAGGCGGGGG | 416 | 20 | 3 |
| hsa-miR-485-3p | GUCAUACACGGCUCUCCUCUCU | 153 | 22 | 3 |
| hsa-let-7b-5p | UGAGGUAGUAGGUUGUGUGGUU | 28 | 22 | 2 |
| hsa-let-7g-5p | UGAGGUAGUAGUUUGUACAGUU | 43 | 22 | 2 |
| hsa-miR-1273f | GGAGAUGGAGGUUGCAGUG | 292 | 19 | 2 |
| hsa-miR-151a-3p | CUAGACUGAAGCUCCUUGAGG | 25 | 21 | 2 |
| hsa-miR-182-5p | UUUGGCAAUGGUAGAACUCACACU | 16 | 24 | 2 |
| hsa-miR-191-5p | CAACGGAAUCCCAAAAGCAGCUG | 8 | 23 | 2 |
| hsa-miR-197-3p | UUCACCACCUUCUCCACCCAGC | 122 | 22 | 2 |
| hsa-miR-423-5p | UGAGGGGCAGAGAGCGAGACUUU | 41 | 23 | 2 |
| hsa-miR-4468 | AGAGCAGAAGGAUGAGAU | 732 | 18 | 2 |
| hsa-miR-644b-5p | UGGGCUAAGGGAGAUGAUUGGGUA | 724 | 24 | 2 |
| hsa-miR-93-5p | CAAAGUGCUGUUCGUGCAGGUAG | 116 | 23 | 2 |
| hsa-let-7d-5p | AGAGGUAGUAGGUUGCAUAGUU | 92 | 22 | 1 |
| hsa-miR-1225-3p | UGAGCCCCUGUGCCGCCCCCAG | 369 | 22 | 1 |
| hsa-miR-1254 | AGCCUGGAAGCUGGAGCCUGCAGU | 270 | 24 | 1 |
| hsa-miR-1273g-3p | ACCACUGCACUCCAGCCUGAG | 210 | 21 | 1 |
| hsa-miR-1275 | GUGGGGGAGAGGCUGUC | 162 | 17 | 1 |
| hsa-miR-1296 | UUAGGGCCCUGGCUCCAUCUCC | 271 | 22 | 1 |
| hsa-miR-1307-5p | UCGACCGGACCUCGACCGGCU | 91 | 21 | 1 |
| hsa-miR-134 | UGUGACUGGUUGACCAGAGGGG | 94 | 22 | 1 |
| hsa-miR-15b-5p | UAGCAGCACAUCAUGGUUUACA | 78 | 22 | 1 |
| hsa-miR-17-5p | CAAAGUGCUUACAGUGCAGGUAG | 145 | 23 | 1 |
| hsa-miR-1972 | UCAGGCCAGGCACAGUGGCUCA | 733 | 22 | 1 |
| hsa-miR-22-3p | AAGCUGCCAGUUGAAGAACUGU | 33 | 22 | 1 |
| hsa-miR-25-3p | CAUUGCACUUGUCUCGGUCUGA | 63 | 22 | 1 |
| hsa-miR-27b-3p | UUCACAGUGGCUAAGUUCUGC | 6 | 21 | 1 |
| hsa-miR-3065-5p | UCAACAAAAUCACUGAUGCUGGA | 226 | 23 | 1 |
| hsa-miR-30d-5p | UGUAAACAUCCCCGACUGGAAG | 31 | 22 | 1 |
| hsa-miR-320a | AAAAGCUGGGUUGAGAGGGCGA | 97 | 22 | 1 |

(continued)

| MICROVESICLES CTX0E03 07EH | | | | |
|---|---|---|---|---|
| MIRNA | MIRNA.SEQUENCE | SEQ ID NO: | MIRNA LENGTH | READ COUNTS |
| hsa-miR-342-3p | UCUCACACAGAAAUCGCACCCGU | 81 | 23 | 1 |
| hsa-miR-3648 | AGCCGCGGGGAUCGCCGAGGG | 259 | 21 | 1 |
| hsa-miR-3652 | CGGCUGGAGGUGUGAGGA | 722 | 18 | 1 |
| hsa-miR-376c | AACAUAGAGGAAAUUCCACGU | 185 | 21 | 1 |
| hsa-miR-378a-3p | ACUGGACUUGGAGUCAGAAGG | 65 | 21 | 1 |
| hsa-miR-409-3p | GAAUGUUGCUCGGUGAACCCCU | 47 | 22 | 1 |
| hsa-miR-433 | AUCAUGAUGGGCUCCUCGGUGU | 174 | 22 | 1 |
| hsa-miR-4417 | GGUGGGCUUCCCGGAGGG | 175 | 18 | 1 |
| hsa-miR-4448 | GGCUCCUUGGUCUAGGGGUA | 231 | 20 | 1 |
| hsa-miR-4454 | GGAUCCGAGUCACGGCACCA | 299 | 20 | 1 |
| hsa-miR-454-3p | UAGUGCAAUAUUGCUUAUAGGGU | 169 | 23 | 1 |
| hsa-miR-4800-3p | CAUCCGUCCGUCUGUCCAC | 549 | 19 | 1 |
| hsa-miR-493-3p | UGAAGGUCUACUGUGUGCCAGG | 83 | 22 | 1 |
| hsa-miR-5095 | UUACAGGCGUGAACCACCGCG | 723 | 21 | 1 |
| hsa-miR-574-3p | CACGCUCAUGCACACACCCACA | 253 | 22 | 1 |
| hsa-miR-665 | ACCAGGAGGCUGAGGCCCCU | 309 | 20 | 1 |
| hsa-miR-720 | UCUCGCUGGGGCCUCCA | 84 | 17 | 1 |
| hsa-miR-99a-5p | AACCCGUAGAUCCGAUCUUGUG | 52 | 22 | 1 |
| hsa-miR-99b-5p | CACCCGUAGAACCGACCUUGCG | 4 | 22 | 1 |

**Table 10: Exosomes EH**

| EXOSOMES CTX0E03 07EH | | | | |
|---|---|---|---|---|
| MIRNA | MIRNA.SEQUENCE | SEQ ID NO: | MIRNA LENGTH | READ COUNTS |
| hsa-miR-1246 | AAUGGAUUUUUGGAGCAGG | 21 | 19 | 111092 |
| hsa-miR-4492 | GGGGCUGGGCGCGCGCC | 34 | 17 | 5188 |
| hsa-miR-4532 | CCCCGGGGAGCCCGGCG | 23 | 17 | 3368 |
| hsa-miR-4488 | AGGGGGCGGGCUCCGGCG | 61 | 18 | 1389 |
| hsa-miR-4485 | UAACGGCCGCGGUACCCUAA | 67 | 20 | 386 |
| hsa-miR-4508 | GCGGGGCUGGGCGCGCG | 135 | 17 | 188 |
| hsa-miR-4516 | GGGAGAAGGGUCGGGGC | 110 | 17 | 135 |
| hsa-miR-4497 | CUCCGGGACGGCUGGGC | 232 | 17 | 73 |
| hsa-miR-1973 | ACCGUGCAAAGGUAGCAUA | 171 | 19 | 50 |
| hsa-miR-3195 | CGCGCCGGGCCCGGGUU | 716 | 17 | 48 |
| hsa-miR-4466 | GGGUGCGGGCCGGCGGGG | 264 | 18 | 43 |

(continued)

| EXOSOMES CTX0E03 07EH | | | | |
|---|---|---|---|---|
| MIRNA | MIRNA.SEQUENCE | SEQ ID NO: | MIRNA LENGTH | READ COUNTS |
| hsa-let-7a-5p | UGAGGUAGUAGGUUGUAUAGUU | 1 | 22 | 20 |
| hsa-miR-99b-5p | CACCCGUAGAACCGACCUUGCG | 4 | 22 | 19 |
| hsa-miR-21-5p | UAGCUUAUCAGACUGAUGUUGA | 9 | 22 | 18 |
| hsa-miR-92a-3p | UAUUGCACUUGUCCCGGCCUGU | 7 | 22 | 18 |
| hsa-miR-3676-5p | AGGAGAUCCUGGGUU | 280 | 15 | 17 |
| hsa-miR-4792 | CGGUGAGCGCUCGCUGGC | 363 | 18 | 15 |
| hsa-miR-664-5p | ACUGGCUAGGGAAAAUGAUUGGAU | 443 | 24 | 13 |
| hsa-miR-100-5p | AACCCGUAGAUCCGAACUUGUG | 3 | 22 | 11 |
| hsa-miR-1291 | UGGCCCUGACUGAAGACCAGCAGU | 294 | 24 | 10 |
| hsa-miR-16-5p | UAGCAGCACGUAAAUAUUGGCG | 29 | 22 | 10 |
| hsa-miR-4284 | GGGCUCACAUCACCCCAU | 191 | 18 | 10 |
| hsa-miR-663b | GGUGGCCCGGCCGUGCCUGAGG | 180 | 22 | 9 |
| hsa-miR-25-3p | CAUUGCACUUGUCUCGGUCUGA | 63 | 22 | 8 |
| hsa-miR-3656 | GGCGGGUGCGGGGGUGG | 251 | 17 | 8 |
| hsa-miR-181a-5p | AACAUUCAACGCUGUCGGUGAGU | 15 | 23 | 7 |
| hsa-miR-26a-5p | UUCAAGUAAUCCAGGAUAGGCU | 12 | 22 | 6 |
| hsa-miR-3654 | GACUGGACAAGCUGAGGAA | 325 | 19 | 6 |
| hsa-miR-644b-5p | UGGGCUAAGGGAGAUGAUUGGGUA | 724 | 24 | 6 |
| hsa-let-7b-5p | UGAGGUAGUAGGUUGUGUGGUU | 28 | 22 | 5 |
| hsa-let-7f-5p | UGAGGUAGUAGAUUGUAUAGUU | 11 | 22 | 5 |
| hsa-miR-1290 | UGGAUUUUUGGAUCAGGGA | 375 | 19 | 5 |
| hsa-miR-4426 | GAAGAUGGACGUACUUU | 359 | 17 | 5 |
| hsa-miR-5096 | GUUUCACCAUGUUGGUCAGGC | 220 | 21 | 5 |
| hsa-miR-125b-5p | UCCCUGAGACCCUAACUUGUGA | 42 | 22 | 4 |
| hsa-miR-1273f | GGAGAUGGAGGUUGCAGUG | 292 | 19 | 4 |
| hsa-miR-191-5p | CAACGGAAUCCCAAAAGCAGCUG | 8 | 23 | 4 |
| hsa-miR-22-3p | AAGCUGCCAGUUGAAGAACUGU | 33 | 22 | 4 |
| hsa-miR-3609 | CAAAGUGAUGAGUAAUACUGGCUG | 216 | 24 | 4 |
| hsa-miR-3687 | CCCGGACAGGCGUUCGUGCGACGU | 190 | 24 | 4 |
| hsa-miR-93-5p | CAAAGUGCUGUUCGUGCAGGUAG | 116 | 23 | 4 |
| hsa-miR-1248 | ACCUUCUUGUAUAAGCACUGUGCUAAA | 269 | 27 | 3 |
| hsa-miR-1273g-3p | ACCACUGCACUCCAGCCUGAG | 210 | 21 | 3 |
| hsa-miR-151a-3p | CUAGACUGAAGCUCCUUGAGG | 25 | 21 | 3 |
| hsa-miR-182-5p | UUUGGCAAUGGUAGAACUCACACU | 16 | 24 | 3 |
| hsa-miR-221-3p | AGCUACAUUGUCUGCUGGGUUUC | 79 | 23 | 3 |

(continued)

| EXOSOMES CTX0E03 07EH | | | | |
|---|---|---|---|---|
| MIRNA | MIRNA.SEQUENCE | SEQ ID NO: | MIRNA LENGTH | READ COUNTS |
| hsa-miR-222-3p | AGCUACAUCUGGCUACUGGGU | 36 | 21 | 3 |
| hsa-miR-29a-3p | UAGCACCAUCUGAAAUCGGUUA | 106 | 22 | 3 |
| hsa-miR-4461 | GAUUGAGACUAGUAGGGCUAGGC | 129 | 23 | 3 |
| hsa-miR-486-5p | UCCUGUACUGAGCUGCCCCGAG | 5 | 22 | 3 |
| hsa-miR-92b-3p | UAUUGCACUCGUCCCGGCCUCC | 13 | 22 | 3 |
| hsa-miR-9-5p | UCUUUGGUUAUCUAGCUGUAUGA | 58 | 23 | 3 |
| hsa-miR-98 | UGAGGUAGUAAGUUGUAUUGUU | 10 | 22 | 3 |
| hsa-let-7d-5p | AGAGGUAGUAGGUUGCAUAGUU | 53 | 22 | 2 |
| hsa-miR-134 | UGUGACUGGUUGACCAGAGGGG | 94 | 22 | 2 |
| hsa-miR-151a-5p | UCGAGGAGCUCACAGUCUAGU | 37 | 21 | 2 |
| hsa-miR-15b-5p | UAGCAGCACAUCAUGGUUUACA | 78 | 22 | 2 |
| hsa-miR-30a-5p | UGUAAACAUCCUCGACUGGAAG | 30 | 22 | 2 |
| hsa-miR-3124-3p | ACUUUCCUCACUCCCGUGAAGU | 734 | 22 | 2 |
| hsa-miR-3653 | CUAAGAAGUUGACUGAAG | 544 | 18 | 2 |
| hsa-let-7c | UGAGGUAGUAGGUUGUAUGGUU | 17 | 22 | 1 |
| hsa-let-7d-3p | CUAUACGACCUGCUGCCUUUCU | 92 | 22 | 1 |
| hsa-let-7g-5p | UGAGGUAGUAGUUUGUACAGUU | 43 | 22 | 1 |
| hsa-let-7i-5p | UGAGGUAGUAGUUUGUGCUGUU | 22 | 22 | 1 |
| hsa-miR-103a-3p | AGCAGCAUUGUACAGGGCUAUGA | 62 | 23 | 1 |
| hsa-miR-106b-5p | UAAAGUGCUGACAGUGCAGAU | 170 | 21 | 1 |
| hsa-miR-1244 | AAGUAGUUGGUUUGUAUGAGAUGGUU | 340 | 26 | 1 |
| hsa-miR-128 | UCACAGUGAACCGGUCUCUUU | 109 | 21 | 1 |
| hsa-miR-1285-3p | UCUGGGCAACAAAGUGAGACCU | 464 | 22 | 1 |
| hsa-miR-1307-3p | ACUCGGCGUGGCGUCGGUCGUG | 124 | 22 | 1 |
| hsa-miR-140-3p | UACCACAGGGUAGAACCACGG | 138 | 21 | 1 |
| hsa-miR-148b-3p | UCAGUGCAUCACAGAACUUUGU | 48 | 22 | 1 |
| hsa-miR-181b-5p | AACAUUCAUUGCUGUCGGUGGGU | 38 | 23 | 1 |
| hsa-miR-193a-3p | AACUGGCCUACAAAGUCCCAGU | 386 | 22 | 1 |
| hsa-miR-1972 | UCAGGCCAGGCACAGUGGCUCA | 733 | 22 | 1 |
| hsa-miR-21-3p | CAACACCAGUCGAUGGGCUGU | 20 | 21 | 1 |
| hsa-miR-2277-3p | UGACAGCGCCCUGCCUGGCUC | 735 | 21 | 1 |
| hsa-miR-23a-3p | AUCACAUUGCCAGGGAUUUCC | 55 | 21 | 1 |
| hsa-miR-23b-3p | AUCACAUUGCCAGGGAUUACC | 59 | 21 | 1 |
| hsa-miR-24-3p | UGGCUCAGUUCAGCAGGAACAG | 119 | 22 | 1 |
| hsa-miR-27a-3p | UUCACAGUGGCUAAGUUCCGC | 46 | 21 | 1 |

(continued)

| **EXOSOMES CTX0E03 07EH** | | | | |
|---|---|---|---|---|
| MIRNA | MIRNA.SEQUENCE | SEQ ID NO: | MIRNA LENGTH | READ COUNTS |
| hsa-miR-27b-3p | UUCACAGUGGCUAAGUUCUGC | 6 | 21 | 1 |
| hsa-miR-299-3p | UAUGUGGGAUGGUAAACCGCUU | 182 | 22 | 1 |
| hsa-miR-30b-5p | UGUAAACAUCCUACACUCAGCU | 96 | 22 | 1 |
| hsa-miR-30c-5p | UGUAAACAUCCUACACUCUCAGC | 66 | 23 | 1 |
| hsa-miR-31-3p | UGCUAUGCCAACAUAUUGCCAU | 172 | 22 | 1 |
| hsa-miR-3196 | CGGGGCGGCAGGGGCCUC | 717 | 18 | 1 |
| hsa-miR-3198 | GUGGAGUCCUGGGGAAUGGAGA | 647 | 22 | 1 |
| hsa-miR-320a | AAAAGCUGGGUUGAGAGGGCGA | 97 | 22 | 1 |
| hsa-miR-329 | AACACACCUGGUUAACCUCUUU | 214 | 22 | 1 |
| hsa-miR-339-5p | UCCCUGUCCUCCAGGAGCUCACG | 402 | 23 | 1 |
| hsa-miR-34a-5p | UGGCAGUGUCUUAGCUGGUUGU | 101 | 22 | 1 |
| hsa-miR-3607-5p | GCAUGUGAUGAAGCAAAUCAGU | 249 | 22 | 1 |
| hsa-miR-3648 | AGCCGCGGGGAUCGCCGAGGG | 259 | 21 | 1 |
| hsa-miR-376c | AACAUAGAGGAAAUUCCACGU | 185 | 21 | 1 |
| hsa-miR-3960 | GGCGGCGGCGGAGGCGGGGG | 416 | 20 | 1 |
| hsa-miR-411-3p | UAUGUAACACGGUCCACUAACC | 482 | 22 | 1 |
| hsa-miR-423-3p | AGCUCGGUCUGAGGCCCCUCAGU | 57 | 23 | 1 |
| hsa-miR-423-5p | UGAGGGGCAGAGAGCGAGACUUU | 41 | 23 | 1 |
| hsa-miR-4417 | GGUGGGCUUCCCGGAGGG | 175 | 18 | 1 |
| hsa-miR-4444 | CUCGAGUUGGAAGAGGCG | 418 | 18 | 1 |
| hsa-miR-4499 | AAGACUGAGAGGAGGGA | 736 | 17 | 1 |
| hsa-miR-4521 | GCUAAGGAAGUCCUGUGCUCAG | 233 | 22 | 1 |
| hsa-miR-4680-5p | AGAACUCUUGCAGUCUUAGAUGU | 737 | 23 | 1 |
| hsa-miR-4709-5p | ACAACAGUGACUUGCUCUCCAA | 575 | 22 | 1 |
| hsa-miR-501-3p | AAUGCACCCGGGCAAGGAUUCU | 26 | 22 | 1 |
| hsa-miR-644b-3p | UUCAUUUGCCUCCCAGCCUACA | 442 | 22 | 1 |
| hsa-miR-654-3p | UAUGUCUGCUGACCAUCACCUU | 336 | 22 | 1 |
| hsa-miR-9-3p | AUAAAGCUAGAUAACCGAAAGU | 183 | 22 | 1 |
| hsa-miR-940 | AAGGCAGGGCCCCCGCUCCCC | 366 | 21 | 1 |
| hsa-miR-99a-5p | AACCCGUAGAUCCGAUCUUGUG | 52 | 22 | 1 |

***Identification of top ranking coding and non-coding RNAs by GENCODE analysis performed in exosomes, MV and producer cells***

**Table 11:** Total number of sequence reads identified by using GENCODE in each tested samples

| CTX0E0307EH cells | CTX0E0307EH EXO | CTX0E0307EH MV | CTX0E0307EI cells | CTX0E0307EIEXO | CTX0E0307EI MV |
|---|---|---|---|---|---|
| 18741941 | 12678688 | 10876797 | 22116110 | 16311289 | 835970 |

Using GENCODE database analysis of the sequence results, seven putative novel miRNA sequences were identified in exosomes (EXO), microvesicles (MV) and producer cells, as shown in Table 12. (nb CTX0E03 07EI MV reads are misrepresented due to the lower amount of starting material - see Table 11). These data are shown graphically in Figure 16, which shows that these sequences are preferentially shuttled into exosomes and microvesicles compared to the cells.

| Gene Symbol | Transcript ID | Length | Type of RNA | CTX0E0307E H cells | CTX0E0307E H EXO | CTX0E0307E H MV | CTX0E0307E I cells | CTX0E0307E IEXO | CTX0E0307EI MV |
|---|---|---|---|---|---|---|---|---|---|
| AC079949.1 | AC079949.1-201 | 57 | Novel miRNA | 2629 | 27006 | 14873 | 2425 | 11433 | 848 |
| AP000318.1 | AP000318.1-201 | 64 | Novel miRNA | 1353 | 9379 | 11002 | 7469 | 2963 | 419 |
| AL161626.1 | AL161626.1-201 | 57 | Novel miRNA | 471 | 4450 | 3712 | 291 | 1263 | 129 |
| AC004943.1 | AC004943.1-201 | 81 | Novel miRNA | 24 | 81 | 43 | 23 | 94 | 5 |
| AL121897.1 | AL121897.1-201 | 89 | Novel miRNA | 6 | 22 | 14 | 2 | 30 | 3 |

**Table 12:** Identification of putative novel miRNA sequences using GENCODE in exosomes (EXO), microvesicles (MV) and producer cells. CTX0E03 07EI MV reads are misrepresented due to the lower amount of starting material (table 11). The transcript IDs are taken from the Ensembl database (www.ensembl.org).

***Validation and of novel miRNAs***

**AC079949.1-201 (SEQ ID NO:738)**
**Gene: AC079949.1 ENSG00000239776**
>12 dna:chromosome chromosome:GRCh37:12:127650616:127650672:1
GGCCGCGCCCCGTTTCCCAGGACAAAGGGCACTCCGCACCGGACCCTGGTCCCAGCG

For AC079949.1-201 putative mature miRNA, **gaccaggguccggugcggagug (SEQ ID NO:745)** was identified as the possible 5' stem mature miRNA using http://mirna.imbb.forth.gr/MatureBayes.html, a tool for finding mature miRNA within a miRNA precursor sequence using a Naive Bays classifier. Its presence validation was performed using AGGGTCCGGTGCGGAGT (SEQ ID NO:746) primer sequence. This sequence was entered in mirbase (http://www.mirbase.org/) and the following miRNA was found with similar sequence: *Bos taurus* miR-2887-1 (Accession No. MIMAT0013845).

bta-miR-2887 : 9-20 (SEQ ID NO:747)

```
AC079949 (5)        2  ggguccggugcg  13
                       ||||||||||||
bta-miR-2887        9  ggguccggugcg  20
```

The presence of this novel miRNA was tested by qRT-PCR on purified exosomes retro transcribed miRNA.

The same analysis was performed using the 3' stem of AC079949, sequence TGCGGAGTGCCCTTTGTCCT (SEQ ID NO:748), but in this case no similar miRNA was identified in mirbase.

**AP000318.1-201 (SEQ ID NO:739)**
**Gene: AP000318.1 ENSG00000266007**
>21 dna:chromosome chromosome:GRCh37:21:35677430:35677493:1
CCCACTCCCTGGCGCCGCTTGTGGAGGGCCCAAGTCCTTCTGATTGAGGCCCAACCCGTGGAAG

For AP000318.1-201 putative mature miRNA, **ggagggcccaaguccuucugau** (SEQ ID NO:744) was identified as the possible 5' stem mature miRNA. Its presence validation was performed using GGAGGGCCCAAGTCCTTCTGAT (SEQ ID NO:749) primer sequence. *Caenorhabditis remanei* miR-55 stem-loop was identified as similar miRNA. Primer validation was again carried out by qRT-PCR.
crm-miR-55-5p : 4-17 (SEQ ID NO:750)

```
AP000318.1          20  cccaaguccuucug  7
                        ||||||| ||||||
crm-miR-55-5p        4  cccaagugcuucug  17
```

**AL161626.1-201 (SEQ ID NO:740)**
**Gene: AL161626.1 ENSG00000241781**
>9 dna:chromosome chromosome:GRCh37:9:79186731:79186787:1
CGCCGGGACCGGGGTCCGGGGCGGAGTGCCCTTCCTCCTGGGAAACGGGGTGCGGC

For AL161626.1-201 putative mature miRNA, **ggcggagugcccuucuuccugg (SEQ ID NO:743)** was identified as the possible 5' stem mature miRNA. Its presence validation was performed using CGGAGTGCCCTTCTTCCT (SEQ ID NO:751) primer sequence. *Zea mays* miR164c stem-loop and *Achypodium distachyon* miR164f stem-loop were identified as similar miRNA. Primer validation was again carried out by qRT-PCR.
zma-miR164c-3p : 4-15 (SEQ ID NO:752)

```
AL161626.1          5  gugcccuucuuc  16
                       ||||||||||||
zma-miR164c-3p      4  gugcccuucuuc  15
```

**AC004943.1 (SEQ ID NO:741)**
**Gene: AC004943.1 ENSG00000265573**
>16 dna:chromosome chromosome:GRCh37:16:72821592:72821672:-1

GCTTCACGTCCCCACCGGCGGCGGCGGCGGTGGCAGTGGCGGCGGCGGCGGCGGTGGCGG
CGGCGGCGGCGGCGGCGGCTC

**AL121897.1 (SEQ ID NO:742)**
**Gene: AL121897.1 ENSG00000264308**
>20 dna:chromosome chromosome:GRCh37:20:30865503:30865591:1

GCCGCCCCCGCCGCCGCCGCCGCCGCCGCCGCCGCCGCCGCCGCCCGCTTTCGGCTCGGG
CCTCAGGTGAGTCGGAGGGGCCGGGCGCC

***Miscellaneous RNA (misc_RNA), including novel putative***

Misc_RNA is short for miscellaneous RNA, a general term for a series of miscellaneous small RNA. Miscellaneous transcript feature are not defined by other RNA keys.

List of top ranking previously known and novel misc_RNAs identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| RPPH1 | RPPH1-201 | 333 | misc RNA | 76 | 2229 | 1785 | 0 | 1077 | 197 |
| RMRP | RMRP-201 | 264 | misc RNA | 139 | 1803 | 1443 | 191 | 659 | 87 |
| RPPH1 | RPPH1-001 | 638 | misc RNA | 182 | 931 | 1372 | 795 | 2017 | 157 |
| VTRNA1-1 | VTRNA1-1-201 | 99 | misc RNA | 43 | 720 | 52 | 247 | 210 | 9 |
| Y_RNA | Y_RNA.321-201 | 93 | Novel misc RN | 159 | 196 | 661 | 960 | 903 | 217 |
| Y_RNA | Y_RNA.725-201 | 95 | Novel misc RN | 1092 | 18 | 74 | 1005 | 39 | 11 |
| Y_RNA | Y_RNA.125-201 | 96 | Novel misc RN | 1079 | 15 | 58 | 906 | 27 | 12 |
| Y_RNA | Y_RNA.118-201 | 99 | Novel misc RN | 134 | 12 | 9 | 156 | 45 | 7 |
| Y_RNA | Y_RNA.394-201 | 109 | Novel misc RN | 9 | 9 | 7 | 33 | 13 | 1 |
| Y_RNA | Y_RNA.687-201 | 111 | Novel misc RN | 36 | 6 | 15 | 103 | 41 | 10 |
| Y_RNA | Y_RNA.144-201 | 102 | Novel misc RN | 129 | 5 | 21 | 187 | 84 | 5 |
| Y_RNA | Y_RNA.337-201 | 105 | Novel misc RN | 7 | 4 | 0 | 15 | 4 | 0 |
| Y_RNA | Y_RNA.413-201 | 97 | Novel misc RN | 136 | 4 | 8 | 125 | 46 | 3 |
| Y_RNA | Y_RNA.30-201 | 103 | Novel misc RN | 74 | 3 | 3 | 62 | 21 | 2 |

**Table 13**: Identification of misc_RNA, including putative novel misc_RNA, sequences using GENCODE in exosomes (EXO), microvesicles (MV) and producer cells. (CTX0E03 07EI MV reads are misrepresented due to the lower amount of starting material - Table 11). The transcript IDs are taken from the Ensembl database (www.ensembl.org).

Among the misc_RNA the following sequences were found preferentially down or up shuttled in exosomes and MV: RPHI, RMRP, and VTRNA1-1 up shuttled and Y_RNA.725-201, and Y_RNA.125-201 down respectively. RPHI is a ribonuclease P RNA component H1. RMRP gene encodes the RNA component of mitochondrial RNA processing endoribonuclease, which cleaves mitochondrial RNA at a priming site of mitochondrial DNA replication. This RNA also interacts with the telomerase reverse transcriptase catalytic subunit to form a distinct ribonucleoprotein complex that has RNA-dependent RNA polymerase activity and produces double-stranded RNAs that can be processed into small interfering RNA. VTRNA1-1 is vault RNA component 1. Vaults are large cytoplasmic ribonucleoproteins and they are composed of a major vault protein, MVP, 2 minor vault proteins, TEP1 and PARP4, and a non-translated RNA component, VTRNA1-1. Y_RNA.725-201, and Y_RNA.125-201 are novel misc_RNAs and their function is not defined.

***Metazoa miscellaneous RNA***

The signal recognition particle RNA, also known as 7SL, 6S, ffs, or 4.5S RNA, is the RNA component of the signal recognition particle (SRP) ribonucleoprotein complex. SRP is a universally conserved ribonucleoprotein that directs the traffic of proteins within the cell and allows them to be secreted. The SRP RNA, together with one or more SRP proteins contributes to the binding and release of the signal peptide. The RNA and protein components of this complex are highly conserved but do vary between the different kingdoms of life.

List of top ranking Metazoa misc_RNAs identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| Metazoa_SRP | Metazoa_SRP.791-201 | 288 | Metazoan signal recogn | 679 | 2324 | 2058 | 771 | 2698 | 465 |
| Metazoa_SRP | Metazoa_SRP.561-201 | 294 | Metazoan signal recogn | 634 | 2006 | 1683 | 744 | 2147 | 432 |
| Metazoa_SRP | Metazoa_SRP.864-201 | 297 | Metazoan signal recogn | 252 | 1884 | 1544 | 78 | 170 | 148 |
| Metazoa_SRP | Metazoa_SRP.824-201 | 297 | Metazoan signal recogn | 438 | 881 | 958 | 505 | 1860 | 342 |
| Metazoa_SRP | Metazoa_SRP.72-201 | 278 | Metazoan signal recogn | 441 | 630 | 631 | 494 | 2184 | 349 |
| Metazoa_SRP | Metazoa_SRP.151-201 | 307 | Metazoan signal recogn | 377 | 464 | 470 | 432 | 1431 | 265 |
| Metazoa_SRP | Metazoa_SRP.208-201 | 277 | Metazoan signal recogn | 382 | 410 | 431 | 422 | 1104 | 242 |
| Metazoa_SRP | Metazoa_SRP.501-201 | 280 | Metazoan signal recogn | 265 | 272 | 266 | 236 | 434 | 44 |
| Metazoa_SRP | Metazoa_SRP.682-201 | 298 | Metazoan signal recogn | 12 | 52 | 21 | 10 | 13 | 2 |

**Table 14:** Identification signal recognition particle RNA (misc_RNA) sequences using GENCODE in exosomes (EXO), microvesicles (MV) and producer cells. The transcript IDs are taken from the Ensembl database (www.ensembl.org).

### *RRNA (ribosomal RNA)*

Ribosomal RNA (rRNA) forms part of the protein-synthesizing organelle known as a ribosome and that is exported to the cytoplasm to help translate the information in messenger RNA (mRNA) into protein. Eukaryotic ribosome (80S) rRNA components are: large unit (rRNA 5S, 5.8S, and 28S) small unit (rRNA 18S). Both rRNA 28S and 5.8S are selectively up-shuttled in exosomes and MV.

List of top ranking rRNA identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| RNA5-8SP6 | RNA5-8SP6-201 | 152 | rRNA | 205008 | 1148190 | 706558 | 213187 | 135909 | 14732 |
| RNA28S5 | RNA28S5-001 | 432 | rRNA | 86111 | 458585 | 516754 | 62829 | 390237 | 47483 |
| RNA18S5 | RNA18S5-001 | 599 | rRNA | 74634 | 52055 | 61639 | 116874 | 138484 | 14616 |
| RNA5-8SP2 | RNA5-8SP2-201 | 152 | rRNA | 6488 | 1719 | 1540 | 9231 | 3112 | 149 |
| RNA5-8SP5 | RNA5-8SP5-201 | 152 | rRNA | 2794 | 7393 | 3924 | 7314 | 3579 | 232 |

**Table 15**: Identification rRNA sequences using GENCODE in exosomes (EXO), microvesicles (MV) and producer cells. The transcript IDs are taken from the Ensembl database (www.ensembl.org).

### *Small nucleolar RNA: snoRNA*

Small nucleolar RNAs (snoRNAs) are a class of small RNA molecules that primarily guides chemical modifications of other RNAs, mainly ribosomal RNAs, transfer RNAs and small nuclear RNAs. There are two main classes of snoRNA, the C/D box snoRNAs which are associated with methylation, and the H/ACA box snoRNAs which are associated with pseudouridylation.

List of top ranking snoRNA identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| SNORD3A | SNORD3A-201 | 216 | snoRNA | 1433 | 2085 | 1621 | 906 | 1732 | 120 |
| SNORD3C | SNORD3C-201 | 216 | snoRNA | 1169 | 1702 | 1220 | 639 | 1176 | 86 |
| SNORD29 | SNORD29-201 | 65 | snoRNA | 28130 | 1633 | 1070 | 36677 | 1752 | 45 |
| SNORD83B | SNORD83B-201 | 93 | snoRNA | 1835 | 675 | 487 | 638 | 575 | 29 |
| SNORD30 | SNORD30-201 | 70 | snoRNA | 29743 | 254 | 244 | 29071 | 283 | 24 |

**Table 16:** Identification of snoRNA sequences using GENCODE in exosomes (EXO), microvesicles (MV) and producer cells. The transcript IDs are taken from the Ensembl database (www.ensembl.org).

### *Small nuclear RNA (snRNA)*

Small nuclear ribonucleic acid (snRNA), also commonly referred to as U-RNA, is a class of small RNA molecules that make up the major spliceosome are named U1, U2, U4, U5, and U6, and participate in several RNA-RNA and RNA-protein interactions. Their primary function is in the processing of pre-mRNA (hnRNA) in the nucleus. They have also been shown to aide in the regulation of transcription factors (7SK RNA) or RNA polymerase II (B2 RNA), and maintaining the telomeres.

List of top ranking snRNA identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| U2 | U2.38-201 | 191 | snRNA | 1354 | 71596 | 49223 | 751 | 35290 | 1919 |
| U2 | U2.6-201 | 192 | snRNA | 834 | 15561 | 13594 | 303 | 8146 | 272 |
| U1 | U1.81-201 | 164 | snRNA | 584 | 10901 | 7307 | 91 | 3197 | 121 |
| U1 | U1.90-201 | 167 | snRNA | 533 | 9927 | 6689 | 48 | 2187 | 84 |
| U2 | U2.7-201 | 191 | snRNA | 201 | 9267 | 3109 | 288 | 6736 | 262 |

**Table 17A:** Identification of snRNA sequences using GENCODE in exosomes (EXO), microvesicles (MV) and producer cells. The transcript IDs are taken from the Ensembl database (www.ensembl.org).

***LincRNA and novel lincRNA***

Large intergenic non-coding RNAs (lincRNAs) are emerging as key regulators of diverse cellular processes. Determining the function of individual lincRNAs remains a challenge. Long non-coding RNAs (long ncRNAs, IncRNA) are non-protein coding transcripts longer than 200 nucleotides.

List of top ranking previously known and novel lincRNAs identified using GENCODE sequence data set:

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| RP11-108M9.3 | RP11-108M9.3-0( | 1761 | Novel lincRNA | 244 | 159 | 240 | 539 | 324 | 45 |
| RP11-329L6.1 | RP11-329L6.1-001 | 507 | Novel lincRNA | 19 | 70 | 41 | 29 | 84 | 2 |
| RP11-160E2.6 | RP11-160E2.6-00 | 637 | Novel lincRNA | 228 | 67 | 115 | 489 | 74 | 6 |
| AC004528.3 | AC004528.3-001 | 107 | Novel lincRNA | 16 | 58 | 46 | 14 | 55 | 4 |
| MALAT1 | MALAT1-201 | 4585 | lincRNA | 150 | 308 | 234 | 26 | 182 | 12 |
| GAS5 | GAS5-007 | 2743 | lincRNA | 12024 | 215 | 120 | 46501 | 875 | 13 |

**Table 17B:** Identification of lincRNA and putative novel lincRNA sequences using GENCODE in exosomes (EXO), microvesicles (MV) and producer cells. The transcript IDs are taken from the Ensembl database (www.ensembl.org).

GAS5 lincRNA is highly expressed in cell producer compared to in exosomes and microvesicles (down shuttled in both exosomes and MV).

***mRNA***

Coding sequencing mRNA were also identified.

| Gene Symbol | Transcript ID | Length | Type of RNA | CTX0E0307EH cells | CTX0E0307EH EXO | CTX0E0307EH MV | CTX0E0307EI cells | CTX0E0307EI EXO | CTX0E0307EI MV |
|---|---|---|---|---|---|---|---|---|---|
| EEF2 | EEF2-201 | 9407 | mRNA | 710 | 578 | 449 | 1155 | 471 | 33 |
| MTRNR2L8 | MTRNR2L8-201 | 1290 | mRNA | 1383 | 548 | 642 | 1323 | 258 | 15 |
| NES | NES-001 | 8635 | mRNA | 668 | 406 | 234 | 1448 | 267 | 20 |
| VIM | VIM-001 | 8316 | mRNA | 563 | 911 | 501 | 1500 | 618 | 36 |

**Table 18:** Identification of mRNA sequences using GENCODE in exosomes (EXO), microvesicles (MV) and producer cells. The transcript IDs are taken from the Ensembl database (www.ensembl.org).

***Example 17A-C: Conclusion***

The main scope of the deep sequence analysis was to identify their miRNA components in neural stem cell-derived vesicles (exosomes and microvesicles). This analysis identified a new set of known and novel miRNAs that are preferentially shuttled into both exosomes and MV. Among the identified miRNAs already included in mirbase database were hsa-miR-1246, hsa-miR-4488, hsa-miR-4492, hsa-miR-4508, hsa-miR-4516, hsa-miR-4532, and among the novel miRNAs were AC079949.1, AP000318.1, AL161626.1, AC004943.1, AL121897.1. Top ranking shuttled miRNAs, including novel ones were validated by qRT-PCR in exosomes.

The size distribution of shuttle RNA, as shown here, is mostly in the range of 20 to 200 nt and other RNA species are released by cells into the extracellular space. By deep sequencing and GENCODE sequence set analysis we found a greater complexity and diversity of non-coding RNA transcripts. We extended this analysis with detailed evaluation and this led to the discovery of preferentially up (defined as log2 fold change ≥ 2) and down (defined as log2 fold change ≤-2) shuttle of other non-coding RNAs in both exosomes and microvesicles. Differentially shuttled non coding RNA were found in almost all the non-coding RNA subtypes, ribosomal RNA (rRNA), small nucleolar (snoRNA), small nuclear RNA (snRNA), microRNA (miRNA), miscellaneous other RNA (misc_RNA, e.g. RMRP, vault RNA, metazoa SRP, and RNY), and large intergenic non-coding RNAs (lincRNAs).

The unequal distribution of the detected RNA species over cellular and shuttle RNA, combined with increasing evidence for their role in gene regulation strongly suggest that cells specifically release these RNAs to modify the function of target cells.

### *D) Deep sequencing of CTX0E03 cell and exosome miRNA expression from* 6 *week bioreactor culture*

Next generation deep sequencing was also carried out on CTX0E03 cells and their derived exosomes, following culture for six weeks in an Integra bioreactor. The results showed that hsa-miR-1246, hsa-miR-4492, hsa-miR-4532, and hsa-miR-4488 are also up-shuttled in exosomes derived from 6 week Integra culture (6W). In EXO 6W a total of 61 miRNA types are up-shuttled. Up-shuttled miRNAs with more than 250 reads per exosome sample are listed in Figure 13E.

### *Conclusions*

Hsa-miR-1246, hsa-miR-4492, hsa-miR-4532, and hsa-miR-4488 are still up-shuttled in EXO 6W as observed on proliferative EXO (07EI & EH; Figure 13 A&B). New up-shuttled miRNAs are also identified, including hsa-miR-4792.

20.53 % of the identified miRNA are up-shuttled in the exosomes derived from 6 week Integra CTX cultures (shown in Figure 13C, middle panel). This compares to 99% of the identified miRNAs that are up-shuttled in the exosomes derived from proliferative CTX0E03 cultures (Figure 13C, top panel).

### *E) Deep sequencing of CTX0E03 cell and exosome miRNA expression from 11 week bioreactor culture*

Next generation deep sequencing was also carried out on CTX0E03 cells and their derived exosomes, following culture for 11 weeks in an Integra bioreactor. Three samples were tested.

In sample 1, 9 miRNA species are up-shuttled, all of which have more than 250 reads, as shown in Figure 13F.

In sample 2, 68 miRNA species are up-shuttled into the exosomes. The miRNAs with more than 250 reads per exosome sample are shown in Figure 13G.

In sample 3, 47 miRNA species are up-shuttled. Figure 13H shows the three miRNA species with a read count >250: hsa-miR-10b-5p, hsa-miR-1246 and hsa-miR-486-5p.

### *Conclusions*

**Table E1:** W11 summary table of reads and log2 values of miRNA types previously reported as up-shuttled in proliferative CTX0E03 exosomes.

| | Reads | | | | | | Log2 | | |
|---|---|---|---|---|---|---|---|---|---|
| MiRNA | Cell1 | Cell2 | Cell3 | EXO1 | EXO2 | EXO3 | EXO1 | EXO2 | EXO3 |
| **hsa-miR-4488** | 0 | 0 | 1 | 0 | 0 | 0 | N/A | N/A | N/A |
| **hsa-miR-4492** | 0 | 1 | 0 | 1 | 0 | 0 | N/A | N/A | N/A |

(continued)

| | Reads | | | | | | Log2 | | |
|---|---|---|---|---|---|---|---|---|---|
| MiRNA | Cell1 | Cell2 | Cell3 | EXO1 | EXO2 | EXO3 | EXO1 | EXO2 | EXO3 |
| **hsa-miR-4532** | 0 | 0 | 0 | 0 | 0 | 0 | N/A | N/A | N/A |
| **hsa-miR-1246** | 483 | 1122 | 3470 | 18 | 2726 | 24152 | -4.20 | 1.26 | 2.99 |

Hsa-miR-1246 is present in 11W exosomes, but was only observed to be up-shuttled in EXO3.

Hsa-miR-4488, hsa-miR-4492, and hsa-miR-4532, identified in proliferative CTX0E03 cells and their exosomes, are almost absent in 11 week samples (both cells and exosomes).

Hsa-miR-486-5p was the only miRNA up-shuttled in all three EXO W11 samples.

An average 12.22% of the identified miRNAs are up-shuttled in the exosomes derived from 11 week Integra CTX0E03 cultures (Figure 13C, lower panel).

Comparative summary tables

*Comparative analysis of miRNA expression in EXO samples sorted by largest reads in EXO derived from proliferative CTX0E03/07EH*

| | Reads | | | | | | LOG2 | | | |
|---|---|---|---|---|---|---|---|---|---|---|
| MiRNA | EXO 07EH | EXO 07EI | EXO WK6 | EXO1 WK11 | EXO2 WK11 | EXO3 WK11 | EXO WK6 | EXO1 WK11 | EXO2 WK11 | EXO3 WK11 |
| hsa-miR-1246 | 111092 | 83958 | 77678 | 18 | 2726 | 24152 | -1.40 | -13.43 | -9.32 | -5.96 |
| hsa-miR-4492 | 5188 | 22482 | 9528 | 1 | | | -2.65 | -15.82 | | |
| hsa-miR-4532 | 3368 | 6419 | 3463 | | | | -2.39 | | | |
| hsa-miR-4488 | 1389 | 20618 | 11048 | | | | -2.12 | | | |
| hsa-miR-4485 | 386 | 43 | 13 | | 11 | 69 | -5.46 | | -8.80 | -5.93 |
| hsa-miR-4508 | 188 | 723 | 1245 | | | 20 | -0.68 | | | -9.51 |
| hsa-miR-4516 | 135 | 904 | 1153 | | | | -0.96 | | | |
| hsa-miR-4497 | 73 | 28 | 61 | | | | -1.64 | | | |
| hsa-miR-1973 | 50 | 5 | 1 | | | | -7.05 | | | |
| hsa-miR-3195 | 48 | 17 | 14 | | | | -3.40 | | | |
| hsa-miR-4466 | 43 | 12 | 15 | | | 9 | -2.38 | | | -5.99 |
| hsa-let-7a-5p | 20 | 15 | 18306 | 69521 | 496463 | 509272 | 7.80 | 9.77 | 9.47 | 9.72 |
| hsa-miR-99b-5p | 19 | 15 | 25938 | 6984 | 56360 | 50611 | 8.35 | 6.50 | 6.37 | 6.44 |
| hsa-miR-21-5p | 18 | 41 | 35754 | 12191 | 134432 | 150516 | 8.01 | 6.50 | 6.82 | 7.21 |
| hsa-miR-92a-3p | 18 | 7 | 15054 | 5504 | 89826 | 73306 | 8.00 | 6.59 | 7.48 | 7.41 |
| hsa-miR-3676-5p | 17 | 174 | 59 | | | | -2.88 | | | |
| hsa-miR-4792 | 15 | 24 | 254 | | | | 1.54 | | | |
| hsa-miR-664-5p | 13 | 8 | 1 | | | | -5.53 | | | |
| hsa-miR-100-5p | 11 | 5 | 30124 | 22 | 56751 | 43080 | 9.68 | -0.69 | 7.50 | 7.32 |
| hsa-miR-1291 | 10 | 17 | 18 | | | 34 | -1.83 | | | -3.79 |
| hsa-miR-16-5p | 10 | 4 | 5502 | 15 | 6580 | 9510 | 7.32 | -1.15 | 4.48 | 5.24 |
| hsa-miR-4284 | 10 | 11 | 5 | | | | -3.26 | | | |
| hsa-miR-663b | 9 | 26 | 39 | | | | -1.03 | | | |
| hsa-miR-25-3p | 8 | | 1383 | 5825 | 42402 | 29320 | 6.09 | 8.21 | 7.94 | 7.63 |
| hsa-miR-3656 | 8 | 10 | 743 | 2023 | 1 | | 4.20 | 5.69 | -8.43 | |
| hsa-miR-181a-5p | 7 | 1 | 81465 | 56064 | 1289686 | 1023049 | 12.19 | 11.69 | 13.08 | 12.96 |
| hsa-miR-3654 | 6 | 13 | 3 | | | 9 | -3.90 | | | -5.19 |
| hsa-miR-26a-5p | 6 | 17 | 18836 | 3542 | 49957 | 54929 | 8.45 | 6.08 | 6.76 | 7.12 |

**Table E2:** Summary table listing miRNA reads and log2. Log2 is calculated using the normalized ratio of either EXO 6W or EXO 11W samples / averaged reads in EXO derived from proliferative cells. Up-shuttled miRNAs (log2 > 2), in EXO derived from CTX0E03 cultured for 6 and 11 weeks in Integra flasks, are highlighted in lighter grey and down-shuttled (log2 <2) in darker grey respectively. The table presents only the top 30 more abundant miRNAs.

*Comparative analysis of miRNA expression in EXO samples sorted by largest reads in EXO derived from 6W Integra CTX0E03 culture*

| | Reads | | | | | | LOG2 | | | |
|---|---|---|---|---|---|---|---|---|---|---|
| MiRNA | EXO 07EH | EXO 07EI | EXO WK6 | EXO1 WK11 | EXO2 WK11 | EXO3 WK11 | EXO WK6 | EXO1 WK11 | EXO2 WK11 | EXO3 WK11 |
| hsa-miR-181a-5p | 7 | 1 | 81465 | 56064 | 1289686 | 1023049 | 12.19 | 11.69 | 13.08 | 12.96 |
| hsa-miR-1246 | 111092 | 83958 | 77678 | 18 | 2726 | 24152 | -1.40 | -13.43 | -9.32 | -5.96 |
| hsa-miR-127-3p | | 6 | 36828 | 29 | 22175 | 48371 | 11.39 | 1.13 | 7.57 | 8.92 |
| hsa-miR-21-5p | 18 | 41 | 35754 | 12191 | 134432 | 150516 | 8.01 | 6.50 | 6.82 | 7.21 |
| hsa-miR-100-5p | 11 | 5 | 30124 | 22 | 56751 | 43080 | 9.68 | -0.69 | 7.50 | 7.32 |
| hsa-miR-99b-5p | 19 | 15 | 25938 | 6984 | 56360 | 50611 | 8.35 | 6.50 | 6.37 | 6.44 |
| hsa-miR-26a-5p | 6 | 17 | 18836 | 3542 | 49957 | 54929 | 8.45 | 6.08 | 6.76 | 7.12 |
| hsa-let-7a-5p | 20 | 15 | 18306 | 69521 | 496463 | 509272 | 7.80 | 9.77 | 9.47 | 9.72 |
| hsa-miR-191-5p | 4 | 1 | 17845 | 10674 | 130737 | 104349 | 10.49 | 9.79 | 10.27 | 10.17 |
| hsa-miR-27b-3p | 1 | | 15232 | 7344 | 33638 | 47061 | 12.55 | 11.55 | 10.60 | 11.31 |
| hsa-miR-92a-3p | 18 | 7 | 15054 | 5504 | 89826 | 73306 | 8.00 | 6.59 | 7.48 | 7.41 |
| hsa-miR-146b-5p | | 1 | 12960 | 12 | 29712 | 13822 | 12.47 | 2.44 | 10.58 | 9.69 |
| hsa-let-7i-5p | 1 | 2 | 12914 | 6928 | 157331 | 149116 | 10.83 | 9.98 | 11.34 | 11.49 |
| hsa-miR-222-3p | 3 | 2 | 12671 | 46 | 89141 | 64302 | 10.03 | 1.97 | 9.75 | 9.50 |
| hsa-let-7c | 1 | 2 | 12414 | | | | 10.77 | | | |
| hsa-miR-4488 | 1389 | 20618 | 11048 | | | | -2.12 | | | |
| hsa-miR-4492 | 5188 | 22482 | 9528 | 1 | | | -2.65 | -15.82 | | |
| hsa-let-7b-5p | 5 | 6 | 8914 | 34 | 86886 | 33259 | 8.40 | 0.41 | 8.59 | 7.43 |
| hsa-let-7f-5p | 5 | 6 | 8766 | 13898 | 293116 | 427140 | 8.38 | 9.09 | 10.35 | 11.11 |
| hsa-miR-92b-3p | 3 | 7 | 8141 | 73468 | 420313 | 291688 | 8.43 | 11.65 | 11.03 | 10.72 |
| hsa-miR-181b-5p | 1 | 3 | 7919 | 6713 | 226010 | 167200 | 9.72 | 9.53 | 11.46 | 11.25 |
| hsa-miR-221-3p | 3 | 3 | 6391 | 4927 | 17702 | 12768 | 8.79 | 8.46 | 7.17 | 6.92 |
| hsa-miR-125b-5p | 4 | 4 | 6107 | 2181 | 54267 | 37113 | 8.31 | 6.87 | 8.37 | 8.04 |
| hsa-miR-151a-3p | 3 | 4 | 6014 | 2776 | 26457 | 29135 | 8.49 | 7.42 | 7.53 | 7.89 |
| hsa-miR-30a-5p | 2 | | 5613 | 15559 | 136508 | 96950 | 10.11 | 11.63 | 11.62 | 11.35 |
| hsa-miR-16-5p | 10 | 4 | 5502 | 15 | 6580 | 9510 | 7.32 | -1.15 | 4.48 | 5.24 |
| hsa-miR-22-3p | 4 | 1 | 5489 | 2914 | 8193 | 10780 | 8.79 | 7.92 | 6.27 | 6.89 |
| hsa-miR-99a-5p | 1 | | 5116 | 14 | 27513 | 17532 | 10.98 | 2.51 | 10.31 | 9.88 |

**Table E3:** Summary table listing miRNA reads and log2. Log2 is calculated using the normalized ratio of either EXO 6W or EXO 11W samples / averaged reads in EXO derived from proliferative cells. Up-shuttled miRNAs (log2 > 2), in EXO derived from CTX0E03 cultured for 6 and 11 weeks in Integra flasks, are highlighted in lighter grey and down-shuttled (log2 <2) in darker grey respectively. The table presents only the top 30 more abundant miRNAs.

*Comparative analysis of miRNA expression in EXO samples sorted by largest reads in EXO3 derived from 11W Integra CTX0E03 culture*

| | Reads | | | | | | LOG2 | | | |
|---|---|---|---|---|---|---|---|---|---|---|
| MiRNA | EXO 07EH | EXO 07EI | EXO WK6 | EXO1 WK11 | EXO2 WK11 | EXO3 WK11 | EXO WK6 | EXO1 WK11 | EXO2 WK11 | EXO3 WK11 |
| hsa-miR-181a-5p | 7 | 1 | 81465 | 56064 | 1289686 | 1023049 | 12.19 | 11.69 | 13.08 | 12.96 |
| hsa-let-7a-5p | 20 | 15 | 18306 | 69521 | 496463 | 509272 | 7.80 | 9.77 | 9.47 | 9.72 |
| hsa-let-7f-5p | 5 | 6 | 8766 | 13898 | 293116 | 427140 | 8.38 | 9.09 | 10.35 | 11.11 |
| hsa-miR-92b-3p | 3 | 7 | 8141 | 73468 | 420313 | 291688 | 8.43 | 11.65 | 11.03 | 10.72 |
| hsa-miR-9-5p | 3 | 3 | 2196 | 155707 | 481660 | 263043 | 7.25 | 13.44 | 11.93 | 11.28 |
| hsa-miR-181b-5p | 1 | 3 | 7919 | 6713 | 226010 | 167200 | 9.72 | 9.53 | 11.46 | 11.25 |
| hsa-miR-21-5p | 18 | 41 | 35754 | 12191 | 134432 | 150516 | 8.01 | 6.50 | 6.82 | 7.21 |
| hsa-let-7i-5p | 1 | 2 | 12914 | 6928 | 157331 | 149116 | 10.83 | 9.98 | 11.34 | 11.49 |
| hsa-miR-191-5p | 4 | 1 | 17845 | 10674 | 130737 | 104349 | 10.49 | 9.79 | 10.27 | 10.17 |
| hsa-miR-30a-5p | 2 | | 5613 | 15559 | 136508 | 96950 | 10.11 | 11.63 | 11.62 | 11.35 |
| hsa-miR-92a-3p | 18 | 7 | 15054 | 5504 | 89826 | 73306 | 8.00 | 6.59 | 7.48 | 7.41 |
| hsa-miR-10a-5p | | 1 | 3754 | 5840 | 132775 | 66316 | 10.69 | 11.37 | 12.74 | 11.96 |
| hsa-miR-222-3p | 3 | 2 | 12671 | 46 | 89141 | 64302 | 10.03 | 1.97 | 9.75 | 9.50 |
| hsa-let-7c-5p | | | | 13106 | 68120 | 59498 | | | | |
| hsa-miR-26a-5p | 6 | 17 | 18836 | 3542 | 49957 | 54929 | 8.45 | 6.08 | 6.76 | 7.12 |
| hsa-miR-423-5p | 1 | 1 | 538 | 2606 | 45587 | 52945 | 6.81 | 9.13 | 10.12 | 10.55 |
| hsa-miR-99b-5p | 19 | 15 | 25938 | 6984 | 56360 | 50611 | 8.35 | 6.50 | 6.37 | 6.44 |
| hsa-miR-127-3p | | 6 | 36828 | 29 | 22175 | 48371 | 11.39 | 1.13 | 7.57 | 8.92 |
| hsa-miR-27b-3p | 1 | | 15232 | 7344 | 33638 | 47061 | 12.55 | 11.55 | 10.60 | 11.31 |
| hsa-miR-100-5p | 11 | 5 | 30124 | 22 | 56751 | 43080 | 9.68 | -0.69 | 7.50 | 7.32 |
| hsa-let-7e-5p | | 2 | 1932 | 5398 | 27921 | 41799 | 8.73 | 10.26 | 9.49 | 10.29 |
| hsa-miR-486-5p | 3 | 1 | 3503 | 44199 | 20054 | 37232 | 8.47 | 12.17 | 7.89 | 9.01 |
| hsa-miR-125b-5p | 4 | 4 | 6107 | 2181 | 54267 | 37113 | 8.31 | 6.87 | 8.37 | 8.04 |
| hsa-let-7b-5p | 5 | 6 | 8914 | 34 | 86886 | 33259 | 8.40 | 0.41 | 8.59 | 7.43 |
| hsa-miR-182-5p | 3 | 15 | 833 | 22 | 33064 | 32491 | 4.32 | -0.88 | 6.53 | 6.73 |
| hsa-miR-30d-5p | | 2 | 3946 | 22 | 26066 | 32148 | 9.76 | 2.32 | 9.39 | 9.91 |
| hsa-miR-25-3p | 8 | | 1383 | 5825 | 42402 | 29320 | 6.09 | 8.21 | 7.94 | 7.63 |
| hsa-miR-151a-3p | 3 | 4 | 6014 | 2776 | 26457 | 29135 | 8.49 | 7.42 | 7.53 | 7.89 |

**Table E4:** Summary table listing miRNA reads and log2. Log2 is calculated using the normalized ratio of either EXO 6W or EXO 11W samples / averaged reads in EXO derived from proliferative cells. Up-shuttled miRNAs (log2 > 2), in EXO derived from CTX0E03 cultured for 6 and 11 weeks in Integra flasks, are high-lighted in lighter grey and down-shuttled (log2 <2) in darker grey respectively. The table presents only the top 30 more abundant miRNAs.

*Conclusions for comparative summary of miRNA reads present in exosome samples*

Hsa-miR-1246, hsa-miR-4492, hsa-miR-4532, and hsa-miR-4488 are the most up-shuttled miRNA types in exosomes derived from proliferative CTX0E03 cells.

Hsa-miR-1246, hsa-miR-4492, hsa-miR-4532, and hsa-miR-4488 are still present in EXO 6W sample, but hsa-miR-4492, hsa-miR-4532, and hsa-miR-4488 are almost absent in EXO 11W samples.

Hsa-miR-181a-5p, hsa-miR-1246, hsa-miR-127-3p, hsa-miR-21-5p, and hsa-miR-100-5p are the top 5 miRNAs present in EXO 6W sample.

Hsa-miR-181a-5p, hsa-let-7a-5p, hsa-let-7f-5p, hsa-miR-92b-3p, and hsa-miR-9-5p are the top 5 miRNAs present in EXO 11W samples.

*Comparitive analysis of miRNA expression in cell samples sorted by largest reads in proliferative cell 07EH*

| | Reads | | | | | | LOG2 | | | |
|---|---|---|---|---|---|---|---|---|---|---|
| miRNA | Cell (07EH) | Cells (07EI) | Cell 6WK | Cell1 WK11 | Cell2 WK11 | Cell3 WK11 | Cell 6WK | Cell1 WK11 | Cell2 WK11 | Cell3 WK11 |
| hsa-let-7a-5p | 75110 | 305060 | 357507 | 82000 | 572638 | 945050 | 0.08 | -0.68 | -0.44 | -0.58 |
| hsa-miR-92b-3p | 9794 | 242715 | 51788 | 7070 | 95904 | 269851 | -1.46 | -2.97 | -1.78 | -1.15 |
| hsa-miR-21-5p | 11943 | 154626 | 109713 | 36935 | 165335 | 350731 | 0.06 | -0.15 | -0.55 | -0.33 |
| hsa-miR-92a-3p | 14359 | 137412 | 136842 | 15296 | 99662 | 238214 | 0.41 | -1.39 | -1.25 | -0.86 |
| hsa-miR-127-3p | 7064 | 110806 | 54214 | 4015 | 20215 | 70609 | -0.40 | -2.79 | -3.03 | -2.09 |
| hsa-miR-100-5p | 52451 | 109290 | 121101 | 8268 | 35093 | 40100 | -0.61 | -3.12 | -3.60 | -4.28 |
| hsa-miR-27b-3p | 16900 | 91902 | 55177 | 15130 | 73464 | 113623 | -0.66 | -1.16 | -1.45 | -1.69 |
| hsa-miR-191-5p | 12591 | 89150 | 58145 | 16783 | 74782 | 151339 | -0.37 | -0.80 | -1.21 | -1.06 |
| hsa-miR-26a-5p | 9900 | 88724 | 164401 | 24518 | 127010 | 271289 | 1.27 | -0.11 | -0.30 | -0.08 |
| hsa-miR-99b-5p | 39457 | 87399 | 46207 | 3177 | 14278 | 22554 | -1.62 | -4.12 | -4.52 | -4.72 |
| hsa-let-7f-5p | 10349 | 78395 | 61473 | 115717 | 457274 | 982774 | -0.06 | 2.21 | 1.63 | 1.87 |
| hsa-miR-181a-5p | 6956 | 47686 | 180839 | 150467 | 1304081 | 1909336 | 2.15 | 3.25 | 3.80 | 3.48 |
| hsa-miR-486-5p | 20310 | 41639 | 4938 | 465 | 1500 | 4379 | -3.85 | -5.90 | -6.78 | -6.10 |
| hsa-miR-30a-5p | 2001 | 35465 | 16099 | 23958 | 94346 | 201534 | -0.47 | 1.47 | 0.88 | 1.11 |
| hsa-miR-98 | 11760 | 30440 | 14559 | | | | -1.61 | | | |
| hsa-miR-151a-3p | 2681 | 29047 | 15661 | 5186 | 22917 | 58870 | -0.42 | -0.65 | -1.07 | -0.57 |
| hsa-miR-21-3p | 4089 | 27733 | 10626 | 2682 | 10247 | 30683 | -1.16 | -1.78 | -2.42 | -1.70 |
| hsa-miR-30d-5p | 1977 | 27307 | 17522 | 4802 | 35022 | 65318 | -0.06 | -0.56 | -0.26 | -0.23 |
| hsa-let-7c | 5103 | 27224 | 70342 | | | | 1.43 | | | |
| hsa-miR-10a-5p | 52927 | 26908 | 28817 | 11983 | 49442 | 69645 | -2.35 | -2.25 | -2.77 | -3.14 |
| hsa-miR-22-3p | 1826 | 26456 | 10088 | 5318 | 33593 | 54768 | -0.79 | -0.35 | -0.26 | -0.42 |
| hsa-miR-182-5p | 5531 | 25885 | 12376 | 2528 | 18519 | 49416 | -1.10 | -2.03 | -1.72 | -1.17 |
| hsa-miR-222-3p | 1422 | 22187 | 10960 | 13094 | 53385 | 90511 | -0.39 | 1.23 | 0.69 | 0.59 |
| hsa-miR-125a-5p | 1451 | 20960 | 9101 | 1406 | 12194 | 16095 | -0.61 | -1.94 | -1.39 | -1.85 |
| hsa-miR-16-5p | 2173 | 19856 | 69449 | 8542 | 42494 | 81960 | 2.20 | 0.54 | 0.29 | 0.37 |
| hsa-let-7b-5p | 2435 | 19774 | 61291 | 13225 | 75017 | 99021 | 1.96 | 1.11 | 1.05 | 0.58 |
| hsa-miR-151a-5p | 1386 | 19773 | 10790 | 1621 | 9192 | 17997 | -0.28 | -1.65 | -1.72 | -1.61 |
| hsa-let-7e-5p | 2449 | 19035 | 14175 | 7948 | 40674 | 84912 | -0.12 | 0.41 | 0.20 | 0.39 |

**Table E5:** Summary table listing miRNA reads and log2. Log2 is calculated using the normalized ratio of either cell 6W or cell 11W samples / averaged reads in proliferative cells. Up-expressed miRNAs (log2 > 2), in CTX0E03 cultured for 6 and 11 weeks in Integra flasks, are highlighted in lighter grey and down-expressed (log2 <2) in darker grey respectively. The table presents only the top 30 more abundant miRNAs.

*Comparative analysis of miRNA expression in cell samples sorted by largest reads in cells cultured for 6 week in integra flasks (Cell 6W)*

| | Reads | | | | | | LOG2 | | | |
|---|---|---|---|---|---|---|---|---|---|---|
| miRNA | Cell (07EH) | Cells (07EI) | Cell 6WK | Cell1 WK11 | Cell2 WK11 | Cell3 WK11 | Cell 6WK | Cell1 WK11 | Cell2 WK11 | Cell3 WK11 |
| hsa-let-7a-5p | 75110 | 305060 | 357507 | 82000 | 572638 | 945050 | 0.08 | -0.68 | -0.44 | -0.58 |
| hsa-miR-181a-5p | 6956 | 47686 | 180839 | 150467 | 1304081 | 1909336 | 2.15 | 3.25 | 3.80 | 3.48 |
| hsa-miR-26a-5p | 9900 | 88724 | 164401 | 24518 | 127010 | 271289 | 1.27 | -0.11 | -0.30 | -0.08 |
| hsa-miR-92a-3p | 14359 | 137412 | 136842 | 15296 | 99662 | 238214 | 0.41 | -1.39 | -1.25 | -0.86 |
| hsa-miR-100-5p | 52451 | 109290 | 121101 | 8268 | 35093 | 40100 | -0.61 | -3.12 | -3.60 | -4.28 |
| hsa-miR-21-5p | 11943 | 154626 | 109713 | 36935 | 165335 | 350731 | 0.06 | -0.15 | -0.55 | -0.33 |
| hsa-let-7c | 5103 | 27224 | 70342 | | | | 1.43 | | | |
| hsa-miR-16-5p | 2173 | 19856 | 69449 | 8542 | 42494 | 81960 | 2.20 | 0.54 | 0.29 | 0.37 |
| hsa-let-7f-5p | 10349 | 78395 | 61473 | 115717 | 457274 | 982774 | -0.06 | 2.21 | 1.63 | 1.87 |
| hsa-let-7b-5p | 2435 | 19774 | 61291 | 13225 | 75017 | 99021 | 1.96 | 1.11 | 1.05 | 0.58 |
| hsa-miR-191-5p | 12591 | 89150 | 58145 | 16783 | 74782 | 151339 | -0.37 | -0.80 | -1.21 | -1.06 |
| hsa-miR-27b-3p | 16900 | 91902 | 55177 | 15130 | 73464 | 113623 | -0.66 | -1.16 | -1.45 | -1.69 |
| hsa-miR-127-3p | 7064 | 110806 | 54214 | 4015 | 20215 | 70609 | -0.40 | -2.79 | -3.03 | -2.09 |
| hsa-miR-92b-3p | 9794 | 242715 | 51788 | 7070 | 95904 | 269851 | -1.46 | -2.97 | -1.78 | -1.15 |
| hsa-miR-9-5p | 518 | 7957 | 49128 | 113170 | 696970 | 1266994 | 3.25 | 5.82 | 5.87 | 5.87 |
| hsa-miR-99b-5p | 39457 | 87399 | 46207 | 3177 | 14278 | 22554 | -1.62 | -4.12 | -4.52 | -4.72 |
| hsa-miR-146b-5p | 4552 | 8434 | 38088 | 7472 | 43047 | 20961 | 1.29 | 0.30 | 0.26 | -1.64 |
| hsa-miR-125b-5p | 1002 | 17965 | 34339 | 2841 | 21418 | 24635 | 1.61 | -0.62 | -0.28 | -0.94 |
| hsa-miR-1246 | 3973 | 1783 | 32042 | 483 | 1122 | 3470 | 1.56 | -3.13 | -4.48 | -3.72 |
| hsa-miR-10a-5p | 52927 | 26908 | 28817 | 11983 | 49442 | 69645 | -2.35 | -2.25 | -2.77 | -3.14 |
| hsa-let-7i-5p | 3015 | 17802 | 21469 | 33910 | 167628 | 432785 | 0.40 | 2.43 | 2.16 | 2.67 |
| hsa-miR-99a-5p | 773 | 2767 | 19124 | 1826 | 11313 | 14604 | 2.54 | 0.51 | 0.58 | 0.08 |
| hsa-miR-30d-5p | 1977 | 27307 | 17522 | 4802 | 35022 | 65318 | -0.06 | -0.56 | -0.26 | -0.23 |
| hsa-let-7g-5p | 959 | 15467 | 16691 | 4240 | 19981 | 36131 | 0.75 | 0.14 | -0.20 | -0.21 |
| hsa-miR-30a-5p | 2001 | 35465 | 16099 | 23958 | 94346 | 201534 | -0.47 | 1.47 | 0.88 | 1.11 |
| hsa-miR-151a-3p | 2681 | 29047 | 15661 | 5186 | 22917 | 58870 | -0.42 | -0.65 | -1.07 | -0.57 |
| hsa-miR-98 | 11760 | 30440 | 14559 | | | | -1.61 | | | |
| hsa-miR-204-5p | 113 | 1378 | 14322 | 1554 | 8147 | 18192 | 3.91 | 2.07 | 1.89 | 2.18 |

**Table E6:** Summary table listing miRNA reads and log2. Log2 is calculated using the normalized ratio of either cell 6W or cell 11W samples / averaged reads in proliferative cells. Up-expressed miRNAs (log2 > 2), in CTX0E03 cultured for 6 and 11 weeks in Integra flasks, are high-lighted in lighter grey and down-expressed (log2 < 2) in darker grey respectively. The table presents only the top 30 more abundant miRNAs.

*Comparative analysis of miRNA expression in cell samples sorted by largest reads in cells cultured 11 week in Integra flasks (Cell1 W11)*

| | Reads | | | | | | LOG2 | | | |
|---|---|---|---|---|---|---|---|---|---|---|
| miRNA | Cell (07EH) | Cells (07EI) | Cell 6WK | Cell1 WK11 | Cell2 WK11 | Cell3 WK11 | Cell 6WK | Cell1 WK11 | Cell2 WK11 | Cell3 WK11 |
| hsa-miR-181a-5p | 6956 | 47686 | 180839 | 150467 | 1304081 | 1909336 | 2.15 | 3.25 | 3.80 | 3.48 |
| hsa-miR-9-5p | 518 | 7957 | 49128 | 113170 | 696970 | 1266994 | 3.25 | 5.82 | 5.87 | 5.87 |
| hsa-let-7f-5p | 10349 | 78395 | 61473 | 115717 | 457274 | 982774 | -0.06 | 2.21 | 1.63 | 1.87 |
| hsa-let-7a-5p | 75110 | 305060 | 357507 | 82000 | 572638 | 945050 | 0.08 | -0.68 | -0.44 | -0.58 |
| hsa-let-7i-5p | 3015 | 17802 | 21469 | 33910 | 167628 | 432785 | 0.40 | 2.43 | 2.16 | 2.67 |
| hsa-miR-21-5p | 11943 | 154626 | 109713 | 36935 | 165335 | 350731 | 0.06 | -0.15 | -0.55 | -0.33 |
| hsa-miR-181b-5p | 1382 | 12606 | 11845 | 17620 | 135852 | 275865 | 0.30 | 2.24 | 2.62 | 2.77 |
| hsa-miR-26a-5p | 9900 | 88724 | 164401 | 24518 | 127010 | 271289 | 1.27 | -0.11 | -0.30 | -0.08 |
| hsa-miR-92b-3p | 9794 | 242715 | 51788 | 7070 | 95904 | 269851 | -1.46 | -2.97 | -1.78 | -1.15 |
| hsa-miR-92a-3p | 14359 | 137412 | 136842 | 15296 | 99662 | 238214 | 0.41 | -1.39 | -1.25 | -0.86 |
| hsa-miR-30a-5p | 2001 | 35465 | 16099 | 23958 | 94346 | 201534 | -0.47 | 1.47 | 0.88 | 1.11 |
| hsa-miR-191-5p | 12591 | 89150 | 58145 | 16783 | 74782 | 151339 | -0.37 | -0.80 | -1.21 | -1.06 |
| hsa-let-7c-5p | | | | 18010 | 106589 | 130919 | | | | |
| hsa-miR-27b-3p | 16900 | 91902 | 55177 | 15130 | 73464 | 113623 | -0.66 | -1.16 | -1.45 | -1.69 |
| hsa-let-7b-5p | 2435 | 19774 | 61291 | 13225 | 75017 | 99021 | 1.96 | 1.11 | 1.05 | 0.58 |
| hsa-miR-423-5p | 1080 | 8893 | 3219 | 9325 | 40672 | 93044 | -1.13 | 1.77 | 1.33 | 1.65 |
| hsa-miR-222-3p | 1422 | 22187 | 10960 | 13094 | 53385 | 90511 | -0.39 | 1.23 | 0.69 | 0.59 |
| hsa-let-7e-5p | 2449 | 19035 | 14175 | 7948 | 40674 | 84912 | -0.12 | 0.41 | 0.20 | 0.39 |
| hsa-miR-16-5p | 2173 | 19856 | 69449 | 8542 | 42494 | 81960 | 2.20 | 0.54 | 0.29 | 0.37 |
| hsa-miR-25-3p | 436 | 5303 | 8387 | 5633 | 31971 | 77502 | 1.19 | 1.98 | 1.92 | 2.33 |
| hsa-miR-127-3p | 7064 | 110806 | 54214 | 4015 | 20215 | 70609 | -0.40 | -2.79 | -3.03 | -2.09 |
| hsa-miR-10a-5p | 52927 | 26908 | 28817 | 11983 | 49442 | 69645 | -2.35 | -2.25 | -2.77 | -3.14 |
| hsa-miR-30d-5p | 1977 | 27307 | 17522 | 4802 | 35022 | 65318 | -0.06 | -0.56 | -0.26 | -0.23 |
| hsa-miR-151a-3p | 2681 | 29047 | 15661 | 5186 | 22917 | 58870 | -0.42 | -0.65 | -1.07 | -0.57 |
| hsa-miR-22-3p | 1826 | 26456 | 10088 | 5318 | 33593 | 54768 | -0.79 | -0.35 | -0.26 | -0.42 |
| hsa-miR-182-5p | 5531 | 25885 | 12376 | 2528 | 18519 | 49416 | -1.10 | -2.03 | -1.72 | -1.17 |
| hsa-miR-181a-3p | 86 | 803 | 1186 | 2490 | 21343 | 44745 | 0.97 | 3.40 | 3.93 | 4.13 |
| hsa-miR-100-5p | 52451 | 109290 | 121101 | 8268 | 35093 | 40100 | -0.61 | -3.12 | -3.60 | -4.28 |

**Table E7**: Summary table listing miRNA reads and log2. Log2 is calculated using the normalized ratio of either cell 6W or cell 11W samples / averaged reads in proliferative cells. Up-expressed miRNAs (log2 > 2), in CTX0E03 cultured for 6 and 11 weeks in Integra flasks, are high-lighted in lighter grey and down-expressed (log2 < 2) in darker grey respectively. The table presents only the top 30 more abundant miRNAs.

*Conclusions for comparative summary of miRNA reads present in cell samples*

Hsa-let-7a-5p, hsa-miR-92b-3p, hsa-miR-21-5p, hsa-miR-92a-3p, and hsa-miR-127-3p are the top 5 most expressed miRNA types in proliferative CTX0E03 cells.

Hsa-let-7a-5p, hsa-miR-181a-5p, hsa-miR-26a-5p, hsa-miR-92a-3p, hsa-miR-100-5p are the top 5 most expressed miRNA types in CTX0E03 Integra 6W culture.

Hsa-miR-181a-5p, hsa-miR-9-5p, hsa-let-7f-5p, hsa-let-7a-5p, and hsa-let-7i-5p are the top 5 most expressed miRNA types in CTX0E03 Integra 11W cultures.

Hsa-miR-181a-5p and hsa-miR-9-5p are up-expressed in all cell samples cultured in Integra flasks (6 and 11 weeks).

Hsa-let-7i-5p, hsa-let-7c-5p, hsa-miR-181a-3p and hsa-miR-181b-5p were solely up-expressed in W11 cells.

Hsa-miR-181 family seems to play an important role in CTX0E03 long term culture and possible differentiation.

**Example 18:** Proteomic analysis

Methods

Exosomes and microvesicle fractions were prepared from a CTX0E03 cell Integra culture (week 2), using differential ultracentrifugation. Exosomes and microvesicles were disrupted in modified RIPA buffer (50mM Tris HCl, pH 8.0, 150mM NaCl, 1% SDS, 0.1% Triton X100, 10mM DTT, 1x Complete protease inhibitor (Roche) and 1x PhosStop phosphatase inhibitor (Roche)) and subjected to manual shearing using a 1mL tuberculin syringe and 25 gauge needle. Samples were re-quantitated post disruption using the Qubit fluorometer (Invitrogen). 20$\mu$g of each sample was loaded onto a 4-12% SDS-PAGE gel (Novex, Invitrogen). The gel was excised into forty segments per lane and gel slices were processed using a robot (ProGest, DigiLab) with the following protocol:

a) wash with 25mM ammonium bicarbonate followed by acetonitrile;
b) reduce with 10mM dithiothreitol at 60°C followed by alkylation with 50mM iodoacetamide at room temperature;
c) digest with trypsin (Promega) at 37°C for 4h;
d) quench with formic acid;
e) the supernatant was analysed by mass spectrometry directly without further processing.

*Mass Spectrometry*

Each gel digest was analysed by nano LC/MS/MS with a Waters NanoAcquity HPLC system interfaced to a ThermoFisher Q Exactive. Peptides were loaded on a trapping column and eluted over a 75μm analytical column at 350nL/min; both columns were packed with Jupiter Proteo resin (Phenomenex). The mass spectrometer was operated in data-dependent mode, with MS and MS/MS performed in the Orbitrap at 70,000 FWHM and 17,500 FWHM resolution, respectively.

Exosomes

2572 proteins were identified by Mass spectrometry in exosomes purified by ultracentrifugation. The exosomes were isolated from the initial stages of an Integra culture (week 2). The gene names and corresponding SWISSPROT accession numbers (in brackets) of all 2572 proteins are listed in Table 19 (in alphabetical order of gene name) and the 100 most abundant proteins are listed in Table 20, in order of decreasing abundance. The characteristic exosome markers CD9, CD81 and Alix (also known as PDCD6IP) are present in the most abundant 100 proteins.

**Table** 19: Gene names and SWISSPROT accession numbers of all 2572 proteins identified in CTX0E03 exosomes (listed in alphabetical order of gene name).

| |
|---|
| A1BG (P04217), A2M (P01023), AACS (Q86V21), AAMP (Q13685), AARS (P49588), AARSD1 (Q9BTE6), AASDHPPT (Q9NRN7), ABCA3 (Q99758), ABCE1 (P61221), ABCF1 (Q8NE71), ABCF3 (Q9NUQ8), ABHD10 (Q9NUJ1), ABHD14B (Q96IU4), ABI1 (Q8IZP0), ABR (Q12979), ACAA2 (P42765), ACACA (Q13085), ACADVL (P49748), ACAP2 (Q15057), ACAT1 (P24752), ACAT2 (Q9BWD1), ACBD7 (Q8N6N7), ACLY (P53396), ACO1 (P21399), ACO2 (Q99798), ACOT1 (Q86TX2), ACOT13 (Q9NPJ3), ACOT7 (O00154), ACP1 (P24666), ACSL1 (P33121), ACSL3 (O95573), ACSL4 (O60488), ACSS2 (Q9NR19), ACTC1 (P68032), ACTG1 (P63261), ACTL6A (096019), ACTN1 (P12814), ACTN4 (O43707), ACTR10 (Q9NZ32), ACTR1A (P61163), ACTR1 B (P42025), ACTR2 (P61160), ACTR3 (P61158), ADAM10 (O14672), ADAM12 (O43184), ADAMTS15 (Q8TE58), ADAMTS16 (Q8TE57), ADAR (P55265), ADAT2 (Q7Z6V5), ADH5 (P11766), ADI1 (Q9BV57), ADK (P55263), ADRBK1 (P25098), ADRM1 (O16186), ADSL (P30566), ADSS (P30520), AEBP1 (Q8IUX7), AFM (P43652), AGL (P35573), AGRN (O00468), AGT (P01019), AHCY (P23526), AHCYL1 (O43865), AHNAK (Q09666), AHSA1 (O95433), AHSG (P02765), AIDA (Q96BJ3), AIFM1 (O95831), AIMP1 (Q12904), AIMP2 (Q13155), AIP (O00170), AK1 (P00568), AK3 (Q9UIJ7), AK4 (P27144), AKAP12 (Q02952), AKAP9 (Q99996), AKR1A1 (P14550), AKR1B1 (P15121), AKR1C1 (Q04828), AKR7A2 (O43488), AKR7A3 (O95154), AKT1 (P31749), ALCAM (Q13740), ALDH16A1 (Q8IZ83), ALDH3A1 (P30838), ALDH7A1 (P49419), ALDH9A1 (P49189), ALDOA (P04075), ALDOC (P09972), ALKBH2 (Q6NS38), ALKBH4 (Q9NXW9), AMBP (P02760), AMDHD2 (Q9Y303), AMPD2 (Q01433), AMZ2 (Q86W34), ANAPC1 (Q9H1A4), ANAPC4 (Q9UJX5), ANAPC5 (Q9UJX4), ANAPC7 (Q9UJX3), ANKFY1 (Q9P2R3), ANKRD28 (015084), ANP32A (P39687), ANP32B (Q92688), ANP32E (Q9BTT0), ANXA1 (P04083), ANXA2 (P07355), ANXA4 (P09525), ANXA5 (P08758), ANXA6 (P08133), ANXA7 (P20073), AP1B1 (Q10567), AP1G1 (O43747), AP1M1 (Q9BXS5), AP1S1 (P61966), AP1S2 (P56377), AP2A1 (O95782), AP2A2 (O94973), AP2B1 (P63010), AP2M1 (Q96CW1), AP2S1 (P53680), AP3B1 (O00203), AP3D1 (O14617), AP3M1 (Q9Y2T2), AP3S1 (Q92572), AP3S2 (P59780), AP4S1 (Q9Y587), APEH (P13798), APEX1 (P27695), API5 (Q9BZZ5), APIP (Q96GX9), APOA1 (P02647), APOA1 BP (Q8NCW5), APOA2 (P02652), APOBEC3C (Q9NRW3), APOC2 (P02655), APOD (P05090), |

(continued)

| |
|---|
| APOH (P02749), APOM (O95445), APPL1 (Q9UKG1), APRT (P07741), AQR (O60306), ARCN1 (P48444), ARF1 (P84077), ARF4 (P18085), ARF5 (P84085), ARF6 (P62330), ARFIP1 (P53367), ARFIP2 (P53365), ARHGAP1 (Q07960), ARHGAP12 (Q8IWW6), ARHGDIA (P52565), ARHGEF1 (Q92888), ARHGEF10 (O15013), ARHGEF7 (Q14155), ARIH1 (Q9Y4X5), ARIH2 (O95376), ARL1 (P40616), ARL2 (P36404), ARL3 (P36405), ARL6IP1 (Q15041), ARL8B (Q9NVJ2), ARMC10 (Q8N2F6), ARMC6 (Q6NXE6), ARMC8 (Q8IUR7), ARMC9 (Q7Z3E5), ARMCX3 (Q9UH62), ARPC1A (Q92747), ARPC1B (O15143), ARPC2 (O15144), ARPC3 (O15145), ARPC4 (P59998), ARPC5 (O15511), ARPC5L (Q9BPX5), ARRDC1 (Q8N5I2), ASB6 (Q9NWX5), ASCC1 (Q8N9N2), ASCC2 (Q9H1I8), ASCC3 (Q8N3C0), ASF1A (Q9Y294), ASH2L (Q9UBL3), ASMTL (095671), ASNA1 (O43681), ASNS (P08243), ASS1 (P00966), ATG16L1 (Q676U5), ATG3 (Q9NT62), ATG4B (Q9Y4P1), ATG7 (O95352), ATIC (P31939), ATL3 (Q6DD88), ATM (Q13315), ATOX1 (O00244), ATP1A1 (P05023), ATP1B1 (P05026), ATP1B3 (P54709), ATP2B1 (P20020), ATP2B4 (P23634), ATP5B (P06576), ATP5E (P56381), ATP5I (P56385), ATP6AP2 (O75787), ATP6V0D1 (P61421), ATP6V1A (P38606), ATP6V1B2 (P21281), ATP6V1C1 (P21283), ATP6V1D (Q9Y5K8), ATP6V1E1 (P36543), ATP6V1G1 (O75348), ATP6V1 H (Q9UI12), ATR (Q13535), ATRN (O75882), ATXN10 (Q9UBB4), B2M (P61769), B3GAT3 (O94766), B3GNT1 (O43505), B4GALT7 (Q9UBV7), BAG2 (O95816), BAIAP2 (Q9UQB8), BANF1 (O75531), BAT1 (Q13838), BAT3 (P46379), BBOX1 (O75936), BCAS2 (O75934), BCAT1 (P54687), BCCIP (Q9P287), BCL2L13 (Q9BXK5), BCLAF1 (Q9NYF8), BDH2 (Q9BUT1), BICD2 (Q8TD16), BLOC1S1 (P78537), BLVRA (P53004), BLVRB (P30043), BMP1 (P13497), BOLA2 (Q9H3K6), BPGM (P07738), BPHL (Q86WA6), BPNT1 (O95861), BRCC3 (P46736), BRE (Q9NXR7), BROX (Q5VW32), BRP16L (POCB43), BSG (P35613), BST1 (Q10588), BTAF1 (O14981), BUB3 (O43684), BUD31 (P41223), BYSL (Q13895), BZW1 (Q7L1Q6), BZW2 (Q9Y6E2), C10orf119 (Q9BTE3), C10orf58 (Q9BRX8), C10orf76 (Q5T2E6), C11orf54 (Q9H0W9), C11orf68 (Q9H3H3), C12orf10 (Q9HB07), C14orf149 (Q96EM0), C14orf166 (Q9Y224), C15orf58 (Q6ZNW5), C16orf13 (Q96S19), C16orf80 (Q9Y6A4), C1D (Q13901), C1orf123 (Q9NWV4), C1orf50 (Q9BV19), C1orf57 (Q9BSD7), C1RL (Q9NZP8), C20orf11 (Q9NWU2), C20orf27 (Q9GZN8), C20orf4 (Q9Y312), C21orf59 (P57076), C22orf25 (Q6ICL3), C22orf28 (Q9Y3I0), C2orf29 (Q9UKZ1), C2orf79 (Q6GMV3), C3orf10 (Q8WUW1), C3orf26 (Q9BQ75), C3orf75 (Q0PNE2), C4orf27 (Q9NWY4), C4orf41 (Q7Z392), C5orf32 (Q9H1C7), C6orf130 (Q9Y530), C6orf211 (Q9H993), C7orf25 (Q9BPX7), C7orf28B (P86790), C7orf41 (Q8N3F0), C7orf59 (Q0VGL1), C9orf142 (Q9BUH6), C9orf23 (Q8N5L8), C9orf41 (Q8N4J0), C9orf64 (Q5T6V5), CA11 (O75493), CAB39 (Q9Y376), CACNA2D1 (P54289), CACYBP (Q9HB71), CAD (P27708), CADM1 (Q9BY67), CADM4 (Q8NFZ8), CALB1 (P05937), CALD1 (Q05682), CALM1 (P62158), CAMK2D (Q13557), CAND1 (Q86VP6), CAP1 (Q01518), CAPN1 (P07384), CAPN2 (P17655), CAPN5 (O15484), CAPNS1 (P04632), CAPS (Q13938), CAPZA1 (P52907), CAPZA2 (P47755), CAPZB (P47756), |

(continued)

| |
|---|
| CARHSP1 (Q9Y2V2), CARKD (Q8IW45), CARM1 (Q86X55), CARS (P49589), CASK (O14936), CASP3 (P42574), CASP6 (P55212), CAT (P04040), CBFB (Q13951), CBR1 (P16152), CBR3 (O75828), CBS (P35520), CBWD2 (Q8IUF1), CBX1 (P83916), CBX3 (Q13185), CBX5 (P45973), CC2D1A (Q6P1 N0), CC2D1B (Q5T0F9), CCAR1 (Q8IX12), CCBL1 (Q16773), CCBL2 (Q6YP21), CCDC22 (O60826), CCDC25 (Q86WR0), CCDC53 (Q9Y3C0), CCDC56 (Q9Y2R0), CCDC93 (Q567U6), CCNC (P24863), CCND2 (P30279), CCNH (P51946), CCT2 (P78371), CCT3 (P49368), CCT4 (P50991), CCT5 (P48643), CCT6A (P40227), CCT7 (Q99832), CCT8 (P50990), CD109 (Q6YHK3), CD151 (P48509), CD276 (Q5ZPR3), CD44 (P16070), CD47 (Q08722), CD59 (P13987), CD63 (P08962), CD81 (P60033), CD9 (P21926), CD99 (P14209), CDC16 (Q13042), CDC23 (Q9UJX2), CDC27 (P30260), CDC34 (P49427), CDC37 (Q16543), CDC40 (O60508), CDC42 (P60953), CDC5L (Q99459), CDCP1 (Q9H5V8), CDH2 (P19022), CDK1 (P06493), CDK2 (P24941), CDK2AP2 (O75956), CDK4 (P11802), CDK5 (Q00535), CDK5RAP3 (Q96JB5), CDK7 (P50613), CDKN2A (P42771), CDKN2AIP (Q9NXV6), CELSR1 (Q9NYQ6), CELSR2 (Q9HCU4), CEP57 (Q86XR8), CFL1 (P23528), CFL2 (Q9Y281), CHAC2 (Q8WUX2), CHAF1B (Q13112), CHD4 (Q14839), CHEK2 (O96017), CHERP (Q8IWX8), CHID1 (Q9BWS9), CHML (P26374), CHMP1B (Q7LBR1), CHMP2A (O43633), CHMP4A (Q9BY43), CHMP4B (Q9H444), CHMP6 (Q96FZ7), CHORDC1 (Q9UHD1), CHP (Q99653), CHRAC1 (Q9NRG0), CHST14 (Q8NCH0), CHST3 (Q7LGC8), CHURC1 (Q8WUH1), CIAO1 (O76071), CIAPIN1 (Q6FI81), CIRH1A (Q969X6), CKAP5 (Q14008), CKB (P12277), CLASP1 (Q7Z460), CLDN3 (015551), CLEC18B (Q6UXF7), CLIC1 (O00299), CLIC4 (Q9Y696), CLLD6 (Q5W111), CLNS1A (P54105), CLP1 (Q92989), CLPB (Q9H078), CLTA (P09496), CLTC (Q00610), CLU (P10909), CMAS (Q8NFW8), CMBL (Q96DG6), CMPK1 (P30085), CNBP (P62633), CNDP2 (Q96KP4), CNN2 (Q99439), CNN3 (Q15417), CNOT1 (A5YKK6), CNOT10 (Q9H9A5), CNOT6L (Q96LI5), CNOT7 (Q9UIV1), CNP (P09543), COASY (Q13057), COBRA1 (Q8WX92), COG1 (Q8WTW3), COG2 (Q14746), COG3 (Q96JB2), COG4 (Q9H9E3), COG5 (Q9UP83), COG6 (Q9Y2V7), COG7 (P83436), COG8 (Q96MW5), COL11A1 (P12107), COL14A1 (Q05707), COL6A1 (P12109), COMMD1 (Q8N668), COMMD10 (Q9Y6G5), COMMD2 (Q86X83), COMMD3 (Q9UBI1), COMMD4 (Q9H0A8), COMMD5 (Q9GZQ3), COMMD6 (Q7Z4G1), COMMD7 (Q86VX2), COMMD8 (Q9NX08), COMMD9 (Q9P000), COMT (P21964), COPA (P53621), COPB1 (P53618), COPB2 (P35606), COPE (014579), COPG (Q9Y678), COPG2 (Q9UBF2), COPS2 (P61201), COPS3 (Q9UNS2), COPS4 (Q9BT78), COPS5 (Q92905), COPS6 (Q7L5N1), COPS7A (Q9UBW8), COPS7B (Q9H9Q2), COPS8 (Q99627), COPZ1 (P61923), CORO1A (P31146), CORO1 B (Q9BR76), CORO1C (Q9ULV4), CORO2B (Q9UQ03), CORO7 (P57737), COTL1 (Q14019), COX5A (P20674), COX5B (P10606), COX6C (P09669), COX7A2 (P14406), CP (P00450), CPD (O75976), CPN2 (P22792), CPNE1 (Q99829), CPNE3 (O75131), CPNE7 (Q9UBL6), CPSF1 (Q10570), CPSF2 (Q9P2I0), CPSF3 (Q9UKF6), CPSF7 (Q8N684), CPXM1 (Q96SM3), CRIP2 (P52943), CRK (P46108), |

(continued)

| |
|---|
| CRLF3 (Q8IUI8), CRTAP (O75718), CRYAB (P02511), CRYM (Q14894), CRYZ (Q08257), CRYZL1 (O95825), CS (O75390), CSDE1 (O75534), CSE1L (P55060), CSK (P41240), CSNK1A1 (P48729), CSNK2A1 (P68400), CSNK2B (P67870), CSRP1 (P21291), CSRP2 (Q16527), CSTB (P04080), CSTF1 (Q05048), CSTF2T (Q9H0L4), CSTF3 (Q12996), CTBP1 (Q13363), CTBP2 (P56545), CTNNA1 (P35221), CTNNB1 (P35222), CTNNBL1 (Q8WYA6), CTNND1 (O60716), CTPS (P17812), CTPS2 (Q9NRF8), CTR9 (Q6PD62), CTSC (P53634), CTSD (P07339), CTSF (Q9UBX1), CTSL2 (060911), CTU1 (Q7Z7A3), CTU2 (Q2VPK5), CUL1 (Q13616), CUL2 (Q13617), CUL3 (Q13618), CUL4A (Q13619), CUL4B (Q13620), CUL5 (Q93034), CWF19L1 (Q69YN2), CXADR (P78310), CXorf26 (Q9BVG4), CYB5A (P00167), CYCS (P99999), CYFIP1 (Q7L576), CYFIP2 (Q96F07), CYR61 (O00622), DAG1 (Q14118), DAK (Q3LXA3), DARS (P14868), DAZAP1 (Q96EP5), DBI (P07108), DBN1 (Q16643), DBNL (Q9UJU6), DBR1 (Q9UK59), DCAF7 (P61962), DCAF8 (Q5TAQ9), DCD (P81605), DCK (P27707), DCLK1 (O15075), DCPS (Q96C86), DCTD (P32321), DCTN1 (Q14203), DCTN2 (Q13561), DCTN3 (O75935), DCTN4 (Q9UJW0), DCTN5 (Q9BTE1), DCTN6 (O00399), DCUN1D1 (Q96GG9), DCUN1D5 (Q9BTE7), DCXR (Q7Z4W1), DDA1 (Q9BW61), DDAH2 (O95865), DDB1 (Q16531), DDB2 (Q92466), DDI2 (Q5TDH0), DDR1 (Q08345), DDT (P30046), DDX1 (Q92499), DDX17 (Q92841), DDX19A (Q9NUU7), DDX21 (Q9NR30), DDX23 (Q9BUQ8), DDX39 (O00148), DDX3X (O00571), DDX5 (P17844), DDX51 (Q8N8A6), DDX6 (P26196), DECR1 (Q16698), DEF (Q68CQ4), DEFA1 (P59665), DENR (O43583), DERA (Q9Y315), DFFA (O00273), DHFR (P00374), DHPS (P49366), DHRS1 (Q96LJ7), DHRS11 (Q6UWP2), DHRS4 (Q9BTZ2), DHX15 (O43143), DHX16 (O60231), DHX29 (Q7Z478), DHX36 (Q9H2U1), DHX9 (Q08211), DIAPH1 (O60610), DIAPH2 (O60879), DIMT1L (Q9UNQ2), DIP2B (Q9P265), DIP2C (Q9Y2E4), DIS3 (Q9Y2L1), DIS3L2 (Q8IYB7), DKC1 (O60832), DLG1 (Q12959), DNAH17 (Q9UFH2), DNAJA1 (P31689), DNAJA2 (O60884), DNAJB1 (P25685), DNAJB4 (Q9UDY4), DNAJC13 (075165), DNAJC3 (Q13217), DNAJC7 (Q99615), DNASE1L1 (P49184), DNM1 (Q05193), DNM1L (O00429), DNM2 (P50570), DNPEP (Q9ULA0), DOCK1 (Q14185), DOCK4 (Q8N1I0), DOCK5 (Q9H7D0), DOCK7 (Q96N67), DOHH (Q9BU89), DOM3Z (O77932), DPCD (Q9BVM2), DPH1 (Q9BZG8), DPH2 (Q9BQC3), DPH5 (Q9H2P9), DPM1 (O60762), DPP3 (Q9NY33), DPP9 (Q86TI2), DPY30 (Q9C005), DPYSL2 (Q16555), DPYSL3 (Q14195), DPYSL4 (014531), DPYSL5 (Q9BPU6), DRG1 (Q9Y295), DRG2 (P55039), DSG1 (Q02413), DSP (P15924), DST (Q03001), DSTN (P60981), DTD1 (Q8TEA8), DTYMK (P23919), DUS2L (Q9NX74), DUSP12 (Q9UNI6), DUSP23 (Q9BVJ7), DUSP3 (P51452), DYM (Q7RTS9), DYNC1H1 (Q14204), DYNC1I2 (Q13409), DYNC1LI1 (Q9Y6G9), DYNC1LI2 (O43237), DYNC2H1 (Q8NCM8), DYNLL1 (P63167), DYNLL2 (Q96FJ2), DYNLRB1 (Q9NP97), DYNLT1 (P63172), ECHDC1 (Q9NTX5), ECHDC3 (Q96DC8), ECHS1 (P30084), ECM29 (Q5VYK3), EDC4 (Q6P2E9), EEA1 (Q15075), EEF1A1 (P68104), EEF1B2 (P24534), EEF1D (P29692), EEF1E1 (O43324), EEF1G (P26641), EEF2 (P13639), EEFSEC (P57772), |

(continued)

| |
|---|
| EFEMP2 (O95967), EFHD2 (Q96C19), EFNB2 (P52799), EFTUD1 (Q7Z2Z2), EFTUD2 (Q15029), EGFR (P00533), EHD1 (Q9H4M9), EHD2 (Q9NZN4), EHD4 (Q9H223), EIF1 (P41567), EIF1AX (P47813), EIF2A (Q9BY44), EIF2AK2 (P19525), EIF2B1 (Q14232), EIF2B2 (P49770), EIF2B3 (Q9NR50), EIF2B4 (Q9UI10), EIF2B5 (Q13144), EIF2C2 (Q9UKV8), EIF2S1 (P05198), EIF2S2 (P20042), EIF2S3 (P41091), EIF3A (Q14152), EIF3B (P55884), EIF3C (Q99613), EIF3D (O15371), EIF3E (P60228), EIF3F (O00303), EIF3G (O75821), EIF3H (O15372), EIF31 (Q13347), EIF3J (O75822), EIF3K (Q9UBQ5), EIF3L (Q9Y262), EIF3M (Q7L2H7), EIF4A1 (P60842), EIF4A2 (Q14240), EIF4A3 (P38919), EIF4E (P06730), EIF4E2 (O60573), EIF4G1 (Q04637), EIF4G2 (P78344), EIF4G3 (O43432), EIF4H (Q15056), EIF5 (P55010), EIF5A (P63241), EIF5B (O60841), EIF6 (P56537), ELAC2 (Q9BQ52), ELAVL1 (Q15717), ELMO2 (Q96JJ3), ELP2 (Q6IA86), ELP3 (Q9H9T3), EMG1 (Q92979), EMILIN1 (Q9Y6C2), EML1 (O00423), EML2 (O95834), EML3 (Q32P44), EML4 (Q9HC35), ENAH (Q8N8S7), ENO1 (P06733), ENO2 (P09104), ENOPH1 (Q9UHY7), ENY2 (Q9NPA8), EPB41L2 (O43491), EPB41L3 (Q9Y2J2), EPHA2 (P29317), EPHB3 (P54753), EPHX1 (P07099), EPM2AIP1 (Q7L775), EPRS (P07814), ERH (P84090), ERI1 (Q8IV48), ERI3 (O43414), ERP44 (Q9BS26), ESD (P10768), ESYT1 (Q9BSJ8), ETF1 (P62495), ETFA (P13804), ETFB (P38117), EXOC1 (Q9NV70), EXOC2 (Q96KP1), EXOC3 (O60645), EXOC4 (Q96A65), EXOC5 (O00471), EXOC6 (Q8TAG9), EXOC7 (Q9UPT5), EXOC8 (Q8IYI6), EXOSC1 (Q9Y3B2), EXOSC2 (Q13868), EXOSC3 (Q9NQT5), EXOSC4 (Q9NPD3), EXOSC5 (Q9NQT4), EXOSC6 (Q5RKV6), EXOSC7 (Q15024), EXOSC8 (Q96B26), EXOSC9 (Q06265), EXTL3 (Q43909), EYA3 (Q99504), EZR (P15311), F3 (P13726), F8 (P00451), F8A1 (P23610), FABP5 (Q01469), FABP7 (O15540), FADD (Q13158), FAF1 (Q9UNN5), FAH (P16930), FAHD2A (Q96GK7), FAM114A2 (Q9NRY5), FAM115A (Q9Y4C2), FAM120A (Q9NZB2), FAM125A (Q96EY5), FAM127A (A6ZKI3), FAM129B (Q96TA1), FAM136A (Q96C01), FAM168A (Q92567), FAM175B (Q15018), FAM188A (Q9H8M7), FAM3A (P98173), FAM3C (Q92520), FAM45B (Q6NSW5), FAM49B (Q9NUQ9), FAM82B (Q96DB5), FAM84B (Q96KN1), FAM98A (Q8NCA5), FAM98B (Q52LJ0), FARP1 (Q9Y4F1), FARP2 (O94887), FARSA (Q9Y285), FARSB (Q9NSD9), FASN (P49327), FAT1 (Q14517), FBL (P22087), FBLN2 (P98095), FBN1 (P35555), FBN2 (P35556), FBXL18 (Q96ME1), FBXO21 (O94952), FBXO22 (Q8NEZ5), FDFT1 (P37268), FDPS (P14324), FEN1 (P39748), FERMT1 (Q9BQL6), FERMT2 (Q96AC1), FGF1 (P05230), FGFRL1 (Q8N441), FGGY (Q96C11), FH (P07954), FHL1 (Q13642), FHL2 (Q14192), FHL3 (Q13643), FIS1 (Q9Y3D6), FKBP1A (P62942), FKBP3 (Q00688), FKBP4 (Q02790), FKBP5 (Q13451), FLII (Q13045), FLNA (P21333), FLNB (O75369), FLNC (Q14315), FLOT1 (O75955), FMNL2 (Q96PY5), FN3K (Q9H479), FN3KRP (Q9HA64), FNTA (P49354), FNTB (P49356), FOLR1 (P15328), FREM2 (Q5SZK8), FRMD8 (Q9BZ67), FSCN1 (Q16658), FSD1 (Q9BTV5), FTH1 (P02794), FTL (P02792), FTO (Q9C0B1), FTSJD2 (Q8N1G2), FUBP1 (Q96AE4), FUCA2 (Q9BTY2), FUK (Q8N0W3), FXR1 (P51114), G3BP1 (Q13283), G3BP2 (Q9UN86), G6PD (P11413), |

(continued)

| |
|---|
| GAA (P10253), GALK1 (P51570), GALK2 (Q01415), GALNT1 (Q10472), GALNT2 (Q10471), GANAB (Q14697), GAP43 (P17677), GAPDH (P04406), GAPVD1 (Q14C86), GAR1 (Q9NY12), GARS (P41250), GART (P22102), GATSL2 (A6NHX0), GBA (P04062), GBE1 (Q04446), GCLM (P48507), GCN1L1 (Q92616), GDI1 (P31150), GDI2 (P50395), GEMIN5 (Q8TEQ6), GEMIN6 (Q8WXD5), GET4 (Q7L5D6), GFAP (P14136), GFPT1 (Q06210), GFPT2 (O94808), GGCT (O75228), GGPS1 (O95749), GINS1 (Q14691), GINS4 (Q9BRT9), GIPC1 (O14908), GIT1 (Q9Y2X7), GLA (P06280), GLB1 (P16278), GLB1L2 (Q8IW92), GLG1 (Q92896), GLIPR2 (Q9H4G4), GLMN (Q92990), GLO1 (Q04760), GLOD4 (Q9HC38), GLRX (P35754), GLRX3 (O76003), GLT25D1 (Q8NBJ5), GLTP (Q9NZD2), GLTPD1 (Q5TA50), GLUD1 (P00367), GLUL (P15104), GMDS (O60547), GMFB (P60983), GMPPA (Q96IJ6), GMPPB (Q9Y5P6), GMPR (P36959), GMPR2 (Q9P2T1), GMPS (P49915), GNA11 (P29992), GNA13 (014344), GNAI2 (P04899), GNAI3 (P08754), GNAQ (P50148), GNAS (Q5JWF2), GNB1 (P62873), GNB2 (P62879), GNB2L1 (P63244), GNB4 (Q9HAV0), GNE (Q9Y223), GNG12 (Q9UBI6), GNG4 (P50150), GNG5 (P63218), GNPDA1 (P46926), GNPNAT1 (Q96EK6), GOLGA7 (Q7Z5G4), GOLGB1 (Q14789), GOLIM4 (000461), GOLM1 (Q8NBJ4), GOLPH3 (Q9H4A6), GORASP2 (Q9H8Y8), GPC1 (P35052), GPC4 (O75487), GPC6 (Q9Y625), GPD1L (Q8N335), GPI (P06744), GPLD1 (P80108), GPM6A (P51674), GPM6B (Q13491), GPN1 (Q9HCN4), GPR56 (Q9Y653), GPS1 (Q13098), GPX1 (P07203), GPX4 (P36969), GRB2 (P62993), GRHPR (Q9UBQ7), GRP (Q3ZCW2), GRPEL1 (Q9HAV7), GRWD1 (Q9BQ67), GSK3A (P49840), GSK3B (P49841), GSN (P06396), GSPT1 (P15170), GSS (P48637), GSTK1 (Q9Y2Q3), GSTM2 (P28161), GSTM3 (P21266), GSTM4 (Q03013), GSTO1 (P78417), GSTP1 (P09211), GSTT2 (P0CG29), GSTZ1 (O43708), GTF2F2 (P13984), GTF2H2 (Q13888), GTF2I (P78347), GTF3C1 (Q12789), GTF3C2 (Q8WUA4), GTF3C4 (Q9UKN8), GTPBP1 (000178), GUK1 (Q16774), GYG1 (P46976), GYS1 (P13807), H2AFY (O75367), H2AFZ (P0C0S5), HADH (Q16836), HAGH (Q16775), HARS (P12081), HAT1 (014929), HAUS3 (Q68CZ6), HAUS4 (Q9H6D7), HBA1 (P69905), HBB (P68871), HCFC1 (P51610), HDAC1 (Q13547), HDAC2 (Q92769), HDAC3 (O15379), HDHD2 (Q9H0R4), HDLBP (Q00341), HEATR1 (Q9H583), HEATR2 (Q86Y56), HEBP1 (Q9NRV9), HECTD3 (Q5T447), HEG1 (Q9ULI3), HELZ (P42694), HERC4 (Q5GLZ8), HEXB (P07686), HGS (O14964), HHIP (Q96QV1), HIBCH (Q6NVY1), HIF1AN (Q9NWT6), HINT1 (P49773), HIP1R (O75146), HIST1H1B (P16401), HIST1H1C (P16403), HIST1H2BM (Q99879), HIST1H2BO (P23527), HIST1H4A (P62805), HIST2H2AA3 (Q6FI13), HIST2H3A (Q71DI3), HK1 (P19367), HK2 (P52789), HLA-A (P30443), HLA-A (P01892), HLCS (P50747), HMGA1 (P17096), HMGB1 (P09429), HMGCL (P35914), HMGCS1 (Q01581), HMGN2 (P05204), HNRNPA1 (P09651), HNRNPA2B1 (P22626), HNRNPA3 (P51991), HNRNPAB (Q99729), HNRNPC (P07910), HNRNPD (Q14103), HNRNPF (P52597), HNRNPH1 (P31943), HNRNPH2 (P55795), HNRNPH3 (P31942), HNRNPK (P61978), HNRNPL (P14866), HNRNPM (P52272), HNRNPR (O43390), |

(continued)

| |
|---|
| HNRNPU (Q00839), HNRNPUL2 (Q1KMD3), HNRPDL (O14979), HNRPLL (Q8WW9), HOOK3 (Q86VS8), HP (P00738), HP1BP3 (Q5SSJ5), HPCAL1 (P37235), HPRT1 (P00492), HPX (P02790), HRAS (P01112), HS6ST2 (Q96MM7), HSD17B10 (Q99714), HSD17B4 (P51659), HSP90AA1 (P07900), HSP90AB1 (P08238), HSP90B1 (P14625), HSPA12A (O43301), HSPA14 (Q0VDF9), HSPA1A (P08107), HSPA2 (P54652), HSPA4 (P34932), HSPA4L (O95757), HSPA5 (P11021), HSPA8 (P11142), HSPA9 (P38646), HSPB1 (P04792), HSPB11 (Q9Y547), HSPBP1 (Q9NZL4), HSPD1 (P10809), HSPE1 (P61604), HSPG2 (P98160), HSPH1 (Q92598), HTATIP2 (Q9BUP3), HTRA1 (Q92743), HTT (P42858), HUWE1 (Q7Z6Z7), HYOU1 (Q9Y4L1), IARS (P41252), ICAM1 (P05362), IDE (P14735), IDH1 (O75874), IDH2 (P48735), IDI1 (Q13907), IDUA (P35475), IFI16 (Q16666), IFI35 (P80217), IFIT5 (Q13325), IFITM3 (Q01628), IGF1R (P08069), IGF2BP2 (Q9Y6M1), IGF2BP3 (O00425), IGF2R (P11717), IGFBP3 (P17936), IGSF3 (O75054), IGSF8 (Q969P0), IKBKAP (O95163), IL1RAP (Q9NPH3), ILF2 (Q12905), ILF3 (Q12906), ILK (Q13418), ILKAP (Q9H0C8), IMP4 (Q96G21), IMPA1 (P29218), IMPA2 (O14732), IMPAD1 (Q9NX62), IMPDH2 (P12268), INF2 (Q27J81), INPP1 (P49441), INPPL1 (O15357), INTS1 (Q8N201), INTS10 (Q9NVR2), INTS3 (Q68E01), INTS5 (Q6P9B9), IPO11 (Q9UI26), IPO13 (O94829), IPO4 (Q8TEX9), IPO5 (O00410), IPO7 (O95373), IPO8 (O15397), IPO9 (Q96P70), IQGAP1 (P46940), IRF2BP2 (Q7Z5L9), IRF3 (Q14653), IRGQ (Q8WZA9), ISG15 (P05161), ISOC1 (Q96CN7), ISPD (A4D126), ISYNA1 (Q9NPH2), ITFG3 (Q9H0X4), ITGA2 (P17301), ITGA3 (P26006), ITGA4 (P13612), ITGA5 (P08648), ITGA6 (P23229), ITGA7 (O13683), ITGAV (P06756), ITGB1 (P05556), ITGB4 (P16144), ITGB8 (P26012), ITPA (Q9BY32), JAM3 (Q9BX67), JUP (P14923), KARS (Q15046), KBTBD4 (Q9NVX7), KBTBD6 (Q86V97), KCTD12 (Q96CX2), KDM1A (O60341), KEAP1 (O14145), KHDRBS1 (Q07666), KHSRP (Q92945), KIAA0174 (P53990), KIAA0196 (O12768), KIAA0319L (Q8IZA0), KIAA0664 (O75153), KIAA0776 (O94874), KIAA1033 (Q2M389), KIAA1279 (Q96EK5), KIAA1468 (Q9P260), KIAA1598 (A0MZ66), KIAA1797 (Q5VW36), KIAA1967 (Q8N163), KIF1A (Q12756), KIF3A (Q9Y496), KIF5B (P33176), KIF5C (O60282), KLC1 (Q07866), KLC2 (Q9H0B6), KLC4 (Q9NSK0), KLHDC3 (Q9BQ90), KLHL13 (Q9P2N7), KNG1 (P01042), KNTC1 (P50748), KPNA1 (P52294), KPNA2 (P52292), KPNA3 (O00505), KPNA4 (O00629), KPNA6 (O60684), KPNB1 (Q14974), KPRP (Q5T749), KRAS (P01116), KRIT1 (O00522), KRT13 (P13646), KRT14 (P02533), KRT71 (Q3SY84), KTN1 (Q86UP2), L1CAM (P32004), LAGE3 (Q14657), LAMA4 (Q16363), LAMA5 (O15230), LAMB1 (P07942), LAMC1 (P11047), LAMP1 (P11279), LAMP2 (P13473), LANCL1 (O43813), LANCL2 (Q9NS86), LAP3 (P28838), LARP1 (Q6PKG0), LARS (Q9P2J5), LASP1 (Q14847), LCAT (P04180), LCMT1 (Q9UIC8), LDHA (P00338), LDHB (P07195), LDLR (P01130), LEFTY2 (O00292), LEPRE1 (Q32P28), LFNG (Q8NES3), LGALS1 (P09382), LGALS3 (P17931), LGALS3BP (Q08380), LHFP (Q9Y693), LIMA1 (Q9UHB6), LIMS1 (P48059), LIN7C (Q9NUP9), LIPG (Q9Y5X9), LLGL1 (Q15334), LMCD1 (Q9NZU5), LMNA (P02545), |

(continued)

| |
|---|
| LMNB1 (P20700), LOXL4 (Q96JB6), LPL (P06858), LRBA (P50851), LRCH3 (Q96II8), LRG1 (P02750), LRP1 (Q07954), LRRC20 (Q8TCA0), LRRC40 (Q9H9A6), LRRC47 (Q8N1G4), LRRC57 (Q8N9N7), LRSAM1 (Q6UWE0), LRWD1 (Q9UFC0), LSM1 (O15116), LSM12 (Q3MHD2), LSM2 (Q9Y333), LSM3 (P62310), LSM4 (Q9Y4Z0), LSM6 (P62312), LSM7 (Q9UK45), LSS (P48449), LTA4H (P09960), LTBP2 (Q14767), LTBP3 (Q9NS15), LUM (P51884), LYPLA1 (O75608), LYPLA2 (O95372), LYPLAL1 (Q5VWZ2), M6PR (P20645), MACF1 (Q9UPN3), MAD1L1 (Q9Y6D9), MAD2L1 (Q13257), MAEA (Q7L5Y9), MAGEE1 (Q9HCI5), MAGOHB (Q96A72), MALT1 (Q9UDY8), MAN1B1 (Q9UKM7), MAN2A1 (Q16706), MANBA (O00462), MAP1B (P46821), MAP1S (Q66K74), MAP2K1 (Q02750), MAP2K2 (P36507), MAP2K3 (P46734), MAP3K4 (Q9Y6R4), MAP4 (P27816), MAP4K4 (O95819), MAPK1 (P28482), MAPK12 (P53778), MAPK3 (P27361), MAPK9 (P45984), MAPKAPK2 (P49137), MAPKSP1 (Q9UHA4), MAPRE1 (Q15691), MAPRE3 (Q9UPY8), MARCKS (P29966), MARCKSL1 (P49006), MARK2 (Q7KZI7), MARS (P56192), MAT2A (P31153), MAT2B (Q9NZL9), MATR3 (P43243), MBD3 (O95983), MBNL1 (Q9NR56), MCAM (P43121), MCAT (Q8IVS2), MCM2 (P49736), MCM3 (P25205), MCM4 (P33991), MCM5 (P33992), MCM6 (Q14566), MCM7 (P33993), MCTS1 (Q9ULC4), MDH1 (P40925), MDH2 (P40926), MDK (P21741), MDN1 (Q9NU22), ME1 (P48163), ME2 (P23368), MED1 (Q15648), MED16 (Q9Y2X0), MED17 (Q9NVC6), MED18 (Q9BUE0), MED20 (Q9H944), MED22 (Q15528), MED23 (Q9ULK4), MED27 (Q6P2C8), MED30 (Q96HR3), MED31 (Q9Y3C7), MEMO1 (Q9Y316), MERIT40 (Q9NWV8), METAP1 (P53582), METAP2 (P50579), METT10D (Q86W50), METTL1 (Q9UBP6), METTL11A (Q9BV86), METTL13 (Q8N6R0), METTL2B (Q6P1Q9), METTL5 (Q9NRN9), MFAP2 (P55001), MFAP4 (P55083), MFGE8 (Q08431), MFI2 (P08582), MGAT4B (Q9UQ53), MGAT5 (Q09328), MGEA5 (O60502), MICAL1 (Q8TDZ2), MIF (P14174), MIF4GD (A9UHW6), MINA (Q8IUF8), MINK1 (Q8N4C8), MIOS (Q9NXC5), MIS12 (Q9H081), MKLN1 (Q9UL63), MLTK (Q9NYL2), MMP14 (P50281), MMS19 (Q96T76), MOB2 (Q70IA6), MOBKL1B (Q9H8S9), MOBKL2A (Q96BX8), MOBKL3 (Q9Y3A3), MOCS2 (O96033), MON2 (Q7Z3U7), MORC2 (Q9Y6X9), MOV10 (Q9HCE1), MOXD1 (Q6UVY6), MPI (P34949), MPP6 (Q9NZW5), MPRIP (Q6WCQ1), MPST (P25325), MPZL1 (O95297), MRC2 (Q9UBG0), MRI1 (Q9BV20), MRTO4 (Q9UKD2), MSH2 (P43246), MSN (P26038), MSTO1 (Q9BUK6), MTA1 (Q13330), MTA2 (O94776), MTAP (Q13126), MTHFD1 (P11586), MTHFS (P49914), MTM1 (Q13496), MTMR1 (Q13613), MTMR6 (Q9Y217), MTMR9 (Q96QG7), MTOR (P42345), MTPN (P58546), MTR (Q99707), MVD (P53602), MVK (Q03426), MVP (Q14764), MYADM (Q96S97), MYBBP1A (Q9BQG0), MYCBP (Q99417), MYD88 (Q99836), MYH10 (P35580), MYH9 (P35579), MYL12B (O14950), MYL6 (P60660), MYO18A (Q92614), MYO1B (O43795), MYO1C (O00159), MYO1E (Q12965), MYO6 (Q9UM54), MYOF (Q9NZM1), MZT1 (Q08AG7), NAA10 (P41227), NAA15 (Q9BXJ9), NAA16 (Q6N069), NAA20 (P61599), NAA30 (Q147X3), NAA38 (O95777), NAA50 (Q9GZZ1), NACA (Q13765), NADSYN1 (Q6IA69), NAE1 (Q13564), NAGK (Q9UJ70), |

(continued)

| |
|---|
| NAGLU (P54802), NAMPT (P43490), NANS (Q9NR45), NAP1L1 (P55209), NAP1L4 (Q99733), NAPA (P54920), NAPG (Q99747), NAPRT1 (Q6XQN6), NARS (O43776), NASP (P49321), NCAM1 (P13591), NCAPD2 (Q15021), NCAPG (Q9BPX3), NCBP1 (Q09161), NCBP2 (P52298), NCDN (Q9UBB6), NCKAP1 (Q9Y2A7), NCKIPSD (Q9NZQ3), NCL (P19338), NCS1 (P62166), NCSTN (Q92542), NDRG3 (Q9UGV2), NDRG4 (Q9ULP0), NDUFA2 (O43678), NDUFA3 (095167), NDUFA5 (Q16718), NDUFAB1 (014561), NDUFS6 (O75380), NEDD4L (Q96PU5), NEFL (P07196), NEK9 (Q8TD19), NES (P48681), NF1 (P21359), NFIC (P08651), NFIX (014938), NFKB2 (Q00653), NHLRC2 (Q8NBF2), NHP2L1 (P55769), NID1 (P14543), NIP7 (Q9Y221), NIT1 (Q86X76), NIT2 (Q9NQR4), NLE1 (Q9NVX2), NLGN4X (Q8N0W4), NLN (Q9BYT8), NMD3 (Q96D46), NME1 (P15531), NME2 (P22392), NME3 (Q13232), NME7 (Q9Y5B8), NMT1 (P30419), NNMT (P40261), NOB1 (Q9ULX3), NOL11 (Q9H8H0), NOL6 (Q9H6R4), NOMO2 (Q5JPE7), NONO (Q15233), NOP10 (Q9NPE3), NOP2 (P46087), NOTCH1 (P46531), NOTCH3 (Q9UM47), NOVA2 (Q9UNW9), NPEPPS (P55786), NPLOC4 (Q8TAT6), NPM1 (P06748), NPM3 (O75607), NPTN (Q9Y639), NPW (Q8N729), NQO1 (P15559), NQO2 (P16083), NR2C2AP (Q86WQ0), NRAS (P01111), NRBP1 (Q9UHY1), NRBP2 (Q9NSY0), NRD1 (O43847), NRP2 (O60462), NSF (P46459), NSMAF (Q92636), NSMCE1 (Q8WV22), NSUN2 (Q08J23), NT5C (Q8TCD5), NT5DC1 (Q5TFE4), NTN1 (O95631), NUBP1 (P53384), NUBP2 (Q9Y5Y2), NUCB1 (Q02818), NUDC (Q9Y266), NUDCD1 (Q96RS6), NUDCD2 (Q8WVJ2), NUDT1 (P36639), NUDT10 (Q8NFP7), NUDT12 (Q9BQG2), NUDT16 (Q96DE0), NUDT16L1 (Q9BRJ7), NUDT2 (P50583), NUDT21 (O43809), NUDT4 (Q9NZJ9), NUDT5 (Q9UKK9), NUMA1 (Q14980), NUP188 (Q5SRE5), NUP37 (Q8NFH4), NUP43 (Q8NFH3), NUP54 (Q7Z3B4), NUP88 (Q99567), NUP93 (Q8N1F7), NUTF2 (P61970), NXN (Q6DKJ4), OBFC2B (Q9BQ15), OCRL (Q01968), ODZ2 (Q9NT68), ODZ3 (Q9P273), OGFOD1 (Q8N543), OGT (015294), OLA1 (Q9NTK5), OLFML3 (Q9NRN5), OPA1 (060313), OPLAH (014841), OSBP (P22059), OSBPL1A (Q9BXW6), OSGEP (Q9NPF4), OTUB1 (Q96FW1), OVCA2 (Q8WZ82), OXCT1 (P55809), OXSR1 (O95747), P4HB (P07237), PA2G4 (Q9UQ80), PAAF1 (Q9BRP4), PABPC1 (P11940), PABPC4 (Q13310), PABPN1 (Q86U42), PACSIN2 (Q9UNF0), PACSIN3 (Q9UKS6), PAF1 (Q8N7H5), PAFAH1B1 (P43034), PAFAH1B2 (P68402), PAFAH1B3 (Q15102), PAICS (P22234), PAIP1 (Q9H074), PAK2 (Q13177), PALD (Q9ULE6), PALLD (Q8WX93), PANK4 (Q9NVE7), PAPOLA (P51003), PAPSS1 (O43252), PARF (Q3YEC7), PARK7 (Q99497), PARN (O95453), PARP1 (P09874), PARP4 (Q9UKK3), PARVA (Q9NVD7), PBK (Q96KB5), PBLD (P30039), PCBP1 (Q15365), PCBP2 (Q15366), PCDHB2 (Q9Y5E7), PCDHGB4 (Q9UN71), PCDHGC3 (Q9UN70), PCID2 (Q5JVF3), PCMT1 (P22061), PCNA (P12004), PCOLCE2 (Q9UKZ9), PCYT2 (Q99447), PDCD10 (Q9BUL8), PDCD2L (Q9BRP1), PDCD4 (Q53EL6), PDCD5 (014737), PDCD6 (O75340), PDCD6IP (Q8WUM4), PDCL3 (Q9H2J4), PDDC1 (Q8NB37), PDE12 (Q6L8Q7), PDE6D (O43924), PDGFC (Q9NRA1), PDIA3 (P30101), PDIA6 (Q15084), PDLIM1 (000151), PDLIM4 (P50479), |

(continued)

| |
|---|
| PDLIM5 (Q96HC4), PDLIM7 (Q9NR12), PDRG1 (Q9NUG6), PDRO (Q6IAA8), PDS5A (Q29RF7), PDXK (O00764), PDXP (Q96GD0), PEA15 (Q15121), PEBP1 (P30086), PEF1 (Q9UBV8), PELO (Q9BRX2), PELP1 (Q8IZL8), PEPD (P12955), PFAS (O15067), PFDN2 (Q9UHV9), PFDN5 (Q99471), PFDN6 (O15212), PFKL (P17858), PFKM (P08237), PFKP (Q01813), PFN1 (P07737), PFN2 (P35080), PGAM1 (P18669), PGAM5 (Q96HS1), PGD (P52209), PGGT1B (P53609), PGK1 (P00558), PGLS (O95336), PGLYRP2 (Q96PD5), PGM1 (P36871), PGM2L1 (Q6PCE3), PGM3 (O95394), PGP (A6NDG6), PGRMC1 (O00264), PGRMC2 (O15173), PHF5A (Q7RTV0), PHGDH (O43175), PHKB (Q93100), PHLDA3 (Q9Y5J5), PHPT1 (Q9NRX4), PIK3CB (P42338), PIK3R4 (Q99570), PIN1 (Q13526), PIP4K2A (P48426), PIPOX (Q9P0Z9), PITPNB (P48739), PKM2 (P14618), PKP1 (Q13835), PLAA (Q9Y263), PLCD3 (Q8N3E9), PLCG1 (P19174), PLD3 (Q8IV08), PLEC (Q15149), PLEKHB2 (Q96CS7), PLIN3 (O60664), PLOD1 (Q02809), PLOD2 (O00469), PLOD3 (O60568), PLRG1 (O43660), PLS1 (O14651), PLS3 (P13797), PLSCR3 (Q9NRY6), PLTP (P55058), PLXNA1 (Q9UIW2), PLXNB2 (O15031), PLXND1 (Q9Y4D7), PM20D2 (Q8IYS1), PML (P29590), PMM2 (O15305), PMPCA (Q10713), PMPCB (O75439), PMVK (Q15126), PNMA2 (Q9UL42), PNO1 (Q9NRX1), PNP (P00491), PODXL (O00592), POLA1 (P09884), POLD1 (P28340), POLD2 (P49005), POLE3 (Q9NRF9), POLR1A (O95602), POLR1B (Q9H9Y6), POLR1C (O15160), POLR1D (Q9Y2S0), POLR1E (Q9GZS1), POLR2A (P24928), POLR2B (P30876), POLR2C (P19387), POLR2E (P19388), POLR2G (P62487), POLR2H (P52434), POLR2J (P52435), POLR2L (P62875), POLR3A (O14802), POLR3B (Q9NW08), POLR3C (Q9BUI4), POLR3F (Q9H1D9), POP1 (Q99575), POP4 (O95707), POP5 (Q969H6), POP7 (O75817), PPA1 (Q15181), PPA2 (Q9H2U2), PPAT (Q06203), PPCS (Q9HAB8), PPIA (P62937), PPIB (P23284), PPID (Q08752), PPIF (P30405), PPIH (O43447), PPIL1 (Q9Y3C6), PPM1A (P35813), PPM1F (P49593), PPM1G (O15355), PPME1 (Q9Y570), PPP1CA (P62136), PPP1CB (P62140), PPP1CC (P36873), PPP1R7 (Q15435), PPP1R8 (Q12972), PPP2CA (P67775), PPP2CB (P62714), PPP2R1A (P30153), PPP2R2A (P63151), PPP2R4 (Q15257), PPP2R5C (O13362), PPP2R5D (O14738), PPP2R5E (O16537), PPP3CA (Q08209), PPP4C (P60510), PPP4R1 (Q8TF05), PPP5C (P53041), PPP6C (O00743), PPP6R3 (Q5H9R7), PPPDE2 (Q6ICB0), PPT1 (P50897), PPWD1 (Q96BP3), PRCP (P42785), PRDX1 (Q06830), PRDX2 (P32119), PRDX3 (P30048), PRDX5 (P30044), PRDX6 (P30041), PREP (P48147), PREPL (Q4J6C6), PRIM1 (P49642), PRIM2 (P49643), PRKACA (P17612), PRKACB (P22694), PRKAG1 (P54619), PRKAR1A (P10644), PRKAR2A (P13861), PRKAR2B (P31323), PRKDC (P78527), PRMT1 (Q99873), PRMT3 (O60678), PRMT5 (O14744), PROM1 (O43490), PROSC (O94903), PRPF19 (Q9UMS4), PRPF31 (Q8WWY3), PRPF4 (O43172), PRPF4B (O13523), PRPF8 (Q6P2Q9), PRPS1 (P60891), PRPS2 (P11908), PRPSAP1 (O14558), PRPSAP2 (O60256), PRSS23 (O95084), PRTFDC1 (Q9NRG1), PSAT1 (Q9Y617), PSMA1 (P25786), PSMA2 (P25787), PSMA3 (P25788), PSMA4 (P25789), PSMA5 (P28066), PSMA6 (P60900), |

(continued)

| |
|---|
| PSMA7 (O14818), PSMB1 (P20618), PSMB2 (P49721), PSMB3 (P49720), PSMB4 (P28070), PSMB5 (P28074), PSMB6 (P28072), PSMB7 (Q99436), PSMB8 (P28062), PSMC1 (P62191), PSMC2 (P35998), PSMC3 (P17980), PSMC4 (P43686), PSMC5 (P62195), PSMC6 (P62333), PSMD1 (Q99460), PSMD10 (O75832), PSMD11 (000231), PSMD12 (O00232), PSMD13 (Q9UNM6), PSMD14 (O00487), PSMD2 (Q13200), PSMD3 (O43242), PSMD4 (P55036), PSMD5 (Q16401), PSMD6 (Q15008), PSMD7 (P51665), PSMD8 (P48556), PSMD9 (O00233), PSME1 (Q06323), PSME2 (Q9UL46), PSME3 (P61289), PSME4 (Q14997), PSMF1 (Q92530), PSMG1 (O95456), PSMG2 (Q969U7), PSMG3 (Q9BT73), PSPC1 (Q8WXF1), PSPH (P78330), PTBP1 (P26599), PTGES3 (Q15185), PTGFRN (Q9P2B2), PTGR1 (Q14914), PTGR2 (Q8N8N7), PTK2 (Q05397), PTK7 (Q13308), PTN (P21246), PTP4A1 (Q93096), PTPN1 (P18031), PTPN11 (Q06124), PTPN23 (Q9H3S7), PTPRA (P18433), PTPRG (P23470), PTPRZ1 (P23471), PUF60 (Q9UHX1), PUM1 (Q14671), PURB (Q96QR8), PUS7 (Q96PZ0), PVR (P15151), PWP1 (Q13610), PXDN (Q92626), PXK (Q7Z7A4), PYCR1 (P32322), PYCRL (Q53H96), PYGB (P11216), PYGL (P06737), OARS (P47897), QDPR (P09417), QKI (Q96PU8), QRICH1 (Q2TAL8), QSOX2 (Q6ZRP7), QTRT1 (Q9BXR0), RAB10 (P61026), RAB11A (P62491), RAB11FIP1 (Q6WKZ4), RAB12 (Q6IQ22), RAB13 (P51153), RAB14 (P61106), RAB18 (Q9NP72), RAB1A (P62820), RAB1B (Q9H0U4), RAB21 (Q9UL25), RAB22A (Q9UL26), RAB23 (Q9ULC3), RAB27A (P51159), RAB2A (P61019), RAB34 (Q9BZG1), RAB35 (Q15286), RAB3A (P20336), RAB3GAP1 (Q15042), RAB3GAP2 (Q9H2M9), RAB4A (P20338), RAB5A (P20339), RAB5B (P61020), RAB5C (P51148), RAB6A (P20340), RAB6B (Q9NRW1), RAB7A (P51149), RAB8A (P61006), RAB8B (Q92930), RABAC1 (Q9UI14), RABGAP1 (Q9Y3P9), RABGGTA (Q92696), RABGGTB (P53611), RABIF (P47224), RAC1 (P63000), RAD1 (O60671), RAD50 (Q92878), RAE1 (P78406), RAI14 (Q9P0K7), RALA (P11233), RALB (P11234), RALY (Q9UKM9), RAN (P62826), RANBP1 (P43487), RANBP2 (P49792), RANBP6 (O60518), RANBP9 (Q96S59), RANGAP1 (P46060), RAP1A (P62834), RAP1B (P61224), RAP1GDS1 (P52306), RAP2B (P61225), RARS (P54136), RASA1 (P20936), RBBP4 (Q09028), RBBP5 (O15291), RBBP7 (O16576), RBBP9 (O75884), RBM12 (Q9NTZ6), RBM15 (Q96T37), RBM17 (Q96I25), RBM22 (Q9NW64), RBM4 (Q9BWF3), RBMX (P38159), RBP1 (P09455), RBPJ (Q06330), RBX1 (P62877), RCC1 (P18754), RCC2 (Q9P258), RCL (O43598), RDX (P35241), RECQL (P46063), REEP5 (Q00765), REEP6 (Q96HR9), REPS1 (Q96D71), RFC4 (P35249), RFC5 (P40937), RFTN1 (Q14699), RHEB (Q15382), RHOA (P61586), RHOB (P62745), RHOC (P08134), RHOF (Q9HBH0), RHOG (P84095), RIC8A (Q9NPQ8), RMND5A (Q9H871), RNASEH2A (O75792), RNASEH2C (Q8TDP1), RNF123 (Q5XPI4), RNF20 (Q5VTR2), RNF213 (Q63HN8), RNF7 (Q9UBF6), RNGTT (O60942), RNH1 (P13489), RNMT (O43148), RNPEP (Q9H4A4), ROBLD3 (Q9Y2Q5), ROCK1 (Q13464), ROCK2 (O75116), ROR1 (Q01973), RP2 (O75695), RPA1 (P27694), RPA2 (P15927), RPA3 (P35244), RPE (Q96AT9), RPF2 (Q9H7B2), RPL10 (P27635), RPL10A (P62906), RPL11 (P62913), RPL12 (P30050), |

(continued)

| |
|---|
| RPL13 (P26373), RPL13A (P40429), RPL14 (P50914), RPL15 (P61313), RPL17 (P18621), RPL18 (Q07020), RPL18A (Q02543), RPL19 (P84098), RPL21 (P46778), RPL22 (P35268), RPL22L1 (Q6P5R6), RPL23 (P62829), RPL23A (P62750), RPL24 (P83731), RPL26 (P61254), RPL27 (P61353), RPL27A (P46776), RPL28 (P46779), RPL3 (P39023), RPL30 (P62888), RPL31 (P62899), RPL32 (P62910), RPL34 (P49207), RPL35 (P42766), RPL35A (P18077), RPL36 (Q9Y3U8), RPL36A (P83881), RPL36AL (Q969Q0), RPL37A (P61513), RPL38 (P63173), RPL4 (P36578), RPL5 (P46777), RPL6 (Q02878), RPL7 (P18124), RPL7A (P62424), RPL8 (P62917), RPL9 (P32969), RPLP0 (P05388), RPLP1 (P05386), RPLP2 (P05387), RPP30 (P78346), RPP40 (075818), RPRD1A (Q96P16), RPS10 (P46783), RPS11 (P62280), RPS12 (P25398), RPS13 (P62277), RPS14 (P62263), RPS15 (P62841), RPS15A (P62244), RPS16 (P62249), RPS17 (P08708), RPS18 (P62269), RPS19 (P39019), RPS2 (P15880), RPS20 (P60866), RPS21 (P63220), RPS23 (P62266), RPS24 (P62847), RPS25 (P62851), RPS26 (P62854), RPS27 (P42677), RPS27A (P62979), RPS27L (Q71UM5), RPS28 (P62857), RPS29 (P62273), RPS3 (P23396), RPS3A (P61247), RPS4X (P62701), RPS4Y1 (P22090), RPS5 (P46782), RPS6 (P62753), RPS6KA3 (P51812), RPS7 (P62081), RPS8 (P62241), RPS9 (P46781), RPSA (P08865), RQCD1 (Q92600), RRAGA (Q7L523), RRAS (P10301), RRAS2 (P62070), RRBP1 (Q9P2E9), RRM1 (P23921), RRM2 (P31350), RRM2B (Q7LG56), RRP12 (Q5JTH9), RRP9 (043818), RSL1D1 (076021), RSU1 (Q15404), RTCD1 (O00442), RTN3 (095197), RTN4 (Q9NQC3), RUVBL1 (Q9Y265), RUVBL2 (Q9Y230), RWDD2B (P57060), S100A10 (P60903), S100A11 (P31949), S100A13 (Q99584), S100A16 (Q96FQ6), S100A4 (P26447), S100A6 (P06703), S100A8 (P05109), SAAL1 (Q96ER3), SACS (Q9NZJ4), SAE1 (Q9UBE0), SAFB2 (Q14151), SAMHD1 (Q9Y3Z3), SAP18 (O00422), SAR1A (Q9NR31), SARM1 (Q6SZW1), SARS (P49591), SART3 (Q15020), SBDS (Q9Y3A5), SBF1 (O95248), SCARB1 (Q8WTV0), SCARB2 (Q14108), SCFD1 (Q8WVM8), SCLY (Q96I15), SCP2 (P22307), SCPEP1 (Q9HB40), SCRG1 (O75711), SCRIB (Q14160), SCRN1 (Q12765), SCRN2 (Q96FV2), SCYL1 (Q96KG9), SCYL2 (Q6P3W7), SDC1 (P18827), SDC2 (P34741), SDCBP (O00560), SDF4 (Q9BRK5), SDHA (P31040), SDK1 (Q7Z5N4), SDSL (Q96GA7), SEC11A (P67812), SEC13 (P55735), SEC22B (O75396), SEC23A (Q15436), SEC23B (Q15437), SEC231P (Q9Y6Y8), SEC24A (O95486), SEC24B (O95487), SEC24C (P53992), SEC24D (O94855), SEC31A (O94979), SEH1L (Q96EE3), SELH (Q8IZQ5), SEMA3A (Q14563), SEPSECS (Q9HD40), 40787 (Q9NVA2), 37500 (Q15019), 38596 (Q99719), 39326 (Q16181), 39692 (Q92599), 40057 (Q9UHD8), SERBP1 (Q8NC51), SERPINA1 (P01009), SERPINA3 (P01011), SERPINA7 (P05543), SERPINB6 (P35237), SERPINB8 (P50452), SERPINE1 (P05121), SERPINE2 (P07093), SERPING1 (P05155), SERPINH1 (P50454), SETD3 (Q86TU7), SETD7 (Q8WTS6), SF3A1 (Q15459), SF3A2 (Q15428), SF3A3 (Q12874), SF3B1 (O75533), SF3B14 (Q9Y3B4), SF3B2 (Q13435), SF3B3 (Q15393), SF3B4 (Q15427), SF3B5 (Q9BWJ5), SFPQ (P23246), SFRP4 (Q6FHJ7), SGTA (O43765), SH3BP4 (Q9P0V3), |

(continued)

| |
|---|
| SH3GL1 (Q99961), SH3GLB1 (Q9Y371), SHBG (P04278), SHC1 (P29353), SHMT1 (P34896), SHMT2 (P34897), SHOC2 (Q9UQ13), SHPK (Q9UHJ6), SKIV2L (Q15477), SKIV2L2 (P42285), SKP1 (P63208), SLC16A1 (P53985), SLC1A3 (P43003), SLC1A5 (Q15758), SLC29A1 (Q99808), SLC2A1 (P11166), SLC31A1 (015431), SLC3A2 (P08195), SLC44A2 (Q8IWA5), SLC5A3 (P53794), SLC7A5 (Q01650), SLC9A3R1 (O14745), SLC9A3R2 (Q15599), SLIRP (Q9GZT3), SMAD4 (Q13485), SMARCA4 (P51532), SMARCA5 (O60264), SMARCC1 (Q92922), SMARCC2 (Q8TAQ2), SMARCD1 (Q96GM5), SMARCD2 (Q92925), SMARCE1 (Q969G3), SMC1A (Q14683), SMC2 (O95347), SMC3 (Q9UQE7), SMC4 (Q9NTJ3), SMC5 (Q8IY18), SMC6 (Q96SB8), SMCHD1 (A6NHR9), SMEK1 (Q6IN85), SMS (P52788), SMU1 (Q2TAY7), SMYD5 (Q6GMV2), SNAP23 (000161), SNAPIN (O95295), SND1 (Q7KZF4), SNF8 (Q96H20), SNRNP200 (O75643), SNRNP40 (Q96DI7), SNRPA1 (P09661), SNRPB (P14678), SNRPD1 (P62314), SNRPD2 (P62316), SNRPD3 (P62318), SNRPE (P62304), SNRPF (P62306), SNRPG (P62308), SNTB1 (Q13884), SNUPN (095149), SNX1 (Q13596), SNX12 (Q9UMY4), SNX17 (Q15036), SNX18 (Q96RF0), SNX2 (O60749), SNX27 (Q96L92), SNX3 (O60493), SNX5 (Q9Y5X3), SNX6 (Q9UNH7), SNX8 (Q9Y5X2), SNX9 (Q9Y5X1), SOD1 (P00441), SORD (Q00796), SORT1 (Q99523), SPAG9 (O60271), SPC24 (Q8NBT2), SPC25 (Q9HBM1), SPG21 (Q9NZD8), SPR (P35270), SPRYD4 (Q8WW59), SPTAN1 (Q13813), SPTBN1 (Q01082), SPTBN2 (O15020), SRGAP2 (O75044), SRI (P30626), SRM (P19623), SRP14 (P37108), SRP19 (P09132), SRP54 (P61011), SRP68 (Q9UHB9), SRP72 (O76094), SRP9 (P49458), SRPX (P78539), SRPX2 (O60687), SRR (Q9GZT4), SRRT (Q9BXP5), SRSF1 (Q07955), SRSF11 (Q05519), SRSF2 (Q01130), SRSF3 (P84103), SRSF6 (Q13247), SRSF7 (Q16629), SRSF9 (Q13242), SRXN1 (Q9BYN0), SSB (P05455), SSBP1 (Q04837), SSRP1 (Q08945), SSSCA1 (O60232), ST13 (P50502), STAG2 (Q8N3U4), STAM (Q92783), STAMBP (O95630), STAT1 (P42224), STAT3 (P40763), STIP1 (P31948), STK24 (Q9Y6E0), STK25 (O00506), STK38L (Q9Y2H1), STOM (P27105), STON2 (Q8WXE9), STRAP (Q9Y3F4), STUB1 (Q9UNE7), STX12 (Q86Y82), STX4 (Q12846), STX5 (Q13190), STX7 (O15400), STXBP1 (P61764), STXBP3 (000186), STYX (Q8WUJ0), SUB1 (P53999), SUDS3 (Q9H7L9), SUGT1 (Q9Y2Z0), SUMO1 (P63165), SUPT16H (Q9Y5B9), SUPT4H1 (P63272), SUPT5H (O00267), SUPT6H (Q7KZ85), SVEP1 (Q4LDE5), SWAP70 (Q9UH65), SYMPK (Q92797), SYNCRIP (O60506), SYNE1 (Q8NF91), SYNE2 (Q8WXH0), SYNGR2 (O43760), SYNJ2BP (P57105), TAB1 (Q15750), TAF9 (Q9Y3D8), TAF9 (Q16594), TAGLN (Q01995), TAGLN2 (P37802), TALDO1 (P37837), TARDBP (Q13148), TARS (P26639), TATDN1 (Q6P1N9), TAX1BP3 (O14907), TBC1D13 (Q9NVG8), TBC1D15 (Q8TC07), TBC1D23 (Q9NUY8), TBC1D24 (Q9ULP9), TBC1D4 (O60343), TBC1D9B (Q66K14), TBCA (O75347), TBCB (Q99426), TBCD (Q9BTW9), TBCE (Q15813), TBL1XR1 (Q9BZK7), TCEA1 (P23193), TCEB1 (Q15369), TCEB2 (Q15370), TCERG1 (O14776), TCP1 (P17987), TDP2 (O95551), TEP1 (Q99973), TEX10 (Q9NXF1), TF (P02787), TFCP2 (Q12800), TFG (Q92734), TFRC (P02786), TGFB1 (P01137), |

(continued)

| |
|---|
| TGFB2 (P61812), TGFBI (Q15582), TGM1 (P22735), TH1L (Q8IXH7), THBS1 (P07996), THBS3 (P49746), THG1L (Q9NWX6), THOC2 (Q8NI27), THOC3 (Q96J01), THOC5 (Q13769), THOC6 (Q86W42), THOC7 (Q6I9Y2), THOP1 (P52888), THUMPD1 (Q9NXG2), THY1 (P04216), THYN1 (Q9P016), TIA1 (P31483), TIGAR (Q9NQ88), TIMM13 (Q9Y5L4), TIMM50 (Q3ZCQ8), TIMM8B (Q9Y5J9), TIMM9 (Q9Y5J7), TIMP1 (P01033), TIPRL (O75663), TKT (P29401), TLN1 (Q9Y490), TLN2 (Q9Y4G6), TM9SF2 (Q99805), TM9SF3 (Q9HD45), TMED10 (P49755), TMED2 (Q15363), TMED7 (Q9Y3B3), TMED9 (Q9BVK6), TMEM167A (Q8TBQ9), TMEM2 (Q9UHN6), TMEM50B (P56557), TMEM87A (Q8NBN3), TMOD3 (Q9NYL9), TNC (P24821), TNPO1 (Q92973), TNPO2 (014787), TNPO3 (Q9Y5L0), TOLLIP (Q9H0E2), TOMM20 (Q15388), TOMM22 (Q9NS69), TOMM34 (Q15785), TOMM5 (Q8N4H5), TOMM70A (O94826), TOP1 (P11387), TOP2B (Q02880), TOR1B (O14657), TP53BP1 (Q12888), TP53RK (Q96S44), TPI1 (P60174), TPM3 (P06753), TPM3L (A6NL28), TPM4 (P67936), TPMT (P51580), TPP1 (O14773), TPP2 (P29144), TPR (P12270), TPRG1 L (Q5T0D9), TPRKB (Q9Y3C4), TPT1 (P13693), TRAF2 (Q12933), TRAP1 (Q12931), TRAPPC1 (Q9Y5R8), TRAPPC2L (Q9UL33), TRAPPC3 (O43617), TRAPPC4 (Q9Y296), TRAPPC5 (Q8IUR0), TRAPPC6A (O75865), TRAPPC6B (Q86SZ2), TRIM22 (Q8IYM9), TRIM25 (Q14258), TRIM28 (Q13263), TRIP12 (Q14669), TRIP13 (Q15645), TRIP6 (Q15654), TRMT1 (Q9NXH9), TRMT112 (Q9UI30), TRMT5 (Q32P41), TRMT6 (Q9UJA5), TRMT61A (Q96FX7), TRNT1 (Q96Q11), TROVE2 (P10155), TRRAP (Q9Y4A5), TSG101 (Q99816), TSKU (Q8WUA8), TSPAN14 (Q8NG11), TSPAN4 (014817), TSPAN5 (P62079), TSPAN6 (O43657), TSPAN9 (O75954), TSSC1 (Q53HC9), TSTA3 (Q13630), TTC1 (Q99614), TTC37 (Q6PGP7), TTC38 (Q5R3I4), TTC5 (Q8N0Z6), TTC9C (Q8N5M4), TTL (Q8NG68), TTLL12 (Q14166), TTN (Q8WZ42), TTR (P02766), TTYH1 (Q9H313), TTYH2 (Q9BSA4), TTYH3 (Q9C0H2), TUBA1B (P68363), TUBA1C (Q9BQE3), TUBB (P07437), TUBB2A (Q13885), TUBB2B (Q9BVA1), TUBB2C (P68371), TUBB3 (Q13509), TUBB4 (P04350), TUBB6 (Q9BUF5), TUBG1 (P23258), TUBGCP2 (Q9BSJ2), TUBGCP3 (Q96CW5), TWF1 (Q12792), TWF2 (Q6IBS0), TXN (P10599), TXNDC17 (Q9BRA2), TXNDC9 (O14530), TXNL1 (O43396), TXNL4B (Q9NX01), TXNRD1 (Q16881), TYMS (P04818), U2AF1 (Q01081), U2AF2 (P26368), UAP1 (Q16222), UBA1 (P22314), UBA2 (Q9UBT2), UBA3 (Q8TBC4), UBA5 (Q9GZZ9), UBA6 (A0AVT1), UBE2D1 (P51668), UBE2D3 (P61077), UBE2E1 (P51965), UBE2G2 (P60604), UBE2I (P63279), UBE2J2 (Q8N2K1), UBE2K (P61086), UBE2L3 (P68036), UBE2M (P61081), UBE2N (P61088), UBE2O (Q9C0C9), UBE2V1 (Q13404), UBE2V2 (Q15819), UBE2Z (Q9H832), UBE3A (Q05086), UBE4A (Q14139), UBE4B (O95155), UBL3 (O95164), UBL4A (P11441), UBL5 (Q9BZL1), UBR1 (Q8IWV7), UBR4 (Q5T4S7), UBTD1 (Q9HAC8), UBXN1 (Q04323), UCHL1 (P09936), UCHL3 (P15374), UCHL5 (Q9Y5K5), UCK2 (Q9BZX2), UFC1 (Q9Y3C8), UFD1L (Q92890), UFSP2 (Q9NUQ7), UGDH (O60701), UGP2 (Q16851), UMPS (P11172), UNC119B (A6NIH7), UNC45A (Q9H3U1), UPF1 (Q92900), UPP1 (Q16831), UROD (P06132), |
| UROS (P10746), USO1 (O60763), USP10 (Q14694), USP11 (P51784), USP14 (P54578), USP15 (Q9Y4E8), USP24 (Q9UPU5), USP39 (Q53GS9), USP5 (P45974), USP7 (Q93009), USP9X (Q93008), UTP15 (Q8TED0), UXS1 (Q8NBZ7), UXT (Q9UBK9), VAC14 (Q08AM6), VAMP3 (Q15836), VAMP5 (O95183), VAPA (Q9P0L0), VAPB (O95292), VARS (P26640), VASN (Q6EMK4), VASP (P50552), VAT1 (Q99536), VAV2 (P52735), VBP1 (P61758), VCAN (P13611), VCL (P18206), VCP (P55072), VIM (P08670), VPRBP (Q9Y4B6), VPS11 (Q9H270), VPS13C (Q709C8), VPS16 (Q9H269), VPS18 (Q9P253), VPS24 (Q9Y3E7), VPS25 (Q9BRG1), VPS26A (O75436), VPS26B (Q4G0F5), VPS28 (Q9UK41), VPS29 (Q9UBQ0), VPS33A (Q96AX1), VPS33B (Q9H267), VPS35 (Q96QK1), VPS36 (Q86VN1), VPS37B (Q9H9H4), VPS39 (Q96JC1), VPS45 (Q9NRW7), VPS4A (Q9UN37), VPS4B (075351), VPS53 (Q5VIR6), VRK1 (Q99986), VTA1 (Q9NP79), VWA1 (Q6PCB0), VWA5A (O00534), WARS (P23381), WASF1 (Q92558), WASL (000401), WDFY1 (Q8IWB7), WDR1 (O75083), WDR11 (Q9BZH6), WDR12 (Q9GZL7), WDR18 (Q9BV38), WDR26 (Q9H7D7), WDR33 (Q9C0J8), WDR4 (P57081), WDR43 (Q15061), WDR45L (Q5MNZ6), WDR48 (Q8TAF3), WDR5 (P61964), WDR54 (Q9H977), WDR55 (Q9H6Y2), WDR59 (Q6PJI9), WDR6 (Q9NNW5), WDR61 (Q9GZS3), WDR73 (Q6P4I2), WDR77 (Q9BQA1), WDR82 (Q6UXN9), WDR91 (A4D1P6), WDR92 (Q96MX6), WNK1 (Q9H4A3), XPNPEP1 (Q9NQW7), XPO1 (014980), XPO4 (Q9C0E2), XPO5 (Q9HAV4), XPO6 (Q96QU8), XPO7 (Q9UIA9), XPOT (O43592), XRCC1 (P18887), XRCC5 (P13010), XRCC6 (P12956), XRN2 (Q9H0D6), YARS (P54577), YBX1 (P67809), YEATS4 (095619), YES1 (P07947), YIPF4 (Q9BSR8), YKT6 (015498), YPEL5 (P62699), YRDC (Q86U90), YTHDF2 (Q9Y5A9), YWHAB (P31946), YWHAE (P62258), YWHAG (P61981), YWHAH (Q04917), YWHAQ (P27348), YWHAZ (P63104), ZC3HAV1L (Q96H79), ZCCHC3 (Q9NUD5), ZER1 (Q7Z7L7), ZFPL1 (O95159), ZFR (Q96KR1), ZMAT2 (Q96NC0), ZNF259 (O75312), ZW10 (O43264), ZWILCH (Q9H900), ZYG11 B (Q9C0D3), ZYX (Q15942), ZZEF1 (O43149). |

**Table 20:** 100 most abundant proteins (name and SwissProt accession number) observed in CTX0E03 exosomes

| **Identified proteins** | **Accession number** |
|---|---|
| Actin, cytoplasmic 2 | P63261 |
| Glyceraldehyde-3-phosphate dehydrogenase | P04406 |
| Histone H4 | P62805 |
| Pyruvate kinase isozymes M1/M2 | P14618 |
| Alpha-enolase | P06733 |
| Heat shock protein HSP 90-beta | P08238 |
| Ubiquitin-40S ribosomal protein S27a | P62979 |
| Heat shock cognate 71 kDa protein | P11142 |
| Haptoglobin | P00738 |
| Heat shock protein HSP 90-alpha | P07900 |
| Phosphoglycerate kinase 1 | P00558 |
| Actin, alpha cardiac muscle 1 | P68032 |
| 40S ribosomal protein S3 | P23396 |
| Elongation factor 1-alpha 1 | P68104 |
| GTP-binding nuclear protein Ran | P62826 |
| Histone H2B type 1-M | Q99879 |
| Peptidyl-prolyl cis-trans isomerase A | P62937 |
| Profilin-1 | P07737 |
| Elongation factor 2 | P13639 |
| Fatty acid synthase | P49327 |
| Tubulin beta-2C chain | P68371 |
| Tubulin alpha-1 B chain | P68363 |
| Tubulin beta chain | P07437 |
| 40S ribosomal protein S11 | P62280 |
| Eukaryotic initiation factor 4A-I | P60842 |
| T-complex protein 1 subunit theta | P50990 |
| 14-3-3 protein theta | P27348 |
| 40S ribosomal protein S18 | P62269 |
| Tubulin beta-3 chain | Q13509 |
| T-complex protein 1 subunit beta | P78371 |
| 40S ribosomal protein S16 | P62249 |
| Heat shock 70 kDa protein 1A/1B | P08107 |
| Histone H3.2 | Q71D13 |
| Transketolase | P29401 |
| 40S ribosomal protein SA | P08865 |
| Clusterin | P10909 |
| Fatty acid-binding protein, brain | O15540 |
| Hemopexin | P02790 |

(continued)

| Identified proteins | Accession number |
|---|---|
| T-complex protein 1 subunit gamma | P49368 |
| Tubulin beta-2B chain | Q9BVA1 |
| Adenosylhomocysteinase | P23526 |
| T-complex protein 1 subunit eta | Q99832 |
| 40S ribosomal protein S15a | P62244 |
| T-complex protein 1 subunit delta | P50991 |
| Vimentin | P08670 |
| Guanine nucleotide-binding protein subunit beta-2-like 1 | P63244 |
| Dihydropyrimidinase-related protein 3 | Q14195 |
| Elongation factor 1-gamma | P26641 |
| Fascin | Q16658 |
| Creatine kinase B-type | P12277 |
| X-ray repair cross-complementing protein 5 | P13010 |
| 40S ribosomal protein S2 | P15880 |
| Histone H2A type 2-A | Q6FI13 |
| 40S ribosomal protein S4, X isoform | P62701 |
| 14-3-3 protein zeta/delta | P63104 |
| Heterogeneous nuclear ribonucleoprotein A1 | P09651 |
| CD81 antigen | P60033 |
| Keratin, type I cytoskeletal 14 | P02533 |
| ATP-citrate synthase | P53396 |
| 40S ribosomal protein S9 | P46781 |
| Transgelin-2 | P37802 |
| Fructose-bisphosphate aldolase A | P04075 |
| Ubiquitin-like modifier-activating enzyme 1 | P22314 |
| Peroxiredoxin-1 | Q06830 |
| 40S ribosomal protein S5 | P46782 |
| T-complex protein 1 subunit epsilon | P48643 |
| 60S ribosomal protein L30 | P62888 |
| T-complex protein 1 subunit alpha | P17987 |
| 60S ribosomal protein L12 | P30050 |
| Cofilin-1 | P23528 |
| Heterogeneous nuclear ribonucleoproteins A2/B1 | P22626 |
| Eukaryotic translation initiation factor 5A-1 | P63241 |
| Phosphoglycerate mutase 1 | P18669 |
| Clathrin heavy chain 1 | Q00610 |
| Dihydropyrimidinase-related protein 2 | Q16555 |
| 60S ribosomal protein L35a | P18077 |

(continued)

| Identified proteins | Accession number |
|---|---|
| T-complex protein 1 subunit zeta | P40227 |
| Carbonyl reductase [NADPH] 1 | P16152 |
| 40S ribosomal protein S3a | P61247 |
| Ferritin heavy chain | P02794 |
| Annexin A2 | P07355 |
| Myosin light polypeptide 6 | P60660 |
| Major vault protein | Q14764 |
| Heterogeneous nuclear ribonucleoprotein D0 | Q14103 |
| 60S acidic ribosomal protein P0 | P05388 |
| X-ray repair cross-complementing protein 6 | P12956 |
| 40S ribosomal protein S20 | P60866 |
| Protein arginine N-methyltransferase 1 | Q99873 |
| 40S ribosomal protein S10 | P46783 |
| Transaldolase | P37837 |
| Histone H2B type 1- | P23527 |
| Triosephosphate isomerase | P60174 |
| Protein S100-A6 | P06703 |
| 40S ribosomal protein S17 | P08708 |
| CD9 antigen | P21926 |
| Filamin-A | P21333 |
| Peptidyl-prolyl cis-trans isomerase FKBP4 | Q02790 |
| Programmed cell death 6-interacting protein | Q8WUM4 |
| Glutathione S-transferase P | P09211 |
| 14-3-3 protein epsilon | P62258 |

Microvesicles

2940 proteins were identified by Mass spectrometry in Microvesicles isolated from the initial stages of an Integra culture (week 2) and purified by centrifugation at 10,000 x g. The gene names and corresponding SWISSPROT accession numbers (in brackets) of all 2940 proteins are listed in Table 21 (in alphabetical order of gene name) and the 100 most abundant proteins are listed in Table 22, in order of decreasing abundance.

**Table 21:** Gene names and SWISSPROT accession numbers of all 2940 proteins identified in CTX0E03 microvesicles (listed in alphabetical order of gene name).

| |
|---|
| A1BG (P04217), AACS (Q86V21), AAMP (Q13685), AARS (P49588), AARSD1 (Q9BTE6), AASDHPPT (Q9NRN7), ABCA3 (Q99758), ABCC1 (P33527), ABCC4 (O15439), ABCE1 (P61221), ABCF1 (Q8NE71), ABCF2 (Q9UG63), ABCF3 (Q9NUQ8), ABHD14B (Q96IU4), ABI1 (Q8IZP0), ABR (Q12979), ACAA1 (P09110), ACAA2 (P42765), ACACA (Q13085), ACADM (P11310), ACADVL (P49748), ACAT1 (P24752), ACAT2 (Q9BWD1), ACBD6 (Q9BR61), ACBD7 (Q8N6N7), ACLY (P53396), ACO1 (P21399), ACO2 (Q99798), ACOT1 (Q86TX2), ACOT13 (Q9NPJ3), ACOT7 (O00154), ACOX1 (Q15067), ACOX3 (O15254), ACP1 (P24666), ACSL1 (P33121), ACSL3 (O95573), ACSL4 (O60488), ACSS2 (Q9NR19), ACTC1 (P68032), ACTG1 (P63261), ACTL6A (096019), ACTN1 (P12814), ACTN4 (O43707), ACTR10 (Q9NZ32), ACTR1A (P61163), ACTR1B (P42025), ACTR2 (P61160), ACTR3 (P61158), ACY1 (Q03154), ADAM10 (O14672), ADAM9 (Q13443), ADAMTS15 (Q8TE58), ADAMTS16 (Q8TE57), ADAR (P55265), ADD1 (P35611), ADD3 (Q9UEY8), ADH5 (P11766), ADK (P55263), ADO (Q96SZ5), ADPRH (P54922), ADRBK1 (P25098), ADRM1 (Q16186), ADSL (P30566), ADSS (P30520), AEBP1 (Q8IUX7), AFM (P43652), AGL (P35573), AGPS (O00116), AGRN (O00468), AHCY (P23526), AHCYL1 (O43865), AHNAK (Q09666), AHNAK2 (Q8IVF2), AHSA1 (O95433), AHSG (P02765), AIDA (Q96BJ3), AIFM1 (O95831), AIMP1 (Q12904), AIMP2 (Q13155), AIP (O00170), AK1 (P00568), AK2 (P54819), AK3 (Q9UIJ7), AK4 (P27144), AKAP12 (Q02952), AKAP9 (Q99996), AKR1A1 (P14550), AKR1B1 (P15121), AKR1C1 (Q04828), AKR7A2 (O43488), AKR7A3 (095154), AKT1 (P31749), ALCAM (Q13740), ALDH16A1 (Q8IZ83), ALDH18A1 (P54886), ALDH2 (P05091), ALDH3A1 (P30838), ALDH7A1 (P49419), ALDH9A1 (P49189), ALDOA (P04075), ALDOC (P09972), ALKBH2 (Q6NS38), ALOX12B (O75342), AMDHD2 (Q9Y303), AMPD2 (Q01433), ANAPC1 (Q9H1A4), ANAPC4 (Q9UJX5), ANAPC5 (Q9UJX4), ANAPC7 (Q9UJX3), ANKFY1 (Q9P2R3), ANKRD17 (O75179), ANKRD28 (O15084), ANKRD52 (Q8NB46), ANP32A (P39687), ANP32B (Q92688), ANP32E (Q9BTT0), |
| ANXA1 (P04083), ANXA11 (P50995), ANXA2 (P07355), ANXA3 (P12429), ANXA4 (P09525), ANXA5 (P08758), ANXA6 (P08133), ANXA7 (P20073), AP1B1 (Q10567), AP1G1 (O43747), AP1M1 (Q9BXS5), AP1S2 (P56377), AP2A1 (O95782), AP2A2 (O94973), AP2B1 (P63010), AP2M1 (Q96CW1), AP2S1 (P53680), AP3B1 (O00203), AP3D1 (O14617), AP3M1 (Q9Y2T2), AP3S1 (Q92572), AP4S1 (Q9Y587), APEH (P13798), APEX1 (P27695), API5 (Q9BZZ5), APIP (Q96GX9), APMAP (Q9HDC9), APOA2 (P02652), APOBEC3C (Q9NRW3), APOH (P02749), APOL2 (Q9BQE5), APPL1 (Q9UKG1), APRT (P07741), AQR (O60306), ARAF (P10398), ARCN1 (P48444), ARF1 (P84077), ARF4 (P18085), ARF6 (P62330), ARFGAP2 (Q8N6H7), ARFIP1 (P53367), ARFIP2 (P53365), ARG1 (P05089), ARHGAP1 (Q07960), ARHGAP5 (Q13017), ARHGDIA (P52565), ARHGEF1 (Q92888), ARHGEF10 (O15013), ARHGEF6 (Q15052), ARHGEF7 (Q14155), ARIH1 (Q9Y4X5), ARIH2 (O95376), ARL1 (P40616), ARL2 (P36404), ARL3 (P36405), ARL6IP1 (Q15041), ARL8A (Q96BM9), ARL8B (Q9NVJ2), ARMC10 (Q8N2F6), ARMC6 (Q6NXE6), ARMC8 (Q8IUR7), ARMC9 (Q7Z3E5), ARPC1A (Q92747), ARPC1 B (O15143), ARPC2 (O15144), ARPC3 (O15145), ARPC4 (P59998), ARPC5 (O15511), ARPC5L (Q9BPX5), ASAH1 (Q13510), ASCC1 (Q8N9N2), ASCC3 (Q8N3C0), ASMTL (O95671), ASNA1 (O43681), ASNS (P08243), ASPSCR1 (Q9BZE9), ASS1 (P00966), ATAD3A (Q9NVI7), ATE1 (O95260), ATG101 (Q9BSB4), ATG16L1 (Q676U5), ATG3 (Q9NT62), ATG4B (Q9Y4P1), ATG7 (O95352), ATIC (P31939), ATL3 (Q6DD88), ATM (Q13315), ATOX1 (O00244), ATP1A1 (P05023), ATP1B1 (P05026), ATP1B3 (P54709), ATP2A2 (P16615), ATP2B1 (P20020), ATP2B4 (P23634), ATP5A1 (P25705), ATP5B (P06576), ATP5C1 (P36542), ATP5E (P56381), ATP5F1 (P24539), ATP5H (O75947), ATP5I (P56385), ATP5L (O75964), ATP5O (P48047), ATP6AP1 (Q15904), ATP6AP2 (O75787), ATP6V0A1 (Q93050), ATP6V0D1 (P61421), ATP6V1A (P38606), ATP6V1B2 (P21281), ATP6V1C1 (P21283), ATP6V1D (Q9Y5K8), ATP6V1E1 (P36543), ATP6V1G1 (O75348), ATP6V1H (Q9UI12), ATR (Q13535), ATRN (O75882), ATXN10 (Q9UBB4), B2M (P61769), B3GAT3 (O94766), B3GNT1 (O43505), BAG2 (O95816), BAG5 (Q9UL15), BAIAP2 (Q9UQB8), BANF1 (O75531), BAT1 (Q13838), BAT3 (P46379), BCAM (P50895), BCAS2 (O75934), BCAT1 (P54687), BCCIP (Q9P287), BCL2L12 (Q9HB09), BDH2 (Q9BUT1), BICD2 (Q8TD16), BLMH (Q13867), BLVRA (P53004), BLVRB (P30043), BMP1 (P13497), BOLA2 (Q9H3K6), BOP1 (Q14137), BPGM (P07738), BPNT1 (O95861), BRCC3 (P46736), BRE (Q9NXR7), BRIX1 (Q8TDN6), BROX (Q5VW32), BRP16L (P0CB43), BSG (P35613), BST1 (Q10588), BTAF1 (O14981), BUB3 (O43684), BUD31 (P41223), BYSL (Q13895), BZW1 (Q7L1Q6), BZW2 (Q9Y6E2), C10orf119 (Q9BTE3), C10orf58 (Q9BRX8), C10orf76 (Q5T2E6), C11orf54 (Q9H0W9), C11orf68 (Q9H3H3), C12orf10 (Q9HB07), C12orf57 (Q99622), C14orf149 (Q96EM0), C14orf166 (Q9Y224), C14orf21 (Q86U38), C15orf58 (Q6ZNW5), C16orf13 (Q96S19), C16orf61 (Q9NRP2), C16orf80 (Q9Y6A4), C18orf21 (Q32NC0), C18orf8 (Q96DM3), C1orf123 (Q9NWV4), C1orf128 (Q9GZP4), C1orf57 (Q9BSD7), C20orf11 (Q9NWU2), C20orf4 (Q9Y312), |

(continued)

| |
|---|
| C21orf33 (P30042), C21orf59 (P57076), C22orf28 (Q9Y3I0), C3orf10 (Q8WUW1), C3orf26 (Q9BQ75), C3orf75 (Q0PNE2), C4orf27 (Q9NWY4), C4orf41 (Q7Z392), C4orf43 (Q96EY4), C5orf33 (Q4G0N4), C6orf211 (Q9H993), C7orf28B (P86790), C7orf50 (Q9BRJ6), C7orf59 (Q0VGL1), C8orf33 (Q9H7E9), C9orf142 (Q9BUH6), C9orf23 (Q8N5L8), C9orf41 (Q8N4J0), C9orf64 (Q5T6V5), CA11 (O75493), CA12 (O43570), CA2 (P00918), CAB39 (Q9Y376), CACNA2D1 (P54289), CACYBP (Q9HB71), CAD (P27708), CADM1 (Q9BY67), CADM4 (Q8NFZ8), CALB1 (P05937), CALD1 (Q05682), CALM1 (P62158), CALR (P27797), CALU (O43852), CAMK1 (Q14012), CAMK2D (Q13557), CAMKV (Q8NCB2), CAND1 (Q86VP6), CANX (P27824), CAP1 (Q01518), CAPN1 (P07384), CAPN2 (P17655), CAPN5 (015484), CAPN7 (Q9Y6W3), CAPNS1 (P04632), CAPRIN1 (Q14444), CAPS (Q13938), CAPZA1 (P52907), CAPZA2 (P47755), CAPZB (P47756), CARHSP1 (Q9Y2V2), CARKD (Q8IW45), CARM1 (Q86X55), CARS (P49589), CASK (014936), CASP14 (P31944), CASP3 (P42574), CASP7 (P55210), CAT (P04040), CBFB (Q13951), CBR1 (P16152), CBR3 (O75828), CBS (P35520), CBX1 (P83916), CBX3 (Q13185), CBX5 (P45973), CC2D1A (Q6P1N0), CCAR1 (Q8IX12), CCBL2 (Q6YP21), CCDC102B (Q68D86), CCDC22 (O60826), CCDC25 (Q86WR0), CCDC93 (Q567U6), CCND2 (P30279), CCNY (Q8ND76), CCT2 (P78371), CCT3 (P49368), CCT4 (P50991), CCT5 (P48643), CCT6A (P40227), CCT7 (Q99832), CCT8 (P50990), CD109 (Q6YHK3), CD151 (P48509), CD276 (Q5ZPR3), CD44 (P16070), CD46 (P15529), CD47 (Q08722), CD58 (P19256), CD59 (P13987), CD63 (P08962), CD81 (P60033), CD9 (P21926), CD97 (P48960), CD99 (P14209), CDC123 (O75794), CDC16 (Q13042), CDC23 (Q9UJX2), CDC34 (P49427), CDC37 (Q16543), CDC40 (O60508), CDC42 (P60953), CDC42BPB (Q9Y5S2), CDC5L (Q99459), CDCP1 (Q9H5V8), CDH2 (P19022), CDK1 (P06493), CDK2 (P24941), CDK4 (P11802), CDK5 (Q00535), CDK5RAP3 (Q96JB5), CDK7 (P50613), CDKN2A (P42771), CDKN2AIP (Q9NXV6), CECR5 (Q9BXW7), CELF1 (Q92879), CELSR1 (Q9NYQ6), CELSR2 (Q9HCU4), CFL1 (P23528), CFL2 (Q9Y281), CHCHD3 (Q9NX63), CHD4 (Q14839), CHEK2 (096017), CHERP (Q8IWX8), CHID1 (Q9BWS9), CHMP1A (Q9HD42), CHMP1B (Q7LBR1), CHMP2A (O43633), CHMP4A (Q9BY43), CHMP4B (Q9H444), CHMP5 (Q9NZZ3), CHMP6 (Q96FZ7), CHN1 (P15882), CHORDC1 (Q9UHD1), CHP (Q99653), CHRAC1 (Q9NRG0), CHST3 (Q7LGC8), CIAO1 (076071), CIAPIN1 (Q6FI81), CIRBP (Q14011), CIRH1A (Q969X6), CISD2 (Q8N5K1), CKAP4 (Q07065), CKAP5 (Q14008), CKB (P12277), CLASP1 (Q7Z460), CLIC1 (O00299), CLIC4 (Q9Y696), CLLD6 (Q5W111), CLNS1A (P54105), CLPB (Q9H078), CLTA (P09496), CLTC (Q00610), CLTCL1 (P53675), CLU (P10909), CMBL (Q96DG6), CMC1 (Q7Z7K0), CMPK1 (P30085), CMTM6 (Q9NX76), CNBP (P62633), CNDP2 (Q96KP4), CNN2 (Q99439), CNN3 (Q15417), CNNM3 (Q8NE01), CNOT1 (A5YKK6), CNOT10 (Q9H9A5), CNOT6L (Q96LI5), CNP (P09543), COASY (Q13057), COBRA1 (Q8WX92), COG1 (Q8WTW3), COG3 (Q96JB2), COG4 (Q9H9E3), COG5 (Q9UP83), COG6 (Q9Y2V7), COL11A1 (P12107), COL14A1 (Q05707), COL18A1 (P39060), COL6A1 (P12109), COMMD10 (Q9Y6G5), |

(continued)

| |
|---|
| COMMD2 (Q86X83), COMMD3 (Q9UBI1), COMMD5 (Q9GZQ3), COMMD8 (Q9NX08), COMMD9 (Q9P000), COMT (P21964), COPA (P53621), COPB1 (P53618), COPB2 (P35606), COPE (014579), COPG (Q9Y678), COPG2 (Q9UBF2), COPS2 (P61201), COPS3 (Q9UNS2), COPS4 (Q9BT78), COPS5 (Q92905), COPS6 (Q7L5N1), COPS7A (Q9UBW8), COPS7B (Q9H9Q2), COPS8 (Q99627), CORO1B (Q9BR76), CORO1C (Q9ULV4), CORO2B (Q9UQ03), CORO7 (P57737), COTL1 (Q14019), COX4NB (O43402), COX5A (P20674), COX5B (P10606), COX6C (P09669), CP (P00450), CPD (O75976), CPNE1 (Q99829), CPNE2 (Q96FN4), CPNE3 (O75131), CPNE4 (Q96A23), CPNE7 (Q9UBL6), CPOX (P36551), CPSF1 (Q10570), CPSF2 (Q9P2I0), CPSF3 (Q9UKF6), CPSF3L (Q5TA45), CPSF6 (Q16630), CPSF7 (Q8N684), CPXM1 (Q96SM3), CRABP2 (P29373), CRIP2 (P52943), CRK (P46108), CRLF3 (Q8IUI8), CRNKL1 (Q9BZJ0), CRTAP (O75718), CRYAB (P02511), CRYM (Q14894), CRYZ (Q08257), CRYZL1 (O95825), CS (O75390), CSDE1 (O75534), CSE1L (P55060), CSK (P41240), CSNK1A1 (P48729), CSNK2A1 (P68400), CSNK2A2 (P19784), CSNK2B (P67870), CSRP1 (P21291), CSRP2 (Q16527), CSTB (P04080), CSTF1 (Q05048), CSTF2T (Q9H0L4), CSTF3 (Q12996), CTBP1 (Q13363), CTBP2 (P56545), CTNNA1 (P35221), CTNNAL1 (Q9UBT7), CTNNB1 (P35222), CTNNBL1 (Q8WYA6), CTNND1 (O60716), CTPS (P17812), CTPS2 (Q9NRF8), CTR9 (Q6PD62), CTSC (P53634), CTSD (P07339), CTSF (Q9UBX1), CTSL2 (O60911), CTTN (Q14247), CTU1 (Q7Z7A3), CUL1 (Q13616), CUL2 (Q13617), CUL3 (Q13618), CUL4A (Q13619), CUL4B (Q13620), CUL5 (Q93034), CUL7 (Q14999), CXADR (P78310), CXCL14 (O95715), CXorf26 (Q9BVG4), CXorf38 (Q8TB03), CYB5R3 (P00387), CYC1 (P08574), CYCS (P99999), CYFIP1 (Q7L576), CYFIP2 (Q96F07), CYR61 (O00622), DAB1 (O75553), DAD1 (P61803), DAG1 (Q14118), DAK (Q3LXA3), DAPK3 (O43293), DARS (P14868), DAZAP1 (Q96EP5), DBI (P07108), DBN1 (Q16643), DBNL (Q9UJU6), DCAF7 (P61962), DCAF8 (Q5TAQ9), DCBLD2 (Q96PD2), DCK (P27707), DCLK1 (O15075), DCPS (Q96C86), DCTD (P32321), DCTN1 (Q14203), DCTN2 (Q13561), DCTN3 (O75935), DCTN4 (Q9UJW0), DCTN5 (Q9BTE1), DCTN6 (O00399), DCUN1D1 (Q96GG9), DCUN1D3 (Q8IWE4), DCUN1D5 (Q9BTE7), DCXR (Q7Z4W1), DDA1 (Q9BW61), DDAH1 (O94760), DDAH2 (O95865), DDB1 (Q16531), DDB2 (Q92466), DDI2 (Q5TDH0), DDOST (P39656), DDR1 (Q08345), DDT (P30046), DDX1 (Q92499), DDX17 (Q92841), DDX18 (Q9NVP1), DDX19A (Q9NUU7), DDX20 (Q9UHI6), DDX21 (Q9NR30), DDX23 (Q9BUQ8), DDX24 (Q9GZR7), DDX27 (Q96GQ7), DDX39 (O00148), DDX3X (O00571), DDX46 (Q7L014), DDX47 (Q9H0S4), DDX49 (Q9Y6V7), DDX5 (P17844), DDX50 (Q9BQ39), DDX51 (Q8N8A6), DDX52 (Q9Y2R4), DDX54 (Q8TDD1), DDX55 (Q8NHQ9), DDX56 (Q9NY93), DDX6 (P26196), DECR1 (Q16698), DECR2 (Q9NUI1), DEF (Q68CQ4), DEK (P35659), DENR (O43583), DERA (Q9Y315), DFFA (O00273), DFFB (O76075), DHCR24 (Q15392), DHCR7 (Q9UBM7), DHFR (P00374), DHPS (P49366), DHRS11 (Q6UWP2), DHRS4 (Q9BTZ2), DHX15 (O43143), DHX16 (060231), DHX29 (Q7Z478), DHX30 (Q7L2E3), DHX32 (Q7L7V1), DHX36 (Q9H2U1), DHX37 (Q8IY37), DHX38 (Q92620), DHX9 (Q08211), |

(continued)

| |
|---|
| DIAPH1 (O60610), DIAPH2 (O60879), DIMT1L (Q9UNQ2), DIP2A (Q14689), DIP2B (Q9P265), DIP2C (Q9Y2E4), DIS3 (Q9Y2L1), DIS3L2 (Q8IYB7), DKC1 (O60832), DLAT (P10515), DLD (P09622), DLG1 (Q12959), DLGAP4 (Q9Y2H0), DLST (P36957), DMD (P11532), DNAJA1 (P31689), DNAJA2 (O60884), DNAJB1 (P25685), DNAJB11 (Q9UBS4), DNAJB4 (Q9UDY4), DNAJB6 (O75190), DNAJC13 (O75165), DNAJC2 (Q99543), DNAJC3 (Q13217), DNAJC7 (Q99615), DNASE1L1 (P49184), DNM1 (Q05193), DNM1L (O00429), DNM2 (P50570), DNMT1 (P26358), DNPEP (Q9ULA0), DOCK1 (Q14185), DOCK4 (Q8N1I0), DOCK5 (Q9H7D0), DOCK7 (Q96N67), DOCK9 (Q9BZ29), DOHH (Q9BU89), DPCD (Q9BVM2), DPH2 (Q9BQC3), DPH5 (Q9H2P9), DPM1 (O60762), DPM3 (Q9P2X0), DPP3 (Q9NY33), DPP9 (Q86TI2), DPY30 (Q9C005), DPYSL2 (Q16555), DPYSL3 (Q14195), DPYSL4 (O14531), DPYSL5 (Q9BPU6), DRG1 (Q9Y295), DRG2 (P55039), DSC1 (Q08554), DSG1 (Q02413), DSP (P15924), DST (Q03001), DSTN (P60981), DTD1 (Q8TEA8), DTNA (Q9Y4J8), DTYMK (P23919), DUS2L (Q9NX74), DUS3L (Q96G46), DUSP12 (Q9UNI6), DUSP3 (P51452), DYM (Q7RTS9), DYNC1H1 (Q14204), DYNC1I2 (Q13409), DYNC1LI1 (Q9Y6G9), DYNC1LI2 (O43237), DYNC2H1 (Q8NCM8), DYNLL1 (P63167), DYNLL2 (Q96FJ2), DYNLRB1 (Q9NP97), DYNLT1 (P63172), EBNA1BP2 (Q99848), ECE1 (P42892), ECHDC1 (Q9NTX5), ECHS1 (P30084), ECM29 (Q5VYK3), EDC3 (Q96F86), EDC4 (Q6P2E9), EEA1 (Q15075), EEF1A1 (P68104), EEF1B2 (P24534), EEF1D (P29692), EEF1E1 (O43324), EEF1G (P26641), EEF2 (P13639), EEF2K (O00418), EEFSEC (P57772), EFEMP2 (O95967), EFHD2 (Q96C19), EFTUD1 (Q7Z2Z2), EFTUD2 (Q15029), EGFR (P00533), EHD1 (Q9H4M9), EHD2 (Q9NZN4), EHD3 (Q9NZN3), EHD4 (Q9H223), EIF1AX (P47813), EIF2A (Q9BY44), EIF2AK2 (P19525), EIF2AK4 (Q9P2K8), EIF2B1 (Q14232), EIF2B2 (P49770), EIF2B3 (Q9NR50), EIF2B4 (Q9UI10), EIF2B5 (Q13144), EIF2C1 (Q9UL18), EIF2C2 (Q9UKV8), EIF2S1 (P05198), EIF2S2 (P20042), EIF2S3 (P41091), EIF3A (Q14152), EIF3B (P55884), EIF3C (Q99613), EIF3D (O15371), EIF3E (P60228), EIF3F (O00303), EIF3G (075821), EIF3H (015372), EIF3I (Q13347), EIF3J (O75822), EIF3K (Q9UBQ5), EIF3L (Q9Y262), EIF3M (Q7L2H7), EIF4A1 (P60842), EIF4A2 (Q14240), EIF4A3 (P38919), EIF4E (P06730), EIF4G1 (Q04637), EIF4G2 (P78344), EIF4H (Q15056), EIF5 (P55010), EIF5A (P63241), EIF5B (060841), EIF6 (P56537), ELAC2 (Q9BQ52), ELAVL1 (Q15717), ELMO2 (Q96JJ3), ELP2 (Q6IA86), ELP3 (Q9H9T3), EMD (P50402), EMG1 (Q92979), EML1 (O00423), EML2 (O95834), EML3 (Q32P44), EML4 (Q9HC35), ENAH (Q8N8S7), ENC1 (014682), ENO1 (P06733), ENO2 (P09104), ENOPH1 (Q9UHY7), ENY2 (Q9NPA8), EPB41L2 (043491), EPB41L3 (Q9Y2J2), EPDR1 (Q9UM22), EPHA2 (P29317), EPHB2 (P29323), EPHB3 (P54753), EPHB4 (P54760), EPHX1 (P07099), EPM2AIP1 (Q7L775), EPN1 (Q9Y6I3), EPRS (P07814), ERBB2IP (Q96RT1), ERGIC1 (Q969X5), ERH (P84090), ERI1 (Q8IV48), ERI3 (043414), ERLIN2 (O94905), ERO1L (Q96HE7), ERP29 (P30040), ERP44 (Q9BS26), ESD (P10768), ESYT1 (Q9BSJ8), ETF1 (P62495), ETFA (P13804), ETFB (P38117), EXOC1 (Q9NV70), EXOC2 (Q96KP1), EXOC3 (O60645), |

(continued)

| |
|---|
| EXOC4 (Q96A65), EXOC5 (O00471), EXOC6 (Q8TAG9), EXOC6B (Q9Y2D4), EXOC7 (Q9UPT5), EXOC8 (Q8IYI6), EXOSC1 (Q9Y3B2), EXOSC10 (Q01780), EXOSC2 (Q13868), EXOSC3 (Q9NQT5), EXOSC4 (Q9NPD3), EXOSC5 (Q9NQT4), EXOSC6 (Q5RKV6), EXOSC7 (Q15024), EXOSC8 (Q96B26), EXOSC9 (Q06265), EZR (P15311), F11R (Q9Y624), F8 (P00451), F8A1 (P23610), FABP5 (Q01469), FABP7 (015540), FADD (Q13158), FAH (P16930), FAHD1 (Q6P587), FAHD2A (Q96GK7), FAM115A (Q9Y4C2), FAM120A (Q9NZB2), FAM125A (Q96EY5), FAM127A (A6ZKI3), FAM129A (Q9BZQ8), FAM129B (Q96TA1), FAM136A (Q96C01), FAM175B (Q15018), FAM3C (Q92520), FAM45B (Q6NSW5), FAM49B (Q9NUQ9), FAM82B (Q96DB5), FAM84B (Q96KN1), FAM96B (Q9Y3D0), FAM98A (Q8NCA5), FAM98B (Q52LJ0), FANCI (Q9NVI1), FAR1 (Q8WVX9), FARP1 (Q9Y4F1), FARP2 (O94887), FARSA (Q9Y285), FARSB (Q9NSD9), FAS (P25445), FASN (P49327), FAT1 (Q14517), FAU (P62861), FBL (P22087), FBLN2 (P98095), FBN1 (P35555), FBN2 (P35556), FBXL18 (Q96ME1), FBXO21 (O94952), FBXO22 (Q8NEZ5), FBXW11 (Q9UKB1), FCF1 (Q9Y324), FDFT1 (P37268), FDPS (P14324), FDXR (P22570), FEN1 (P39748), FERMT1 (Q9BQL6), FERMT2 (Q96AC1), FFR (Q9UID3), FGFBP3 (Q8TAT2), FH (P07954), FHL1 (Q13642), FHL2 (Q14192), FHL3 (Q13643), FIBP (O43427), FKBP10 (Q96AY3), FKBP1A (P62942), FKBP2 (P26885), FKBP3 (Q00688), FKBP4 (Q02790), FKBP5 (Q13451), FLG (P20930), FLG2 (Q5D862), FLII (Q13045), FLNA (P21333), FLNB (O75369), FLNC (Q14315), FLOT1 (O75955), FLOT2 (Q14254), FMNL2 (Q96PY5), FN3K (Q9H479), FN3KRP (Q9HA64), FNTA (P49354), FNTB (P49356), FOLR1 (P15328), FREM2 (Q5SZK8), FRG1 (Q14331), FRMD5 (Q7Z6J6), FRMD8 (Q9BZ67), FRYL (094915), FSCN1 (Q16658), FSD1 (Q9BTV5), FTH1 (P02794), FTL (P02792), FTO (Q9C0B1), FTSJD2 (Q8N1G2), FUBP1 (Q96AE4), FUBP3 (Q96I24), FUCA2 (Q9BTY2), FUK (Q8N0W3), FUS (P35637), FXR1 (P51114), FXR2 (P51116), FYCO1 (Q9BQS8), FYN (P06241), G3BP1 (Q13283), G3BP2 (Q9UN86), G6PD (P11413), GAA (P10253), GALK1 (P51570), GALK2 (Q01415), GALNT1 (Q10472), GALNT2 (Q10471), GALNT7 (Q86SF2), GAN (Q9H2C0), GANAB (Q14697), GAP43 (P17677), GAPDH (P04406), GAPVD1 (Q14C86), GAR1 (Q9NY12), GARS (P41250), GART (P22102), GATSL2 (A6NHX0), GBA (P04062), GBE1 (Q04446), GBF1 (Q92538), GCDH (Q92947), GCLC (P48506), GCLM (P48507), GCN1L1 (Q92616), GDI1 (P31150), GDI2 (P50395), GEMIN4 (P57678), GEMIN5 (Q8TEQ6), GEMIN6 (Q8WXD5), GET4 (Q7L5D6), GFAP (P14136), GFM1 (Q96RP9), GFPT1 (Q06210), GFPT2 (O94808), GGCT (O75223), GGPS1 (O95749), GINS1 (Q14691), GINS2 (Q9Y248), GINS4 (Q9BRT9), GIPC1 (O14908), GIT1 (Q9Y2X7), GLA (P06280), GLB1L2 (Q8IW92), GLE1 (Q53GS7), GLG1 (Q92896), GLIPR2 (Q9H4G4), GLMN (Q92990), GLO1 (Q04760), GLOD4 (Q9HC38), GLRX (P35754), GLRX3 (O76003), GLT25D1 (Q8NBJ5), GLT25D2 (Q8IYK4), GLTP (Q9NZD2), GLUD1 (P00367), GLUL (P15104), GMDS (O60547), GMFB (P60983), GMPPA (Q96IJ6), GMPPB (Q9Y5P6), GMPR (P36959), GMPR2 (Q9P2T1), GMPS (P49915), GNA11 (P29992), GNA12 (Q03113), GNA13 (Q14344), GNAI1 (P63096), |

(continued)

| |
|---|
| GNAI2 (P04899), GNAI3 (P08754), GNAQ (P50148), GNAS (Q5JWF2), GNB1 (P62873), GNB1L (Q9BYB4), GNB2 (P62879), GNB2L1 (P63244), GNB4 (Q9HAV0), GNE (Q9Y223), GNG10 (P50151), GNG12 (Q9UBI6), GNG4 (P50150), GNG5 (P63218), GNL3 (Q9BVP2), GNPDA1 (P46926), GNPNAT1 (Q96EK6), GOLGA7 (Q7Z5G4), GOLM1 (Q8NBJ4), GOLPH3 (Q9H4A6), GORASP2 (Q9H8Y8), GOT1 (P17174), GOT2 (P00505), GPC1 (P35052), GPC4 (O75487), GPC6 (Q9Y625), GPD1L (Q8N335), GPHN (Q9NQX3), GPI (P06744), GPM6A (P51674), GPN1 (Q9HCN4), GPR50 (Q13585), GPR56 (Q9Y653), GPS1 (Q13098), GPSM1 (Q86YR5), GPX1 (P07203), GPX4 (P36969), GRB2 (P62993), GRHPR (Q9UBQ7), GRP (Q3ZCW2), GRWD1 (Q9BQ67), GSDMA (Q96QA5), GSK3A (P49840), GSK3B (P49841), GSN (P06396), GSPT1 (P15170), GSR (P00390), GSS (P48637), GSTK1 (Q9Y2Q3), GSTM2 (P28161), GSTM3 (P21266), GSTM4 (Q03013), GSTO1 (P78417), GSTP1 (P09211), GSTT2 (P0CG29), GSTZ1 (O43708), GTF2E2 (P29084), GTF2F2 (P13984), GTF2H3 (Q13889), GTF21 (P78347), GTF3C2 (Q8WUA4), GTF3C3 (Q9Y5Q9), GTF3C4 (Q9UKN8), GTPBP1 (000178), GTPBP4 (Q9BZE4), GUK1 (Q16774), GYG1 (P46976), GYS1 (P13807), H1F0 (P07305), H1FX (Q92522), H2AFX (P16104), H2AFY (O75367), H2AFZ (P0C0S5), HADH (Q16836), HADHA (P40939), HARS (P12081), HAT1 (O14929), HAUS3 (Q68CZ6), HAUS4 (Q9H6D7), HBA1 (P69905), HBB (P68871), HBS1L (Q9Y450), HBXIP (O43504), HCFC1 (P51610), HDAC1 (Q13547), HDAC2 (Q92769), HDDC2 (Q7Z4H3), HDGF (P51858), HDGFRP2 (Q7Z4V5), HDHD2 (Q9H0R4), HDLBP (Q00341), HEATR1 (Q9H583), HEATR2 (Q86Y56), HEBP1 (Q9NRV9), HECTD3 (Q5T447), HERC4 (Q5GLZ8), HEXB (P07686), HGS (O14964), HHIP (Q96QV1), HINT1 (P49773), HINT2 (Q9BX68), HINT3 (Q9NQE9), HIP1R (O75146), HIST1H1B (P16401), HIST1H1C (P16403), HIST1H1D (P16402), HIST1H1E (P10412), HIST1H2AD (P20671), HIST1H2BJ (P06899), HIST1H2BM (Q99879), HIST1H2BO (P23527), HIST1H4A (P62805), HIST2H2AA3 (Q6FI13), HIST2H2AB (Q8IUE6), HIST2H2BE (Q16778), HIST2H3A (Q71DI3), HIST3H2BB (Q8N257), HK1 (P19367), HK2 (P52789), HLA-A (P30443), HLA-A (P01892), HLA-B (P03989), HMGA1 (P17096), HMGB1 (P09429), HMGB2 (P26583), HMGCL (P35914), HMGCS1 (Q01581), HMGN1 (P05114), HMGN2 (P05204), HMGN4 (O00479), HNRNPA0 (Q13151), HNRNPA1 (P09651), HNRNPA2B1 (P22626), HNRNPA3 (P51991), HNRNPAB (Q99729), HNRNPC (P07910), HNRNPD (Q14103), HNRNPF (P52597), HNRNPH1 (P31943), HNRNPH2 (P55795), HNRNPH3 (P31942), HNRNPK (P61978), HNRNPL (P14866), HNRNPM (P52272), HNRNPR (O43390), HNRNPU (Q00839), HNRNPUL1 (Q9BUJ2), HNRNPUL2 (Q1KMD3), HNRPDL (014979), HNRPLL (Q8WW9), HOOK3 (Q86VS8), HP (P00738), HP1BP3 (Q5SSJ5), HPCAL1 (P37235), HPRT1 (P00492), HPX (P02790), HRAS (P01112), HRNR (Q86YZ3), HSD17B10 (Q99714), HSD17B12 (Q53GQ0), HSD17B4 (P51659), HSDL2 (Q6YN16), HSP90AA1 (P07900), HSP90AB1 (P08238), HSP90B1 (P14625), HSPA12A (O43301), HSPA14 (Q0VDF9), HSPA1A (P08107), HSPA4 (P34932), HSPA4L (O95757), HSPA5 (P11021), HSPA8 (P11142), HSPA9 (P38646), HSPB1 (P04792), HSPBP1 (Q9NZL4), |

(continued)

| |
|---|
| HSPD1 (P10809), HSPE1 (P61604), HSPG2 (P98160), HSPH1 (Q92598), HTRA1 (Q92743), HTT (P42858), HUWE1 (Q7Z6Z7), HYOU1 (Q9Y4L1), IARS (P41252), ICAM1 (P05362), IDE (P14735), IDH1 (O75874), IDH2 (P48735), IDH3A (P50213), IDI1 (Q13907), IFI16 (Q16666), IFIT5 (Q13325), IFITM3 (Q01628), IFRD2 (Q12894), IFT172 (Q9UG01), IGF1R (P08069), IGF2BP2 (Q9Y6M1), IGF2BP3 (O00425), IGF2R (P11717), IGFBP3 (P17936), IGFBP5 (P24593), IGHG1 (P01857), IGHG2 (P01859), IGSF3 (O75054), IGSF8 (Q969P0), IKBKAP (O95163), IKBKB (O14920), IL1RAP (Q9NPH3), ILF2 (Q12905), ILF3 (Q12906), ILK (Q13418), ILKAP (Q9H0C8), IMMT (Q16891), IMP3 (Q9NV31), IMPA1 (P29218), IMPA2 (O14732), IMPAD1 (Q9NX62), IMPDH1 (P20839), IMPDH2 (P12268), INA (Q16352), INF2 (Q27J81), INPP1 (P49441), INPPL1 (O15357), INTS10 (Q9NVR2), INTS3 (Q68E01), INTS7 (Q9NVH2), INTS8 (Q75QN2), IPO11 (Q9UI26), IPO4 (Q8TEX9), IPO5 (O00410), IPO7 (O95373), IPO8 (O15397), IPO9 (Q96P70), IQGAP1 (P46940), IRF2BP2 (Q7Z5L9), IRF3 (Q14653), IRGQ (Q8WZA9), ISOC1 (Q96CN7), ISYNA1 (Q9NPH2), ITFG3 (Q9H0X4), ITGA2 (P17301), ITGA3 (P26006), ITGA4 (P13612), ITGA5 (P08648), ITGA6 (P23229), ITGA7 (Q13683), ITGAV (P06756), ITGB1 (P05556), ITGB1BP1 (O14713), ITGB3 (P05106), ITGB4 (P16144), ITGB5 (P18084), ITGB8 (P26012), ITPA (Q9BY32), JAM3 (Q9BX67), JUP (P14923), KARS (Q15046), KATNB1 (Q9BVA0), KBTBD6 (Q86V97), KCTD21 (Q4G0X4), KDM1A (O60341), KEAP1 (Q14145), KHDRBS1 (Q07666), KHSRP (Q92945), KIAA0020 (Q15397), KIAA0090 (Q8N766), KIAA0174 (P53990), KIAA0196 (Q12768), KIAA0664 (O75153), KIAA0776 (O94874), KIAA1033 (Q2M389), KIAA1279 (Q96EK5), KIAA1598 (A0MZ66), KIAA1797 (Q5VW36), KIAA1949 (Q6NYC8), KIAA1967 (Q8N163), KIDINS220 (Q9ULH0), KIF1A (Q12756), KIF2A (O00139), KIF5B (P33176), KIF5C (O60282), KLC1 (Q07866), KLHDC4 (Q8TBB5), KLHL13 (Q9P2N7), KLHL22 (Q53GT1), KLHL26 (Q53HC5), KNTC1 (P50748), KPNA1 (P52294), KPNA2 (P52292), KPNA3 (O00505), KPNA4 (O00629), KPNA6 (O60684), KPNB1 (Q14974), KPRP (Q5T749), KRAS (P01116), KRIT1 (O00522), KRT13 (P13646), KRT14 (P02533), KRT71 (Q3SY84), KTN1 (Q86UP2), L1CAM (P32004), LACTB2 (Q53H82), LAMA1 (P25391), LAMA4 (Q16363), LAMA5 (O15230), LAMB1 (P07942), LAMB2 (P55268), LAMC1 (P11047), LAMP1 (P11279), LAMP2 (P13473), LANCL1 (O43813), LANCL2 (Q9NS86), LAP3 (P28838), LARP1 (Q6PKG0), LARS (Q9P2J5), LAS1L (Q9Y4W2), LASP1 (Q14847), LBR (Q14739), LCMT1 (Q9UIC8), LDHA (P00338), LDHB (P07195), LDLR (P01130), LEFTY2 (O00292), LEPRE1 (Q32P28), LGALS1 (P09382), LGALS3 (P17931), LGALS3BP (Q08380), LGALS7 (P47929), LIMA1 (Q9UHB6), LIMS1 (P48059), LIN7C (Q9NUP9), LIPG (Q9Y5X9), LLGL1 (Q15334), LMAN1 (P49257), LMAN2 (Q12907), LMCD1 (Q9NZU5), LMNA (P02545), LMNB1 (P20700), LMNB2 (Q03252), LNPEP (Q9UIQ6), LOH12CR1 (Q969J3), LONP1 (P36776), LOR (P23490), LOXL4 (Q96JB6), LPHN2 (O95490), LPL (P06858), LRBA (P50851), LRG1 (P02750), LRP1 (Q07954), LRPPRC (P42704), LRRC1 (Q9BTT6), LRRC40 (Q9H9A6), LRRC47 (Q8N1G4), LRRC57 (Q8N9N7), LRRC59 (Q96AG4), LRRC8A (Q8IWT6), LRSAM1 (Q6UWE0), |

(continued)

| |
|---|
| LSM1 (015116), LSM12 (Q3MHD2), LSM2 (Q9Y333), LSM4 (Q9Y4Z0), LSM6 (P62312), LSM7 (Q9UK45), LSS (P48449), LTA4H (P09960), LTBP2 (Q14767), LTBP3 (Q9NS15), LTN1 (O94822), LUC7L (Q9NQ29), LUC7L2 (Q9Y383), LUC7L3 (O95232), LYAR (Q9NX58), LYPLA1 (O75608), LYPLA2 (O95372), LYPLAL1 (Q5VWZ2), LZTR1 (Q8N653), M6PR (P20645), MACF1 (Q9UPN3), MACF1 (Q96PK2), MACROD1 (Q9BQ69), MAD1L1 (Q9Y6D9), MAD2L1 (Q13257), MAGEE1 (Q9HCI5), MAK16 (Q9BXY0), MALT1 (Q9UDY8), MAN1A2 (O60476), MAN1B1 (Q9UKM7), MAN2C1 (Q9NTJ4), MAP1B (P46821), MAP1LC3A (Q9H492), MAP1LC3B2 (A6NCE7), MAP2K1 (Q02750), MAP2K2 (P36507), MAP2K3 (P46734), MAP2K4 (P45985), MAP2K7 (014733), MAP4 (P27816), MAP4K4 (095819), MAPK1 (P28482), MAPK14 (016539), MAPK3 (P27361), MAPKSP1 (Q9UHA4), MAPRE1 (Q15691), MAPRE3 (Q9UPY8), MARCKS (P29966), MARCKSL1 (P49006), MARK2 (Q7KZI7), MARS (P56192), MAT2A (P31153), MAT2B (Q9NZL9), MATR3 (P43243), MBD3 (Q95983), MBLAC2 (Q68D91), MBNL1 (Q9NR56), MBNL2 (Q5VZF2), MCAM (P43121), MCM2 (P49736), MCM3 (P25205), MCM4 (P33991), MCM5 (P33992), MCM6 (Q14566), MCM7 (P33993), MCTS1 (Q9ULC4), MDH1 (P40925), MDH2 (P40926), MDK (P21741), MDN1 (Q9NU22), ME1 (P48163), ME2 (P23368), MED1 (Q15648), MED10 (Q9BTT4), MED11 (Q9P086), MED17 (Q9NVC6), MED18 (Q9BUE0), MED20 (Q9H944), MED23 (Q9ULK4), MED24 (O75448), MED28 (Q9H204), MED31 (Q9Y3C7), MEMO1 (Q9Y316), MEN1 (O00255), MERIT40 (Q9NWV8), METAP1 (P53582), METAP2 (P50579), METHOD (Q86W50), METTL1 (Q9UBP6), METTL11A (Q9BV86), METTL13 (Q8N6R0), METTL2B (Q6P1Q9), METTL5 (Q9NRN9), METTL9 (Q9H1A3), MFAP2 (P55001), MFAP4 (P55083), MFGE8 (Q08431), MFI2 (P08582), MGEA5 (O60502), MICA (Q29983), MICAL1 (Q8TDZ2), MIF (P14174), MINA (Q8IUF8), MIOS (Q9NXC5), MKI67IP (Q9BYG3), MLEC (Q14165), MLLT4 (P55196), MLST8 (Q9BVC4), MLTK (Q9NYL2), MMP14 (P50281), MMP2 (P08253), MMS19 (Q96T76), MOB2 (Q70IA6), MOBKL1B (Q9H8S9), MOBKL2A (Q96BX8), MOBKL3 (Q9Y3A3), MOCS2 (O96033), MOGS (Q13724), MON2 (Q7Z3U7), MORC2 (Q9Y6X9), MOV10 (Q9HCE1), MOXD1 (Q6UVY6), MPG (P29372), MPI (P34949), MPP6 (Q9NZW5), MPRIP (Q6WCQ1), MPST (P25325), MPZL1 (O95297), MRC2 (Q9UBG0), MRE11A (P49959), MRI1 (Q9BV20), MRPS27 (Q92552), MRPS28 (Q9Y2Q9), MRPS33 (Q9Y291), MRPS34 (P82930), MRPS6 (P82932), MRTO4 (Q9UKD2), MSH2 (P43246), MSH3 (P20585), MSH6 (P52701), MSN (P26038), MSTO1 (Q9BUK6), MTA1 (Q13330), MTA2 (O94776), MTAP (Q13126), MTHFD1 (P11586), MTHFS (P49914), MTM1 (Q13496), MTMR1 (Q13613), MTMR2 (Q13614), MTMR6 (Q9Y217), MTMR9 (Q96QG7), MTOR (P42345), MTPN (P58546), MTR (Q99707), MTRR (Q9UBK8), MVD (P53602), MVK (Q03426), MVP (Q14764), MX1 (P20591), MYADM (Q96S97), MYBBP1A (Q9BQG0), MYCBP (Q99417), MYD88 (Q99836), MYH10 (P35580), MYH14 (Q7Z406), MYH9 (P35579), MYL12B (O14950), MYL6 (P60660), MYO18A (Q92614), MYO1B (O43795), MYO1C (000159), MYO1E (Q12965), MYO5A (Q9Y4I1), MYO6 (Q9UM54), MYOF (Q9NZM1), NAA10 (P41227), NAA15 (Q9BXJ9), |

(continued)

| |
|---|
| NAA16 (Q6N069), NAA25 (Q14CX7), NAA38 (O95777), NAA50 (Q9GZZ1), NACA (Q13765), NAE1 (Q13564), NAGK (Q9UJ70), NAGLU (P54802), NAMPT (P43490), NANS (Q9NR45), NAP1L1 (P55209), NAP1L4 (Q99733), NAPA (P54920), NAPG (Q99747), NAPRT1 (Q6XQN6), NARFL (Q9H6Q4), NARS (O43776), NASP (P49321), NAT10 (Q9H0A0), NAT9 (Q9BTE0), NCAM1 (P13591), NCAN (014594), NCAPD2 (Q15021), NCAPG (Q9BPX3), NCBP1 (Q09161), NCCRP1 (Q6ZVX7), NCDN (Q9UBB6), NCKAP1 (Q9Y2A7), NCKIPSD (Q9NZQ3), NCL (P19338), NCLN (Q969V3), NCS1 (P62166), NCSTN (Q92542), NDOR1 (Q9UHB4), NDRG3 (Q9UGV2), NDRG4 (Q9ULP0), NDUFA2 (O43678), NDUFA7 (095182), NDUFAB1 (014561), NDUFB4 (095168), NDUFC2 (O95298), NDUFS5 (O43920), NDUFS6 (O75380), NEDD8 (Q15843), NEFL (P07196), NEFM (P07197), NEK6 (Q9HC98), NEK9 (Q8TD19), NES (P48681), NF1 (P21359), NF2 (P35240), NFIX (Q14938), NHLRC2 (Q8NBF2), NHP2L1 (P55769), NID1 (P14543), NIP7 (Q9Y221), NIPSNAP1 (Q9BPW8), NIT1 (Q86X76), NIT2 (Q9NQR4), NKRF (015226), NLE1 (Q9NVX2), NLGN4X (Q8N0W4), NLN (Q9BYT8), NMD3 (Q96D46), NME2 (P22392), NME3 (013232), NME7 (Q9Y5B8), NMT1 (P30419), NNMT (P40261), NOB1 (Q9ULX3), NOC2L (Q9Y3T9), NOC3L (Q8WTT2), NOC4L (Q9BVI4), NOG (Q13253), NOL11 (Q9H8H0), NOL6 (Q9H6R4), NOL9 (Q5SY16), NOMO2 (Q5JPE7), NONO (Q15233), NOP10 (Q9NPE3), NOP16 (Q9Y3C1), NOP2 (P46087), NOP56 (O00567), NOP58 (Q9Y2X3), NOS1AP (O75052), NOSIP (Q9Y314), NOTCH2 (Q04721), NOVA2 (Q9UNW9), NPC1 (015118), NPC2 (P61916), NPEPPS (P55786), NPLOC4 (Q8TAT6), NPM1 (P06748), NPTN (Q9Y639), NPW (Q8N729), NQO1 (P15559), NQO2 (P16083), NRAS (P01111), NRBP1 (Q9UHY1), NRD1 (O43847), NRP1 (014786), NRP2 (O60462), NSDHL (015738), NSF (P46459), NSUN2 (Q08J23), NSUN5 (Q96P11), NSUN6 (Q8TEA1), NT5C (Q8TCD5), NT5C2 (P49902), NT5C3L (Q969T7), NT5E (P21589), NTN1 (095631), NUBP1 (P53384), NUBP2 (Q9Y5Y2), NUCB1 (Q02818), NUCKS1 (Q9H1E3), NUDC (Q9Y266), NUDCD1 (Q96RS6), NUDCD2 (Q8WVJ2), NUDT1 (P36639), NUDT10 (Q8NFP7), NUDT16 (Q96DE0), NUDT16L1 (Q9BRJ7), NUDT21 (O43809), NUDT4 (Q9NZJ9), NUDT5 (Q9UKK9), NUMA1 (Q14980), NUP188 (Q5SRE5), NUP210 (Q8TEM1), NUP37 (Q8NFH4), NUP43 (Q8NFH3), NUP54 (Q7Z3B4), NUP62 (P37198), NUP85 (Q9BW27), NUP88 (Q99567), NUP93 (Q8N1F7), NUTF2 (P61970), NXF1 (Q9UBU9), NXN (Q6DKJ4), NXT1 (Q9UKK6), OAT (P04181), OBSL1 (O75147), OCRL (Q01968), ODR4 (Q5SWX8), ODZ2 (Q9NT68), ODZ3 (Q9P273), OGFOD1 (Q8N543), OGT (O15294), OLA1 (Q9NTK5), OLFML3 (Q9NRN5), OPA1 (O60313), ORC3 (Q9UBD5), OSBP (P22059), OSBPL6 (Q9BZF3), OSGEP (Q9NPF4), OTUB1 (Q96FW1), OVCA2 (Q8WZ82), OXCT1 (P55809), OXSR1 (O95747), P4HA1 (P13674), P4HB (P07237), PA2G4 (Q9UQ80), PAAF1 (Q9BRP4), PABPC1 (P11940), PABPC4 (Q13310), PABPN1 (Q86U42), PACSIN2 (Q9UNF0), PACSIN3 (Q9UKS6), PAF1 (Q8N7H5), PAFAH1B1 (P43034), PAFAH1B2 (P68402), PAFAH1B3 (Q15102), PAICS (P22234), PAIP1 (Q9H074), PAK1IP1 (Q9NWT1), PAK2 (013177), PALD (Q9ULE6), PALLD (Q8WX93), PANK4 (Q9NVE7), PAPOLA (P51003), |

(continued)

| |
|---|
| PAPSS1 (O43252), PARK7 (Q99497), PARN (O95453), PARP1 (P09874), PARP4 (Q9UKK3), PARVA (Q9NVD7), PBLD (P30039), PCBD1 (P61457), PCBP1 (Q15365), PCBP2 (Q15366), PCDHB2 (Q9Y5E7), PCDHGC3 (Q9UN70), PCID2 (Q5JVF3), PCMT1 (P22061), PCNA (P12004), PCOLCE2 (Q9UKZ9), PCYOX1 (Q9UHG3), PCYOX1L (Q8NBM8), PCYT2 (Q99447), PDCD10 (Q9BUL8), PDCD11 (Q14690), PDCD4 (Q53EL6), PDCD5 (O14737), PDCD6 (O75340), PDCD6IP (Q8WUM4), PDCL3 (Q9H2J4), PDDC1 (Q8NB37), PDE12 (Q6L8Q7), PDGFRA (P16234), PDIA3 (P30101), PDIA4 (P13667), PDIA5 (Q14554), PDIA6 (Q15084), PDLIM1 (O00151), PDLIM4 (P50479), PDLIM5 (Q96HC4), PDLIM7 (Q9NR12), PDRO (Q6IAA8), PDS5A (Q29RF7), PDS5B (Q9NTI5), PDXK (O00764), PDXP (Q96GD0), PEA15 (Q15121), PEBP1 (P30086), PECI (075521), PEF1 (Q9UBV8), PELO (Q9BRX2), PELP1 (Q8IZL8), PEPD (P12955), PES1 (O00541), PFAS (015067), PFDN1 (O60925), PFDN2 (Q9UHV9), PFDN4 (Q9NQP4), PFDN5 (Q99471), PFDN6 (O15212), PFKL (P17858), PFKM (P08237), PFKP (Q01813), PFN1 (P07737), PFN2 (P35080), PGAM1 (P18669), PGAM5 (Q96HS1), PGD (P52209), PGGT1B (P53609), PGK1 (P00558), PGLS (O95336), PGLYRP2 (Q96PD5), PGM1 (P36871), PGM2L1 (Q6PCE3), PGM3 (O95394), PGP (A6NDG6), PGRMC1 (O00264), PGRMC2 (O15173), PHB (P35232), PHB2 (Q99623), PHF5A (Q7RTV0), PHF6 (Q8IWS0), PHGDH (O43175), PHKB (Q93100), PHLDA1 (Q8WV24), PHLDA3 (Q9Y5J5), PHLDB1 (Q86UU1), PHPT1 (Q9NRX4), PI15 (O43692), PI4KA (P42356), PICALM (Q13492), PIGT (Q969N2), PIK3CA (P42336), PIK3R4 (Q99570), PIN1 (013526), PIP4K2A (P48426), PIP4K2B (P78356), PIP4K2C (Q8TBX8), PIPOX (Q9P0Z9), PIPSL (A2A3N6), PITPNB (P48739), PKM2 (P14618), PKP1 (013835), PLAA (Q9Y263), PLCB3 (Q01970), PLCD1 (P51178), PLCD3 (Q8N3E9), PLCG1 (P19174), PLCG2 (P16885), PLD3 (Q8IV08), PLEC (Q15149), PLIN2 (Q99541), PLIN3 (O60664), PLK1 (P53350), PLOD1 (Q02809), PLOD2 (O00469), PLOD3 (O60568), PLRG1 (O43660), PLS1 (014651), PLS3 (P13797), PLSCR3 (Q9NRY6), PLTP (P55058), PLXNA1 (Q9UIW2), PLXNB2 (015031), PLXND1 (Q9Y4D7), PMM2 (015305), PMPCA (Q10713), PMPCB (O75439), PMVK (Q15126), PNMA2 (Q9UL42), PNN (Q9H307), PNO1 (Q9NRX1), PNP (P00491), PNPLA2 (Q96AD5), PODXL (O00592), POLD1 (P28340), POLD2 (P49005), POLE3 (Q9NRF9), POLR1A (O95602), POLR1B (Q9H9Y6), POLR1C (015160), POLR1D (Q9Y2S0), POLR2A (P24928), POLR2B (P30876), POLR2C (P19387), POLR2E (P19388), POLR2G (P62487), POLR2H (P52434), POLR2J (P52435), POLR2K (P53803), POLR3A (014802), POLR3B (Q9NW08), POLR3C (Q9BUI4), POP1 (Q99575), POP4 (O95707), POP7 (075817), POR (P16435), PPA1 (015181), PPA2 (Q9H2U2), PPAN (Q9NQ55), PPAP2A (014494), PPAT (Q06203), PPCS (Q9HAB8), PPFIBP1 (Q86W92), PPIA (P62937), PPIB (P23284), PPIC (P45877), PPID (Q08752), PPIF (P30405), PPIH (O43447), PPIL1 (Q9Y3C6), PPM1F (P49593), PPM1G (015355), PPME1 (Q9Y570), PPP1CA (P62136), PPP1CB (P62140), PPP1CC (P36873), PPP1R14B (Q96C90), PPP1R7 (Q15435), PPP1R8 (Q12972), PPP2CA (P67775), PPP2CB (P62714), PPP2R1A (P30153), |

(continued)

| |
|---|
| PPP2R2A (P63151), PPP2R2D (Q66LE6), PPP2R4 (Q15257), PPP2R5D (Q14738), PPP2R5E (Q16537), PPP3CA (Q08209), PPP4C (P60510), PPP4R1 (Q8TF05), PPP5C (P53041), PPP6C (O00743), PPP6R3 (Q5H9R7), PPPDE2 (Q6ICB0), PPT1 (P50897), PPWD1 (Q96BP3), PRCP (P42785), PRDX1 (Q06830), PRDX2 (P32119), PRDX3 (P30048), PRDX4 (Q13162), PRDX6 (P30041), PREP (P48147), PREPL (Q4J6C6), PRIM1 (P49642), PRIM2 (P49643), PRKAA1 (Q13131), PRKACA (P17612), PRKACB (P22694), PRKAG1 (P54619), PRKAR1A (P10644), PRKAR2A (P13861), PRKCA (P17252), PRKCI (P41743), PRKCSH (P14314), PRKDC (P78527), PRKRA (O75569), PRMT1 (Q99873), PRMT10 (Q6P2P2), PRMT3 (O60678), PRMT5 (O14744), PRMT7 (Q9NVM4), PROSC (O94903), PRPF19 (Q9UMS4), PRPF3 (O43395), PRPF31 (Q8WWY3), PRPF4 (043172), PRPF40A (O75400), PRPF4B (013523), PRPF6 (O94906), PRPF8 (Q6P2Q9), PRPS1 (P60891), PRPS2 (P11908), PRPSAP2 (O60256), PRRC1 (Q96M27), PRSS23 (O95084), PRTFDC1 (Q9NRG1), PSAP (P07602), PSAT1 (Q9Y617), PSD3 (Q9NYI0), PSENEN (Q9NZ42), PSIP1 (O75475), PSMA1 (P25786), PSMA2 (P25787), PSMA3 (P25788), PSMA4 (P25789), PSMA5 (P28066), PSMA6 (P60900), PSMA7 (014818), PSMB1 (P20618), PSMB2 (P49721), PSMB3 (P49720), PSMB4 (P28070), PSMB5 (P28074), PSMB6 (P28072), PSMB7 (Q99436), PSMC1 (P62191), PSMC2 (P35998), PSMC3 (P17980), PSMC4 (P43686), PSMC5 (P62195), PSMC6 (P62333), PSMD1 (Q99460), PSMD10 (O75832), PSMD11 (O00231), PSMD12 (O00232), PSMD13 (Q9UNM6), PSMD14 (O00487), PSMD2 (Q13200), PSMD3 (O43242), PSMD4 (P55036), PSMD5 (Q16401), PSMD6 (Q15008), PSMD7 (P51665), PSMD8 (P48556), PSMD9 (O00233), PSME1 (Q06323), PSME2 (Q9UL46), PSME3 (P61289), PSME4 (Q14997), PSMG1 (O95456), PSMG2 (Q969U7), PSPC1 (Q8WXF1), PSPH (P78330), PTBP1 (P26599), PTGES2 (Q9H7Z7), PTGES3 (Q15185), PTGFRN (Q9P2B2), PTGR1 (Q14914), PTHLH (P12272), PTK2 (Q05397), PTK7 (Q13308), PTMA (P06454), PTN (P21246), PTP4A1 (Q93096), PTPN1 (P18031), PTPN11 (Q06124), PTPN23 (Q9H3S7), PTPRA (P18433), PTPRE (P23469), PTPRG (P23470), PTPRJ (Q12913), PTPRZ1 (P23471), PUF60 (Q9UHX1), PURA (Q00577), PURB (Q96QR8), PUS1 (Q9Y606), PUS7 (Q96PZ0), PVR (P15151), PVRL2 (Q92692), PWP1 (Q13610), PWP2 (Q15269), PXDN (Q92626), PXK (Q7Z7A4), PXN (P49023), PYCR1 (P32322), PYCRL (Q53H96), PYGB (P11216), PYGL (P06737), OARS (P47897), QDPR (P09417), QKI (Q96PU8), QTRT1 (Q9BXR0), RAB10 (P61026), RAB11A (P62491), RAB11FIP1 (Q6WKZ4), RAB12 (Q6IQ22), RAB13 (P51153), RAB14 (P61106), RAB18 (Q9NP72), RAB1A (P62820), RAB1B (Q9H0U4), RAB21 (Q9UL25), RAB22A (Q9UL26), RAB23 (Q9ULC3), RAB27A (P51159), RAB2A (P61019), RAB2B (Q8WUD1), RAB32 (Q13637), RAB34 (Q9BZG1), RAB35 (Q15286), RAB3A (P20336), RAB3GAP1 (Q15042), RAB3GAP2 (Q9H2M9), RAB4A (P20338), RAB5A (P20339), RAB5B (P61020), RAB5C (P51148), RAB6A (P20340), RAB7A (P51149), RAB8A (P61006), RAB8B (Q92930), RABAC1 (Q9UI14), RABGAP1 (Q9Y3P9), RABGGTA (Q92696), RABGGTB (P53611), RABL2A (Q9UBK7), RABL3 (Q5HYI8), RAC1 (P63000), RAC3 (P60763), RAD23B (P54727), |

(continued)

| |
|---|
| RAD50 (Q92878), RAE1 (P78406), RAF1 (P04049), RALA (P11233), RALB (P11234), RALY (Q9UKM9), RAN (P62826), RANBP1 (P43487), RANBP2 (P49792), RANGAP1 (P46060), RAP1A (P62834), RAP1B (P61224), RAP1GDS1 (P52306), RAP2B (P61225), RAPH1 (Q70E73), RARS (P54136), RASA1 (P20936), RASA3 (Q14644), RBBP4 (Q09028), RBBP5 (Q15291), RBBP7 (Q16576), RBM12 (Q9NTZ6), RBM14 (Q96PK6), RBM15 (Q96T37), RBM22 (Q9NW64), RBM25 (P49756), RBM26 (Q5T8P6), RBM28 (Q9NW13), RBM39 (Q14498), RBM4 (Q9BWF3), RBM8A (Q9Y5S9), RBMX (P38159), RBP1 (P09455), RBPJ (Q06330), RBX1 (P62877), RCC1 (P18754), RCC2 (Q9P258), RCL (O43598), RCL1 (Q9Y2P8), RCN1 (Q15293), RDH11 (Q8TC12), RDH13 (Q8NBN7), RDX (P35241), RECQL (P46063), RELA (Q04206), REPS1 (Q96D71), RETSAT (Q6NUM9), RFC2 (P35250), RFC3 (P40938), RFC4 (P35249), RFC5 (P40937), RFFL (Q8WZ73), RFTN1 (Q14699), RHEB (Q15382), RHOA (P61586), RHOB (P62745), RHOC (P08134), RHOF (Q9HBH0), RHOG (P84095), RHOT2 (Q8IXI1), RIC8A (Q9NPQ8), RNASEH2C (Q8TDP1), RNF114 (Q9Y508), RNF20 (Q5VTR2), RNF213 (Q63HN8), RNF7 (Q9UBF6), RNGTT (O60942), RNH1 (P13489), RNMT (043148), RNPEP (Q9H4A4), ROBLD3 (Q9Y2Q5), ROCK1 (Q13464), ROCK2 (O75116), RP2 (O75695), RPA1 (P27694), RPA2 (P15927), RPA3 (P35244), RPE (Q96AT9), RPF2 (Q9H7B2), RPIA (P49247), RPL10 (P27635), RPL10A (P62906), RPL11 (P62913), RPL12 (P30050), RPL13 (P26373), RPL13A (P40429), RPL14 (P50914), RPL15 (P61313), RPL17 (P18621), RPL18 (Q07020), RPL18A (Q02543), RPL19 (P84098), RPL21 (P46778), RPL22 (P35268), RPL22L1 (Q6P5R6), RPL23 (P62829), RPL23A (P62750), RPL24 (P83731), RPL26 (P61254), RPL26L1 (Q9UNX3), RPL27 (P61353), RPL27A (P46776), RPL28 (P46779), RPL29 (P47914), RPL3 (P39023), RPL30 (P62888), RPL31 (P62899), RPL32 (P62910), RPL34 (P49207), RPL35 (P42766), RPL35A (P18077), RPL36 (Q9Y3U8), RPL36A (P83881), RPL36AL (Q969Q0), RPL37 (P61927), RPL37A (P61513), RPL38 (P63173), RPL4 (P36578), RPL5 (P46777), RPL6 (Q02878), RPL7 (P18124), RPL7A (P62424), RPL7L1 (Q6DKI1), RPL8 (P62917), RPL9 (P32969), RPLP0 (P05388), RPLP1 (P05386), RPLP2 (P05387), RPN1 (P04843), RPN2 (P04844), RPP30 (P78346), RPP38 (P78345), RPRD1A (Q96P16), RPRD1B (Q9NQG5), RPS10 (P46783), RPS11 (P62280), RPS12 (P25398), RPS13 (P62277), RPS14 (P62263), RPS15 (P62841), RPS15A (P62244), RPS16 (P62249), RPS17 (P08708), RPS18 (P62269), RPS19 (P39019), RPS2 (P15880), RPS20 (P60866), RPS21 (P63220), RPS23 (P62266), RPS24 (P62847), RPS25 (P62851), RPS26 (P62854), RPS27 (P42677), RPS27A (P62979), RPS27L (Q71UM5), RPS28 (P62857), RPS29 (P62273), RPS3 (P23396), RPS3A (P61247), RPS4X (P62701), RPS4Y1 (P22090), RPS5 (P46782), RPS6 (P62753), RPS6KA1 (Q15418), RPS6KA3 (P51812), RPS7 (P62081), RPS8 (P62241), RPS9 (P46781), RPSA (P08865), RQCD1 (Q92600), RRAGC (Q9HB90), RRAS2 (P62070), RRBP1 (Q9P2E9), RRM1 (P23921), RRM2 (P31350), RRM2B (Q7LG56), RRP1 (P56182), RRP12 (Q5JTH9), RRP1B (Q14684), RRP7A (Q9Y3A4), RRP9 (043818), RRS1 (Q15050), RSL1D1 (076021), RSL24D1 (Q9UHA3), |

(continued)

| |
|---|
| RSPRY1 (Q96DX4), RSU1 (Q15404), RTCD1 (O00442), RTKN (Q9BST9), RTN3 (O95197), RTN4 (Q9NQC3), RUVBL1 (Q9Y265), RUVBL2 (Q9Y230), RWDD2B (P57060), S100A10 (P60903), S100A11 (P31949), S100A13 (Q99584), S100A16 (Q96FQ6), S100A2 (P29034), S100A4 (P26447), S100A6 (P06703), S100A7 (P31151), S100A8 (P05109), S100A9 (P06702), SAAL1 (Q96ER3), SACS (Q9NZJ4), SAE1 (Q9UBE0), SAMHD1 (Q9Y3Z3), SAP18 (O00422), SAR1A (Q9NR31), SARM1 (Q6SZW1), SARNP (P82979), SARS (P49591), SARS2 (Q9NP81), SART3 (Q15020), SBDS (Q9Y3A5), SBF1 (O95248), SCARB1 (Q8WTV0), SCARB2 (Q14108), SCCPDH (Q8NBX0), SCFD1 (Q8WVM8), SCFD2 (Q8WU76), SCP2 (P22307), SCPEP1 (Q9HB40), SCRG1 (075711), SCRIB (Q14160), SCRN1 (Q12765), SCRN2 (Q96FV2), SCYL1 (Q96KG9), SDC2 (P34741), SDC4 (P31431), SDCBP (O00560), SDCCAG1 (O60524), SDCCAG3 (Q96C92), SDHA (P31040), SDHB (P21912), SDK1 (Q7Z5N4), SDSL (Q96GA7), SEC13 (P55735), SEC14L2 (O76054), SEC22B (O75396), SEC23A (015436), SEC23B (Q15437), SEC23IP (Q9Y6Y8), SEC24A (O95486), SEC24B (O95487), SEC24C (P53992), SEC24D (O94855), SEC31A (O94979), SEC61B (P60468), SEC61G (P60059), SEH1L (Q96EE3), SELH (Q8IZQ5), SELO (Q9BVL4), SEMA3A (Q14563), SENP3 (Q9H4L4), SEPSECS (Q9HD40), 40422 (Q9P0V9), 40787 (Q9NVA2), 37500 (Q15019), 38596 (Q99719), 39326 (Q16181), 40057 (Q9UHD8), SERBP1 (Q8NC51), SERPINB12 (Q96P63), SERPINB3 (P29508), SERPINB6 (P35237), SERPINH1 (P50454), SESN2 (P58004), SET (Q01105), SETD3 (Q86TU7), SF3A1 (Q15459), SF3A2 (Q15428), SF3A3 (Q12874), SF3B1 (O75533), SF3B14 (Q9Y3B4), SF3B2 (Q13435), SF3B3 (Q15393), SF3B4 (Q15427), SF3B5 (Q9BWJ5), SFN (P31947), SFPQ (P23246), SFRP4 (Q6FHJ7), SFXN3 (Q9BWM7), SGTA (O43765), SH3BGRL3 (Q9H299), SH3BP4 (Q9P0V3), SH3GL1 (Q99961), SH3GLB1 (Q9Y371), SHC1 (P29353), SHMT1 (P34896), SHMT2 (P34897), SHOC2 (Q9UQ13), SHPK (Q9UHJ6), SIRT5 (Q9NXA8), SKIV2L (Q15477), SKIV2L2 (P42285), SKP1 (P63208), SLC12A2 (P55011), SLC12A4 (Q9UP95), SLC16A1 (P53985), SLC1A3 (P43003), SLC1A5 (Q15758), SLC25A10 (Q9UBX3), SLC25A11 (Q02978), SLC25A13 (Q9UJS0), SLC25A22 (Q9H936), SLC25A3 (Q00325), SLC25A5 (P05141), SLC25A6 (P12236), SLC26A2 (P50443), SLC29A1 (Q99808), SLC29A2 (Q14542), SLC2A1 (P11166), SLC30A1 (Q9Y6M5), SLC38A1 (Q9H2H9), SLC3A2 (P08195), SLC44A2 (Q8IWA5), SLC4A2 (P04920), SLC4A7 (Q9Y6M7), SLC5A3 (P53794), SLC5A6 (Q9Y289), SLC6A8 (P48029), SLC7A1 (P30825), SLC7A5 (Q01650), SLC9A3R1 (O14745), SLC9A3R2 (Q15599), SLIRP (Q9GZT3), SLK (Q9H2G2), SMAD1 (Q15797), SMAD2 (Q15796), SMARCA4 (P51532), SMARCA5 (O60264), SMARCB1 (Q12824), SMARCC1 (Q92922), SMARCC2 (Q8TAQ2), SMARCD2 (Q92925), SMC1A (Q14683), SMC2 (O95347), SMC3 (Q9UQE7), SMC4 (Q9NTJ3), SMC5 (Q8IY18), SMCHD1 (A6NHR9), SMEK1 (Q6IN85), SMG1 (Q96Q15), SMN1 (Q16637), SMS (P52788), SMU1 (Q2TAY7), SMYD3 (Q9H7B4), SMYD5 (Q6GMV2), SNAP23 (O00161), SND1 (Q7KZF4), SNF8 (Q96H20), SNRNP200 (O75643), SNRNP40 (Q96DI7), SNRNP70 (P08621), SNRPA1 (P09661), SNRPB (P14678), |

(continued)

| |
|---|
| SNRPB2 (P08579), SNRPD1 (P62314), SNRPD2 (P62316), SNRPD3 (P62318), SNRPE (P62304), SNRPF (P62306), SNRPG (P62308), SNTB1 (Q13884), SNTB2 (Q13425), SNX1 (Q13596), SNX12 (Q9UMY4), SNX17 (Q15036), SNX18 (Q96RF0), SNX2 (O60749), SNX27 (Q96L92), SNX3 (O60493), SNX5 (Q9Y5X3), SNX6 (Q9UNH7), SNX9 (Q9Y5X1), SOD1 (P00441), SOD2 (P04179), SORD (Q00796), SORT1 (Q99523), SPATS2L (Q9NUQ6), SPC24 (Q8NBT2), SPCS2 (Q15005), SPCS3 (P61009), SPG21 (Q9NZD8), SPIN1 (Q9Y657), SPR (P35270), SPRR1B (P22528), SPRR2E (P22531), SPTAN1 (Q13813), SPTBN1 (Q01082), SPTBN2 (O15020), SR140 (O15042), SRBD1 (Q8N5C6), SRCRL (A1L4H1), SRGAP2 (O75044), SRI (P30626), SRM (P19623), SRP14 (P37108), SRP19 (P09132), SRP54 (P61011), SRP68 (Q9UHB9), SRP72 (O76094), SRP9 (P49458), SRPK1 (Q96SB4), SRPR (P08240), SRPRB (Q9Y5M8), SRPX (P78539), SRPX2 (O60687), SRR (Q9GZT4), SRRM1 (Q8IYB3), SRRM2 (Q9UQ35), SRRT (Q9BXP5), SRSF1 (Q07955), SRSF10 (O75494), SRSF11 (Q05519), SRSF2 (Q01130), SRSF3 (P84103), SRSF5 (Q13243), SRSF6 (Q13247), SRSF7 (Q16629), SRSF9 (Q13242), SRXN1 (Q9BYN0), SSB (P05455), SSBP1 (Q04837), SSR1 (P43307), SSR3 (Q9UNL2), SSRP1 (Q08945), SSSCA1 (O60232), SSU72 (Q9NP77), ST13 (P50502), STAG1 (Q8WVM7), STAM (Q92783), STAMBP (O95630), STAT1 (P42224), STAT2 (P52630), STAT3 (P40763), STAU1 (O95793), STIP1 (P31948), STK10 (O94804), STK24 (Q9Y6E0), STK25 (O00506), STK38 (Q15208), STK38L (Q9Y2H1), STOM (P27105), STOML2 (Q9UJZ1), STON2 (Q8WXE9), STRAP (Q9Y3F4), STT3A (P46977), STUB1 (Q9UNE7), STX12 (Q86Y82), STX4 (Q12846), STX5 (Q13190), STXBP1 (P61764), STXBP3 (O00186), STYX (Q8WUJ0), SUB1 (P53999), SUCLA2 (Q9P2R7), SUCLG2 (Q96I99), SUGT1 (Q9Y2Z0), SULF2 (Q8IWU5), SUMO1 (P63165), SUPT16H (Q9Y5B9), SUPT4H1 (P63272), SUPT5H (O00267), SUPT6H (Q7KZ85), SUSD5 (O60279), SVEP1 (Q4LDE5), SVIL (O95425), SWAP70 (Q9UH65), SYMPK (Q92797), SYNCRIP (O60506), SYNGR2 (O43760), SYNJ2BP (P57105), SYNM (O15061), SYPL1 (Q16563), TAB1 (Q15750), TAF9 (Q9Y3D8), TAGLN (Q01995), TAGLN2 (P37802), TALDO1 (P37837), TAOK1 (Q7L7X3), TARDBP (Q13148), TARS (P26639), TATDN1 (Q6P1N9), TAX1BP3 (O14907), TBC1D13 (Q9NVG8), TBC1D15 (Q8TC07), TBC1D23 (Q9NUY8), TBC1D24 (Q9ULP9), TBC1D4 (O60343), TBC1D9B (Q66K14), TBCA (O75347), TBCB (Q99426), TBCC (Q15814), TBCD (Q9BTW9), TBCE (Q15813), TBK1 (Q9UHD2), TBL1XR1 (Q9BZK7), TBL2 (Q9Y4P3), TBL3 (Q12788), TBPL1 (P62380), TCEA1 (P23193), TCEB1 (Q15369), TCEB2 (Q15370), TCERG1 (O14776), TCF25 (Q9BQ70), TCP1 (P17987), TELO2 (Q9Y4R8), TEX10 (Q9NXF1), TEX15 (Q9BXT5), TF (P02787), TFCP2 (Q12800), TFG (Q92734), TFRC (P02786), TGFB1 (P01137), TGFB2 (P61812), TGFBI (Q15582), TGFBRAP1 (Q8WUH2), TGM1 (P22735), TGM3 (Q08188), TH1L (Q8IXH7), THBS1 (P07996), THBS3 (P49746), THG1L (Q9NWX6), THOC2 (Q8NI27), THOC3 (Q96J01), THOC5 (Q13769), THOC6 (Q86W42), THOC7 (Q6I9Y2), THOP1 (P52888), THTPA (Q9BU02), THUMPD1 (Q9NXG2), THUMPD3 (Q9BV44), THY1 (P04216), THYN1 (Q9P016), |

(continued)

| |
|---|
| TIA1 (P31483), TIAL1 (Q01085), TIGAR (Q9NQ88), TIMM13 (Q9Y5L4), TIMM44 (O43615), TIMM50 (Q3ZCQ8), TIMM8A (O60220), TIMM8B (Q9Y5J9), TIMM9 (Q9Y5J7), TIMP2 (P16035), TIPRL (O75663), TJP1 (Q07157), TKT (P29401), TLN1 (Q9Y490), TLN2 (Q9Y4G6), TM9SF3 (Q9HD45), TMED10 (P49755), TMED2 (Q15363), TMED5 (Q9Y3A6), TMED7 (Q9Y3B3), TMED9 (Q9BVK6), TMEFF2 (Q9UIK5), TMEM132A (Q24JP5), TMEM2 (Q9UHN6), TMEM30A (Q9NV96), TMEM33 (P57088), TMOD3 (Q9NYL9), TMPO (P42166), TMX1 (Q9H3N1), TNC (P24821), TNKS1BP1 (Q9C0C2), TNPO1 (Q92973), TNPO2 (O14787), TNPO3 (Q9Y5L0), TOM1L2 (Q6ZVM7), TOMM20 (Q15388), TOMM34 (Q15785), TOMM5 (Q8N4H5), TOMM70A (O94826), TOP1 (P11387), TOP2A (P11388), TOP2B (Q02880), TP53I3 (Q53FA7), TP53RK (Q96S44), TPBG (Q13641), TPD52 (P55327), TPI1 (P60174), TPM1 (P09493), TPM2 (P07951), TPM3 (P06753), TPM3L (A6NL28), TPM4 (P67936), TPP2 (P29144), TPT1 (P13693), TRA2A (Q13595), TRA2B (P62995), TRAF2 (Q12933), TRAP1 (Q12931), TRAPPC1 (Q9Y5R8), TRAPPC2L (Q9UL33), TRAPPC3 (O43617), TRAPPC4 (Q9Y296), TRAPPC5 (Q8IUR0), TRIM16 (O95361), TRIM22 (Q8IYM9), TRIM25 (Q14258), TRIM26 (Q12899), TRIM28 (Q13263), TRIM47 (Q96LD4), TRIM5 (Q9C035), TRIO (O75962), TRIP13 (Q15645), TRIP6 (Q15654), TRMT1 (Q9NXH9), TRMT112 (Q9UI30), TRMT5 (Q32P41), TRMT6 (Q9UJA5), TRMT61A (Q96FX7), TRNT1 (Q96Q11), TROVE2 (P10155), TRRAP (Q9Y4A5), TSG101 (Q99816), TSKU (Q8WUA8), TSN (Q15631), TSPAN14 (Q8NG11), TSPAN6 (O43657), TSR1 (Q2NL82), TSSC1 (Q53HC9), TSTA3 (Q13630), TTC1 (Q99614), TTC15 (Q8WVT3), TTC27 (Q6P3X3), TTC37 (Q6PGP7), TTC38 (Q5R3I4), TTC7B (Q86TV6), TTC9C (Q8N5M4), TTL (Q8NG68), TTLL12 (Q14166), TTN (Q8WZ42), TTYH1 (Q9H313), TTYH3 (Q9C0H2), TUBA1B (P68363), TUBA4A (P68366), TUBB (P07437), TUBB2B (Q9BVA1), TUBB2C (P68371), TUBB3 (Q13509), TUBB6 (Q9BUF5), TUBG1 (P23258), TUBGCP2 (Q9BSJ2), TUBGCP3 (Q96CW5), TUFM (P49411), TWF1 (Q12792), TWF2 (Q6IBS0), TXN (P10599), TXNDC17 (Q9BRA2), TXNDC5 (Q8NBS9), TXNDC9 (O14530), TXNL1 (O43396), TXNRD1 (Q16881), TYK2 (P29597), TYMS (P04818), U2AF1 (Q01081), U2AF2 (P26368), UAP1 (Q16222), UBA1 (P22314), UBA2 (Q9UBT2), UBA3 (Q8TBC4), UBA52 (P62987), UBA6 (A0AVT1), UBE2D1 (P51668), UBE2D3 (P61077), UBE2E1 (P51965), UBE2G2 (P60604), UBE2I (P63279), UBE2J2 (Q8N2K1), UBE2K (P61086), UBE2L3 (P68036), UBE2M (P61081), UBE2N (P61088), UBE2O (Q9C0C9), UBE2S (Q16763), UBE2V1 (Q13404), UBE2V2 (Q15819), UBE3A (Q05086), UBE3C (Q15386), UBE4A (Q14139), UBE4B (095155), UBFD1 (014562), UBL3 (095164), UBL4A (P11441), UBL5 (Q9BZL1), UBLCP1 (Q8WVY7), UBP1 (Q9NZI7), UBQLN2 (Q9UHD9), UBR1 (Q8IWV7), UBR4 (Q5T4S7), UBTD1 (Q9HAC8), UBXN1 (Q04323), UBXN6 (Q9BZV1), UCHL1 (P09936), UCHL3 (P15374), UCHL5 (Q9Y5K5), UCK2 (Q9BZX2), UFC1 (Q9Y3C8), UFD1L (Q92890), UGDH (060701), UGGT1 (Q9NYU2), UGP2 (Q16851), ULK3 (Q6PHR2), UMPS (P11172), UNC119B (A6NIH7), UNC45A (Q9H3U1), UPF1 (Q92900), UPP1 (Q16831), UQCRC1 (P31930), UQCRC2 (P22695), UQCRFS1 (P47985), |
| URB1 (O60287), URB2 (Q14146), UROD (P06132), UROS (P10746), USO1 (O60763), USP10 (Q14694), USP11 (P51784), USP13 (Q92995), USP14 (P54578), USP15 (Q9Y4E8), USP24 (Q9UPU5), USP39 (Q53GS9), USP5 (P45974), USP7 (Q93009), USP9X (Q93008), UTP15 (Q8TED0), UTP18 (Q9Y5J1), UTP20 (075691), UTP6 (Q9NYH9), UTRN (P46939), UXS1 (Q8NBZ7), UXT (Q9UBK9), VAC14 (Q08AM6), VAMP3 (Q15836), VAMP5 (O95183), VAPA (Q9P0L0), VAPB (O95292), VARS (P26640), VASP (P50552), VAT1 (Q99536), VAV2 (P52735), VBP1 (P61758), VCAN (P13611), VCL (P18206), VCP (P55072), VDAC1 (P21796), VDAC2 (P45880), VDAC3 (Q9Y277), VIM (P08670), VPRBP (Q9Y4B6), VPS11 (Q9H270), VPS13A (Q96RL7), VPS13C (Q709C8), VPS16 (Q9H269), VPS18 (Q9P253), VPS24 (Q9Y3E7), VPS25 (Q9BRG1), VPS26A (O75436), VPS26B (Q4G0F5), VPS28 (Q9UK41), VPS29 (Q9UBQ0), VPS33A (Q96AX1), VPS33B (Q9H267), VPS35 (Q96QK1), VPS36 (Q86VN1), VPS37B (Q9H9H4), VPS39 (Q96JC1), VPS41 (P49754), VPS45 (Q9NRW7), VPS4A (Q9UN37), VPS4B (075351), VPS53 (Q5VIR6), VPS8 (Q8N3P4), VRK1 (Q99986), VTA1 (Q9NP79), VWA1 (Q6PCB0), VWA5A (O00534), WARS (P23381), WASF2 (Q9Y6W5), WASL (000401), WBSCR22 (O43709), WDFY1 (Q8IWB7), WDR1 (O75083), WDR11 (Q9BZH6), WDR12 (Q9GZL7), WDR18 (Q9BV38), WDR26 (Q9H7D7), WDR3 (Q9UNX4), WDR36 (Q8NI36), WDR4 (P57081), WDR43 (Q15061), WDR45L (Q5MNZ6), WDR48 (Q8TAF3), WDR5 (P61964), WDR54 (Q9H977), WDR6 (Q9NNW5), WDR61 (Q9GZS3), WDR73 (Q6P4I2), WDR74 (Q6RFH5), WDR75 (Q8IWA0), WDR77 (Q9BQA1), WDR82 (Q6UXN9), WDR92 (Q96MX6), WHSC2 (Q9H3P2), WRNIP1 (Q96S55), XP32 (Q5T750), XPC (Q01831), XPNPEP1 (Q9NQW7), XPO1 (014980), XPO4 (Q9C0E2), XPO5 (Q9HAV4), XPO6 (Q96QU8), XPO7 (Q9UIA9), XPOT (O43592), XRCC1 (P18887), XRCC5 (P13010), XRCC6 (P12956), XRN2 (Q9H0D6), YARS (P54577), YBX1 (P67809), YES1 (P07947), YKT6 (O15498), YRDC (Q86U90), YTHDC1 (Q96MU7), YTHDF2 (Q9Y5A9), YWHAB (P31946), YWHAE (P62258), YWHAG (P61981), YWHAH (Q04917), YWHAQ (P27348), YWHAZ (P63104), ZC3H15 (Q8WU90), ZC3HAV1 (Q7Z2W4), ZC3HAV1L (Q96H79), ZCCHC3 (Q9NUD5), ZFAND1 (Q8TCF1), ZFR (Q96KR1), ZMAT2 (Q96NC0), ZNF259 (075312), ZNF326 (Q5BKZ1), ZNF330 (Q9Y3S2), ZNF622 (Q969S3), ZNF765 (Q7L2R6), ZNFX1 (Q9P2E3), ZW10 (O43264), ZWILCH (Q9H900), ZYG11B (Q9C0D3), ZYX (Q15942). |

**Table 22:** 100 most abundant proteins (name and SwissProt accession number) in CTX0E03 microvesicles

| Identified proteins | Accession number |
|---|---|
| Actin, cytoplasmic 2 | P63261 |
| Histone H4 | P62805 |
| Histone H2B | Q99879 |
| Histone H3.2 | Q71DI3 |
| Histone H2B type 1 | P23527 |
| Glyceraldehyde-3-phosphate dehydrogenase | P04406 |
| Histone H2A type 2-A | Q6FI13 |
| Ubiquitin-40S ribosomal protein S27a | P62979 |
| Annexin A2 | P07355 |
| Alpha-enolase | P06733 |
| Pyruvate kinase isozymes M1/M2 | P14618 |
| 60S ribosomal protein L6 | Q02878 |
| Histone H2B type 2-E | Q16778 |
| Heat shock cognate 71 kDa protein | P11142 |
| Actin, alpha cardiac muscle 1 | P68032 |
| Heat shock protein HSP 90-beta | P08238 |
| Histone H2B type 1-J | P06899 |
| Elongation factor 1-alpha 1 | P68104 |
| Tubulin beta-2C chain | P68371 |
| 60S ribosomal protein L18 | Q07020 |
| Tubulin beta chain | P07437 |
| 40S ribosomal protein S2 | P15880 |
| 40S ribosomal protein S11 | P62280 |
| Histone H2B type 3-B | Q8N257 |
| Tubulin alpha-1 B chain | P68363 |
| 40S ribosomal protein S3 | P23396 |
| 40S ribosomal protein S3a | P61247 |
| Histone H2A type 1-D | P20671 |
| Elongation factor 2 | P13639 |
| Heat shock protein HSP 90-alpha | P07900 |
| GTP-binding nuclear protein Ran | P62826 |
| 60S ribosomal protein L4 | P36578 |
| 40S ribosomal protein S9 | P46781 |
| Profilin-1 | P07737 |
| 60S ribosomal protein L13a | P40429 |
| Phosphoglycerate kinase 1 | P00558 |
| Fatty acid synthase | P49327 |

(continued)

| Identified proteins | Accession number |
|---|---|
| Annexin A1 | P04083 |
| Histone H2A.Z | P0C0S5 |
| Vimentin | P08670 |
| 40S ribosomal protein S6 | P62753 |
| Moesin | P26038 |
| Peptidyl-prolyl cis-trans isomerase A | P62937 |
| 60S ribosomal protein L26 | P61254 |
| 60S ribosomal protein L3 | P39023 |
| 40S ribosomal protein S8 | P62241 |
| 60S ribosomal protein L28 | P46779 |
| Ezrin | P15311 |
| 40S ribosomal protein S4, X isoform | P62701 |
| 60S ribosomal protein L7a | P62424 |
| 60S ribosomal protein L13 | P26373 |
| 60S ribosomal protein L7 | P18124 |
| 40S ribosomal protein S23 | P62266 |
| 60S ribosomal protein L5 | P46777 |
| Eukaryotic initiation factor 4A-I | P60842 |
| 40S ribosomal protein S24 | P62847 |
| Tubulin beta-2B chain | Q9BVA1 |
| 60S ribosomal protein L8 | P62917 |
| 60S ribosomal protein L15 | P61313 |
| 60S ribosomal protein L10 | P27635 |
| Peroxiredoxin-1 | Q06830 |
| Keratin, type I cytoskeletal 14 | P02533 |
| 14-3-3 protein theta | P27348 |
| 40S ribosomal protein S18 | P62269 |
| Transketolase | P29401 |
| 60S ribosomal protein L24 | P83731 |
| Histone H1.5 | P16401 |
| Cofilin-1 | P23528 |
| Dihydropyrimidinase-related protein 3 | Q14195 |
| 60S ribosomal protein L21 | P46778 |
| 60S ribosomal protein L36 | Q9Y3U8 |
| Sodium/potassium-transporting ATPase subunit alpha-1 | P05023 |
| 40S ribosomal protein S16 | P62249 |
| T-complex protein 1 subunit gamma | P49368 |
| Heterogeneous nuclear ribonucleoprotein A1 | P09651 |

(continued)

| Identified proteins | Accession number |
|---|---|
| 60S ribosomal protein L14 | P50914 |
| Heat shock 70 kDa protein 1A/1B | P08107 |
| T-complex protein 1 subunit theta | P50990 |
| 60S ribosomal protein L30 | P62888 |
| Protein S100-A6 | P06703 |
| 40S ribosomal protein SA | P08865 |
| CD44 antigen | P16070 |
| 60S ribosomal protein L35a | P18077 |
| Tubulin beta-3 chain | Q13509 |
| T-complex protein 1 subunit delta | P50991 |
| 4F2 cell-surface antigen heavy chain | P08195 |
| T-complex protein 1 subunit beta | P78371 |
| Myosin-9 | P35579 |
| Adenosylhomocysteinase | P23526 |
| Filamin-A | P21333 |
| Fatty acid-binding protein, brain | 015540 |
| Myristoylated alanine-rich C-kinase substrate | P29966 |
| T-complex protein 1 subunit eta | Q99832 |
| Fascin | Q16658 |
| Fructose-bisphosphate aldolase A | P04075 |
| 60S ribosomal protein L27 | P61353 |
| 60S ribosomal protein L17 | P18621 |
| Heterogeneous nuclear ribonucleoproteins A2/B1 | P22626 |
| 60S ribosomal protein L10a | P62906 |
| 60S ribosomal protein L35 | P42766 |

*Discussion of proteomic data*

CD63 (also known as MLA1 and TSPAN30), TSG101 (also known as ESCRT-I complex subunit TSG101), CD109 (also known as 150 kDa TGF-beta-1-binding protein) and thy-1 (also known as CD90) were detected in both exosomes and microvesicles.

Other tetraspanins were also detected: Tetraspanin-4, -5, -6, -9 and 14 were detected in the exosome fraction; tetraspanins-6 and -14 were detected in the microvesicles.

CD133 (also known as AC133, Prominin-1, PROM1, PROML1 and MSTP061) was detected in the exosomes but not the microvesicles.

CD53 (also known as MOX44 and TSPAN25), CD82 (also known as KAI1, SAR2, ST6 and TSPAN27), CD37 (also known as TSPAN26) and CD40 ligand (also known as CD40LG, CD40L and TNFSF5) were not detected in the exosomes or the microvesicles.

Nestin, GFAP and tubulin beta-3 chain (also known as TUBB3) were detected in both the exosome and microvesicle fractions, with tubulin beta-3 chain being particularly prominent within the top 100 proteins in both fractions. Sox2, DCX, GALC, GDNF and IDO were not detected.

Selectins and TNFRI (also known as TNF receptor 1, TNFRSF1A, TNFAR and TNFR1) were not detected.

Integrin alpha-2, -3, -4, -5, -6, -7, -V and integrin beta-1, -4 and -8 were detected in both exosome and microvesicle fractions. Integrin beta-3 and -5 were detected in the microvesicles only.

MHC Class I antigens (*e.g.* HLA_A1, HLA-A2 and HLA-B27) were detected in both the exosomes and microvesicles.

Cell-adhesion molecules (*e.g.* CADM1, CADM4, ICAM1, JAM3, L1CAM, NCAM) were detected in both the exosomes and microvesicles.

Cytoskeletal proteins (*e.g.* actin, vimentin, keratins, catenins, dystroglucan, neurofilament polypeptide, microtubule-associated protein, tubulin, desmoplaktin, plectin, plakophilin, septin, spectrin, talin, vinculin and zyxin) were detected in both the exosome and microvesicle fractions.

GTPases, clathrin, chaperones, heat-shock proteins (*e.g.* Hsp90, Hsp70), splicing factors, translation factors, annexins and growth factors (*e.g.* TGF-beta) were detected in both the exosomes and microvesicles.

Galectin-3, TIMP-1, thrombosponding-1, EGF receptor and CSK were detected in both the exosomes and microvesicles.

Figure 17 compares the proteomic data from the exosomes and microvesicles. Figure 17A illustrates the number of unique proteins within each micro particle population, isolated from week 2 Integra culture system. Figure 17B compares the biological processes associated with the identified proteins within each micro particle population, isolated from week 2 Integra system. The proteins identified within exosomes and microvesicles are associated with very similar biological processes.

Proteins associated with biotin metabolism were only found in exosomes and proteins involved in tryptophan biosynthesis and taurine/alpha-linolenic acid metabolism were only identified in microvesicles.

Figure 17C compares the CTX0E03 proteome to the Mesenchymal Stem Cell exosome proteome disclosed in Lai et al 2012, in which a total of 857 proteins were identified in exosomes released from mesenchymal stem cells.

Figure 17D compares the biological processes associated with the identified proteins within the MSC derived exosomes (Lim 2012) with the neural stem cell derived exosomes of the invention. The three biological processes found to be associated with the MSC derived exosomes only are (in decreasing order of significance): Asthma; phenylalanine, tyrosine and tryptophan biosynthesis; and primary immunodeficiency. The thirty biological processes found to be associated only with the neural stem cell derived exosomes are shown in Figure 18; the most significant biological function identified relates to RNA polymerase.

A further comparison of the 197 biological processes shared by both MSC derived exosomes and NSC derived exosomes shows that NSC exosomes contain notably more processes involved in RNA degradation, the Ribosome and spliceosomes, when compared to MSC exosomes.

The above comparison indicates a number of significant differences between NSC derived exosomes and MSC derived exosomes (as characterised by Lim *et al* 2012). The 4 most significant biological differences identified as present in NSC exosomes compared to being very low/absent in those identified by the Lim's group, all involve proteins associated with the production, packaging, function and degradation of genetic material, i.e RNA polymerase, RNA degradation, Ribosome and spliceosomes.

**Example 20:** Functional analysis of individual miRNAs

Methods

***MiRNA mimic transfection and evaluation of cell proliferation by Cyquant***

Twenty four hours prior to transfection, glioma cells, U373 or U87, were seeded into a 96-well plate. MiRNA transfection optimization was performed using AllStars Negative Control siRNA AF 488 (Qiagen). MiRNA transfection efficiency was 100% when the following conditions were used. 20 nM of each miRNA mimics (Qiagen), hsa-miR-1246 (SEQ ID No. 21), hsa-miR-4492 (SEQ ID no. 34), hsa-miR-4532 (SEQ ID No. 23), and hsa-miR-4488 (SEQ ID No. 61) were combined with Lipofectamine® 2000 (Invitrogen) and transfection performed according to manufacturer's instructions.

***Experiment 1 (U373MG; 2500 cells/well; 10%FBS)***

2500 U373MG cells were seeded per 96-well and cultured in DMEM glutamax/10%FBS for 24hrs, 48hrs and 72hrs post-transfection. Cell proliferation was measured by CyQUANT® Cell Proliferation Assay Kit (Invitrogen). Briefly, following removal of the culture medium, 200 μl of the CyQUANT® GR dye/cell-lysis buffer was added into each well of the 96-well plate and incubated for 15 min. Fluorescence intensity of each well was obtained using a GloMax™ 96 microplate (Promega) plate counter at excitation and emission wavelengths of 480 and 520 nm, respectively.

***Experiment 2 (U373MG; 2500 cell/well; 2%FBS)***

2500 U373MG cells were seeded per 96-well and cultured in DMEM glutamax/2%FBS for 24hrs, 48hrs and 72hrs post-transfection. Cell proliferation was measured by CyQUANT® Cell Proliferation Assay Kit (Invitrogen). Briefly, following removal of the culture medium, 200 μl of the CyQUANT® GR dye/cell-lysis buffer was added into each well of the 96-well plate and incubated for 15 min. Fluorescence intensity of each well was obtained using a GloMax™ 96 microplate (Promega) plate counter at excitation and emission wavelengths of 480 and 520 nm, respectively.

***Experiment 3 (U87; 9000 cells/well; basal)***

9000 U87 cells were seeded per 96 well and cultured in EMEM+2nM glutamine for 0hrs, 24hrs, 48hrs and 72hrs post-transfection. Cell proliferation was measured by CyQUANT® Cell Proliferation Assay Kit (Invitrogen). Briefly, following removal of the culture medium, 200 μl of the CyQUANT® GR dye/cell-lysis buffer was added into each well of the 96-well plate and incubated for 15 min. Fluorescence intensity of each well was obtained using a GloMax™ 96 Microplate (Promega) plate counter at excitation and emission wavelengths of 480 and 520 nm, respectively.

Results

Next generation sequence (NGS) analysis of miRNA contents in CTX0E03-derived exosomes revealed the presence of a set of top-ranked miRNAs, hsa-mir-1246, hsa-mir-4488, hsa-mir-4492, and hsa-mir-4532. To assess the functionality of these individual miRNAs in reducing glioma cell proliferation, each (mimic) miRNA was transfected into two cell line models of glioma: U373MG and U87.

The incidence of reduction of cell proliferation was dependent on the glioma model and cell culturing conditions, but each of the four miRNAs tested significantly reduced tumour cell proliferation in at least one of the models. hsa-mir-4492 and hsa-mir-4532 significantly reduced cell proliferation in each of the models tested. The results of Experiments 1 to 3 are shown in Figure 23A, B and C, respectively.

**Example 21:** Tolerability and pilot efficacy of exosomes in U-87MG human glioblastoma subcutaneous xenografts.

**OBJECTIVE**

To assess the tolerability and pilot efficacy of exosomes in U-87MG subcutaneous xenografts

**METHODS**

ANIMALS

| | |
|---|---|
| **Number** | 30 |
| **No. of Groups & No per Group** | 5 groups, 5 mice/group |
| **Species** | *Mus musculus* |
| **Strain** | Athymic nude (Hsd:AthymicNude-Fxn1$^{nu}$) |
| **Age** | 5-7 weeks |
| **Gender** | Female |
| **Body Weight** | N/A |
| **Animal ID** | Transponder chip according to PRECOS Standard Operating Methods (SOMs) |
| **Acclimatisation** | ≥1 week |
| **Implantation site** | Left flank |
| **Anaesthesia** | In accordance with PRECOS SOMs |
| **Housing** | According to PPL 70/7317 and PRECOS Standard Operating Procedures (SOPs) |
| **Animal** | Harlan UK |

CELL LINES, *IN VITRO* EXPANSION

| | |
|---|---|
| **Cell Line Name** | U-87MG |
| **Supplier and catalogue number** | ECACC, 89081402 |
| **Culture Medium** | EMEM culture medium (Sigma, UK) containing 10% (v/v) heat inactivated foetal bovine serum (Hyclone, Thermo Scientific, UK) |
| **Number of mice to be implanted** | 30 |
| **Number of cells per mouse** | $8x10^6$ per mouse |
| **Matrigel/Cultrex/PBS/other** | PBS supplemented with 0.1% glucose |
| **Volume of diluent per mouse** | 0.1mL |
| **Number of batches** | 1 |

Cells will be harvested, washed in the culture medium described above and cells with viability of ≥90% will be re-suspended for *in vivo* administration. Cells will be stored on ice for a minimum period of time (e.g. no longer than 30 minutes) prior to implantation.

IMPLANTATION

*TRANSPONDER IMPLANTATION:* Implanted at initiation (tumour implantation).

*TUMOUR IMPLANTATION:* $8x10^6$ viable cells in 100μl PBS + 0.1% glucose will be injected subcutaneously into the left flank of each mouse. A total of 30 mice will be implanted.

DATA CAPTURE

Body weight, dosing and any comments relating to clinical condition will be captured in real-time using the study management software, StudyDirector (StudyLog Systems Inc.). Data will be exported into Microsoft Excel and/or Graph-Pad Prism for subsequent data analysis and transformation.

Study specific data capture schedules will be created in Excel and completed by the study team. These data capture schedules will include study specific clinical observations; the recording of these observations and will be included in the final report and uploaded to the study folder at the end of the study.

*BODY WEIGHT:* Mice will be weighed x3 weekly during the dosing phase, weekly thereafter; clinical condition monitored daily for the duration of the study by an experienced technician.

*TUMOUR MONITORING (inc. BLI):* Tumour will be measured 3 times a week and tumour volumes will be estimated using the formula $0.5(LxW^2)$ by measuring the tumour in two dimensions using electronic callipers for the duration of the study.

*TREATMENT INITIATION AND DURATION:* The mice will be randomly allocated to the treatment groups (e.g. using a stratified randomisation software tool) such that there is a similar distribution of tumour size within and between treatment groups. Dosing will be initiated when the mean tumour volume of groups approximates $100\text{-}150mm^3$. The study will terminate 3 weeks following initiation.

TEST & REFERENCE SUBSTANCE ID STORAGE & FORMULATION

*Test & Reference Substance ID and storage.*

| Compound ID | Compound Source | Compound Storage | Vehicle Name | Vehicle Source | Vehicle Storage | Post-formulation storage |
|---|---|---|---|---|---|---|
| Exosome 0 | Reneuron Ltd | -80°C | 0.9% saline | ReNeuron Ltd. | 2-8°C | 2-8°C Kept on ice during administration |

(continued)

| Compound ID | Compound Source | Compound Storage | Vehicle Name | Vehicle Source | Vehicle Storage | Post-formulation storage |
|---|---|---|---|---|---|---|
| Temozolomide | PRECOS Ltd | RT powder | 10% DMSO | PRECOS | n/a | +4°C |

Prepare dosing solutions freshly made before dosing.

DOSING

Mice will be dosed according to the following dosing schedule:

| Group (No per group) | Compound ID | Dose mg/kg | Dose Volume (mg/ml) | Dose conc. (mg/ml) | Route | Dosing Frequency (bid/qd/tid) including wording e.g. twice daily etc. |
|---|---|---|---|---|---|---|
| 1(5) | Vehicle | n/a | 50μl[1] | 0 | Intratumoural | Once only |
| 2(5) | Exosome 0 | 1 | 50μl[1] | TBD[2] | Intratumoural | Once only |
| 3(5) | Exosome 0 | 0.5 | 50μl[1] | TBD[2] | Intratumoural | Once only |
| 4(5) | Exosome 0 | 0.1 | 50μl[1] | TBD[2] | Intratumoural | Once only |
| 5(5) | Temozolomide | 5 | 10.0 | 5 | p.o. | Daily (q.d.) |

[1] Dose calculated on mean body weight and delivered in a fixed volume of 50μl.
[2] Concentration to be determined, dependent upon mean group body weight.

STUDY ENDPOINTS/BODY WEIGHT LOSS (BWL) DURING THE STUDY

- Terminate any mouse with sudden body weight loss approaching 20%
- Any mouse with continuous BWL approaching 20% over several daily measurements will be removed and terminated.

After one measurement of **body weight loss (BWL)> 10%,** a dose holiday will be given to the individual mouse. All dose holidays **must** be recorded on a Protocol Deviation.

*Whether to give dose holidays to all the mice or the individual mouse in the group should be done so in consultation with the client, but is ultimately at the Named Persons (or appointed deputies) discretion based on the severity/incidence of the BWL.*

TERMINATION

Each mouse will remain in the study until terminated (day 21), or until circumstances necessitate removal of an animal from the study e.g. loss of clinical condition and/or body weight.

Animals may also be terminated at any time during the study if any adverse effects are noted according to Home Office Project Licence PPL 70/7317.

Termination will be performed in accordance with United Kingdom Home Office Animals, (Scientific Procedures) Act 1986 and PRECOS SOPs.

TERMINAL SAMPLES

At termination the tumour will be excised and weighed. Tumours will be fixed in 10% Neutral Buffered Formalin and processing to FFPE blocks.

## ANIMAL WELFARE AND REGULATION GUIDELINES

*Housing And Environment*

Mice will be housed and cared for in accordance with the UK Animals (Scientific Procedures) Act 1986 (ASPA) and in line with the Directive 2010/63/EU of the European Parliament and of the Council of 22 September 2010 "on the protection of animals used for scientific purposes" and according to the and PRECOS Policies, SOPs and SOMs.

*Animal Welfare Monitoring*

This study will be conducted in line with the FELASSA Guidelines on Pain & Suffering in Experimental Animals and the NCRI Guidelines for the welfare and use of animals in Cancer Research (Workman et al., British Journal of Cancer (2010) 102, 1555-1577).

An experienced technician will check the condition of the mice at least daily. Unexpected adverse effects will be recorded and reported to the Named Animal Care & Welfare Officer (NACWO) and Named Veterinary Surgeon (NVS).

Animals may be terminated at any time during the study if any unexpected adverse effects are noted according to Home Office Project Licence PPL 70/7317 and the permitted severity band.

## STATISTICS AND REPORTING

*Statistical methods*

Statistical analysis if required will be performed in appropriate using the Minitab or PRISM statistical programmes for the PC.

## RESULTS

Tumours were implanted on day 0 and measured from day 6; tumours were measured three times weekly by callipers and the tumour volume calculated. When tumours reached a mean tumour volume of ~165mm$^3$ they were assigned to treatment groups based on mean tumour volume per cage in order to achieve a minimum amount of variation between and within groups.

The individual tumour volumes of each group on the day of assignment are presented in Figure 24; the mice were dosed on study day 12. The raw data for individual tumour volumes can be found in Figure 32.

Mouse body weights were monitored for the duration of the study. The data, expressed as the mean + standard error of the mean (% of the pre-dose weight), is presented graphically in Figure 25 (the dotted vertical line indicates the commencement of the dosing phase); the raw data for individual body weights can be found in Figures 30 and 31, absolute and relative measurements respectively. Body weight was stable over the duration of the study for each of the test agents and no adverse effects relating to the dosing protocols were documented in any of the treatment groups.

Mouse IDs recruited in dosing groups 1-4 received a single dose of increasing dose levels of Exosome 0 on study day 12. The Temozolomide dosing phase continued until study day 46 (35 oral doses). However, a number of mice were terminated prior to this point due to a number of listed adverse effects (see Figure 34; tumours reached the maximum permitted size as defined by UKCCCR guidelines (mean diameter 15mm)).

Figure 26 summarises the mean tumour volume for the treatment groups measured during the study, expressed as the group mean + standard error of the mean (% of the pre-dose volume).

One tumour from vehicle group 1 (ID4; 00077E7FDB-14) failed to demonstrate progressive growth following assignment and regressed to zero volume by day 29, as this is an untreated group the mouse has been classified as an outlier and removed from all analysis.

Loss of mice due to early terminations results in a reduction of the mean tumour volume from day 27 onwards. Figure 27 displays the tumour volume data (group mean + standard error of the mean; % pre-dose volume) of Figure 26 but in a truncated format *i.e.* all the line plots are graphed up to study day 25 before termination as a result of adverse effects occurred. The raw data for individual tumour volumes and individual tumour plots are detailed in appendices 3 and 4 respectively.

Mean tumour volumes were analysed statistically using a two-way ANOVA test (Figure 27 data set; GraphPad Prism; GraphPad Software, Inc.) for day 25. (The tumour volume data of G1 mouse ID14 was excluded from the statistical analysis; individual TV plots detailed in Appendix 4.) Although **there was a trend showing reduction in tumour volume for both 1mg/kg Exosome 0** (group 2) and Temozolomide (group 5), no statistically significant reduction in tumour volume was observed when compared to the vehicle group over the course of the study (GraphPad Prism; two-way ANOVA). The Bonferroni multiple comparison post-test did indicate a statistically significant reduction in tumour volume on day 25 for group 5 versus group 1 (p>0.01).

Terminal tumour weights were analysed statistically using a one-way ANOVA test (GraphPad Prism; GraphPad Software, Inc.); individual group comparisons were carried out on the total group tumour weights. No statistically significant differences among the mean tumour weight for each treatment group were observed using one-way ANOVA (*p=0.2703)*.

From the final tumour weight assessment (Figure 28; expressed as group mean + standard error of the mean (tumour weight)), **what is noticeable in the Exosome 0 1mg/kg dosing group (group 1) is that some of the tumours showed sensitivity to the treatment (IDs 12 and 21).** Additionally, Temozolomide appears to increase the latency of the tumour instead of a significant decrease in tumour volume.

In survival analysis (Figure 29) utilising mean tumour diameter (15mm) as the humane survival endpoint, a **trend in increased survival was observed for 1mg/kg Exosome 0** and Temozolomide. However, no significant increase in survival for any of the treatment groups was observed when compared with the vehicle group ($p$=0.3651; Log-Rank (Mantel-Cox) test; GraphPad Prism; GraphPad Software, Inc.). Temozolomide did result in an increase in survival compared with the two lower doses of Exosome 0 (groups 3 and 4; $p \geq 0.05$).

## DISCUSSION

The primary objective of this pilot study was to assess the effect of several doses of Exosome 0 on the growth of U87MG subcutaneous glioblastoma xenografts and a dose of Temozolomide; an oral alkylating agent commonly used for the treatment of glioblastoma multiforme.

The agents under test in this study were well tolerated with no loss of body weight or adverse effects relating to treatment noted; however, tumour size and ulceration resulted in a decrease in mice per group from day 27. As the group sizes in this pilot study were already small the statistical significance that could be achieved with the test agents was therefore limited. The three dosing levels of the Exosome 0 were inefficacious in significantly reducing tumour volume (or tumour weight) when compared to the vehicle group, however, 40% of the tumours treated with the highest dose of Exosome 0 (1mg/kg; Group 1) showed sensitivity to the treatment.

Similarly the reduction in tumour size with Temozolomide was not significant, which also suggest the group sizes were too small to achieve statistical significance.

A trend in increased survival was also observed for 1mg/kg Exosome 0 and Temozolomide however significance was not achieved *versus* the vehicle group.

In conclusion, the dose levels of the Exosome 0 used in this study were well tolerated, but efficacy was emerging but not significant which was confounded by group size. Samples collected from this study could be analysed further for effect on proliferation, angiogenesis, necrosis and apoptosis. Further investigation using larger group sizes and higher dose of Exosome, if tolerated and soluble, could yield significant results.

<u>**Example 22:**</u> Histological Evaluation of Slides for U-87MG human glioblastoma subcutaneous xenografts

### *Summary*

Tissues for histopathological examination (from Example 21) were stained with haematoxylin and eosin before being subjected to histopathological evaluation. This examination was to determine any differences in the appearance of U87 human glioblastoma tumours in animals given Exosome 0 or Temozolomide when compared to those given a vehicle alone.

In one animal given 1mg/kg Exosome 0 there was a particularly dramatic and effective ablation of the tumour mass.

### *Study Aims*

The study was designed to investigate the properties of Exosome 0 in an in vivo model of tumourogenesis. This study was an investigation of the activity of this product in vivo, to assess tolerability and compare with an existing agent (temozolomide).

Temozolomide is an oral chemotherapy drug. It is an alkylating agent used for the treatment of glioblastoma. Temozolomide is also indicated for relapsed Grade III anaplastic astrocytoma, replacing the less well tolerated PCV (Procarbazine-Lomustine-Vincristine) regimen.

### *Methods*

Histological Examination to determine any differences in the appearance of U87 human glioblastoma tumours in animals given Exosome 0 or Temozolomide when compared to those given a vehicle alone.

***Results***

**1. Microscopic Findings**

The tumours examined were large ovoid masses of apparently comparable sizes in the majority of cases, although the masses in animals 12 (Exosome 1.0 mg/kg), 1 and 9 (Temozolomide) were noticeably smaller. The majority of the tumours had necrotic centres and other necrotic foci with in the mass. The extent of the necrosis was quite variable and did affect the appearance of the tumours, but there was no clear difference in the extent of necrosis between the groups. The tumour cells themselves were clonal with a little dysplasia and occasional apoptotic cells. The mitotic rate was relatively low and appeared to be consistent between treatment groups. The response of the host seemed variable, with several showing no evidence of a host response at all while other showed a slight to moderate inflammatory response with some fibroplasia in occasional animals forming a rudimentary capsule.

***a) Vehicle Controls***

The tumours in the vehicle control group all had the appearance as described above. The extent of central necrosis was minimal in animal 20, but in the rest of the animals was fairly extensive. The inflammatory response in 17 was greater than in the other members of this group.

***b) Exosome 0 (1mg/kg)***

The majority of tumours had an appearance that was indistinguishable from the tumours that were seen in the vehicle control animals. There was however one animal (12) where there was a dramatic response. In this animal the tumour appeared to have completely infarcted and there were no viable tumour cells visible in the section presented, only dense fibrous tissue and a slight infiltration of inflammatory cells, a large proportion of which appeared to be macrophages.

***c) Exosome 0 (0.5 mg/kg)***

The tumours in this group had an appearance that was indistinguishable from the tumours that were seen in the vehicle control animals.

***d) Exosome 0 (0.1 mg/kg)***

The tumours in this group had an appearance that was indistinguishable from the tumours that were seen in the vehicle control animals.

***e) Temozolomide (5 mg/kg)***

Three of the tumours in this group had an appearance that was indistinguishable from the tumours that were seen in the vehicle control animals, but there were two animals (1 and 9) where there seemed to be a response to treatment. In both animals the tumour had shrunk to quite a small size but there still appeared to be a substantial number of tumour cells in the section. These cells displayed rather more atypia than was seen in the other tumours, perhaps indicating an selective killing of the majority of cells, but potentially a selection of a more malignant phenotype by the test item. In two of the remaining animals (19 and 25) in this group tumours appeared similar in size to those seen in the vehicle control, there was however a clearly reduce cellularity compared to the vehicle control groups, but the increased atypia seen in animals 1 and 9 was again apparent. In the remaining animal the appearance was similar to the vehicle control.

**2. Discussion and Conclusion**

The consistent appearance of the tumour indicates a robust test system, which is suitable for assessment of efficacy. In one animal given 1mg/kg Exosome 0 there was a particularly dramatic and effective ablation of the tumour mass. In the animals that had received Temozolomide effects were seen in more animals, but the long term efficacy is perhaps more questionable as the effect appeared to be the selection of more atypical tumour cells, potentially with resistance to Temozolomide.

Given the consistency of the tumour appearance, it would suggest that the genotype of the tumours is well preserved and that, without being bound by theory - the large difference seen in animal 12 may be a result of a specific Exozome/host interaction, rather than a direct effect of Exosome 0 on the tumour.

**REFERENCES**

Ambros et al RNA 2003. 9: 277-279
Banerjee, S., Williamson, D., Habib, N., Gordon, M., Chataway, J. (2011) Age and Ageing 40:7-13
Chung et al.,Cell Stem Cell, 2, 113-117, 2008
Dai, L.J., Moniri, M.R., Zeng, Z.R. et al. (2011) Cancer Lett 305(1):8-20.
Ding, D. C., Shyu, W. C., Lin S. Z. (2011) Cell Transplant 20: 5-14
Einstein, O., Ben-Hur, T. (2008) Arch Neurol 65:452-456
Gennaro (2000) Remington: The Science and Practice of Pharmacy. 20th edition, ISBN: 0683306472
Hassani Z, O'Reilly J, Pearse Y, Stroemer P, Tang E, Sinden J, Price J, Thuret S. "Human neural progenitor cell engraftment increases neurogenesis and microglial recruitment in the brain of rats with stroke." PLoS One. 2012;7(11):e50444. doi: 10.1371/journal.pone.0050444. Epub 2012 Nov 21.
Hodges et al. Cell Transplant. 2007;16(2):101-15
Horie, N., Pereira, N.P., Niizuma, K. Sun, G. et al. (2011) Stem Cells 29:274-285.
Hulkower, K. I., Herber, R. L., (2011) Pharmaceutics 3:107-124
Katsuda, Kosaka, Takeshita, Ochiya. Proteomics 2013, 00, 1-17
Katsuda, Tsuchiya, Kosaka, Yoshioka, Takagaki, Oki, Takeshita, Sakai, Kuroda, Ochiya. Scientific Reports 2013, 3:1197, p1-11.
Kornblum, Stroke 2007, 38:810-816
Lai et al "Proteolytic Potential of the MSC Exosome Proteome: Implications for an Exosome-Mediated Delivery of Therapeutic Proteasome". International Journal of Proteomics (2012) Article ID 971907, 14 pages.
Littlewood, T. D., Hancock, D. C., Danielian, P. S. et al. (1995) Nucleic Acid Research 23:1686-1690.
Miljan, E.A. & Sinden, J.D. (2009) Current Opinion in Molecular Therapeutics 4:394-403
Miljan EA, Hines SJ, Pande P, Corteling RL, Hicks C, Zbarsky V, Umachandran, M, Sowinski P, Richardson S, Tang E, Wieruszew M, Patel S, Stroemer P, Sinden JD. Implantation of c-mycER TAM immortalized human mesencephalic-derived clonal cell lines ameliorates behavior dysfunction in a rat model of Parkinson's disease. Stem Cells Dev. 2009 Mar;18(2):307-19
Mitchell et al Journal of Immunological Methods 335 (2008) 98-105
Pollock et al, Exp Neurol. 2006 May;199(1):143-55.
Mark F Pittenger; Alastair M Mackay; Stephen C Beck; Rama K Jaiswal; et al Science; Apr 2, 1999; 284, 5411
Shah, K., (2012) Adv Drug Deliv Rev 64(8):739-748.
Smith, E. J., Stroemer, R.P., Gorenkova, N., Nakajima, M. et al. (2012) Stem Cells 30:785-796.
Stevenato, L., Corteling, R., Stroemer, P., Hope, A. et al. (2009) BMC Neuroscience 10:86
Stroemer, P., Patel, S., Hope, A., Oliveira, C., Pollock, K., Sinden, J. (2009) Neurorehabil Neural Repair 23: 895-909.
Théry, C., Ostrowski, M., Segura, E. et al. (2009) Nature Reviews Immunology 9: 581-593
Their et al, "Direct Conversion of Fibroblasts into Stably Expandable Neural Stem Cells". Cell Stem Cell. 2012 Mar 20.
Timmers, L., Lim, S. K., Arslan, F., Armstrong, J. S. et al. (2007) Stem Cell Res 1: 129-137
Yuan, S.J., Martin, J,, Elia, J., Flippin, J. et al. (2011) PLoS ONE 6:e17540

## Claims

1. A neural stem cell exosome for use in a method of treating glioma.

2. The neural stem cell exosome for use according to claim 1, wherein the glioma is glioblastoma.

3. The neural stem cell exosome for use according to claim 2, wherein the exosome (i) inhibits glioblastoma cell migration and/or (ii) induces differentiation of a glioblastoma cell.

4. The neural stem cell exosome for use according to claim 2 or claim 3, wherein the exosome:

   inhibits glioblastoma cell migration as determined using a transmembrane cell migration assay; and/or
   induces differentiation as determined by a reduction in nestin expression.

5. The neural stem cell exosome for use according to any one of claims 1-4, wherein the exosome is derived from a neural stem cell that:

   (a) is proliferating;
   (b) does not express DCX or GFAP; and/or
   (c) has been cultured in a multi-compartment bioreactor for less than 4 weeks and optionally no more than 1 week.

6. The neural stem cell exosome for use according to any one of claims 1-5, wherein the exosome is derived from a neural stem cell line, optionally wherein the neural stem cell line is conditionally-immortalised and/or grown in serum free medium, and optionally wherein the neural stem cell line is CTX0E03 having ECACC Accession No. 04091601, STR0C05 having ECACC Accession No.04110301 and HPC0A07 having ECACC Accession No.04092302.

7. The neural stem cell exosome for use according to any preceding claim, wherein the exosome has:

   (a) a size of between 30 nm and 1000 nm, or between 30 and 200 nm, or between 30 and 100 nm, as determined by electron microscopy; or
   (b) a density in sucrose of 1.1-1.2 g/ml.

8. The neural stem cell exosome for use according to any preceding claim, comprising RNA, optionally wherein the RNA is mRNA and/or miRNA, optionally wherein the exosome comprises:

   one, two, three or four of hsa-miR-1246, hsa-miR-4492, hsa-miR-4488 and hsa-miR-4532;
   one, two, three, four or five of hsa-miR-181a-5p, hsa-miR-1246, hsa-miR-127-3p, hsa-miR-21-5p, and hsa-miR-100-5p;
   one, two, three, four or five of hsa-miR-181a-5p, hsa-let-7a-5p, hsa-let-7f-5p, hsa-miR-92b-3p, and hsa-miR-9-5p; or
hsa-miR-486-5p.

**9.** The neural stem cell exosome for use according to any preceding claim, comprising at least 10 of the proteins present in Table 20 or Table 22; and/or comprising one or more of:

(a) a lipid selected from ceramide, cholesterol, sphingomyelin, phosphatidylserine, phosphatidylinositol, and/or phosphatidylcholine;
(b) miRNA, optionally selected from hsa-let-7g, hsa-miR-101, hsa-miR-10a, hsa-miR-10b, hsa-miR-126, hsa-miR-128, hsa-miR-129-5p, hsa-miR-130a, hsa-miR-134, hsa-miR-137, hsa-miR-155, hsa-miR-15a, hsa-miR-15b, hsa-miR-16, hsa-miR-17, hsa-miR-182, hsa-miR-183, hsa-miR-185, hsa-miR-18b, hsa-miR-192, hsa-miR-194, hsa-miR-195, hsa-miR-20a, hsa-miR-20b, hsa-miR-210, hsa-miR-218, hsa-miR-301a, hsa-miR-302a, hsa-miR-302c, hsa-miR-345, hsa-miR-375, hsa-miR-378, hsa-miR-7, hsa-miR-9, hsa-miR-93, hsa-miR-96, and hsa-miR-99a;
(c) a tetraspanin, optionally selected from CD63, CD81,CD9, CD53, CD82 and/or CD37;
(d) TSG101, Alix, CD109 and/or thy-1; and/or
(e) CD133.

**10.** The neural stem cell exosome for use according to any of claims 1 to 9, wherein the glioma is treated by inhibiting migration of the cancer cells or wherein the glioma is treated by inducing differentiation of the cancer cells; optionally wherein the exosome is as defined in claim 5

**Patentansprüche**

**1.** Neuronales Stammzellenexosom zur Anwendung in einem Verfahren zur Behandlung von Gliomen.

**2.** Neuronales Stammzellenexosom zur Anwendung nach Anspruch 1, wobei das Gliom ein Glioblastom ist.

**3.** Neuronales Stammzellenexosom zur Anwendung nach Anspruch 2, wobei das Exosom (i) Zellmigration von Glioblastomen hemmt und/oder (ii) die Differenzierung einer Glioblastomzelle bewirkt.

**4.** Neuronales Stammzellenexosom zur Anwendung nach einem der Ansprüche 2 oder 3, wobei das Exosom:

Zellmigration von Glioblastomen wie vorgegeben unter Anwendung eines transmembranen Zellmigrationstests hemmt; und/oder
die Differenzierung wie vorgegeben durch eine Nestinexpressionreduzierung bewirkt.

**5.** Neuronales Stammzellenexosom zur Anwendung nach einem der Ansprüche 1-4, wobei das Exosom von einer neuronalen Stammzelle abgeleitet ist, die:

(a) sich vermehrt;
(b) weder DCX noch GFAP exprimiert; und/oder
(c) weniger als 4 Wochen und gegebenenfalls nicht mehr als 1 Woche in einem Bioreaktor mit mehreren Abteilungen kultiviert wurde.

**6.** Neuronales Stammzellenexosom zur Anwendung nach einem der Ansprüche 1-5, wobei das Exosom von einer neuronalen Stammzellenlinie abgeleitet ist, wobei die neuronale Stammzellenlinie gegebenenfalls bedingt verewigt und/oder in einem serumfreien Medium gewachsen ist, und wobei die neuronale Stammzellenlinie gegebenenfalls CTX0E03 mit der ECACC-Zugangsnummer 04091601, STR0C05 mit der ECACC-Zugangsnummer 04110301 und HPC0A07 mit der ECACC-Zugangsnummer 04092302 ist.

**7.** Neuronales Stammzellenexosom zur Anwendung nach einem der vorhergehenden Ansprüche, wobei das Exosom:

(a) eine Größe zwischen 30 nm und 1000 nm oder zwischen 30 und 200 nm oder zwischen 30 und 100 nm, wie durch Elektronenmikroskopie vorgegeben, aufweist; oder
(b) eine Saccharose-Dichte von 1,1-1,2 g/ml aufweist.

8. Neuronales Stammzellenexosom zur Anwendung nach einem der vorhergehenden Ansprüche, das RNA umfasst, wobei das RNA gegebenenfalls mRNA und/oder miRNA ist, wobei das Exosom gegebenenfalls Folgendes umfasst:

   ein, zwei, drei oder vier hsa-miR-1246, hsa-miR-4492, hsa-miR-4488 und hsa- miR-4532;
   ein, zwei, drei, vier oder fünf hsa-miR-181a-5p, hsa-miR-1246, hsa-miR-127-3p, hsa-miR-21-5p, und hsa-miR-100-5p;
   ein, zwei, drei, vier oder fünf hsa-miR-181a-5p, hsa-let-7a-5p, hsa-let-7f-5p, hsa-miR-92b-3p, und hsa-miR-9-5p; oder hsa-miR-486-5p.

9. Neuronales Stammzellenexosom zur Anwendung nach einem der vorhergehenden Ansprüche, das mindestens 10 der in Tabelle 20 oder Tabelle 22 angegebenen Proteine umfasst; und/oder eines oder mehrere der Folgenden umfasst:

   (a) ein aus Ceramid, Cholesterin, Sphingomyelin, Phosphatidylserin, Phosphatidylinositol und/oder Phosphatidylcholin ausgewähltes Lipid;
   (b) miRNA, gegebenenfalls ausgewählt aus hsa-let-7g, hsa-miR-101, hsa-miR-10a, hsa-miR- 10b, hsa-miR-126, hsa-miR-128, hsa-miR-129-5p, hsa-miR-130a, hsa-miR-134, hsa-miR-137, hsa-miR-155, hsa-miR-15a, hsa-miR-15b, hsa-miR-16, hsa-miR-17, hsa- miR-182, hsa-miR-183, hsa-miR-185, hsa-miR-18b, hsa-miR-192, hsa-miR-194, hsa- miR-195, hsa-miR-20a, hsa-miR-20b, hsa-miR-210, hsa-miR-218, hsa-miR-301a, hsa-miR-302a, hsa-miR-302c, hsa-miR-345, hsa-miR-375, hsa-miR-378, hsa-miR-7, hsa-miR-9, hsa-miR-93, hsa-miR-96, und hsa-miR-99a;
   (c) ein Tetraspanin, das gegebenenfalls aus CD63, CD81,CD9, CD53, CD82 und/oder CD37 ausgewählt ist;
   (d) TSG101, Alix, CD109 und/oder thy-1; und/oder
   (e) CD133.

10. Neuronales Stammzellenexosom zur Anwendung nach einem der Ansprüche 1-9, wobei das Gliom durch Hemmen der Migration von Krebszellen behandelt wird oder wobei das Gliom durch Bewirken einer Differenzierung der Krebszellen behandelt wird; wobei das Exosom gegebenenfalls nach Anspruch 5 definiert wird.

**Revendications**

1. Exosome de cellules souches neurales destiné à être utilisé dans un procédé de traitement du gliome.

2. Exosome de cellules souches neurales destiné à être utilisé selon la revendication 1, dans lequel le gliome est le glioblastome.

3. Exosome de cellules souches neurales destiné à être utilisé selon la revendication 2, dans lequel l’exosome (i) inhibe la migration de cellules de glioblastome et/ou (ii) induit la différenciation d’une cellule de glioblastome.

4. Exosome de cellules souches neurales destiné à être utilisé selon la revendication 2 ou la revendication 3, dans lequel l’exosome :

   inhibe la migration de cellules de glioblastome telle que déterminée en utilisant un essai de migration de cellules transmembranaires ; et/ou
   induit la différenciation telle que déterminée par une réduction en termes d’expression de nestine.

5. Exosome de cellules souches neurales destiné à être utilisé selon l’une quelconque des revendications 1 à 4, dans lequel l’exosome est dérivé d’une cellule souche neurale qui :

   (a) prolifère ;
   (b) n’exprime pas le DCX ou le GFAP ; et/ou
   (c) a été mise en culture dans un bioréacteur à compartiments multiples pendant moins de 4 semaines et éventuellement pas plus de 1 semaine.

6. Exosome de cellules souches neurales destiné à être utilisé selon l’une quelconque des revendications 1 à 5, dans lequel l’exosome est dérivé d’une lignée de cellules souches neurales, dans lequel la lignée de cellules souches neurales est éventuellement immortalisée sous condition et/ou cultivée dans un milieu exempt de sérum, et dans lequel la lignée de cellules souches neurales est éventuellement CTX0E03 ayant le numéro d'accès 04091601 à l'ECACC, STR0C05 ayant le numéro d'accès 04110301 à l'ECACC et HPC0A07 ayant le numéro d'accès 04092302 à l'ECACC.

**7.** Exosome de cellules souches neurales destiné à être utilisé selon l'une quelconque des revendications précédentes, dans lequel l'exosome comporte :

(a) une taille comprise entre 30 nm et 1 000 nm, ou entre 30 et 200 nm, ou entre 30 et 100 nm, telle que déterminée par microscopie électronique ; ou
(b) une densité dans le sucrose comprise entre 1,1 et 1,2 g/ml.

**8.** Exosome de cellules souches neurales destiné à être utilisé selon l'une quelconque des revendications précédentes, comprenant de l'ARN, dans lequel l'ARN est éventuellement l'ARNm et/ou l'ARNmi, dans lequel l'exosome comprend éventuellement :

un, deux, trois ou quatre parmi hsa-miR-1246, hsa-miR-4492, hsa-miR-4488 et hsa-miR-4532 ;
un, deux, trois, quatre ou cinq parmi hsa-miR-181a-5p, hsa-miR-1246, hsa-miR-127-3p, hsa-miR-21-5p, et hsa-miR-100-5p ;
un, deux, trois, quatre ou cinq parmi hsa-miR-181a-5p, hsa-let-7a-5p, hsa-let-7f-5p, hsa-miR-92b-3p, et hsa-miR-9-5p ; ou hsa-miR-486-5p.

**9.** Exosome de cellules souches neurales destiné à être utilisé selon l'une quelconque des revendications précédentes, comprenant au moins 10 des protéines présentes dans le Tableau 20 ou dans le Tableau 22 ; et/ou comprenant l'au moins un parmi :

(a) un lipide choisi parmi le céramide, le cholestérol, la sphingomyéline, la phosphatidylsérine, le phosphatidylinositol, et/ou la phosphatidylcholine ;
(b) l'ARNmi, éventuellement choisi parmi hsa-let-7g, hsa-miR-101, hsa-miR-10a, hsa-miR- 10b, hsa-miR-126, hsa-miR-128, hsa-miR-129-5p, hsa-miR-130a, hsa-miR-134, hsa-miR-137, hsa-miR-155, hsa-miR-15a, hsa-miR-15b, hsa-miR-16, hsa-miR-17, hsa- miR-182, hsa-miR-183, hsa-miR-185, hsa-miR-18b, hsa-miR-192, hsa-miR-194, hsa- miR-195, hsa-miR-20a, hsa-miR-20b, hsa-miR-210, hsa-miR-218, hsa-miR-301a, hsa-miR-302a, hsa-miR-302c, hsa-miR-345, hsa-miR-375, hsa-miR-378, hsa-miR-7, hsa-miR-9, hsa-miR-93, hsa-miR-96, et hsa-miR-99a ;
(c) une tétraspanine, éventuellement choisie parmi CD63, CD81,CD9, CD53, CD82 et/ou CD37 ;
(d) TSG101, Alix, CD109 et/ou thy-1 ; et/ou
(e) CD133.

**10.** Exosome de cellules souches neurales destiné à être utilisé selon l'une quelconque des revendications 1 à 9, dans lequel le gliome est traité en inhibant la migration des cellules cancéreuses ou dans lequel le gliome est traité en induisant la différenciation des cellules cancéreuses ; dans lequel l'exosome est éventuellement tel que défini selon la revendication 5.

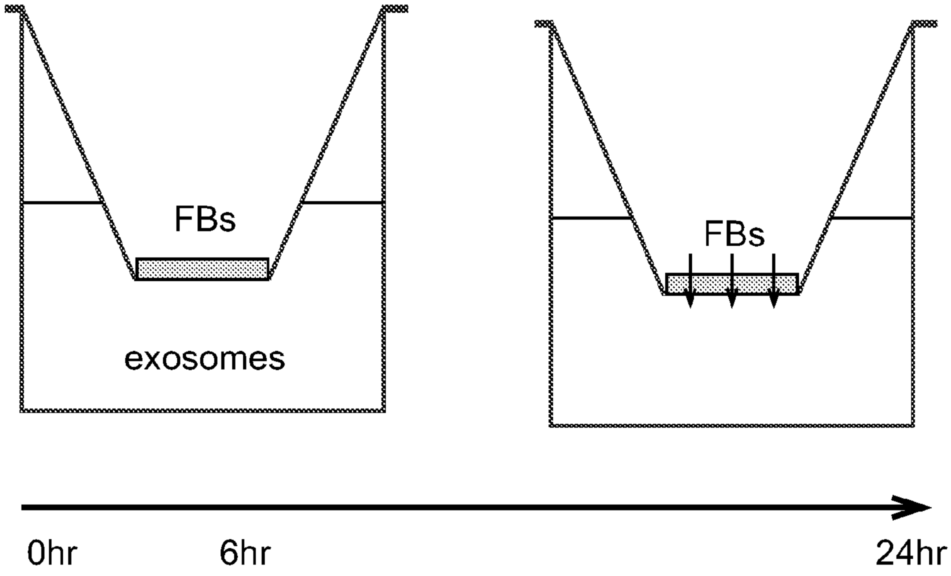
FBs
exosomes
FBs
0hr
6hr
24hr

Figure 1

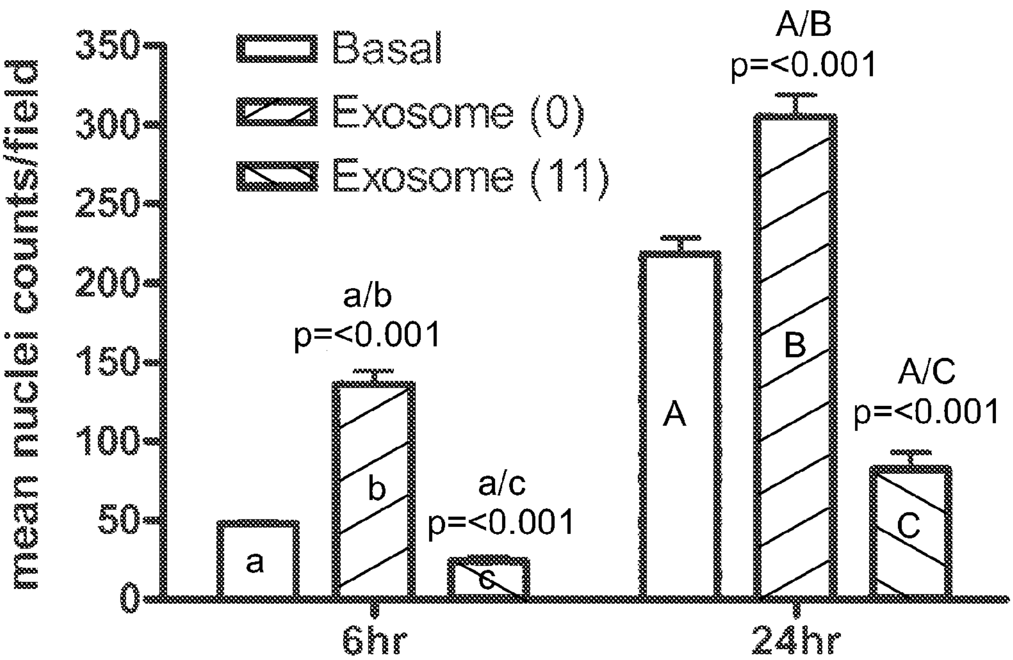
mean nuclei counts/field
350
300
250
200
150
100
50
0
Basal
Exosome (0)
Exosome (11)
a/b
p=<0.001
a/c
p=<0.001
A/B
p=<0.001
A/C
p=<0.001
a
b
c
A
B
C
6hr
24hr

Figure 2

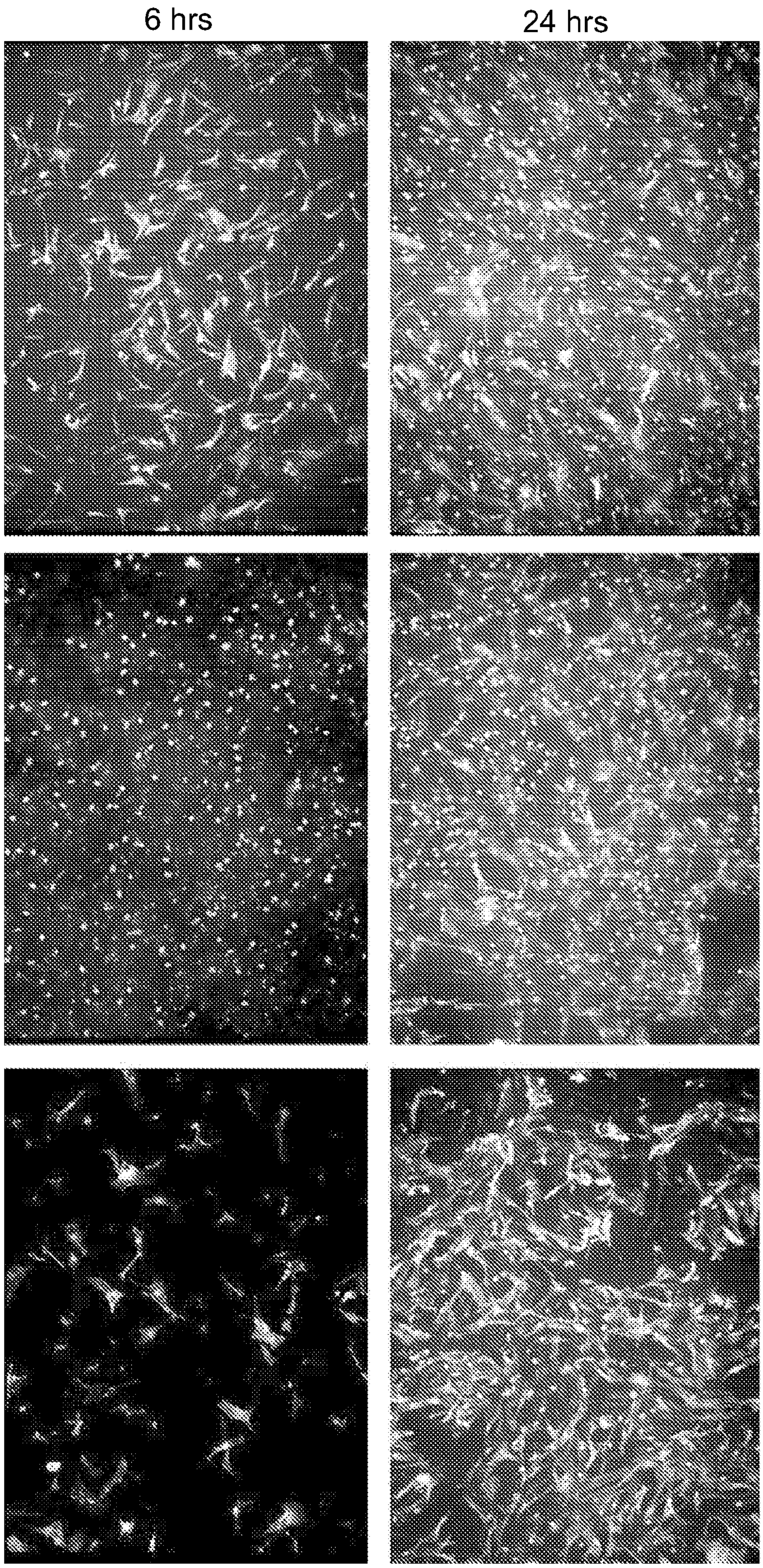

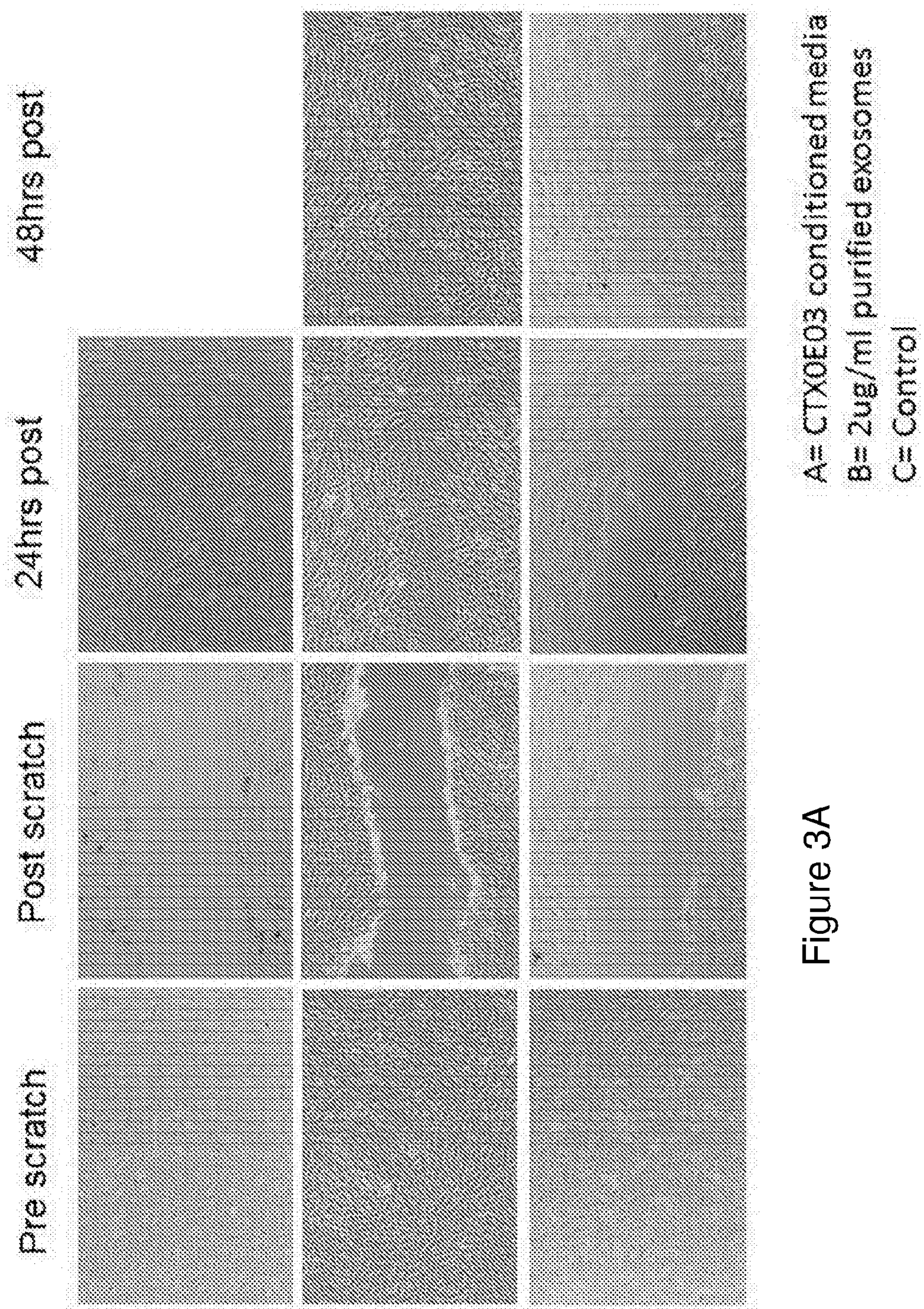
Pre scratch
Post scratch
24hrs post
48hrs post
A
B
C
A= CTX0E03 conditioned media
B= 2ug/ml purified exosomes
C= Control

Figure 3A

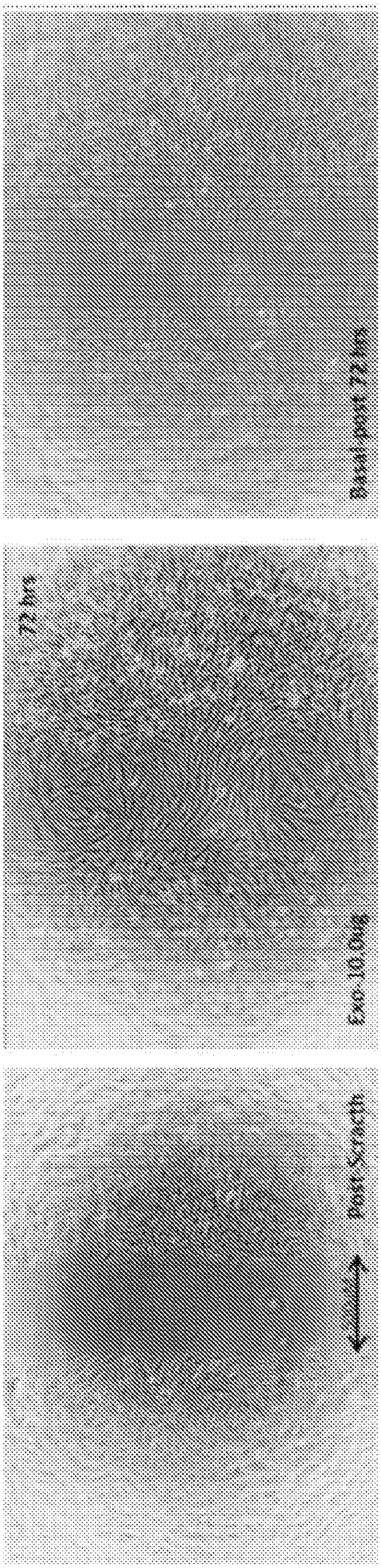


Figure 3B

# Figure 3C

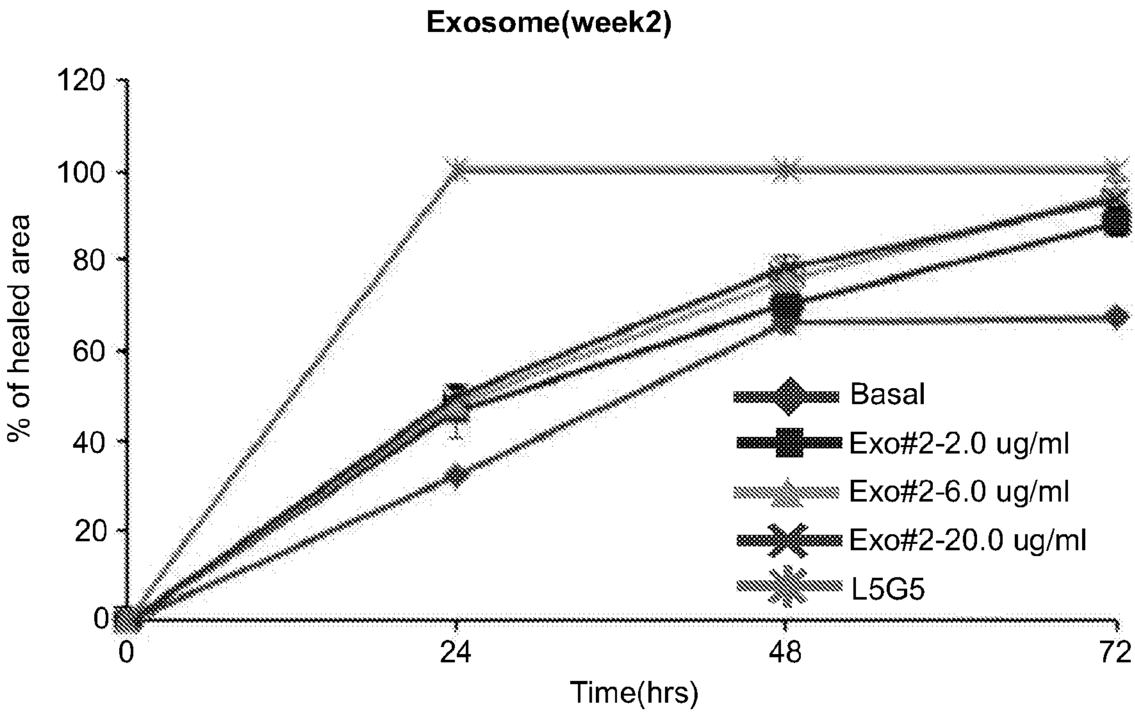
Exosome(week2)
120
100
80
60
40
20
0
% of healed area
0
24
48
72
Time(hrs)
Basal
Exo#2-2.0 ug/ml
Exo#2-6.0 ug/ml
Exo#2-20.0 ug/ml
L5G5

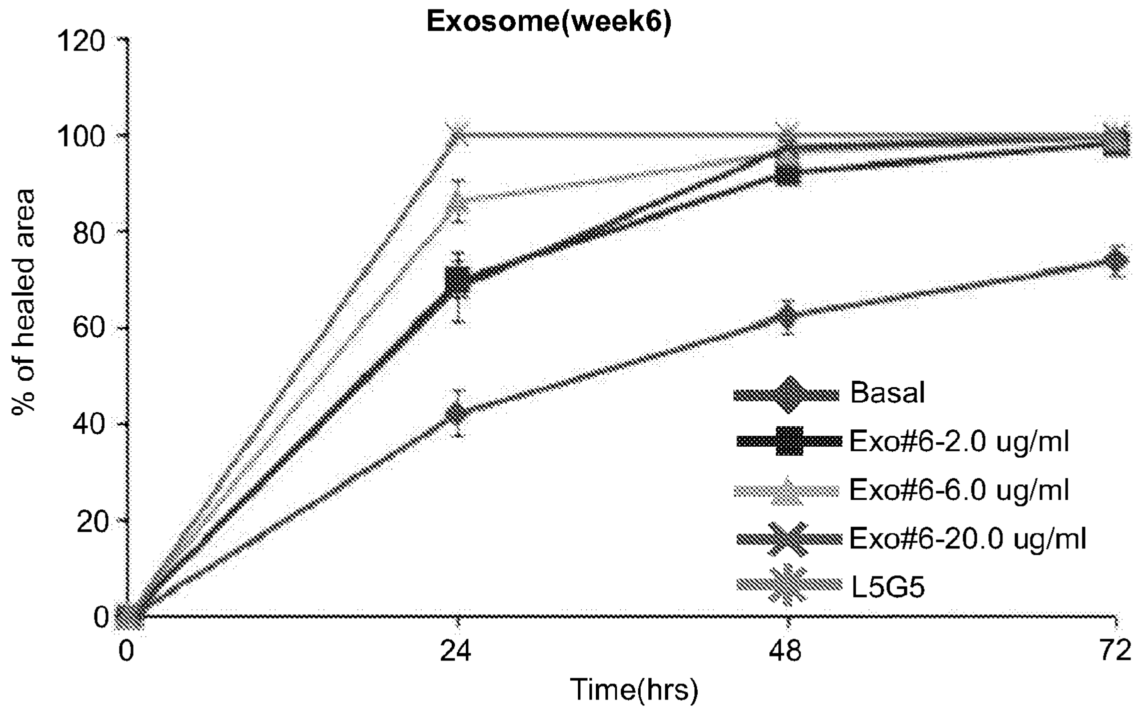
Exosome(week6)
120
100
80
60
40
20
0
% of healed area
0
24
48
72
Time(hrs)
Basal
Exo#6-2.0 ug/ml
Exo#6-6.0 ug/ml
Exo#6-20.0 ug/ml
L5G5

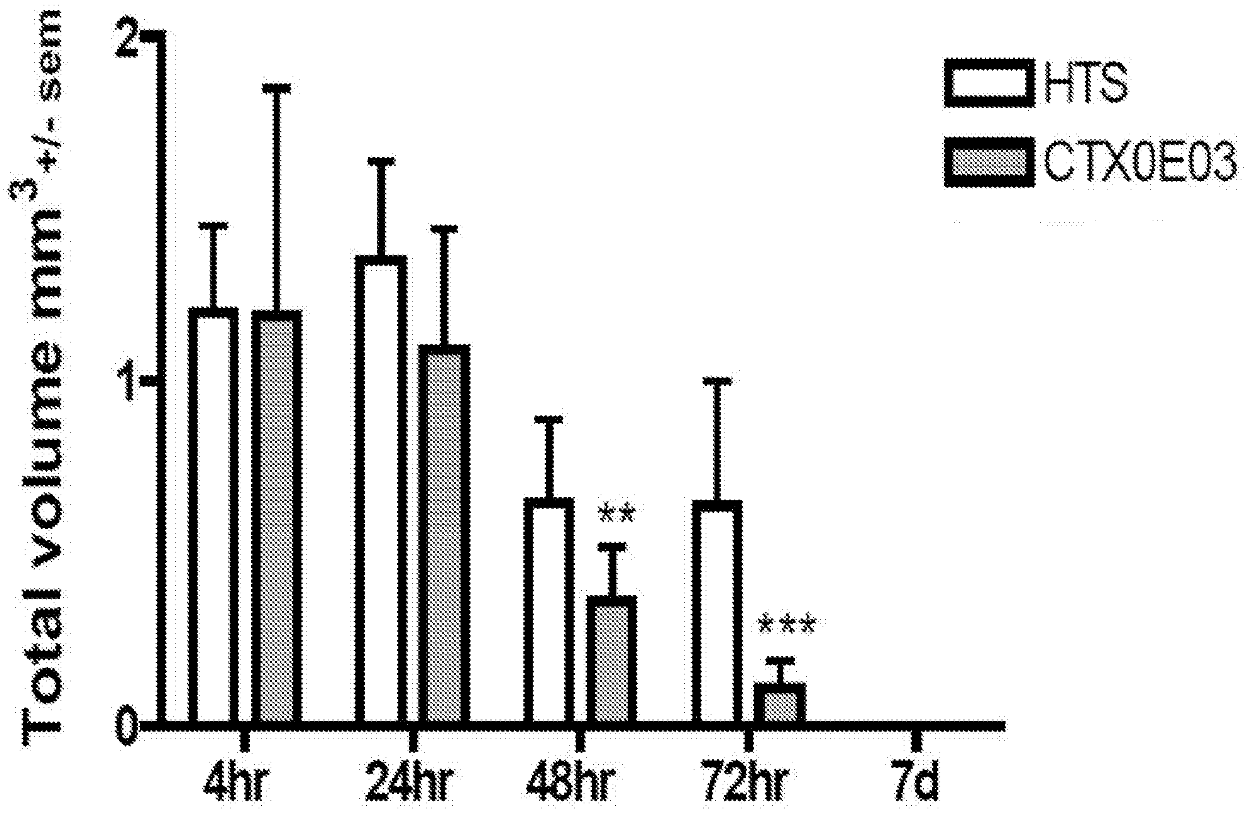


Figure 3D

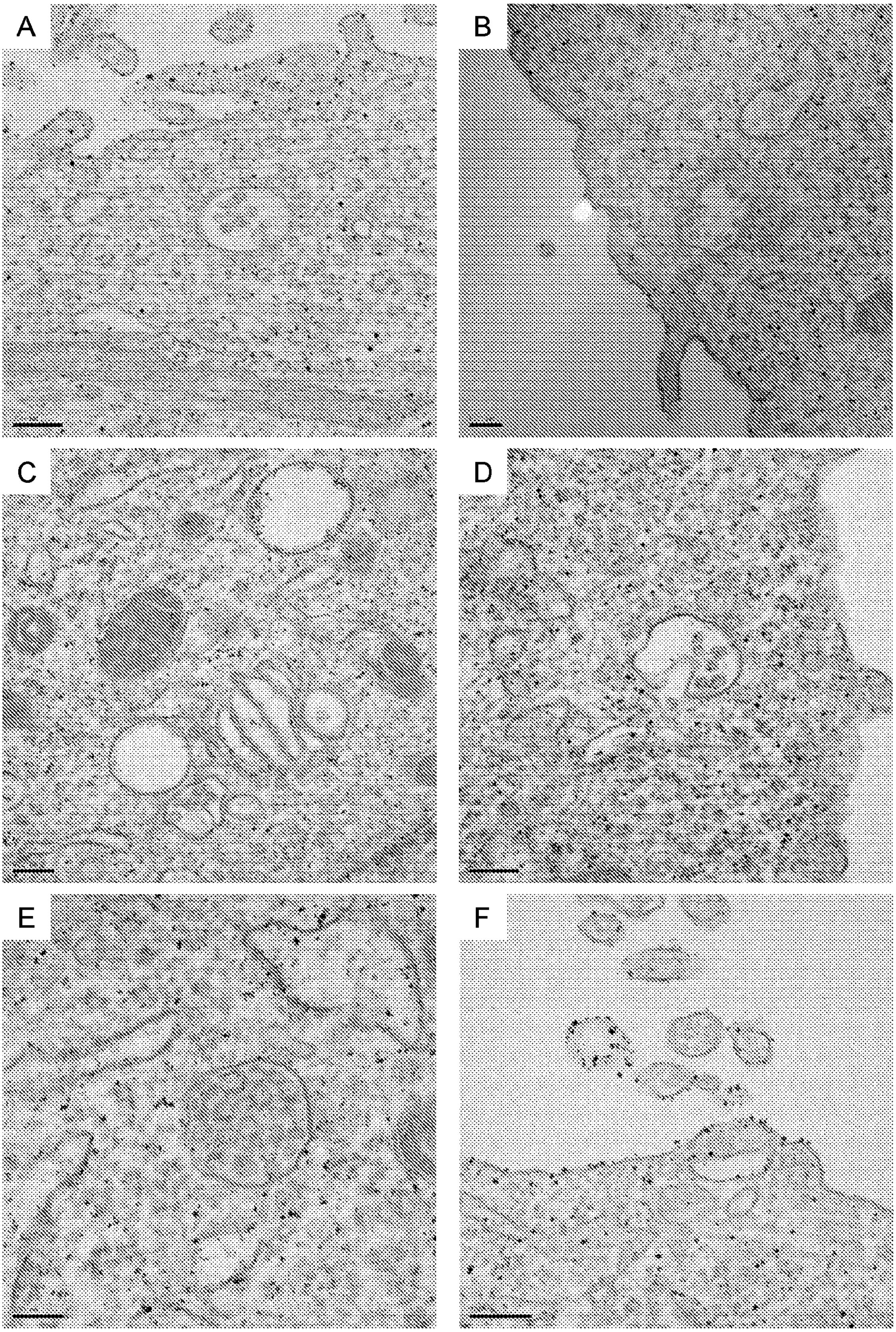


Figure 4

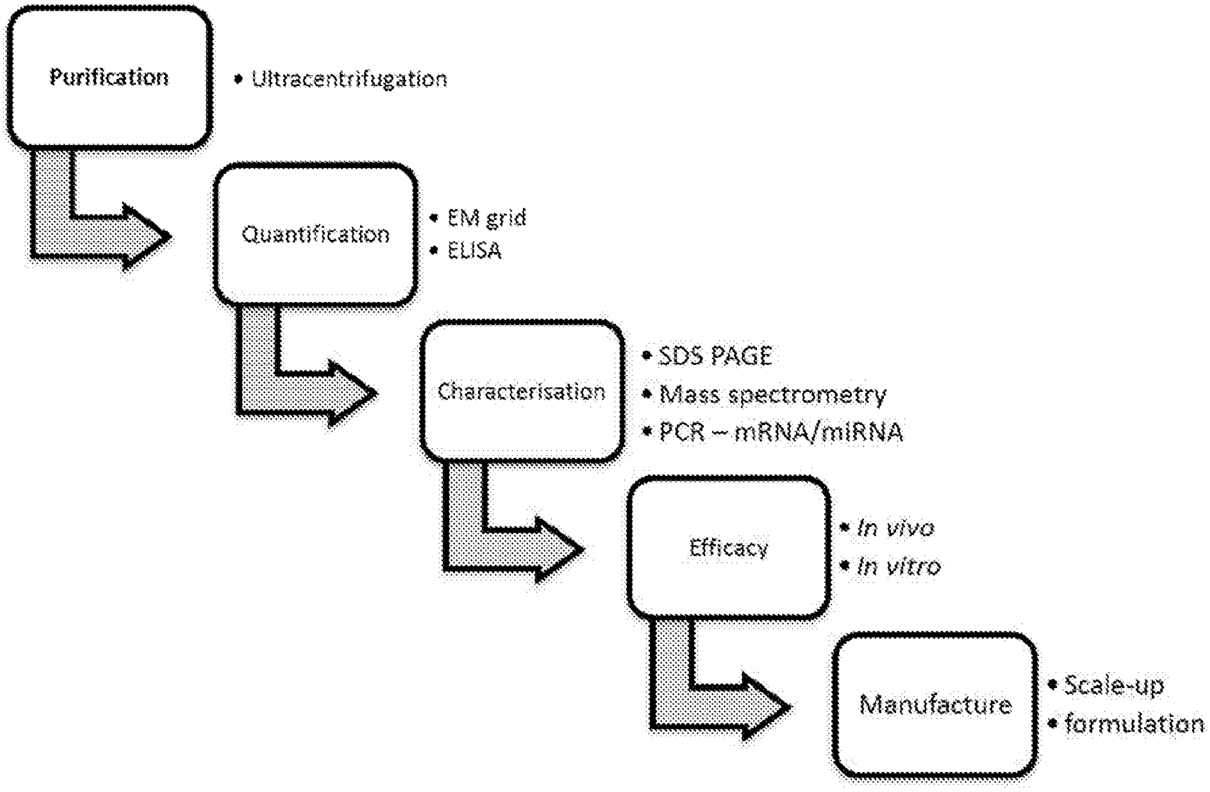
Purification
• Ultracentrifugation
Quantification
• EM grid
• ELISA
Characterisation
• SDS PAGE
• Mass spectrometry
• PCR – mRNA/miRNA
Efficacy
• In vivo
• In vitro
Manufacture
• Scale-up
• formulation

Figure 5

Figure 6

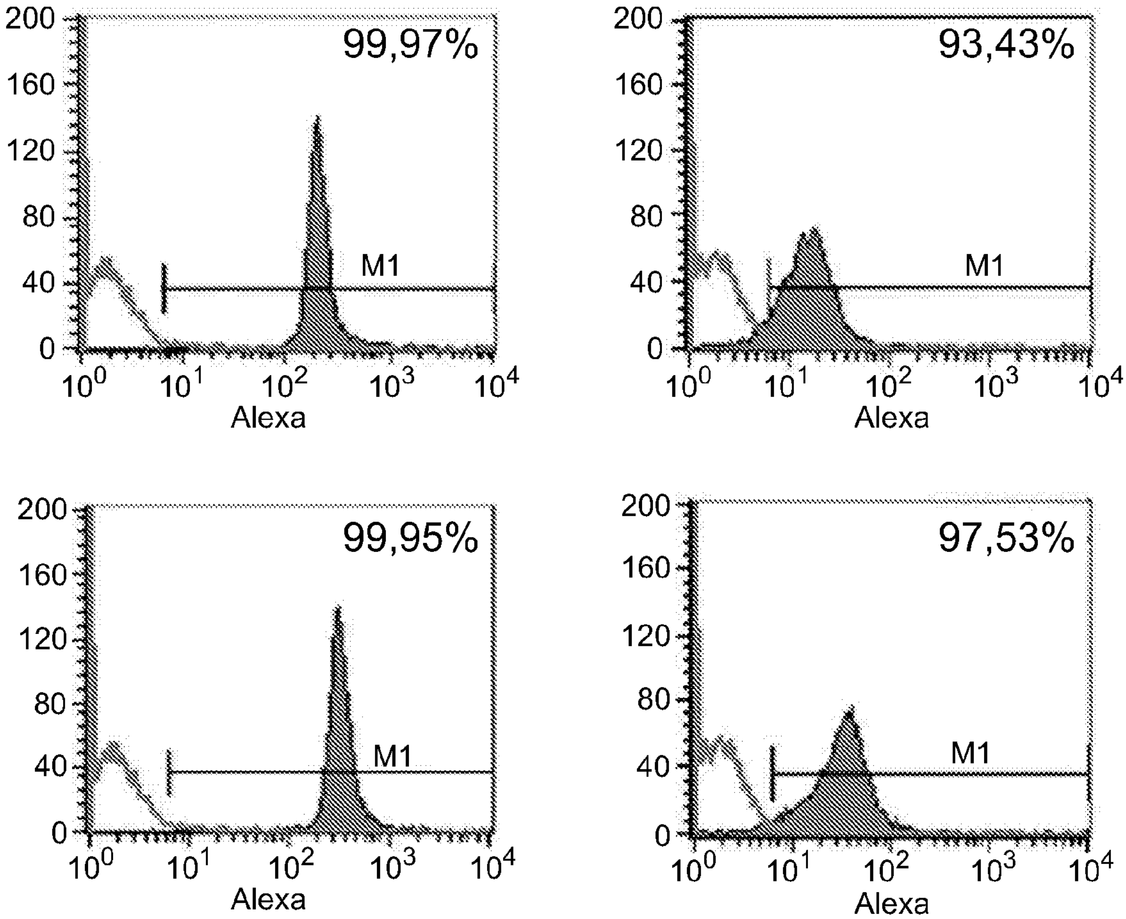
200
160
120
80
40
0
99,97%
M1
$10^0$ $10^1$ $10^2$ $10^3$ $10^4$
Alexa
93,43%
M1
Alexa
99,95%
M1
Alexa
97,53%
M1
Alexa

Figure 7A

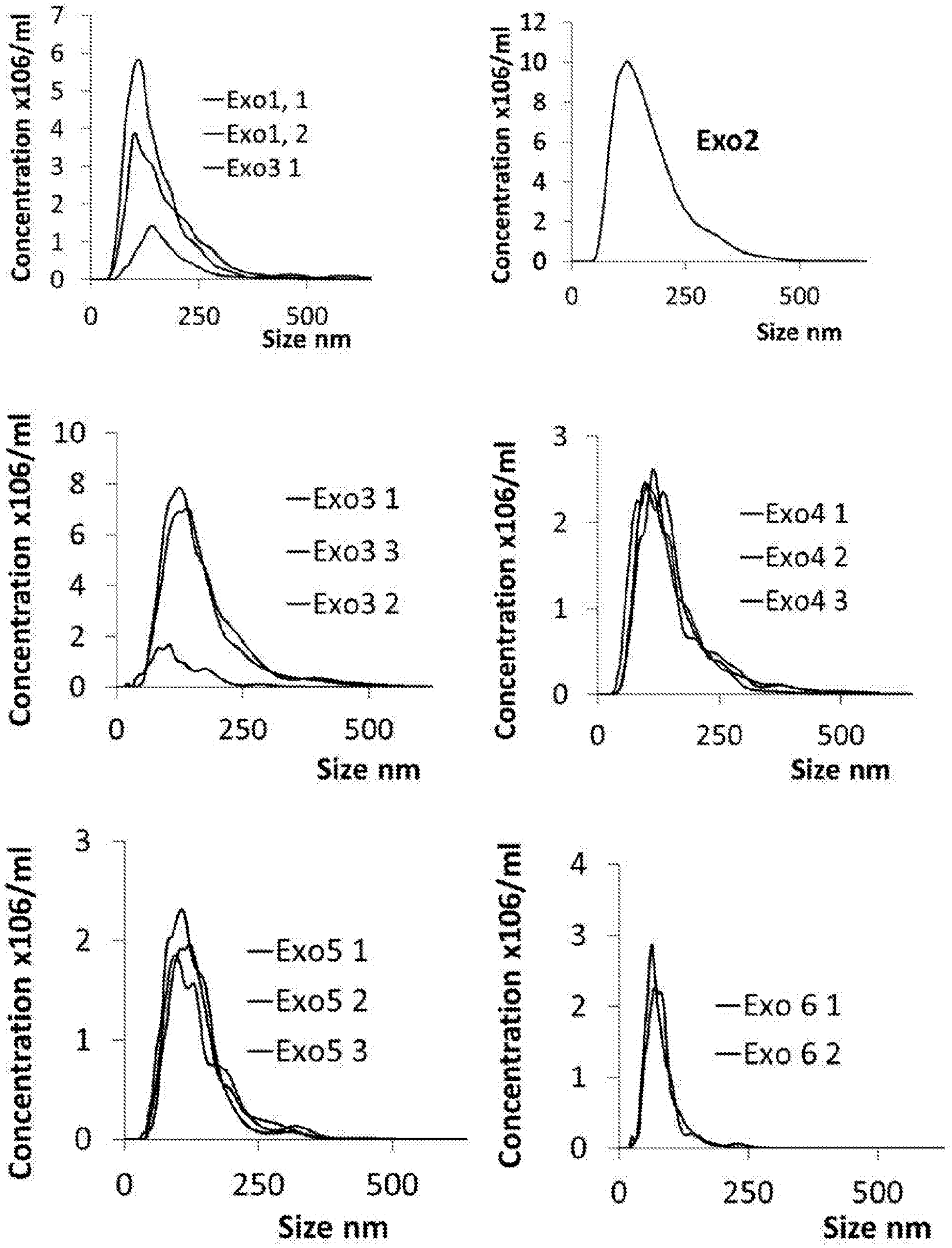
Concentration x106/ml
Size nm
Exo1, 1
Exo1, 2
Exo3 1
Exo2
Exo3 1
Exo3 3
Exo3 2
Exo4 1
Exo4 2
Exo4 3
Exo5 1
Exo5 2
Exo5 3
Exo 6 1
Exo 6 2

Figure 7B

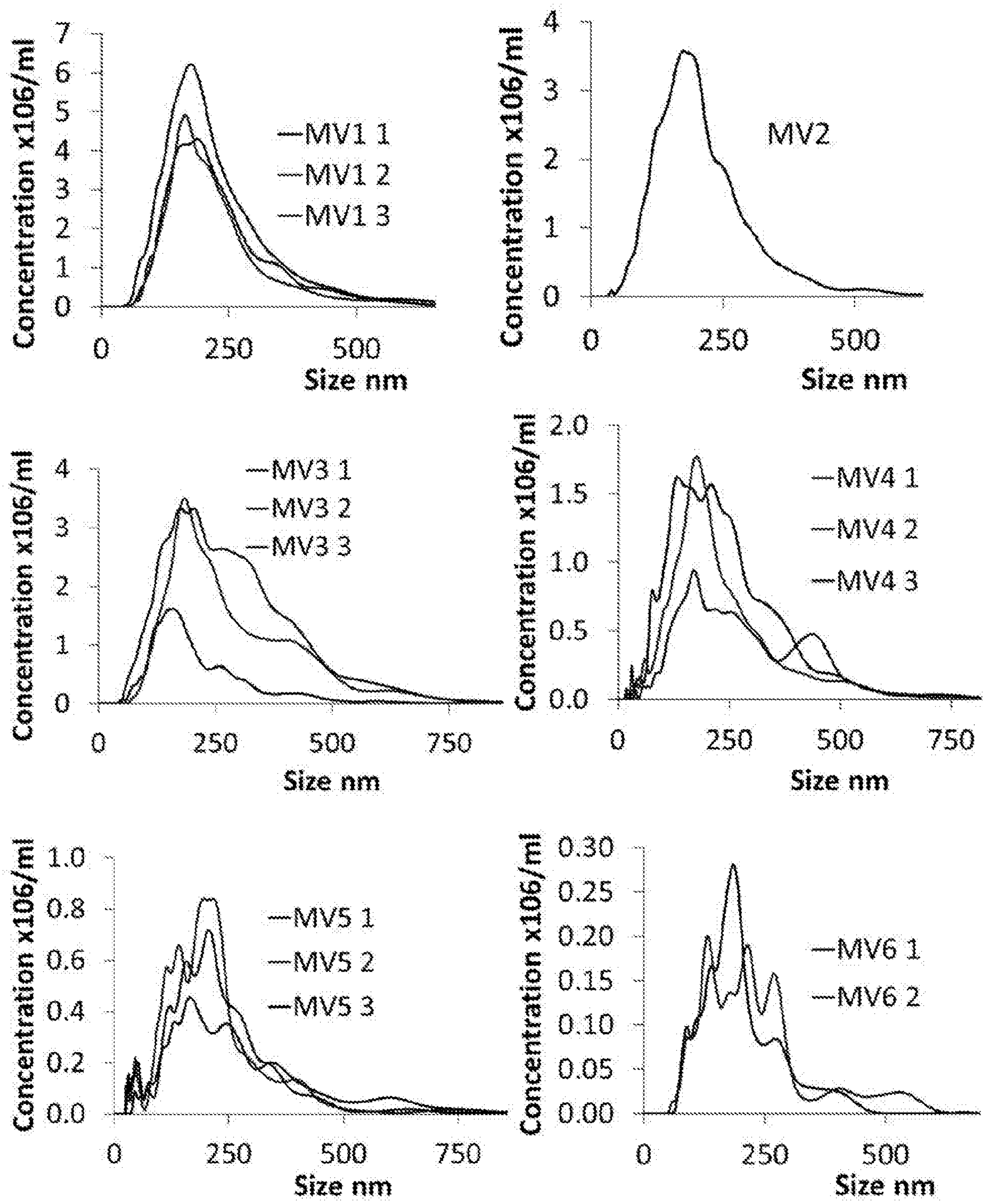
Concentration x106/ml
Size nm
MV1 1
MV1 2
MV1 3
MV2
MV3 1
MV3 2
MV3 3
MV4 1
MV4 2
MV4 3
MV5 1
MV5 2
MV5 3
MV6 1
MV6 2

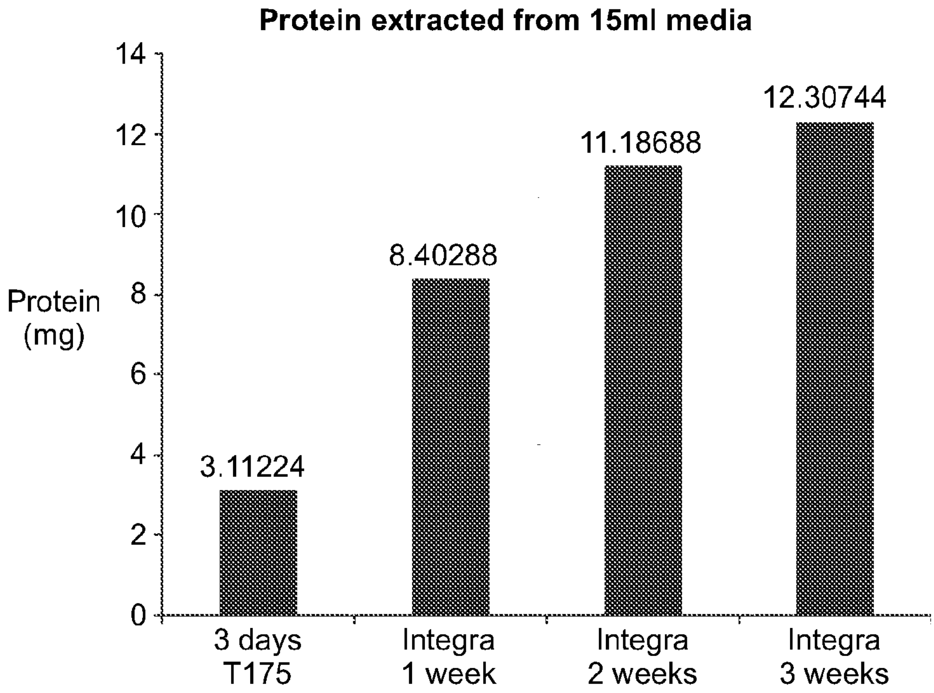


Figure 8A

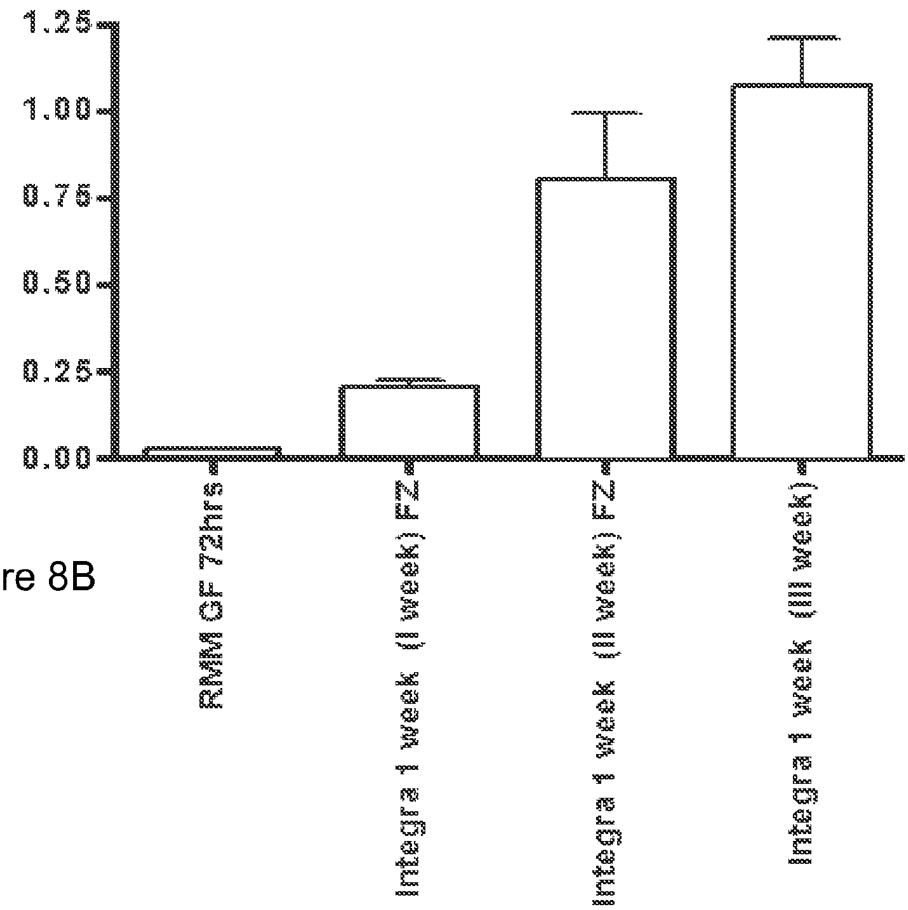


Figure 8B

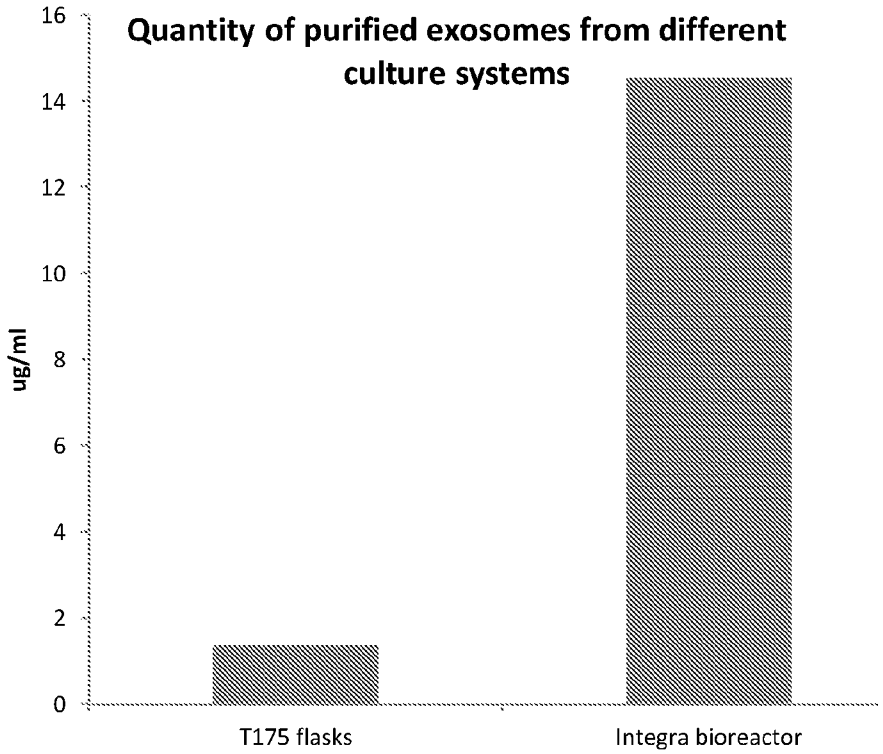
Quantity of purified exosomes from different culture systems
16
14
12
10
8
6
4
2
0
ug/ml
T175 flasks
Integra bioreactor

Figure 9

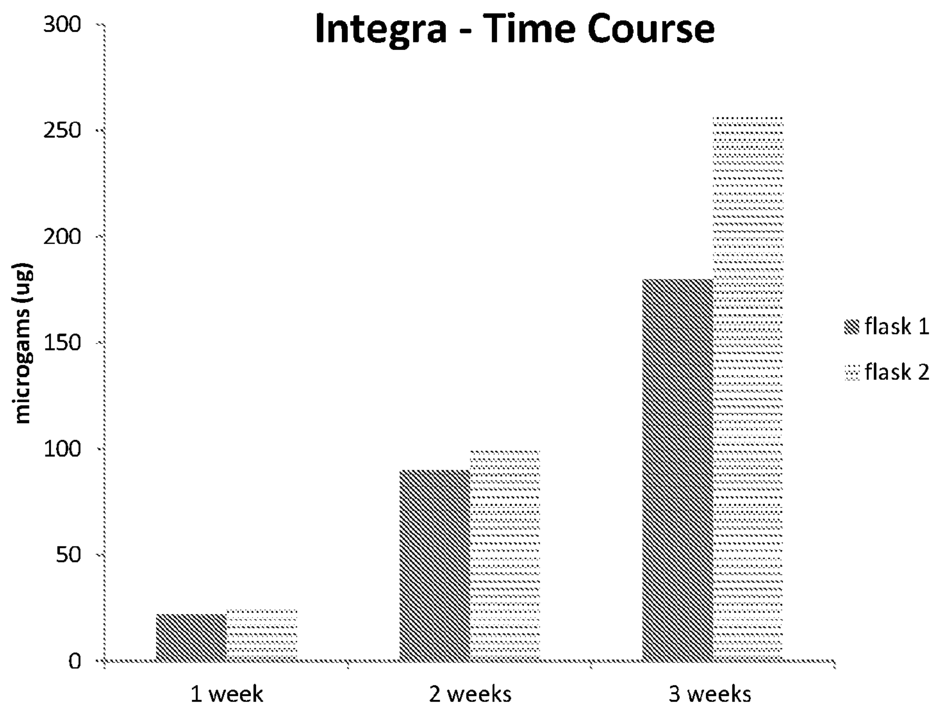


Figure 10A

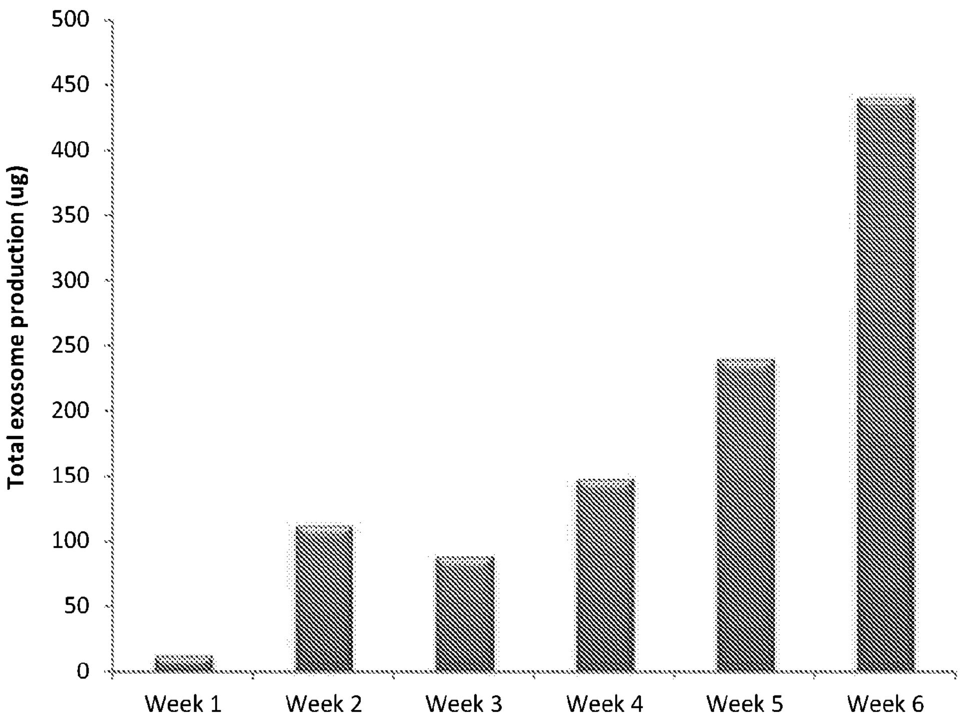


Figure 10B

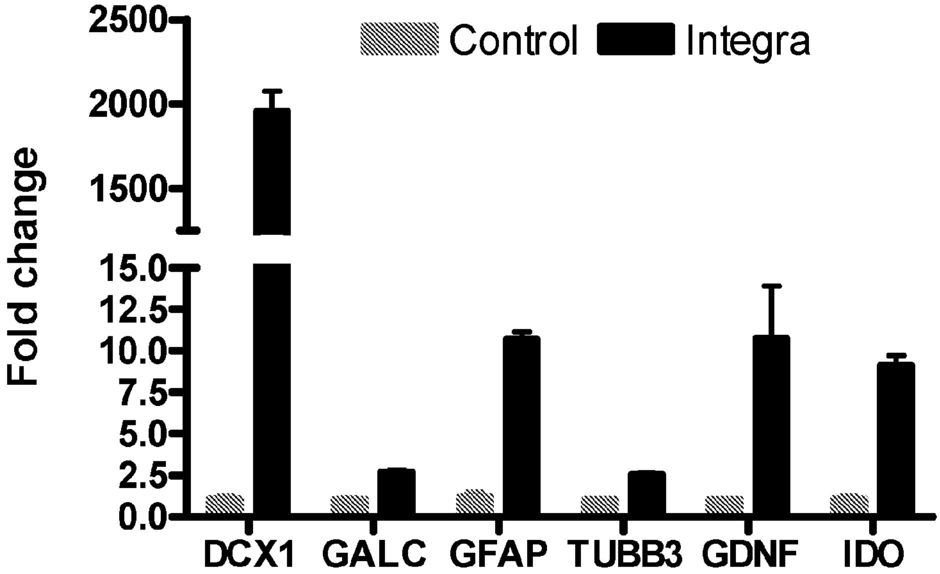
2500
2000
1500
15.0
12.5
10.0
7.5
5.0
2.5
0.0
Fold change
Control
Integra
DCX1
GALC
GFAP
TUBB3
GDNF
IDO

Figure 11

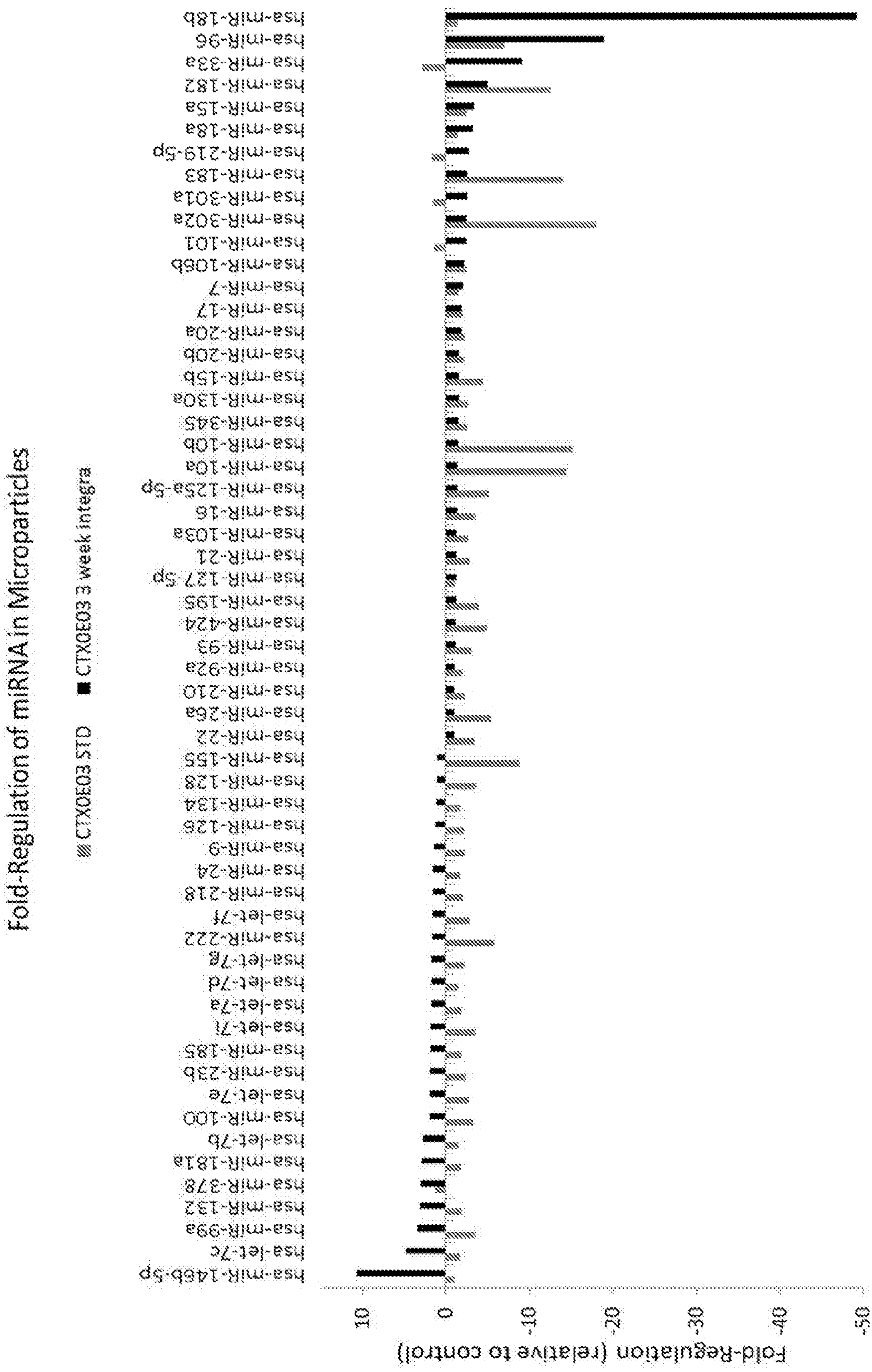
Fold-Regulation of miRNA in Microparticles
CTXOE03 STD
CTXOE03 3 week integra
Fold-Regulation (relative to control)
10
0
-10
-20
-30
-40
-50
hsa-miR-18b
hsa-miR-96
hsa-miR-33a
hsa-miR-182
hsa-miR-15a
hsa-miR-18a
hsa-miR-219-5p
hsa-miR-183
hsa-miR-301a
hsa-miR-302a
hsa-miR-101
hsa-miR-106b
hsa-miR-7
hsa-miR-17
hsa-miR-20a
hsa-miR-20b
hsa-miR-15b
hsa-miR-130a
hsa-miR-345
hsa-miR-10b
hsa-miR-10a
hsa-miR-125a-5p
hsa-miR-16
hsa-miR-103a
hsa-miR-21
hsa-miR-127-5p
hsa-miR-195
hsa-miR-424
hsa-miR-93
hsa-miR-92a
hsa-miR-210
hsa-miR-26a
hsa-miR-22
hsa-miR-155
hsa-miR-128
hsa-miR-134
hsa-miR-126
hsa-miR-9
hsa-miR-24
hsa-miR-218
hsa-let-7f
hsa-miR-222
hsa-let-7g
hsa-let-7d
hsa-let-7a
hsa-let-7i
hsa-miR-185
hsa-miR-23b
hsa-let-7e
hsa-miR-100
hsa-let-7b
hsa-miR-181a
hsa-miR-378
hsa-miR-132
hsa-miR-99a
hsa-let-7c
hsa-miR-146b-5p

Figure 12

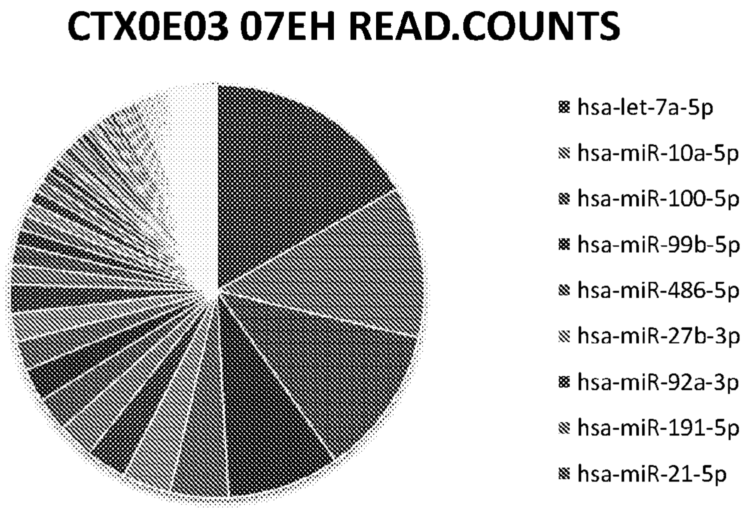
CTX0E03 07EH READ.COUNTS
hsa-let-7a-5p
hsa-miR-10a-5p
hsa-miR-100-5p
hsa-miR-99b-5p
hsa-miR-486-5p
hsa-miR-27b-3p
hsa-miR-92a-3p
hsa-miR-191-5p
hsa-miR-21-5p

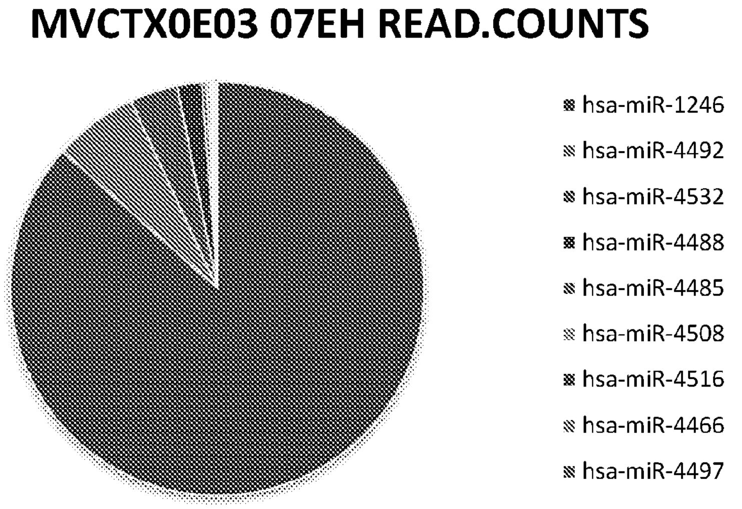
MVCTX0E03 07EH READ.COUNTS
hsa-miR-1246
hsa-miR-4492
hsa-miR-4532
hsa-miR-4488
hsa-miR-4485
hsa-miR-4508
hsa-miR-4516
hsa-miR-4466
hsa-miR-4497

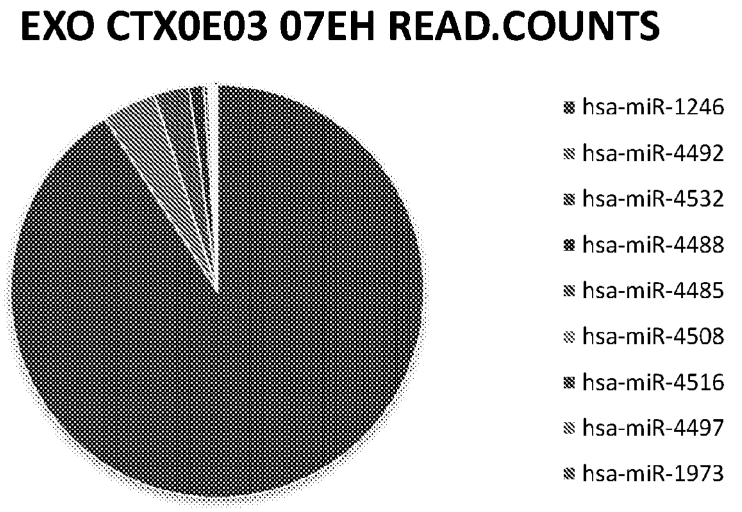
EXO CTX0E03 07EH READ.COUNTS
hsa-miR-1246
hsa-miR-4492
hsa-miR-4532
hsa-miR-4488
hsa-miR-4485
hsa-miR-4508
hsa-miR-4516
hsa-miR-4497
hsa-miR-1973

Figure 13A

## CTX0E03 07EI READ.COUNTS

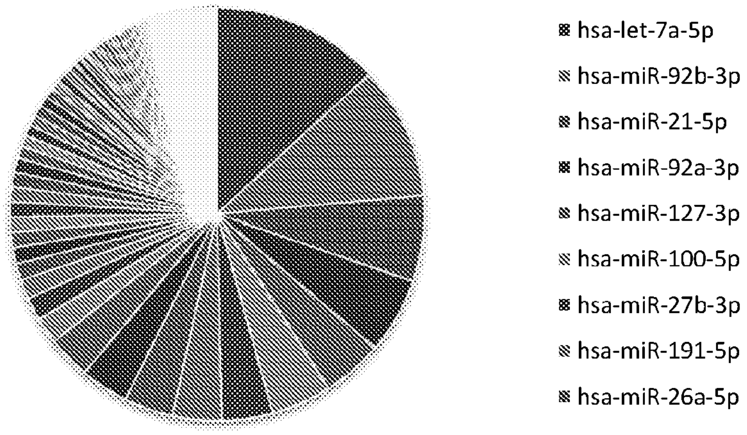


## MVCTX0E03 07EI READ.COUNTS

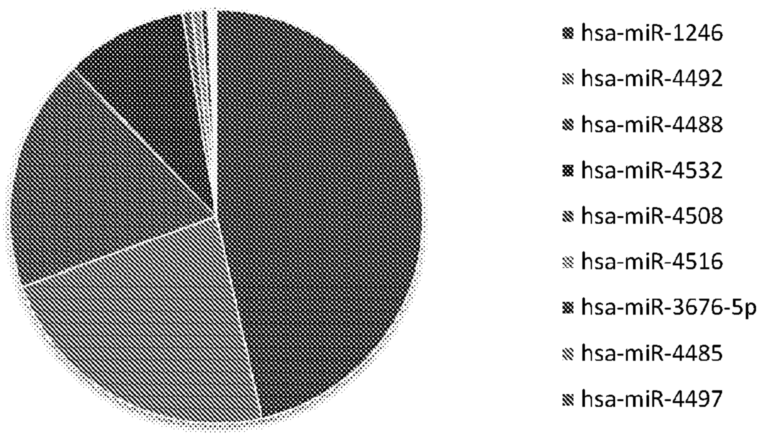


## EXO CTX0E03 07EI READ.COUNTS

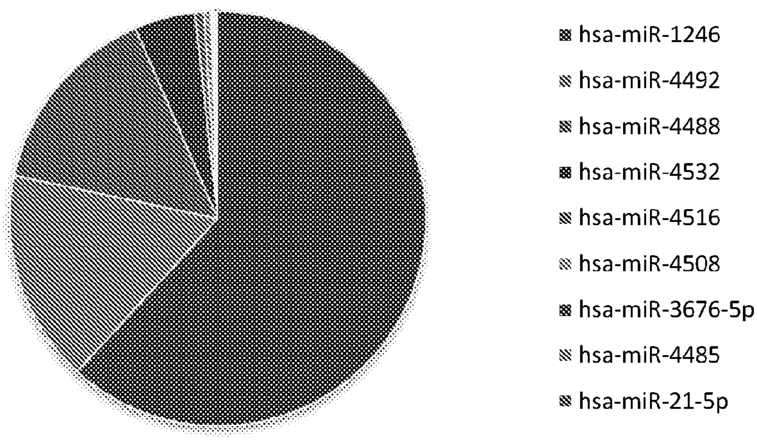


Figure 13B

Figure 13C

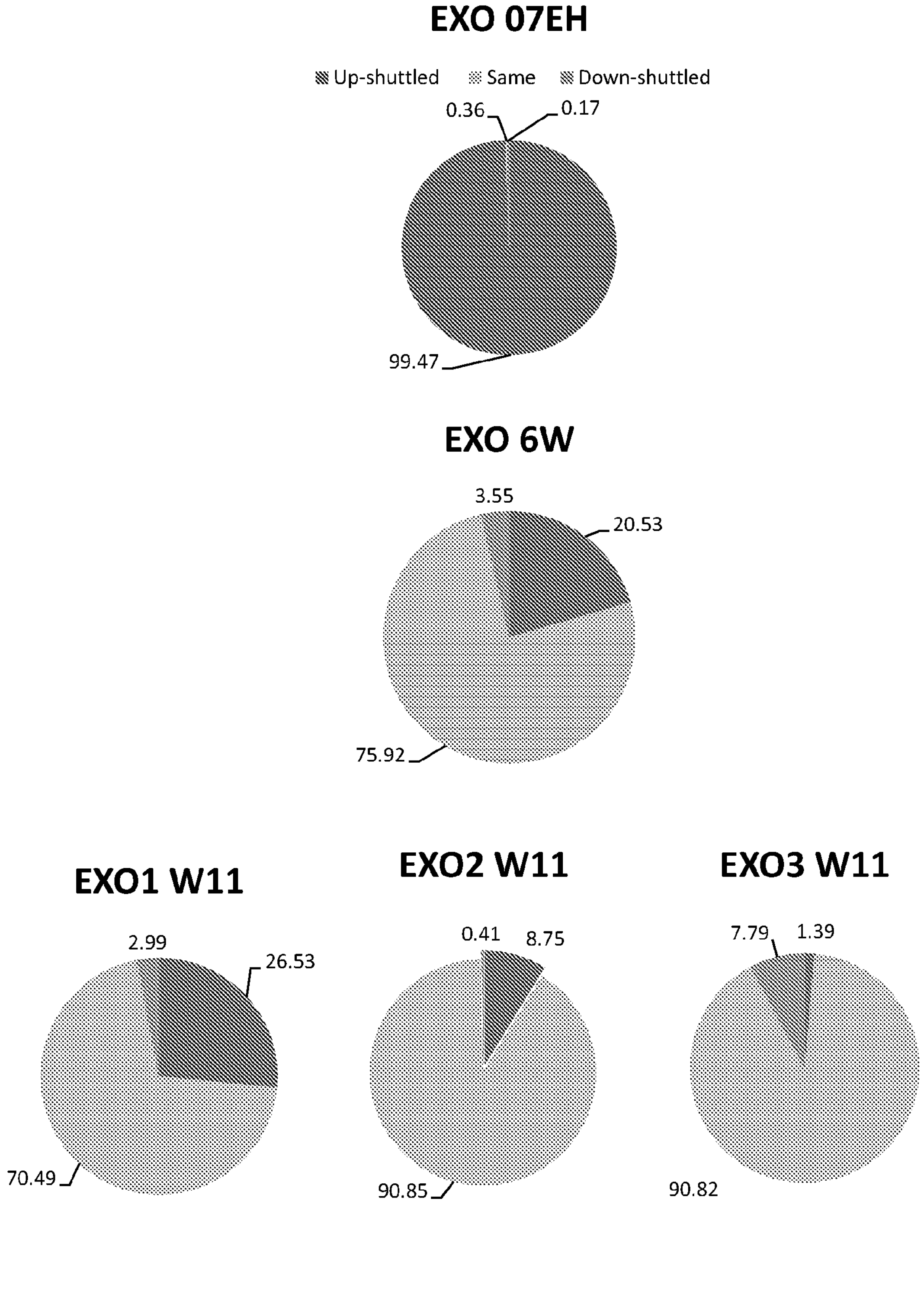
EXO 07EH
Up-shuttled
Same
Down-shuttled
0.36
0.17
99.47
EXO 6W
3.55
20.53
75.92
EXO1 W11
2.99
26.53
70.49
EXO2 W11
0.41
8.75
90.85
EXO3 W11
7.79
1.39
90.82

Figure 13D – Standard Culture

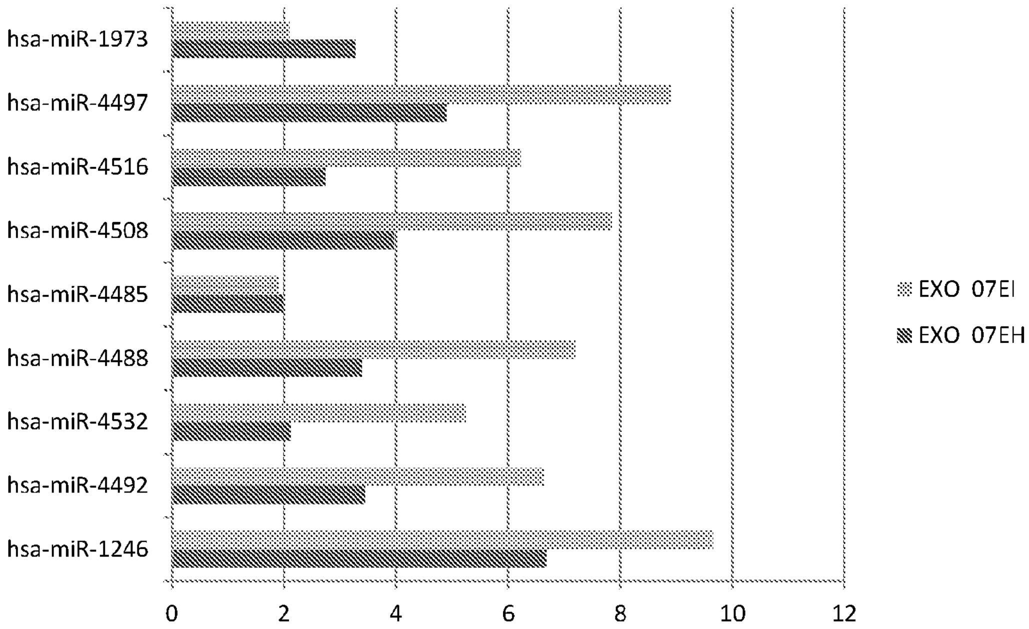


Figure 13E – Exo 6 Week

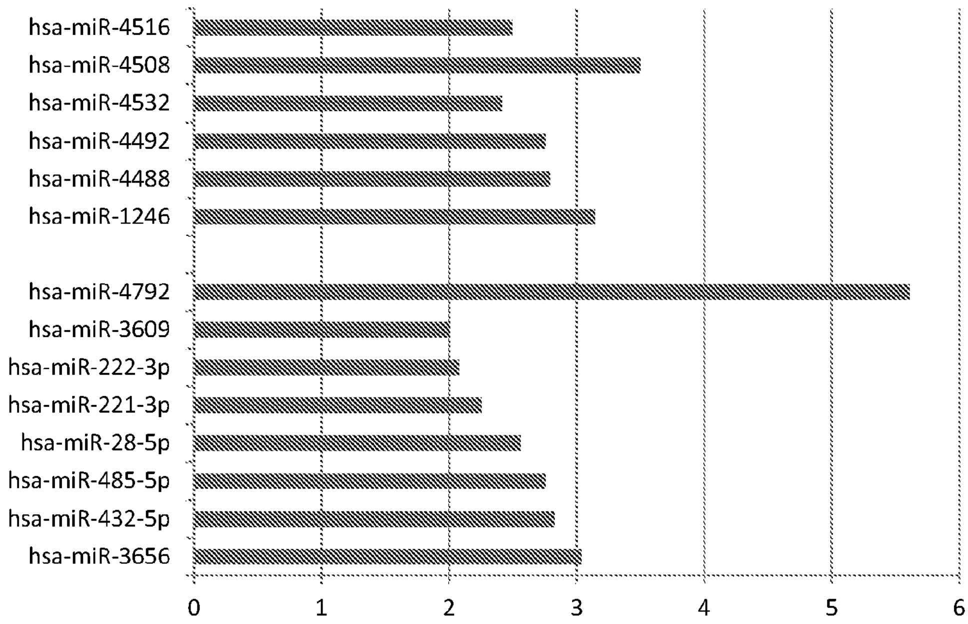

Figure 13F

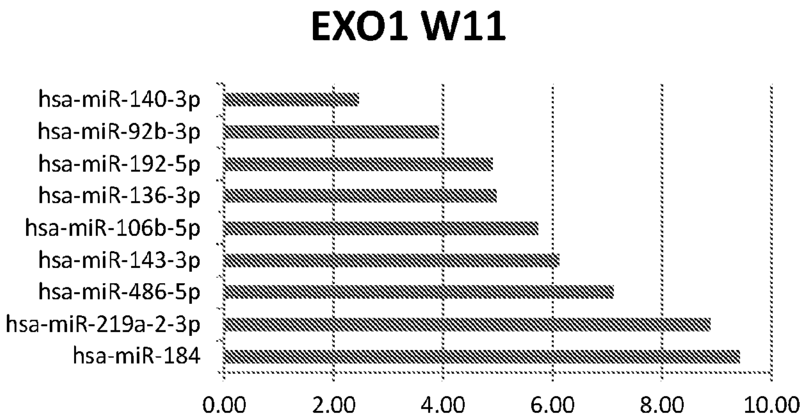


Figure 13G

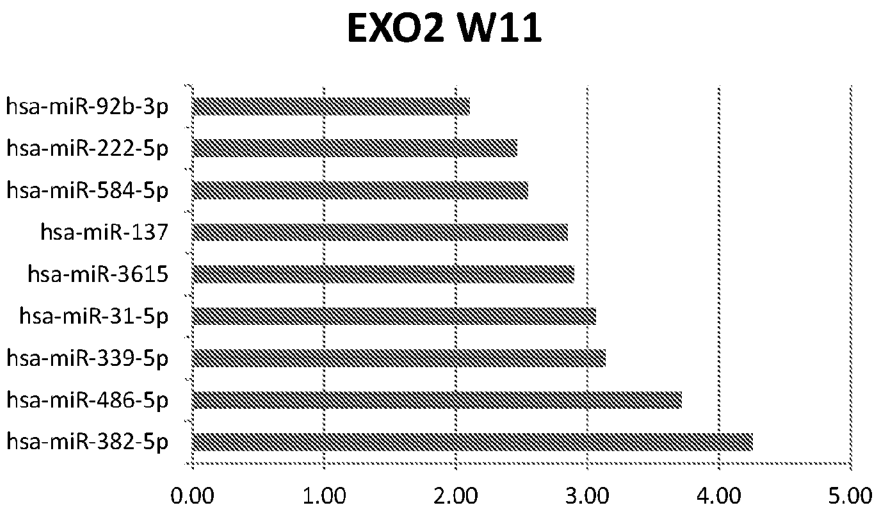


Figure 13H

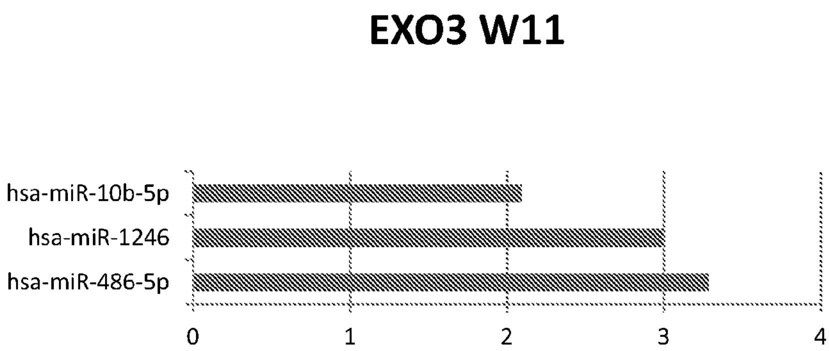

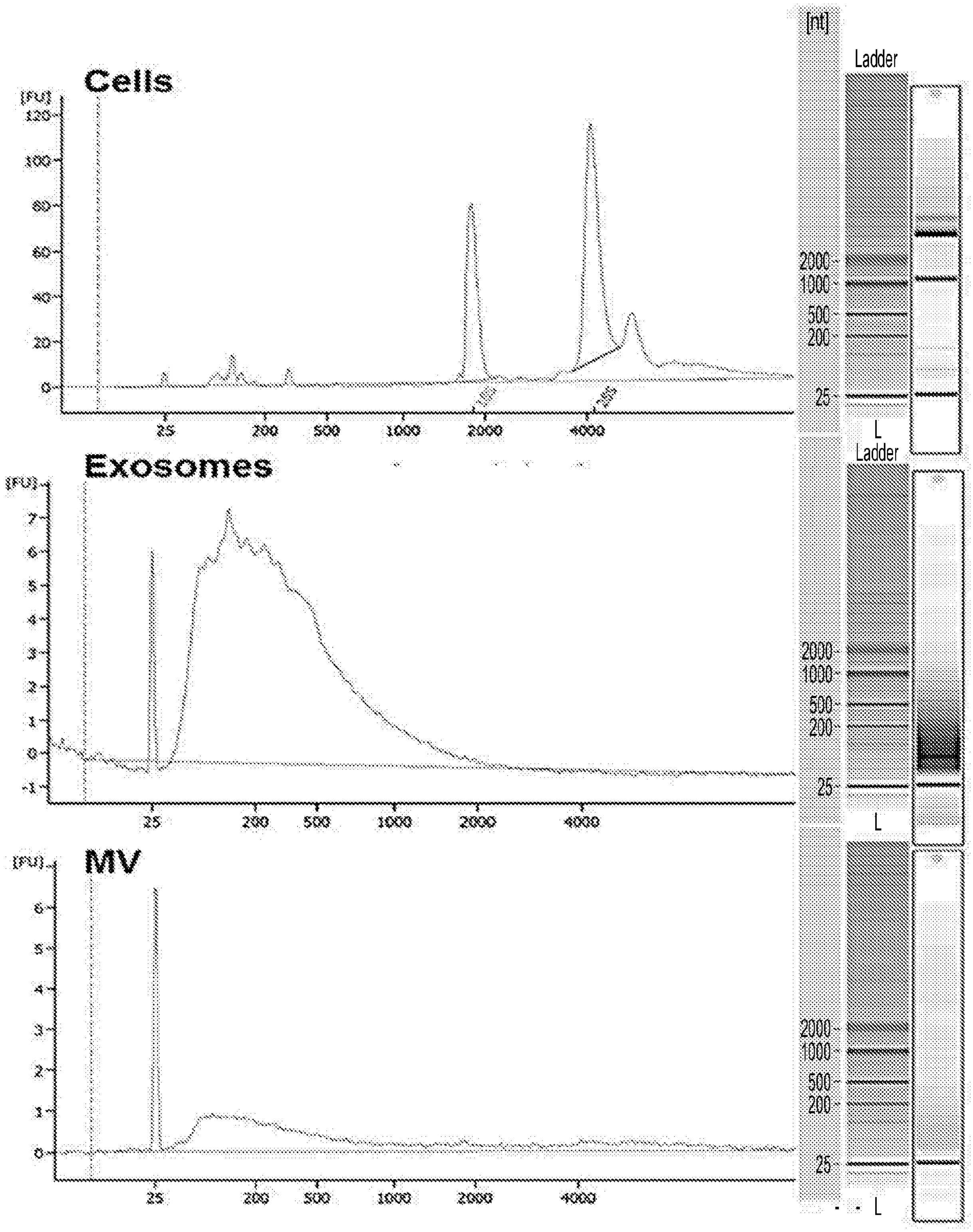
Cells
[FU]
120
100
80
60
40
20
0
25
200
500
1000
2000
4000
Exosomes
[FU]
7
6
5
4
3
2
1
0
-1
MV
[nt]
Ladder
2000
1000
500
200
25
L

Figure 14

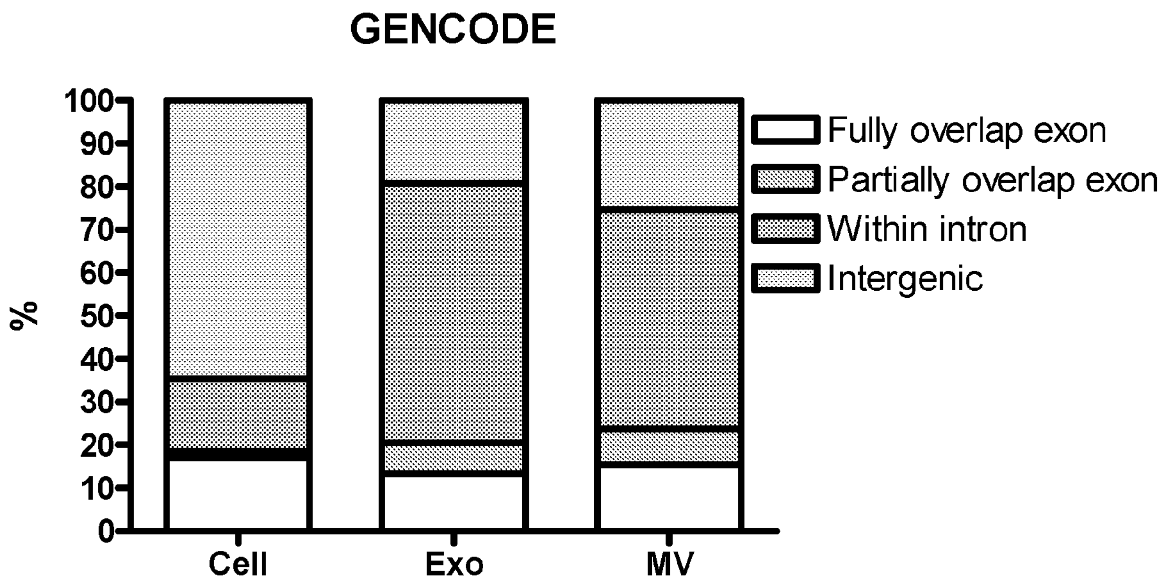


Figure 15

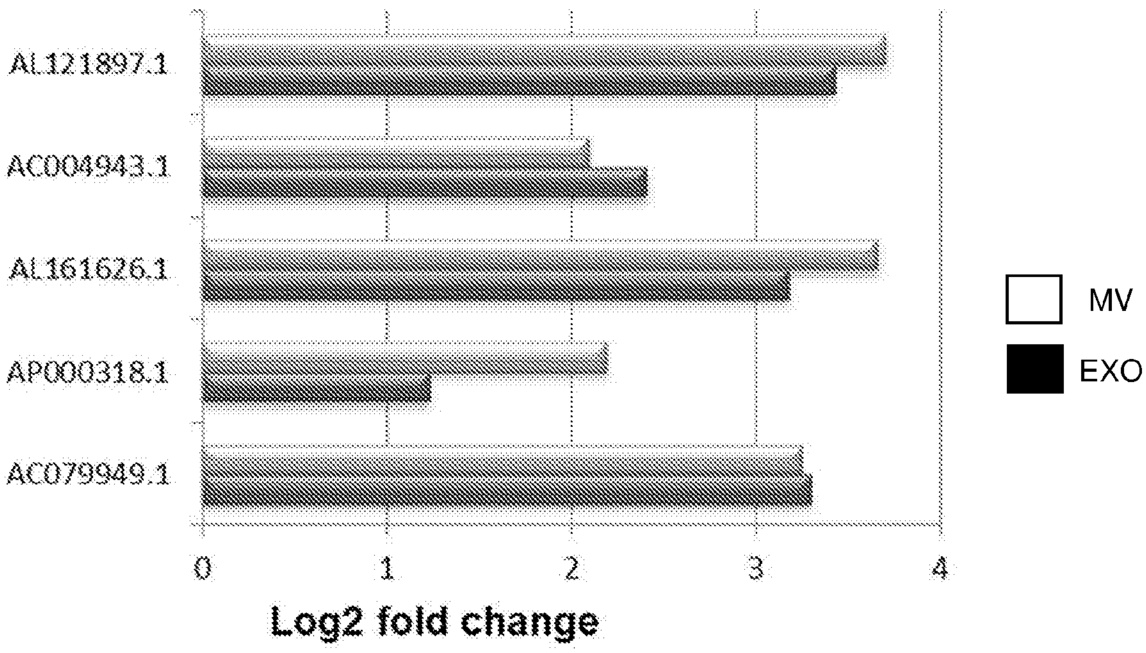


Figure 16

Figure 17A

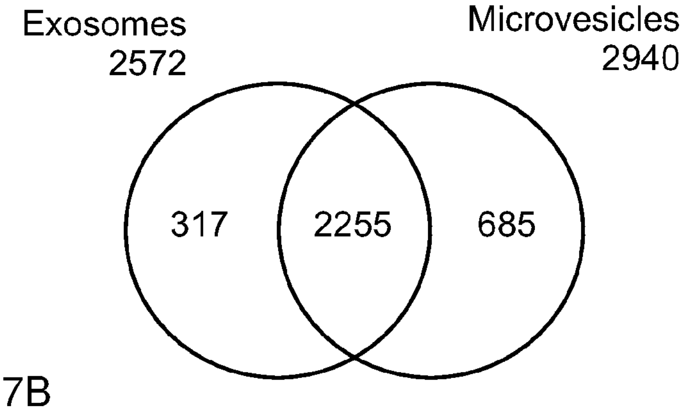


Figure 17B

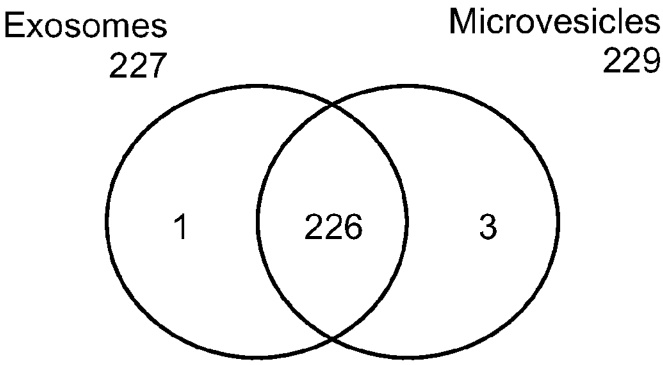


Figure 17C

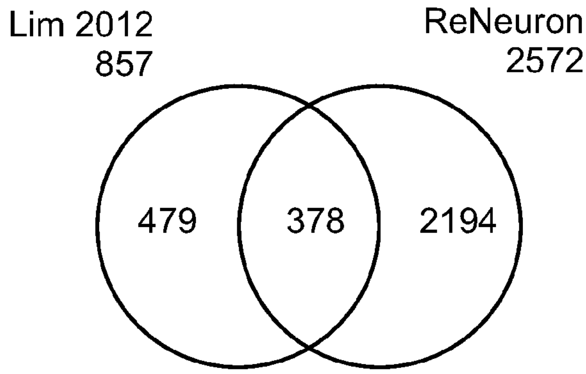


Figure 17D

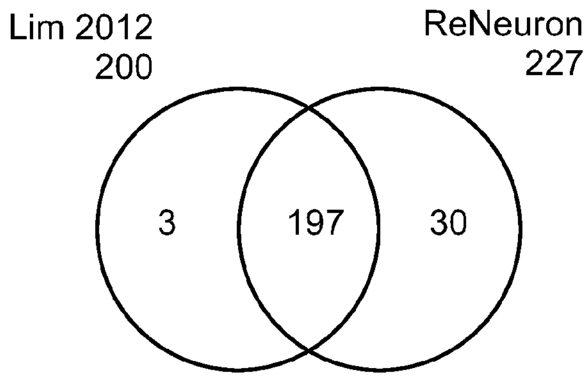

Figure 18

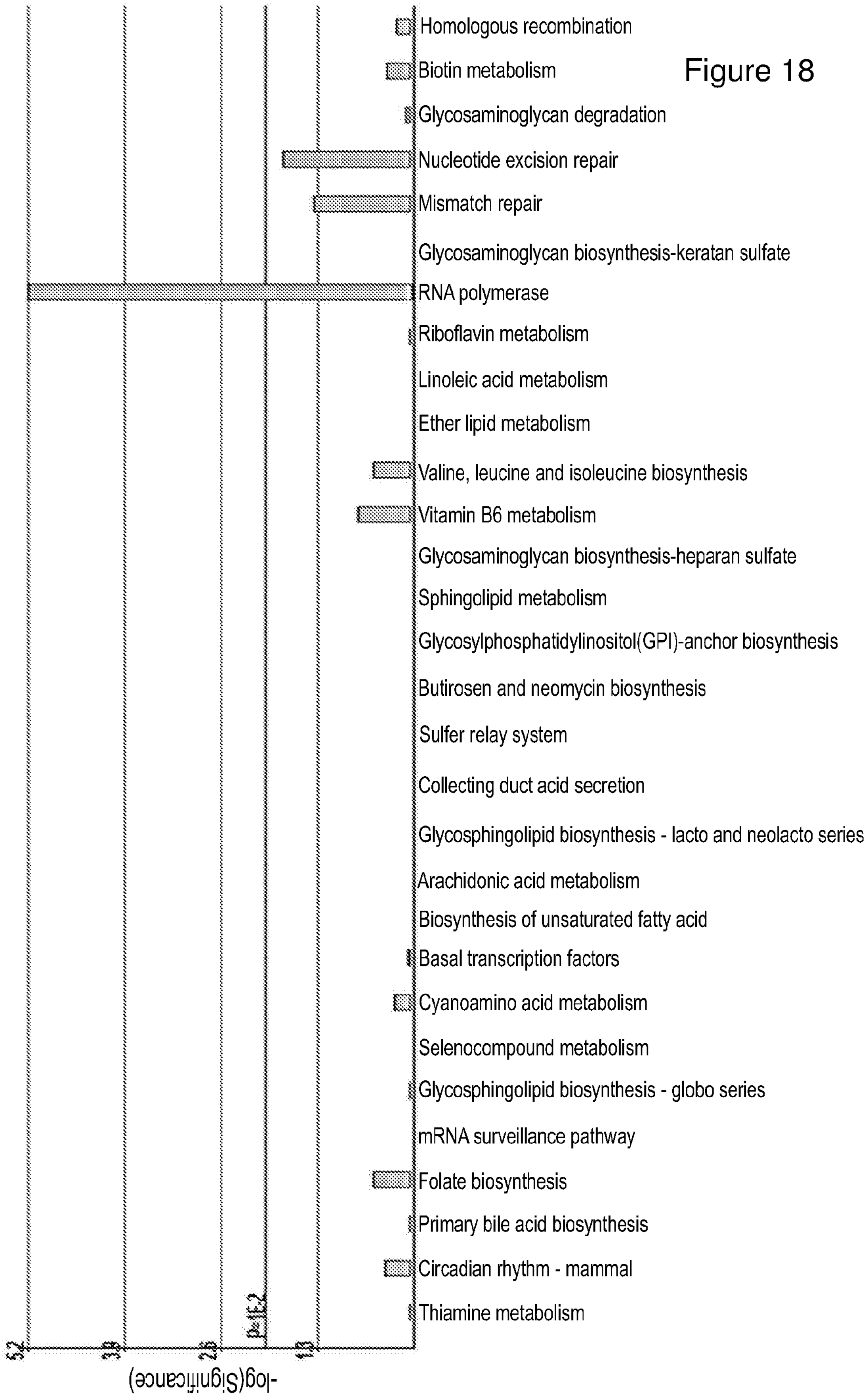
Homologous recombination
Biotin metabolism
Glycosaminoglycan degradation
Nucleotide excision repair
Mismatch repair
Glycosaminoglycan biosynthesis-keratan sulfate
RNA polymerase
Riboflavin metabolism
Linoleic acid metabolism
Ether lipid metabolism
Valine, leucine and isoleucine biosynthesis
Vitamin B6 metabolism
Glycosaminoglycan biosynthesis-heparan sulfate
Sphingolipid metabolism
Glycosylphosphatidylinositol(GPI)-anchor biosynthesis
Butirosen and neomycin biosynthesis
Sulfer relay system
Collecting duct acid secretion
Glycosphingolipid biosynthesis - lacto and neolacto series
Arachidonic acid metabolism
Biosynthesis of unsaturated fatty acid
Basal transcription factors
Cyanoamino acid metabolism
Selenocompound metabolism
Glycosphingolipid biosynthesis - globo series
mRNA surveillance pathway
Folate biosynthesis
Primary bile acid biosynthesis
Circadian rhythm - mammal
Thiamine metabolism
5.2
3.9
2.6
p=1E-2
1.3
-log(Significance)

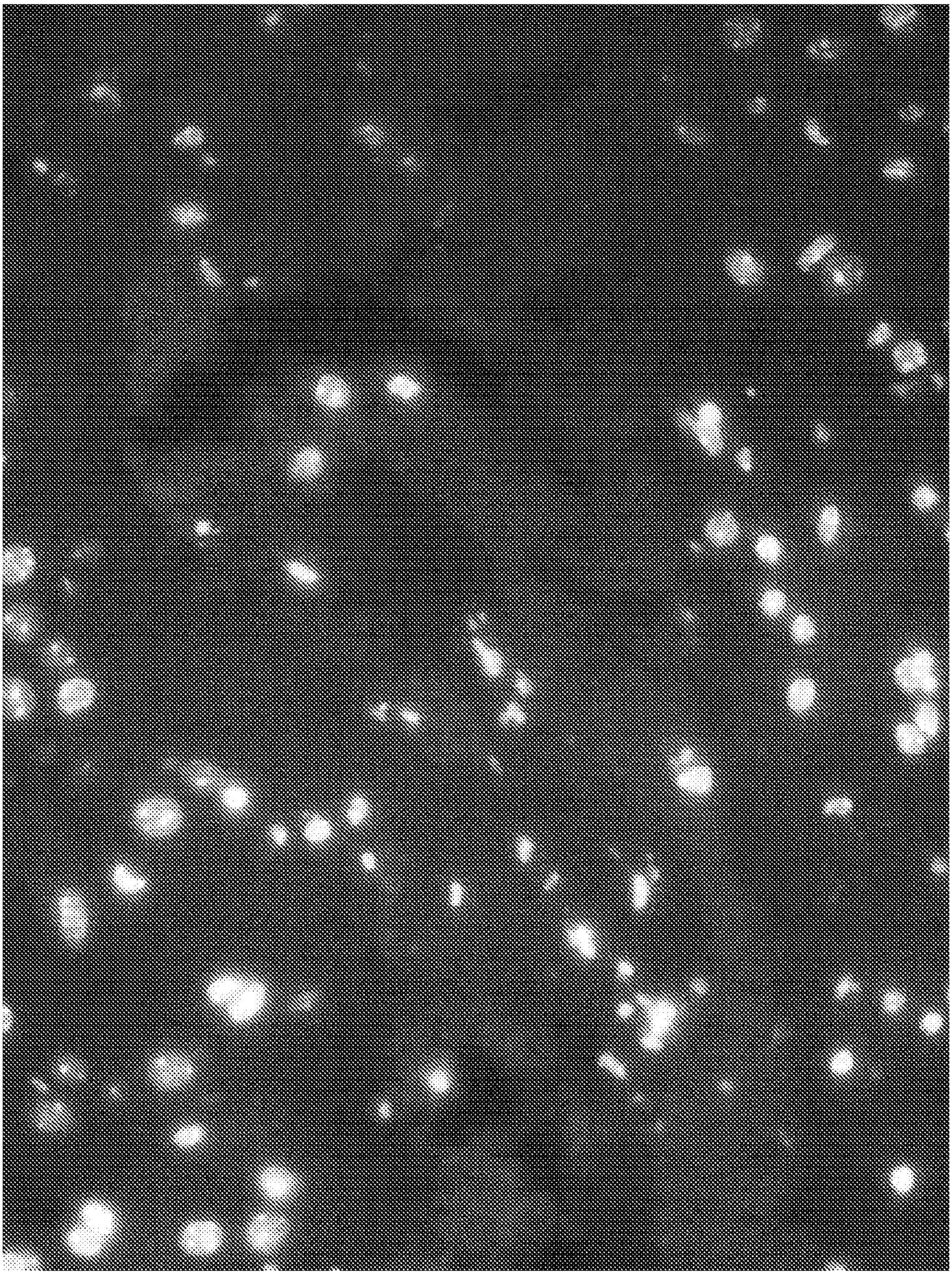

Figure 19

Figure 20

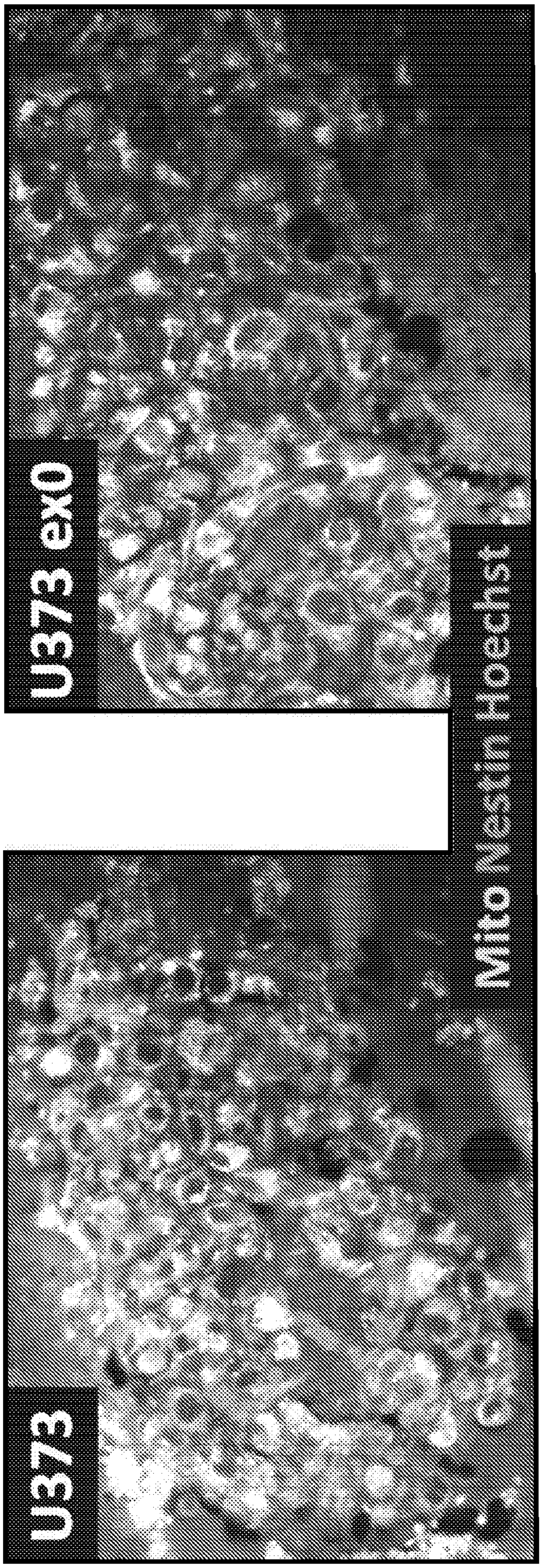

Figure 21

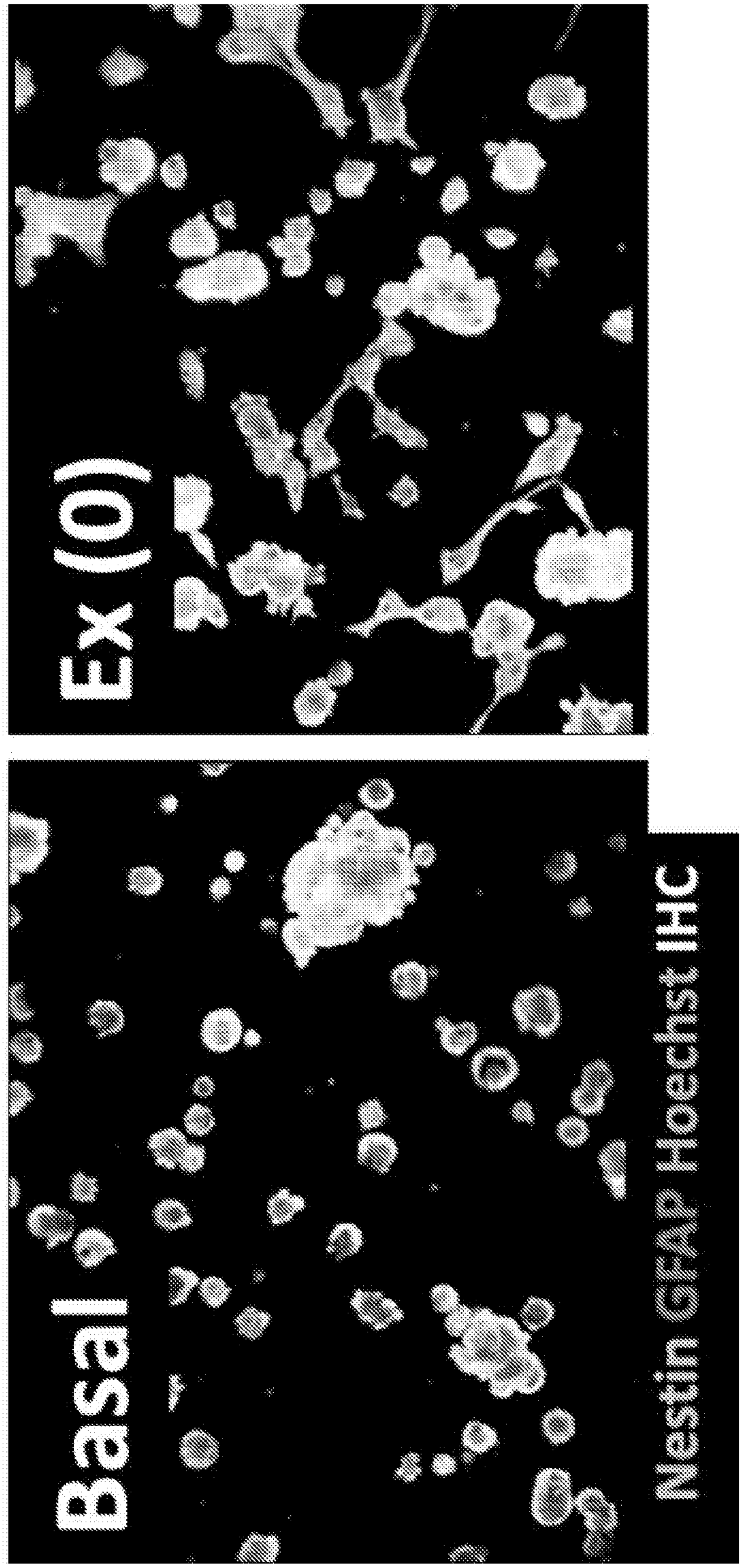

Figure 22A

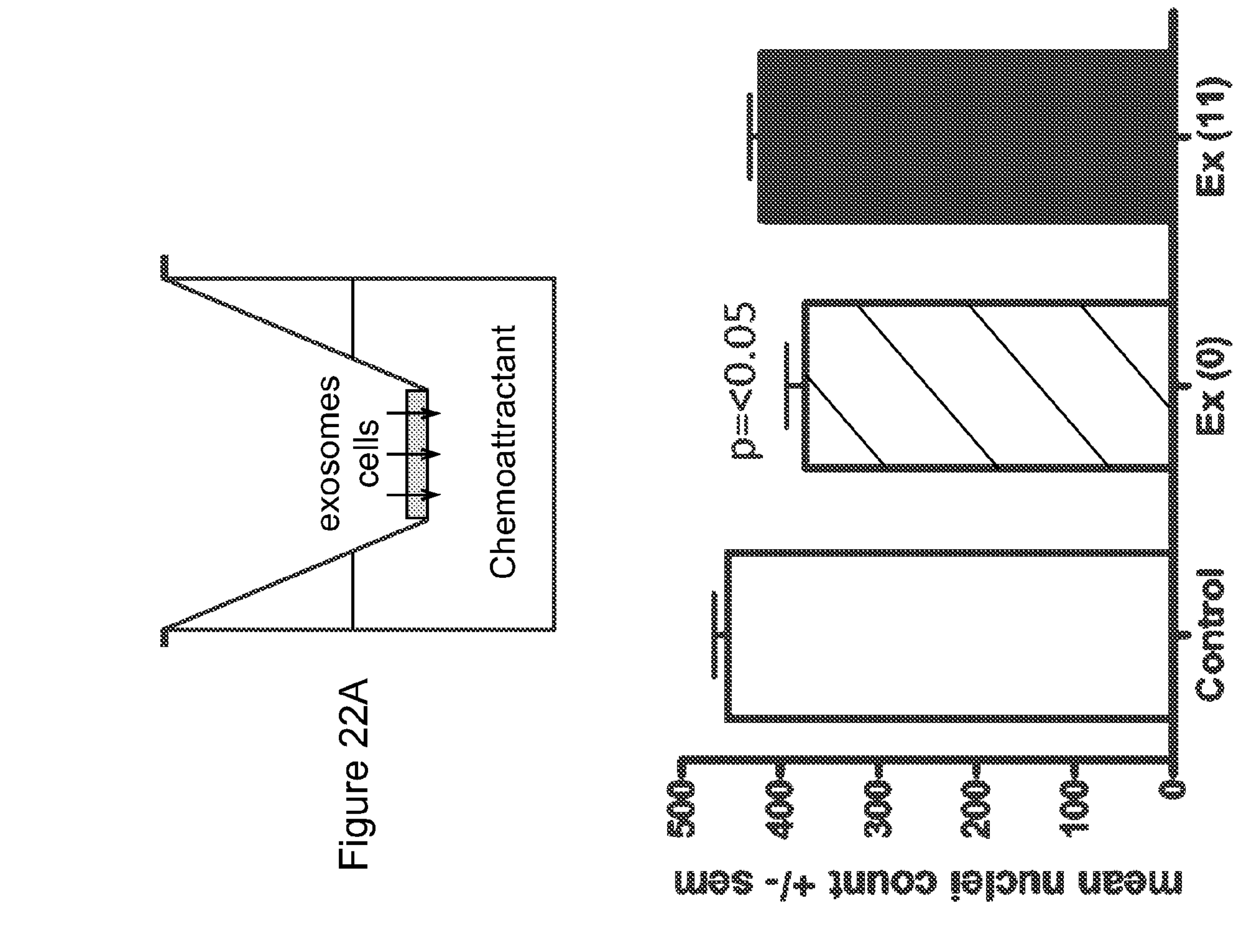


Figure 22B

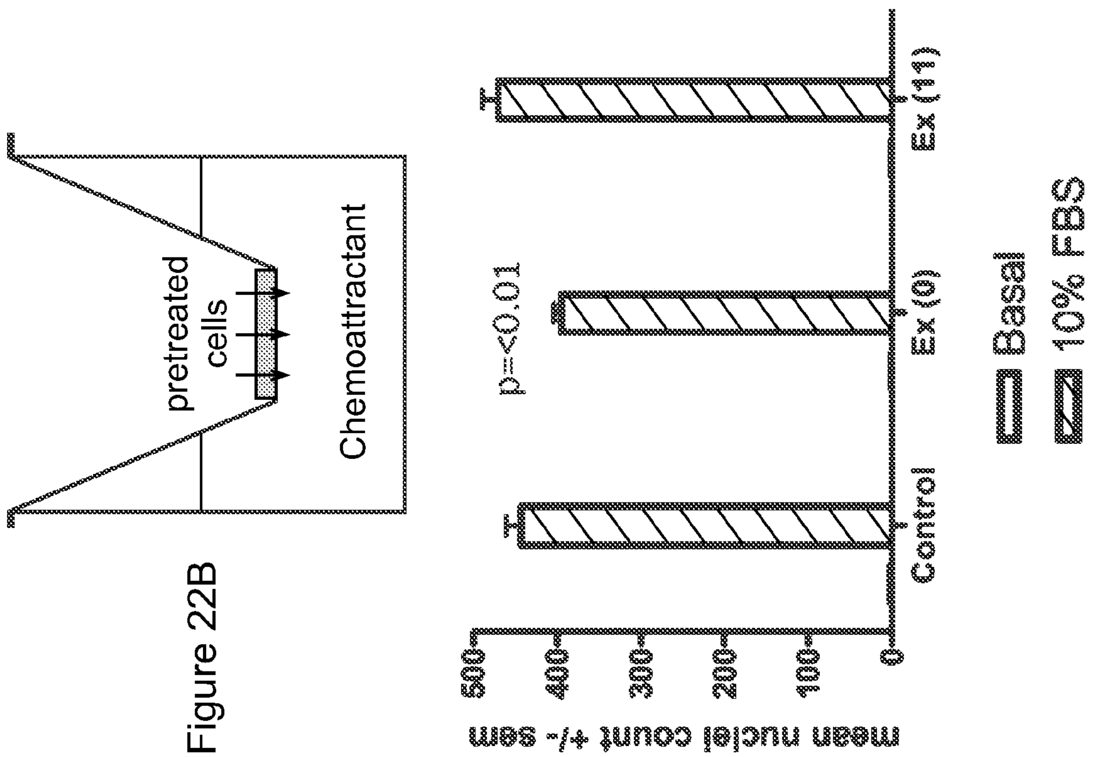

Figure 22C

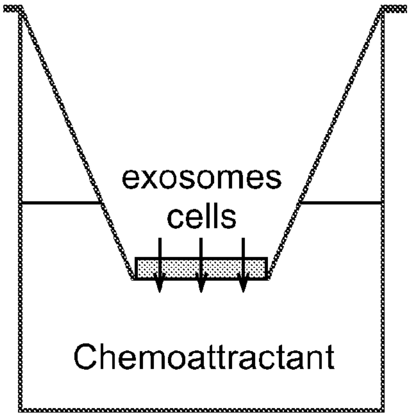
exosomes
cells
Chemoattractant

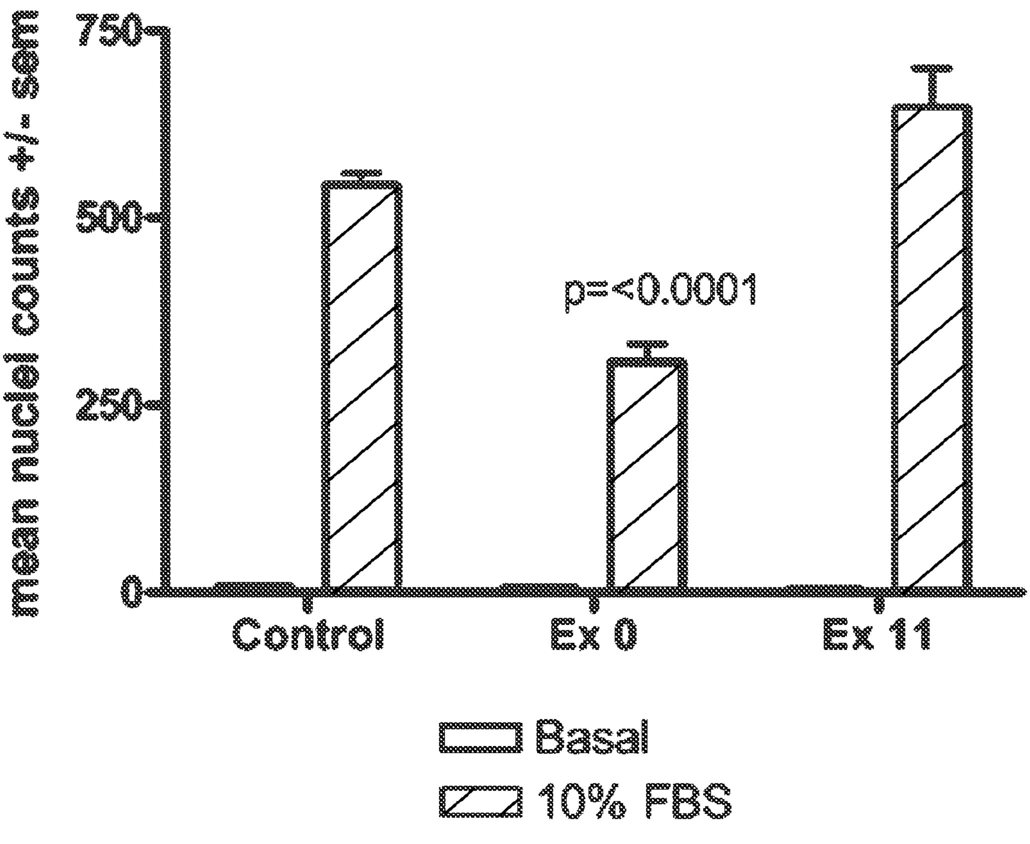
mean nuclei counts +/- sem
750
500
250
0
p=<0.0001
Control
Ex 0
Ex 11
Basal
10% FBS

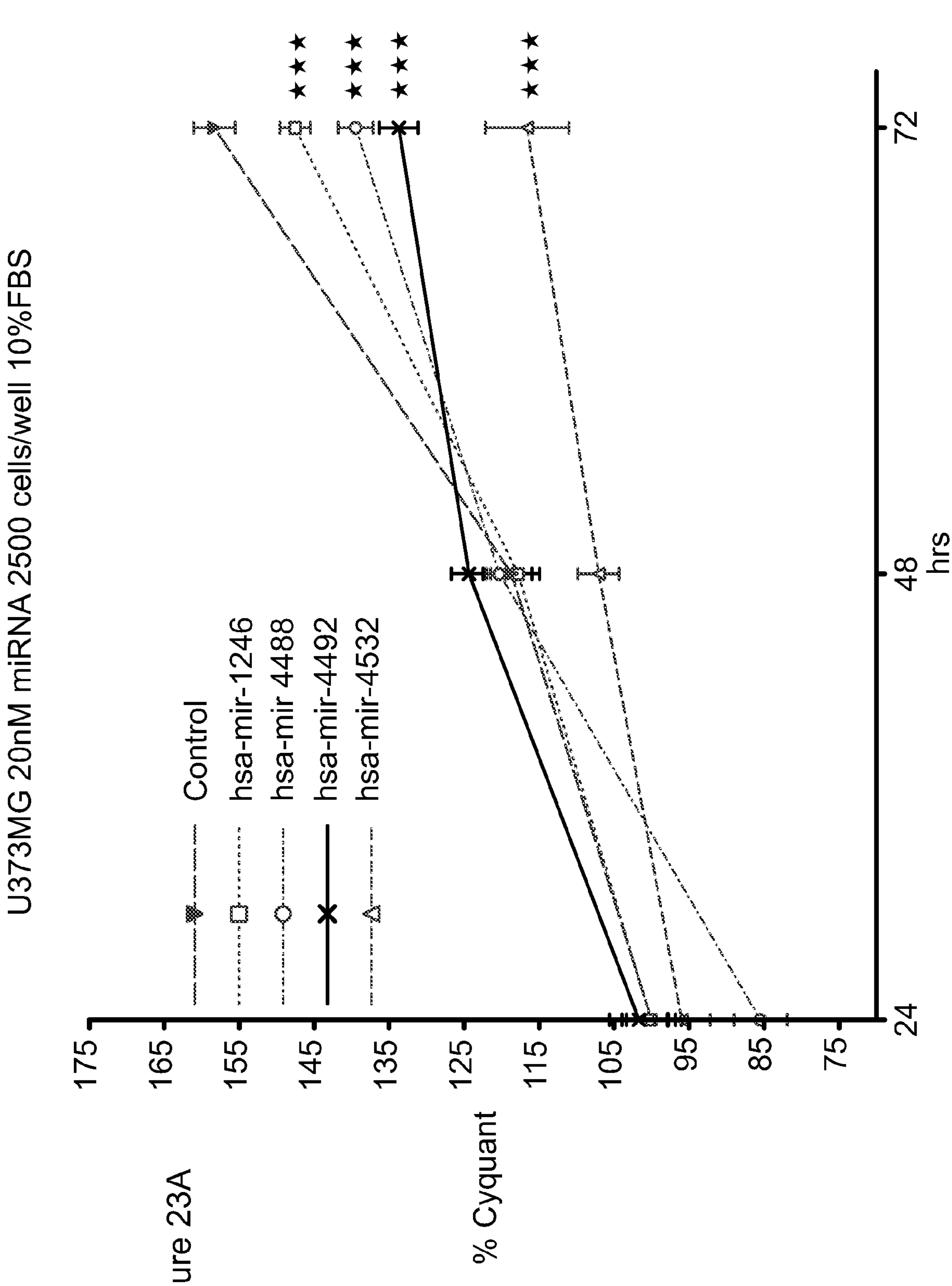
U373MG 20nM miRNA 2500 cells/well 10%FBS
Control
hsa-mir-1246
hsa-mir 4488
hsa-mir-4492
hsa-mir-4532
% Cyquant
175
165
155
145
135
125
115
105
95
85
75
24
48
72
hrs
★★★
★★★
★★★
★★★

Figure 23A

Figure 23B

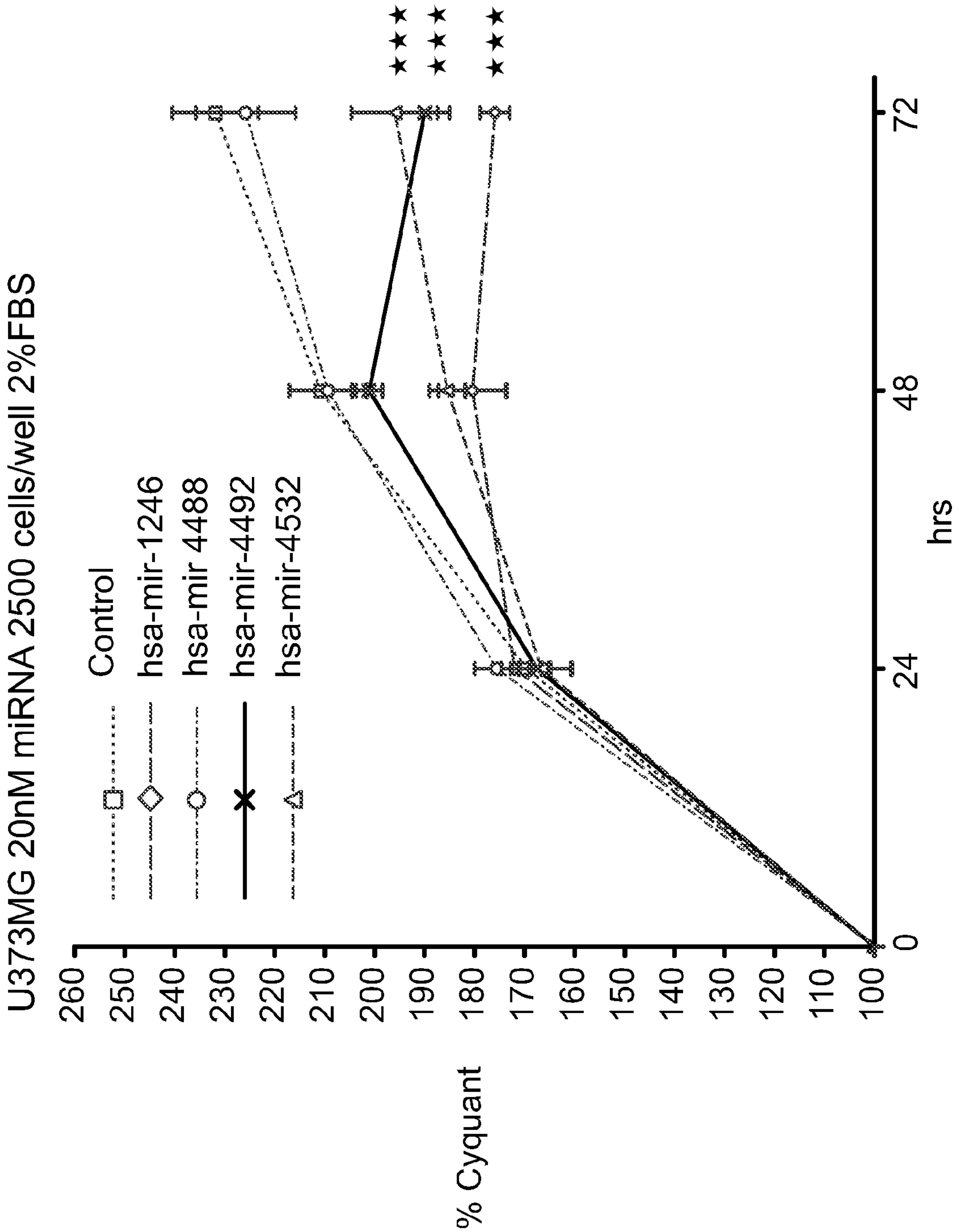
U373MG 20nM miRNA 2500 cells/well 2%FBS
Control
hsa-mir-1246
hsa-mir 4488
hsa-mir-4492
hsa-mir-4532
% Cyquant
260
250
240
230
220
210
200
190
180
170
160
150
140
130
120
110
100
0
24
48
72
hrs
★★★
★★★
★★★

Figure 23C

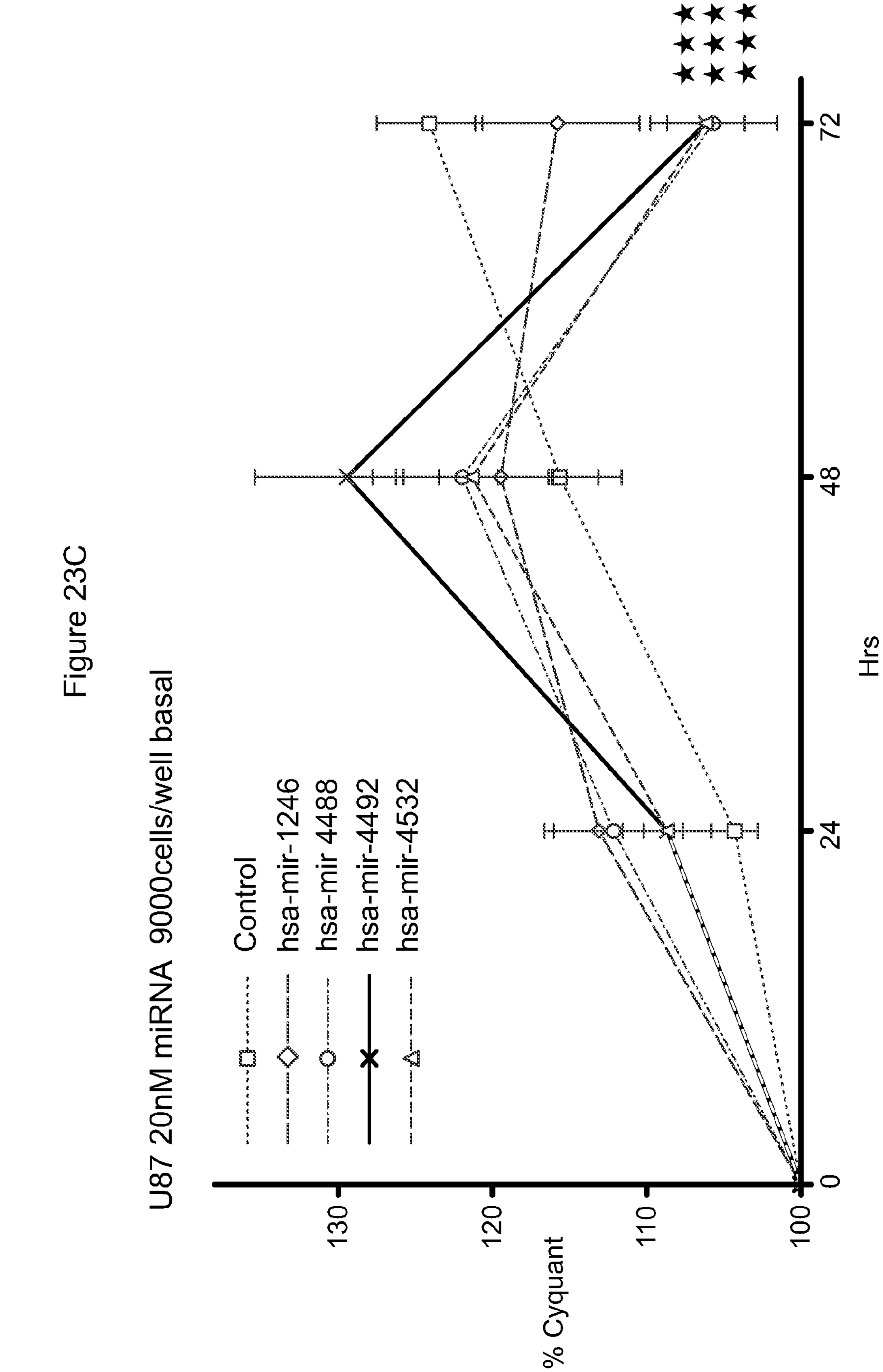
U87 20nM miRNA 9000cells/well basal
Control
hsa-mir-1246
hsa-mir 4488
hsa-mir-4492
hsa-mir-4532
% Cyquant
100
110
120
130
0
24
48
72
Hrs

Figure 24: Tumour volume at assignment

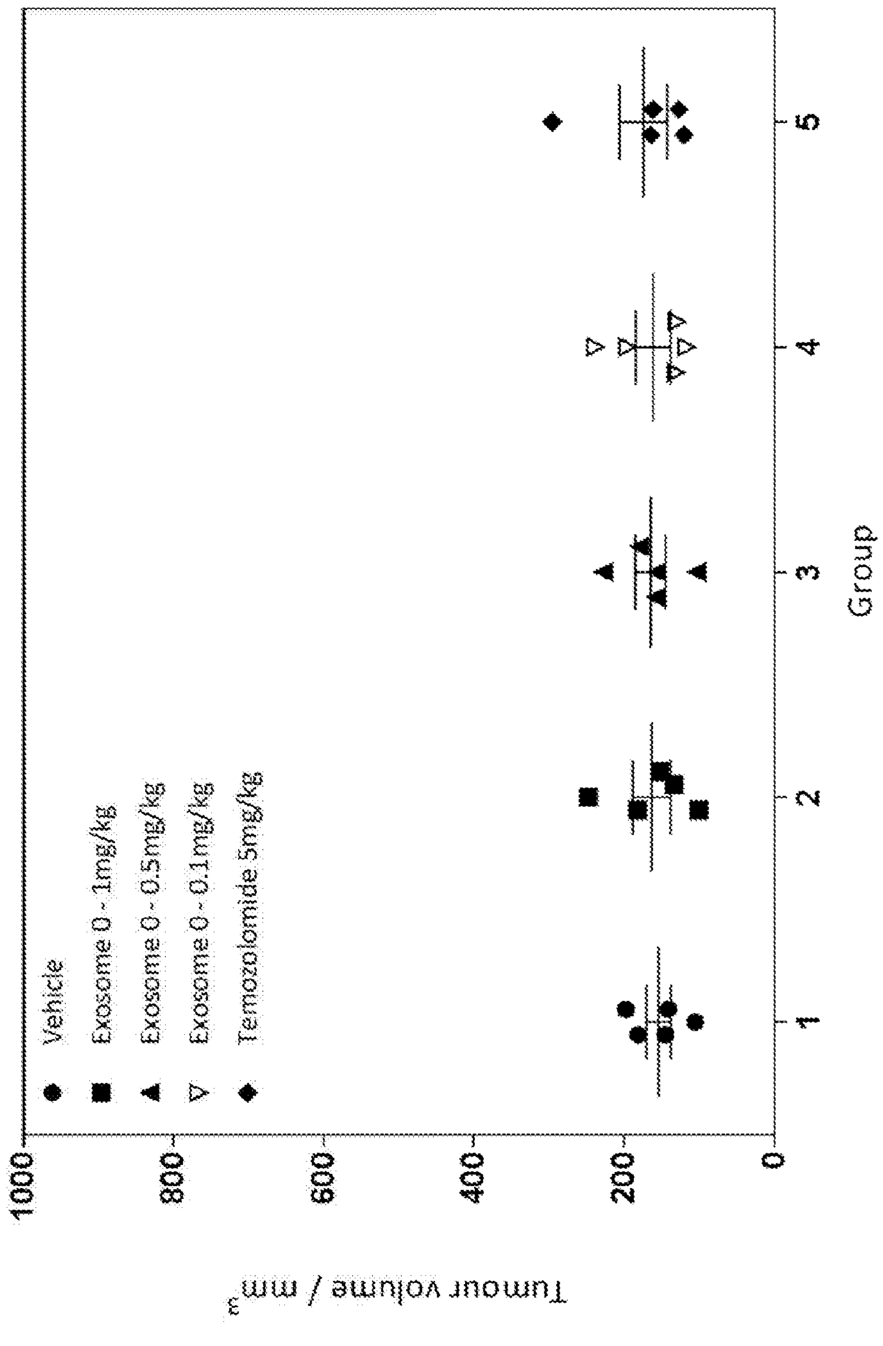

Figure 25: Mean body weights

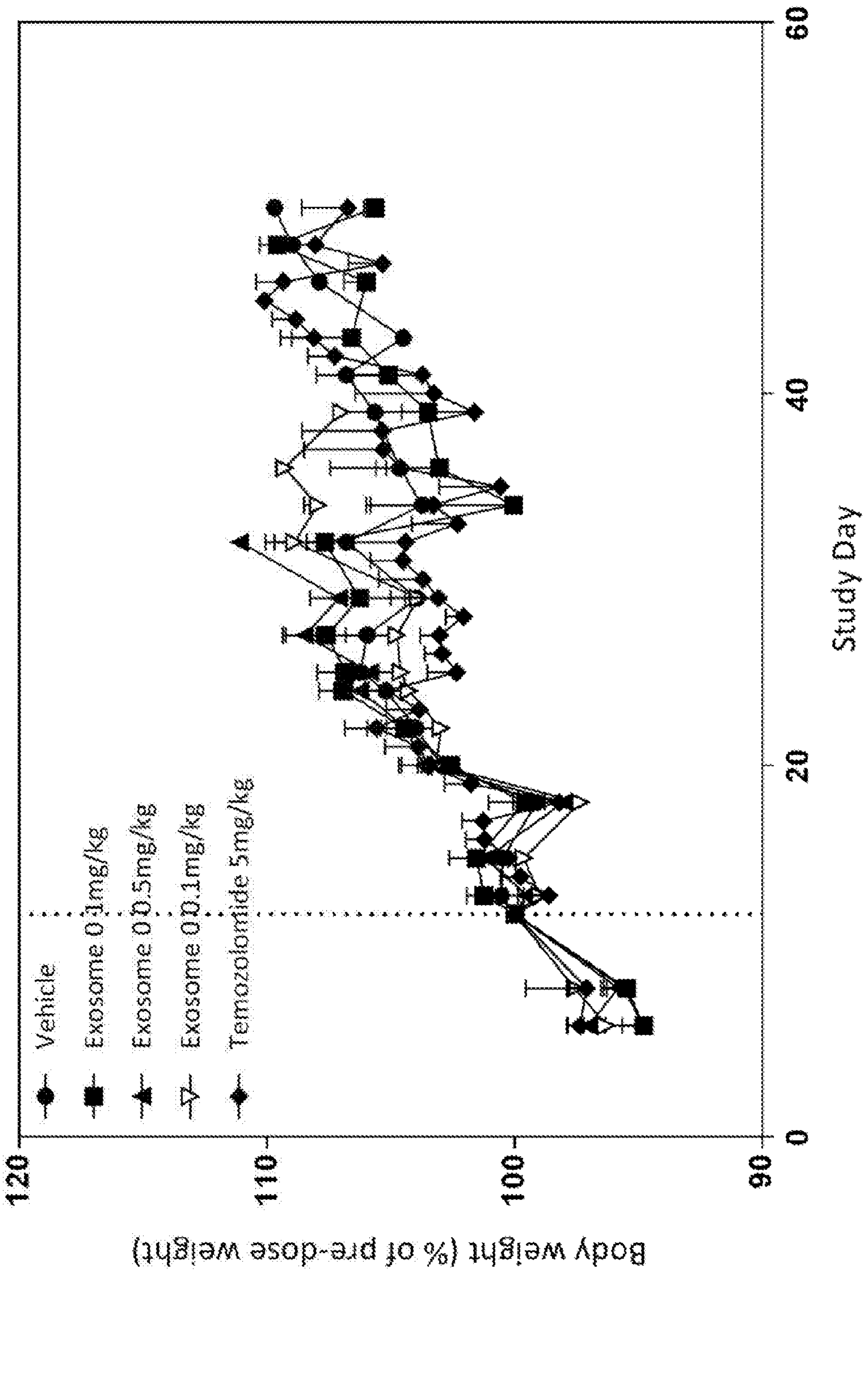

Figure 26: Mean tumour volume by study day[1]

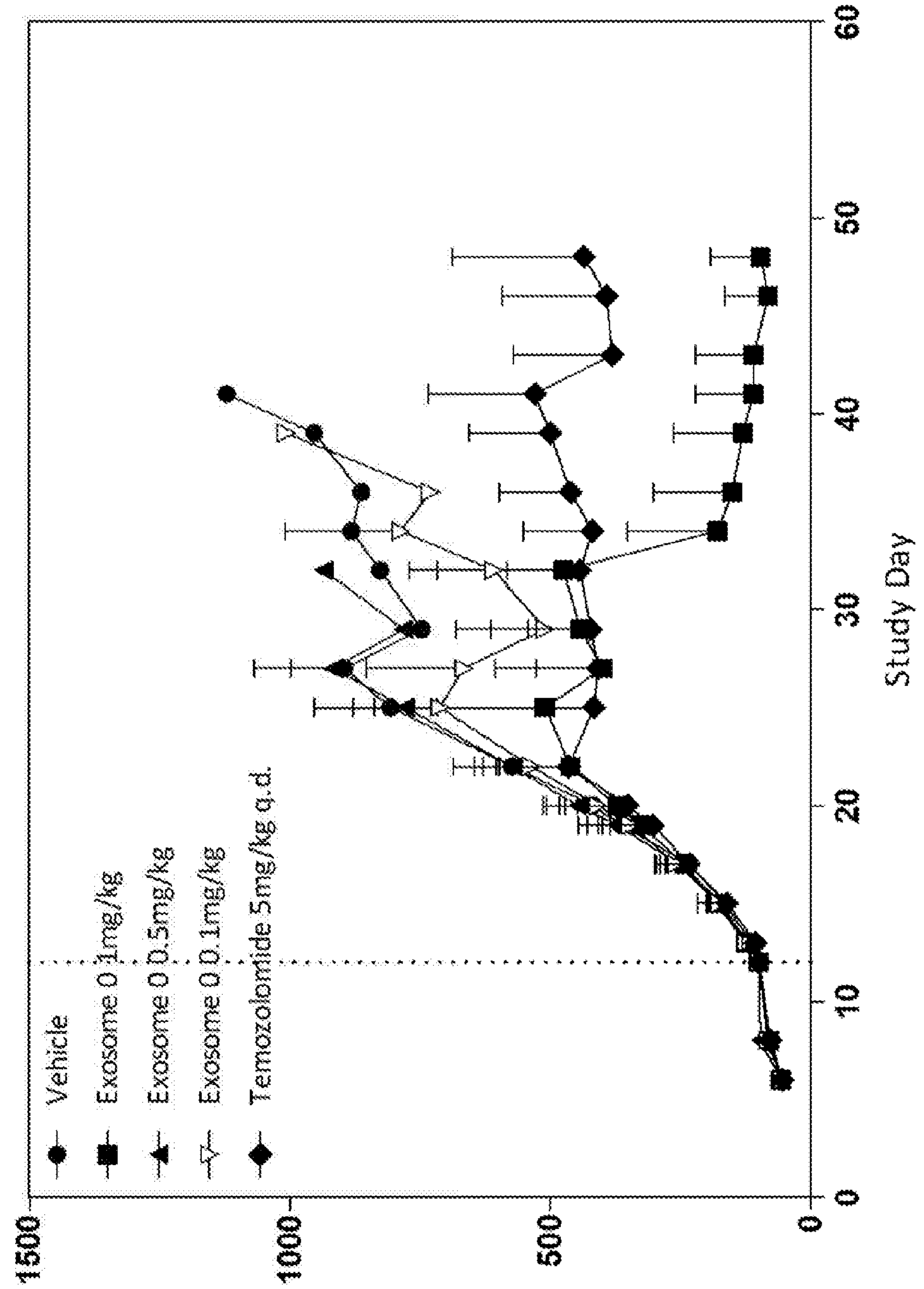

Figure 27: Mean tumour volume by study day – Day 25 truncated format

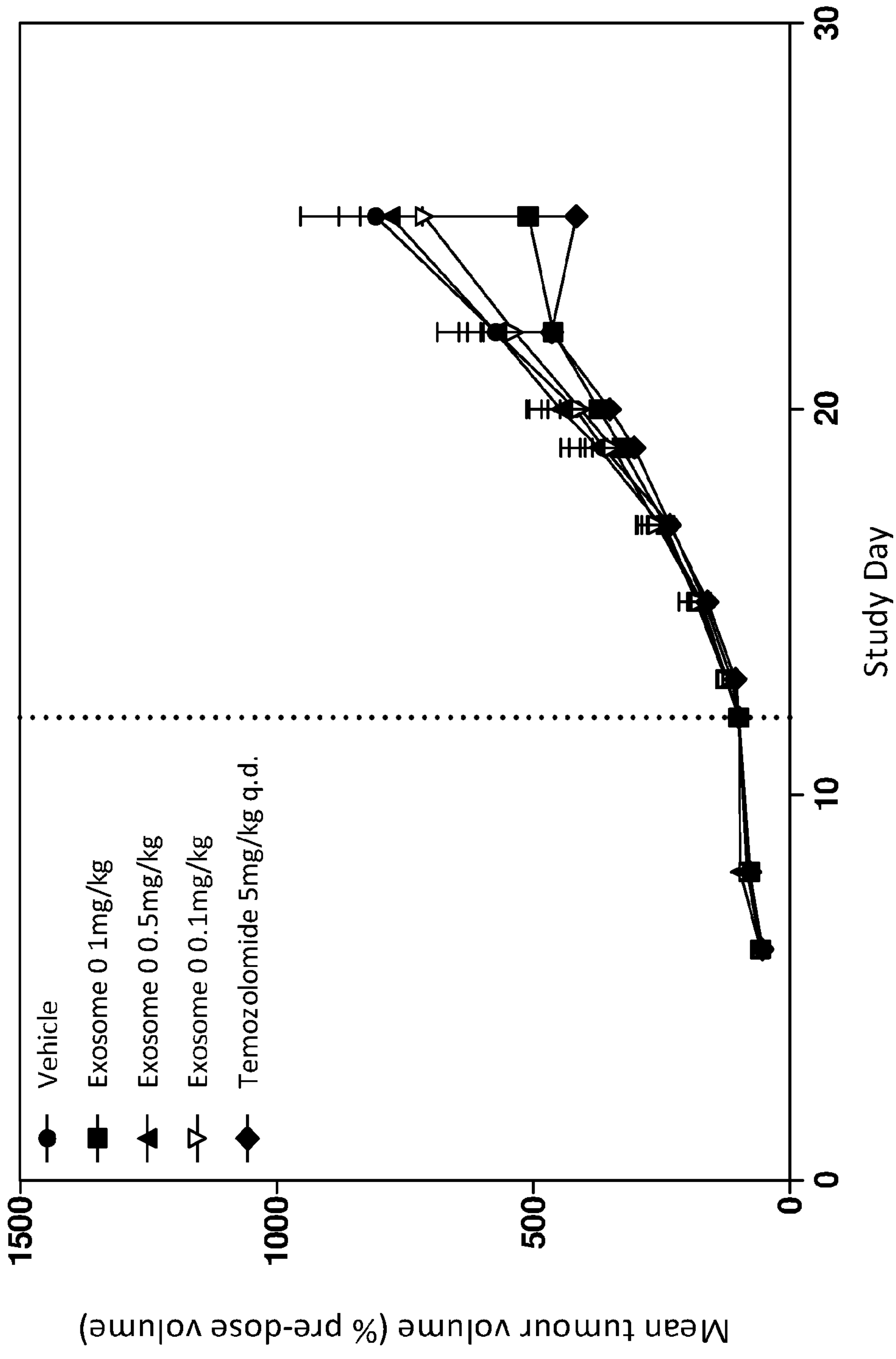

Figure 28: Final tumour weights

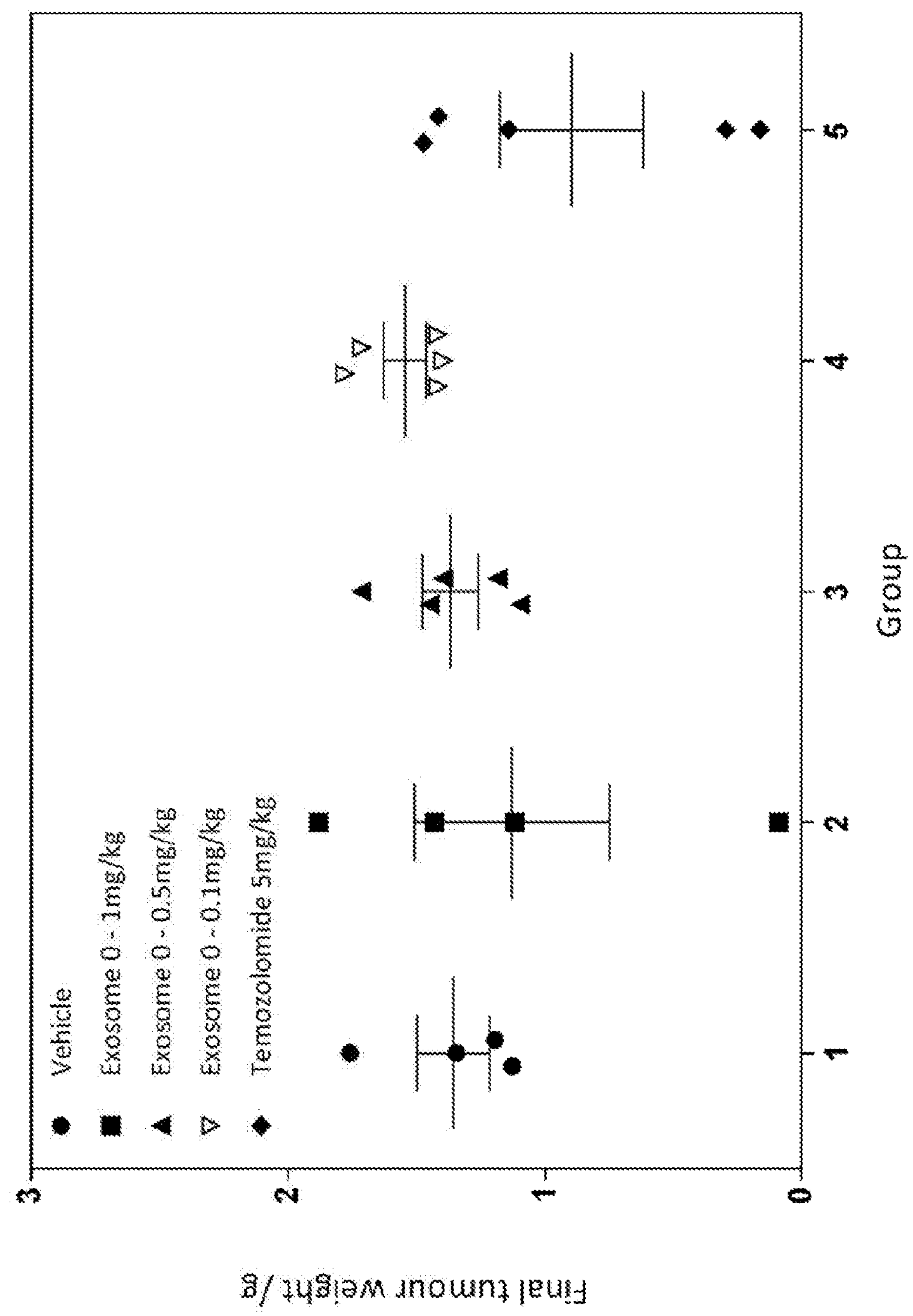

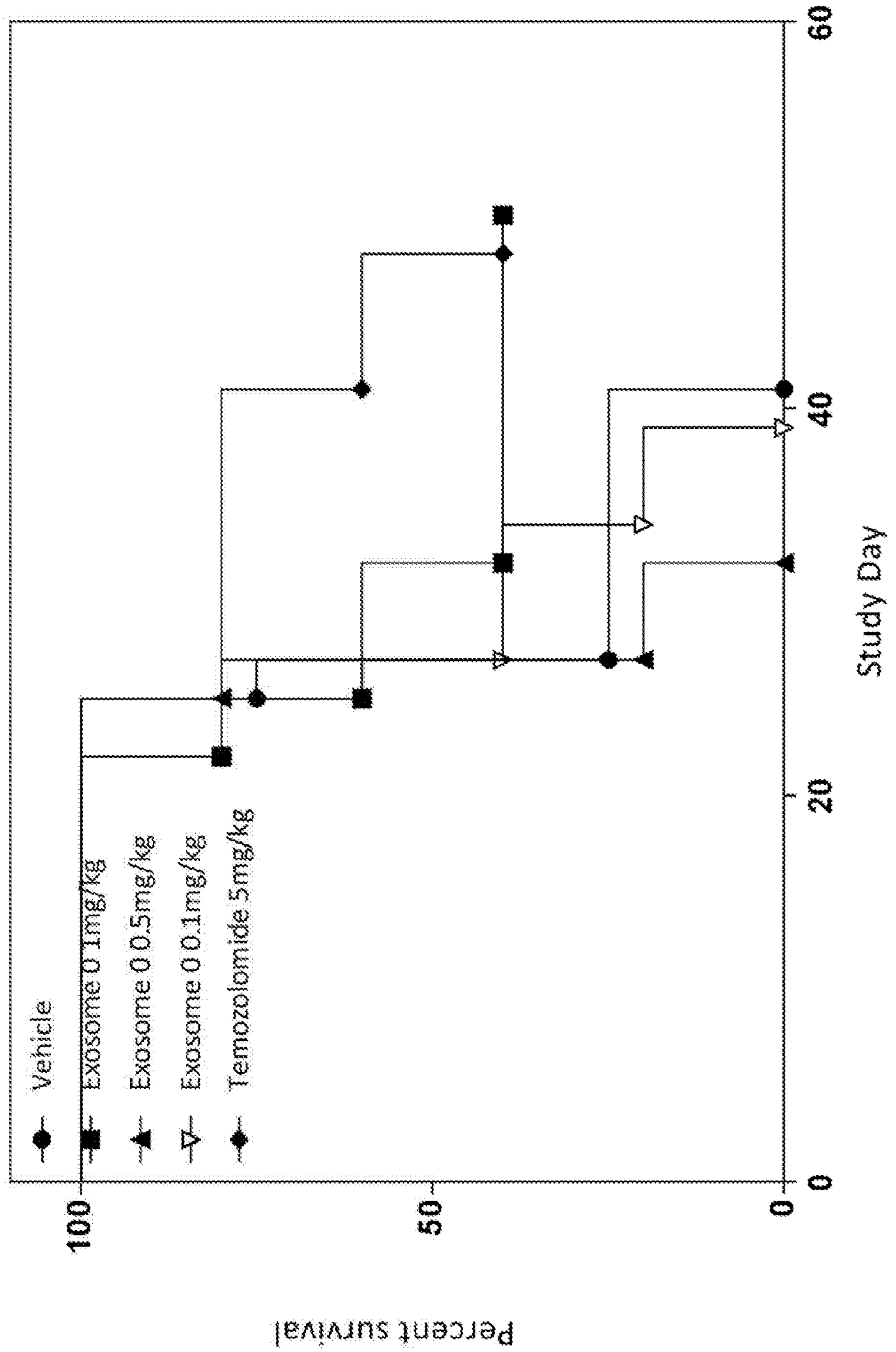


Figure 29: Survival plot for the dosing phase

# Figure 30: Individual body weights – Absolute

**Absolute**

| Animal ID | Group | Sex | Fate Code | Fate Day | 4/30 | 5/2 | 5/6 | 5/7 | 5/8 | 5/9 | 5/10 | 5/11 | 5/12 | 5/13 | 5/14 | 5/15 | 5/16 | 5/17 | 5/18 | 5/19 | 5/20 | 5/21 | 5/22 | 5/23 |
|---|---|---|---|---|---|---|---|---|---|---|---|---|---|---|---|---|---|---|---|---|---|---|---|---|
| | | | | | Date/Study Day | | | | | | | | | | | | | | | | | | | |
| | | | | | Pre-Dosing | | Dosing & Recovery | | | | | | | | | | | | | | | | | |
| | | | | | 6 | 8 | 12 | 13 | 14 | 15 | 16 | 17 | 18 | 19 | 20 | 21 | 22 | 23 | 24 | 25 | 26 | 27 | 28 | 29 |
| 00077E6CC9-7 | 1 | F | TS | 27 | 24.3 | 24.3 | 25.4 | 25.7 | | 26.4 | | | 25.5 | | 26.4 | | 27.0 | | 27.0 | 28.0 | | 28.3 | | |
| 00077E7D9C-3 | 1 | F | TS | 25 | 22.1 | 22.1 | 23.6 | 23.3 | | 23.3 | | | 23.5 | | 24.4 | | 25.4 | | 26.2 | 26.1 | | | | |
| 00077E7ED6-17 | 1 | F | TS | 41 | 22.7 | 24.0 | 25.0 | 25.5 | | 25.2 | | | 24.1 | | 24.8 | | 25.2 | | 25.7 | 25.9 | | 26.6 | | 26.6 |
| 00077E7FDB-14 | 1 | F | SS | 50 | 26.7 | 26.1 | 26.7 | 26.7 | | 26.8 | | | 26.0 | | 27.7 | | 26.9 | | 26.7 | 27.3 | | 27.2 | | 27.1 |
| 00077E8B08-20 | 1 | F | TS | 27 | 22.3 | 22.5 | 23.7 | 23.9 | | 23.4 | | | 24.1 | | 24.8 | | 24.8 | | 25.1 | 24.8 | | 24.7 | | |
| 00077E6AFE-21 | 2 | F | SS | 50 | 20.8 | 20.8 | 21.4 | 22.1 | | 22.3 | | | 21.6 | | 22.1 | | 22.5 | | 22.5 | 22.7 | | 22.4 | | 21.9 |
| 00077E6C4D-12 | 2 | F | SS | 50 | 22.0 | 22.6 | 23.3 | 23.8 | | 24.3 | | | 24.3 | | 24.7 | | 24.9 | | 25.3 | 24.9 | | 25.1 | | 25.1 |
| 00077E7EF9-18 | 2 | F | TS | 32 | 24.0 | 24.1 | 24.9 | 25.1 | | 24.8 | | | 23.9 | | 25.0 | | 24.8 | | 26.2 | 26.5 | | 27.5 | | 27.1 |
| 00077E8B5F-30 | 2 | F | TS | 25 | 23.5 | 23.2 | 25.3 | 25.5 | | 25.2 | | | 25.2 | | 26.1 | | 27.3 | | 27.5 | 27.3 | | | | |
| 00077E8C0D-4 | 2 | F | TS | 22 | 22.9 | 23.3 | 24.6 | 24.4 | | 24.6 | | | 23.9 | | 24.6 | | 25.3 | | | | | | | |
| 00077E6ABD-11 | 3 | F | TS | 25 | 23.0 | 22.8 | 23.9 | 23.8 | | 24.0 | | | 23.2 | | 24.6 | | 24.6 | | 25.5 | 25.1 | | | | |
| 00077E6B0F-22 | 3 | F | TS | 27 | 24.4 | 24.0 | 25.1 | 25.7 | | 25.9 | | | 24.8 | | 25.9 | | 26.2 | | 26.7 | 26.3 | | 27.1 | | |
| 00077E6D43-16 | 3 | F | TS | 27 | 26.7 | 26.1 | 26.6 | 25.9 | | 26.5 | | | 25.4 | | 26.9 | | 27.3 | | 27.2 | 27.2 | | 28.3 | | |
| 00077E7DF2-13 | 3 | F | TS | 27 | 24.1 | 24.1 | 25.3 | 25.2 | | 25.6 | | | 25.3 | | 26.9 | | 27.1 | | 27.5 | 27.6 | | 28.0 | | |
| 00077E8C4C-5 | 3 | F | TS | 32 | 21.7 | 21.4 | 22.6 | 22.3 | | 22.9 | | | 22.3 | | 23.7 | | 23.7 | | 24.2 | 24.4 | | 24.6 | | 24.2 |
| 00077E6B73-26 | 4 | F | TS | 25 | 24.4 | 23.5 | 24.8 | 24.2 | | 25.0 | | | 24.1 | | 25.8 | | 26.0 | | 26.6 | 26.7 | | | | |
| 00077E6D2A-28 | 4 | F | TS | 27 | 22.5 | 22.1 | 23.3 | 23.0 | | 23.2 | | | 23.1 | | 23.9 | | 23.5 | | 23.9 | 24.1 | | 25.1 | | |
| 00077E6D5A-2 | 4 | F | TS | 34 | 23.2 | 25.7 | 24.6 | 24.0 | | 23.8 | | | 23.7 | | 25.0 | | 24.2 | | 24.7 | 24.6 | | 24.8 | | 25.3 |
| 00077E6D6E-23 | 4 | F | TS | 25 | 25.5 | 25.9 | 26.0 | 26.6 | | 26.6 | | | 25.9 | | 27.3 | | 28.1 | | 28.4 | 28.5 | | | | |
| 00077E7DCC-29 | 4 | F | TS | 39 | 24.3 | 24.3 | 25.8 | 25.5 | | 25.5 | | | 24.4 | | 26.5 | | 26.5 | | 26.3 | 26.4 | | 27.3 | | 27.1 |
| 00077E6B14-1 | 5 | F | SS | 50 | 21.8 | 21.3 | 22.5 | 22.2 | 22.1 | 22.4 | 22.2 | 22.3 | 21.6 | 22.3 | 22.6 | 22.5 | 22.7 | 22.9 | 23.2 | 22.5 | 23.1 | 23.1 | 22.9 | 23.4 |
| 00077E6BD0-27 | 5 | F | TS | 41 | 23.7 | 24.1 | 24.6 | 24.9 | 24.3 | 24.6 | 24.9 | 24.9 | 24.1 | 25.0 | 25.4 | 25.2 | 25.8 | 25.0 | 25.1 | 24.8 | 24.9 | 24.9 | 24.7 | 24.4 |
| 00077E80AD-19 | 5 | F | TS | 48 | 23.0 | 22.7 | 23.4 | 22.2 | 23.1 | 23.0 | 23.8 | 23.4 | 22.6 | 23.4 | 23.7 | 24.1 | 25.1 | 25.1 | 25.6 | 24.6 | 24.5 | 24.6 | 24.3 | 24.7 |
| 00077E8A89-25 | 5 | F | TS | 22 | 22.3 | 22.4 | 23.1 | 22.8 | 23.3 | 23.5 | 23.3 | 23.6 | 23.0 | 23.9 | 24.6 | 24.8 | 25.0 | | | | | | | |
| 00077E8CC4-9 | 5 | F | SS | 50 | 25.3 | 25.3 | 25.6 | 25.5 | 26.2 | 26.0 | 26.5 | 26.6 | 25.8 | 26.8 | 27.1 | 27.3 | 27.3 | 26.8 | 27.2 | 26.5 | 26.4 | 26.4 | 26.2 | 26.5 |

| Animal ID | Group | Sex | Fate Code | Fate Day | 5/24 | 5/25 | 5/26 | 5/27 | 5/28 | 5/29 | 5/30 | 5/31 | 6/1 | 6/2 | 6/3 | 6/4 | 6/5 | 6/6 | 6/7 | 6/8 | 6/9 | 6/10 | 6/11 | 6/13 |
|---|---|---|---|---|---|---|---|---|---|---|---|---|---|---|---|---|---|---|---|---|---|---|---|---|
| | | | | | 30 | 31 | 32 | 33 | 34 | 35 | 36 | 37 | 38 | 39 | 40 | 41 | 42 | 43 | 44 | 45 | 46 | 47 | 48 | 50 |
| 00077E6CC9-7 | 1 | F | TS | 27 | | | | | | | | | | | | | | | | | | | | |
| 00077E7D9C-3 | 1 | F | TS | 25 | | | | | | | | | | | | | | | | | | | | |
| 00077E7ED6-17 | 1 | F | TS | 41 | | | 27.1 | | 26.5 | | 26.3 | | | 26.8 | | 27.0 | | | | | | | | |
| 00077E7FDB-14 | 1 | F | SS | 50 | | | 28.1 | | 27.1 | | 27.8 | | | 27.8 | | 28.2 | | 27.9 | | | 28.8 | | 29.1 | 29.3 |
| 00077E8B08-20 | 1 | F | TS | 27 | | | | | | | | | | | | | | | | | | | | |
| 00077E6AFE-21 | 2 | F | SS | 50 | | | 22.2 | | 20.7 | | 21.5 | | | 21.7 | | 22.1 | | 22.3 | | | 22.5 | | 23.3 | 22.7 |
| 00077E6C4D-12 | 2 | F | SS | 50 | | | 25.0 | | 24.1 | | 24.6 | | | 24.6 | | 24.9 | | 25.4 | | | 24.9 | | 25.7 | 24.5 |
| 00077E7EF9-18 | 2 | F | TS | 32 | | | 27.9 | | | | | | | | | | | | | | | | | |
| 00077E8B5F-30 | 2 | F | TS | 25 | | | | | | | | | | | | | | | | | | | | |
| 00077E8C0D-4 | 2 | F | TS | 22 | | | | | | | | | | | | | | | | | | | | |
| 00077E6ABD-11 | 3 | F | TS | 25 | | | | | | | | | | | | | | | | | | | | |
| 00077E6B0F-22 | 3 | F | TS | 27 | | | | | | | | | | | | | | | | | | | | |
| 00077E6D43-16 | 3 | F | TS | 27 | | | | | | | | | | | | | | | | | | | | |
| 00077E7DF2-13 | 3 | F | TS | 27 | | | | | | | | | | | | | | | | | | | | |
| 00077E8C4C-5 | 3 | F | TS | 32 | | | 25.1 | | | | | | | | | | | | | | | | | |
| 00077E6B73-26 | 4 | F | TS | 25 | | | | | | | | | | | | | | | | | | | | |
| 00077E6D2A-28 | 4 | F | TS | 27 | | | | | | | | | | | | | | | | | | | | |
| 00077E6D5A-2 | 4 | F | TS | 34 | | | 26.6 | | 26.7 | | | | | | | | | | | | | | | |
| 00077E6D6E-23 | 4 | F | TS | 25 | | | | | | | | | | | | | | | | | | | | |
| 00077E7DCC-29 | 4 | F | TS | 39 | | | 28.3 | | 27.7 | | 28.2 | | | 27.6 | | | | | | | | | | |
| 00077E6B14-1 | 5 | F | SS | 50 | 23.8 | 23.8 | 23.7 | 23.5 | 23.5 | 23.0 | 24.2 | 24.2 | 23.9 | 23.1 | 23.2 | 23.3 | 23.8 | 23.9 | 24.2 | 24.7 | 24.3 | 23.4 | 23.8 | 23.6 |
| 00077E6BD0-27 | 5 | F | TS | 41 | 24.2 | 24.8 | 24.2 | 23.9 | 23.6 | 23.0 | 23.7 | 23.6 | 23.7 | 22.9 | 23.3 | 23.4 | | | | | | | | |
| 00077E80AD-19 | 5 | F | TS | 48 | 24.5 | 25.0 | 25.3 | 24.7 | 25.2 | 24.5 | 25.5 | 25.8 | 26.1 | 24.9 | 25.7 | 25.4 | 25.6 | 25.9 | 25.9 | 25.8 | 26.1 | 25.3 | 25.7 | |
| 00077E8A89-25 | 5 | F | TS | 22 | | | | | | | | | | | | | | | | | | | | |
| 00077E8CC4-9 | 5 | F | SS | 50 | 27.1 | 26.8 | 27.1 | 26.1 | 26.9 | 26.1 | 27.0 | 27.5 | 27.5 | 26.7 | 27.0 | 27.6 | 27.3 | 27.5 | 27.7 | 28.2 | 27.8 | 26.6 | 27.8 | 27.8 |

# Figure 31: Individual body weights – Relative

Relative

| Animal ID | Group | Sex | Fate Code | Fate Day | 4/30 (6) | 5/2 (8) | 5/6 (12) | 5/7 (13) | 5/8 (14) | 5/9 (15) | 5/10 (16) | 5/11 (17) | 5/12 (18) | 5/13 (19) | 5/14 (20) | 5/15 (21) | 5/16 (22) | 5/17 (23) | 5/18 (24) | 5/19 (25) | 5/20 (26) | 5/21 (27) | 5/22 (28) | 5/23 (29) |
|---|---|---|---|---|---|---|---|---|---|---|---|---|---|---|---|---|---|---|---|---|---|---|---|---|
| | | | | | Date/Study Day: Pre-Dosing | | Dosing & Recovery | | | | | | | | | | | | | | | | | |
| 00077E6CC9-7 | 1 | F | TS | 27 | 95.7% | 95.7% | 100.0% | 101.2% | | 103.9% | | | 100.4% | | 103.9% | | 106.3% | | 106.3% | 110.2% | | 111.4% | | |
| 00077E7D9C-3 | 1 | F | TS | 25 | 93.6% | 93.6% | 100.0% | 98.7% | | 98.7% | | | 99.6% | | 103.4% | | 107.6% | | 111.0% | 110.6% | | | | |
| 00077E7ED6-17 | 1 | F | TS | 41 | 90.8% | 96.0% | 100.0% | 102.0% | | 100.8% | | | 96.4% | | 99.2% | | 100.8% | | 102.8% | 103.6% | | 106.4% | | 106.4% |
| 00077E7FDB-14 | 1 | F | SS | 50 | 100.0% | 97.8% | 100.0% | 100.0% | | 100.4% | | | 97.4% | | 103.7% | | 100.7% | | 100.0% | 102.2% | | 101.9% | | 101.5% |
| 00077E8B08-20 | 1 | F | TS | 27 | 94.1% | 94.9% | 100.0% | 100.8% | | 98.7% | | | 101.7% | | 104.6% | | 104.6% | | 105.9% | 104.6% | | 104.2% | | |
| 00077E6AFE-21 | 2 | F | SS | 50 | 97.2% | 97.2% | 100.0% | 103.3% | | 104.2% | | | 100.9% | | 103.3% | | 105.1% | | 105.1% | 106.1% | | 104.7% | | 102.3% |
| 00077E6C4D-12 | 2 | F | SS | 50 | 94.4% | 97.0% | 100.0% | 102.1% | | 104.3% | | | 104.3% | | 106.0% | | 106.9% | | 108.6% | 106.9% | | 107.7% | | 107.7% |
| 00077E7EF9-18 | 2 | F | TS | 32 | 96.4% | 96.8% | 100.0% | 100.8% | | 99.6% | | | 96.0% | | 100.4% | | 99.6% | | 105.2% | 106.4% | | 110.4% | | 108.8% |
| 00077E8B5F-30 | 2 | F | TS | 25 | 92.9% | 91.7% | 100.0% | 100.8% | | 99.6% | | | 99.6% | | 103.2% | | 107.9% | | 108.7% | 107.9% | | | | |
| 00077E8C0D-4 | 2 | F | TS | 22 | 93.1% | 94.7% | 100.0% | 99.2% | | 100.0% | | | 97.2% | | 100.0% | | 102.8% | | | | | | | |
| 00077E6ABD-11 | 3 | F | TS | 25 | 96.2% | 95.4% | 100.0% | 99.6% | | 100.4% | | | 97.1% | | 102.9% | | 102.9% | | 106.7% | 105.0% | | | | |
| 00077E6B0F-22 | 3 | F | TS | 27 | 97.2% | 95.6% | 100.0% | 102.4% | | 103.2% | | | 98.8% | | 103.2% | | 104.4% | | 106.4% | 104.8% | | 108.0% | | |
| 00077E6D43-16 | 3 | F | TS | 27 | 100.4% | 98.1% | 100.0% | 97.4% | | 99.6% | | | 95.5% | | 101.1% | | 102.6% | | 102.3% | 102.3% | | 106.4% | | |
| 00077E7DF2-13 | 3 | F | TS | 27 | 95.3% | 95.3% | 100.0% | 99.6% | | 101.2% | | | 100.0% | | 106.3% | | 107.1% | | 108.7% | 109.1% | | 110.7% | | |
| 00077E8C4C-5 | 3 | F | TS | 32 | 96.0% | 94.7% | 100.0% | 98.7% | | 101.3% | | | 98.7% | | 104.9% | | 104.9% | | 107.1% | 108.0% | | 108.8% | | 107.1% |
| 00077E6B73-26 | 4 | F | TS | 25 | 98.4% | 94.8% | 100.0% | 97.6% | | 100.8% | | | 97.2% | | 104.0% | | 104.8% | | 107.3% | 107.7% | | | | |
| 00077E6D2A-28 | 4 | F | TS | 27 | 96.6% | 94.8% | 100.0% | 98.7% | | 99.6% | | | 99.1% | | 102.6% | | 100.9% | | 102.6% | 103.4% | | 107.7% | | |
| 00077E6D5A-2 | 4 | F | TS | 34 | 94.3% | 104.5% | 100.0% | 97.6% | | 96.7% | | | 96.3% | | 101.6% | | 98.4% | | 100.4% | 100.0% | | 100.8% | | 102.8% |
| 00077E6D6E-23 | 4 | F | TS | 25 | 98.1% | 99.6% | 100.0% | 102.3% | | 102.3% | | | 99.6% | | 105.0% | | 108.1% | | 109.2% | 109.6% | | | | |
| 00077E7DCC-29 | 4 | F | TS | 39 | 94.2% | 94.2% | 100.0% | 98.8% | | 98.8% | | | 94.6% | | 102.7% | | 102.7% | | 101.9% | 102.3% | | 105.8% | | 105.0% |
| 00077E6B14-1 | 5 | F | SS | 50 | 96.9% | 94.7% | 100.0% | 98.7% | 98.2% | 99.6% | 98.7% | 99.1% | 96.0% | 99.1% | 100.4% | 100.0% | 100.9% | 101.8% | 103.1% | 100.0% | 102.7% | 102.7% | 101.8% | 104.0% |
| 00077E6BD0-27 | 5 | F | TS | 41 | 96.3% | 98.0% | 100.0% | 101.2% | 98.8% | 100.0% | 101.2% | 101.2% | 98.0% | 101.6% | 103.3% | 102.4% | 104.9% | 101.6% | 102.0% | 100.8% | 101.2% | 101.2% | 100.4% | 99.2% |
| 00077E80AD-19 | 5 | F | TS | 48 | 98.3% | 97.0% | 100.0% | 94.9% | 98.7% | 98.3% | 101.7% | 100.0% | 96.6% | 100.0% | 101.3% | 103.0% | 107.3% | 107.3% | 109.4% | 105.1% | 104.7% | 105.1% | 103.8% | 105.6% |
| 00077E8A89-25 | 5 | F | TS | 22 | 96.5% | 97.0% | 100.0% | 98.7% | 100.9% | 101.7% | 100.9% | 102.2% | 99.6% | 103.5% | 106.5% | 107.4% | 108.2% | | | | | | | |
| 00077E8CC4-9 | 5 | F | SS | 50 | 98.8% | 98.8% | 100.0% | 99.6% | 102.3% | 101.6% | 103.5% | 103.9% | 100.8% | 104.7% | 105.9% | 106.6% | 106.6% | 104.7% | 106.3% | 103.5% | 103.1% | 103.1% | 102.3% | 103.5% |

| Animal ID | Group | Sex | Fate Code | Fate Day | 5/24 (30) | 5/25 (31) | 5/26 (32) | 5/27 (33) | 5/28 (34) | 5/29 (35) | 5/30 (36) | 5/31 (37) | 6/1 (38) | 6/2 (39) | 6/3 (40) | 6/4 (41) | 6/5 (42) | 6/6 (43) | 6/7 (44) | 6/8 (45) | 6/9 (46) | 6/10 (47) | 6/11 (48) | 6/13 (50) |
|---|---|---|---|---|---|---|---|---|---|---|---|---|---|---|---|---|---|---|---|---|---|---|---|---|
| 00077E6CC9-7 | 1 | F | TS | 27 | | | | | | | | | | | | | | | | | | | | |
| 00077E7D9C-3 | 1 | F | TS | 25 | | | | | | | | | | | | | | | | | | | | |
| 00077E7ED6-17 | 1 | F | TS | 41 | | | 108.4% | | 106.0% | | 105.2% | | | 107.2% | | 108.0% | | | | | | | | |
| 00077E7FDB-14 | 1 | F | SS | 50 | | | 105.2% | | 101.5% | | 104.1% | | | 104.1% | | 105.6% | | 104.5% | | | 107.9% | | 109.0% | 109.7% |
| 00077E8B08-20 | 1 | F | TS | 27 | | | | | | | | | | | | | | | | | | | | |
| 00077E6AFE-21 | 2 | F | SS | 50 | | | 103.7% | | 96.7% | | 100.5% | | | 101.4% | | 103.3% | | 104.2% | | | 105.1% | | 108.9% | 106.1% |
| 00077E6C4D-12 | 2 | F | SS | 50 | | | 107.3% | | 103.4% | | 105.6% | | | 105.6% | | 106.9% | | 109.0% | | | 106.9% | | 110.3% | 105.2% |
| 00077E7EF9-18 | 2 | F | TS | 32 | | | 112.0% | | | | | | | | | | | | | | | | | |
| 00077E8B5F-30 | 2 | F | TS | 25 | | | | | | | | | | | | | | | | | | | | |
| 00077E8C0D-4 | 2 | F | TS | 22 | | | | | | | | | | | | | | | | | | | | |
| 00077E6ABD-11 | 3 | F | TS | 25 | | | | | | | | | | | | | | | | | | | | |
| 00077E6B0F-22 | 3 | F | TS | 27 | | | | | | | | | | | | | | | | | | | | |
| 00077E6D43-16 | 3 | F | TS | 27 | | | | | | | | | | | | | | | | | | | | |
| 00077E7DF2-13 | 3 | F | TS | 27 | | | | | | | | | | | | | | | | | | | | |
| 00077E8C4C-5 | 3 | F | TS | 32 | | | 111.1% | | | | | | | | | | | | | | | | | |
| 00077E6B73-26 | 4 | F | TS | 25 | | | | | | | | | | | | | | | | | | | | |
| 00077E6D2A-28 | 4 | F | TS | 27 | | | | | | | | | | | | | | | | | | | | |
| 00077E6D5A-2 | 4 | F | TS | 34 | | | 108.1% | | 108.5% | | | | | | | | | | | | | | | |
| 00077E6D6E-23 | 4 | F | TS | 25 | | | | | | | | | | | | | | | | | | | | |
| 00077E7DCC-29 | 4 | F | TS | 39 | | | 109.7% | | 107.4% | | 109.3% | | | 107.0% | | | | | | | | | | |
| 00077E6B14-1 | 5 | F | SS | 50 | 105.8% | 105.8% | 105.3% | 104.4% | 104.4% | 102.2% | 107.6% | 107.6% | 106.2% | 102.7% | 103.1% | 103.6% | 105.8% | 106.2% | 107.6% | 109.8% | 108.0% | 104.0% | 105.8% | 104.9% |
| 00077E6BD0-27 | 5 | F | TS | 41 | 98.4% | 100.8% | 98.4% | 97.2% | 95.9% | 93.5% | 96.3% | 95.9% | 96.3% | 93.1% | 94.7% | 95.1% | | | | | | | | |
| 00077E80AD-19 | 5 | F | TS | 48 | 104.7% | 106.8% | 108.1% | 105.6% | 107.7% | 104.7% | 109.0% | 110.3% | 111.5% | 106.4% | 109.8% | 108.5% | 109.4% | 110.7% | 110.7% | 110.3% | 111.5% | 108.1% | 109.8% | |
| 00077E8A89-25 | 5 | F | TS | 22 | | | | | | | | | | | | | | | | | | | | |
| 00077E8CC4-9 | 5 | F | SS | 50 | 105.9% | 104.7% | 105.9% | 102.0% | 105.1% | 102.0% | 105.5% | 107.4% | 107.4% | 104.3% | 105.5% | 107.8% | 106.6% | 107.4% | 108.2% | 110.2% | 108.6% | 103.9% | 108.6% | 108.6% |

# Figure 32: Individual tumour volume measurements

| Animal ID | Group | Sex | Fate | | Date/Study Day | | | | | | | | | | | | | | | | | | | | |
|---|---|---|---|---|---|---|---|---|---|---|---|---|---|---|---|---|---|---|---|---|---|---|---|---|---|
| | | | | | Pre-Dosing | | Dosing & Recovery | | | | | | | | | | | | | | | | | | |
| | | | Code | Day | 4/30 6 | 5/2 8 | 5/6 12 | 5/7 13 | 5/9 15 | 5/11 17 | 5/13 19 | 5/14 20 | 5/16 22 | 5/19 25 | 5/21 27 | 5/23 29 | 5/26 32 | 5/28 34 | 5/30 36 | 6/2 39 | 6/4 41 | 6/6 43 | 6/9 46 | 6/11 48 | 6/13 50 |
| 00077E6CC9-7 | 1 | F | TS | 27 | 65.0 | 121.1 | 146.1 | 140.3 | 227.3 | 343.2 | 571.4 | 612.1 | 870.7 | 1,248.1 | 1,803.0 | | | | | | | | | | |
| 00077E7D9C-3 | 1 | F | TS | 25 | 152.6 | 178.5 | 198.4 | 233.9 | 374.7 | 529.9 | 874.7 | 998.4 | 1,313.2 | 1,765.0 | | | | | | | | | | | |
| 00077E7ED6-17 | 1 | F | TS | 41 | 55.2 | 92.3 | 106.0 | 132.6 | 196.0 | 221.2 | 343.2 | 400.0 | 537.8 | 740.7 | 819.3 | 1,107.1 | 1,225.3 | 1,306.8 | 1,278.5 | 1,412.4* | 1,661.0* | | | | |
| 00077E7FDB-14 | 1 | F | SS | 50 | 78.7 | 94.1 | 181.8 | 226.5 | 228.1 | 216.3 | 194.5 | 173.8 | 122.4 | 37.0 | 20.7 | 0.0 | 0.0 | 0.0 | 0.0 | 0.0 | 0.0 | 0.0 | 0.0 | 0.0 | 0.0 |
| 00077E8B08-20 | 1 | F | TS | 27 | 65.6 | 108.8 | 142.2 | 172.7 | 192.8 | 287.1 | 372.1 | 467.0 | 676.0 | 1,030.2 | 1,561.6 | | | | | | | | | | |
| 00077E64FE-21 | 2 | F | SS | 50 | 66.7 | 100.0 | 101.1 | 131.4 | 163.3 | 134.0 | 124.2 | 113.7 | 71.3 | 36.1 | 23.7 | 22.3 | 17.9 | 10.6 | 0.0 | 0.0 | 0.0 | 0.0 | 0.0 | 0.0 | 0.0 |
| 00077E6C4D-12 | 2 | F | SS | 50 | 116.6 | 134.0 | 248.4 | 305.9 | 461.3 | 509.9 | 548.4 | 596.8 | 619.0 | 681.2 | 771.5 | 777.7 | 643.5 | 578.2 | 497.7 | 433.0 | 364.5 | 353.3 | 271.5 | 317.5 | 261.6 |
| 00077E7EF9-18 | 2 | F | TS | 32 | 65.7 | 121.8 | 135.0 | 154.8 | 198.2 | 244.5 | 391.6 | 431.4 | 624.3 | 936.5 | 1,175.8 | 1,376.3 | 1,679.8 | | | | | | | | |
| 00077E8B5F-30 | 2 | F | TS | 25 | 111.4 | 157.7 | 183.0 | 216.6 | 395.7 | 590.6 | 831.7 | 916.2 | 1,160.9 | 1,887.0 | | | | | | | | | | | |
| 00077E8C0D-4 | 2 | F | TS | 22 | 121.8 | 131.2 | 153.2 | 219.6 | 284.2 | 514.0 | 800.0 | 1,004.3 | 1,317.7 | | | | | | | | | | | | |
| 00077E6ABD-11 | 3 | F | TS | 25 | 118.4 | 178.8 | 228.2 | 234.2 | 343.9 | 530.5 | 943.2 | 1,105.9 | 1,344.4 | 1,869.7 | | | | | | | | | | | |
| 00077E6B0F-22 | 3 | F | TS | 27 | 89.2 | 188.7 | 158.8 | 197.6 | 285.9 | 492.7 | 686.7 | 831.7 | 1,040.8 | 1,352.0 | 1,671.3 | | | | | | | | | | |
| 00077E6D43-16 | 3 | F | TS | 27 | 60.3 | 202.2 | 180.3 | 206.7 | 251.3 | 278.4 | 406.6 | 487.4 | 719.1 | 1,059.5 | 1,580.5 | | | | | | | | | | |
| 00077E7DF2-13 | 3 | F | TS | 27 | 88.9 | 142.2 | 156.1 | 208.1 | 311.3 | 425.3 | 595.6 | 742.6 | 976.6 | 1,298.4 | 1,705.2 | | | | | | | | | | |
| 00077E9C4C-5 | 3 | F | TS | 32 | 118.6 | 97.5 | 104.4 | 178.5 | 240.9 | 367.1 | 468.3 | 550.9 | 679.8 | 909.9 | 1,137.8 | 1,298.0 | 1,552.1 | | | | | | | | |
| 00077E6B73-26 | 4 | F | TS | 25 | 102.3 | 175.7 | 238.2 | 255.9 | 411.9 | 654.4 | 840.4 | 1,038.5 | 1,306.3 | 1,574.7 | | | | | | | | | | | |
| 00077E6D2A-28 | 4 | F | TS | 27 | 51.6 | 86.2 | 130.2 | 161.2 | 238.2 | 320.3 | 458.4 | 531.6 | 910.3 | 1,261.3 | 1,671.5 | | | | | | | | | | |
| 00077E6D5A-2 | 4 | F | TS | 34 | 109.7 | 130.7 | 131.4 | 205.2 | 221.8 | 362.4 | 422.0 | 463.7 | 601.9 | 839.8 | 928.5 | 998.4 | 1,166.1 | 1,639.9 | | | | | | | |
| 00077E6D6E-23 | 4 | F | TS | 25 | 82.9 | 169.1 | 195.3 | 231.9 | 332.8 | 488.6 | 691.9 | 862.5 | 1,055.0 | 1,562.9 | | | | | | | | | | | |
| 00077E7DCC-29 | 4 | F | TS | 39 | 100.7 | 97.7 | 117.2 | 150.3 | 228.3 | 278.4 | 402.0 | 423.6 | 489.3 | 550.9 | 650.5 | 660.7 | 810.7 | 919.4 | 1,187.6 | 1,636.6* | | | | | |
| 00077E6814-1 | 5 | F | SS | 50 | 111.4 | 109.7 | 128.4 | 125.3 | 153.6 | 176.4 | 203.4 | 185.9 | 222.9 | 244.5 | 248.8 | 251.6 | 281.3 | 274.4 | 281.6 | 330.7 | 220.5 | 228.1 | 260.9 | 218.1 | 202.2 |
| 00077E6BD0-27 | 5 | F | TS | 41 | 85.0 | 188.7 | 162.7 | 210.8 | 263.8 | 429.3 | 552.2 | 711.5 | 866.2 | 1,146.9 | 1,184.1 | 1,122.3 | 1,237.1 | 1,187.8 | 1,328.7 | 1,439.4* | 1,800.0* | | | | |
| 00077E80AD-19 | 5 | F | TS | 48 | 81.2 | 115.3 | 165.5 | 210.9 | 291.2 | 382.2 | 528.2 | 593.0 | 786.5 | 932.9 | 903.5 | 1,049.3 | 1,145.2 | 1,070.3 | 1,079.6 | 1,225.3* | 1,286.5* | 1,321.4* | 1,376.0* | 1,639.9* | |
| 00077E8A89-25 | 5 | F | TS | 22 | 136.5 | 198.8 | 296.4 | 318.2 | 525.3 | 752.3 | 1,039.5 | 1,185.5 | 1,669.2 | | | | | | | | | | | | |
| 00077E8CC4-9 | 5 | F | SS | 50 | 82.5 | 75.7 | 121.1 | 72.5 | 178.6 | 312.7 | 330.7 | 402.7 | 517.5 | 588.8 | 523.4 | 523.4 | 440.1 | 402.7 | 537.8 | 508.1 | 480.0 | 452.8 | 423.8 | 427.7 | 439.6 |

Tumour ulceration grade 1

Tumour ulceration grade 2

Figure 33: Individual group plots

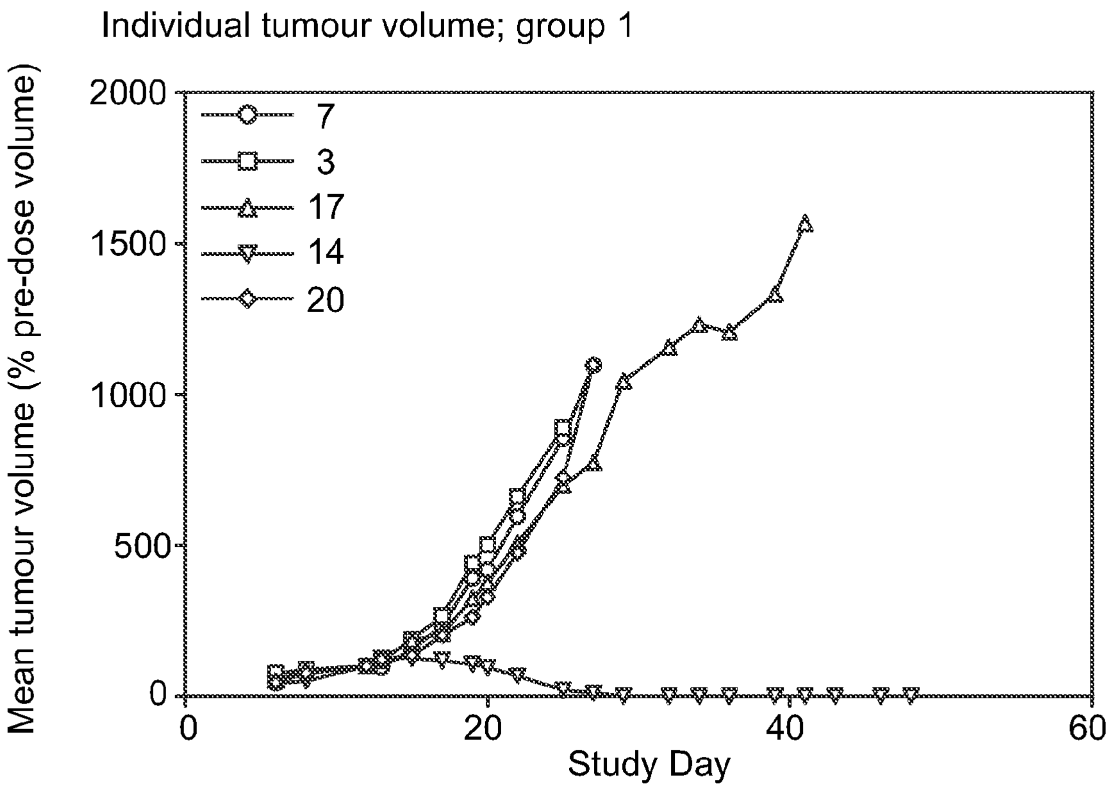


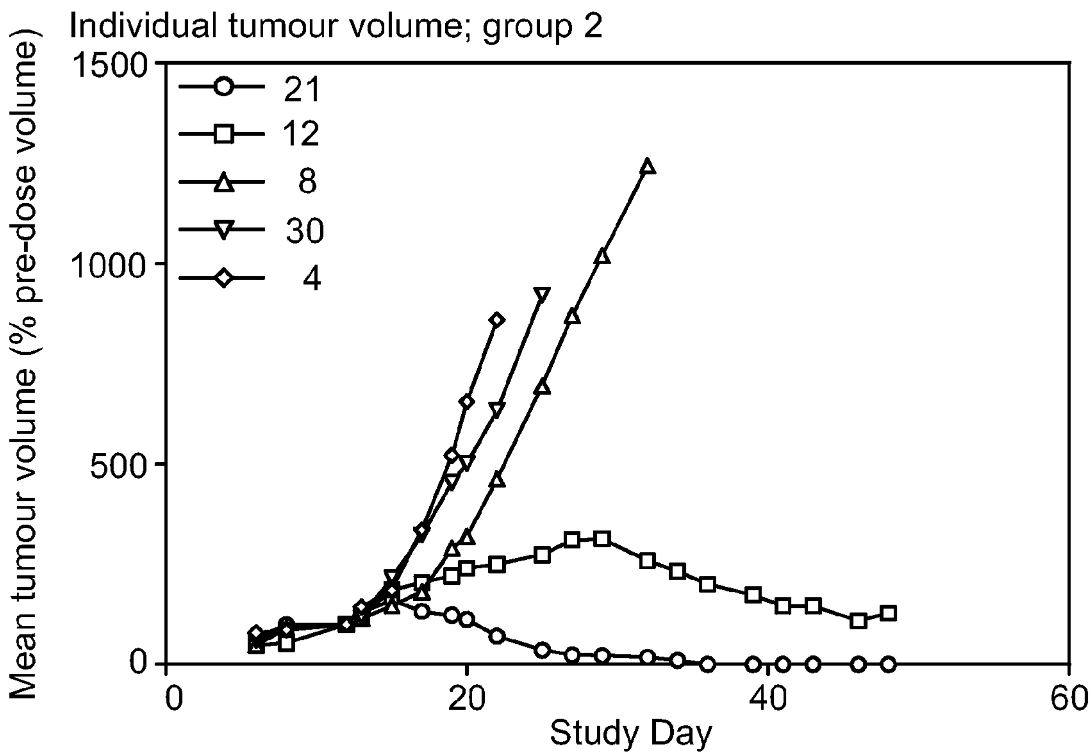

Figure 33(contd): Individual group plots

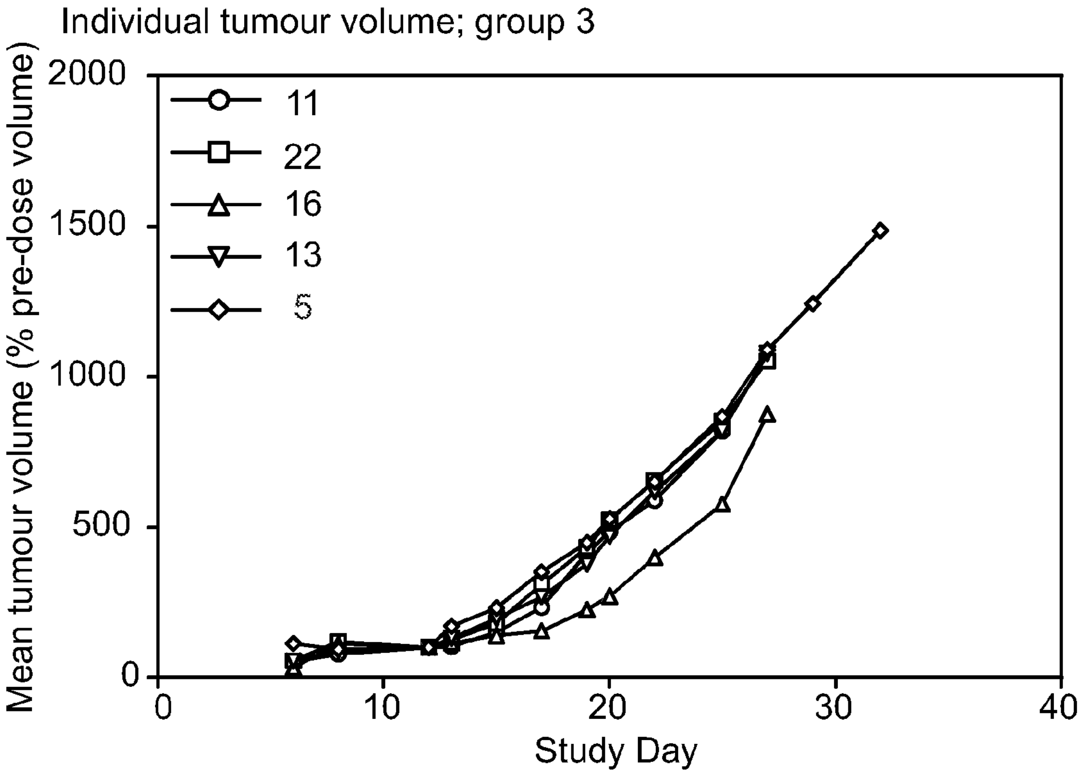


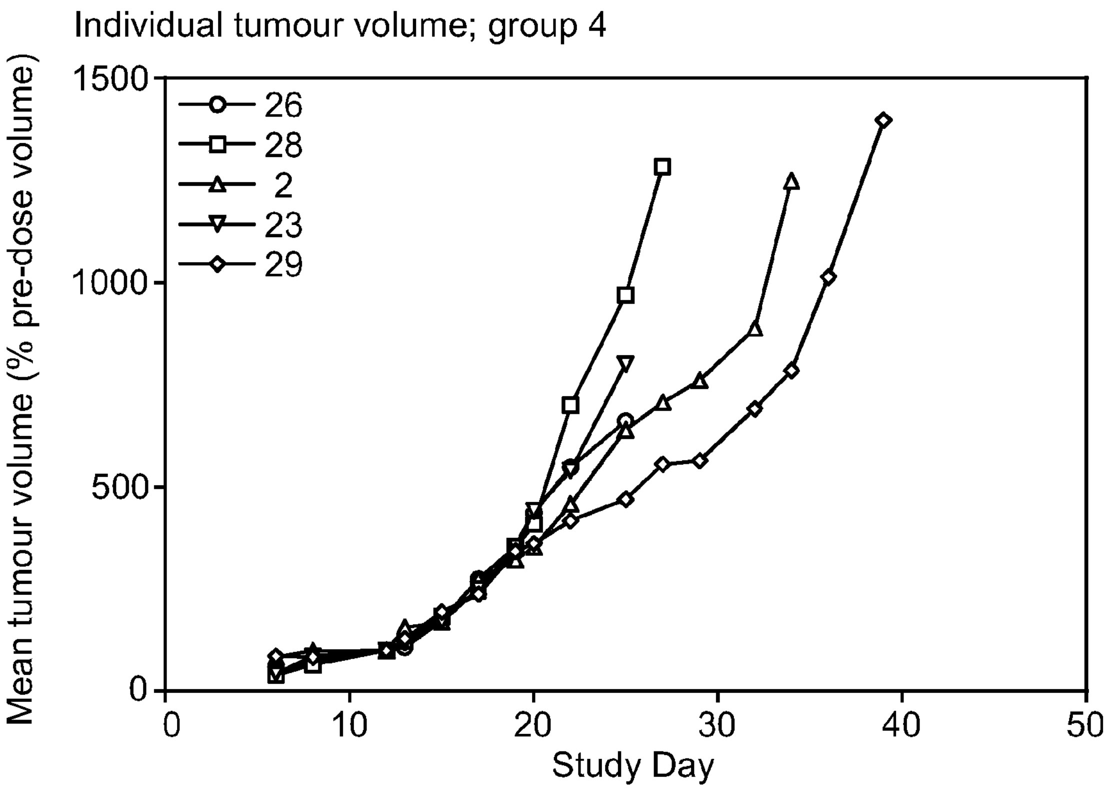

Figure 33(contd): Individual group plots

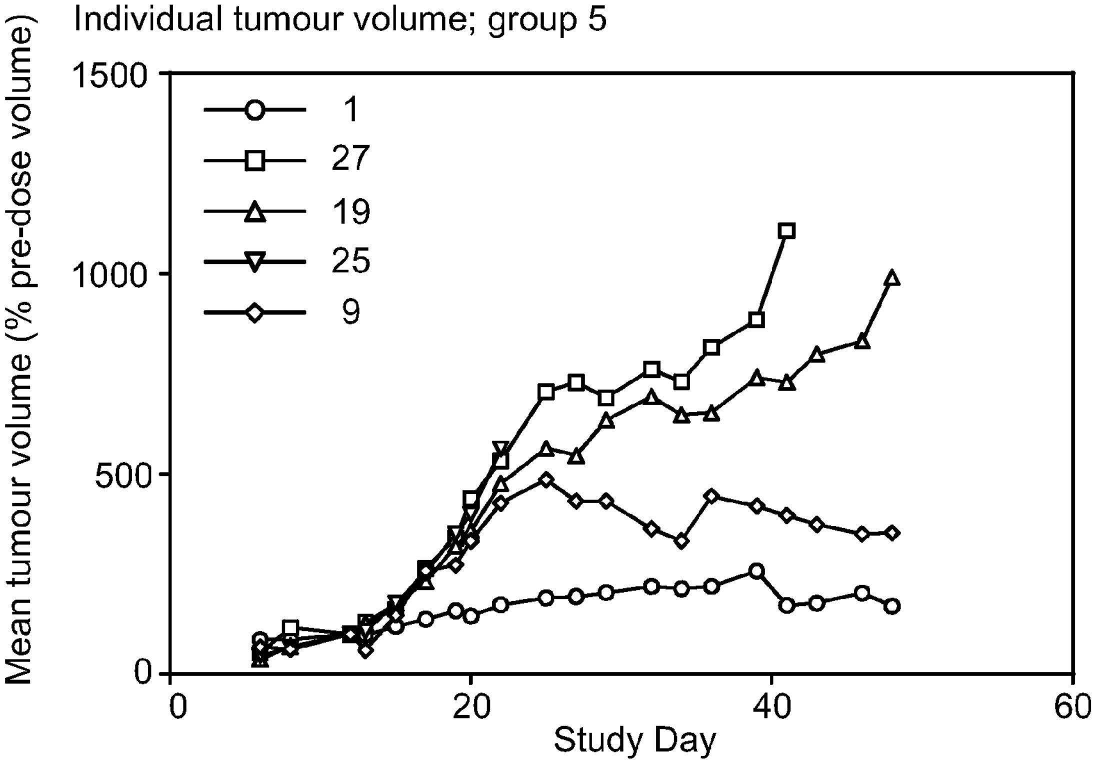

Figure 34: Tumour weight

| Group | Animal ID | Date | Day | Final Tumour Weight/ g | Endpoint |
|---|---|---|---|---|---|
| 1 | 00077E6CC9-7 | 21/05/2014 | 27 | 1.347 | Oversized tumour |
| 1 | 00077E7D9C-3 | 19/05/2014 | 25 | 1.761 | Oversized tumour |
| 1 | 00077E7ED6-17 | 04/06/2014 | 41 | 1.128 | Oversized tumour |
| 1 | 00077E7FDB-14 | 13/06/2014 | 50 | No tumour present | Study Termination |
| 1 | 00077E8B08-20 | 21/05/2014 | 27 | 1.197 | Oversized tumour |
| 2 | 00077E6AFE-21 | 13/06/2014 | 50 | No tumour present | Study Termination |
| 2 | 00077E6C4D-12 | 13/06/2014 | 50 | 0.091 | Study Termination |
| 2 | 00077E7EF9-18 | 26/05/2014 | 32 | 1.882 | Oversized tumour |
| 2 | 00077E8B5F-30 | 19/05/2014 | 25 | 1.432 | Oversized tumour |
| 2 | 00077E8C0D-4 | 16/05/2014 | 22 | 1.119 | Oversized tumour |
| 3 | 00077E6ABD-11 | 19/05/2014 | 25 | 1.717 | Oversized tumour |
| 3 | 00077E6B0F-22 | 21/05/2014 | 27 | 1.398 | Oversized tumour |
| 3 | 00077E6D43-16 | 21/05/2014 | 27 | 1.098 | Oversized tumour |
| 3 | 00077E7DF2-13 | 21/05/2014 | 27 | 1.45 | Oversized tumour |
| 3 | 00077E8C4C-5 | 26/05/2014 | 32 | 1.185 | Oversized tumour |
| 4 | 00077E6B73-26 | 19/05/2014 | 25 | 1.714 | Oversized tumour |
| 4 | 00077E6D2A-28 | 21/05/2014 | 27 | 1.399 | Oversized tumour |
| 4 | 00077E6D5A-2 | 28/05/2014 | 34 | 1.42 | Overszied tumour |
| 4 | 00077E6D6E-23 | 19/05/2014 | 25 | 1.42 | Oversized tumour |
| 4 | 00077E7DCC-29 | 02/06/2014 | 39 | 1.781 | Oversized tumour |
| 5 | 00077E6B14-1 | 13/06/2014 | 50 | 0.164 | Study Termination |
| 5 | 00077E6BD0-27 | 04/06/2014 | 41 | 1.474 | Oversized tumour |
| 5 | 00077E80AD-19 | 11/06/2014 | 48 | 1.418 | Oversized tumour |
| 5 | 00077E8A89-25 | 16/05/2014 | 22 | 1.143 | Oversized tumour |
| 5 | 00077E8CC4-9 | 13/06/2014 | 50 | 0.297 | Study Termination |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009105044 A
- WO 2013150303 A
- WO 2014013258 A
- US 5851832 A
- US 6777233 B
- US 6468794 B
- US 5753506 A
- WO 2005121318 A
- US 7416888 B
- EP 1645626 B1
- US 7514259 B
- GB 2013050879 W
- WO 2012004611 A
- GB 1314573 A
- GB 1317887 A


### Non-patent literature cited in the description


- **HASSANI ; O'REILLY ; PEARSE ; STROEMER et al.** *PLoS One,* 2012, vol. 7 (11
- **FONSATO et al.** *Stem Cells,* September 2012, vol. 30 (9), 1985-98
- **MILJAN et al.** *Stem Cells Dev.,* 2009
- **KATSUDA et al.** *Proteomics,* 2013
- **KATSUDA et al.** *Scientific Reports,* 2013
- **GENNARO.** Remington: The Science and Practice of Pharmacy. 2000
- **E W MARTIN.** Remington's Pharmaceutical Sciences
- **ISHIWATA et al.** *World J Gastroenterol.,* 28 January 2011, vol. 17 (4), 409-418
- **KAUR et al.** *BMC cancer,* 2012, vol. 12, 120
- **ROFSTAD BR.** *J. Cancer,* 1994, vol. 70, 804-812
- **JACOBS et al.** *ASN Neuro. 2011,* 2011, vol. 3 (3
- **MOKRY et al.** *Stem Cells Dev.,* 2004, vol. 13, 658-664
- **WORKMAN et al.** *British Journal of Cancer,* 2010, vol. 102, 1555-1577
- **AMBROS et al.** *RNA,* 2003, vol. 9, 277-279
- **BANERJEE, S. ; WILLIAMSON, D. ; HABIB, N. ; GORDON, M. ; CHATAWAY, J.** *Age and Ageing,* 2011, vol. 40, 7-13
- **CHUNG et al.** *Cell Stem Cell,* 2008, vol. 2, 113-117
- **DAI, L.J. ; MONIRI, M.R. ; ZENG, Z.R. et al.** *Cancer Lett,* 2011, vol. 305 (1), 8-20
- **DING, D. C. ; SHYU, W. C. ; LIN S. Z.** *Cell Transplant,* 2011, vol. 20, 5-14
- **EINSTEIN, O. ; BEN-HUR, T.** *Arch Neurol,* 2008, vol. 65, 452-456
- **HASSANI Z ; O'REILLY J ; PEARSE Y ; STROEMER P ; TANG E ; SINDEN J ; PRICE J ; THURET S.** Human neural progenitor cell engraftment increases neurogenesis and microglial recruitment in the brain of rats with stroke. *PLoS One,* 2012, vol. 7 (11), e50444
- **HODGES et al.** *Cell Transplant.,* 2007, vol. 16 (2), 101-15
- **HORIE, N. ; PEREIRA, N.P. ; NIIZUMA, K. ; SUN, G. et al.** *Stem Cells,* 2011, vol. 29, 274-285
- **HULKOWER, K. I. ; HERBER, R. L.** *Pharmaceutics,* 2011, vol. 3, 107-124
- **KATSUDA ; KOSAKA ; TAKESHITA ; OCHIYA.** *Proteomics,* 2013, vol. 00, 1-17
- **KATSUDA ; TSUCHIYA ; KOSAKA ; YOSHIOKA ; TAKAGAKI ; OKI ; TAKESHITA ; SAKAI ; KURODA ; OCHIYA.** *Scientific Reports,* 2013, vol. 3 (1197), 1-11
- **KORNBLUM.** *Stroke,* 2007, vol. 38, 810-816
- **LAI et al.** Proteolytic Potential of the MSC Exosome Proteome: Implications for an Exosome-Mediated Delivery of Therapeutic Proteasome. *International Journal of Proteomics,* 2012, 14
- **LITTLEWOOD, T. D. ; HANCOCK, D. C. ; DANIELIAN, P. S. et al.** *Nucleic Acid Research,* 1995, vol. 23, 1686-1690
- **MILJAN, E.A. ; SINDEN, J.D.** *Current Opinion in Molecular Therapeutics,* 2009, vol. 4, 394-403
- **MILJAN EA ; HINES SJ ; PANDE P ; CORTELING RL ; HICKS C ; ZBARSKY V ; UMACHANDRAN, M ; SOWINSKI P ; RICHARDSON S ; TANG E.** Implantation of c-mycER TAM immortalized human mesencephalic-derived clonal cell lines ameliorates behavior dysfunction in a rat model of Parkinson's disease. *Stem Cells Dev.,* March 2009, vol. 18 (2), 307-19

- **MITCHELL et al.** *Journal of Immunological Methods,* 2008, vol. 335, 98-105
- **POLLOCK et al.** *Exp Neurol.,* May 2006, vol. 199 (1), 143-55
- **MARK F PITTENGER ; ALASTAIR M MACKAY ; STEPHEN C BECK ; RAMA K JAISWAL et al.** *Science,* 02 April 1999, vol. 284, 5411
- **SHAH, K.** *Adv Drug Deliv Rev,* 2012, vol. 64 (8), 739-748
- **SMITH, E. J. ; STROEMER, R.P. ; GORENKOVA, N. ; NAKAJIMA, M. et al.** *Stem Cells,* 2012, vol. 30, 785-796
- **STEVENATO, L. ; CORTELING, R. ; STROEMER, P. ; HOPE, A. et al.** *BMC Neuroscience,* 2009, vol. 10, 86
- **STROEMER, P. ; PATEL, S. ; HOPE, A. ; OLIVEIRA, C. ; POLLOCK, K. ; SINDEN, J.** *Neurorehabil Neural Repair,* 2009, vol. 23, 895-909
- **THÉRY, C. ; OSTROWSKI, M. ; SEGURA, E. et al.** *Nature Reviews Immunology,* 2009, vol. 9, 581-593
- **THEIR et al.** Direct Conversion of Fibroblasts into Stably Expandable Neural Stem Cells. *Cell Stem Cell,* 20 March 2012
- **TIMMERS, L. ; LIM, S. K ; ARSLAN, F. ; ARMSTRONG, J. S. et al.** *Stem Cell Res,* 2007, vol. 1, 129-137
- **YUAN, S.J. ; MARTIN, J ; ELIA, J. ; FLIPPIN, J. et al.** *PLoS ONE,* 2011, vol. 6, e17540

SEQUENCE LISTING

<110> Reneuron Limited

<120> Stem Cell Microparticles and miRNA

<130> P062223WO

<141> 2014-02-12

<150> GB 1314573.5
<151> 2013-08-14

<150> GB 1317887.6
<151> 2013-10-09

<160> 752

<170> SeqWin2010, version 1.0

<210> 1
<211> 22
<212> RNA
<213> Homo sapiens

<400> 1
ugagguagua gguuguauag uu 22

<210> 2
<211> 23
<212> RNA
<213> Homo sapiens

<400> 2
uacccuguag auccgaauuu gug 23

<210> 3
<211> 22
<212> RNA
<213> Homo sapiens

<400> 3
aacccguaga uccgaacuug ug 22

<210> 4
<211> 22
<212> RNA
<213> Homo sapiens

<400> 4
cacccguaga accgaccuug cg 22

<210> 5
<211> 22
<212> RNA
<213> Homo sapiens

<400> 5
uccuguacug agcugccccg ag 22

<210> 6
<211> 21
<212> RNA
<213> Homo sapiens

<400> 6
uucacagugg cuaaguucug c 21

<210> 7
<211> 22
<212> RNA

<213> Homo sapiens

<400> 7
uauugcacuu gucccggccu gu 22

<210> 8
<211> 23
<212> RNA
<213> Homo sapiens

<400> 8
caacggaauc ccaaaagcag cug 23

<210> 9
<211> 22
<212> RNA
<213> Homo sapiens

<400> 9
uagcuuauca gacugauguu ga 22

<210> 10
<211> 22
<212> RNA
<213> Homo sapiens

<400> 10
ugagguagua aguuguauug uu 22

<210> 11
<211> 22
<212> RNA
<213> Homo sapiens

<400> 11
ugagguagua gauuguauag uu 22

<210> 12
<211> 22
<212> RNA
<213> Homo sapiens

<400> 12
uucaaguaau ccaggauagg cu 22

<210> 13
<211> 22
<212> RNA
<213> Homo sapiens

<400> 13
uauugcacuc gucccggccu cc 22

<210> 14
<211> 22
<212> RNA
<213> Homo sapiens

<400> 14
ucggauccgu cugagcuugg cu 22

<210> 15
<211> 23
<212> RNA
<213> Homo sapiens

<400> 15
aacauucaac gcugucggug agu 23

<210> 16
<211> 24
<212> RNA
<213> Homo sapiens

<400> 16
uuuggcaaug guagaacuca cacu 24

<210> 17
<211> 22
<212> RNA
<213> Homo sapiens

<400> 17
ugagguagua gguuguaugg uu 22

<210> 18
<211> 21
<212> RNA
<213> Homo sapiens

<400> 18
ugguagacua uggaacguag g 21

<210> 19
<211> 22
<212> RNA
<213> Homo sapiens

<400> 19
ugagaacuga auuccauagg cu 22

<210> 20
<211> 21
<212> RNA
<213> Homo sapiens

<400> 20
caacaccagu cgaugggcug u 21

<210> 21
<211> 19
<212> RNA
<213> Homo sapiens

<400> 21
aauggauuuu uggagcagg 19

<210> 22
<211> 22
<212> RNA
<213> Homo sapiens

<400> 22
ugagguagua guuugugcug uu 22

<210> 23
<211> 17
<212> RNA
<213> Homo sapiens

<400> 23
ccccggggag cccggcg 17

<210> 24
<211> 22
<212> RNA
<213> Homo sapiens

<400> 24
uauggcacug guagaauuca cu 22

<210> 25
<211> 21
<212> RNA
<213> Homo sapiens

<400> 25
cuagacugaa gcuccuugag g 21

<210> 26
<211> 22
<212> RNA
<213> Homo sapiens

<400> 26
aaugcacccg ggcaaggauu cu 22

<210> 27
<211> 22
<212> RNA
<213> Homo sapiens

<400> 27
ugagguagga gguuguauag uu 22

<210> 28
<211> 22
<212> RNA
<213> Homo sapiens

<400> 28
ugagguagua gguugugugg uu 22

<210> 29
<211> 22

<212> RNA
<213> Homo sapiens

<400> 29
uagcagcacg uaaauauugg cg 22

<210> 30
<211> 22
<212> RNA
<213> Homo sapiens

<400> 30
uguaaacauc cucgacugga ag 22

<210> 31
<211> 22
<212> RNA
<213> Homo sapiens

<400> 31
uguaaacauc cccgacugga ag 22

<210> 32
<211> 23
<212> RNA
<213> Homo sapiens

<400> 32
agguuacccg agcaacuuug cau 23

<210> 33
<211> 22
<212> RNA
<213> Homo sapiens

<400> 33
aagcugccag uugaagaacu gu 22

<210> 34
<211> 17
<212> RNA
<213> Homo sapiens

<400> 34
ggggcugggc gcgcgcc 17

<210> 35
<211> 24
<212> RNA
<213> Homo sapiens

<400> 35
ucccugagac ccuuuaaccu guga 24

<210> 36
<211> 21
<212> RNA
<213> Homo sapiens

<400> 36
agcuacaucu ggcuacuggg u 21

<210> 37
<211> 21
<212> RNA
<213> Homo sapiens

<400> 37
ucgaggagcu cacagucuag u 21

<210> 38
<211> 23
<212> RNA
<213> Homo sapiens

<400> 38
aacauucauu gcugucggug ggu 23

<210> 39
<211> 22
<212> RNA
<213> Homo sapiens

<400> 39
accuggcaua caauguagau uu 22

<210> 40
<211> 22
<212> RNA
<213> Homo sapiens

<400> 40
caaagaauuc uccuuuuggg cu 22

<210> 41
<211> 23
<212> RNA
<213> Homo sapiens

<400> 41
ugaggggcag agagcgagac uuu 23

<210> 42
<211> 22
<212> RNA
<213> Homo sapiens

<400> 42
ucccugagac ccuaacuugu ga 22

<210> 43
<211> 22
<212> RNA
<213> Homo sapiens

<400> 43
ugagguagua guuuguacag uu 22

<210> 44
<211> 22
<212> RNA
<213> Homo sapiens

<400> 44
augcaccugg gcaaggauuc ug 22

<210> 45
<211> 22
<212> RNA
<213> Homo sapiens

<400> 45
uguaaacauc cuugacugga ag 22

<210> 46
<211> 21
<212> RNA
<213> Homo sapiens

<400> 46
uucacagugg cuaaguuccg c 21

<210> 47
<211> 22
<212> RNA
<213> Homo sapiens

<400> 47
gaauguugcu cggugaaccc cu 22

<210> 48
<211> 22
<212> RNA
<213> Homo sapiens

<400> 48
ucagugcauc acagaacuuu gu 22

<210> 49
<211> 22
<212> RNA
<213> Homo sapiens

<400> 49
acggguuagg cucuugggag cu 22

<210> 50
<211> 21
<212> RNA
<213> Homo sapiens

<400> 50
aauauaacac agauggccug u 21

<210> 51
<211> 22

<212> RNA
<213> Homo sapiens

<400> 51
uauacaaggg caagcucucu gu 22

<210> 52
<211> 22
<212> RNA
<213> Homo sapiens

<400> 52
aacccguaga uccgaucuug ug 22

<210> 53
<211> 22
<212> RNA
<213> Homo sapiens

<400> 53
agagguagua gguugcauag uu 22

<210> 54
<211> 22
<212> RNA
<213> Homo sapiens

<400> 54
ucagugcacu acagaacuuu gu 22

<210> 55
<211> 21
<212> RNA
<213> Homo sapiens

<400> 55
aucacauugc cagggauuuc c 21

<210> 56
<211> 22
<212> RNA
<213> Homo sapiens

<400> 56
cacuagauug ugagcuccug ga 22

<210> 57
<211> 23
<212> RNA
<213> Homo sapiens

<400> 57
agcucggucu gaggccccuc agu 23

<210> 58
<211> 23
<212> RNA
<213> Homo sapiens

<400> 58
ucuuugguua ucuagcugua uga 23

<210> 59
<211> 21
<212> RNA
<213> Homo sapiens

<400> 59
aucacauugc cagggauuac c 21

<210> 60
<211> 23
<212> RNA
<213> Homo sapiens

<400> 60
cacccggcug ugugcacaug ugc 23

<210> 61
<211> 18
<212> RNA
<213> Homo sapiens

<400> 61
agggggcggg cuccggcg 18

<210> 62
<211> 23
<212> RNA
<213> Homo sapiens

<400> 62
agcagcauug uacagggcua uga 23

<210> 63
<211> 22
<212> RNA
<213> Homo sapiens

<400> 63
cauugcacuu gucucggucu ga 22

<210> 64
<211> 21
<212> RNA
<213> Homo sapiens

<400> 64
uuaauaucgg acaaccauug u 21

<210> 65
<211> 21
<212> RNA
<213> Homo sapiens

<400> 65
acuggacuug gagucagaag g 21

<210> 66
<211> 23
<212> RNA
<213> Homo sapiens

<400> 66
uguaaacauc cuacacucuc age 23

<210> 67
<211> 20
<212> RNA
<213> Homo sapiens

<400> 67
uaacggccgc gguacccuaa 20

<210> 68
<211> 22
<212> RNA
<213> Homo sapiens

<400> 68
ucacaaguca ggcucuuggg ac 22

<210> 69
<211> 21
<212> RNA
<213> Homo sapiens

<400> 69
uccgguucuc agggcuccac c 21

<210> 70
<211> 22
<212> RNA
<213> Homo sapiens

<400> 70
uuaucagaau cuccaggggu ac 22

<210> 71
<211> 22
<212> RNA
<213> Homo sapiens

<400> 71
cuuucagucg gauguuuaca gc 22

<210> 72
<211> 22
<212> RNA
<213> Homo sapiens

<400> 72
cuuggcaccu agcaagcacu ca 22

<210> 73
<211> 22

<212> RNA
<213> Homo sapiens

<400> 73
ugagaaccac gucugcucug ag 22

<210> 74
<211> 22
<212> RNA
<213> Homo sapiens

<400> 74
uuauaauaca accugauaag ug 22

<210> 75
<211> 22
<212> RNA
<213> Homo sapiens

<400> 75
ugagaccucu ggguucugag cu 22

<210> 76
<211> 22
<212> RNA
<213> Homo sapiens

<400> 76
gcugacuccu aguccagggc uc 22

<210> 77
<211> 22
<212> RNA
<213> Homo sapiens

<400> 77
cuuucagucg gauguuugca gc 22

<210> 78
<211> 22
<212> RNA
<213> Homo sapiens

<400> 78
uagcagcaca ucaugguuua ca 22

<210> 79
<211> 23
<212> RNA
<213> Homo sapiens

<400> 79
agcuacauug ucugcugggu uuc 23

<210> 80
<211> 21
<212> RNA
<213> Homo sapiens

<400> 80
aggcaagaug cuggcauagc u 21

<210> 81
<211> 23
<212> RNA
<213> Homo sapiens

<400> 81
ucucacacag aaaucgcacc cgu 23

<210> 82
<211> 22
<212> RNA
<213> Homo sapiens

<400> 82
caucaucguc ucaaaugagu cu 22

<210> 83
<211> 22
<212> RNA
<213> Homo sapiens

<400> 83
ugaaggucua cugugugcca gg 22

<210> 84
<211> 17
<212> RNA
<213> Homo sapiens

<400> 84
ucucgcuggg gccucca 17

<210> 85
<211> 23
<212> RNA
<213> Homo sapiens

<400> 85
uggaagacua gugauuuugu ugu 23

<210> 86
<211> 22
<212> RNA
<213> Homo sapiens

<400> 86
cagugcaaug augaaagggc au 22

<210> 87
<211> 21
<212> RNA
<213> Homo sapiens

<400> 87
cugaccuaug aauugacagc c 21

<210> 88
<211> 22
<212> RNA
<213> Homo sapiens

<400> 88
uuguacaugg uaggcuuuca uu 22

<210> 89
<211> 22
<212> RNA
<213> Homo sapiens

<400> 89
uucccuuugu cauccuaugc cu 22

<210> 90
<211> 21
<212> RNA
<213> Homo sapiens

<400> 90
uucaaguaau ucaggauagg u 21

<210> 91
<211> 21
<212> RNA
<213> Homo sapiens

<400> 91
ucgaccggac cucgaccggc u 21

<210> 92
<211> 22
<212> RNA
<213> Homo sapiens

<400> 92
cuauacgacc ugcugccuuu cu 22

<210> 93
<211> 22
<212> RNA
<213> Homo sapiens

<400> 93
uuauaaagca augagacuga uu 22

<210> 94
<211> 22
<212> RNA
<213> Homo sapiens

<400> 94
ugugacuggu ugaccagagg gg 22

<210> 95
<211> 23

<212> RNA
<213> Homo sapiens

<400> 95
ucuuggagua ggucauuggg ugg 23

<210> 96
<211> 22
<212> RNA
<213> Homo sapiens

<400> 96
uguaaacauc cuacacucag cu 22

<210> 97
<211> 22
<212> RNA
<213> Homo sapiens

<400> 97
aaaagcuggg uugagagggc ga 22

<210> 98
<211> 22
<212> RNA
<213> Homo sapiens

<400> 98
caagcuugua ucuauaggua ug 22

<210> 99
<211> 22
<212> RNA
<213> Homo sapiens

<400> 99
ugcggggcua gggcuaacag ca 22

<210> 100
<211> 22
<212> RNA
<213> Homo sapiens

<400> 100
accaucgacc guugauugua cc 22

<210> 101
<211> 22
<212> RNA
<213> Homo sapiens

<400> 101
uggcaguguc uuagcugguu gu 22

<210> 102
<211> 22
<212> RNA
<213> Homo sapiens

<400> 102
accacugacc guugacugua cc 22

<210> 103
<211> 22
<212> RNA
<213> Homo sapiens

<400> 103
ugauauguuu gauauauuag gu 22

<210> 104
<211> 22
<212> RNA
<213> Homo sapiens

<400> 104
uaacagucua cagccauggu cg 22

<210> 105
<211> 22
<212> RNA
<213> Homo sapiens

<400> 105
aacauucaac euguegguga gu 22

<210> 106
<211> 22
<212> RNA
<213> Homo sapiens

<400> 106
uagcaccauc ugaaaueggu ua 22

<210> 107
<211> 23
<212> RNA
<213> Homo sapiens

<400> 107
cagugcaaua guauugucaa age 23

<210> 108
<211> 21
<212> RNA
<213> Homo sapiens

<400> 108
uaguagaccg uauagcguac g 21

<210> 109
<211> 21
<212> RNA
<213> Homo sapiens

<400> 109
ucacagugaa ccggucucuu u 21

<210> 110
<211> 17
<212> RNA
<213> Homo sapiens

<400> 110
gggagaaggg ucggggc 17

<210> 111
<211> 23
<212> RNA
<213> Homo sapiens

<400> 111
aaugacacga ucacucccgu uga 23

<210> 112
<211> 21
<212> RNA
<213> Homo sapiens

<400> 112
acucuuuccc uguugcacua c 21

<210> 113
<211> 22
<212> RNA
<213> Homo sapiens

<400> 113
cagugcaaug uuaaaagggc au 22

<210> 114
<211> 22
<212> RNA
<213> Homo sapiens

<400> 114
cuuucaguca gauguuugcu gc 22

<210> 115
<211> 22
<212> RNA
<213> Homo sapiens

<400> 115
uggugggccg cagaacaugu gc 22

<210> 116
<211> 23
<212> RNA
<213> Homo sapiens

<400> 116
caaagugcug uucgugcagg uag 23

<210> 117
<211> 22

<212> RNA
<213> Homo sapiens

<400> 117
aaucguacag ggucauccac uu 22

<210> 118
<211> 22
<212> RNA
<213> Homo sapiens

<400> 118
ucaggcucag uccccucccg au 22

<210> 119
<211> 22
<212> RNA
<213> Homo sapiens

<400> 119
uggcucaguu cagcaggaac ag 22

<210> 120
<211> 22
<212> RNA
<213> Homo sapiens

<400> 120
ucugugagac caaagaacua cu 22

<210> 121
<211> 23
<212> RNA
<213> Homo sapiens

<400> 121
ucuggcuccg ugucuucacu ccc 23

<210> 122
<211> 22
<212> RNA
<213> Homo sapiens

<400> 122
uucaccaccu ucuccaccca gc 22

<210> 123
<211> 23
<212> RNA
<213> Homo sapiens

<400> 123
uuuggcacua gcacauuuuu gcu 23

<210> 124
<211> 22
<212> RNA
<213> Homo sapiens

<400> 124
acucggcgug gcgucggucg ug 22

<210> 125
<211> 23
<212> RNA
<213> Homo sapiens

<400> 125
aggcagugua guuagcugau ugc 23

<210> 126
<211> 22
<212> RNA
<213> Homo sapiens

<400> 126
gccugcuggg guggaaccug gu 22

<210> 127
<211> 22
<212> RNA
<213> Homo sapiens

<400> 127
aaguucuguu auacacucag gc 22

<210> 128
<211> 23
<212> RNA
<213> Homo sapiens

<400> 128
ucaagagcaa uaacgaaaaa ugu 23

<210> 129
<211> 23
<212> RNA
<213> Homo sapiens

<400> 129
gauugagacu aguagggcua ggc 23

<210> 130
<211> 22
<212> RNA
<213> Homo sapiens

<400> 130
agggcuuagc ugcuugugag ca 22

<210> 131
<211> 22
<212> RNA
<213> Homo sapiens

<400> 131
aauugcacgg uauccaucug ua 22

<210> 132
<211> 21
<212> RNA
<213> Homo sapiens

<400> 132
ugucuugcag gccgucaugc a 21

<210> 133
<211> 20
<212> RNA
<213> Homo sapiens

<400> 133
guagaggaga uggcgcaggg 20

<210> 134
<211> 23
<212> RNA
<213> Homo sapiens

<400> 134
agugccugag ggaguaagag ccc 23

<210> 135
<211> 17
<212> RNA
<213> Homo sapiens

<400> 135
gcggggcugg gcgcgcg 17

<210> 136
<211> 22
<212> RNA
<213> Homo sapiens

<400> 136
uggugggcac agaaucugga cu 22

<210> 137
<211> 23
<212> RNA
<213> Homo sapiens

<400> 137
uauggcuuuu cauuccuaug uga 23

<210> 138
<211> 21
<212> RNA
<213> Homo sapiens

<400> 138
uaccacaggg uagaaccacg g 21

<210> 139
<211> 24

<212> RNA
<213> Homo sapiens

<400> 139
aauccuugga accuaggugu gagu 24

<210> 140
<211> 21
<212> RNA
<213> Homo sapiens

<400> 140
gcaguccaug ggcauauaca c 21

<210> 141
<211> 22
<212> RNA
<213> Homo sapiens

<400> 141
uuugugaccu gguccacuaa cc 22

<210> 142
<211> 21
<212> RNA
<213> Homo sapiens

<400> 142
uacaguacug ugauaacuga a 21

<210> 143
<211> 22
<212> RNA
<213> Homo sapiens

<400> 143
auauaauaca accugcuaag ug 22

<210> 144
<211> 22
<212> RNA
<213> Homo sapiens

<400> 144
aaaguucuga gacacuccga cu 22

<210> 145
<211> 23
<212> RNA
<213> Homo sapiens

<400> 145
caaagugcuu acagugcagg uag 23

<210> 146
<211> 23
<212> RNA
<213> Homo sapiens

<400> 146
uaaagugcuu auagugcagg uag 23

<210> 147
<211> 22
<212> RNA
<213> Homo sapiens

<400> 147
cugcccuggc ccgagggacc ga 22

<210> 148
<211> 22
<212> RNA
<213> Homo sapiens

<400> 148
aacuggcccu caaagucccg cu 22

<210> 149
<211> 22
<212> RNA
<213> Homo sapiens

<400> 149
caaaaacugc aguuacuuuu gc 22

<210> 150
<211> 22
<212> RNA
<213> Homo sapiens

<400> 150
aucauacaag gacaauuucu uu 22

<210> 151
<211> 23
<212> RNA
<213> Homo sapiens

<400> 151
aucaacagac auuaauuggg cgc 23

<210> 152
<211> 22
<212> RNA
<213> Homo sapiens

<400> 152
aaggagcuca cagucuauug ag 22

<210> 153
<211> 22
<212> RNA
<213> Homo sapiens

<400> 153
gucauacacg gcucuccucu cu 22

<210> 154
<211> 23
<212> RNA
<213> Homo sapiens

<400> 154
ugaggcucug uuagccuugg cuc 23

<210> 155
<211> 22
<212> RNA
<213> Homo sapiens

<400> 155
cgucccgggg cugcgcgagg ca 22

<210> 156
<211> 22
<212> RNA
<213> Homo sapiens

<400> 156
ugccuacuga gcugaaacac ag 22

<210> 157
<211> 23
<212> RNA
<213> Homo sapiens

<400> 157
aacauucauu guugucggug ggu 23

<210> 158
<211> 21
<212> RNA
<213> Homo sapiens

<400> 158
cacauuacac ggucgaccuc u 21

<210> 159
<211> 22
<212> RNA
<213> Homo sapiens

<400> 159
ccgcacugug gguacuugcu gc 22

<210> 160
<211> 22
<212> RNA
<213> Homo sapiens

<400> 160
acaggugagg uucuugggag cc 22

<210> 161
<211> 22

<212> RNA
<213> Homo sapiens

<400> 161
ucucugggcc ugugucuuag gc 22

<210> 162
<211> 17
<212> RNA
<213> Homo sapiens

<400> 162
gugggggaga ggcuguc 17

<210> 163
<211> 23
<212> RNA
<213> Homo sapiens

<400> 163
ugugcaaauc caugcaaaac uga 23

<210> 164
<211> 23
<212> RNA
<213> Homo sapiens

<400> 164
cagugcaaug auauugucaa age 23

<210> 165
<211> 22
<212> RNA
<213> Homo sapiens

<400> 165
agaggcuggc cgugaugaau uc 22

<210> 166
<211> 23
<212> RNA
<213> Homo sapiens

<400> 166
uagcaccauu ugaaaucagu guu 23

<210> 167
<211> 22
<212> RNA
<213> Homo sapiens

<400> 167
aagggcuucc ucucugcagg ac 22

<210> 168
<211> 22
<212> RNA
<213> Homo sapiens

<400> 168
caggucgucu ugcagggcuu cu 22

<210> 169
<211> 23
<212> RNA
<213> Homo sapiens

<400> 169
uagugcaaua uugcuuauag ggu 23

<210> 170
<211> 21
<212> RNA
<213> Homo sapiens

<400> 170
uaaagugcug acagugcaga u 21

<210> 171
<211> 19
<212> RNA
<213> Homo sapiens

<400> 171
accgugcaaa gguagcaua 19

<210> 172
<211> 22
<212> RNA
<213> Homo sapiens

<400> 172
ugcuaugcca acauauugcc au 22

<210> 173
<211> 22
<212> RNA
<213> Homo sapiens

<400> 173
cuuaucagau uguauuguaa uu 22

<210> 174
<211> 22
<212> RNA
<213> Homo sapiens

<400> 174
aucaugaugg gcuccucggu gu 22

<210> 175
<211> 18
<212> RNA
<213> Homo sapiens

<400> 175
ggugggcuuc ccggaggg 18

<210> 176
<211> 21
<212> RNA
<213> Homo sapiens

<400> 176
ugagaugaag cacuguagcu c 21

<210> 177
<211> 23
<212> RNA
<213> Homo sapiens

<400> 177
ugugcaaauc uaugcaaaac uga 23

<210> 178
<211> 22
<212> RNA
<213> Homo sapiens

<400> 178
caugccuuga guguaggacc gu 22

<210> 179
<211> 22
<212> RNA
<213> Homo sapiens

<400> 179
aucaaggauc uuaaacuuug cc 22

<210> 180
<211> 22
<212> RNA
<213> Homo sapiens

<400> 180
gguggcccgg ccgugccuga gg 22

<210> 181
<211> 24
<212> RNA
<213> Homo sapiens

<400> 181
uugcagcugc cugggaguga cuuc 24

<210> 182
<211> 22
<212> RNA
<213> Homo sapiens

<400> 182
uaugugggau gguaaaccgc uu 22

<210> 183
<211> 22

<212> RNA
<213> Homo sapiens

<400> 183
auaaagcuag auaaccgaaa gu 22

<210> 184
<211> 22
<212> RNA
<213> Homo sapiens

<400> 184
acugcaguga aggcacuugu ag 22

<210> 185
<211> 21
<212> RNA
<213> Homo sapiens

<400> 185
aacauagagg aaauuccacg u 21

<210> 186
<211> 22
<212> RNA
<213> Homo sapiens

<400> 186
cagcagcaau ucauguuuug aa 22

<210> 187
<211> 22
<212> RNA
<213> Homo sapiens

<400> 187
uacccauugc auaucggagu ug 22

<210> 188
<211> 22
<212> RNA
<213> Homo sapiens

<400> 188
uugcauaguc acaaaaguga uc 22

<210> 189
<211> 23
<212> RNA
<213> Homo sapiens

<400> 189
ugaggaugga uagcaaggaa gcc 23

<210> 190
<211> 24
<212> RNA
<213> Homo sapiens

<400> 190
cccggacagg cguucgugcg acgu 24

<210> 191
<211> 18
<212> RNA
<213> Homo sapiens

<400> 191
gggcucacau caccccau 18

<210> 192
<211> 22
<212> RNA
<213> Homo sapiens

<400> 192
uaugugccuu uggacuacau cg 22

<210> 193
<211> 22
<212> RNA
<213> Homo sapiens

<400> 193
aaacauucgc ggugcacuuc uu 22

<210> 194
<211> 20
<212> RNA
<213> Homo sapiens

<400> 194
uaaagagccc uguggagaca 20

<210> 195
<211> 23
<212> RNA
<213> Homo sapiens

<400> 195
gcaaagcaca cggccugcag aga 23

<210> 196
<211> 21
<212> RNA
<213> Homo sapiens

<400> 196
aauaauacau gguugaucuu u 21

<210> 197
<211> 22
<212> RNA
<213> Homo sapiens

<400> 197
ugagaccagg acuggaugca cc 22

<210> 198
<211> 22
<212> RNA
<213> Homo sapiens

<400> 198
aaaaguacuu gcggauuuug cu 22

<210> 199
<211> 22
<212> RNA
<213> Homo sapiens

<400> 199
ucaccagccc uguguucccu ag 22

<210> 200
<211> 21
<212> RNA
<213> Homo sapiens

<400> 200
cucccacaug caggguuugc a 21

<210> 201
<211> 22
<212> RNA
<213> Homo sapiens

<400> 201
agagcuuagc ugauugguga ac 22

<210> 202
<211> 22
<212> RNA
<213> Homo sapiens

<400> 202
agagguugcc cuuggugaau uc 22

<210> 203
<211> 22
<212> RNA
<213> Homo sapiens

<400> 203
aaucauacag ggacauccag uu 22

<210> 204
<211> 23
<212> RNA
<213> Homo sapiens

<400> 204
agguugggau cgguugcaau gcu 23

<210> 205
<211> 22

<212> RNA
<213> Homo sapiens

<400> 205
auguagggcu aaaagccaug gg 22

<210> 206
<211> 21
<212> RNA
<213> Homo sapiens

<400> 206
uugugcuuga ucuaaccaug u 21

<210> 207
<211> 23
<212> RNA
<213> Homo sapiens

<400> 207
uagcccccag gcuucacuug gcg 23

<210> 208
<211> 22
<212> RNA
<213> Homo sapiens

<400> 208
agggacggga cgcggugcag ug 22

<210> 209
<211> 22
<212> RNA
<213> Homo sapiens

<400> 209
auauacaggg ggagacucuu au 22

<210> 210
<211> 21
<212> RNA
<213> Homo sapiens

<400> 210
accacugcac uccagccuga g 21

<210> 211
<211> 21
<212> RNA
<213> Homo sapiens

<400> 211
auccccagau acaauggaca a 21

<210> 212
<211> 22
<212> RNA
<213> Homo sapiens

<400> 212
gggguuccug gggaugggau uu 22

<210> 213
<211> 22
<212> RNA
<213> Homo sapiens

<400> 213
cugggagagg guuguuuacu cc 22

<210> 214
<211> 22
<212> RNA
<213> Homo sapiens

<400> 214
aacacaccug guuaaccucu uu 22

<210> 215
<211> 22
<212> RNA
<213> Homo sapiens

<400> 215
cuccuauaug augccuuucu uc 22

<210> 216
<211> 24
<212> RNA
<213> Homo sapiens

<400> 216
caaagugaug aguaauacug gcug 24

<210> 217
<211> 22
<212> RNA
<213> Homo sapiens

<400> 217
cuccugacuc cagguccugu gu 22

<210> 218
<211> 23
<212> RNA
<213> Homo sapiens

<400> 218
gaggcugaug ugaguagacc acu 23

<210> 219
<211> 23
<212> RNA
<213> Homo sapiens

<400> 219
ugaguggggc ucccgggacg gcg 23

<210> 220
<211> 21
<212> RNA
<213> Homo sapiens

<400> 220
guuucaccau guuggucagg c 21

<210> 221
<211> 21
<212> RNA
<213> Homo sapiens

<400> 221
aauauuauac agucaaccuc u 21

<210> 222
<211> 21
<212> RNA
<213> Homo sapiens

<400> 222
cuauacaauc uacugucuuu c 21

<210> 223
<211> 22
<212> RNA
<213> Homo sapiens

<400> 223
uagcagcaca uaaugguuug ug 22

<210> 224
<211> 22
<212> RNA
<213> Homo sapiens

<400> 224
uggagagaaa ggcaguuccu ga 22

<210> 225
<211> 21
<212> RNA
<213> Homo sapiens

<400> 225
aggcggagac uugggcaauu g 21

<210> 226
<211> 23
<212> RNA
<213> Homo sapiens

<400> 226
ucaacaaaau cacugaugcu gga 23

<210> 227
<211> 19

<212> RNA
<213> Homo sapiens

<400> 227
acuggccugg gacuaccgg 19

<210> 228
<211> 23
<212> RNA
<213> Homo sapiens

<400> 228
ugagcgccuc gacgacagag ccg 23

<210> 229
<211> 22
<212> RNA
<213> Homo sapiens

<400> 229
cuuagcaggu uguauuauca uu 22

<210> 230
<211> 22
<212> RNA
<213> Homo sapiens

<400> 230
auggccagag cucacacaga gg 22

<210> 231
<211> 20
<212> RNA
<213> Homo sapiens

<400> 231
ggcuccuugg ucuaggggua 20

<210> 232
<211> 17
<212> RNA
<213> Homo sapiens

<400> 232
cuccgggacg gcugggc 17

<210> 233
<211> 22
<212> RNA
<213> Homo sapiens

<400> 233
gcuaaggaag uccugugcuc ag 22

<210> 234
<211> 22
<212> RNA
<213> Homo sapiens

<400> 234
ggagaaauua uccuuggugu gu 22

<210> 235
<211> 20
<212> RNA
<213> Homo sapiens

<400> 235
aaaagugauu gcaguguuug 20

<210> 236
<211> 21
<212> RNA
<213> Homo sapiens

<400> 236
ccaguccugu gccugccgcc u 21

<210> 237
<211> 21
<212> RNA
<213> Homo sapiens

<400> 237
aucauagagg aaaauccacg u 21

<210> 238
<211> 22
<212> RNA
<213> Homo sapiens

<400> 238
gaaguuguuc gugguggauu cg 22

<210> 239
<211> 22
<212> RNA
<213> Homo sapiens

<400> 239
cagcccggau cccagcccac uu 22

<210> 240
<211> 22
<212> RNA
<213> Homo sapiens

<400> 240
ugaaacauac acgggaaacc uc 22

<210> 241
<211> 22
<212> RNA
<213> Homo sapiens

<400> 241
aaacaaacau ggugcacuuc uu 22

<210> 242
<211> 22
<212> RNA
<213> Homo sapiens

<400> 242
ucagcaaaca uuuauugugu gc 22

<210> 243
<211> 22
<212> RNA
<213> Homo sapiens

<400> 243
caagcucgug ucuguggguc cg 22

<210> 244
<211> 21
<212> RNA
<213> Homo sapiens

<400> 244
uaggacacau ggucuacuuc u 21

<210> 245
<211> 21
<212> RNA
<213> Homo sapiens

<400> 245
cucacugaac aaugaaugca a 21

<210> 246
<211> 23
<212> RNA
<213> Homo sapiens

<400> 246
accuuggcuc uagacugcuu acu 23

<210> 247
<211> 22
<212> RNA
<213> Homo sapiens

<400> 247
caccuugcgc uacucagguc ug 22

<210> 248
<211> 22
<212> RNA
<213> Homo sapiens

<400> 248
uccgucucag uuacuuuaua gc 22

<210> 249
<211> 22

<212> RNA
<213> Homo sapiens

<400> 249
gcaugugaug aagcaaauca gu 22

<210> 250
<211> 23
<212> RNA
<213> Homo sapiens

<400> 250
ucccccaggu gugauucuga uuu 23

<210> 251
<211> 17
<212> RNA
<213> Homo sapiens

<400> 251
ggcgggugcg ggggugg 17

<210> 252
<211> 22
<212> RNA
<213> Homo sapiens

<400> 252
ccucccacac ccaaggcuug ca 22

<210> 253
<211> 22
<212> RNA
<213> Homo sapiens

<400> 253
cacgcucaug cacacaccca ca 22

<210> 254
<211> 23
<212> RNA
<213> Homo sapiens

<400> 254
agcagcauug uacagggcua uca 23

<210> 255
<211> 22
<212> RNA
<213> Homo sapiens

<400> 255
cugaagcuca gagggcucug au 22

<210> 256
<211> 23
<212> RNA
<213> Homo sapiens

<400> 256
uaaggugcau cuagugcaga uag 23

<210> 257
<211> 22
<212> RNA
<213> Homo sapiens

<400> 257
ccuauucuug auuacuuguu uc 22

<210> 258
<211> 21
<212> RNA
<213> Homo sapiens

<400> 258
agggcccccc cucaauccug u 21

<210> 259
<211> 21
<212> RNA
<213> Homo sapiens

<400> 259
agccgcgggg aucgccgagg g 21

<210> 260
<211> 21
<212> RNA
<213> Homo sapiens

<400> 260
aaucauucac ggacaacacu u 21

<210> 261
<211> 22
<212> RNA
<213> Homo sapiens

<400> 261
uacgcgcaga ccacaggaug uc 22

<210> 262
<211> 21
<212> RNA
<213> Homo sapiens

<400> 262
cuggauggcu ccuccauguc u 21

<210> 263
<211> 22
<212> RNA
<213> Homo sapiens

<400> 263
aguugccuuu uuguucccau gc 22

<210> 264
<211> 18
<212> RNA
<213> Homo sapiens

<400> 264
gggugcgggc cggcgggg 18

<210> 265
<211> 22
<212> RNA
<213> Homo sapiens

<400> 265
acccuaucaa uauugucucu gc 22

<210> 266
<211> 23
<212> RNA
<213> Homo sapiens

<400> 266
ccggucccag gagaaccugc aga 23

<210> 267
<211> 22
<212> RNA
<213> Homo sapiens

<400> 267
ugaguauuac auggccaauc uc 22

<210> 268
<211> 22
<212> RNA
<213> Homo sapiens

<400> 268
ccaaaacugc aguuacuuuu gc 22

<210> 269
<211> 27
<212> RNA
<213> Homo sapiens

<400> 269
accuucuugu auaagcacug ugcuaaa 27

<210> 270
<211> 24
<212> RNA
<213> Homo sapiens

<400> 270
agccuggaag cuggagccug cagu 24

<210> 271
<211> 22

<212> RNA
<213> Homo sapiens

<400> 271
uuagggcccu ggcuccaucu cc 22

<210> 272
<211> 23
<212> RNA
<213> Homo sapiens

<400> 272
acuccauuug uuuugaugau gga 23

<210> 273
<211> 23
<212> RNA
<213> Homo sapiens

<400> 273
cccaguguuc agacuaccug uuc 23

<210> 274
<211> 22
<212> RNA
<213> Homo sapiens

<400> 274
gaggguuggg uggaggcucu cc 22

<210> 275
<211> 21
<212> RNA
<213> Homo sapiens

<400> 275
ugcacggcac uggggacacg u 21

<210> 276
<211> 20
<212> RNA
<213> Homo sapiens

<400> 276
acugccccag gugcugcugg 20

<210> 277
<211> 21
<212> RNA
<213> Homo sapiens

<400> 277
gaacggcuuc auacaggagu u 21

<210> 278
<211> 21
<212> RNA
<213> Homo sapiens

<400> 278
aggggugcua ucugugauug a 21

<210> 279
<211> 22
<212> RNA
<213> Homo sapiens

<400> 279
uaaugccccu aaaaauccuu au 22

<210> 280
<211> 15
<212> RNA
<213> Homo sapiens

<400> 280
aggagauccu ggguu 15

<210> 281
<211> 22
<212> RNA
<213> Homo sapiens

<400> 281
aaugcaccug ggcaaggauu ca 22

<210> 282
<211> 22
<212> RNA
<213> Homo sapiens

<400> 282
cgucaacacu ugcugguuuc cu 22

<210> 283
<211> 22
<212> RNA
<213> Homo sapiens

<400> 283
ugucuuacuc ccucaggcac au 22

<210> 284
<211> 21
<212> RNA
<213> Homo sapiens

<400> 284
gccccgggca gugugaucau c 21

<210> 285
<211> 24
<212> RNA
<213> Homo sapiens

<400> 285
aaagacauag gauagaguca ccuc 24

<210> 286
<211> 22
<212> RNA
<213> Homo sapiens

<400> 286
auaauacaug guuaaccucu uu 22

<210> 287
<211> 23
<212> RNA
<213> Homo sapiens

<400> 287
uauucauuua uccccagccu aca 23

<210> 288
<211> 23
<212> RNA
<213> Homo sapiens

<400> 288
aggaagcccu ggaggggcug gag 23

<210> 289
<211> 20
<212> RNA
<213> Homo sapiens

<400> 289
cggcucuggg ucugugggga 20

<210> 290
<211> 22
<212> RNA
<213> Homo sapiens

<400> 290
cuauacggcc uccuagcuuu cc 22

<210> 291
<211> 18
<212> RNA
<213> Homo sapiens

<400> 291
cgggcguggu ggugggg 18

<210> 292
<211> 19
<212> RNA
<213> Homo sapiens

<400> 292
ggagauggag guugcagug 19

<210> 293
<211> 21

<212> RNA
<213> Homo sapiens

<400> 293
ugcaggacca agaugagccc u 21

<210> 294
<211> 24
<212> RNA
<213> Homo sapiens

<400> 294
uggcccugac ugaagaccag cagu 24

<210> 295
<211> 22
<212> RNA
<213> Homo sapiens

<400> 295
uaacacuguc ugguaaagau gg 22

<210> 296
<211> 21
<212> RNA
<213> Homo sapiens

<400> 296
cuccguuugc cuguuucgcu g 21

<210> 297
<211> 22
<212> RNA
<213> Homo sapiens

<400> 297
cuggcccucu cugcccuucc gu 22

<210> 298
<211> 21
<212> RNA
<213> Homo sapiens

<400> 298
caaaacguga ggcgcugcua u 21

<210> 299
<211> 20
<212> RNA
<213> Homo sapiens

<400> 299
ggauccgagu cacggcacca 20

<210> 300
<211> 17
<212> RNA
<213> Homo sapiens

<400> 300
gagacugggg uggggcc 17

<210> 301
<211> 21
<212> RNA
<213> Homo sapiens

<400> 301
uuagugcaua gucuuugguc u 21

<210> 302
<211> 22
<212> RNA
<213> Homo sapiens

<400> 302
uuaauuuuuu guuucgguca cu 22

<210> 303
<211> 23
<212> RNA
<213> Homo sapiens

<400> 303
uaauccuugc uaccugggug aga 23

<210> 304
<211> 22
<212> RNA
<213> Homo sapiens

<400> 304
aaaaguaauu gugguuuugg cc 22

<210> 305
<211> 24
<212> RNA
<213> Homo sapiens

<400> 305
cugaagugau guguaacuga ucag 24

<210> 306
<211> 22
<212> RNA
<213> Homo sapiens

<400> 306
auucuaauuu cuccacgucu uu 22

<210> 307
<211> 22
<212> RNA
<213> Homo sapiens

<400> 307
gacuauagaa cuuucccccu ca 22

<210> 308
<211> 21
<212> RNA
<213> Homo sapiens

<400> 308
aauggcgcca cuaggguugu g 21

<210> 309
<211> 20
<212> RNA
<213> Homo sapiens

<400> 309
accaggaggc ugaggccccu 20

<210> 310
<211> 22
<212> RNA
<213> Homo sapiens

<400> 310
acuccagccc cacagccuca gc 22

<210> 311
<211> 23
<212> RNA
<213> Homo sapiens

<400> 311
ccaguuaccg cuuccgcuac cgc 23

<210> 312
<211> 22
<212> RNA
<213> Homo sapiens

<400> 312
auccgcgcuc ugacucucug cc 22

<210> 313
<211> 22
<212> RNA
<213> Homo sapiens

<400> 313
uuuccggcuc gcgugggugu gu 22

<210> 314
<211> 22
<212> RNA
<213> Homo sapiens

<400> 314
auauacaggg ggagacucuc au 22

<210> 315
<211> 22

<212> RNA
<213> Homo sapiens

<400> 315
accguggcuu ucgauuguua cu 22

<210> 316
<211> 22
<212> RNA
<213> Homo sapiens

<400> 316
ccaauauuac ugugcugcuu ua 22

<210> 317
<211> 23
<212> RNA
<213> Homo sapiens

<400> 317
caaagugcuc auagugcagg uag 23

<210> 318
<211> 21
<212> RNA
<213> Homo sapiens

<400> 318
ccucccaugc caagaacucc c 21

<210> 319
<211> 22
<212> RNA
<213> Homo sapiens

<400> 319
ugguuuaccg ucccacauac au 22

<210> 320
<211> 22
<212> RNA
<213> Homo sapiens

<400> 320
cugggaggug gauguuuacu uc 22

<210> 321
<211> 22
<212> RNA
<213> Homo sapiens

<400> 321
cugggagaag gcuguuuacu cu 22

<210> 322
<211> 20
<212> RNA
<213> Homo sapiens

<400> 322
uuggccaugg ggcugcgcgg 20

<210> 323
<211> 21
<212> RNA
<213> Homo sapiens

<400> 323
ucucucggcu ccucgcggcu c 21

<210> 324
<211> 22
<212> RNA
<213> Homo sapiens

<400> 324
ucacccugca ucccgcaccc ag 22

<210> 325
<211> 19
<212> RNA
<213> Homo sapiens

<400> 325
gacuggacaa gcugaggaa 19

<210> 326
<211> 24
<212> RNA
<213> Homo sapiens

<400> 326
gaaaaugaug aguagugacu gaug 24

<210> 327
<211> 22
<212> RNA
<213> Homo sapiens

<400> 327
uaguggauga ugcacucugu gc 22

<210> 328
<211> 17
<212> RNA
<213> Homo sapiens

<400> 328
accccacucc ugguacc 17

<210> 329
<211> 22
<212> RNA
<213> Homo sapiens

<400> 329
cacccccugu uuccuggccc ac 22

<210> 330
<211> 21
<212> RNA
<213> Homo sapiens

<400> 330
acacaugggu ggcuguggcc u 21

<210> 331
<211> 22
<212> RNA
<213> Homo sapiens

<400> 331
ugugacagau ugauaacuga aa 22

<210> 332
<211> 22
<212> RNA
<213> Homo sapiens

<400> 332
cagggaaaug ggaagaacua ga 22

<210> 333
<211> 22
<212> RNA
<213> Homo sapiens

<400> 333
cgaaaacagc aauuaccuuu gc 22

<210> 334
<211> 23
<212> RNA
<213> Homo sapiens

<400> 334
ugagugugug ugugugagug ugu 23

<210> 335
<211> 21
<212> RNA
<213> Homo sapiens

<400> 335
ucuaguaaga guggcagucg a 21

<210> 336
<211> 22
<212> RNA
<213> Homo sapiens

<400> 336
uaugucugcu gaccaucacc uu 22

<210> 337
<211> 23

<212> RNA
<213> Homo sapiens

<400> 337
cugggaucuc cggggucuug guu 23

<210> 338
<211> 21
<212> RNA
<213> Homo sapiens

<400> 338
cugacuguug ccguccucca g 21

<210> 339
<211> 22
<212> RNA
<213> Homo sapiens

<400> 339
cuauacaacc uacugccuuc cc 22

<210> 340
<211> 26
<212> RNA
<213> Homo sapiens

<400> 340
aaguaguugg uuuguaugag augguu 26

<210> 341
<211> 23
<212> RNA
<213> Homo sapiens

<400> 341
aggaugagca aagaaaguag auu 23

<210> 342
<211> 22
<212> RNA
<213> Homo sapiens

<400> 342
uugcuugaac ccaggaagug ga 22

<210> 343
<211> 23
<212> RNA
<213> Homo sapiens

<400> 343
uggaguccag gaaucugcau uuu 23

<210> 344
<211> 21
<212> RNA
<213> Homo sapiens

<400> 344
ucagugcaug acagaacuug g 21

<210> 345
<211> 22
<212> RNA
<213> Homo sapiens

<400> 345
uguaacagca acuccaugug ga 22

<210> 346
<211> 21
<212> RNA
<213> Homo sapiens

<400> 346
uagcagcaca gaaauauugg c 21

<210> 347
<211> 23
<212> RNA
<213> Homo sapiens

<400> 347
uaauacugcc ggguaaugau gga 23

<210> 348
<211> 21
<212> RNA
<213> Homo sapiens

<400> 348
uaacagucuc cagucacggc c 21

<210> 349
<211> 22
<212> RNA
<213> Homo sapiens

<400> 349
cucaguagcc aguguagauc cu 22

<210> 350
<211> 23
<212> RNA
<213> Homo sapiens

<400> 350
ucagcaccag gauauuguug gag 23

<210> 351
<211> 20
<212> RNA
<213> Homo sapiens

<400> 351
auaugggguuu acuaguuggu 20

<210> 352
<211> 22
<212> RNA
<213> Homo sapiens

<400> 352
ugagggacag augccagaag ca 22

<210> 353
<211> 23
<212> RNA
<213> Homo sapiens

<400> 353
uagugaguua gagaugcaga gcc 23

<210> 354
<211> 23
<212> RNA
<213> Homo sapiens

<400> 354
cgcauccccu agggcauugg ugu 23

<210> 355
<211> 21
<212> RNA
<213> Homo sapiens

<400> 355
gugcauugua guugcauugc a 21

<210> 356
<211> 22
<212> RNA
<213> Homo sapiens

<400> 356
cucgugggcu cuggccacgg cc 22

<210> 357
<211> 22
<212> RNA
<213> Homo sapiens

<400> 357
agaucgaccg uguuauauuc gc 22

<210> 358
<211> 22
<212> RNA
<213> Homo sapiens

<400> 358
aucgggaaug ucguguccgc cc 22

<210> 359
<211> 17

<212> RNA
<213> Homo sapiens

<400> 359
gaagauggac guacuuu 17

<210> 360
<211> 22
<212> RNA
<213> Homo sapiens

<400> 360
uggcggcggu aguuaugggc uu 22

<210> 361
<211> 22
<212> RNA
<213> Homo sapiens

<400> 361
uuucuauuuc ucaguggggc uc 22

<210> 362
<211> 22
<212> RNA
<213> Homo sapiens

<400> 362
aggacuggac ucccggcagc cc 22

<210> 363
<211> 18
<212> RNA
<213> Homo sapiens

<400> 363
cggugagcgc ucgcuggc 18

<210> 364
<211> 22
<212> RNA
<213> Homo sapiens

<400> 364
aggaccuucc cugaaccaag ga 22

<210> 365
<211> 22
<212> RNA
<213> Homo sapiens

<400> 365
aggcggggcg ccgcgggacc gc 22

<210> 366
<211> 21
<212> RNA
<213> Homo sapiens

<400> 366
aaggcagggc ccccgcuccc c 21

<210> 367
<211> 22
<212> RNA
<213> Homo sapiens

<400> 367
caagcucgcu ucuaugggucug 22

<210> 368
<211> 21
<212> RNA
<213> Homo sapiens

<400> 368
agaggauacc cuuuguaugu u 21

<210> 369
<211> 22
<212> RNA
<213> Homo sapiens

<400> 369
ugagccccug ugccgccccc ag 22

<210> 370
<211> 21
<212> RNA
<213> Homo sapiens

<400> 370
uccuucugcu ccgucccccag 21

<210> 371
<211> 22
<212> RNA
<213> Homo sapiens

<400> 371
agaaggaaau ugaauucauu ua 22

<210> 372
<211> 21
<212> RNA
<213> Homo sapiens

<400> 372
agugaaugau ggguucugac c 21

<210> 373
<211> 19
<212> RNA
<213> Homo sapiens

<400> 373
aucccaccac ugccaccau 19

<210> 374
<211> 25
<212> RNA
<213> Homo sapiens

<400> 374
gaacccauga gguugaggcu gcagu 25

<210> 375
<211> 19
<212> RNA
<213> Homo sapiens

<400> 375
uggauuuuug gaucaggga 19

<210> 376
<211> 18
<212> RNA
<213> Homo sapiens

<400> 376
acguuggcuc ugguggug 18

<210> 377
<211> 18
<212> RNA
<213> Homo sapiens

<400> 377
cagggaggug aaugugau 18

<210> 378
<211> 22
<212> RNA
<213> Homo sapiens

<400> 378
cuccuggggc ccgcacucuc gc 22

<210> 379
<211> 23
<212> RNA
<213> Homo sapiens

<400> 379
agcugguguu gugaaucagg ccg 23

<210> 380
<211> 22
<212> RNA
<213> Homo sapiens

<400> 380
cagugguuuu acccuauggu ag 22

<210> 381
<211> 22

<212> RNA
<213> Homo sapiens

<400> 381
ugcccugugg acucaguucu gg 22

<210> 382
<211> 22
<212> RNA
<213> Homo sapiens

<400> 382
gcccaaaggu gaauuuuuug gg 22

<210> 383
<211> 21
<212> RNA
<213> Homo sapiens

<400> 383
cggcggggac ggcgauuggu c 21

<210> 384
<211> 20
<212> RNA
<213> Homo sapiens

<400> 384
ccccagggcg acgcggcggg 20

<210> 385
<211> 22
<212> RNA
<213> Homo sapiens

<400> 385
accuugccuu gcugcccggg cc 22

<210> 386
<211> 22
<212> RNA
<213> Homo sapiens

<400> 386
aacuggccua caaaguccca gu 22

<210> 387
<211> 23
<212> RNA
<213> Homo sapiens

<400> 387
aguuuugcag guuugcaucc agc 23

<210> 388
<211> 22
<212> RNA
<213> Homo sapiens

<400> 388
auaagacgaa caaaagguuu gu 22

<210> 389
<211> 22
<212> RNA
<213> Homo sapiens

<400> 389
uuggggaaac ggccgcugag ug 22

<210> 390
<211> 22
<212> RNA
<213> Homo sapiens

<400> 390
agaguugagu cuggacgucc cg 22

<210> 391
<211> 22
<212> RNA
<213> Homo sapiens

<400> 391
ccuguucucc auuacuuggc uc 22

<210> 392
<211> 23
<212> RNA
<213> Homo sapiens

<400> 392
agaauugcgu uuggacaauc agu 23

<210> 393
<211> 22
<212> RNA
<213> Homo sapiens

<400> 393
acugauuucu uuugguguuc ag 22

<210> 394
<211> 22
<212> RNA
<213> Homo sapiens

<400> 394
caucuggcau ccgucacaca ga 22

<210> 395
<211> 21
<212> RNA
<213> Homo sapiens

<400> 395
gcugcaccgg agacugggua a 21

<210> 396
<211> 21
<212> RNA
<213> Homo sapiens

<400> 396
uacccagucu ccggugcagc c 21

<210> 397
<211> 21
<212> RNA
<213> Homo sapiens

<400> 397
accugaauua ccaaaagcuu u 21

<210> 398
<211> 21
<212> RNA
<213> Homo sapiens

<400> 398
ccaggcucug cagugggaac u 21

<210> 399
<211> 22
<212> RNA
<213> Homo sapiens

<400> 399
cugcccuagu cuagcugaag cu 22

<210> 400
<211> 22
<212> RNA
<213> Homo sapiens

<400> 400
uggggcggag cuuccggagg cc 22

<210> 401
<211> 23
<212> RNA
<213> Homo sapiens

<400> 401
agguuguccg uggugaguuc gca 23

<210> 402
<211> 23
<212> RNA
<213> Homo sapiens

<400> 402
ucccuguccu ccaggagcuc acg 23

<210> 403
<211> 22

<212> RNA
<213> Homo sapiens

<400> 403
caaucagcaa guauacugcc cu 22

<210> 404
<211> 22
<212> RNA
<213> Homo sapiens

<400> 404
caaucacuaa cuccacugcc au 22

<210> 405
<211> 22
<212> RNA
<213> Homo sapiens

<400> 405
aaucacuaac cacacggcca gg 22

<210> 406
<211> 22
<212> RNA
<213> Homo sapiens

<400> 406
uuuaagaaaa caccauggag au 22

<210> 407
<211> 23
<212> RNA
<213> Homo sapiens

<400> 407
agggacuuuu gggggcagau gug 23

<210> 408
<211> 20
<212> RNA
<213> Homo sapiens

<400> 408
ccguguuucc cccacgcuuu 20

<210> 409
<211> 23
<212> RNA
<213> Homo sapiens

<400> 409
aguggaugau ggagacucgg uac 23

<210> 410
<211> 22
<212> RNA
<213> Homo sapiens

<400> 410
guagauucuc cuucuaugag ua 22

<210> 411
<211> 20
<212> RNA
<213> Homo sapiens

<400> 411
acugggcuug gagucagaag 20

<210> 412
<211> 22
<212> RNA
<213> Homo sapiens

<400> 412
uguccucuag ggccugcagu cu 22

<210> 413
<211> 22
<212> RNA
<213> Homo sapiens

<400> 413
ggaggaaccu uggagcuucg gc 22

<210> 414
<211> 21
<212> RNA
<213> Homo sapiens

<400> 414
uuucagauaa caguauuaca u 21

<210> 415
<211> 21
<212> RNA
<213> Homo sapiens

<400> 415
ugugcagcag gccaaccgag a 21

<210> 416
<211> 20
<212> RNA
<213> Homo sapiens

<400> 416
ggcggcggcg gaggcggggg 20

<210> 417
<211> 20
<212> RNA
<213> Homo sapiens

<400> 417
uguuccucug ucucccagac 20

<210> 418
<211> 18
<212> RNA
<213> Homo sapiens

<400> 418
cucgaguugg aagaggcg 18

<210> 419
<211> 22
<212> RNA
<213> Homo sapiens

<400> 419
uggggauuug gagaaguggu ga 22

<210> 420
<211> 22
<212> RNA
<213> Homo sapiens

<400> 420
auggcaucgu ccccuggugg cu 22

<210> 421
<211> 23
<212> RNA
<213> Homo sapiens

<400> 421
agggaaaaaa aaaaggauuu guc 23

<210> 422
<211> 22
<212> RNA
<213> Homo sapiens

<400> 422
uccaggcagg agccggacug ga 22

<210> 423
<211> 22
<212> RNA
<213> Homo sapiens

<400> 423
uaggggcagc agaggaccug gg 22

<210> 424
<211> 26
<212> RNA
<213> Homo sapiens

<400> 424
cacaggacug acuccucacc ccagug 26

<210> 425
<211> 22

<212> RNA
<213> Homo sapiens

<400> 425
cacacaagug gcccccaaca cu 22

<210> 426
<211> 20
<212> RNA
<213> Homo sapiens

<400> 426
cgccccuccu gcccccacag 20

<210> 427
<211> 24
<212> RNA
<213> Homo sapiens

<400> 427
ggugggaugg agagaaggua ugag 24

<210> 428
<211> 21
<212> RNA
<213> Homo sapiens

<400> 428
aguggaccga ggaaggaagg a 21

<210> 429
<211> 22
<212> RNA
<213> Homo sapiens

<400> 429
agugggg aac ccuuccauga gg 22

<210> 430
<211> 22
<212> RNA
<213> Homo sapiens

<400> 430
aauccuuugu cccuggguga ga 22

<210> 431
<211> 22
<212> RNA
<213> Homo sapiens

<400> 431
aauccacgcu gagcuuggca uc 22

<210> 432
<211> 25
<212> RNA
<213> Homo sapiens

<400> 432
aaaggauucu gcugucgguc ccacu 25

<210> 433
<211> 23
<212> RNA
<213> Homo sapiens

<400> 433
ucggggauca ucaugucacg aga 23

<210> 434
<211> 21
<212> RNA
<213> Homo sapiens

<400> 434
gcgacccaua cuugguuuca g 21

<210> 435
<211> 21
<212> RNA
<213> Homo sapiens

<400> 435
ucagcuacua ccucuauuag g 21

<210> 436
<211> 21
<212> RNA
<213> Homo sapiens

<400> 436
uagauaaaau auugguaccu g 21

<210> 437
<211> 22
<212> RNA
<213> Homo sapiens

<400> 437
ucaguuccag gccaaccagg cu 22

<210> 438
<211> 24
<212> RNA
<213> Homo sapiens

<400> 438
ucagaacaaa ugccgguucc caga 24

<210> 439
<211> 22
<212> RNA
<213> Homo sapiens

<400> 439
acucaaaacc cuucagugac uu 22

<210> 440
<211> 22
<212> RNA
<213> Homo sapiens

<400> 440
augcugacau auuuacuaga gg 22

<210> 441
<211> 21
<212> RNA
<213> Homo sapiens

<400> 441
uggguuuacg uugggagaac u 21

<210> 442
<211> 22
<212> RNA
<213> Homo sapiens

<400> 442
uucauuugcc ucccagccua ca 22

<210> 443
<211> 24
<212> RNA
<213> Homo sapiens

<400> 443
acuggcuagg gaaaaugauu ggau 24

<210> 444
<211> 23
<212> RNA
<213> Homo sapiens

<400> 444
gcagcagaga auaggacuac guc 23

<210> 445
<211> 22
<212> RNA
<213> Homo sapiens

<400> 445
uauguaauau gguccacauc uu 22

<210> 446
<211> 22
<212> RNA
<213> Homo sapiens

<400> 446
uauguaacau gguccacuaa cu 22

<210> 447
<211> 22

<212> RNA
<213> Homo sapiens

<400> 447
cgaaucauua uuugcugcuc ua 22

<210> 448
<211> 23
<212> RNA
<213> Homo sapiens

<400> 448
uuaaugcuaa ucgugauagg ggu 23

<210> 449
<211> 22
<212> RNA
<213> Homo sapiens

<400> 449
aucacacaaa ggcaacuuuu gu 22

<210> 450
<211> 21
<212> RNA
<213> Homo sapiens

<400> 450
gccccugggc cuauccuaga a 21

<210> 451
<211> 21
<212> RNA
<213> Homo sapiens

<400> 451
augaccuaug aauugacaga c 21

<210> 452
<211> 22
<212> RNA
<213> Homo sapiens

<400> 452
aucauagagg aaaauccaug uu 22

<210> 453
<211> 21
<212> RNA
<213> Homo sapiens

<400> 453
cggggcagcu caguacagga u 21

<210> 454
<211> 22
<212> RNA
<213> Homo sapiens

<400> 454
acugcugagc uagcacuucc cg 22

<210> 455
<211> 18
<212> RNA
<213> Homo sapiens

<400> 455
ucgaggagcu cacagucu 18

<210> 456
<211> 22
<212> RNA
<213> Homo sapiens

<400> 456
gugaacgggc gccaucccga gg 22

<210> 457
<211> 23
<212> RNA
<213> Homo sapiens

<400> 457
ccucagggcu guagaacagg gcu 23

<210> 458
<211> 23
<212> RNA
<213> Homo sapiens

<400> 458
uaagugcuuc cauguuuuag uag 23

<210> 459
<211> 22
<212> RNA
<213> Homo sapiens

<400> 459
aaaaacugag acuacuuuug ca 22

<210> 460
<211> 23
<212> RNA
<213> Homo sapiens

<400> 460
uaagugcuuc cauguuugag ugu 23

<210> 461
<211> 22
<212> RNA
<213> Homo sapiens

<400> 461
uacgucaucg uugucaucgu ca 22

<210> 462
<211> 21
<212> RNA
<213> Homo sapiens

<400> 462
cauuauuacu uuugguacgc g 21

<210> 463
<211> 21
<212> RNA
<213> Homo sapiens

<400> 463
uaauuuuaug uauaagcuag u 21

<210> 464
<211> 22
<212> RNA
<213> Homo sapiens

<400> 464
ucugggcaac aaagugagac cu 22

<210> 465
<211> 23
<212> RNA
<213> Homo sapiens

<400> 465
uacccuguag aaccgaauuu gug 23

<210> 466
<211> 23
<212> RNA
<213> Homo sapiens

<400> 466
cugauaagaa cagaggccca gau 23

<210> 467
<211> 22
<212> RNA
<213> Homo sapiens

<400> 467
agaauugugg cuggacaucu gu 22

<210> 468
<211> 24
<212> RNA
<213> Homo sapiens

<400> 468
agcgcgggcu gagcgcugcc aguc 24

<210> 469
<211> 22

<212> RNA
<213> Homo sapiens

<400> 469
uguuguacuu uuuuuuuugu uc 22

<210> 470
<211> 22
<212> RNA
<213> Homo sapiens

<400> 470
uaacugguug aacaacugaa cc 22

<210> 471
<211> 23
<212> RNA
<213> Homo sapiens

<400> 471
aaaagugcuu acagugcagg uag 23

<210> 472
<211> 22
<212> RNA
<213> Homo sapiens

<400> 472
aaucauacac gguugaccua uu 22

<210> 473
<211> 22
<212> RNA
<213> Homo sapiens

<400> 473
uagcaccauu ugaaaucggu ua 22

<210> 474
<211> 22
<212> RNA
<213> Homo sapiens

<400> 474
gcugcgcuug gauuucgucc cc 22

<210> 475
<211> 22
<212> RNA
<213> Homo sapiens

<400> 475
uaauacugcc ugguaaugau ga 22

<210> 476
<211> 23
<212> RNA
<213> Homo sapiens

<400> 476
ugucacucgg cucggcccac uac 23

<210> 477
<211> 22
<212> RNA
<213> Homo sapiens

<400> 477
ugagaacuga auuccauggg uu 22

<210> 478
<211> 22
<212> RNA
<213> Homo sapiens

<400> 478
cugugcgugu gacagcggcu ga 22

<210> 479
<211> 21
<212> RNA
<213> Homo sapiens

<400> 479
cagcagcaca cugugguuug u 21

<210> 480
<211> 22
<212> RNA
<213> Homo sapiens

<400> 480
caacaaauca cagucugcca ua 22

<210> 481
<211> 22
<212> RNA
<213> Homo sapiens

<400> 481
ugaccuggga cucggacagc ug 22

<210> 482
<211> 22
<212> RNA
<213> Homo sapiens

<400> 482
uauguaacac gguccacuaa cc 22

<210> 483
<211> 22
<212> RNA
<213> Homo sapiens

<400> 483
cugcgcaagc uacugccuug cu 22

<210> 484
<211> 22
<212> RNA
<213> Homo sapiens

<400> 484
aacuagcucu guggauccug ac 22

<210> 485
<211> 22
<212> RNA
<213> Homo sapiens

<400> 485
uuaugguuug ccugggacug ag 22

<210> 486
<211> 22
<212> RNA
<213> Homo sapiens

<400> 486
cuguugccac uaaccucaac cu 22

<210> 487
<211> 23
<212> RNA
<213> Homo sapiens

<400> 487
uccaguacca cgugucaggg cca 23

<210> 488
<211> 22
<212> RNA
<213> Homo sapiens

<400> 488
uccugucuuu ccuuguugga gc 22

<210> 489
<211> 23
<212> RNA
<213> Homo sapiens

<400> 489
uuacaguugu ucaaccaguu acu 23

<210> 490
<211> 22
<212> RNA
<213> Homo sapiens

<400> 490
acgcccuucc cccccuucuu ca 22

<210> 491
<211> 22

<212> RNA
<213> Homo sapiens

<400> 491
gcucugacuu uauugcacua cu 22

<210> 492
<211> 22
<212> RNA
<213> Homo sapiens

<400> 492
uucugccucu guccaggucc uu 22

<210> 493
<211> 22
<212> RNA
<213> Homo sapiens

<400> 493
aaucagugaa ugccuugaac cu 22

<210> 494
<211> 22
<212> RNA
<213> Homo sapiens

<400> 494
acaguagucu gcacauuggu ua 22

<210> 495
<211> 22
<212> RNA
<213> Homo sapiens

<400> 495
aguucuucag uggcaagcuu ua 22

<210> 496
<211> 23
<212> RNA
<213> Homo sapiens

<400> 496
ccuccguguu accuguccuc uag 23

<210> 497
<211> 22
<212> RNA
<213> Homo sapiens

<400> 497
agacccuggu cugcacucua uc 22

<210> 498
<211> 22
<212> RNA
<213> Homo sapiens

<400> 498
uucuggaauu cugugugagg ga 22

<210> 499
<211> 21
<212> RNA
<213> Homo sapiens

<400> 499
caucccuugc augguggagg g 21

<210> 500
<211> 22
<212> RNA
<213> Homo sapiens

<400> 500
cuagguaugg ucccagggau cc 22

<210> 501
<211> 22
<212> RNA
<213> Homo sapiens

<400> 501
uagguuaucc guguugccuu cg 22

<210> 502
<211> 23
<212> RNA
<213> Homo sapiens

<400> 502
acugcccuaa gugcuccuuc ugg 23

<210> 503
<211> 23
<212> RNA
<213> Homo sapiens

<400> 503
uaagugcuuc cauguuuugg uga 23

<210> 504
<211> 20
<212> RNA
<213> Homo sapiens

<400> 504
ccucugggcc cuuccuccag 20

<210> 505
<211> 21
<212> RNA
<213> Homo sapiens

<400> 505
cuguacaggc cacugccuug c 21

<210> 506
<211> 22
<212> RNA
<213> Homo sapiens

<400> 506
ugccuacuga gcugauauca gu 22

<210> 507
<211> 25
<212> RNA
<213> Homo sapiens

<400> 507
aaauaugaug aaacucacag cugag 25

<210> 508
<211> 22
<212> RNA
<213> Homo sapiens

<400> 508
aagaugugga aaaauuggaa uc 22

<210> 509
<211> 23
<212> RNA
<213> Homo sapiens

<400> 509
cucucaccac ugcccuccca cag 23

<210> 510
<211> 22
<212> RNA
<213> Homo sapiens

<400> 510
uggguuccug gcaugcugau uu 22

<210> 511
<211> 23
<212> RNA
<213> Homo sapiens

<400> 511
acuuaaacgu ggauguacuu gcu 23

<210> 512
<211> 22
<212> RNA
<213> Homo sapiens

<400> 512
aauucccuug uagauaaccc gg 22

<210> 513
<211> 22

<212> RNA
<213> Homo sapiens

<400> 513
cuguacagcc uccuagcuuu cc 22

<210> 514
<211> 22
<212> RNA
<213> Homo sapiens

<400> 514
caaauucgua ucuaggggaa ua 22

<210> 515
<211> 22
<212> RNA
<213> Homo sapiens

<400> 515
ugcuggauca gugguucgag uc 22

<210> 516
<211> 23
<212> RNA
<213> Homo sapiens

<400> 516
guccaguuuu cccaggaauc ccu 23

<210> 517
<211> 24
<212> RNA
<213> Homo sapiens

<400> 517
gcugguuuca uauggugguu uaga 24

<210> 518
<211> 23
<212> RNA
<213> Homo sapiens

<400> 518
ucuggcaagu aaaaaacucu cau 23

<210> 519
<211> 20
<212> RNA
<213> Homo sapiens

<400> 519
gugcauugcu guugcauugc 20

<210> 520
<211> 22
<212> RNA
<213> Homo sapiens

<400> 520
uuuccuaccc uaccugaaga cu 22

<210> 521
<211> 21
<212> RNA
<213> Homo sapiens

<400> 521
acagggccgc agauggagac u 21

<210> 522
<211> 22
<212> RNA
<213> Homo sapiens

<400> 522
gaaaucaagc gugggugaga cc 22

<210> 523
<211> 22
<212> RNA
<213> Homo sapiens

<400> 523
uuugaggcua cagugagaug ug 22

<210> 524
<211> 23
<212> RNA
<213> Homo sapiens

<400> 524
cggcccgggc ugcugcuguu ccu 23

<210> 525
<211> 22
<212> RNA
<213> Homo sapiens

<400> 525
aaccaucgac cguugagugg ac 22

<210> 526
<211> 22
<212> RNA
<213> Homo sapiens

<400> 526
ugggucuuug cgggcgagau ga 22

<210> 527
<211> 21
<212> RNA
<213> Homo sapiens

<400> 527
ugauugucca aacgcaauuc u 21

<210> 528
<211> 22
<212> RNA
<213> Homo sapiens

<400> 528
uaggauuaca agugucggcc ac 22

<210> 529
<211> 22
<212> RNA
<213> Homo sapiens

<400> 529
ugcccugccu guuuucuccu uu 22

<210> 530
<211> 22
<212> RNA
<213> Homo sapiens

<400> 530
cggggagaga acgcagugac gu 22

<210> 531
<211> 22
<212> RNA
<213> Homo sapiens

<400> 531
aaucugagaa ggcgcacaag gu 22

<210> 532
<211> 23
<212> RNA
<213> Homo sapiens

<400> 532
uugaagagga ggugcucugu agc 23

<210> 533
<211> 24
<212> RNA
<213> Homo sapiens

<400> 533
gaugcgccgc ccacugcccc gcgc 24

<210> 534
<211> 21
<212> RNA
<213> Homo sapiens

<400> 534
uuaagacuug cagugauguu u 21

<210> 535
<211> 23

<212> RNA
<213> Homo sapiens

<400> 535
caaagacugc aauuacuuuu gcg 23

<210> 536
<211> 25
<212> RNA
<213> Homo sapiens

<400> 536
ugggaacggg uuccggcaga cgcug 25

<210> 537
<211> 22
<212> RNA
<213> Homo sapiens

<400> 537
cccugugccc ggcccacuuc ug 22

<210> 538
<211> 22
<212> RNA
<213> Homo sapiens

<400> 538
ccaauauugg cugugcugcu cc 22

<210> 539
<211> 22
<212> RNA
<213> Homo sapiens

<400> 539
uuuaacaugg ggguaccugc ug 22

<210> 540
<211> 22
<212> RNA
<213> Homo sapiens

<400> 540
caaccucgac gaucuccuca gc 22

<210> 541
<211> 22
<212> RNA
<213> Homo sapiens

<400> 541
cccaauacac ggucgaccuc uu 22

<210> 542
<211> 22
<212> RNA
<213> Homo sapiens

<400> 542
uuuuucauua uugcuccuga cc 22

<210> 543
<211> 20
<212> RNA
<213> Homo sapiens

<400> 543
acuguaaacg cuuucugaug 20

<210> 544
<211> 18
<212> RNA
<213> Homo sapiens

<400> 544
cuaagaaguu gacugaag 18

<210> 545
<211> 23
<212> RNA
<213> Homo sapiens

<400> 545
ugagcaccac acaggccggg cgc 23

<210> 546
<211> 21
<212> RNA
<213> Homo sapiens

<400> 546
auaggcacca aaaagcaaca a 21

<210> 547
<211> 22
<212> RNA
<213> Homo sapiens

<400> 547
uggcagugua uuguuagcug gu 22

<210> 548
<211> 22
<212> RNA
<213> Homo sapiens

<400> 548
gaagaacugu ugcauuugcc cu 22

<210> 549
<211> 19
<212> RNA
<213> Homo sapiens

<400> 549
cauccguccg ucuguccac 19

<210> 550
<211> 22
<212> RNA
<213> Homo sapiens

<400> 550
gggagccagg aaguauugau gu 22

<210> 551
<211> 22
<212> RNA
<213> Homo sapiens

<400> 551
caaaacuggc aauuacuuuu gc 22

<210> 552
<211> 21
<212> RNA
<213> Homo sapiens

<400> 552
uccucuucuc ccuccuccca g 21

<210> 553
<211> 23
<212> RNA
<213> Homo sapiens

<400> 553
agcuucuuua cagugcugcc uug 23

<210> 554
<211> 20
<212> RNA
<213> Homo sapiens

<400> 554
cgggcguggu ggugggggug 20

<210> 555
<211> 23
<212> RNA
<213> Homo sapiens

<400> 555
cuggagauau ggaagagcug ugu 23

<210> 556
<211> 22
<212> RNA
<213> Homo sapiens

<400> 556
uggguggucu ggagauuugu gc 22

<210> 557
<211> 19

<212> RNA
<213> Homo sapiens

<400> 557
gaugaugcug cugaugcug 19

<210> 558
<211> 22
<212> RNA
<213> Homo sapiens

<400> 558
ucucccaacc cuuguaccag ug 22

<210> 559
<211> 21
<212> RNA
<213> Homo sapiens

<400> 559
ugauauguuu gauauugggu u 21

<210> 560
<211> 22
<212> RNA
<213> Homo sapiens

<400> 560
cgggguuuug agggcgagau ga 22

<210> 561
<211> 22
<212> RNA
<213> Homo sapiens

<400> 561
acugcauuau gagcacuuaa ag 22

<210> 562
<211> 22
<212> RNA
<213> Homo sapiens

<400> 562
uaaucucagc uggcaacugu ga 22

<210> 563
<211> 23
<212> RNA
<213> Homo sapiens

<400> 563
caggcaguga cuguucagac guc 23

<210> 564
<211> 22
<212> RNA
<213> Homo sapiens

<400> 564
agauguccag ccacaauucu cg 22

<210> 565
<211> 23
<212> RNA
<213> Homo sapiens

<400> 565
uguguacaca cgugccaggc gcu 23

<210> 566
<211> 20
<212> RNA
<213> Homo sapiens

<400> 566
aaaagcuggg uugagagggu 20

<210> 567
<211> 22
<212> RNA
<213> Homo sapiens

<400> 567
aggugguccg uggcgcguuc gc 22

<210> 568
<211> 22
<212> RNA
<213> Homo sapiens

<400> 568
caggcacggg agcucaggug ag 22

<210> 569
<211> 21
<212> RNA
<213> Homo sapiens

<400> 569
uaacgcauaa uauggacaug u 21

<210> 570
<211> 22
<212> RNA
<213> Homo sapiens

<400> 570
ucaggugugg aaacugaggc ag 22

<210> 571
<211> 22
<212> RNA
<213> Homo sapiens

<400> 571
aagcaauacu guuaccugaa au 22

<210> 572
<211> 21
<212> RNA
<213> Homo sapiens

<400> 572
gaccgagagg gccucggcug u 21

<210> 573
<211> 23
<212> RNA
<213> Homo sapiens

<400> 573
ugggccaggg agcagcuggu ggg 23

<210> 574
<211> 22
<212> RNA
<213> Homo sapiens

<400> 574
accgaagacu gugcgcuaau cu 22

<210> 575
<211> 22
<212> RNA
<213> Homo sapiens

<400> 575
acaacaguga cuugcucucc aa 22

<210> 576
<211> 22
<212> RNA
<213> Homo sapiens

<400> 576
ugcugggggc cacaugagug ug 22

<210> 577
<211> 21
<212> RNA
<213> Homo sapiens

<400> 577
ccaaaucuug aucagaagcc u 21

<210> 578
<211> 22
<212> RNA
<213> Homo sapiens

<400> 578
agggggaugg cagagcaaaa uu 22

<210> 579
<211> 21

<212> RNA
<213> Homo sapiens

<400> 579
auccuugcua ucugggugcu a 21

<210> 580
<211> 22
<212> RNA
<213> Homo sapiens

<400> 580
aaaaguaauu gugguuuuug cc 22

<210> 581
<211> 22
<212> RNA
<213> Homo sapiens

<400> 581
aaaaguaauu gcggauuuug cc 22

<210> 582
<211> 22
<212> RNA
<213> Homo sapiens

<400> 582
aaaaguaauu gcggucuuug gu 22

<210> 583
<211> 20
<212> RNA
<213> Homo sapiens

<400> 583
cgugccaccc uuuuccccag 20

<210> 584
<211> 17
<212> RNA
<213> Homo sapiens

<400> 584
ucccaccgcu gccaccc 17

<210> 585
<211> 21
<212> RNA
<213> Homo sapiens

<400> 585
uggacugccc ugaucuggag a 21

<210> 586
<211> 22
<212> RNA
<213> Homo sapiens

<400> 586
uuuagagacg gggucuugcu cu 22

<210> 587
<211> 22
<212> RNA
<213> Homo sapiens

<400> 587
ucuacagugc acgugucucc ag 22

<210> 588
<211> 21
<212> RNA
<213> Homo sapiens

<400> 588
agggagggac gggggcugug c 21

<210> 589
<211> 22
<212> RNA
<213> Homo sapiens

<400> 589
ccaguauuaa cugugcugcu ga 22

<210> 590
<211> 21
<212> RNA
<213> Homo sapiens

<400> 590
ugagugccgg ugccugcccu g 21

<210> 591
<211> 21
<212> RNA
<213> Homo sapiens

<400> 591
caagucacua gugguuccgu u 21

<210> 592
<211> 22
<212> RNA
<213> Homo sapiens

<400> 592
ucugcaagug ucagaggcga gg 22

<210> 593
<211> 22
<212> RNA
<213> Homo sapiens

<400> 593
auuguccuug cuguuuggag au 22

<210> 594
<211> 22
<212> RNA
<213> Homo sapiens

<400> 594
ugaccgauuu cuccuggugu uc 22

<210> 595
<211> 22
<212> RNA
<213> Homo sapiens

<400> 595
gauaucagcu caguaggcac cg 22

<210> 596
<211> 21
<212> RNA
<213> Homo sapiens

<400> 596
cacagcaagu guagacaggc a 21

<210> 597
<211> 22
<212> RNA
<213> Homo sapiens

<400> 597
agcuuuuggg aauucaggua gu 22

<210> 598
<211> 22
<212> RNA
<213> Homo sapiens

<400> 598
uauaaaauga gggcaguaag ac 22

<210> 599
<211> 22
<212> RNA
<213> Homo sapiens

<400> 599
aggauuucag aaauacuggu gu 22

<210> 600
<211> 22
<212> RNA
<213> Homo sapiens

<400> 600
cggguggauc acgaugcaau uu 22

<210> 601
<211> 22

<212> RNA
<213> Homo sapiens

<400> 601
ugggcuggca gggcaagugc ug 22

<210> 602
<211> 22
<212> RNA
<213> Homo sapiens

<400> 602
ugugggaucu ggaggcaucu gg 22

<210> 603
<211> 22
<212> RNA
<213> Homo sapiens

<400> 603
uuucuucuua gacauggcaa cg 22

<210> 604
<211> 22
<212> RNA
<213> Homo sapiens

<400> 604
uuagccaauu guccaucuuu ag 22

<210> 605
<211> 23
<212> RNA
<213> Homo sapiens

<400> 605
uggagaucca gugcucgccc gau 23

<210> 606
<211> 22
<212> RNA
<213> Homo sapiens

<400> 606
ugaaacugga gcgccuggag ga 22

<210> 607
<211> 21
<212> RNA
<213> Homo sapiens

<400> 607
agcggugcuc cugcgggccg a 21

<210> 608
<211> 21
<212> RNA
<213> Homo sapiens

<400> 608
gagguuuggg gaggauuugc u 21

<210> 609
<211> 22
<212> RNA
<213> Homo sapiens

<400> 609
ucaggacacu ucugaacuug ga 22

<210> 610
<211> 23
<212> RNA
<213> Homo sapiens

<400> 610
uagcagcggg aacaguucug cag 23

<210> 611
<211> 24
<212> RNA
<213> Homo sapiens

<400> 611
ucugggcaca ggcggaugga cagg 24

<210> 612
<211> 22
<212> RNA
<213> Homo sapiens

<400> 612
caaaaacugc aauuacuuuu gc 22

<210> 613
<211> 20
<212> RNA
<213> Homo sapiens

<400> 613
aaaacugcag uuacuuuugc 20

<210> 614
<211> 22
<212> RNA
<213> Homo sapiens

<400> 614
ugugucacuc gaugaccacu gu 22

<210> 615
<211> 21
<212> RNA
<213> Homo sapiens

<400> 615
acagucugcu gagguuggag c 21

<210> 616
<211> 23
<212> RNA
<213> Homo sapiens

<400> 616
ugugcuugcu cgucccgccc gca 23

<210> 617
<211> 23
<212> RNA
<213> Homo sapiens

<400> 617
gggaugguag accggugacg ugc 23

<210> 618
<211> 21
<212> RNA
<213> Homo sapiens

<400> 618
ccccaccucc ucucuccuca g 21

<210> 619
<211> 22
<212> RNA
<213> Homo sapiens

<400> 619
uggaguguga caaugguguu ug 22

<210> 620
<211> 21
<212> RNA
<213> Homo sapiens

<400> 620
gugggcgggg gcaggugugu g 21

<210> 621
<211> 22
<212> RNA
<213> Homo sapiens

<400> 621
acccgucccg uucguccccg ga 22

<210> 622
<211> 22
<212> RNA
<213> Homo sapiens

<400> 622
cggaugagca aagaaagugg uu 22

<210> 623
<211> 22

<212> RNA
<213> Homo sapiens

<400> 623
cuggacugag ccaugcuacu gg 22

<210> 624
<211> 26
<212> RNA
<213> Homo sapiens

<400> 624
gaugaugaug gcagcaaauu cugaaa 26

<210> 625
<211> 22
<212> RNA
<213> Homo sapiens

<400> 625
ggcgacaaaa cgagacccug uc 22

<210> 626
<211> 20
<212> RNA
<213> Homo sapiens

<400> 626
ucguuugccu uuuucugcuu 20

<210> 627
<211> 22
<212> RNA
<213> Homo sapiens

<400> 627
ugugagguug gcauuguugu cu 22

<210> 628
<211> 22
<212> RNA
<213> Homo sapiens

<400> 628
ccaccucccc ugcaaacguc ca 22

<210> 629
<211> 23
<212> RNA
<213> Homo sapiens

<400> 629
uauggcuuuu uauuccuaug uga 23

<210> 630
<211> 23
<212> RNA
<213> Homo sapiens

<400> 630
uuauugcuua agaauacgcg uag 23

<210> 631
<211> 21
<212> RNA
<213> Homo sapiens

<400> 631
cauaaaguag aaagcacuac u 21

<210> 632
<211> 22
<212> RNA
<213> Homo sapiens

<400> 632
ggugcagugc ugcaucucug gu 22

<210> 633
<211> 22
<212> RNA
<213> Homo sapiens

<400> 633
caggccauau ugugcugccu ca 22

<210> 634
<211> 22
<212> RNA
<213> Homo sapiens

<400> 634
cugccaauuc cauaggucac ag 22

<210> 635
<211> 22
<212> RNA
<213> Homo sapiens

<400> 635
uaacacuguc ugguaacgau gu 22

<210> 636
<211> 21
<212> RNA
<213> Homo sapiens

<400> 636
gcugggaagg caaagggacg u 21

<210> 637
<211> 22
<212> RNA
<213> Homo sapiens

<400> 637
acagcaggca cagacaggca gu 22

<210> 638
<211> 21
<212> RNA
<213> Homo sapiens

<400> 638
ucuggcuguu guggugugca a 21

<210> 639
<211> 22
<212> RNA
<213> Homo sapiens

<400> 639
ugccuggaac auaguaggga cu 22

<210> 640
<211> 20
<212> RNA
<213> Homo sapiens

<400> 640
uagaggaagc uguggagaga 20

<210> 641
<211> 22
<212> RNA
<213> Homo sapiens

<400> 641
uccccuucug caggccugcu gg 22

<210> 642
<211> 22
<212> RNA
<213> Homo sapiens

<400> 642
uucagccagg cuagugcagu cu 22

<210> 643
<211> 22
<212> RNA
<213> Homo sapiens

<400> 643
agaaggggug aaauuuaaac gu 22

<210> 644
<211> 19
<212> RNA
<213> Homo sapiens

<400> 644
aucgggcccu cggcgccgg 19

<210> 645
<211> 23

<212> RNA
<213> Homo sapiens

<400> 645
ccugggcagc guguggcuga agg 23

<210> 646
<211> 20
<212> RNA
<213> Homo sapiens

<400> 646
ucuggccagc uacgucccca 20

<210> 647
<211> 22
<212> RNA
<213> Homo sapiens

<400> 647
guggaguccu ggggaaugga ga 22

<210> 648
<211> 22
<212> RNA
<213> Homo sapiens

<400> 648
aaaagcuggg uugagagggc aa 22

<210> 649
<211> 23
<212> RNA
<213> Homo sapiens

<400> 649
uuuaguguga uaauggcguu uga 23

<210> 650
<211> 22
<212> RNA
<213> Homo sapiens

<400> 650
ucagcaggca ggcuggugca gc 22

<210> 651
<211> 21
<212> RNA
<213> Homo sapiens

<400> 651
ugaguguugu cuacgagggc a 21

<210> 652
<211> 22
<212> RNA
<213> Homo sapiens

<400> 652
auuguagaac cuaagauugg cc 22

<210> 653
<211> 22
<212> RNA
<213> Homo sapiens

<400> 653
cuucccccca guaaucuuca uc 22

<210> 654
<211> 22
<212> RNA
<213> Homo sapiens

<400> 654
uuuguucguu cggcucgcgu ga 22

<210> 655
<211> 19
<212> RNA
<213> Homo sapiens

<400> 655
acuggacuug gaggcagaa 19

<210> 656
<211> 22
<212> RNA
<213> Homo sapiens

<400> 656
gagcaaugua gguagacugu uu 22

<210> 657
<211> 22
<212> RNA
<213> Homo sapiens

<400> 657
uguguggauc cuggaggagg ca 22

<210> 658
<211> 22
<212> RNA
<213> Homo sapiens

<400> 658
uuugggacug aucuugaugu cu 22

<210> 659
<211> 20
<212> RNA
<213> Homo sapiens

<400> 659
gcucggacug agcagguggg 20

<210> 660
<211> 23
<212> RNA
<213> Homo sapiens

<400> 660
uucgggcugg ccugcugcuc cgg 23

<210> 661
<211> 22
<212> RNA
<213> Homo sapiens

<400> 661
uaauacuguc ugguaaaacc gu 22

<210> 662
<211> 22
<212> RNA
<213> Homo sapiens

<400> 662
accugucugu ggaaaggagc ua 22

<210> 663
<211> 17
<212> RNA
<213> Homo sapiens

<400> 663
uuggaggcgu ggguuuu 17

<210> 664
<211> 22
<212> RNA
<213> Homo sapiens

<400> 664
ccaggaggcg gaggaggugg ag 22

<210> 665
<211> 22
<212> RNA
<213> Homo sapiens

<400> 665
cuagugcucu ccguuacaag ua 22

<210> 666
<211> 22
<212> RNA
<213> Homo sapiens

<400> 666
cgcgcggccg ugcucggagc ag 22

<210> 667
<211> 22

<212> RNA
<213> Homo sapiens

<400> 667
ugugacaaua gagaugaaca ug 22

<210> 668
<211> 22
<212> RNA
<213> Homo sapiens

<400> 668
uuuggacaga aaacacgcag gu 22

<210> 669
<211> 22
<212> RNA
<213> Homo sapiens

<400> 669
aacuguuugc agaggaaacu ga 22

<210> 670
<211> 22
<212> RNA
<213> Homo sapiens

<400> 670
aacucguguu caaagccuuu ag 22

<210> 671
<211> 22
<212> RNA
<213> Homo sapiens

<400> 671
uuucuucuua gacauggcag cu 22

<210> 672
<211> 22
<212> RNA
<213> Homo sapiens

<400> 672
cuuccggucu gugagccccg uc 22

<210> 673
<211> 23
<212> RNA
<213> Homo sapiens

<400> 673
cugggggacg cgugagcgcg age 23

<210> 674
<211> 21
<212> RNA
<213> Homo sapiens

<400> 674
auacauguca gauuguaugc c 21

<210> 675
<211> 22
<212> RNA
<213> Homo sapiens

<400> 675
aacgggaaug caggcuguau cu 22

<210> 676
<211> 23
<212> RNA
<213> Homo sapiens

<400> 676
ucugaguucc uggagccugg ucu 23

<210> 677
<211> 22
<212> RNA
<213> Homo sapiens

<400> 677
gagcaggcga ggcugggcug aa 22

<210> 678
<211> 22
<212> RNA
<213> Homo sapiens

<400> 678
agaagauugc agaguaaguu cc 22

<210> 679
<211> 25
<212> RNA
<213> Homo sapiens

<400> 679
agcggggagg aagugggcgc ugcuu 25

<210> 680
<211> 22
<212> RNA
<213> Homo sapiens

<400> 680
ugagggcucc aggugacggu gg 22

<210> 681
<211> 25
<212> RNA
<213> Homo sapiens

<400> 681
caugcugacc ucccuccugc cccag 25

<210> 682
<211> 23
<212> RNA
<213> Homo sapiens

<400> 682
ucaggcaaag ggauauuuac aga 23

<210> 683
<211> 22
<212> RNA
<213> Homo sapiens

<400> 683
aaggcccggg cuuuccuccc ag 22

<210> 684
<211> 22
<212> RNA
<213> Homo sapiens

<400> 684
ugcggggaca ggccagggca uc 22

<210> 685
<211> 22
<212> RNA
<213> Homo sapiens

<400> 685
agccaggcuc ugaagggaaa gu 22

<210> 686
<211> 21
<212> RNA
<213> Homo sapiens

<400> 686
cgccugccca gcccuccugc u 21

<210> 687
<211> 21
<212> RNA
<213> Homo sapiens

<400> 687
auagcaauug cucuuuugga a 21

<210> 688
<211> 22
<212> RNA
<213> Homo sapiens

<400> 688
aauguuggaa uccucgcuag ag 22

<210> 689
<211> 23

<212> RNA
<213> Homo sapiens

<400> 689
ucugcacugu gaguuggcug gcu 23

<210> 690
<211> 21
<212> RNA
<213> Homo sapiens

<400> 690
uugaaaggcu auuucuuggu c 21

<210> 691
<211> 21
<212> RNA
<213> Homo sapiens

<400> 691
ugcuguauug ucagguagug a 21

<210> 692
<211> 24
<212> RNA
<213> Homo sapiens

<400> 692
agggcuggac ucagcggcgg agcu 24

<210> 693
<211> 24
<212> RNA
<213> Homo sapiens

<400> 693
aauuugguuu cugaggcacu uagu 24

<210> 694
<211> 22
<212> RNA
<213> Homo sapiens

<400> 694
ugaggacagg gcaaauucac ga 22

<210> 695
<211> 21
<212> RNA
<213> Homo sapiens

<400> 695
uuucccuuuc cauccuggca g 21

<210> 696
<211> 23
<212> RNA
<213> Homo sapiens

<400> 696
cagggcucag ggauuggaug gag 23

<210> 697
<211> 22
<212> RNA
<213> Homo sapiens

<400> 697
auucugcauu uuuagcaagu uc 22

<210> 698
<211> 22
<212> RNA
<213> Homo sapiens

<400> 698
aacggcaaug acuuuuguac ca 22

<210> 699
<211> 22
<212> RNA
<213> Homo sapiens

<400> 699
gaaaguaauu gcuguuuuug cc 22

<210> 700
<211> 22
<212> RNA
<213> Homo sapiens

<400> 700
aaaaguuauu gcgguuuugg cu 22

<210> 701
<211> 21
<212> RNA
<213> Homo sapiens

<400> 701
aaaaguaauu gcgguuuuug c 21

<210> 702
<211> 22
<212> RNA
<213> Homo sapiens

<400> 702
caagaaccuc aguugcuuuu gu 22

<210> 703
<211> 22
<212> RNA
<213> Homo sapiens

<400> 703
auauuaccau uagcucaucu uu 22

<210> 704
<211> 22
<212> RNA
<213> Homo sapiens

<400> 704
uaaaacuuua agugugccua gg 22

<210> 705
<211> 22
<212> RNA
<213> Homo sapiens

<400> 705
cagagugaca agcugguuaa ag 22

<210> 706
<211> 21
<212> RNA
<213> Homo sapiens

<400> 706
acuggcauua gugggacuuu u 21

<210> 707
<211> 24
<212> RNA
<213> Homo sapiens

<400> 707
uacagaugca gauucucuga cuuc 24

<210> 708
<211> 22
<212> RNA
<213> Homo sapiens

<400> 708
cucauuuaag uagucugaug cc 22

<210> 709
<211> 19
<212> RNA
<213> Homo sapiens

<400> 709
uuauugucac guucugauu 19

<210> 710
<211> 22
<212> RNA
<213> Homo sapiens

<400> 710
uucuggauaa caugcugaag cu 22

<210> 711
<211> 22

<212> RNA
<213> Homo sapiens

<400> 711
uugugucaau augcgaugau gu 22

<210> 712
<211> 22
<212> RNA
<213> Homo sapiens

<400> 712
cacacacugc aauuacuuuu gc 22

<210> 713
<211> 21
<212> RNA
<213> Homo sapiens

<400> 713
cacaagguau ugguauuacc u 21

<210> 714
<211> 22
<212> RNA
<213> Homo sapiens

<400> 714
uccauuacac uacccugccu cu 22

<210> 715
<211> 22
<212> RNA
<213> Homo sapiens

<400> 715
ugugcgcagg gagaccucuc cc 22

<210> 716
<211> 17
<212> RNA
<213> Homo sapiens

<400> 716
cgcgccgggc ccggguu 17

<210> 717
<211> 18
<212> RNA
<213> Homo sapiens

<400> 717
cggggcggca ggggccuc 18

<210> 718
<211> 18
<212> RNA
<213> Homo sapiens

<400> 718
gaggcugaag gaagaugg 18

<210> 719
<211> 17
<212> RNA
<213> Homo sapiens

<400> 719
gcugggcgag gcuggca 17

<210> 720
<211> 22
<212> RNA
<213> Homo sapiens

<400> 720
guggguacgg cccagugggg gg 22

<210> 721
<211> 17
<212> RNA
<213> Homo sapiens

<400> 721
gcuucuguag uguaguc 17

<210> 722
<211> 18
<212> RNA
<213> Homo sapiens

<400> 722
cggcuggagg ugugagga 18

<210> 723
<211> 21
<212> RNA
<213> Homo sapiens

<400> 723
uuacaggcgu gaaccaccgc g 21

<210> 724
<211> 24
<212> RNA
<213> Homo sapiens

<400> 724
ugggcuaagg gagaugauug ggua 24

<210> 725
<211> 21
<212> RNA
<213> Homo sapiens

<400> 725
ccgucgccgc cacccgagcc g 21

<210> 726
<211> 22
<212> RNA
<213> Homo sapiens

<400> 726
gguccagagg ggagauaggu uc 22

<210> 727
<211> 24
<212> RNA
<213> Homo sapiens

<400> 727
cauagcccgg ucgcugguac auga 24

<210> 728
<211> 17
<212> RNA
<213> Homo sapiens

<400> 728
ccccgccacc gccuugg 17

<210> 729
<211> 24
<212> RNA
<213> Homo sapiens

<400> 729
ugucagugac uccugccccu uggu 24

<210> 730
<211> 22
<212> RNA
<213> Homo sapiens

<400> 730
ucuggggaug aggacagugu gu 22

<210> 731
<211> 21
<212> RNA
<213> Homo sapiens

<400> 731
ucucaguaag uggcacucug u 21

<210> 732
<211> 18
<212> RNA
<213> Homo sapiens

<400> 732
agagcagaag gaugagau 18

<210> 733
<211> 22

<212> RNA
<213> Homo sapiens

<400> 733
ucaggccagg cacaguggcu ca 22

<210> 734
<211> 22
<212> RNA
<213> Homo sapiens

<400> 734
acuuuccuca cucccgugaa gu 22

<210> 735
<211> 21
<212> RNA
<213> Homo sapiens

<400> 735
ugacagcgcc cugccuggcu c 21

<210> 736
<211> 17
<212> RNA
<213> Homo sapiens

<400> 736
aagacugaga ggaggga 17

<210> 737
<211> 23
<212> RNA
<213> Homo sapiens

<400> 737
agaacucuug cagucuuaga ugu 23

<210> 738
<211> 57
<212> DNA
<213> Homo sapiens

<220>
<223> genomic sequence of miRNA precursor

<400> 738
ggccgcgccc cgtttcccag gacaaagggc actccgcacc ggaccctggt cccagcg 57

<210> 739
<211> 64
<212> DNA
<213> Homo sapiens

<220>
<223> genomic sequence of miRNA precursor

<400> 739

cccactccct ggcgccgctt gtggagggcc caagtccttc tgattgaggc ccaacccgtg 60
gaag 64

<210> 740
<211> 56
<212> DNA
<213> Homo sapiens

<220>
<223> genomic sequence of miRNA precursor

<400> 740
cgccgggacc ggggtccggg gcggagtgcc cttcctcctg ggaaacgggg tgcggc 56

<210> 741
<211> 81
<212> DNA
<213> Homo sapiens

<220>
<223> genomic sequence of miRNA precursor

<400> 741

gcttcacgtc cccaccggcg gcggcggcgg tggcagtggc ggcggcggcg gcggtggcgg 60
cggcggcggc ggcggcggct c 81

<210> 742
<211> 89
<212> DNA
<213> Homo sapiens

<220>
<223> genomic sequence of miRNA precursor

<400> 742

gccgcccccg ccgccgccgc cgccgccgcc gccgccgccg ccgcccgctt tcggctcggg 60
cctcaggtga gtcggagggg ccgggcgcc 89

<210> 743
<211> 22
<212> RNA
<213> Homo sapiens

<400> 743
ggcggagugc ccuucuuccu gg 22

<210> 744
<211> 22
<212> RNA
<213> Homo sapiens

<400> 744
ggagggccca aguccuucug au 22

<210> 745
<211> 22
<212> RNA
<213> Homo sapiens

<400> 745
gaccaggguc cggugcggag ug 22

<210> 746
<211> 17
<212> DNA
<213> Homo sapiens

<220>
<223> genomic primer sequence

<400> 746
agggtccggt gcggagt 17

<210> 747
<211> 12
<212> RNA
<213> Bos taurus

<400> 747
ggguccggug cg 12

<210> 748
<211> 20
<212> DNA
<213> Homo sapiens

<220>
<223> genomic primer sequence

<400> 748
tgcggagtgc cctttgtcct 20

<210> 749
<211> 22
<212> DNA
<213> Homo sapiens

<220>
<223> genomic primer sequence

<400> 749
ggagggccca agtccttctg at 22

<210> 750
<211> 14
<212> RNA
<213> Caenorhabditis remanei

<400> 750
cccaagugcu ucug 14

<210> 751

<211> 18
<212> DNA
<213> Homo sapiens

<220>
<223> genomic primer sequence

<400> 751
cggagtgccc ttcttcct 18

<210> 752
<211> 12
<212> RNA
<213> Zea mays

<400> 752
gugcccuucu uc 12